# EUROPÄISCHE PATENTANMELDUNG

(11) **EP 2 048 242 A1**
(43) Veröffentlichungstag der Anmeldung: **15.04.2009**
(21) Anmeldenummer: 07019664.7
(22) Anmeldetag: 08.10.2007
(51) Int. Cl.: C12Q 1/68

(54) **Microarray für die Expressionsanalyse der zellulären Glykosylierungsmaschinerie**

(71) Anmelder: Max-Delbrück-Centrum für Molekulare Medizin (MDC), 13125 Berlin (DE)
(72) Erfinder: Kemmner, Wolfgang, 13469 Berlin (DE)
(74) Vertreter: Vossius & Partner

(57) **Zusammenfassung**

Die vorliegende Erfindung betrifft einen Träger, auf den ein Set von Sonden aufgebracht ist, wobei das Set umfasst: (a) Sonden, die in der Lage sind, spezifisch mit Nukleinsäuren zu hybridisieren, die Fucosyltransferasen kodieren; (b) Sonden, die in der Lage sind, spezifisch mit Nukleinsäuren zu hybridisieren, die Galactosyltransferasen kodieren; (c) Sonden, die in der Lage sind, spezifisch mit Nukleinsäuren zu hybridisieren, die Acetyl-glucosaminyltransferasen kodieren; (d) Sonden, die in der Lage sind, spezifisch mit Nukleinsäuren zu hybridisieren, die Acetyl-galactosaminyltransferasen kodieren; (e) Sonden, die in der Lage sind, spezifisch mit Nukleinsäuren zu hybridisieren, die Sialyltransferasen kodieren; (f) Sonden, die in der Lage sind, spezifisch mit Nukleinsäuren zu hybridisieren, die Zucker-Sulfotransferasen kodieren; (g) Sonden, die in der Lage sind, spezifisch mit Nukleinsäuren zu hybridisieren, die Lectine kodieren; (h) Sonden, die in der Lage sind, spezifisch mit Nukleinsäuren zu hybridisieren, die Selectine kodieren; (i) Sonden, die in der Lage sind, spezifisch mit Nukleinsäuren zu hybridisieren, die Fucosidasen kodieren; (j) Sonden, die in der Lage sind, spezifisch mit Nukleinsäuren zu hybridisieren, die Neuraminidasen (Sialidasen) kodieren; (k) Sonden, die in der Lage sind, spezifisch mit Nukleinsäuren zu hybridisieren, die Nucleotid-Zucker-Epimerasen kodieren; (l) Sonden, die in der Lage sind, spezifisch mit Nukleinsäuren zu hybridisieren, die Zucker-Kinasen kodieren; (m) Sonden, die in der Lage sind, spezifisch mit Nukleinsäuren zu hybridisieren, die Zucker-Epimerasen kodieren; (n) Sonden, die in der Lage sind, spezifisch mit Nukleinsäuren zu hybridisieren, die Zucker-Transporter kodieren; und (o) Sonden, die in der Lage sind, spezifisch mit Nukleinsäuren zu hybridisieren, die für Enzyme des Fucose- oder Sialinsäure-Metabolismus kodieren. Die Erfindung betrifft ferner einen Kit, welcher die erfindungsgemäßen Sonden umfasst sowie die Verwendung des erfindungsgemäßen Trägers oder Kits zur quantitativen Bestimmung von Expressionsprofilen in einer Probe, die einer Zelle, einem Gewebe oder einem Organismus entnommen ist. Die vorliegende Erfindung betrifft weiterhin eine diagnostische Zusammensetzung sowie die Verwendung des erfindungsgemäßen Sets von Sonden zur Herstellung diagnostischer Zusammensetzungen oder einer diagnostischen Vorrichtung zur Diagnose von Tumoren, entzündlichen und/oder neurologischen Erkrankungen.

## Beschreibung

Die vorliegende Erfindung betrifft einen Träger, auf den ein Set von Sonden aufgebracht ist, wobei das Set umfasst: (a) Sonden, die in der Lage sind, spezifisch mit Nukleinsäuren zu hybridisieren, die Fucosyltransferasen kodieren; (b) Sonden, die in der Lage sind, spezifisch mit Nukleinsäuren zu hybridisieren, die Galactosyltransferasen kodieren; (c) Sonden, die in der Lage sind, spezifisch mit Nukleinsäuren zu hybridisieren, die N-Acetyl-glucosaminyltransferasen kodieren; (d) Sonden, die in der Lage sind, spezifisch mit Nukleinsäuren zu hybridisieren, die N-Acetyl-galactosaminyltransferasen kodieren; (e) Sonden, die in der Lage sind, spezifisch mit Nukleinsäuren zu hybridisieren, die Sialyltransferasen kodieren; (f) Sonden, die in der Lage sind, spezifisch mit Nukleinsäuren zu hybridisieren, die Zucker-Sulfotransferasen kodieren; (g) Sonden, die in der Lage sind, spezifisch mit Nukleinsäuren zu hybridisieren, die Lectine kodieren; (h) Sonden, die in der Lage sind, spezifisch mit Nukleinsäuren zu hybridisieren, die Selectine kodieren; (i) Sonden, die in der Lage sind, spezifisch mit Nukleinsäuren zu hybridisieren, die Fucosidasen kodieren; (j) Sonden, die in der Lage sind, spezifisch mit Nukleinsäuren zu hybridisieren, die Neuraminidasen (Sialidasen) kodieren; (k) Sonden, die in der Lage sind, spezifisch mit Nukleinsäuren zu hybridisieren, die Nucleotid-Zucker-Epimerasen kodieren; (l) Sonden, die in der Lage sind, spezifisch mit Nukleinsäuren zu hybridisieren, die Zucker-Kinasen kodieren; (m) Sonden, die in der Lage sind, spezifisch mit Nukleinsäuren zu hybridisieren, die Zucker-Epimerasen kodieren; (n) Sonden, die in der Lage sind, spezifisch mit Nukleinsäuren zu hybridisieren, die Zucker-Transporter kodieren; und (o) Sonden, die in der Lage sind, spezifisch mit Nukleinsäuren zu hybridisieren, die für Enzyme des Fucose- oder Sialinsäure-Metabolismus kodieren. Die Erfindung betrifft ferner einen Kit, welcher die erfindungsgemäßen Sonden umfasst sowie die Verwendung des erfindungsgemäßen Trägers oder Kits zur quantitativen Bestimmung von Expressionsprofilen in einer Probe, die einer Zelle, einem Gewebe oder einem Organismus entnommen ist. Die vorliegende Erfindung betrifft weiterhin eine diagnostische Zusammensetzung sowie die Verwendung des erfindungsgemäßen Sets von Sonden zur Herstellung diagnostischer Zusammensetzungen oder einer diagnostischen Vorrichtung zur Diagnose von Tumoren, entzündlichen und/oder neurologischen Erkrankungen.

In der Beschreibung werden eine Reihe an Dokumenten inklusive Patentanmeldungen und Hersteller-Gebrauchsanweisungen aus dem Stand der Technik genannt. Während der Offenbarungsgehalt dieser Dokumente als nicht relevant für die Patentfähigkeit der Erfindung angesehen wird, ist er per Referenz in die vorliegende Beschreibung inkorporiert.

Eine wesentliche posttranslationale Modifikation ist die Glykosylierung, bei der Zucker enzymatisch an Proteine angehängt werden. Die Glykosylierung dient verschiedenen Funktionen, wie z.B. der Stabilisierung von Proteinen durch Schutz vor proteolytischem Abbau. Auch die korrekte Proteinkonformation und damit verbunden die Affinität für Bindungspartner kann von der Glykosylierung abhängen. Des weiteren beeinflusst die Glykosylierung von Proteinen deren intrazellulären Transport, trägt zur Zellinteraktion bei oder dient als struktureller Bestandteil von Zellmembranen. Die wichtigsten Zucker, die in Glykoproteinen eine Rolle spielen, sind Fucose, Galactose, Mannose, Glucose, Xylose, N-Acetylgalactosamin, N-Acetylglucosamin und N-Acetylneuraminsäure. Die Bindung an Proteine kann N-glykosidisch oder auch O-glykosidisch erfolgen. Die N-Glykosylierung erfolgt am endoplasmatischen Retikulum durch die Bindung eines Zuckers an die freie Aminogruppe von Asparagin. Die O-Glykosylierung hingegen erfolgt am GolgiApparat durch Bindung des Zuckers an die Hydroxygruppe von Serin, Threonin, Hydroxyprolin oder Hydroxylysin.

Für die Struktur des spezifischen Glykosylierungsmusters einer Zelle sind sowohl die Expression als auch die Aktivität der Moleküle der Glykosylierungsmaschinerie, wie zum Beispiel der Glykosyltransferasen, der abbauenden Enzyme (z.B. der Sialidasen), der Zucker-Transporter und der Glykoprotein-bindenden Moleküle, verantwortlich. Die große Anzahl an beschriebenen Glykosyltransferasen liegt wahrscheinlich in der hohen dreifachen Spezifität der Glykosyltransferasen begründet. Diese sind spezifisch für i) das Substrat, also den Zucker, welcher übertragen wird, ii) den Akzeptor, auf den dieser Zucker übertragen wird und iii) die Art der Bindung, welche zwischen den beiden Partnern geknüpft wird.

Sialyltransferasen, die die Übertragung einer Nukleotid-aktivierten Sialinsäure auf spezifische Zuckerseitenketten katalysieren, spielen für Glykoprotein-produzierende Zellen eine wichtige Rolle, da die terminale Sialinsäure von hoher Bedeutung für die biologische Aktivität und/oder die Halbwertszeit der Glykoproteine ist. Die Expression von Sialyltransferasen ist auch in der Diagnostik von Tumorerkrankungen von großer Bedeutung.

Auch Enzyme und Transporter, welche eine wichtige Rolle für den Zuckermetabolismus spielen, können für die Diagnostik verschiedener Erkrankungen von wesentlicher Bedeutung sein. Des weiteren sind diese Enzyme und Transporter für die rekombinante Herstellung von Glykoproteinen von Interesse.

Eine weitere wichtige Gruppe von Molekülen sind Lectine wie beispielsweise die Galectine, die Selectine und Sialinsäure-bindenden Lectine. Diese Lectine sind die Rezeptoren der Zuckerseitenketten auf der Zellmembran. Viele dieser Lectine sind an immunologischen Erkennungsmechanismen und z.B. der Induktion von Apoptose/Anoikis beteiligt.

Spezifische Veränderungen der Glykanstrukturen auf der Zelloberfläche sind charakteristisch für eine Reihe von Krankheitsbildern und können daher zu deren Diagnose herangezogen werden. Ein Beispiel ist die frühe Erkennung von Karzinomen mit einem erhöhten Metastasierungspotential. So tragen Leberzellen an ihrer Oberfläche Rezeptoren, die bestimmte Glykoproteine binden und auf diese Weise die Halbwertszeit von Serumproteinen regulieren. Das auf der Tumorzelloberfläche vorhandene Glykan Thomsen-Friedenreich TF wird von den Rezeptoren gebunden und erleichtert dadurch die Metastasierung der Tumorzellen in die Leber. Damit im Zusammenhang steht die Sialyltransferase ST6GalNAc-II, deren Überexpression mit einer erhöhten TF-Präsenz korreliert und die von prognostischer Bedeutung ist (Schneider *et al.,* 2001). Schon länger ist bekannt, dass bestimmte saure Zucker (Sialinsäuren) verstärkt in den Glykanstrukturen metastasierender Tumorzellen auftreten. Damit in Übereinstimmung steht, dass auch die Aktivität des die Bildung dieser Strukturen katalysierenden Enzyms (Sialyltransferase ST6Gal-I) in metastasierenden kolorektalen Karzinomen erhöht ist (Kemmner *et al,* 1994). Ähnliche Ergebnisse wurden in Untersuchungen von Magen-, Nieren- und Mammakarzinomen gefunden.
Andere Glykanstrukturen sind für die Bindung von im Blut zirkulierenden Tumorzellen an spezifische Rezeptoren auf den Blutgefäßendothelien, den Selectinen, verantwortlich. Auch dieser Vorgang ist für die Metastasierung des kolorektalen Karzinoms von großer Bedeutung und kann zur Abschätzung der Prognose eines Patienten eingesetzt werden. Die Selectine sind selbst Glykoproteine, die normalerweise glykan-abhängige Wechselwirkungen der Leukozyten mit dem Endothelium initiieren. Diese führen zur Einwanderung der Leukozyten in Entzündungsherde. Glykanstrukturen sind also auch von wesentlicher Bedeutung für die Organisation der Immunabwehr und besitzen auch in diesem Bereich diagnostisches Potential.

In US Patent 6566060 wird die Expression von Glykosyltransferasen analysiert mit der Zielsetzung der Ermittlung des tumorigenen Potentials von Gehirnzellen und der Behandlung von neurologischen Erkrankungen.

Die US Patentanmeldung US 2004/0204381 beschreibt Reagenzien und Methoden zur Behandlung und Vorbeugung von Erkrankungen, welche auf Veränderungen des Sialyierungs- oder Glykosylierungsmusters von Proteinen beruhen.

US Patentanmeldung 2003/0175734 beschreibt Materialien und Methoden zur Bestimmung der Veranlagung von Zellen, maligne Phänotypen, im besonderen Gehirntumoren, zu entwickeln. Diese Methoden basieren auf der Analyse von Expressionsmustern von Onkogenen und Tumorsuppressorgenen.

WO02059350 offenbart die Verwendung von Sialyltransferasen, insbesondere der Sialyltransferase ST6GalNAc-II für die Diagnose und Therapie von Tumorerkrankungen.

Im Stand der Technik ist jedoch kein Ansatz bekannt, welcher die umfassende Untersuchung des Glykosylierungsapparates bietet.

Aufgabe der vorliegenden Erfindung war somit die Bereitstellung verbesserter und/oder modifizierter Mittel und Methoden zur Analyse des Glykosylierungsapparates.
Diese Aufgabe wird durch die Bereitstellung der in den Ansprüchen gekennzeichneten Ausführungsformen gelöst.

Folglich betrifft die vorliegende Erfindung einen Träger, auf den ein Set von Sonden aufgebracht ist, wobei das Set umfasst: (a) Sonden, die in der Lage sind, spezifisch mit Nukleinsäuren zu hybridisieren, die Fucosyltransferasen kodieren; (b) Sonden, die in der Lage sind, spezifisch mit Nukleinsäuren zu hybridisieren, die Galactosyltransferasen kodieren; (c) Sonden, die in der Lage sind, spezifisch mit Nukleinsäuren zu hybridisieren, die N-Acetyl-glucosaminyltransferasen kodieren; (d) Sonden, die in der Lage sind, spezifisch mit Nukleinsäuren zu hybridisieren, die N-Acetyl-galactosaminyltransferasen kodieren; (e) Sonden, die in der Lage sind, spezifisch mit Nukleinsäuren zu hybridisieren, die Sialyltransferasen kodieren; (f) Sonden, die in der Lage sind, spezifisch mit Nukleinsäuren zu hybridisieren, die Zucker-Sulfotransferasen kodieren; (g) Sonden, die in der Lage sind, spezifisch mit Nukleinsäuren zu hybridisieren, die Lectine kodieren; (h) Sonden, die in der Lage sind, spezifisch mit Nukleinsäuren zu hybridisieren, die Selectine kodieren; (i) Sonden, die in der Lage sind, spezifisch mit Nukleinsäuren zu hybridisieren, die Fucosidasen kodieren; (j) Sonden, die in der Lage sind, spezifisch mit Nukleinsäuren zu hybridisieren, die Neuraminidasen (Sialidasen) kodieren; (k) Sonden, die in der Lage sind, spezifisch mit Nukleinsäuren zu hybridisieren, die Nucleotid-Zucker-Epimerasen kodieren; (l) Sonden, die in der Lage sind, spezifisch mit Nukleinsäuren zu hybridisieren, die Zucker-Kinasen kodieren; (m) Sonden, die in der Lage sind, spezifisch mit Nukleinsäuren zu hybridisieren, die Zucker-Epimerasen kodieren; (n) Sonden, die in der Lage sind, spezifisch mit Nukleinsäuren zu hybridisieren, die Zucker-Transporter kodieren; und (o) Sonden, die in der Lage sind, spezifisch mit Nukleinsäuren zu hybridisieren, die für Enzyme des Fucose- oder Sialinsäure-Metabolismus kodieren.

Unter dem Begriff Träger wird ein Vorrichtung verstanden, welche eine Fläche aufweist, auf welcher die erfindungsgemäßen Sonden aufgebracht werden können. Diese Fläche im Sinne der Erfindung kann jede beliebige Fläche sein. Die Fläche kann eine Beschichtung sein, welche auf den Träger aufgebracht wurde, oder aber die Oberfläche des Trägers selbst. Geeignete Materialen für den Träger sind dem Fachmann geläufig und schließen Kunststoffe, Glas, Silica, Gold, Edelstahl, Teflon, Nylon and Silizium ein. Beschichtungen im Sinne der Erfindung, sofern sie Anwendung finden, schließen Poly-L-Lysin- und Aminosilan-Beschichtungen sowie Epoxid- und Aldehyd-aktivierte Oberflächen ein.
In einer bevorzugten Ausführungsform ist der Träger miniaturisiert, beispielsweise in Form eines Chips, einer Scheibe, einem Bead oder einer Mikrotiterplatte. Der Träger im erfindungsgemäßen Sinne ermöglicht die parallele Analyse mehrerer Einzelnachweise in einer geringen Menge von Probenmaterial.
In einer weiteren bevorzugten Ausführungsform wird jeder Sonde eine definierte Koordinate zugeordnet, um so die Auswertung der Ergebnisse, z.B. in automatisierten Testverfahren mit Hilfe von Robotern, zu ermöglichen. Des weiteren vermeidet die Verwendung von Trägern eine Vermischung bzw. ein "spill-over" zwischen Proben und erlaubt zusätzlich eine langfristige Konservierung.

Unter dem Begriff Sonde wird ein Nukleinsäuremolekül verstanden, das vorzugsweise in molekularbiologischen Hybridisierungsmethoden zur sequenzspezifischen Detektion von DNA- und RNA-Molekülen eingesetzt wird. Die Herstellung der Sonden kann mit jedem im Stand der Technik bekannten Verfahren erfolgen, z. B. können die Sonden enzymatisch und chemisch synthetisiert werden. Die Sonden können zum Beispiel mittels "in-situ" Synthese durch Photolithographie oder Ink-Jet-Systeme direkt auf den Träger synthetisiert werden (Gao *et al.,* 2001; Lausted *et al.,* 2004) oder kontaktfrei durch Piezo- oder mit Hilfe von Stahlnadeln, die bei Oberflächenkontakt kleinste Volumina ablegen, aufgetragen werden. Das Auftragen der Sonden kann zum Beispiel mit Hilfe von Spotting Robotern erfolgen (Auburn *et al.,* 2005).

Unter einem Nukleinsäuremolekül werden dabei alle natürlich vorkommenden Nukleinsäuremoleküle wie beispielsweise DNA oder RNA als auch künstliche Basenanaloga wie beispielsweise PNA (Peptide Nucleotide Acids; Harris and Winssinger, 2005) oder LNA (Locked Nucleic Acids; Castoldi *et al.,* 2006) verstanden.

Fucosyltransferasen (EC 2.4.1.-) gehören zur Gruppe der Glykosyltransferasen, im besonderen zur Gruppe der Hexosyltransferasen und katalysieren die Übertragung von Fucose von einem aktivierten Zuckerphosphat, z.B. GDP-Fucose, auf ein Protein oder einen Zucker.

Galactosyltransferasen (EC 2.4.1.-) gehören zur Gruppe der Glykosyltransferasen, im besonderen zur Gruppe der Hexosyltransferasen und katalysieren die Übertragung von Galactose von einem aktivierten Zuckerphosphat, z.B. UDP-Galactose, auf ein Protein oder einen Zucker.

N-Acetyl-glucosaminyltransferasen (EC 2.4.1.-) gehören zur Gruppe der Glykosyltransferasen, im besonderen zur Gruppe der Hexosyltransferasen und katalysieren die Übertragung von N-Acetylglucosamin von einem aktivierten Zuckerphosphat, z.B. UDP-GIcNAc, auf ein Protein oder einen Zucker.

N-Acetyl-galactosaminyltransferasen (EC 2.4.1.-) gehören zur Gruppe der Glykosyltransferasen, im besonderen zur Gruppe der Hexosyltransferasen und katalysieren die Übertragung von N-Acetylgalactosamin von einem aktivierten Zuckerphosphat, z.B. UDP-GaINAc, auf ein Protein oder einen Zucker.

Sialyltransferasen, (EC 2.4.99.-) gehören zur Gruppe der Glykosyltransferasen und katalysieren die Übertragung von Nukleotid-aktivierter Sialinsäure, z.B. CMP-NeuAc, auf ein Protein oder einen Zucker.

Zucker-Sulfotransferasen (EC 2.8.2) gehören zur Gruppe der Sulfotransferasen und katalysieren die Übertragung einer Sulfatgruppe des Nukleotidanalogs 3'-Phosphoadenosine-5'-phosphosulfate (PAPS) auf einen Zucker.

Lectine sind Glykoproteine die in der Lage sind, spezifisch an Zuckerreste von Zellen bzw. Zellmembranen zu binden und von dort aus biochemische Reaktionen auszulösen. Lectine besitzen keine enzymatische Aktivität.

Selectine sind eine Gruppe von kohlenhydratbindenden Adhäsionsmolekülen, welche vor allem an Zell-Zell-Interaktionen des Immunsystems beteiligt sind. Im Rahmen einer Inflammation vermitteln Selectine zum Beispiel das "Rolling" von Leukozyten auf aktivierten Endothelzellen und tragen zur Verlangsamung der Leukozyten und ihren Austritt aus dem Blutstrom bei (Sperandio, 2006).

Fucosidasen (EC 3.2.1.-) gehören zur Gruppe der Glycosidasen und katalysieren die hydrolytische Abspaltung von Fucose von Zuckerresten.

Neuraminidasen (EC 3.2.1.-) gehören zur Gruppe der Glycosidasen und katalysieren die hydrolytische Abspaltung der terminalen Sialinsäurereste von Glykoproteinen der Zelloberfläche. Neuraminidasen sind dem Fachmann auch als Sialidasen bekannt.

Nucleotid-Zucker-Epimerasen (EC 5.1.3.-) gehören zur Gruppe der Isomerasen und katalysieren die stereochemische Umwandlung eines Nukleotid-Zuckers in seine isomere Struktur (d.h. Moleküle gleicher Summenformel, aber anderer Strukturformel).

Zucker-Kinasen (EC 2.7.-.-) gehören zur Gruppe der Transferasen, speziell der Phosphotransferasen. Zucker-Kinasen katalysieren die Phosphorylierung von Hydroxygruppen von Zuckermolekülen.

Zucker-Epimerasen (EC 5.1.3.-) gehören zur Gruppe der Isomerasen, und katalysieren die stereochemische Umwandlung eines Zuckermoleküls in seine isomere Struktur (d.h. Moleküle gleicher Summenformel, aber anderer Strukturformel).

Zucker-Transporter sind Proteine, welche spezifische Zucker und nukleotid-aktivierte Zucker, wie beispielsweise GDP-Fucose, binden und dem Zuckertransport dienen. Dazu gehört zum einen der Zuckertransport innerhalb der Zelle, z.B. vom Cytoplasma in den Golgiapparat oder das Endoplasmatische Reticulum als auch zum anderen der Zuckertransport aus dem extrazellulären Raum in die Zelle.

Enzyme des Fucose- oder Sialinsäure-Metabolismus sind Teil der Stoffwechselkette, die die Synthese der Sialinsäure oder Fucose aus Glukose katalysieren. Des weiteren gehören zur Gruppe der Enzyme des Fucose- oder Sialinsäure-Metabolismus auch Enzyme, die die Umwandlung von in die Zelle aufgenommener Fucose und Sialinsäure zu den Nukleotid-aktivierten Zuckern CMP-Sialinsäure und GDP-Fucose katalysieren. Enzyme des Fucose- oder Sialinsäure-Metabolismus sind, beispielsweise, N-Acetylneuraminsäuresynthase (NANS; EC 2.5.1.56), CMP-N-Acetylneuraminsäurehydroxylase (CMAH; EC 1.14.18.2), N-Acetylneuraminat-Pyruvat Lyase (NPL; EC 4.1.3.3), N-Acetylglucosamin-Kinase (NAGK; EC 2.7.1.59), Glucosamin (UDP-N-Acetyl)-2-Epimerase/N-Acetylmannosaminkinase (GNE; EC 5.1.3.14), Fucose-1-Phosphatguanylyltransferase (FPGT; EC 2.7.11.1), Cytidin-Monophosphat N-Acetylneuraminsäuresynthetase (CMAS; EC 2.7.7.43), Phosphoglucomutase 3 (PGM3; EC 5.4.2.3), UDP-Galactose-4-Epimerase (GALE; EC 5.1.3.2), Transplantations-Antigen P35B (Fx/TSTA3; EC 1.1.1.271), Fucokinase (FUK; EC 2.7.1.52), Sialylsäureacetylesterase (SIAE/OAE; EC 3.1.1.53) und Renin-Bindeprotein (RENBP; EC 5.1.3.8).

Für die oben genannten Enzyme wurde auf die Klassifizierung nach dem EC-Nummern-System Bezug genommen, das im Stand der Technik üblich ist. Dieses System weist jedem Enzym einen Zahlencode aus vier Zahlen zu. Die erste Zahl bezeichnet dabei die Enzymklasse. Insgesamt sind Enzyme in sechs Enzymklassen unterteilt: Oxidoreduktasen, Transferasen, Hydrolasen, Lyasen, Isomerasen und Ligasen. Die weiteren Zahlen bieten eine noch detailliertere Charakterisierung der Enzyme wie zum Beispiel Informationen über die Substrate, die übertragene Gruppe sowie die Art der gebildeten oder gespaltenen Bindung. Diese Charakterisierung ist hierarchisch aufgebaut und hängt von der jeweiligen Enzymklasse ab.

Umfassen im Sinne der Erfindung bedeutet, dass außer den genannten Sonden zusätzliche Kontrollsonden auf dem Träger aufgebracht sein können. Als Kontrolle können beispielsweise Sonden verwendet werden, die für Gene von Arabidopsis thaliana (Ackersenf) kodieren und an welche humane RNA nicht bindet. Damit kann die Effizienz der Fluoreszenzmarkierung auf dem cDNA-Niveau und die gleichmäßige Qualität der Arrays überprüft werden, indem bei jeder Untersuchung genau titrierte Mengen von entsprechender RNA von Arabidopsis thaliana mit umgesetzt und hybridisiert werden. Weitere Beispiele für zusätzlichen Sonden sind unter anderem "Guidance Spots" zur korrekten Ausrichtung des Trägers und qualitativen Analyse der Ergebnisse. Außerdem können auch Sonden verwendet werden, die Nukleinsäuren binden, deren Expression sich zwischen verschiedenen Geweben und Zelllinien nicht verändern sollte (Housekeeping-Gene), und die somit als interne Kontrollen dienen.
Auch berücksichtigt im Sinne der Erfindung sind Träger, die nur solche Sonden enthalten, die wie oben definiert spezifisch mit Nukleinsäuren hybridisieren, die Fucosyltransferasen, Galactosyltransferasen, Acetyl-glucosaminyltransferasen, Acetyl-galactosaminyltransferasen, Sialyltransferasen, Zucker-Sulfotransferasen, Lectine, Selectine, Fucosidasen, Neuraminidasen (Sialidasen), Nucleotid-Zucker-Epimerasen, Zucker-Kinasen, Zucker-Epimerasen, Zucker-Transporter und Enzyme des Fucose- oder Sialinsäure-Metabolismus kodieren.

Der Begriff hybridisieren im Sinne der Erfindung bezeichnet das Binden der erfindungsgemäßen Sonden an einen komplementären Strang von Polynukleotiden, wodurch diese ein Hybrid formen. Methoden zur Durchführung von Hybridisierungsexperimenten sind im Stand der Technik bekannt und der Fachmann kennt die entsprechenden Bedingungen, welche zur erfindungsgemäßen Hybridisierung genutzt werden müssen. Solche Hybridisierungstechniken können in Lehrbüchern wie z.B. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), oder Higgins and Hames (Eds.) "Nucleic acid hybridization, a practical approach" IRL Press Oxford, Washington DC, (1985) gefunden werden.
Stringente Hybridisierungsbedingungen schließen Bedingungen ein, welche z.B. umfassen: eine Inkubation über Nacht bei 65°C in 4x SSC (600 mM Natriumchlorid, 60 mM Natriumzitrat) gefolgt von einem Waschschritt bei 65°C in 0,1x SSC für eine Stunde. Alternativ kann bei 42°C in einer Lösung welche 50% Formamid, 5x SSC (750 mM Natriumchlorid, 75 mM Natriumzitrat), 50 mM Natriumphosphat (pH 7,6), 5x Denhardt's Lösung, 10% Dextransulfat und 20 µg/ml denaturierte, gescherte DNA aus Lachssperma beinhaltet inkubiert werden, gefolgt von Waschschritten in 0,1x SSC von 5 bis 20 Minuten bei ungefähr 65°C. Diese Hybridisierungsbedingungen sind dem Fachmann als hoch-stringente Hybridisierungsbedingungen bekannt.

Auch berücksichtigt im Sinne der Erfindung sind weniger stringente Hybridisierungsbedingungen. Weniger stringente Bedingungen für die Hybridisierung und die Signaldetektion können beispielsweise durch Variieren der Temperatur oder der Konzentration der Salze erreicht werden. Wenig stringente Hybridisierungsbedingungen werden beispielsweise durch Inkubation bei 50°C in 4x SSC erreicht. Des weiteren können durch Veränderungen der Konzentration von Formamid weniger stringente Bedingungen erreicht werden (niedrigere Formamidkonzentrationen resultieren in weniger stringenter Hybridisierung), welche beispielsweise einschließen: eine Inkubation über Nacht bei 37°C in einer Lösung beinhaltend 30% Formamid, 6xSSPE (20X SSPE = 3M NaCl, 0,2M NaH₂PO4, 0,02M EDTA, pH 7,4), 0,5% SDS, 100 µg/ml DNA aus Lachssperma und anschließendes Waschen bei 50°C mit 1X SSPE und 0,1% SDS. Diese Bedingungen können weiter variiert werden, indem alternative Blockingreagenzien für die Unterdrückung von Hintergrundsignalen verwendet werden. Solche alternativen Blockingreagenzien sind zum Beispiel Denhardt's Lösung, BLOTTO, Heparin, denaturierte DNA aus Lachssperma, sowie weitere kommerziell erhältliche Reagenzien. Bei Verwendung von alternativen Blockingreagenzien müssen die Hybridisierungsbedingungen eventuell an die jeweiligen Versuchsbedingungen angepasst werden.
Bevorzugte Sonden gemäß der vorliegenden Erfindung sind Sonden, welche unter stringenten Bedingungen an eine Spezies der oben genannten Nukleinsäuren binden, ohne Kreuzreaktionen mit anderen Nukleinsäuren einzugehen.

Der erfindungsgemäße Träger bietet den Vorteil der kompletten Erfassung der Moleküle der Glykosylierungsmaschinerie in einem Ansatz sowie eine gegenüber anderen Techniken kurze Durchführungszeit und geringe Menge an notwendigem Untersuchungsmaterial. Somit bietet dieser umfassende Ansatz eine sensitive, rasche und parallele Analytik der Komponenten des Glykosylierungsapparates auf der Grundlage von Microarrays. Mit Hilfe des Arrays ist es in kurzer Zeit und ausgehend von geringen Zellzahlen möglich, die Expression der Gesamtheit der Glykosyltransferasen und anderer Moleküle der Glykosylierungsmaschinerie zu detektieren. Das ist beispielsweise von Vorteil für die Diagnostik von insbesondere Tumorerkrankungen oder entzündlichen und/oder neurologischen Erkrankungen.

Unter dem Begriff Tumor im Sinne der Erfindung wird die Neubildungen von Körpergeweben (Neoplasien) verstanden, die durch Fehlregulationen des Zellwachstums entstehen. Es werden gutartige (benigne) und bösartige (maligne) Tumoren unterschieden.

Unter entzündlichen Erkrankungen im Sinne der Erfindung werden Erkrankungen verstanden, die eine erste Reaktion des Immunsystems auf Infektionen oder Reize wie z.B. physikalische Reize, Mikroorganismen, Allergene, Toxine oder fehlfunktionierende Enzyme darstellen, mit der Funktion, den Schädigungsreiz zu beseitigen oder zu reparieren. Häufig auftretende entzündliche Erkrankungen sind beispielsweise Arthritis, Myokarditis, Dermatitis, Otitis, Pneumonie, Rheumatische Erkrankungen, Crohn's Disease, Ulcerative Colitis, Spondylitis, Osteoarthritis oder Psoriasis.

Unter neurologischen Erkrankungen im Sinne der Erfindung werden Erkrankungen des Nervensystems verstanden. Die betroffenen Organsysteme sind das Zentralnervensystem und das periphere Nervensystem inklusive ihrer Umgebungsstrukturen. Neurologische Erkrankungen umfassen beispielsweise Multiple Sklerose, Hirnhautentzündungen, Rückenmarksentzündungen, Hirninfarkt, Parkinson, Gehirntumoren, Migräne, epileptische Anfallsleiden, Demenzen und Muskeldystrophien.

Auch für die Analyse der Glykosylierung in Produktionszelllinien kann die erfindungsgemäße Bestimmung der Expression der Gesamtheit der Glykosyltransferasen und anderer Moleküle des Glykosylierungsapparates von Vorteil sein. Da Glykane entscheidend an der Ausbildung korrekt gefalteter, funktioneller Proteine beteiligt sind, bietet diese Expressionsanalyse eine innovative Technologie für die Herstellung wirkungsoptimierter, glykosylierter Biopharmazeutika. Die Bedeutung dieses Wissens um das Glykosylierungsmuster für die pharmakologische Bioaktivität und Bioverfügbarkeit von Proteinen wird besonders deutlich, wenn bei fehlender oder falscher Glykosylierung der therapeutische Einsatz und Erfolg von rekombinanten Proteinen hinter dem erwarteten Potential liegt.

Ein weiterer Vorteil liegt in der Möglichkeit der einfachen und schnellen qualitativen und quantitativen Erfassung von Veränderungen der verschiedenen in einer Zelllinie expremierten Moleküle des Glykosylierungsapparates nach einem spezifischen Glykoengineering. Zelllinien müssen häufig zusätzlichem Glykoengineering unterzogen werden, entweder um bestimmte Glykosyltransferase-Aktivitäten zu verstärken und/oder um andere auszuschalten. Auf solche spezifischen Eingriffe können möglicherweise auch andere Elemente der Glykosylierungsmaschinerie reagieren. Der ganzheitliche Ansatz des erfindungsgemäßen Trägers bietet somit die Möglichkeit auch überraschende, nicht vorhergesehene Expressionsänderungen detektieren zu können. Ein weiterer Vorteil liegt in der geringen benötigten Probenmenge, da bereits eine Zelle ausreichend Probenmaterial liefert. Somit bietet der erfindungsgemäße Träger ein kosten- und arbeitssparendes Verfahren im Vergleich zu herkömmlichen Methoden wie beispielsweise der Zuckeranalytik per HPLC. Weitere Vorteile der Erfindung sind im Zusammenhang mit bevorzugten Ausführungsformen genannt.

In einer bevorzugten Ausführungsform ist der Träger ein fester Träger.
Ein fester Träger im Sinne der Erfindung ist ein Träger, der sich besonders durch hohe Stabilität und die Möglichkeit der Miniaturisierung auszeichnet.

In einer weiteren bevorzugten Ausführungsform besteht der Träger aus Glas.

In einer weiteren bevorzugten Ausführungsform sind die Sonden Oligonukleotidsonden. Oligonukleotidsonden im Sinne der Erfindung sind wenigstens 30 Nukleotide lang. Oligonukleotidsonden können sowohl molekularbiologisch als auch synthetisch (wie beispielsweise die von Affymetrix verwendeten Oligonukleotidsonden, US Patente 5 445 934 und 5 744 305) hergestellt werden. In einer weiteren bevorzugten Ausführungsform kann ein erfindungsgemäßes Protein durch mehrere verschiedene Oligonukleotide repräsentiert sein.

In einer weiteren bevorzugten Ausführungsform werden auf den Träger Sonden aufgebracht, die 40 bis 100 Basen lang sind. In einer stärker bevorzugten Ausführungsform sind die Sonden 50 bis 90 Basen lang, noch stärker bevorzugt 60 bis 80 Basen und am stärksten bevorzugt etwa 70 Basen lang. Dabei sind alle in diese Intervalle fallenden Werte, z.B. Sonden mit einer Länge von 62, 65, 68, 72, 75 oder 78 Basen, explizit in den bevorzugten Ausführungsformen eingeschlossen.

In einer weiteren bevorzugten Ausführungsform des erfindungsgemäßen Trägers sind die Fucosyltransferasen, Galactosyltransferasen, Acetyl-glucosaminyltransferasen, Acetyl-galactosaminyltransferasen, Sialyltransferasen, Zucker-Sulfotransferasen, Lectine, Selectine, Fucosidasen, Neuraminidasen (Sialidasen), Nucleotid-Zucker-Epimerasen, Zucker-Kinasen, Zucker-Epimerasen, Zucker-Transporter und/oder Enzyme des Fucose- oder Sialinsäure-Metabolismus aus Tabelle 1 ausgewählt.

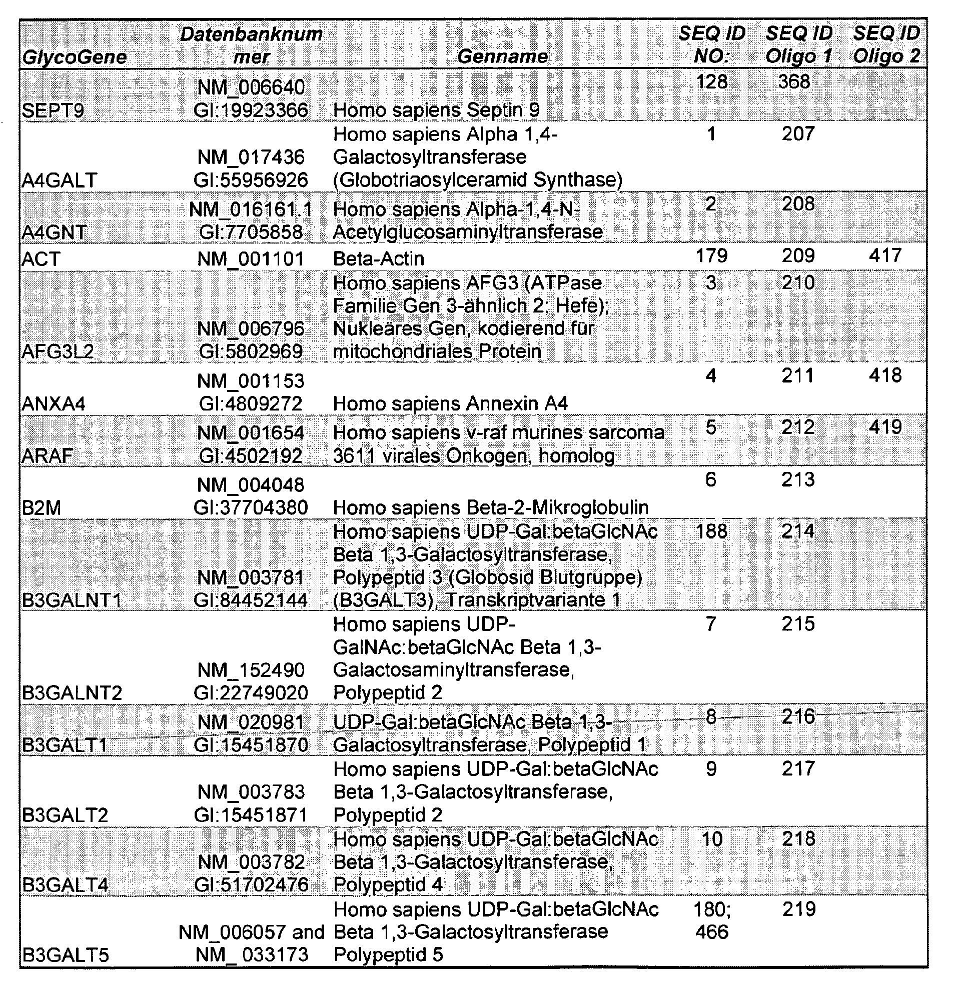

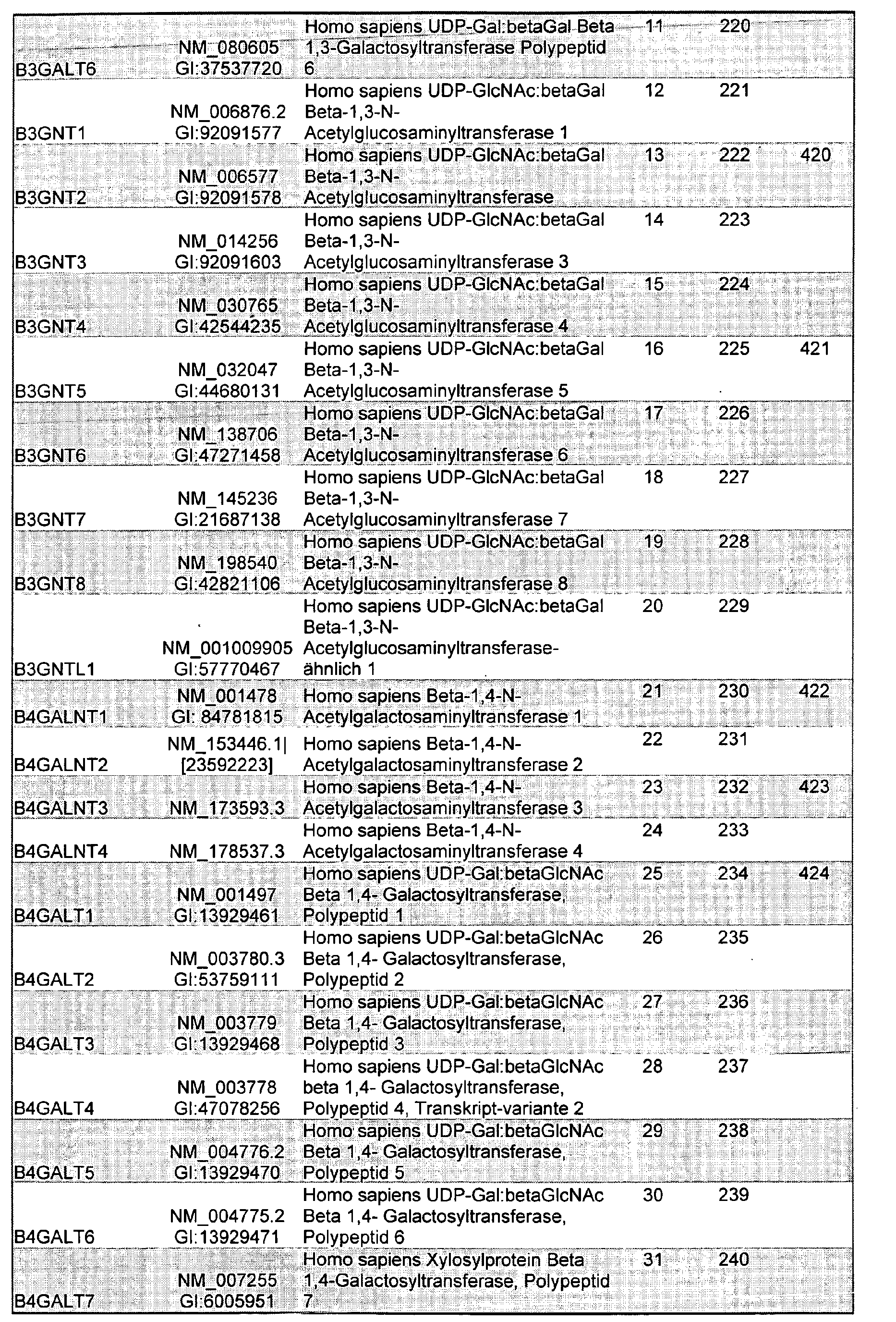

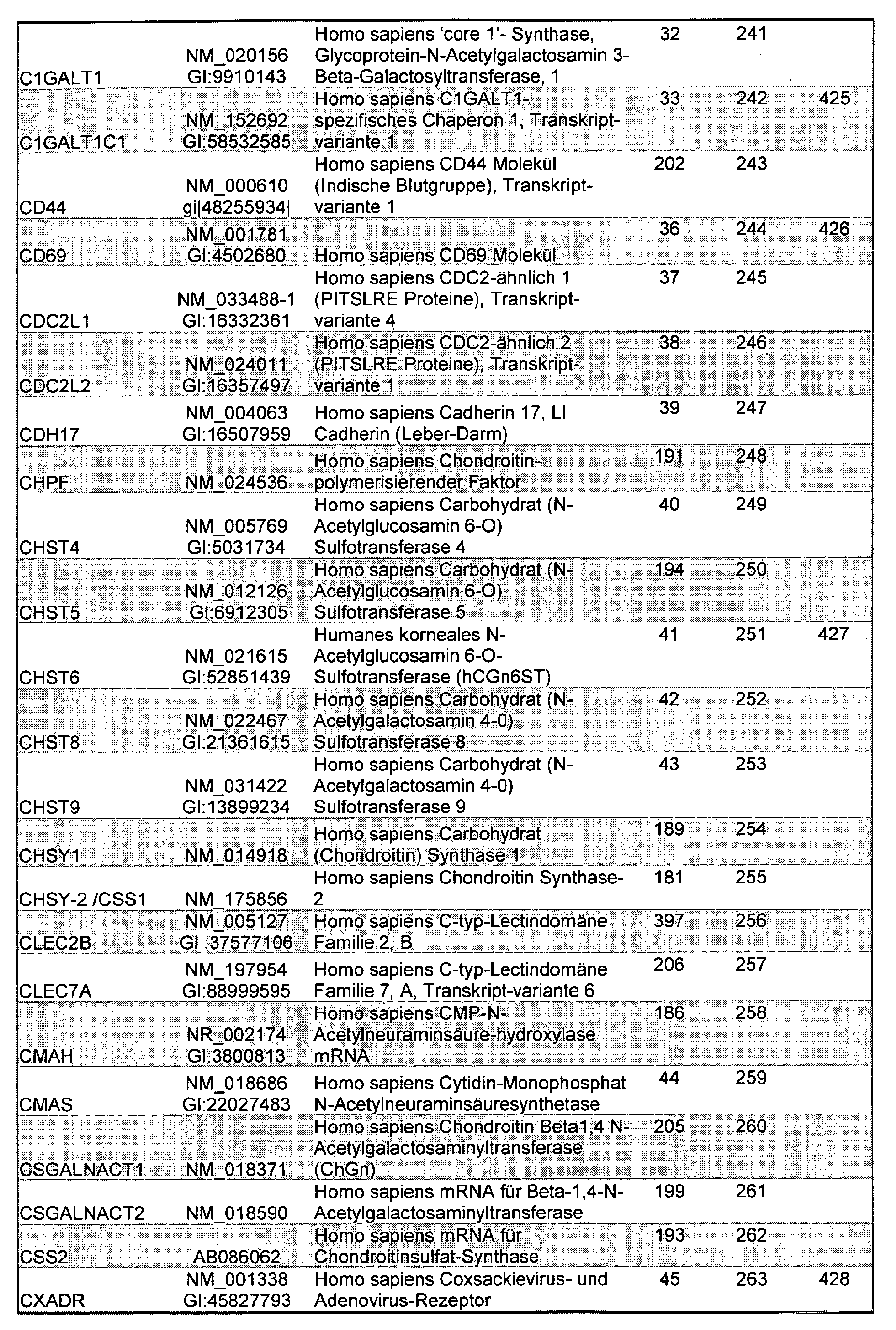

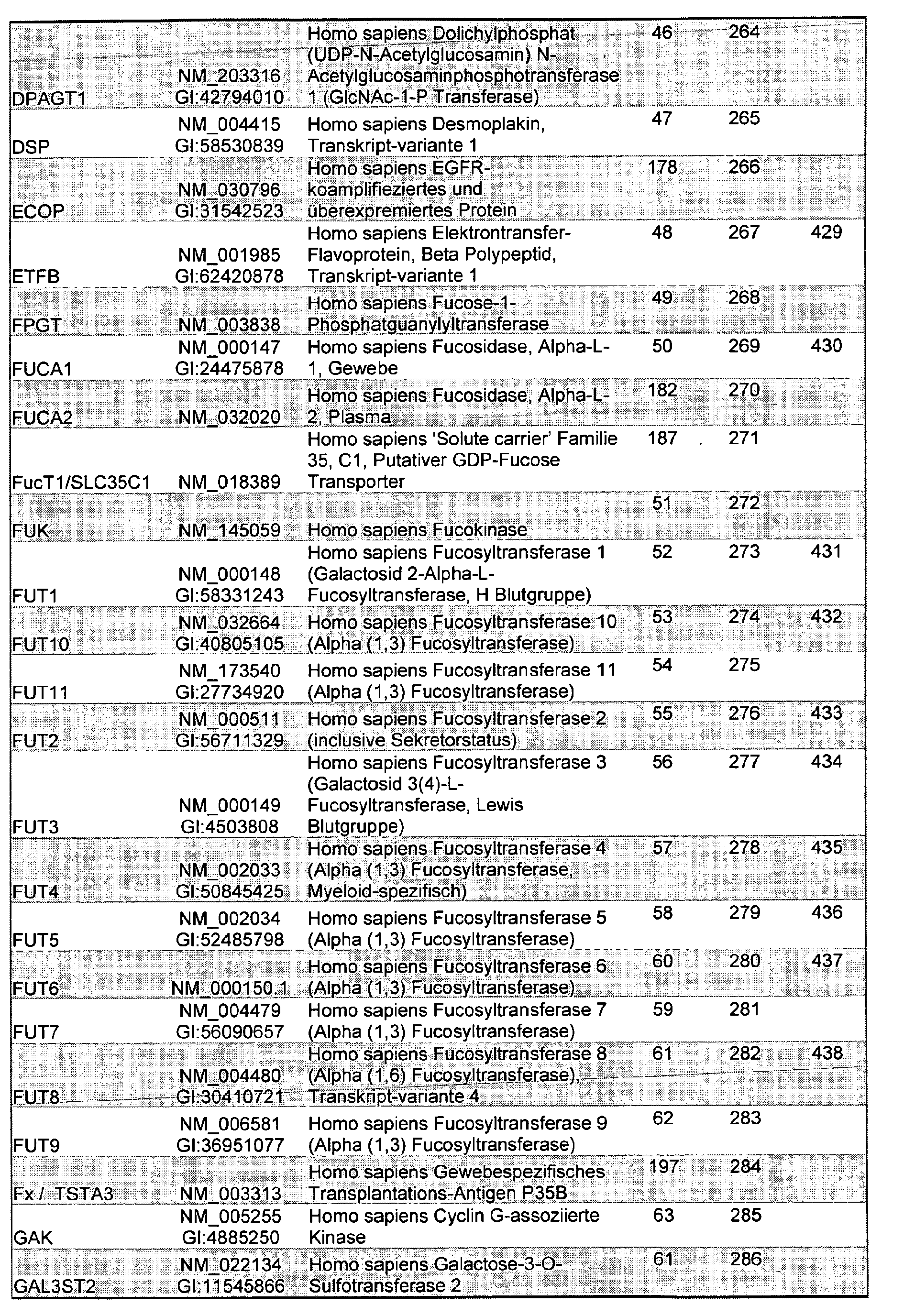

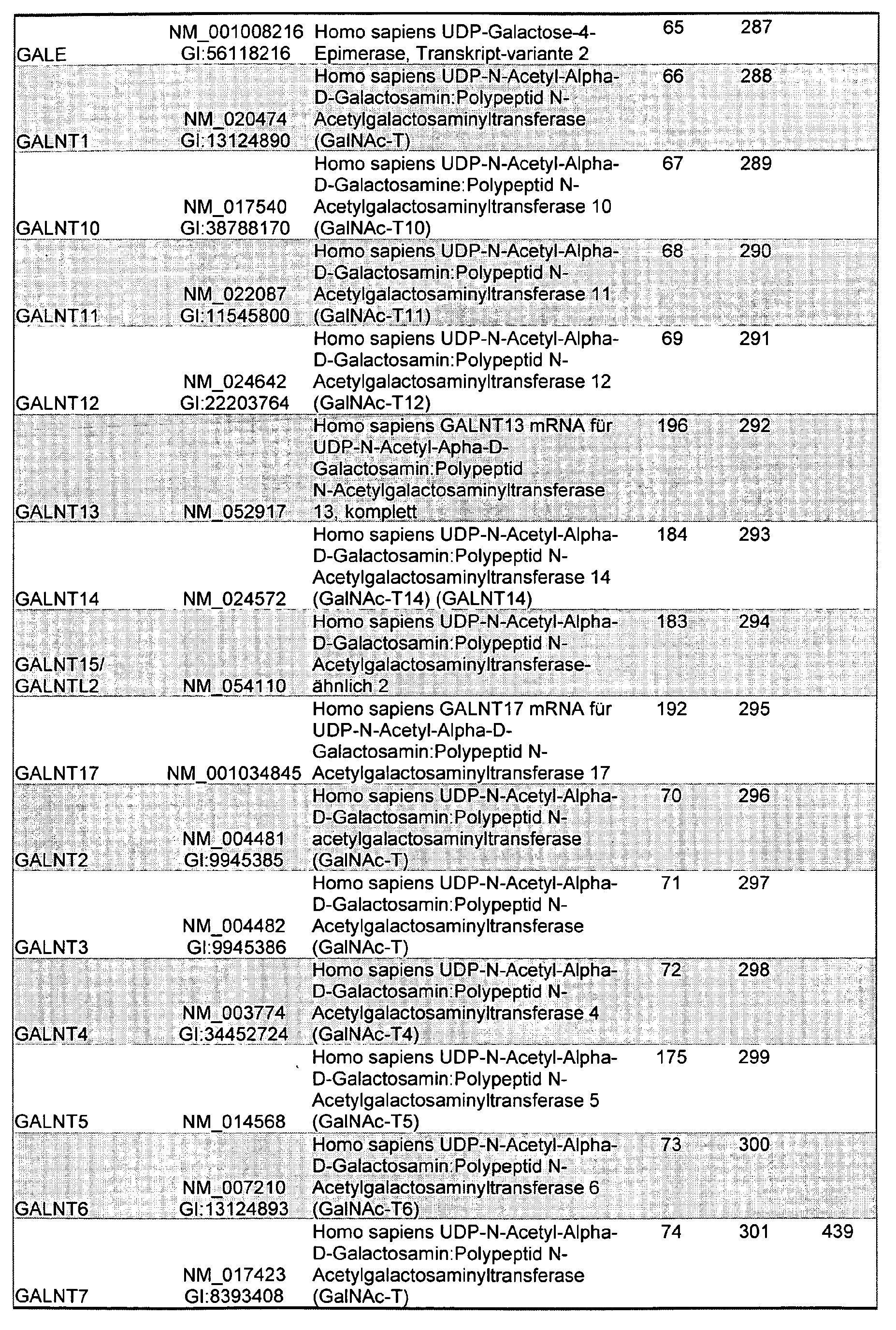

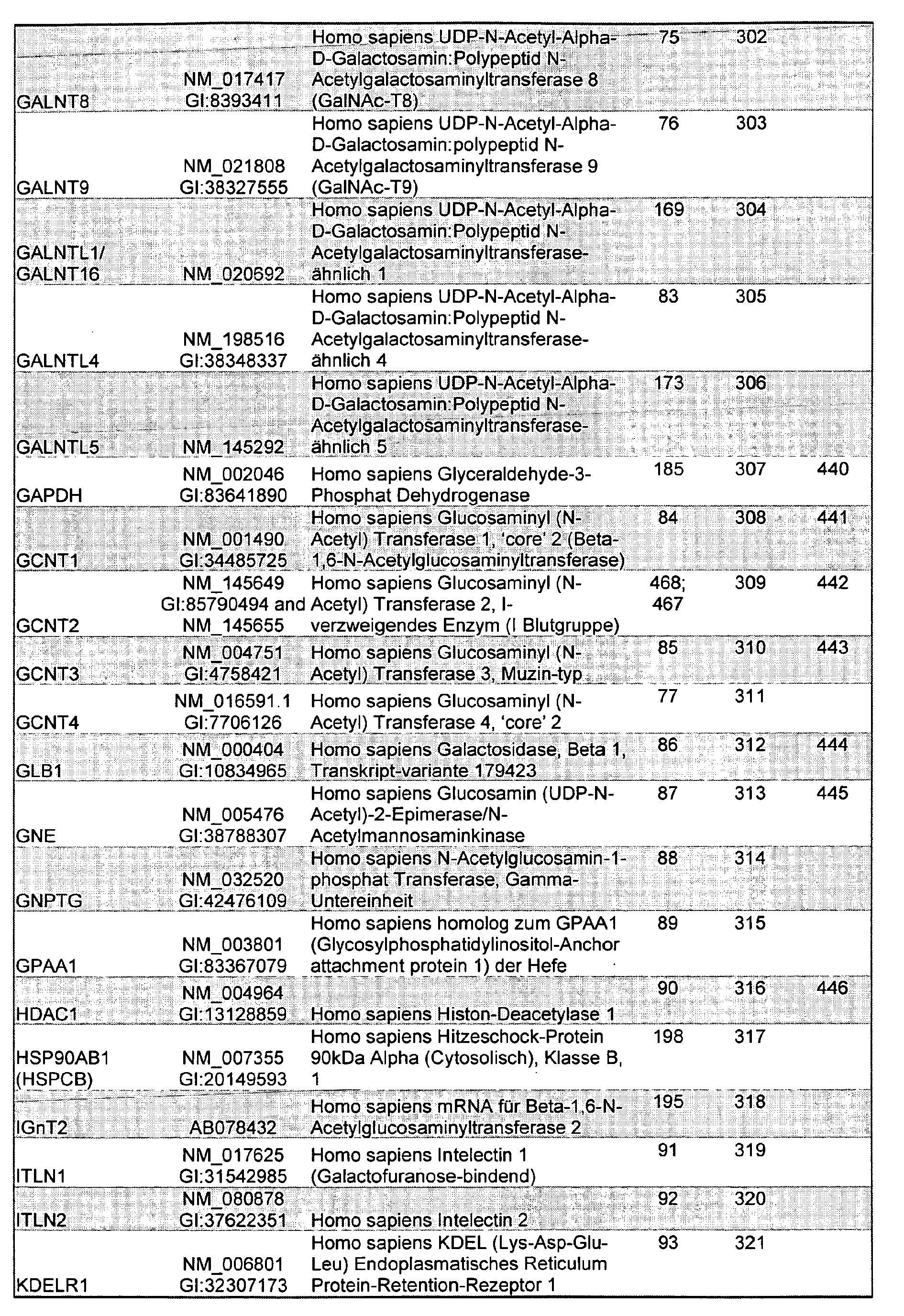

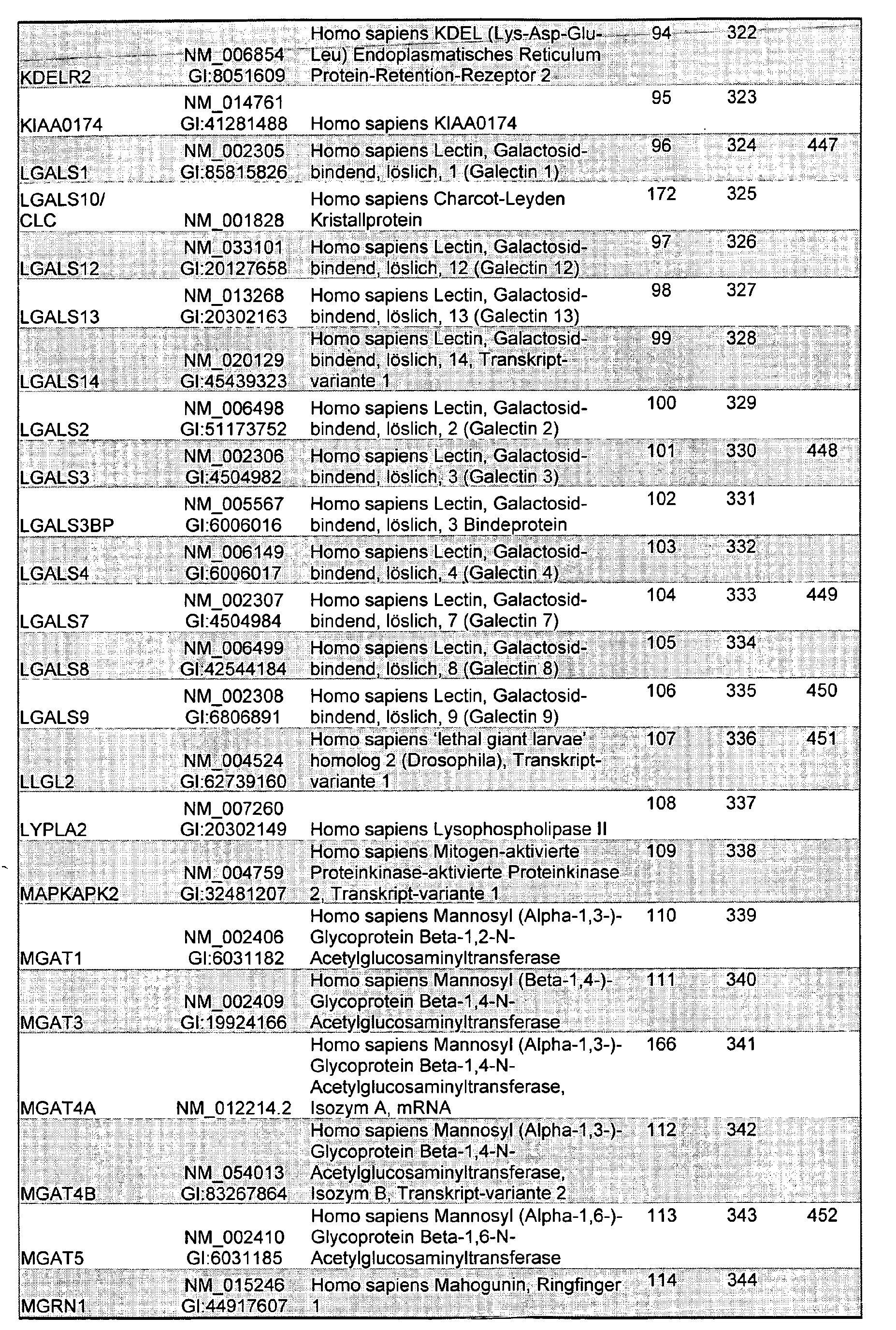

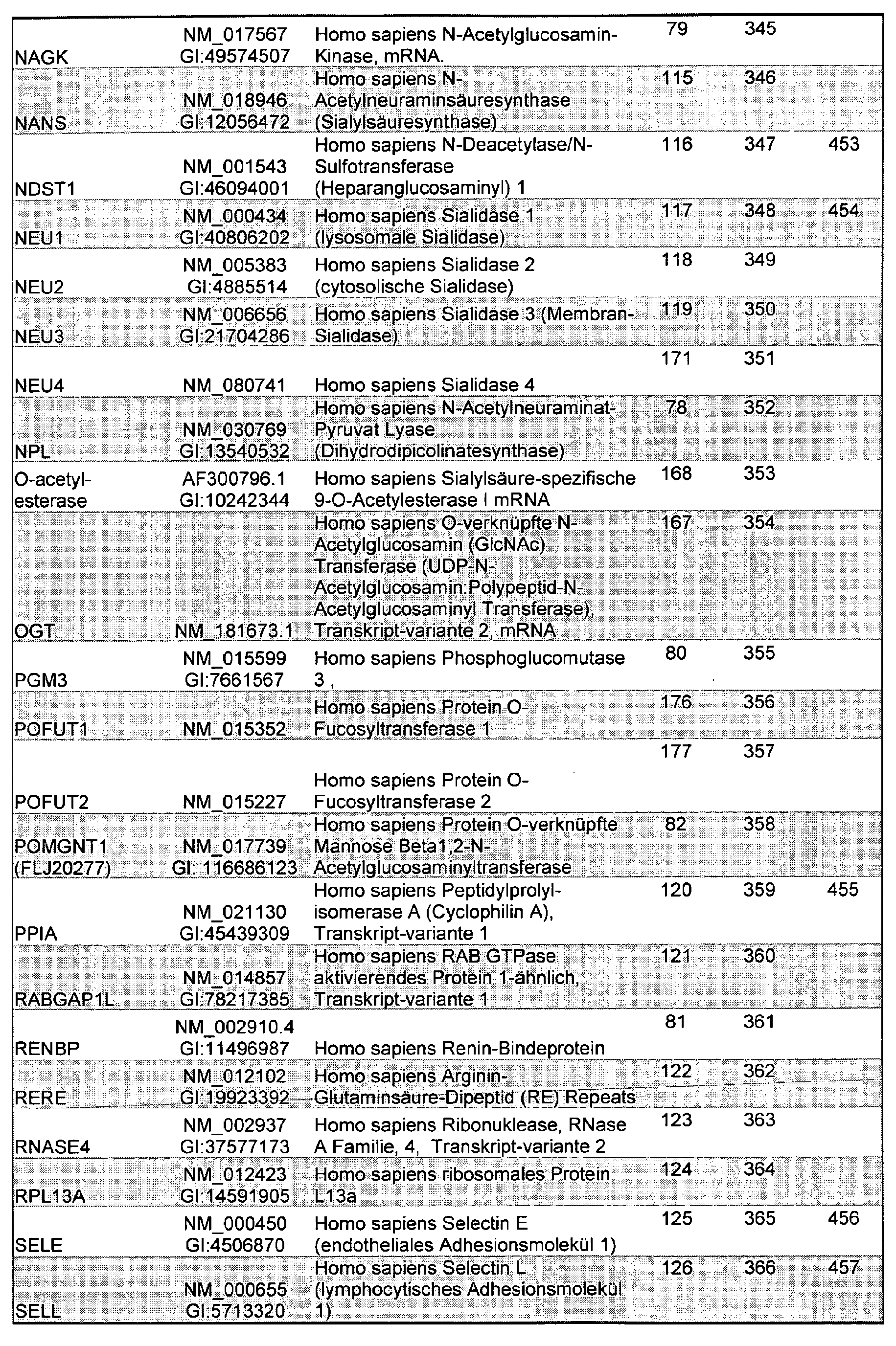

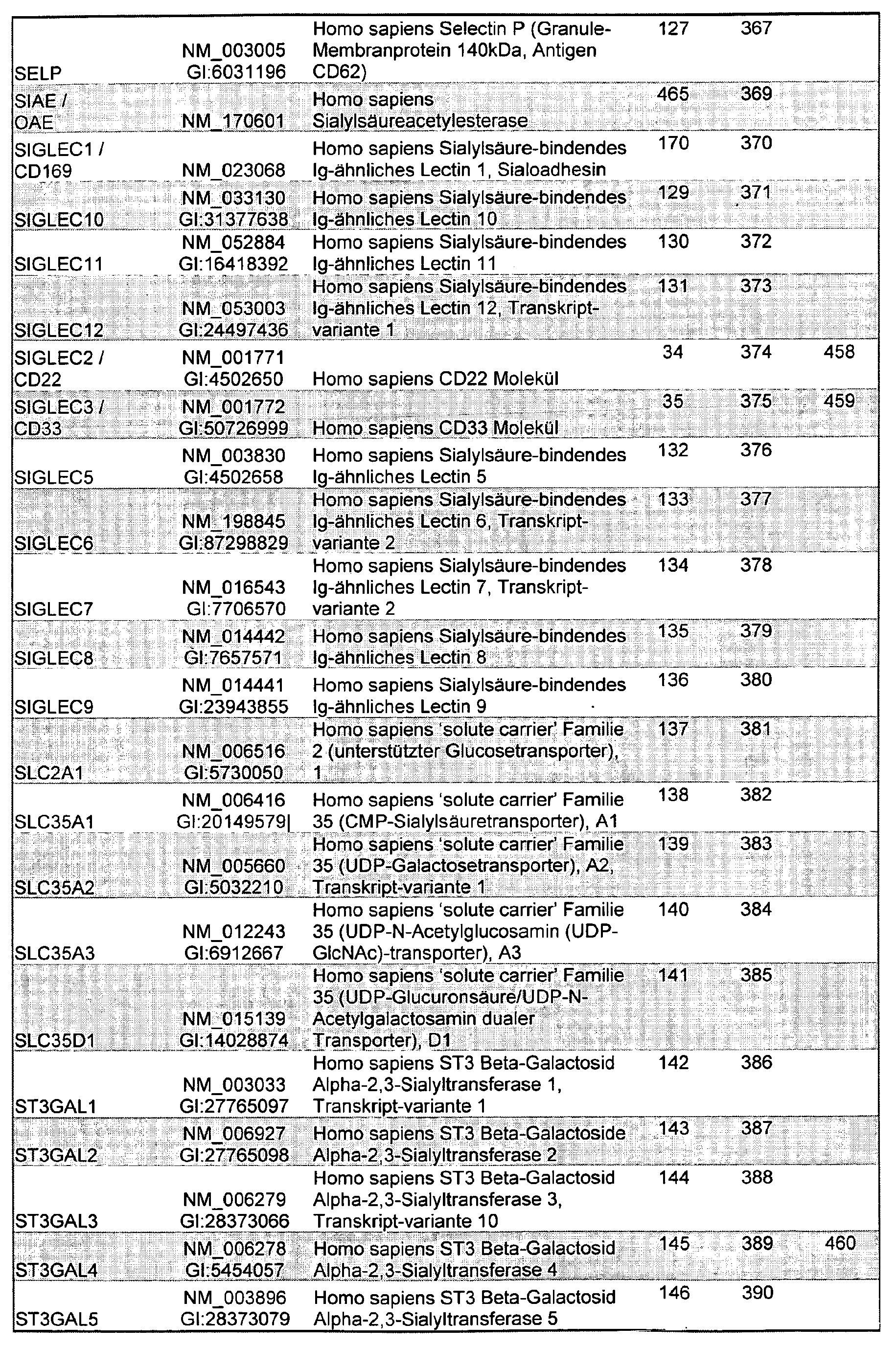

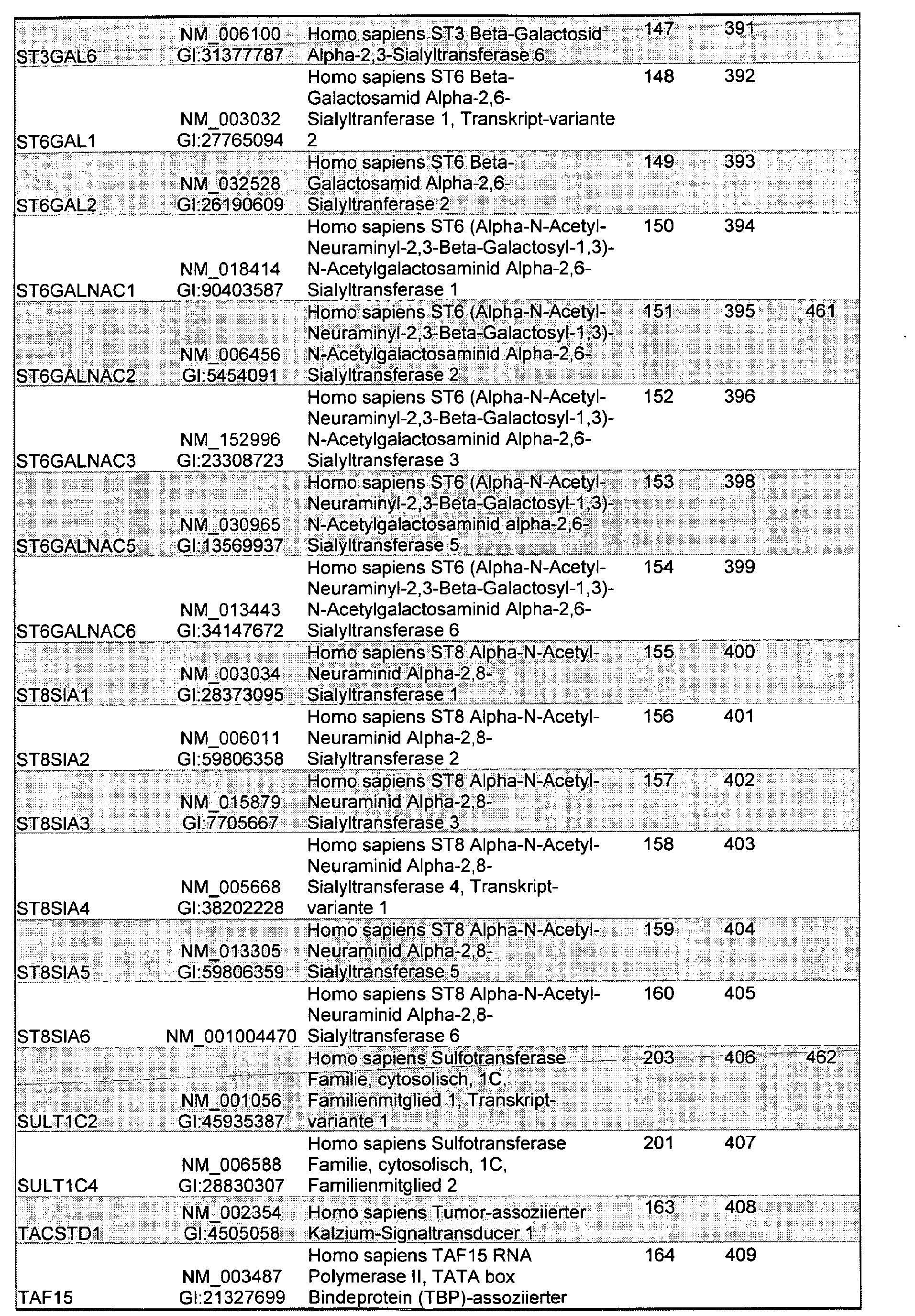

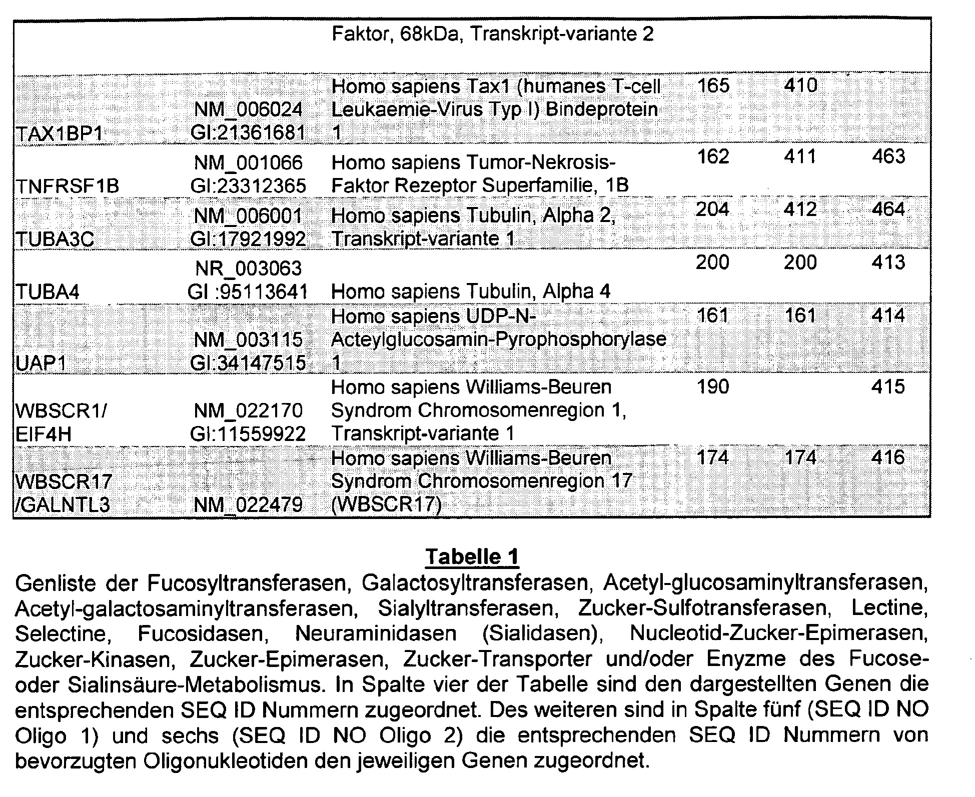

In einer weiteren bevorzugten Ausführungsform des erfindungsgemäßen Trägers sind die Sonden ausgewählt aus den in Tabelle 1 aufgeführten bevorzugten Oligonukleotiden.

In einer weiteren bevorzugten Ausführungsform ist jedes der Proteine von Tabelle 1 durch mindestens eine Sonde auf dem Träger repräsentiert.

Die Erfindung betrifft weiterhin einen Kit, umfassend das Set von Sonden wie vorstehend definiert.

In einer bevorzugten Ausführungsform umfasst der erfindungsgemäße Kit mindestens je eine Sonde für die in Tabelle 1 genannten Proteine. Die im Kit enthaltenen Sonden können in der Herstellung eines Trägers, beispielsweise des erfindungsgemäßen Trägers, aber auch als Sonden in Hybridisierungsverfahren wie z.B. Southern Blotting oder Northern Blotting eingesetzt werden. Diese Methoden an sich sind im Stand der Technik hinreichend bekannt.

Die vorliegende Erfindung betrifft auch die Verwendung des erfindungsgemäßen Trägers oder Kits zum quantitativen Bestimmen der Expression von Nukleinsäuren, die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen (Sialidasen), Nucleotid-Zucker-Epimerasen, Zucker-Kinasen, Zucker-Epimerasen, Zucker-Transporter und Enzyme des Fucose- oder Sialinsäure-Metabolismus kodieren, in einer Probe, die einer Zelle, einem Gewebe oder einem Organismus entnommen ist.

Unter dem Begriff quantitative Bestimmung wird das Messen der Menge eines Zielmoleküls oder einer dazu proportionalen Größe verstanden. Dabei kann diese Menge als absoluter Wert (z.B. der Signalintensität des Zielmoleküles) oder als Verhältnis angegeben werden. Die Darstellung der Quantität in Form eines Verhältnisses, z.B. zwischen den Signalintensitäten des Zielmoleküls und eines parallel analysierten Kontrollmoleküls, bietet entscheidende Vorteile. So können zum Beispiel Experimente miteinander verglichen werden, welche zu verschiedenen Zeitpunkten, unter anderen Bedingungen, mit anderen Methoden oder anderem Ausgangsmaterial durchgeführt wurden. Methoden zur Analyse und Bestimmung der Expressionsdaten sind dem Fachmann bekannt.

Unter einer Probe, die einer Zelle, einem Gewebe oder einem Organismus entnommen ist versteht man eine Probe, welche beispielsweise mit Hilfe im Stand der Technik bekannter Methoden aus Zellen, Geweben oder einem Organismus gewonnen wird. Beispielsweise kann mittels Biopsie eine Probe aus einem lebenden Organismus gewonnen werden. Zusätzlich können Methoden wie zum Beispiel "Laser Capture Microdissection" eingesetzt werden, um einzelne Zellen zu isolieren. Diese Methoden an sich sind im Stand der Technik ausreichend bekannt, wie beispielsweise in Kamme *et al.,* 2003 beschrieben. Die so gewonnen Zellen und/oder Gewebe sowie Zellen aus Zellkulturen können dann mittels dem Fachmann bekannter Methoden lysiert werden. Mit Hilfe dieser Methoden können Proben beispielsweise aus einer einzigen Zelle gewonnen werden. Es können aber auch Proben aus einer größeren Anzahl von Zellen, beispielsweise 10³ bis 10⁵ Zellen gewonnen werden.

Des weiteren betrifft die Erfindung ein Verfahren zum quantitativen Bestimmen der Expression von Nukleinsäuren, die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen (Sialidasen), Nucleotid-Zucker-Epimerasen, Zucker-Kinasen, Zucker-Epimerasen, Zucker-Transporter und Enzyme des Fucose- oder Sialinsäure-Metabolismus kodieren, umfassend die Schritte des Inkontaktbringens des erfindungsgemäßen Trägers mit einer Präparation markierter Nukleinsäuren, die mittels reverser Transkription der mRNAs erhalten wurde, die in einer Probe vorliegen, die einer Zelle, einem Gewebe oder einem Organismus entnommen ist, und Quantifizieren der Menge an Markierung, die an die Sonden auf dem Träger gebunden ist.

Dabei schließen markierte Nukleinsäuren unter anderem cDNAs oder cRNAs ein, welche eine detektierbare Markierung, beispielsweise eine radioaktive oder eine Lumineszenz- bzw. Fluoreszenz -Markierung tragen, oder welche biotinyliert sind. Die Herstellung markierter cDNAs ist dem Fachmann hinreichend bekannt und wird zum Beispiel in Manduchi *et al.,* 2002 beschrieben. Dabei können beispielsweise bei der reversen Transkription der RNA markierte Nukleotide direkt in die entstehende cDNA eingebaut werden, oder es werden bestimmte reaktive Nukleotide eingebaut, welche dann in einem zweiten Schritt mit einem Marker gekoppelt werden (z.B. indirektes Amino Allyl cDNA Labelling), oder cDNAs können mit einer sogenannten "Capture Sequence" ausgestattet werden, welche eine Markierung der bereits an den Array gebundenen cDNAs durch Fluoreszenzdendrimere ermöglicht (z.B. Genisphere 3DNA Array labelling kits). Auch die Herstellung markierter cRNAs ist dem Fachmann hinreichend bekannt. So wird beispielsweise die mRNA als Matrize zur cDNA Synthese genutzt und diese nach Aufreinigung in einer *in vitro* Transkription mit markierten Ribonukleotiden in cRNA umgeschrieben. Häufig verwendete Marker sind beispielsweise die Fluoreszenzmarker Cy3, Cy5, Alexa Fluor 546 oder Alexa Fluor 647, sowie im Falle radioaktiver Markierung P³²-markierte Nukleotide zum direkten Markieren. Dabei können sowohl verschiedene Proben mit verschiedenen Markern (z.B. verschiedener Fluoreszenzmarker) markiert und anschließend mit einem gemeinsamen Träger in Kontakt gebracht werden (dual colour labeling) oder es kann für alle Proben der gleiche Marker (beispielsweise Biotin) verwendet werden, wobei dann jede Proben mit jeweils einem Träger in Kontakt gebracht wird (single colour labeling).

Eine bevorzugte Ausführungsform des erfindungsgemäßen Verfahrens umfasst des weiteren die Bestimmung der Expression der Fucosyltransferasen, Galactosyltransferasen, N-Acetyl-glucosaminyltransferasen, N-Acetylgalactosaminyl-transferasen, Sialyltransferasen, Zucker-Sulfotransferasen, Lectine, Selectine, Fucosidasen, Neuraminidasen (Sialidasen), Nucleotid-Zucker-Epimerasen, Zucker-Kinasen, Zucker-Epimerasen, Zucker-Transporter und Enzyme des Fucose- oder Sialinsäure-Metabolismus, wobei das Bestimmen der Expression: (i) Bestimmen der Menge an mRNA mittels PCR, Ribonuklease Protection Assay oder SAGE ("Serial Analysis of Gene Expression"); und/oder (ii) Bestimmen der Menge an Protein umfasst.

Somit wird im Rahmen dieser bevorzugten Ausführungsform die Bestimmung der Expression mittels des erfindungsgemäßen Trägers oder Kits um weitere, mit anderen Verfahren ermittelte Expressionsdaten bereichert.

Methoden zur Bestimmung der Menge an mRNA mittels PCR an sich sind dem Fachmann hinreichend bekannt und schließen beispielsweise real-time PCR und real competitive PCR ein. Methoden zur Bestimmung der Menge an mRNA mittels Ribonuklease Protection Assay oder SAGE an sich sind im Stand der Technik hinreichend bekannt und werden zum Beispiel in Ding und Cantor, 2004, beschrieben.

In einer weiteren bevorzugten Ausführungsform des erfindungsgemäßen Verfahrens erfolgt das Bestimmen der Expression der Fucosyltransferasen, Galactosyltransferasen, N-Acetyl-glucosaminyltransferasen, N-Acetylgalactosaminyl-transferasen, Sialyltransferasen, Zucker-Sulfotransferasen, Lectine, Selectine, Fucosidasen, Neuraminidasen, Sialidasen, Nucleotid-Zucker-Epimerasen, Zucker-Kinasen, Zucker-Epimerasen, Zucker-Transporter und Enzyme des Fucose-oder Sialinsäure-Metabolismus mittels Antikörpern oder Aptameren, die spezifisch für die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen, Sialidasen, Nucleotid-Zucker-Epimerasen, Zucker-Kinasen, Zucker-Epimerasen, Zucker-Transporter und Enzyme des Fucose- oder Sialinsäure-Metabolismus sind.

Der Begriff Antikörper im Sinne der Erfindung umfasst spezifische Antikörper oder Antiköperfragmente oder Antikörperderivate. Fragmente von Antikörpern sind z.B. Fab- oder F(ab')₂ - Fragmente. Derivate schließen scFvs ein. Verfahren zur Generierung spezifischer Antikörper sind aus dem Stand der Technik bekannt. Übliche Nachweisverfahren in denen die Antikörper Verwendung finden sind, wie oben bereits erwähnt, unter anderem ELISA, RIA oder auch Protein-Arrays aber auch Immunfluoreszenz, Durchflußzytometrie und andere Nachweisverfahren. Die Antikörper binden hierbei spezifisch an die Zielmoleküle. Die Antikörper-Bindung kann z.B. durch Markierung der primären Antikörper sichtbar gemacht werden oder wird mit Hilfe von Antiköper-bindenden zweiten Antikörper detektiert, die dann ihrerseits markiert sind. Die Antikörper können z.B. mit fluoreszierenden Substanzen, durch radioaktive Markierung oder eine enzymatische Markierung modifiziert sein. Immunologische Nachweisverfahren unter Verwendung von spezifischen Antikörpern sind an sich aus dem Stand der Technik bekannt (z.B. Harlow *et al.,* 1988) und schließen beispielsweise Western Blotting, ELISA, RIA, Protein-Arrays, Immunfluoreszenz oder Durchflußzytometrie ein. Geeignete Antikörper im Sinne der Erfindung sind beispielsweise, jedoch nicht ausschließlich, CD22 Antikörper, Antikörper gegen Thomsen-Friedenreich Glykotop sowie Antikörper für Sialyl-Lewis X und Sialyl-Lewis A Antigene.

Der Begriff Aptamer im Sinne der Erfindung umfasst Nukleinsäuren, die aufgrund ihrer dreidimensionalen Struktur spezifisch an ein Zielmolekül binden. Aptamere an sich sind im Stand der Technik ausreichend bekannt, z.B. in Osborne *et al.,* 1997.

In einer bevorzugten Ausführungsform der erfindungsgemäßen Verwendung oder des erfindungsgemäßen Verfahrens zeigt die Expression den Glykosylierungsstatus der Zelle, des Gewebes oder des Organismus an.

Unter Glykosylierungsstatus wird die Anzahl und die Art der Zuckerseitenketten von Proteinen auf/in einer Zelle verstanden. Die mit Hilfe der Expressionsanalyse gewonnene Kenntnis über die Beschaffenheit des Glykosylierungsapparates ermöglicht eine Korrelation mit dem Glykosylierungsstatus einer Zelle. Beispielsweise kann mit dem Wissen um eine verminderte Expression des Enzyms UDP-N-Acetylglucosamin-2-Epimerase/N-Acetylmannosamin-Kinase (GNE) - dem Schlüsselenzym der Sialinsäure-Synthese - eine Korrelation zur Dichte der Sialinsäure auf der Zelloberfläche hergestellt werden.

Die erfindungsgemäße Nutzung der Expressionsdaten zur Analyse des Glykosylierungsstatus kann zum Beispiel für das Profiling von Zelllinien zur Herstellung glykosylierter Biopharmazeutika genutzt werden. Mit Hilfe des Arrays wird die Auswahl der für die Biosynthese einer gewünschten Glykanstruktur optimalen Zelllinie vereinfacht. Des weiteren kann überprüft werden, ob Enzyme exprimiert werden, die die Synthese einer gewünschten Glykanstruktur verringern oder verhindern können.

Auch für die Überprüfung der Effekte von Glykoengineering ist die erfindungsgemäße Analyse von Expressionsmustern von Vorteil. Zelllinien müssen häufig zusätzlichem Glykoengineering unterzogen werden, entweder um bestimmte Glykosyltransferase-Aktivitäten zu verstärken und/oder um andere z.B. durch siRNA-Technologie zu reduzieren oder auszuschalten. Der Erfolg dieses Glykoengineering und die damit möglicherweise verbundenen zusätzlichen Veränderungen des Expressionsprofils können mit Hilfe des erfindungsgemäßen Verfahrens analysiert werden.

Auch der Einfluss von Änderungen in Zellkulturparametern wie beispielsweise Serum-Entzug oder Veränderungen in der O₂- und/oder CO₂- Gaskonzentration kann verfolgt werden, da diese einen wichtigen Einfluss auf das Glykosylierungsmuster von Produktzelllinien haben. Zusätzlich oder alternativ können die Expressionsmuster auch zur Prozessüberwachung der Konstanz von Zellkultivierungsprozessen während der Produktionsphase dienen.

In einer weiteren bevorzugten Ausführungsform der erfindungsgemäßen Verwendung oder des erfindungsgemäßen Verfahrens zeigt die Expression den Krankheitszustand, die Malignität, und/oder das Metastasierungspotential der Zelle, des Gewebes oder des Organismus an.

Der Begriff Malignität im Sinne der Erfindung beschreibt einen Krankheitsverlauf, der fortschreitend zerstörerisch wirkt und möglicherweise auch zum Tod führen kann. Im Zusammenhang mit Tumorerkrankungen bezeichnet Malignität die Fähigkeit eines Tumors, die Basalmembran zu durchdringen und Metastasen zu bilden. Ein solcher Tumor wird umgangssprachlich als Krebs bezeichnet. In Abhängigkeit von der Fähigkeit von Tumoren/Tumorzellen, solche Metastasen zu entwickeln, unterscheidet man niedrig-maligne und hochmaligne Tumoren.

Unter dem Begriff Metastasierungspotential versteht man die Fähigkeit von Zellen (im Allgemeinen Tumorzellen), Verbindungen zum umliegenden Gewebe zu kappen. Bei dieser Kappung können sich Zellverbände ablösen und abwandern. Ein zusätzlicher Defekt der Adhäsionsmoleküle auf der Oberfläche (maligner) Zellen, die für den Zellverbund unerlässlich sind, erleichtert dieses Abwandern zusätzlich.

Mit Hilfe der erfindungsgemäßen Verwendung oder des erfindungsgemäßen Verfahrens können so beispielsweise Expressionsprofile von Karzinomen unterschiedlicher Prognose aufgenommen werden und die Expressionsdaten mit Kategorien wie der Metastasierung des Karzinoms und der Überlebenszeit der Patienten korreliert werden, um so bei anderen Patienten Vorhersagen treffen zu können. Somit stellt ein Array-basiertes Profiling von tumor-exprimierten Glykosylierungsenzymen eine innovative Technologie für Diagnostik oder Therapieprädiktion zur Verfügung.

Für all diese Verwendungen und Verfahren bietet die vorliegende Erfindung hochparallele und rasch durchzuführende Alternativen zu aufwendigen Glykananalysen.

Die Erfindung betrifft des weiteren eine diagnostische Zusammensetzung umfassend das Set von Sonden wie oben definiert.

Des weiteren betrifft die Erfindung die Verwendung des erfindungsgemäßen Sets von Sonden zur Herstellung einer oder mehrerer diagnostischer Zusammensetzungen oder einer diagnostischen Vorrichtung zur Diagnose von Tumoren, entzündlichen und/oder neurologischen Erkrankungen.

Die Messung der Aktivität von Enzymen, deren Aktivität die Glykosylierung gewebs- und differenzierungsspezifisch reguliert, kann für diagnostische Zwecke eingesetzt werden. Zusätzlich kann auch die mRNA-Expression dieser Glyogene von diagnostischem Wert sein. So ist beispielsweise bekannt, dass bestimmte saure Zucker (Sialinsäuren) verstärkt in den Glykanstrukturen metastasierender Tumorzellen auftreten. Damit in Übereinstimmung steht, dass auch die Expression des die Bildung dieser Strukturen katalysierenden Enzyms (Sialyltransferase ST6Gal-I) in metastasierenden kolorektalen Karzinomen erhöht ist (Dall'Olio *et al.,* 1989; Kemmner *et al.,* 1994). Ähnliche Ergebnisse wurden in Untersuchungen von Magen-, Nieren-, Prostata und Mammakarzinomen gefunden (siehe Tabelle 2).

Auch die Immunabwehr hängt von einer regulierten Aktivierung der Glykosyltransferasen ab. Untersuchungen zeigen, dass sich das Expressionsprofil verschiedener Glykosyltransferasen während der T-Lymphozytenentwicklung in einer zeitlich und räumlich kontrollierten Art verändert (Baum, 2002). Zum Beispiel wird die Zuckersequenz NeuAcα2,6Gal, das Produkt der ST6Gal-I Sialyltransferase nur auf reifen medullären Thymozyten gefunden. Die Glykosylierung spielt darüber hinaus auch eine wichtige Rolle für die Aktivierung des HIV-Viruses (Lefebvre *et al.,* 1994) oder den Befall von Epithelzellen durch Influenzaviren (Stevens *et al.,* 2006).

Ein anderes hoch glykosyliertes Protein ist das Prion-Protein, das für die spongiformen Enzephalopathien, wie die bovine spongiforme Enzephalopathie (BSE) der Rinder und die Creutzfeldt-Jakob Krankheit des Menschen verantwortlich gemacht wird. Die Glykosylierung ist einer der Faktoren, die die Konversion des "normalen" Proteins in die krankheitsverursachende Form kontrollieren und diese hängt ab von den relativen Aktivitäten der Glykosyltransferasen der Wirtszelle.

Veränderungen des Expressionsmusters der Glycogene wurden darüber hinaus bei Alzheimer, neuromuskulären und hereditären Erkrankungen (CGD), die z.B. zu geistiger Retardation führen, nachgewiesen.

Tabelle 2 fasst wichtige Arbeiten zusammen, die zeigen, in welcher Weise sich die Expressionsmuster dieser Glycogene in verschiedenen Krankheitsbildern unterscheiden

Wie bereits oben definiert versteht man unter dem Begriff Tumor im Sinne der Erfindung die Neubildungen von Körpergeweben (Neoplasien), die durch Fehlregulationen des Zellwachstums entstehen. Es werden gutartige (benigne) und bösartige (maligne) Tumoren unterschieden.

Wie oben bereits definiert, versteht man unter entzündlichen Erkrankungen im Sinne der Erfindung Erkrankungen, die eine erste Reaktion des Immunsystems auf Infektionen oder Reize wie z.B. physikalische Reize, Mikroorganismen, Allergene, Toxine oder fehlfunktionierende Enzyme darstellen, mit der Funktion, den Schädigungsreiz zu beseitigen oder zu reparieren. Häufig auftretende entzündliche Erkrankungen sind beispielsweise Arthritis, Myokarditis, Dermatitis, Otitis, Pneumonie, Rheumatische Erkrankungen, Crohn's Disease, Ulcerative Colitis, Spondylitis, Osteoarthritis oder Psoriasis.

Unter neurologischen Erkrankungen im Sinne der Erfindung werden, wie oben bereits definiert, Erkrankungen des Nervensystems verstanden. Die betroffenen Organsysteme sind das Zentralnervensystem und das periphere Nervensystem inklusive ihrer Umgebungsstrukturen. Neurologische Erkrankungen umfassen beispielsweise Multiple Sklerose, Hirnhautentzündungen, Rückenmarksentzündungen, Hirninfarkt, Parkinson, Gehirntumoren, Migräne, epileptische Anfallsleiden, Demenzen und Muskeldystrophien.

**Tabelle 2 Expressionsmuster von Glyogenen bei unterschiedlichen Krankheitsbildern.**

| | |
|---|---|
| **Krankheit** | **Veröffentlichung** |
| Alzheimer | • B. Espinosa et al.; 2001; J.Neuropathol.Exp.Neurol. 60(5):441. |
| | • Y. Huang et al.; 2004; Eur.J.Neurosci. 20(12):3489. |
| | • L. A. Robertson et al.; 2004; J.Alzheimers.Dis. 6(5):489. |
| Krebserkrankungen | • F. Schneider et al.; 2001; Cancer Res. 61(11):4605. |
| | • E. Y. Song et al.; 2001; Cancer Invest 19(8):799. |
| | • T. Petretti et al.; 2000; Gut 46(3):359. |
| | • T. Takahashi et al.; 2000; Int.J.Cancer 88(6):914. |
| | • I. Brockhausen; 1999; Biochim.Biophys.Acta 1473(1):67. |
| | • J. Burchell et al.; 1999 ; Glycobiology. 9(12):1307. |
| | • J. W. Dennis et al.; 1999; Biochim.Biophys.Acta 1473(1):21. |
| | • T. F. Orntoft and E. M. Vestergaard; 1999; Electrophoresis 20(2):362. |
| | • T. Petretti et al.; 1999; Biochim.Biophys.Acta 1428(2-3):209. |
| | • T. Kudo et al.; 1998 ; Lab.Invest. 78(7):797. |
| | • H. Ito et al.; 1997; Int.J.Cancer 71(4):556. |
| | • G. Sotiropoulou et al.; 2002; Mol. Med. 8(1):42. |
| | • S. Saito et al.; 2002; Oncol.Rep. 9(6):1251. |
| | • Y. Ide et al.; 2006; Biochem.Biophys.Res.Commun. 341 (2):478. |
| | • X. L. Jin et al.; 2004; Hepatobiliary.Pancreat.Dis.Int. 3(2):292. |
| | • J. Jaeken and G. Matthijs; 2001; Annu.Rev.Genomics Hum.Genet. 2:129. |
| Hereditäre Erkrankungen | • B. S. Miller and H. H. Freeze; 2003; Rev.Endocr.Metab Disord. 4(1):103. |
| | |
| | • E. A. Eklund and H. H. Freeze; 2006; NeuroRx. 3(2):254. |
| | • L. Sturla et al.; 2005; Glycobiology 15(10):924. |
| | • E. Martin-Rendon and D. J. Blake; 2003; Trends Pharmacol.Sci. 24(4):178. |
| Erkrankungen des | • J. B. Lowe; 2001; Cell 104(6):809. |
| Immunsystems | • G. Alvarez et al.; 1999; Immunol.Invest. 28(1):9. |
| | • J. S. Axford; 1999; Biochim.Biophys.Acta 1455(2-3):219. |
| | • P. J. Delves; 1998; Autoimmunity 27(4):239. |
| | • L. Galli Stampino et al.; 1997; Cancer Res. 57(15):3214. |
| | • A. Orlacchio et al.; 1997; J.Neurol.Sci. 151(2):177. |
| | • J. C. Lefebvre et al.; 1994; Virology 199:265. |
| | • T. Feizi and M. Larkin; 1990; Glycobiology 1(1):17. [Erratum in |
| | Glycobiology 1991 Jun;1(3):315]. |
| | • M. Demetriou et al.; 2001; Nature 409(6821):733. |
| | • M. Bunting et al.; 2002; Curr.Opin.Hematol. 9(1):30. |
| | • E. I. Buzas et al.; 2006; Autoimmunity 39(8):691. |
| | • M. Lanteri et al.; 2003; Glycobiology 13(12):909. |
| Muskeldystrophien | • A. Yoshida et al.; 2001; Dev.Cell 1(5):717. |
| | • B. Xia et al.; 2002; Dev.Biol. 242(1):58. |
| | • T. Endo and H. Manya; 2006; 417:137. |
| | |
| Neurologische | • H. Narimatsu; 2006; CNS.Neurol.Disord.Drug Targets. 5(4):441. |
| Erkrankungen | |
| Prionen-vermittelte | • P. M. Rudd et al.; 2002; Curr. Opin. Struct. Biol. 12(5):578. |
| Erkrankungen | • C. J. Bosques and B. Imperiali; 2003; Proc.Natl.Acad.Sci.U.S.A. 100(13):7593. |
| | • M. Ermonval et al.; 2003; Biochimie 85(1-2):33. |
| Grippeerkrankungen (Influenza) | • J. Stevens et al.; 2006; J.Mol.Biol. 355(5):1143. |
| | • K. F. Winklhofer et al.; 2003; Traffic. 4(5):313. |
| | • M. Matrosovich et al.; 2003; J.Virol. 77(15):8418. |
| | • S. J. Morris et al.; 1999; J.Gen.Virol. 80(Pt 1):137. |

In einer bevorzugten Ausführungsform der erfindungsgemäßen Verwendung ist der Tumor ausgewählt aus der Gruppe bestehend aus kolorektalem Karzinom, Magenkarzinom, Mammakarzinom, Pankreaskarzinom, Melanom, Sarkom und Lymphome, wie beispielsweise Hodgkin Lymphome und Nicht-Hodgkin Lymphome.

Die Figuren zeigen:
**Figur 1****:**
   Hybridisierung des erfindungsgemäßen Trägers mit cDNA Proben. Auf der linken Seite ist der gesamte Array mit insgesamt 4 Feldern dargestellt. Im rechten Teil ist einer der 4 Subarrays vergrößert dargestellt. cDNAs wurden mit Hilfe des Invitrogen direkt Kits markiert.
**Figur 2****:**
   Immunhistochemische Färbungen von Pankreaskarzinomzellen mit einem für das Glykoprotein CD22 spezifischen Antikörper. p16- Transfektanten entsprechen mit p16 transfizierten Capanzellen; mock-Transfektanten entsprechen Capanzellen, die mit dem gleichen Vektor mit Antibiotikaresistenz jedoch ohne Targetgensequenz transfiziert wurden; RAJI-Zellen dienen als Positivkontrolle. Rot zeigt die Fluoreszenz des CD22-spezifischen Antikörpers. In Blau sind die Zellkerne (DAPI-Färbung) markiert.
**Figur 3****:**
   Bestimmung der membrangebundenen Sialinsäure auf der Oberfläche von Pankreaskarzinomzellen. p16 sind p16-transfizierte Capanzellen; mock sind mit Vektor mit Antibiotikaresistenz jedoch ohne Targetgensequenz transfizierte Capanzellen.

Die Beispiele erläutern die Erfindung. Die Beispiele sollen nicht als Limitierung der Erfindung angesehen werden. Die Beispiele sind lediglich zur Illustration der Erfindung beigefügt und die Erfindung wird lediglich durch die Ansprüche begrenzt.

### Beispiel 1:

### Probenvorbereitung und Array-Hybridisierung

Gesamt-RNA wird mittels RNeasy extrahiert und die Qualität und Menge der erhaltenen RNA mit Hilfe des BioAnalyzers (Agilent) überprüft. Anschließend wird die RNA in einer reversen Transkription mit Fluoreszenzfarbstoffen markiert. Verschiedene Verfahren zur Markierung der cDNA-Proben wurden gemäß der Anleitung des Herstellers durchgeführt und gestestet (siehe Tabelle 2). Insgesamt waren dies 7 verschiedene Labelling-Methoden mit verschiedenen Farbstoffen, wie Alexa 647/546 oder Cy3/Cy5 sowie mit verschiedenen reversen Transkriptasen. Im Vergleich dazu wurden die schon länger verwendeten Methoden von Amersham direkt/indirekt mit Cy3/Cy5 - Farbstoffen und ein Kit von Invitrogen direkt getestet. Die besten Ergebnisse bezüglich der Signalintensität zeigte das letztgenannte Verfahren.

**Tabelle 2: Tests verschiedener cDNA Labelling-Methoden**

| **Verfahren** | **Ergebnis** |
|---|---|
| Amersham direkt Cy3/Cy5 | schwache Signale, nicht zufriedenstellend |
| 3DNA Array 350 Cy3/Cy5 | Signale nicht zufriedenstellend |
| Amersham indirekt Cy3/Cy5 | Signale nicht genügend reproduzierbar |
| 3DNA Array 900 Alexa 647/546 | ungeeignet |
| 3DNA Array 350 Alexa 546/647, Powerscript II | deutliche rote, gelbe und grüne Signale |
| 3DNA Array 350 Alexa 546/647, Superscript II | deutliche rote, gelbe und grüne Signale |
| Invitrogen direkt, Alexa 546/647 | deutliche rote, gelbe und grüne Signale |

Die Qualität des Labellings wird dann über NanoDrop-Messungen überprüft und die markierte cDNA in der aHyb-Station (Miltenyi) auf den Array hybridisiert. Die Fluoreszenz wird mit Hilfe des AGILENT-Scanners gemessen und die Daten werden mit Hilfe der AGILENT-Software ausgewertet.

### Beispiel 2:

### Vergleich der Expressionsmuster ausgesuchter Targetmoleküle mittels GlycoProfiler Array und real time PCR in transfizierten Capan-1 Pankreaskarzinomzellen.

Die Pankreaszelllinie CAPAN-1, welche mit dem Tumorsuppressor-Gen p16 transfiziert wurde (p16), sowie eine mocktransifizierte Kontrollzelllinie (CAPAN-1 transfiziert mit dem gleichen Vektor mit Antibiotikaresistenzgenen aber ohne Targetgensequenz) wurden mit dem GlycoProfiler Array charakterisiert.
Der Tumorsuppressor p16 ist ein Inhibitor von Cyclin-abhängigen Kinasen (cdk4+6), die eine wichtige Rolle bei der Kontrolle der Zellzyklus spielen, in dem sie das Produkt der Retinoblastoms (pRb) phosphorylieren. Die Expression von p16 ist im normalen Zellzyklus streng kontrolliert. Die Microarray-Untersuchungen zeigen deutliche Unterschiede in der Genexpression der Glykosylierungsgene zwischen der p16- und der mock-transfizierten Linie. Dies bestätigt, dass Glykosylierung und Glykosylierungs-abhängige Funktionen wie die Lectinbindung oder Anoikis-Induktion durch die Transfektion mit p16 drastisch verändert werden.
Zusätzlich wurden die Hybridisierungs-Ergebnisse des Microarrays mit quantitativer real time RT-PCR (Taqman) überprüft. Die PCR-Ergebnisse dienen dabei als Goldstandard, um die Spezifität der Microarray-Ergebnisse zu bewerten. Dazu wurde eine Bibliothek von PCR-Primern für 17 der wichtigsten Targetmoleküle des GlycoProfiler Arrays etabliert. Wie in Tabelle 3 dargestellt, stimmen die Ergebnisse der Microarray Untersuchung gut mit der real-time PCR überein. So zeigen z.B. im Fall des Enzyms UDP-N-Acetylglucosamin-2-Epimerase/N-Acetylmannosamin-Kinase (GNE) sowohl die PCR- als auch die Arraydaten eine sehr geringe Expression des Enzyms in p16-transfizierten Zellen (Tabelle 3). UDP-N-Acetylglucosamin-2-Epimerase/ NAcetylmannosamin-Kinase ist das Schlüsselenzym der Biosynthese von Sialinsäuren, welches die ersten beiden Schritte des Stoffwechselweges reguliert und somit entscheidend an dessen Regulation beteiligt ist. Die beiden Methoden stimmen auch für die Expression der CMP-NeuAc-Synthetase (CMAS) überein, eines weiteren wichtigen "down-stream" gelegenen Enzyms des Sialinsäurestoffwechsels, welches in p16- transfizierten Zellen stark erhöht ist. Wie auch die weiteren Beispiele zeigen (Tabelle 3) ist der Microarray in der Lage, das Genexpressionsprofil der Zellen in zufriedenstellender Weise zu bestimmen.

**Tabelle 3: Unterschiede in der Genexpression ausgewählter Targetmoleküle mittels GlycoProfiler Array (zweite Spalte) und real time PCR (erste Spalte). Expressionswerte sind dargestellt als Quotienten aus der Expression (relative amount) von p16/mock.**

| **Gen** | **Taqman** | **GlycoProfiler** |
|---|---|---|
| **B3GNT3** | 0,09 | 0,14 |
| **B3GNT5** | 4,98 | 1,93 |
| **C2GNT-L** | 0,33 | 0,18 |
| **C2GNT-M** | 0,09 | 0,10 |
| **CD22** | 0,02 | 0,51 |
| **CMAS** | 3,00 | 2,30 |
| **GNE** | 0,01 | 0,04 |
| **FUT6** | 0,20 | 0,91 |
| **FUT8** | 0,21 | 0,31 |
| **Gal-1** | 1,11 | 1,29 |
| **Gal-3** | 0,64 | 0,48 |
| **MGAT3** | 0,57 | 2,09 |
| **MGAT5** | 0,37 | 0,72 |
| **NANS** | 0,57 | 0,93 |
| **ST3Gal IV** | 1,05 | 0,96 |
| **ST6Gal I** | 1,43 | 1,12 |
| **ST6GaINAc II** | 0,36 | 0,51 |

### Beispiel 3:

### CD22-Expression in Transfektanten von Capan-1 Pankreaskarzinomzellen.

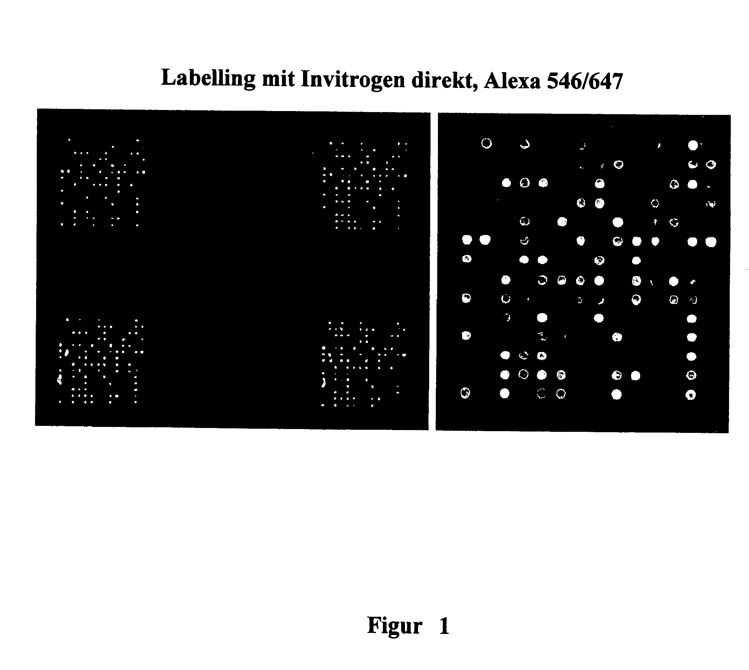

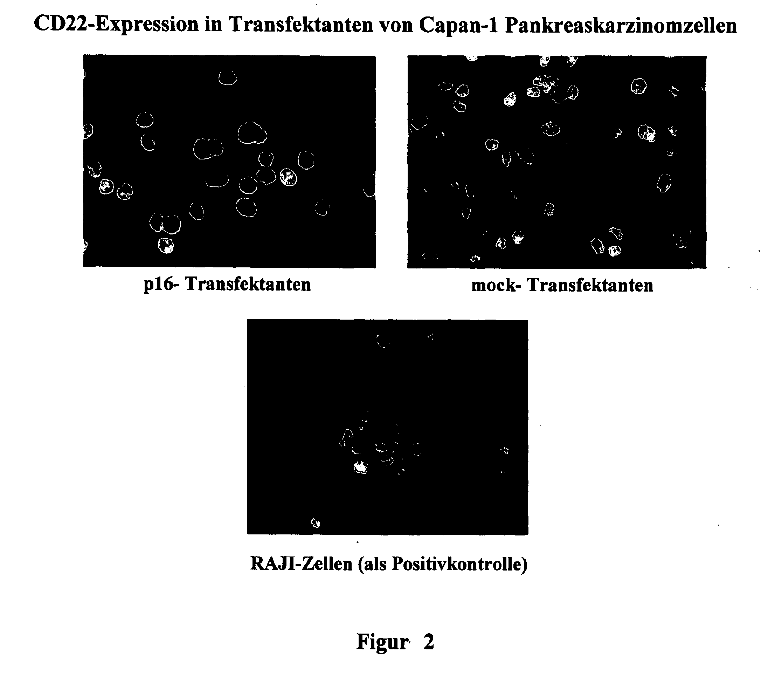

Sowohl der GlycoProfile Array als auch RT-PCR zeigen, dass CD22, ein Sialinsäurebindendes Lectin, stärker in mock- als in p16- transfizierten Zellen exprimiert wird. Wie in Abbildung 2 gezeigt, kann diese CD22-Minderexpression in p16-Zellen auch in immunhistochemischen Untersuchungen bestätigt werden. Die Färbung der mock-Zellen durch einen CD22-Antikörper ist wesentlich stärker als die der p16-transfizierten Zellen (Abb. 2). In diesem Fall ist es also möglich direkt von der Expression der mRNA von CD22 auf das Vorliegen des Lectinproteins CD22 zu schließen.

### Beispiel 4:

### Membrangebundene Menge an Sialinsäure auf der Oberfläche von transfizierten Capan-1 Pankreaskarzinomzellen.

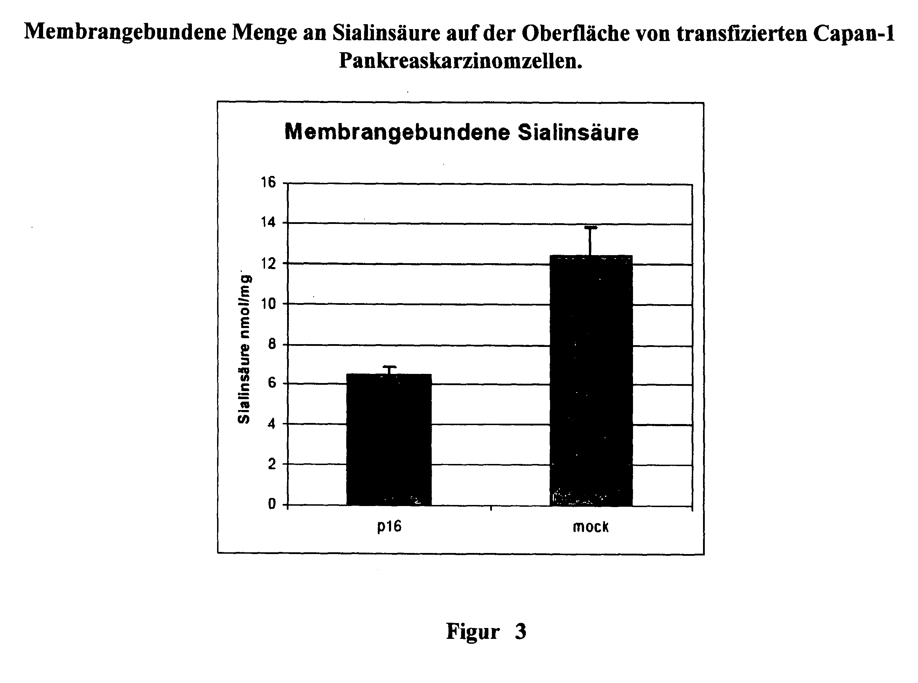

Untersuchungen der Menge membrangebundener Sialinsäure auf der Zelloberfläche von p16- und mock-transfizierten Zellen mit einem sehr empfindlichen HPLCgestützten Nachweisverfahren (Hara *et al*.,1987) zeigen, dass eine verringerte Sialinsäureproduktion und Sialinsäure-Dichte auf Glykokonjugaten der Zellmembran in p16-transfizierten Zellen vorliegt (Abb. 3). Auf der Zelloberfläche von p16-Transfektanten wurde signifikant weniger Sialinsäure als auf den Mock-Transfektanten nachgewiesen (6,5 nmol Sialinsäure/mg Protein im Vergleich zu 12,4 nmol/mg).
In Kombination mit der in Beispiel 2 gezeigten verminderten Expression des Enzyms GNE - dem Schlüsselenzym der Sialinsäure-Synthese - in p16- transfizierten Zellen zeigen diese Ergebnisse, dass aus der mRNA Expression von GNE zumindest zum Teil auf die Dichte der Sialinsäure auf der Zelloberfläche geschlossen werden kann.

### Beispiel 5:

### Glykoprofiling von Zelllinien unter verschiedenen Kulturbedingungen

Das Glykoprofiling kann zur Überwachung zeitlicher Veränderungen des Expressionsmusters des Glykosylierungsapparates von Zelllinien verwendet werden, die z.B. veränderten Kulturbedingungen ausgesetzt werden. Von besonderer Bedeutung für die Kultur von Produktionszelllinien ist der Einfluss des Serumgehalts, sowie von Temperatur und Gasen. Daher ist es von Interesse, das Expressionsmuster des Glykosylierungsapparates von Zelllinien unter Serumdepletion oder bei Kultivierung in Serum-freien Medien mit Hilfe der Microarray-Technik zu charakterisieren.
Die Extraktion der Gesamt-RNA sowie Markierung der cDNA, Hybridisierung mit dem Array und Datenauswertung erfolgen wie in Beispiel 2 beschrieben.

### Beispiel 6:

### Glykoprofiling von Glykoengineerten Produktionszellinien

Zelllinien können durch Transfektion von Glykosyltransferasen oder durch deren Ausschalten über siRNA-Techniken glykoengineert werden. Um zu klären, in welcher Weise sich solche Eingriffe auf dem Niveau des Glykoprofilings widerspiegeln und ob möglicherweise auch ganz andere als die veränderten Elemente der Glykosylierungsmaschinerie auf solche spezifischen Eingriffe reagieren, kann der GlycoProfiler Array verwendet werden. Damit können qualitative und quantitative Veränderungen der verschiedenen in einer Zelllinie exprimierten Glykosyltransferasen nach einem spezifischen Glykoengineering erfasst werden.

Dazu werden ausgewählte Glykosyltransferasen, wie die Sialyltransferase ST6Gal-1, in Zelllinien überexprimiert oder durch siRNA reduziert oder ausgeschaltet und das Expressionsmuster des Glykosylierungsapparates der Zellen analysiert. Die Extraktion der Gesamt-RNA sowie Markierung der cDNA, Hybridisierung mit dem Array und Datenauswertung erfolgen wie in Beispiel 2 beschrieben.

### Beispiel 7:

### Glycoprofiling von humanen Tumorgeweben

Quantitative Messungen einzelner Glykanstrukturen auf Zellen oder im Gewebe sind aufgrund ihrer Komplexität und des geringen Vorkommens sehr schwierig. Die oben genannten Beispiele zeigen jedoch, dass das Expressionsprofil der in einer Zelle vorhandenen Glykosyltransferasen Hinweise auf die gebildeten Glykanstrukturen geben und damit von diagnostischem Wert sein kann.
Jeweils 20 Proben kolorektaler Karzinome, Magenkarzinome und Mammakarzinome und der dazugehörigen Normalgewebe werden untersucht und die Profile mit den Ergebnissen der real time PCR verglichen.
Dazu werden die Gewebe am Kryostat geschnitten und von einem experimentellen Pathologen begutachtet. Die Auswahl und Begutachtung des zu untersuchenden Materials ist von besonderer und größter Bedeutung für die nachfolgenden Untersuchungen. Nur Biopsien, die zu mehr als 60% aus Karzinomgewebe bestehen und keine Lymphfollikel, wenig Binde- und nur geringe Mengen an Fettgewebe enthalten und überdies keine Anzeichen von Nekrose zeigen, werden weiter prozessiert. Die Extraktion der Gesamt-RNA sowie Markierung der cDNA, Hybridisierung mit dem Array und Datenauswertung erfolgen wie in Beispiel 2 beschrieben.
Auf diese Art können Expressionsprofile von Karzinomen unterschiedlicher Prognose aufgenommen werden und die Expressionsdaten mit Kategorien wie der Metastasierung des Karzinoms und der Überlebenszeit der Patenten korreliert werden.

### Weitere Literatur

Auburn RP, Kreil DP, Meadows LA, Fischer B, Matilla SS, Russell S. Robotic spotting of cDNA and oligonucleotide microarrays. Trends Biotechnol. 2005 Jul;23(7):374-9.

Ausubel, "Current Protocols in Molecular Biology", Green Publishing Associates and Wiley Interscience, N.Y.,1989

Baum LG. Developing a taste for sweets. Immunity. 2002 Jan;16(1):5-8.

Castoldi M, Schmidt S, Benes V, Noerholm M, Kulozik AE, Hentze MW, Muckenthaler MU A sensitive array for microRNA expression profiling (miChip) based on locked nucleic acids (LNA). RNA. 2006 May;12(5):913-20.

Dall'Olio F, Malagolini N, di Stefano G, Minni F, Marrano D, Serfini-Cessi F. Increased CMP-NeuAc:Gal beta 1,4GlcNAc-R alpha 2,6 sialyltransferase activity in human colorectal cancer tissues. Int J Cancer. 1989 Sep 15;44(3):434-9.

Ding C, and Cantor C.R., Quantitative Analysis of Nucleic Acids - the Last Few Years of Progress. Journal of Biochemistry and Molecular Biology. 2004 37, 1-10.

Gao X, LeProust E, Zhang H, Srivannavit O, Gulari E, Yu P, Nishiguchi C, Xiang Q, Zhou X. A flexible light-directed DNA chip synthesis gated by deprotection using solution photogenerated acids. Nucleic Acids Res. 2001 Nov 15;29(22):4744-50.

Hara S, Takemori Y, Yamaguchi M, Nakamura M, Ohkura Y. Fluorometric highperformance liquid chromatography of N-acetyl- and N-glycolylneuraminic acids and its application to their microdetermination in human and animal sera, glycoproteins, and glycolipids. Anal Biochem. 1987 Jul;164(1):138-45.

Harlow et al., Antibodies: A Laboratory Manual, Cold Spring Harbor Laboratory Press, 1988

Harris and Winssinger, PNA encoding (PNA=peptide nucleic acid): from solutionbased libraries to organized microarrays. Chemistry. 2005 Nov 18;11(23):6792-801.

Higgins and Hames (Eds.) "Nucleic acid hybridization, a practical approach" IRL Press Oxford, Washington DC, 1985

Kamme F, Salunga R, Yu J, Tran DT, Zhu J, Luo L, Bittner A, Guo HQ, Miller N, Wan J, Erlander M. Single-cell microarray analysis in hippocampus CA1: demonstration and validation of cellular heterogeneity. J Neurosci. 2003 May 1;23(9):3607-15.

Kemmner W, Kruck D, Schlag P., Different sialyltransferase activities in human colorectal carcinoma cells from surgical specimens detected by specific glycoprotein and glycolipid acceptors. Clin Exp Metastasis. 1994 May;12(3):245-54.

Lausted C, Dahl T, Warren C, King K, Smith K, Johnson M, Saleem R, Aitchison J, Hood L, Lasky SR. POSaM: a fast, flexible, open-source, inkjet oligonucleotide synthesizer and microarrayer. Genome Biol. 2004;5(8):R58. Epub 2004 Jul 27.

Lefebvre JC, Giordanengo V, Doglio A, Cagnon L, Breittmayer JP, Peyron JF, Lesimple J. Altered sialylation of CD45 in HIV-1-infected T lymphocytes. Virology. 1994 Mar;199(2):265-74.

Manduchi E, Scearce LM, Brestelli JE, Grant GR, Kaestner KH, Stoeckert CJ Jr. Comparison of different labeling methods for two-channel high-density microarray experiments. Physiol Genomics. 2002 Sep 3;10(3):169-79.

Osborne, SE et al.: Aptamers as therapeutic and diagnostic reagents: problems and prospects. Curr Opin Chem Biol. 1997 Jun;1(1):5-9

Sambrook, Russell "Molecular Cloning, A Laboratory Manual", Cold Spring Harbor Laboratory, N.Y., 2001

Schneider F, Kemmner W, Haensch W, Franke G, Gretschel S, Karsten U, Schlag PM. Overexpression of sialyltransferase CMP-sialic acid:Galbeta1,3GalNAc-R alpha6-Sialyltransferase is related to poor patient survival in human colorectal carcinomas. CancerRes. 2001 Jun 1;61(11):4605-11.

Sperandio, M.: Selectins and glycosyltransferases in leukocyte rolling in vivo. FEBS J. 2006 Oct;273(19):4377-89

Stevens J, Blixt O, Glaser L, Taubenberger JK, Palese P, Paulson JC, Wilson IA. Glycan microarray analysis of the hemagglutinins from modern and pandemic influenza viruses reveals different receptor specificities. J Mol Biol. 2006 Feb 3;355(5):1143-55

## Patentansprüche

1. Träger, auf den ein Set von Sonden aufgebracht ist, wobei das Set umfasst:
(a) Sonden, die in der Lage sind, spezifisch mit Nukleinsäuren zu hybridisieren, die Fucosyltransferasen kodieren;
(b) Sonden, die in der Lage sind, spezifisch mit Nukleinsäuren zu hybridisieren, die Galactosyltransferasen kodieren;
(c) Sonden, die in der Lage sind, spezifisch mit Nukleinsäuren zu hybridisieren, die N-Acetyl-glucosaminyltransferasen kodieren;
(d) Sonden, die in der Lage sind, spezifisch mit Nukleinsäuren zu hybridisieren, die N-Acetyl-galactosaminyltransferasen kodieren;
(e) Sonden, die in der Lage sind, spezifisch mit Nukleinsäuren zu hybridisieren, die Sialyltransferasen kodieren;
(f) Sonden, die in der Lage sind, spezifisch mit Nukleinsäuren zu hybridisieren, die Zucker-Sulfotransferasen kodieren;
(g) Sonden, die in der Lage sind, spezifisch mit Nukleinsäuren zu hybridisieren, die Lectine kodieren;
(h) Sonden, die in der Lage sind, spezifisch mit Nukleinsäuren zu hybridisieren, die Selectine kodieren;
(i) Sonden, die in der Lage sind, spezifisch mit Nukleinsäuren zu hybridisieren, die Fucosidasen kodieren;
(j) Sonden, die in der Lage sind, spezifisch mit Nukleinsäuren zu hybridisieren, die Neuraminidasen (Sialidasen) kodieren;
(k) Sonden, die in der Lage sind, spezifisch mit Nukleinsäuren zu hybridisieren, die Nucleotid-Zucker-Epimerasen kodieren;
(l) Sonden, die in der Lage sind, spezifisch mit Nukleinsäuren zu hybridisieren, die Zucker-Kinasen kodieren;
(m) Sonden, die in der Lage sind, spezifisch mit Nukleinsäuren zu hybridisieren, die Zucker-Epimerasen kodieren; und
(n) Sonden, die in der Lage sind, spezifisch mit Nukleinsäuren zu hybridisieren, die Zucker-Transporter kodieren; und
(o) Sonden, die in der Lage sind, spezifisch mit Nukleinsäuren zu hybridisieren, die für Enzyme des Fucose- oder Sialinsäure-Metabolismus kodieren.

2. Träger nach Anspruch 1, der ein fester Träger ist.

3. Träger nach Anspruch 2, wobei der Träger Glas ist.

4. Träger nach einem der Ansprüche 1 bis 3, wobei die Sonden OligonukleotidSonden sind.

5. Träger nach einem der vorangegangenen Ansprüche, wobei die Sonden eine Länge von etwa 70 Basen haben.

6. Träger nach einem der vorangegangenen Ansprüche, wobei die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen, Sialidasen, Nucleotid-Zucker-Epimerasen, Zucker-Kinasen, Zucker-Epimerasen, Zucker-Transporter und/oder Enzyme des Fucose- oder Sialinsäure-Metabolismus aus Tabelle 1 ausgewählt sind.

7. Träger nach einem der vorangegangenen Ansprüche, wobei jedes der Proteine von Tabelle 1 durch mindestens eine Sonde auf dem Träger repräsentiert ist.

8. Kit, umfassend das Set von Sonden wie in einem der Ansprüche 1 oder 4 bis 7 definiert.

9. Verwendung des Trägers nach einem der Ansprüche 1 bis 7 oder des Kits nach Anspruch 8 zum quantitativen Bestimmen der Expression von Nukleinsäuren, die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen, Sialidasen, Nucleotid-Zucker-Epimerasen, Zucker-Kinasen, Zucker-Epimerasen, Zucker-Transporter und Enzyme des Fucose- oder Sialinsäure-Metabolismus kodieren, in einer Probe, die einer Zelle, einem Gewebe oder einem Organismus entnommen ist.

10. Verfahren zum quantitativen Bestimmen der Expression von Nukleinsäuren, die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen (Sialidasen), Nucleotid-Zucker-Epimerasen, Zucker-Kinasen, Zucker-Epimerasen, Zucker-Transporter und Enzyme des Fucose- oder Sialinsäure-Metabolismus kodieren, umfassend die Schritte
(a) Inkontaktbringen des Trägers nach einem der Ansprüche 1 bis 7 mit einer Präparation markierter Nukleinsäuren, die mittels reverser Transkription der mRNAs erhalten wurde, die in einer Probe vorliegen, die einer Zelle, einem Gewebe oder einem Organismus entnommen ist; und
(b) Quantifizieren der Menge an Markierung, die an die Sonden auf dem Träger gebunden ist.

11. Verfahren nach Anspruch 10, des weiteren umfassend den Schritt
(c) Bestimmen der Expression der Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen, Sialidasen, Nucleotid-Zucker-Epimerasen, Zucker-kinasen, Zucker-Epimerasen, Zucker-Transporter und Enzyme des Fucose- oder Sialinsäure-Metabolismus, wobei das Bestimmen der Expression in Schritt (c)
(I) Bestimmen der Menge an mRNA mittels PCR, Ribonuklease Protection Assay oder SAGE ("Serial Analysis of Gene Expression"); und/oder
(II) Bestimmen der Menge an Protein umfasst.

12. Verfahren nach Anspruch 11, wobei das Bestimmen in Schritt (c) mittels Antikörpern erfolgt, die spezifisch für die Fucosyltransferasen, Galactosyltransferasen, N-Acetyl-glucosaminyltransferasen, N-Acetyl-galactosaminyltransferasen, Sialyltransferasen, Zucker-Sulfotransferasen, Lectine, Selectine, Fucosidasen, Neuraminidasen, Sialidasen, Nucleotid-Zucker-Epimerasen, Zucker-kinasen, Zucker-Epimerasen, Zucker-Transporter und Enzyme des Fucose- oder Sialinsäure-Metabolismus sind.

13. Verwendung nach Anspruch 9 oder Verfahren nach einem der Ansprüche 10 bis 12, wobei die Expression
(a) den Glykosylierungsstatus;
(b) den Krankheitszustand;
(c) die Malignität; und/oder
(d) das Metastasierungspotential
der Zelle, des Gewebes oder des Organismus anzeigt.

14. Diagnostische Zusammensetzung umfassend das Set von Sonden wie in einem der Ansprüche 1 oder 4 bis 7 definiert.

15. Verwendung des Sets von Sonden wie in einem der Ansprüche 1 oder 4 bis 7 definiert zur Herstellung einer oder mehrerer diagnostischer Zusammensetzungen oder einer diagnostischen Vorrichtung zur Diagnose von Tumoren, entzündlichen und/oder neurologischen Erkrankungen.

16. Verwendung nach Anspruch 15, wobei der Tumor ausgewählt ist aus der Gruppe bestehend aus kolorektalem Karzinom, Magenkarzinom, Mammakarzinom, Pankreaskarzinom, Melanom, Sarkom und Lymphome, wie beispielsweise Hodgkin Lymphome und Nicht-Hodgkin Lymphome..


SEQUENCE LISTING

<110> Max-Delbrück-Centrum für Molekulare Medizin (MDC)

<120> Microarray für die Expressionsanalyse der zellulären
Glykosylierungsmaschinerie

<130> M2778 EP

<160> 468

<170> PatentIn version 3.4

<210> 1
<211> 2106
<212> DNA
<213> Homo sapiens

<400> 1
actgccctcc ggccgccgcg ccgcccgccc gccggggccc cgctgtccgc cgcccgccgc 60

cgctggagct agagatggat ttgcagccgc tgcaagtgtg tggaagggcc gtgttcgtgt 120

tggcaaagaa ggtcggctgc tgagccaggg cgtgtctccc ggaggcctgt gggctgccag 180

gatccccacc tctctgcaat gggctgccca ggctgaccag ccggttcctg ctggaagctc 240

ctggtctgat ctggggatac catgtccaag ccccccgacc tcctgctgcg gctgctccgg 300

ggcgccccaa ggcagcgggt ctgcaccctg ttcatcatcg gcttcaagtt cacgtttttc 360

gtctccatca tgatctactg gcacgttgtg ggagagccca aggagaaagg gcagctctat 420

aacctgccag cagagatccc ctgccccacc ttgacacccc ccaccccacc ctcccacggc 480

cccactccag gcaacatctt cttcctggag acttcagacc ggaccaaccc caacttcctg 540

ttcatgtgct cggtggagtc ggccgccaga actcaccccg aatcccacgt gctggtcctg 600

atgaaagggc ttccgggtgg caacgcctct ctgccccggc acctgggcat ctcacttctg 660

agctgcttcc cgaatgtcca gatgctcccg ctggacctgc gggagctgtt ccgggacaca 720

cccctggccg actggtacgc ggccgtgcag gggcgctggg agccctacct gctgcccgtg 780

ctctccgacg cctccaggat cgcactcatg tggaagttcg gcggcatcta cctggacacg 840

gacttcattg ttctcaagaa cctgcggaac ctgaccaacg tgctgggcac ccagtcccgc 900

tacgtcctca acggcgcgtt cctggccttc gagcgccggc acgagttcat ggcgctgtgc 960

atgcgggact tcgtggacca ctacaacggc tggatctggg gtcaccaggg cccgcagctg 1020

ctcacgcggg tcttcaagaa gtggtgttcc atccgcagcc tggccgagag ccgcgcctgc 1080

cgcggcgtca ccaccctgcc ccctgaggcc ttctacccca tcccctggca ggactggaag 1140

aagtactttg aggacatcaa ccccgaggag ctgccgcggc tgctcagtgc cacctatgct 1200

gtccacgtgt ggaacaagaa gagccagggc acgcggttcg aggccacgtc cagggcactg 1260

ctggcccagc tgcatgcccg ctactgcccc acgacgcacg aggccatgaa aatgtacttg 1320

tgaggggccc gccaggtcac ctccccaacc tgctcctgat ggggcactgg gccgcccttc 1380

ccggggaggc aagattgagg gcccgggaga gggaggcccg agctgccacc gggcttaggc 1440

aggctgttga ggagctgtgg gagcaggccc agtgggaggc tgtggacacc ccgaggacag 1500

tgtcctgtct cgaggcaggg ctgacacatg gtgccatagc cagcggaggg cgctcagtga 1560

gtgccccggg ccttctagac aacaggcagg aaggatgaac ctcagggcac ccccaggtgg 1620

tgcggaaagc caggcagttg ggacagaggt gcccacgagg gcagaggccg gtgctaaggg 1680

gatggggaag aagggacaag attcccagag aggagaggag gctgttggta ggaaagtggc 1740

agggctgggg gagacccagc cccaagggtc cggggcggag gatgctttgt tcttttctgg 1800

ttttggttcc tctttcgcgg ggggtggggg aggtcaacag ggactgagtg gggcagaggc 1860

ccagaagtgc cagcctgggg agccgtttgg gggcagcccc ttctgcccac cccatccttc 1920

ttcctctcca gagatgccag gggggcgtgt atgctctgcc ccttccctca gacaggggct 1980

gggtggggag gctctttagg ctcaggagaa gcattttaaa gaaaccccca ccctgccgcc 2040

cgcattataa acacaggaga ataatcaata gaataaaagt gaccgactgt caaaaaaaaa 2100

aaaaaa 2106

<210> 2
<211> 1292
<212> DNA
<213> Homo sapiens

<400> 2
gttaactgca tttgcagcta gaagttaggc tctgattcac tgttttgtat tttctaaaag 60

ggttatatgt aatttgaaag atagacctgc caagacgtga gatctgtgtt ctccttggtt 120

agagctaaca tttttggtga ggaaagcact gcaggagcag gctggcacag agaagaggac 180

atgcggaagg agctccagct ctccctgtca gtcaccttgc tgcttgtctg tggcttcctc 240

taccagttca ccctgaagtc cagctgcctc ttctgtttgc cttctttcaa gtcccaccag 300

gggctggaag ccctcctgag ccacagacgt ggcattgtgt ttctagagac ctcagagaga 360

atggagccac cccatttggt ctcctgttcc gtagagtctg ctgccaagat ttatcctgag 420

tggcctgtgg tgttctttat gaagggtctt actgattcca caccgatgcc ctcaaactcc 480

acatacccag ctttttcctt cctgtcagca atagacaacg ttttcctctt ccctttggat 540

atgaaaaggc tgcttgaaga cacaccattg ttttcatggt acaatcaaat caacgccagc 600

gcagagagaa actggctcca catcagctcg gatgcatccc gcctggccat catctggaaa 660

tacggtggca tctacatgga caccgatgtc atctccatca ggcccatccc tgaggagaac 720

tttttggctg cgcaggcttc tcggtactct agtaatggaa tatttgggtt cctcccccac 780

cacccctttt tgtgggaatg catggaaaac tttgttgaac actataattc agccatttgg 840

ggcaaccaag gccctgagtt gatgacaagg atgttgaggg tatggtgtaa acttgaagac 900

ttccaggagg tgagcgacct caggtgtctg aacatatcct tcttacaccc ccaaagattt 960

taccccatct cctatcgaga gtggaggcgc tactatgaag tgtgggatac agagccaagc 1020

ttcaatgtct cttatgccct gcatttgtgg aaccacatga accaggaggg gcgggctgtg 1080

attagaggaa gcaacacact ggtggaaaat ctctatcgca agcactgtcc caggacttac 1140

agggacctga ttaaaggccc agaggggtca gtgactgggg agctgggtcc aggtaacaaa 1200

taaagctaac actcgtttgc tgctgctgca gtgtggaaat ggaaaaaaaa aaaaaaaaaa 1260

aaaaaaaaaa aaaaaaaaaa aaaaaaaaaa aa 1292

<210> 3
<211> 2963
<212> DNA
<213> Homo sapiens

<400> 3
cgcttcgctg cctggcggcc tttccccgag ccgagcccgc ttcccgaggc cgccagtcgt 60

cgcctgcccg cgcctgcgca gcgtcccggg ccgagagggc cacggcggcg gccatggcgc 120

accgctgttt gcggctgtgg ggccggggcg gctgctggcc ccgcggccta cagcagctcc 180

tcgtgcctgg cggcgtgggc ccgggcgagc agccctgcct ccggacgctt taccgatttg 240

ttacaactca agcaagggcc agcagaaatt ctcttttgac agatataatt gctgcttatc 300

aaagattctg ttctcgaccc ccaaaaggat ttggaaaata ctttcctaat ggaaaaaatg 360

gaaaaaaagc tagtgaacct aaagaagtta tgggagagaa aaaagaatca aagccagctg 420

ctaccacacg ctcttctgga ggaggaggtg gtggcggtgg aaaacgaggt ggcaagaaag 480

atgattctca ctggtggtcc aggtttcaga agggtgacat tccatgggac gacaaggatt 540

tcaggatgtt cttcctctgg actgctctgt tctggggtgg agtcatgttt tacttgctgc 600

tcaagagatc cgggagagaa atcacttgga aggactttgt caataactat ctttcaaaag 660

gagtagtaga cagattggaa gtcgtcaaca agcgttttgt tcgagtgacc tttacaccag 720

gaaaaactcc tgttgatggg caatacgttt ggtttaatat tggcagtgtg gacacctttg 780

aacggaatct ggaaacttta cagcaggaat tgggcataga aggagaaaat cgggtgcctg 840

ttgtctacat tgctgaaagt gatggctctt ttctgctgag catgctgcct acggtgctca 900

tcatcgcctt cttgctctac accatcagaa gagggcctgc tggcattggc cggacaggcc 960

gagggatggg cggactcttc agtgtcggag aaaccactgc caaggtctta aaggatgaaa 1020

ttgatgtgaa gttcaaagat gtggctggct gtgaggaggc caagctagag atcatggaat 1080

ttgtgaattt cttgaaaaac ccaaagcagt atcaagacct aggagcaaaa atcccaaagg 1140

gtgccattct cactggtcct ccaggcactg ggaagacgct gctagctaag gccacagccg 1200

gagaagccaa tgtccccttc atcaccgtta gtggatctga gtttttggag atgttcgttg 1260

gtgtgggccc tgctagagtc cgagacttat ttgcccttgc tcggaagaat gccccttgca 1320

tcctcttcat cgatgaaatc gatgcggtgg gaaggaagag aggaagaggc aactttggag 1380

ggcagagtga gcaggagaac acactcaacc agctgctggt ggagatggat ggttttaata 1440

caacaacaaa tgtcgtcatt ttggccggca ccaatcgacc agatatcctg gaccccgcgc 1500

tacttaggcc ggggcgtttc gacaggcaga tctttattgg accaccagac ataaaaggaa 1560

gagcttctat tttcaaagtt catctccgac cgctaaaact ggacagtacc ctggagaagg 1620

ataaattggc aagaaaactg gcatctttaa ctccagggtt ttcaggtgct gatgttgcta 1680

atgtctgtaa tgaagctgcg ttgattgctg caaggcatct gtcagattcc ataaatcaga 1740
aacactttga acaggcaatt gagcgagtga ttggtggctt agagaagaaa acgcaggttc 1800
tgcagcctga ggagaagaag actgtggcat accacgaagc aggccatgcg gttgccggct 1860
ggtatctgga gcacgcagac ccgcttttaa aggtatccat catcccacgt ggcaaaggac 1920
taggttatgc tcagtattta ccaaaagaac aatacctcta taccaaagag cagctcttgg 1980
ataggatgtg tatgacttta ggtggtcgag cctctgaaga aatcttcttt ggaagaatta 2040
caactggtgc tcaagatgac ttgagaaaag taactcagag tgcatatgcc caaattgttc 2100
agtttggcat gaatgaaaag gttgggcaaa tctcctttga cctcccacgt cagggggaca 2160
tggtattgga gaaaccttac agtgaagcca ctgcaagatt gatagatgat gaagtacgaa 2220
tacttattaa tgatgcttat aaaagaacag tagctcttct cacagaaaag aaagctgacg 2280
tggagaaggt tgctcttctg ttgttagaaa aagaagtatt agataagaat gatatggttg 2340
aacttttggg ccccagacca tttgcggaaa aatctaccta tgaagaattt gtggaaggca 2400
ctggcagctt ggatgaggac acctcacttc cagaaggcct taaggactgg aacaaggagc 2460
gggaaaagga gaaagaggag cccccgggtg agaaagttgc caactagagg cccagaggga 2520
ggccatctca gtctctccac tgtggtttca gctggtgcat tatttcagct gtggctttca 2580
gaagaatggg aatgctgcgc tgattttagc cagccactgg cccagctgaa atgatgggga 2640
aaggagtcct tagtcctttc agcctcagag gtcacagtgg gtggcaggtg actttccgga 2700
ggccttgagg gaaatgcaca ctgtcccata gcctcattgg gctcccagac gtgctggaaa 2760
ggttgagccc agagtggccg aggctggacc ctgtggcacc aagtggggtc ggctgaccgt 2820
gtggcaggga tcgttgcact ggactcttgg cgtgtgggaa gggatgcttt ctctttgtcg 2880
cccactcttc attcctgttt ctcctcagtt cccctgtgca gatgggctgt gaaattaaat 2940
tggagtcttg ataagaacat ttt 2963

<210> 4
<211> 1982
<212> DNA
<213> Homo sapiens

<400> 4
gcagaggagg agcgcacgcc ggcctcgaag aacttctgct tgggtggctg aactctgatc 60
ttgacctaga gtcatggcca tggcaaccaa aggaggtact gtcaaagctg cttcaggatt 120
caatgccatg gaagatgccc agaccctgag gaaggccatg aaagggctcg gcaccgatga 180
agacgccatt attagcgtcc ttgcctaccg caacaccgcc cagcgccagg agatcaggac 240
agcctacaag agcaccatcg gcagggactt gatagacgac ctgaagtcag aactgagtgg 300
caacttcgag caggtgattg tggggatgat gacgcccacg gtgctgtatg acgtgcaaga 360
gctgcgaagg gccatgaagg gagccggcac tgatgagggc tgcctaattg agatcctggc 420
ctcccggacc cctgaggaga tccggcgcat aagccaaacc taccagcagc aatatggacg 480

gagccttgaa gatgacattc gctctgacac atcgttcatg ttccagcgag tgctggtgtc 540

tctgtcagct ggtgggaggg atgaaggaaa ttatctggac gatgctctcg tgagacagga 600

tgcccaggac ctgtatgagg ctggagagaa gaaatggggg acagatgagg tgaaatttct 660

aactgttctc tgttcccgga accgaaatca cctgttgcat gtgtttgatg aatacaaaag 720

gatatcacag aaggatattg aacagagtat taaatctgaa acatctggta gctttgaaga 780

tgctctgctg gctatagtaa agtgcatgag gaacaaatct gcatattttg ctgaaaagct 840

ctataaatcg atgaagggct tgggcaccga tgataacacc ctcatcagag tgatggtttc 900

tcgagcagaa attgacatgt tggatatccg ggcacacttc aagagactct atggaaagtc 960

tctgtactcg ttcatcaagg gtgacacatc tggagactac aggaaagtac tgcttgttct 1020

ctgtggagga gatgattaaa ataaaaatcc cagaaggaca ggaggattct caacactttg 1080

aattttttta acttcatttt tctacactgc tattatcatt atctcagaat gcttatttcc 1140

aattaaaacg cctacagctg cctcctagaa tatagactgt ctgtattatt attcacctat 1200

aattagtcat tatgatgctt taaagctgta cttgcatttc aaagcttata agatataaat 1260

ggagatttta aagtagaaat aaatatgtat tccatgtttt taaaagatta ctttctactt 1320

tgtgtttcac agacattgaa tatattaaat tattccatat tttcttttca gtgaaaaatt 1380

ttttaaatgg aagactgttc taaaatcact tttttcccta atccaatttt tagagtggct 1440

agtagtttct tcatttgaaa ttgtaagcat ccggtcagta agaatgccca tccagttttc 1500

tatatttcat agtcaaagcc ttgaaagcat ctacaaatct ctttttttag gttttgtcca 1560

tagcatcagt tgatccttac taagtttttc atgggagact tccttcatca catcttatgt 1620

tgaaatcact ttctgtagtc aaagtatacc aaaaccaatt tatctgaact aaattctaaa 1680

gtatggttat acaaaccata tacatctggt taccaaacat aaatgctgaa cattccatat 1740

tattatagtt aatgtcttaa tccagcttgc aagtgaatgg aaaaaaaaat aagcttcaaa 1800

ctaggtattc tgggaatgat gtaatgctct gaatttagta tgatataaag aaaacttttt 1860

tgtgctaaaa atacttttta aaatcaattt tgttgattgt agtaatttct atttgcactg 1920

tgcctttcaa ctccagaaac attctaagat gtacttggat ttaattaaaa agttcacttt 1980

gt 1982

<210> 5
<211> 2442
<212> DNA
<213> Homo sapiens

<400> 5
aggaggcgga ctctgtgagg aaacaagaag agaggcccaa gatggagacg gcggcggctg 60

tagcggcgtg acaggagccc catggcacct gcccagcccc acctcagccc atcttgacaa 120

aatctaaggc tccatggagc caccacgggg cccccctgcc aatggggccg agccatcccg 180

ggcagtgggc accgtcaaag tatacctgcc caacaagcaa cgcacggtgg tgactgtccg 240

ggatggcatg agtgtctacg actctctaga caaggccctg aaggtgcggg gtctaaatca 300

ggactgctgt gtggtctacc gactcatcaa gggacgaaag acggtcactg cctgggacac 360
agccattgct cccctggatg gcgaggagct cattgtcgag gtccttgaag atgtcccgct 420
gaccatgcac aattttgtac ggaagacctt cttcagcctg gcgttctgtg acttctgcct 480
taagtttctg ttccatggct tccgttgcca aacctgtggc tacaagttcc accagcattg 540
ttcctccaag gtccccacag tctgtgttga catgagtacc aaccgccaac agttctacca 600
cagtgtccag gatttgtccg gaggctccag acagcatgag gctccctcga accgccccct 660
gaatgagttg ctaacccccc agggtcccag cccccgcacc cagcactgtg acccggagca 720
cttccccttc cctgccccag ccaatgcccc cctacagcgc atccgctcca cgtccactcc 780
caacgtccat atggtcagca ccacggcccc catggactcc aacctcatcc agctcactgg 840
ccagagtttc agcactgatg ctgccggtag tagaggaggt agtgatggaa cccccccggg 900
gagccccagc ccagccagcg tgtcctcggg gaggaagtcc ccacattcca agtcaccagc 960
agagcagcgc gagcggaagt ccttggccga tgacaagaag aaagtgaaga acctggggta 1020
ccgggactca ggctattact gggaggtacc acccagtgag gtgcagctgc tgaagaggat 1080
cgggacgggc tcgtttggca ccgtgtttcg agggcggtgg catggcgatg tggccgtgaa 1140
ggtgctcaag gtgtcccagc ccacagctga gcaggcccag gctttcaaga atgagatgca 1200
ggtgctcagg aagacgcgac atgtcaacat cttgctgttt atgggcttca tgacccggcc 1260
gggatttgcc atcatcacac agtggtgtga gggctccagc ctctaccatc acctgcatgt 1320
ggccgacaca cgcttcgaca tggtccagct catcgacgtg gcccggcaga ctgcccaggg 1380
catggactac ctccatgcca agaacatcat ccaccgagat ctcaagtcta acaacatctt 1440
cctacatgag gggctcacgg tgaagatcgg tgactttggc ttggccacag tgaagactcg 1500
atggagcggg gcccagccct tggagcagcc ctcaggatct gtgctgtgga tggcagctga 1560
ggtgatccgt atgcaggacc cgaaccccta cagcttccag tcagacgtct atgcctacgg 1620
ggttgtgctc tacgagctta tgactggctc actgccttac agccacattg gctgccgtga 1680
ccagattatc tttatggtgg gccgtggcta tctgtccccg gacctcagca aaatctccag 1740
caactgcccc aaggccatgc ggcgcctgct gtctgactgc ctcaagttcc agcgggagga 1800
gcggcccctc ttcccccaga tcctggccac aattgagctg ctgcaacggt cactccccaa 1860
gattgagcgg agtgcctcgg aaccctcctt gcaccgcacc caggccgatg agttgcctgc 1920
ctgcctactc agcgcagccc gccttgtgcc ttaggccccg cccaagccac cagggagcca 1980
atctcagccc tccacgccaa ggagccttgc ccaccagcca atcaatgttc gtctctgccc 2040
tgatgctgcc tcaggatccc ccattcccca ccctgggaga tgagggggtc cccatgtgct 2100
tttccagttc ttctggaatt gggggacccc cgccaaagac tgagccccct gtctcctcca 2160
tcatttggtt tcctcttggc tttggggata cttctaaatt ttgggagctc ctccatctcc 2220
aatggctggg atttgtggca gggattccac tcagaacctc tctggaattt gtgcctgatg 2280
tgccttccac tggattttgg ggttcccagc accccatgtg gatttggggg ggtccctttt 2340

gtgtctcccc cgccattcaa ggactcctct ctttcttcac caagaagcac agaattctgc 2400

tgggcctttg cttgtttaaa aaaaaaaaaa aaaaaaaaaa aa 2442

<210> 6
<211> 987
<212> DNA
<213> Homo sapiens

<400> 6
aatataagtg gaggcgtcgc gctggcgggc attcctgaag ctgacagcat tcgggccgag 60

atgtctcgct ccgtggcctt agctgtgctc gcgctactct ctctttctgg cctggaggct 120

atccagcgta ctccaaagat tcaggtttac tcacgtcatc cagcagagaa tggaaagtca 180

aatttcctga attgctatgt gtctgggttt catccatccg acattgaagt tgacttactg 240

aagaatggag agagaattga aaaagtggag cattcagact tgtctttcag caaggactgg 300

tctttctatc tcttgtacta cactgaattc acccccactg aaaaagatga gtatgcctgc 360

cgtgtgaacc atgtgacttt gtcacagccc aagatagtta agtgggatcg agacatgtaa 420

gcagcatcat ggaggtttga agatgccgca tttggattgg atgaattcca aattctgctt 480

gcttgctttt taatattgat atgcttatac acttacactt tatgcacaaa atgtagggtt 540

ataataatgt taacatggac atgatcttct ttataattct actttgagtg ctgtctccat 600

gtttgatgta tctgagcagg ttgctccaca ggtagctcta ggagggctgg caacttagag 660

gtggggagca gagaattctc ttatccaaca tcaacatctt ggtcagattt gaactcttca 720

atctcttgca ctcaaagctt gttaagatag ttaagcgtgc ataagttaac ttccaattta 780

catactctgc ttagaatttg ggggaaaatt tagaaatata attgacagga ttattggaaa 840

tttgttataa tgaatgaaac attttgtcat ataagattca tatttacttc ttatacattt 900

gataaagtaa ggcatggttg tggttaatct ggtttatttt tgttccacaa gttaaataaa 960

tcataaaact tgatgtgtta tctctta 987

<210> 7
<211> 2022
<212> DNA
<213> Homo sapiens

<400> 7
aggcggaggg ggcggggctt cggccggcta agccgcggcc acccgcggtc tgagggaccg 60

gaggagcggg gccgggagct gcgaggagcc ctccagacgt cgccgagcgc gagggcgggc 120

gtgctcggag tggtcgctcg tcagccgccg cccctcagtc tccgcacttg caggtcccct 180

ccctctccgc cgggacgcgg gagagcccgg ctcgcggcgg gggcggccaa tgcgaaactg 240

gctggtgctg ctgtgcccgt gtgtgctcgg ggccgcgctg cacctctggc tgcggctgcg 300

ctccccgccg cccgcctgcg cctccggggc cggccctgca gatcagttgg ccttatttcc 360

tcagtggaaa tctactcact atgatgtggt agttggcgtg ttgtcagctc gcaataacca 420

tgaacttcga aacgtgataa gaagcacctg gatgagacat ttgctacagc atcccacatt 480

aagtcaacgt gtgcttgtga agttcataat aggtgctcat ggctgtgaag tgcctgtgga 540
agacagggaa gatccttatt cctgtaaact actcaacatc acaaatccag ttttgaatca 600
ggaaattgaa gcgttcagtc tgtccgaaga cacttcatcg gggctgcctg aggatcgagt 660
tgtcagcgtg agtttccgag ttctctaccc catcgttatt accagtcttg gagtgttcta 720
cgatgccaat gatgtgggtt tccagaggaa catcactgtc aaactttatc aggcagaaca 780
agaggaggcc ctcttcattg ctcgcttcag tcctccaagc tgtggtgtgc aggtgaacaa 840
gctgtggtac aagcccgtgg aacaattcat cttaccagag agctttgaag gtacaatcgt 900
gtgggagagc caagacctcc acggccttgt gtcaagaaat ctccacaaag tgacagtgaa 960
tgatggaggg ggagttctca gagtcattac agctggggag ggtgcattgc ctcatgaatt 1020
cttggaaggt gtggagggag ttgcaggtgg ttttatatat actattcagg aaggtgatgc 1080
tctcttacac aaccttcatt ctcgccctca aagacttatt gatcatataa ggaatctcca 1140
tgaggaagat gccttactga aggaggaaag cagcatctat gatgatattg tttttgtgga 1200
tgttgtcgac acttatcgta atgttcctgc aaaattattg aacttctata gatggactgt 1260
ggaaacaacg agcttcaatt tgttgctgaa gacagatgat gactgttaca tagacctcga 1320
agctgtattt aataggattg tccaaaagaa tctggatggg cctaattttt ggtggggaaa 1380
tttcagactg aattgggcag ttgaccgaac cggaaagtgg caggagttgg agtacccgag 1440
ccccgcttac cctgcctttg catgtgggtc aggatatgtg atctccaagg acatcgtcaa 1500
gtggctggca agcaactcgg ggaggttaaa gacctatcag ggtgaagatg taagcatggg 1560
catctggatg gctgccatag gacctaaaag ataccaggac agtctgtggc tgtgtgagaa 1620
gacctgtgag acaggaatgc tgtcttctcc tcagtattct ccgtgggaac tgacggaact 1680
gtggaaactg aaggaacggt gcggtgatcc ttgtcgatgt caagcaagat aacagggact 1740
tgaattagca gagtctaaaa tcagggcagg caaacgataa tctgagtgca agtctgagga 1800
gtcccagggt ttagcagtag actgtatggt ctttcaagag agttccagac tggcactttc 1860
acccagaacc aatgcggtgt ttcttaatgt ttgcacaaat ttccttaaaa atcaacttgt 1920
actgtagcat aagaaaagtt tttatttatc tattggaaga ttatcagaaa aataccaagt 1980
tattttataa aagaaaaaat atttctaaaa aaaaaaaaaa aa 2022

<210> 8
<211> 2168
<212> DNA
<213> Homo sapiens

<400> 8
ggctacgcag cttgctcctg gcacgggcac cttgaatctc ctcctcacac agatggagac 60
catgcttgat ttcctgaact tgtagtaaga agaaggaaaa cacagcacgc tggagccaac 120
agagttaaga ggaagattta tgagtcatgg aaccctccat cagatttgga agaaagtaga 180
atgagcgcag aggtgacaga cagccactga ggcccatgga caatctccac ctcacgcttc 240

tctatcaaac ttgaagattt attagtaata tgctgccttt ggaagatgaa aacaaactag 300
tgccaaggag gcgtattctt caatatttgg aatagacgtg ttctcaagac aatggcttca 360
aaggtctcct gtttgtatgt tttgacagtt gtgtgctggg ccagcgctct ctggtacttg 420
agtataactc gccctacttc ttcttacact ggctccaaac cattcagcca cctaacagtt 480
gccaggaaaa acttcacctt tggcaacata agaactcgac ctatcaaccc acattctttt 540
gaatttctta tcaacgagcc caataaatgt gagaaaaaca ttccttttct tgttatcctc 600
atcagcacca ctcacaagga atttgatgcc cgtcaggcaa tcagagagac gtggggggat 660
gagaacaact ttaaggggat caagatagcc accctgttcc tcctgggcaa gaatgctgat 720
cctgttctca atcagatggt ggagcaagag agccaaatct tccatgatat catcgtggag 780
gactttattg actcctacca taaccttacc ctcaaaacat taatggggat gagatgggtg 840
gccacttttt gttcaaaagc caagtatgtc atgaaaacag acagcgacat ttttgtaaac 900
atggacaatc ttatttataa attactgaaa ccctccacca agccacgaag aaggtatttt 960
actggctatg tcattaatgg aggaccgatt cgggatgtcc gcagtaaatg gtatatgccc 1020
agggatttgt acccagacag taactaccca cctttctgtt cggggactgg ctacatcttt 1080
tcagccgatg tagctgaact catttacaag acctcactcc acacaaggct gcttcacctt 1140
gaagacgtat atgtgggact gtgtcttcga aagctgggca tacatccttt ccagaacagt 1200
ggcttcaatc actggaaaat ggcctacagt ttgtgtaggt atcgccgagt tatcactgtg 1260
catcagatct ctccagaaga aatgcacaga atctggaatg acatgtcaag caagaaacat 1320
ctcagatgtt aggattttta ccaatgtaaa tatgtttctt ttcttttttt aagaaatggg 1380
acctaaggtg ttggtatttt ccaggtgtcg ggggaaatga actggtgaag gggttttgta 1440
aagtttttgc ttcctgctat aagttctttt cttggattac caatttatga atgttagact 1500
ctggtcatag aaacaataaa tgagttagaa gggccagatt tcattctcag tcccagagca 1560
ttgctattta tctcaaaaag tgacttccaa acaactctta ggattgacgt accgtgcatc 1620
tgagataaaa atttggttct gggaaactga aactcacagt aatgtgtcat atcatccctg 1680
caaaaattaa tacacaaata gaaaccattt tcaaaagcaa ttcagaaagg atgcacagtc 1740
aggaagacac actggatgtg attattaata tcgtgtgtgt tgttacatta tatttttaca 1800
tatattccca tgtaatgtgt acagtctttg cagttccacc aagaaatgaa cttggtacct 1860
gcagagtggc tgcagttaaa tagatgggag tttaaatttg agaatcaaac attctatgtg 1920
tttggaagac aactctgctt gctcatccaa ggattaaatc tggtcagcag gtggaatgtg 1980
tataaaatgc tacttaacaa agtaaacaaa agattttttt tttctttttt tttctttctt 2040
ttttgttttg ctctttcaga acaaacatta aatggtgcct ccaaggaaac tttgccaaat 2100
ataatctcac ctgcttcctt ccagacagtg tcgctaagtg catttcacag tttttggatc 2160
tggcaggc 2168

<210> 9

<211>  3215
<212>  DNA
<213>  Homo sapiens

<400>  9
cctgtgcagc agctgaggaa ccgtggattt catattatag actaaaaccc cattaaaact      60

gctcaaaatc cttcctgcag ctgccaggca acaacgaaag aagagaggta aatcctattc     120

ttttccaata caactgaagc actacatttt agctctggct gctttacatt gcagctcagt     180

gttattagta gaaatatgga tactgagacg agaacacagc actgcattgt ccagccagga     240

aaaatagcag atgtaaaaag cttcaatgca tcaactgtcg ggaagagtca acagtgctac     300

aagcagaacg ggcaactaca gctcttttgt ttaacgaaag agagaatatg aaagaaaggg     360

aaaatttcag aagactagga cccatatgaa caaggagggt aactcgaaga caagcagaca     420

gatggacact ttggatactg tgaaaagcaa tcgcaggagg cagactgttg ggggatgtgc     480

gcatgttcga tagcatcttt tttgctgaag tgatggcgtg ccaaaagtat tttcagtggg     540

cataatcctc ttcacataaa tggcctgacc aaggagaatg actacaagag agacaatgtg     600

actgaattag aaaatgattg ccaaagaata gtattaagga gaagaaaaca tttttgtcac     660

caatctctca tataccacta ctggatattt acaacatgct tcagtggagg agaagacact     720

gctgctttgc aaagatgacc tggaatgcca aaaggtctct gttccgcact catcttattg     780

gagtactttc tctagtgttt ctttttgcta tgtttttgtt tttcaatcat catgactggc     840

tgccaggcag agctggattc aaagaaaacc ctgtgacata cactttccga ggatttcggt     900

caacaaaaag tgagacaaac cacagctccc ttcggaacat ttggaaagaa acagtccctc     960

aaaccctgag gcctcaaaca gcaactaact ctaataacac agacctgtca ccacaaggag    1020

ttacaggcct ggagaataca cttagtgcca atggaagtat ttacaatgaa aaaggtactg    1080

gacatccaaa ttcttaccat ttcaaatata ttattaatga gcctgaaaaa tgccaagaga    1140

aaagtccttt tttaatacta ctaatagctg cagagcctgg acaaatagaa gctagaagag    1200

ctattcggca aacttggggc aatgaaagtc tagcacctgg tattcaaatc acaagaatat    1260

ttttgttggg cttaagtatt aagctaaatg gctaccttca acgtgcaata ctggaagaaa    1320

gcagacaata tcatgatata attcaacagg aatacttaga tacgtactat aatttgacca    1380

ttaaaacact aatgggcatg aactgggttg caacatactg tccacatatt ccatatgtta    1440

tgaaaactga cagtgacatg tttgtcaaca ctgaatattt aatcaataag ttactgaagc    1500

cagatctgcc tcccagacat aactatttca ctggttacct aatgcgagga tatgcaccca    1560

atcgaaacaa agatagcaag tggtacatgc caccagacct ctacccaagt gagcgttatc    1620

ctgtcttctg ttctggaact ggttatgttt tttctggaga tctggcagaa aagattttta    1680

aagtttcttt aggtatccgc cgtttgcact tggaagatgt atatgtaggg atctgtcttg    1740

ccaagttgag aattgatcct gtaccccctc ccaatgagtt tgtgttcaat cactggcgag    1800

tctcttattc gagctgtaaa tacagccacc taattacctc tcatcagttc cagcctagtg    1860

aactgataaa atactggaac catttacaac aaaataagca caatgcctgt gccaacgcag    1920

caaaagaaaa ggcaggcagg tatcgccacc gtaaactaca ttagaaaaga caattttttt 1980

tcaatgtgca atttgtaaat attgctaaaa gcatgtatag ttaggaactg attacatccg 2040

taggacaagt tttagttaaa actcatcaca taaagaaatt caagaagtat ttttttaatt 2100

tctgaagaag ttaattctta aaactataac attatataac aaaaaaggtt tcccaaaaca 2160

atctatttaa aaaactgtat aaggagattc tgtgtattaa catgcaataa caagcatgca 2220

taaatcaatg gttcaagtct tctgttaggg ggccaataaa atgtatctgc atatgttttc 2280

cacataaatt ttaattcaag aaatgacagt caaaagatcc ttcattttag attaagcttt 2340

tcattttaat atataattta atgtaaataa aacatcacta tcaattttaa ggaaactttt 2400

taattgtgca aaggataaat tttttgacct attttagggt tctaaatgca ataagattta 2460

gttgagttat tccacaaaca cattataaag ttcagatgtt tcatcaatgc agttctcacg 2520

aaagtattta ctttttaaaa ataactgaga tattatttta aatttctttt attaatactt 2580

tcttttatta atatatgggg gaaaattatt ttgacatgac gtggtaaaat gtgaaaaact 2640

aatgtgtctc aggctcaagt ttttatagtt attaaatgtt tcaaaataga caagttttgt 2700

ttcctcattg atgttaagaa ccaaactcct atttcaatga gttattggat tagaccaatt 2760

actgcactct taaacagcac caccatttaa tttcatgtaa tatctaactt cgaatatatc 2820

tgtaaaggat aatcgaagca aaagtaatca cttaaaggca caaataggat gtactgttga 2880

aaaagataaa gagtgcaggt gcagtttcat tcaacacatt tttaagatgc atgtctgcca 2940

aaatgcaaca tacgggaagt ttatttcctg acagcaggtg tacacatgcc aacacttaat 3000

cattttatgg cacctatttc tttcttggag tgccaagttt gcaaacctgc agtttttaat 3060

ttggtagatg acaaatattc tgaatcacca attaaaaacc tttttgggag ggatggggaa 3120

aactacaaac gtttgacaaa cacaattcta ggatgaacaa tgtatacaat gcacttttat 3180

gaagttttta aaaataaagg aaaacaaaaa acttt 3215

<210> 10
<211> 1704
<212> DNA
<213> Homo sapiens

<400> 10
gaggcggggg aggggccgtg agtgccgcag tcggccagcc atggagcgga gcttgctggc 60

ggcgaggccg cggcgacaag gtagccaccc ccgcagcatg cctcgaccgc ggtccgcagc 120

tgcaccgcct ctccccgccc cccagggtgc gctggtcccg gtcgcgcgct cagacctccg 180

catcccgggc gtggtcggtt aagtccccgg ccgtgaccca ggcccgggga gctagtctcc 240

gcccttcgct cttacggatc ccctcggagt acgccgcacc atgcagctca ggctcttccg 300

gcgcctcctt ctcgccgctt tgctgctggt gatcgtctgg accctcttcg ggccttcggg 360

gttgggggag gagctgctga gcctctcact agcctccctg ctcccagccc ccgcctcacc 420

ggggccgccc ctggccctgc cccgcctctt gatccccaac caggaagctt gcagtggtcc 480

cggggcccct cccttcctgc tcatcctggt gtgcacggct ccggagaacc tgaaccagag 540
aaacgccatt cgggcttcgt ggggcgggct gcgcgaggcc cgggggctca gggtacagac 600
gctattcttg ctgggagagc cgaacgcaca gcaccccgtg tggggttccc aggggagtga 660
cctggcctcg gagtcagcag cccaggggga tatcttgcag gccgccttcc aggactccta 720
ccgcaacctc accctaaaga ccctcagcgg gctgaactgg gctgagaaac actgccccat 780
ggcccgatac gtcctcaaga cggacgatga tgtgtatgtc aacgtccctg aactggtatc 840
agagctggtc ttgcgagggg gccgttgggg gcaatgggag agaagcacgg aaccccagag 900
agaggctgag caggaaggag gccaggtttt gcacagcgag gaagtgcctc ttctgtactt 960
gggccgggtg cactggcgcg tgaacccctc tcggacaccg gggggcaggc accgcgtatc 1020
agaggagcag tggcctcaca cctggggccc ctttccaccc tatgcctcag gcacggggta 1080
tgtgctgtca gcgtctgctg tgcagctcat tctcaaggtg gccagccggg caccccttct 1140
cccattagag gatgtctttg tgggggtaag tgcccgacga ggaggcctcg ccccaacaca 1200
gtgtgtcaag ctggctggtg ccacccacta cccgctagac cggtgctgct atgggaaatt 1260
cctgctgacg tcccacaggc tggacccctg gaagatgcag gaagcctgga agctggtggg 1320
tggctctgac ggggaaagga ctgcgccctt ttgctcctgg ttccagggag tcctgggcat 1380
cctgcggtgt cgagcaatag cctggcttca gagctgagag tgcctggggc cacaggaaag 1440
gcaggaacag gaccttctct ctcccaggcc caacgcaggg gccctcactg gctgcagctg 1500
atctgtttcc ttataccaga tcctcagtct cactaaagac agcgatatgg agacacccca 1560
ggggcctggc ccgccagccc aaaagatggt catcgggaag agaaaaagaa aaaaatgctg 1620
cagttgttct ctcaagctag ggcagaagag gggtgtcaag ctcctcaata aacttgtctc 1680
cacttcaaaa aaaaaaaaaa aaaa 1704

<210> 11
<211> 2793
<212> DNA
<213> Homo sapiens

<400> 11
ggcctgggcc gccggcccgg cgcgggcgcc atgaagctgc tgcggcgggc gtggcggcgg 60
cgggcggcgc taggcctggg cacgctggcg ctgtgcgggg cggcgctgct ctacctggcg 120
cgctgcgcgg ccgagcccgg ggaccccagg gcgatgtcgg gccgcagccc gcctcccccc 180
gcgcccgcgc gcgccgccgc cttcctggca gtgctggtgg ccagcgcgcc ccgcgccgcc 240
gagcgccgca gcgtgatccg cagcacgtgg cttgcgcggc gcggggcccc gggcgacgtg 300
tgggcgcgct ttgccgtggg cacggccggc ctgggcgccg aggagcggcg cgccctggag 360
cgggagcagg cgcggcacgg ggacctgctg ctgctgcccg cgctgcgcga cgcctacgaa 420
aacctcacgg ccaaggtgct ggccatgctg gcctggctgg acgagcacgt ggccttcgag 480
ttcgtgctca aggcggacga cgactccttc gcgcggctgg acgcgctgct ggccgagctg 540
cgcgcccgcg agcccgcgcg ccgccgccgc ctctactggg gcttcttctc gggccgcggc 600

cgcgtcaagc cgggggggcg ctggcgcgag gccgcctggc aactctgcga ctactacctg 660
ccctacgcgc tgggcggcgg ctacgtgctc tcggccgacc tggtgcacta cctgcgcctc 720
agccgcgact acctgcgcgc ctggcacagc gaggacgtgt ctctgggcgc ctggctggcg 780
ccggtggacg tccagcggga gcacgacccg cgcttcgaca ccgaataccg gtcccgcggc 840
tgcagcaacc agtacctggt gacgcacaag cagagcctgg aggacatgct ggagaagcac 900
gcgacgctgg cgcgcgaggg ccgcctgtgc aagcgcgagg tgcagctgcg cctgtcctac 960
gtgtacgact ggtccgcgcc gccctcgcag tgctgccaga gaagggaggg catcccctga 1020
gccgccgcgg cccggccctc cgggacacct gcttcacccg gcggcgcctt ggggcaggtg 1080
ccgagcgggc gcactacgcc cgggccccaa ggcccccgtc ccgcagccac gcttgtggtc 1140
gctgcgtccc ggtctgcgtt tgggagaccc ctgggggttg ccggggcagc gcgccgtgtc 1200
caggtggagg tgcccgttcc tggacctcag cgagcctgag ccgggcccgg ccgcacgctg 1260
acccccgtgc tgtccccgac cggctcacgg ggctgggctc cgatcttccg tgtctcttat 1320
cagtggcgtt tctcacgtct gcgtctcaga tctaacgtgg tttcacatca atccgctttc 1380
atgggatttt ggtctctgtc cagtgacttc gtggtaaatg taactcagtg tttgcttgcg 1440
acttatttat aaatattgta agtttgtgtc gatgagtgta agttggcagt gcgcacgtct 1500
cggttttttt acatgattta aggaaagact tttatgtcag aacttggtgc ctgtaccgtc 1560
aaccccgctg ctgcccgtgt ttaaacgcag gagaacttta aaactggcca tctatctttt 1620
cagtgtacaa gtcactgaac ccattgtttc tttctgaaga gactttcctt tcaaggcttc 1680
ccatgggtcc gcgccacaca gggccggtgc tgctttattt cagactctgc cccaggttcc 1740
aggaatccga accccggagt gctgacgcgg ttccccaact tccgccttaa gaaaacagga 1800
ccagccggca ccaggcccgt ctctcacgta ctttaacaca tccttgaaag cccctcgttt 1860
aatgagaaaa gcgaacactg cggtccttgc caaagtaaaa tgaagctgcc ccaggacaag 1920
gggttaccat gagctccctg gagtccgacg cgggttttct ctctggggga cctgggtggt 1980
ccccgctgtg gtctttgttg tcccactttg ggaccgggtc cagtctgggg tctagtctcg 2040
agcatcaggg tcaggctcgg ggcagggctg ggttaggctc cgggtcagtc ttgccatggg 2100
tttgggagca ggtttgggtt acttgcgttt gaaggcagca gtggtctcag gaggaagaaa 2160
cgggggcggg agagagtggt gatctgtggt cagtgggtca gtgacctgca cggtgattct 2220
cccacctcca aaaggtaggg gtgggactgg aggcgtccct aggtcaggcc gttgagttcg 2280
agctccgatg ggccaccttg aatccaggac tgaccgcccg tgtgtgcaca gtttgttctt 2340
ggacgaggac tcgtgaggat cgagggctgg ggaccccggt gtgagcagga tggggccctg 2400
ccctcccgtg ggagttgtgg actcgagccc aggggctgcc cgtcacagcg gtgtcccagg 2460
tccctgccat ccgattttac ctgggatgtc ttctctggag tttggaattg cttgaggaac 2520
cctgcgtgtg cttggagagg ccagagggct tgctgagaac cccatggaca gtggagagcg 2580
ggattcgaac caagggctgg actcccacac ctctggcctg cgtcgcccag ttctttgtgg 2640

ctctgaagaa ttggccgctg tggaaaagag caaatgtccg agacccccaa caggaagagt 2700

ctaaaaatcc agtttgcaac cacttctgac ctacaaaaaa atggaaattt agtgtttttc 2760

agcctaagac attaaatttc atatcagaac aaa 2793

<210> 12
<211> 2077
<212> DNA
<213> Homo sapiens

<400> 12
gcggcgggcg gggcggcgct cggggctcgg ctggcctcgg ctcgcctcgg ctgcgctcgg 60

caggctgcgg taaatccggg cttgcggccg ctggcgtagt ctgtggccgg gtggtcgttg 120

ctgcgcgccc cgagccccga gagccatgca gatgtcctac gccatccggt gcgccttcta 180

ccagctgctg ctggccgcgc tcatgctggt ggcgatgctg cagctgctct acctgtcgct 240

gctgtccgga ctgcacgggc aggaggagca agaccaatat tttgagttct ttcccccgtc 300

cccacggtcc gtggaccagg tcaaggcgca gctccgcacc gcgctggcct ctggaggcgt 360

cctggacgct agcggcgatt accgcgtcta caggggcctg ctgaagacca ccatggaccc 420

caacgatgtg atcctggcca cgcacgccag cgtggacaac ctgctgcacc tgtcgggtct 480

gctggagcgc tgggagggcc cgctgtccgt gtcggtgttc gcggccacca aggaggaggc 540

gcagctggcc acggtgctgg cctacgcgct gagcagccac tgccccgaca tgcgcgccag 600

ggtcgccatg cacctcgtgt gcccctcgcg ttacgaggca gccgtgcccg acccccggga 660

gccgggggag tttgccctgc tgcggtcctg ccaggaggtc tttgacaagc tagccagggt 720

ggcccagccc gggattaatt atgcgctggg caccaatgtc tcctacccca ataacctgct 780

gaggaatctg gctcgtgagg gggccaacta tgccctggtg atcgatgtgg acatggtgcc 840

cagcgagggg ctgtggagag gcctgcggga aatgctggat cagagcaacc agtggggagg 900

caccgcgctg gtggtgcctg ccttcgaaat ccgaagagcc cgccgcatgc ccatgaacaa 960

aaacgagctg gtgcagctct accaggttgg cgaggtgcgg cccttctatt atgggttgtg 1020

caccccctgc caggcaccca ccaactattc ccgctgggtc aacctgccgg aagagagctt 1080

gctgcggccc gcctacgtgg taccttggca ggacccctgg gagccattct acgtggcagg 1140

aggcaaggtg cccaccttcg acgagcgctt tcggcagtac ggcttcaacc gaatcagcca 1200

ggcctgcgag ctgcatgtgg cggggtttga ttttgaggtc ctgaacgaag gtttcttggt 1260

tcataagggc ttcaaagaag cgttgaagtt ccatccccaa aaggaggctg aaaatcagca 1320

caataagatc ctatatcgcc agttcaaaca ggagttgaag gccaagtacc ccaactctcc 1380

ccgacgctgc tgagcccttc cctcccctaa tctgagaagt cagcctcttg gctcctcagg 1440

ccaccattta ggcctgactg gggtaagaaa tgtcgctcca ctttacagag gtagctgtgg 1500

tgttgaaaca ctggacttgg atatggggtg ctgggatcga ttcctagctt taccactaac 1560

tagctgtgtg gccttgagta aatcccgtta cctctctgag cctcggttac cctgtctgta 1620

aaaagggagg tgagaatacc tacctcacgg aactgttggg aggctcagat gagatgctat 1680

atgtgaaaac attctgtaag cttcgtacaa atgtgaagta ttaatattat cgcagtatta 1740

ttgttgttat tattattgtt attattaaca atcttgggtg ggtagtagga gagcaaaaag 1800

tatgaatggg atggagctaa gaagtctgaa tacttaatga aatggacttt ttggaaagaa 1860

atcagatgaa ggcataaaat ttagttctta gctcttgaac agaagcctaa aattcctggt 1920

tctctcaggg cttcgccttc aagggttctg gaggagggaa gggtctgcag gttccatggg 1980

tgacagcctg agatctgtcc cttcaacggg ctgggctggg tatgtgccta ccgatgacaa 2040

tgtgtaaata aatgcgtgtt cacacccaca aaaaaaa 2077

<210> 13
<211> 2788
<212> DNA
<213> Homo sapiens

<400> 13
ggcgcagcgg cagcggcagc agcggcaaca agtgccggag gctagcagag ccaagccgga 60

gcagtccctg ccgccgacac cgccgggccg cccgtccggg gcgccgcgca tggagcgtga 120

gctgcggcgg tcgccgggct gagccgcgcg gagcggccgg gacgtggatg tggccgcgat 180

ctcccgccct tgcccccgcc ccgccgagct ggagctgctc ccggacaaga tatgagaaat 240

gagtgttgga cgtcgaagaa taaagttgtt gggtatcctg atgatggcaa atgtcttcat 300

ttattttatt atggaagtct ccaaaagcag tagccaagaa aaaaatggaa aaggggaagt 360

aataataccc aaagagaagt tctggaagat atctacccct cccgaggcat actggaaccg 420

agagcaagag aagctgaacc ggcagtacaa ccccatcctg agcatgctga ccaaccagac 480

gggggaggcg ggcaggctct ccaatataag ccatctgaac tactgcgaac ctgacctgag 540

ggtcacgtcg gtggttacgg gttttaacaa cttgccggac agatttaaag actttctgct 600

gtatttgaga tgccgcaatt attcactgct tatagatcag ccggataagt gtgcaaagaa 660

acctttcttg ttgctggcga ttaagtccct cactccacat tttgccagaa ggcaagcaat 720

ccgggaatcc tggggccaag aaagcaacgc agggaaccaa acggtggtgc gagtcttcct 780

gctgggccag acacccccag aggacaacca ccccgacctt tcagatatgc tgaaatttga 840

gagtgagaag caccaagaca ttcttatgtg gaactacaga gacactttct tcaacttgtc 900

tctgaaggaa gtgctgtttc tcaggtgggt aagtacttcc tgcccagaca ctgagtttgt 960

tttcaagggc gatgacgatg tttttgtgaa cacccatcac atcctgaatt acttgaatag 1020

tttatccaag accaaagcca aagatctctt cataggtgat gtgatccaca atgctggacc 1080

tcatcgggat aagaagctga agtactacat cccagaagtt gtttactctg gcctctaccc 1140

accctatgca gggggagggg ggttcctcta ctccggccac ctggccctga ggctgtacca 1200

tatcactgac caggtccatc tctaccccat tgatgacgtt tatactggaa tgtgccttca 1260

gaaactcggc ctcgttccag agaaacacaa aggcttcagg acatttgata tcgaggagaa 1320

aaacaaaaat aacatctgct cctatgtaga tctgatgtta gtacatagta gaaaacctca 1380

agagatgatt gatatttggt ctcagttgca gagtgctcat ttaaaatgct aaaatagata 1440

caaactcaat tttgcataga aaggtgtatt ttgaatagtt cccatgttgt gttctcacat 1500

tagagtaatt tctatattaa accatgaaaa ttgcctttat gagtgatacc catttgaggg 1560

cctctaaacc cttcaatttg gtactcacgt gaagagggaa agcggaagat ggtaattttt 1620

ttttatggat gatatggcag gatgattggt tctgatctta ccggctagtg gtcattttta 1680

aaaaacttgt accctcttat ctgaaatcct gtttctggaa tttggccatt ttaagtgatt 1740

ttgtttgccc tcttctataa tattcctact tcccataata atgactgatt tatttgtaat 1800

tcaggtattt ataaacctat tggctacaaa gactttgtta aacattatcc agtggttttc 1860

gtgaaatgga attatgttta tttttatggg atttgggtaa attttaaatt gtctagaaaa 1920

ctgaaatttc agttgtcagt tgtggaattc agtttttcaa ttgtggaaat ttcctgccac 1980

cccaacagta tttttgtgtg ttaattaatt ttgcaaaatg agaatcatgg tgtgacactc 2040

atctaattta tcttgttgtg atgttatggt cataataagg agaaagaggg tttaattttt 2100

cttgtatttg gtttcctggt ggtatcatag tgtaatttta gtatttgaaa atcagtgtga 2160

ttccttaatg gccaactgaa gattgaattg ccgctaacaa ccatatcgtg ttagtgaatt 2220

ttcaatatgg accaggaagg catatgtatt ttgaacttga gtgaaaaggt tgaagttaca 2280

gacttttgca tagatggttt gtcaatttaa aattccagaa tttattattg ccatattttc 2340

acatgctgct tatacaagat tattattgag tagtaactgc ttccctgtct atgtagaagt 2400

gcctgtgttt ttatttattg ttcagatcaa agaccaaaac attttcttaa atatatttta 2460

tgtaatattt tatttgtata cagtgttgtt gatgaaatat ttaactagag catgatattt 2520

taaatgttaa ggtgtaacat atgttaaata aaactgttat ttttgaattt taaaatttgt 2580

tttttggggg tatgaactac tagagtttaa aattctgcca aactattact tatatgtact 2640

attgtgtaac atactttctt gaaatatttt tgtttataga attgaaggtt cttatcagat 2700

gggatactgg ggactataaa caatggaaat aaagccactg tatttttaaa aaaaaaaaaa 2760

aaaaaaaaaa aaaaaaaaaa aaaaaaaa 2788

<210> 14
<211> 2720
<212> DNA
<213> Homo sapiens

<400> 14
agaggggcgg gagctctggc tcaggtaaaa actctttctt cggctcgcga gctgagagga 60

gcaggtagag gggcagaggc gggactgtcg tctgggggag ccgcccagga ggctcctcag 120

gccgacccca gaccctggct ggccaggatg aagtatctcc ggcaccggcg gcccaatgcc 180

accctcattc tggccatcgg cgctttcacc ctcctcctct tcagtctgct agtgtcacca 240

cccacctgca aggtccagga gcagccaccg gcgatccccg aggccctggc ctggcccact 300

ccacccaccc gcccagcccc ggccccgtgc catgccaaca cctctatggt cacccacccg 360

gacttcgcca cgcagccgca gcacgttcag aacttcctcc tgtacagaca ctgccgccac 420
tttcccctgc tgcaggacgt gcccccctct aagtgcgcgc agccggtctt cctgctgctg 480
gtgatcaagt cctcccctag caactatgtg cgccgcgagc tgctgcggcg cacgtggggc 540
cgcgagcgca aggtacgggg tttgcagctg cgcctcctct tcctggtggg cacagcctcc 600
aacccgcacg aggcccgcaa ggtcaaccgg ctgctggagc tggaggcaca gactcacgga 660
gacatcctgc agtgggactt ccacgactcc ttcttcaacc tcacgctcaa gcaggtcctg 720
ttcttacagt ggcaggagac aaggtgcgcc aacgccagct tcgtgctcaa cggggatgat 780
gacgtctttg cacacacaga caacatggtc ttctacctgc aggaccatga ccctggccgc 840
cacctcttcg tggggcaact gatccaaaac gtgggcccca tccgggcttt ttggagcaag 900
tactatgtgc cagaggtggt gactcagaat gagcggtacc caccctattg tgggggtggt 960
ggcttcttgc tgtcccgctt cacggccgct gccctgcgcc gtgctgccca tgtcttggac 1020
atcttcccca ttgatgatgt cttcctgggt atgtgtctgg agcttgaggg actgaagcct 1080
gcctcccaca gcggcatccg cacgtctggc gtgcgggctc catcgcaacg cctgtcctcc 1140
tttgacccct gcttctaccg agacctgctg ctggtgcacc gcttcctacc ttatgagatg 1200
ctgctcatgt gggatgcgct gaaccagccc aacctcacct gcggcaatca gacacagatc 1260
tactgagtca gcatcagggt ccccagcctc tgggctcctg tttccatagg aaggggcgac 1320
accttcctcc caggaagctg agacctttgt ggtctgagca taagggagtg ccagggaagg 1380
tttgaggttt gatgagtgaa tattctggct ggcgaactcc tacacatcct tcaaaaccca 1440
cctggtactg ttccagcatc ttccctggat ggctggagga actccagaaa atatccatct 1500
tctttttgtg gctgctaatg gcagaagtgc ctgtgctaga gttccaactg tggatgcatc 1560
cgtcccgttt gagtcaaagt cttacttccc tgctctcacc tactcacaga cgggatgcta 1620
agcagtgcac ctgcagtggt ttaatggcag ataagctccg tctgcagttc caggccagcc 1680
agaaactcct gtgtccacat agagctgacg tgagaaatat ctttcagccc aggagagagg 1740
ggtcctgatc ttaacccttt cctgggtctc agacaactca gaaggttggg gggataccag 1800
agaggtggtg gaataggacc gccccctcct tacttgtggg atcaaatgct gtaatggtgg 1860
aggtgtgggc agaggaggga ggcaagtgtc ctttgaaagt tgtgagagct cagagtttct 1920
ggggtcctca ttaggagccc ccatccctgt gttccccaag aattcagaga acagcactgg 1980
ggctggaatg atctttaatg ggcccaaggc caacaggcat atgcctcact actgcctgga 2040
gaagggagag attcaggtcc tccagcagcc tccctcaccc agtatgtttt acagattacg 2100
gggggaccgg gtgagccagt gaccccctgt agcccccagc ttcaggcctc agtgtctgcc 2160
agtcaagctt cacaggcatt gtgatggggc agccttgggg aatataaaat tttgtgaaga 2220
cttggagatc tttttttttt tttaagcaaa tttacaagtt tcaacagaca agtccacatt 2280
catccctaaa agtctcattt tccagtagaa aatatacact ggtaaaaacg gggcatgggg 2340
ccgtggctca gggctgtaat tctagcacat tgggagacca aagtgggagg atcacttgag 2400

cccaggagtt ctggatcctg tctctgcaca aaataaaaaa ttactcaggc gtggtggtgc 2460

tcacatgcct gtagtcccag ctatacttgg gaggctgagg cgagaggatc gcttgagccc 2520

aggagttgga ggctgcagtg aaccatgatt gcgccactgt actccactgg gcggcaataa 2580

gaagacaaaa acataaaaca ggacatgtgt gaggcaaaag ctgcaggaat ttctatcagg 2640

cagatctgac ctcatcccac ccaccccctg ctcagatacc cttcatagct ccttattgct 2700

ttcagctcat aaccccacat 2720

<210> 15
<211> 1554
<212> DNA
<213> Homo sapiens

<400> 15
attcccacct cctccagaag ccccgcccac tcccgagccc cgagagctcc gcgcacctgg 60

gcgccatccg ccctggctcc gctgcacgag ctccacgccc gtaccccggc gtcacgctca 120

gcccgcggtg ctcgcacacc tgagactcat ctcgcttcga ccccgccgcc gccgccgccc 180

ggcatcctga gcacggagac agtctccagc tgccgttcat gcttcctccc cagccttccg 240

cagcccacca gggaaggggc ggtaggagtg gccttttacc aaagggaccg gcgatgctct 300

gcaggctgtg ctggctggtc tcgtacagct tggctgtgct gttgctcggc tgcctgctct 360

tcctgaggaa ggcggccaag cccgcaggag accccacggc ccaccagcct ttctgggctc 420

ccccaacacc ccgtcacagc cggtgtccac ccaaccacac agtgtctagc gcctctctgt 480

ccctgcctag ccgtcaccgt ctcttcttga cctatcgtca ctgccgaaat ttctctatct 540

tgctggagcc ttcaggctgt tccaaggata ccttcttgct cctggccatc aagtcacagc 600

ctggtcacgt ggagcgacgt gcggctatcc gcagcacgtg gggcagggtg gggggatggg 660

ctaggggccg gcagctgaag ctggtgttcc tcctaggggt ggcaggatcc gctcccccag 720

cccagctgct ggcctatgag agtagggagt ttgatgacat cctccagtgg gacttcactg 780

aggacttctt caacctgacg ctcaaggagc tgcacctgca gcgctgggtg gtggctgcct 840

gcccccaggc ccatttcatg ctaaagggag atgacgatgt ctttgtccac gtccccaacg 900

tgttagagtt cctggatggc tgggacccag cccaggacct cctggtggga gatgtcatcc 960

gccaagccct gcccaacagg aacactaagg tcaaatactt catcccaccc tcaatgtaca 1020

gggccaccca ctacccaccc tatgctggtg ggggaggata tgtcatgtcc agagccacag 1080

tgcggcgcct ccaggctatc atggaagatg ctgaactctt ccccattgat gatgtctttg 1140

tgggtatgtg cctgaggagg ctggggctga gccctatgca ccatgctggc ttcaagacat 1200

ttggaatccg gcggcccctg gaccccttag acccctgcct gtataggggg ctcctgctgg 1260

ttcaccgcct cagccccctc gagatgtgga ccatgtgggc actggtgaca gatgaggggc 1320

tcaagtgtgc agctggcccc ataccccagc gctgaagggt gggttgggca acagcctgag 1380

agtggactca gtgttgattc tctatcgtga tgcgaaattg atgcctgctg ctctacagaa 1440

aatgccaact tggtttttta actcctctca ccctgttagc tctgattaaa aacactgcaa 1500

cccaaaaaaa aaaaaaaaaa aaaaaaaaaa aaaaaaaaaa aaaaaaaaaa aaaa 1554

<210> 16
<211> 4131
<212> DNA
<213> Homo sapiens

<400> 16
atcccgcccg catacagccc gcatcccgcc ggggaagcga gcccagtcca gcgctgcccg 60

tccagtcctc gcccaagatt taaagcccgc aagttttgtt cttgagacca gcgactttag 120

ctccgatgcg ggaaggaaag ccgacctccg atttggacat ttaaagagct gggcttgaac 180

ttcgtgagtt tcgctctaaa ctgcccttga aatgaagctg gacttggagg tggcatggaa 240

tattcacatg ggagagccgc atgaggccgc ccaccacgct tcctgaagga tgcccgtgtg 300

gaagaatttt gacgtgccag tgtcctcgtt ctacagggtg ttccattctt ccgcaatctc 360

agaaaaatgg gactaaaaga aactattttg taaaataaga agacttccat ttttaatgac 420

caacatgtat taagatggac acctactcta cgaaacacga agttctatgg tctcgaagaa 480

gcccgtgcct gtttaaaact gatcctaact aaaaacagac ttgagtggat atgagaatgt 540

tggttagtgg cagaagagtc aaaaaatggc agttaattat tcagttattt gctacttgtt 600

ttttagcgag cctcatgttt ttttgggaac caatcgataa tcacattgtg agccatatga 660

agtcatattc ttacagatac ctcataaata gctatgactt tgtgaatgat accctgtctc 720

ttaagcacac ctcagcgggg cctcgctacc aatacttgat taaccacaag gaaaagtgtc 780

aagctcaaga cgtcctcctt ttactgtttg taaaaactgc tcctgaaaac tatgatcgac 840

gttccggaat tagaaggacg tggggcaatg aaaattatgt tcggtctcag ctgaatgcca 900

acatcaaaac tctgtttgcc ttaggaactc ctaatccact ggagggagaa gaactacaaa 960

gaaaactggc ttgggaagat caaaggtaca atgatataat tcagcaagac tttgttgatt 1020

ctttctacaa tcttactctg aaattactta tgcagttcag ttgggcaaat acctattgtc 1080

cacatgccaa atttcttatg actgctgatg atgacatatt tattcacatg ccaaatctga 1140

ttgagtacct tcaaagttta gaacaaattg gtgttcaaga cttttggatt ggtcgtgttc 1200

atcgtggtgc ccctcccatt agagataaaa gcagcaaata ctacgtgtcc tatgaaatgt 1260

accagtggcc agcttaccct gactacacag ccggagctgc ctatgtaatc tccggtgatg 1320

tagctgccaa agtctatgag gcatcacaga cactaaattc aagtctttac atagacgatg 1380

tgttcatggg cctctgtgcc aataaaatag ggatagtacc gcaggaccat gtgttttttt 1440

ctggagaggg taaaactcct tatcatccct gcatctatga aaaaatgatg acatctcatg 1500

gacacttaga agatctccag gacctttgga agaatgctac agatcctaaa gtaaaaacca 1560

tttccaaagg tttttttggt caaatatact gcagattaat gaagataatt ctcctttgta 1620

aaattagcta tgtggacaca tacccttgta gggctgcgtt tatctaatag tacttgaatg 1680

ttgtatgttt tcactgtcac tgagtcaaac ctggatgaaa aaaaccttta aatgttcgtc 1740

tataccctaa gtaaaatgag gacgaaagac aaatattttg aaagcctagt ccatcagaat 1800
gtttctttga ttctagaagc tgtttaatat cacttatcta cttcattgcc taagttcatt 1860
tcaaagaatt tgtatttaga aaaggtttat attattagtg aaaacaaaac taaagggaag 1920
ttcaagttct catgtaatgc cacatatata cttgaggtgt agagatgtta ttaagaagtt 1980
ttgatgttag aataattgct tttggaaaat accaaatgaa cgtacagtac aacatttcaa 2040
ggaaatgaat atattgttag accaggtaag caagtttatt tttgttaaag agcacttggt 2100
ggaggtagta ggggcaggga aaggtcagca taggagagaa agttcatgaa tctggtaaaa 2160
cagtctcttg ttcttaagag gagatgtaga aaaatgtgta caatgttatt ataaacagac 2220
aaatcacgtc ttaccacatc catgtagcta ctggtgttag agtcattaaa ataccttttt 2280
ttgcatcttt tttcaaagtt taatgtgaac ttttagaaaa gtgattaatg ttgccctaat 2340
actttatatg tttttaatgg attttttttt aagtattaga aaatgacaca taacacgggc 2400
agctggttgc tcatagggtc cttctctagg gagaaaccat tgttaattca aataagctga 2460
ttttaatgac gttttcaact ggtttttaaa tattcaatat tggtctgtgt ttaagtttgt 2520
tatttgaatg taatttacat agaggaatat aataatggag agacttcaaa tggaaagaca 2580
gaacattaca agcctaatgt ctccataatt ttataaaatg aaatcttagt gtctaaatcc 2640
ttgtactgat tactaaaatt aacccactcc tccccaacaa ggtcttataa accacagcac 2700
tttgttccaa gttcagagtt ttaaattgag agcattaaac atcaaagtta taatatctaa 2760
aacaatttat ttttcatcaa taactgtcag aggtgatctt tattttctaa atatttcaaa 2820
cttgaaaaca gagtaaaaaa gtgatagaaa agttgccagt ttggggttaa agcattttta 2880
aagctgcatg ttccttgtaa tcaaagagat gtgtctgaga tctaatagag taagttacat 2940
ttattttaca aagcaggata aaaatgtggc tataatacac actacctccc ttcactacag 3000
aaagaactag gtggtgtcta ctgctaggga gattatatga aggccaaaat aatgacttca 3060
gcaagagtga ctgaactcac tctaaggcct ttgactgcag aggcacctgt tagggaaaat 3120
cagatgtctc atataataag gtgatgtcgg aaacacgcaa aacaaaacga aaaaagattt 3180
ctcagtatac acaactgaat gatgatactt acaattttta gcaggtagct ttttaatgtt 3240
tacagaaatt ttaatttttt tctattttga aatttgaggc ttgtttacat tgcttagata 3300
atttagaatt tttaactaat gtcaaaacta cagtgtcaaa cattctaggt tgtagttact 3360
ttcagagtag atacagggtt ttagatcatt acagtttaag ttttctgacc aattaaaaaa 3420
acatagagaa caaaagcata tttgaccaag caacaagctt ataattaatt tttattagtt 3480
gattgattaa tgatgtattg ccttttgccc atatataccc tgtgtatcta tacttggaag 3540
tgtttaaggt tgccattggt tgaaaacata agtgtctctg gccatcaaag tgatcttgtt 3600
tacagcagtg cttttgtgaa acaattattt atttgctgaa agagctcttc tgaactgtgt 3660
ccttttaatt tttgcttaga atagaatgga acaagtttaa atttcaagga aatatgaagg 3720
cacttccttt ttttctaaga aggaagttgc tagatgattc cttcatcaca cttacttaaa 3780

gtactgagaa gagtatctgt aaataaaagg gttccaacct tttaaaaaag aaggaaaaaa 3840

ctttttggtg ctccagtgta gggctatctt tttaaaaaat gtcaacaaag ggaaaataaa 3900

ctatcagctt ggatggtcac ttgaatagaa gatggttata cacagtgtta ttgttaaaat 3960

ttttttacct tttggttggt ttgcatcttt tttccatatt gttaatttta taccaaaatg 4020

ttaaatattt gtattacttg aattttgctc ttgtatggca aaataattag tgagtttaaa 4080

aaaaatctat agtttccaat aaacaactga aaaattatca tgaaaaaaaa a 4131

<210> 17
<211> 2510
<212> DNA
<213> Homo sapiens

<400> 17
gaacctcagt gtgtgaagta aagggattaa aggctagtct caggctgggg atggctcctg 60

tctatttctt ctctctcaga gactgcagat ggcttttccc tgccgcaggt ccctgactgc 120

caagactctg gcctgcctcc tggtgggcgt gagtttctta gcactgcagc agtggttcct 180

ccaggcgcca aggtccccgc gggaggagag gtccccgcag gaggagacgc cagagggtcc 240

caccgacgct cccgcggctg acgagccgcc ctcggagctc gtccccgggc ccccgtgcgt 300

ggcgaacgcc tcggcgaacg ccacggccga cttcgagcag ctgcccgcgc gcatccagga 360

cttcctgcgg taccgccact gccgccactt cccgctgctt tgggacgcac cggccaagtg 420

cgccggcggc cgaggcgtgt tcctgctcct ggcggtgaag tcggcgcctg agcactacga 480

gcgacgcgag ctcatccggc gcacgtgggg gcaagagcgc agctacggcg ggcggccagt 540

gcgccgcctc tttctattgg gcaccccggg ccccgaggac gaggcgcgcg cggagcggct 600

ggcggagctg gtggcgctgg aggcgcgcga gcacggcgac gtgctgcagt gggccttcgc 660

ggacaccttc ctcaacctca cgctcaagca cctgcacttg ctcgactggc tggctgcacg 720

ctgcccgcac gcgcgctttc tgctcagcgg cgacgacgac gtgttcgtgc acaccgccaa 780

cgtagtccgc ttcctgcagg cgcagccacc cggccgccac ctgttctccg gccagctcat 840

ggagggctcc gtgcccatcc gcgacagctg gagcaagtac ttcgtgccgc cgcagctctt 900

ccccgggtcc gcttacccgg tgtactgcag cggcggcggc ttcctcctgt ccggccccac 960

ggcccgggcc ctgcgcgcgg ccgcccgcca caccccgctc ttccccatcg acgacgccta 1020

catgggcatg tgtctggagc gcgccggcct ggcgcccagc ggccacgagg gcatccggcc 1080

cttcggcgtg cagctgcctg gcgcacagca gtcctccttc gacccctgca tgtaccgcga 1140

gttgctgcta gtgcaccgct tcgcgcccta cgagatgctg ctcatgtgga aggcgctgca 1200

cagccccgcg ctcagctgtg accggggaca ccgggtctcc tgaggccagt tgggcggctt 1260

cagccccggg cctccaacca tgtccatgct gagaaggcag ctttcccgct ctgggtacct 1320

tacgtcctgc ccagctctgt gcacctgaac cccagctgcg cactgaaatc agctggggtg 1380

gggggtgtgg aaaatgccta catcctggct ccatctcccg aagtttcgat ttgattagtc 1440

tggggtggac ccagacatgt taagtatttt ttaagttcct ccagtgatgc gaatgtgcag 1500

ctaggcctga ggaccactcg gctagactat ctcttcatcc tcgcaaagcc agctccaccg 1560
ccctctctgc aagaattccg ggcccctcgc tcccacactc gggtcctctt gagcagtgga 1620
gcaagggaga cctgggagcg tgggagccag gatcagcgcc ccctgccatg tgcctacaaa 1680
tgtcagttgt gatttccact gtttacaagt gagtggagct ggagctgggc tgacagtatc 1740
aggtggatcc cgcttccccc tcccccaaga agtcagccaa cacgcagctg aggcgcatgt 1800
ggtggccttc ttcccaccac taccccagta caccgtgagg tagaaatctt caccgtgcaa 1860
agtggaaacc agaggcccgg tcagacagtg actaatccag ggccgtggca ttcccagaca 1920
gcacaccact gtggtcccct ccacactcac cccaaccaaa gctaatggcc tagttgggtc 1980
ctgcccgcca ataatcaccc ccacgggtca gagacaggct ccttgccggg gtctgggcct 2040
caggctcagt gggccttgga caacccagca gggagttccg gggagtccga agtggagaaa 2100
ggctggtggg aacatggagg ccagtgttgg ggagcctgtg gaggcaggtg tgtagaattg 2160
tgttcgggag gtgggggatc tgagaccgaa gtggacagtg gttaagattg tggggccggg 2220
cgaggtggct cacgcctgta atcccagcac tttgggaggc tgaggaggtc ggatcatgag 2280
gtcaagagtt cgagaccagc ctggccaata tggtgaaacc ccgtctctat tgggagtaca 2340
aaaattagcc ggccatagtg gctcgtgcct gtaatctcag ctatttggga ggctgaggca 2400
ggagaatcac ttgaacctgg gaggcggagg ttgcagtgag ccgagatcgt gccactgcac 2460
tccagcctgg gcgacagagc aagactgcat ctcaaaaaaa aaaaaaaaaa 2510

<210> 18
<211> 1434
<212> DNA
<213> Homo sapiens

<400> 18
gggttaaggc acctgggcca ccgcccctgc cggcccctcc tcccgcggcc gggaagagga 60
aagtcgcggc gggggcgcgg cccggtctcc gtccccaccc gcccgccgtc ccgccggccc 120
gagccgtggc gcccagagct gcgagccgct cgcccctccg ccgctccggc ccgggccgcc 180
atgtcgctgt ggaagaaaac cgtctaccgg agtctgtgcc tggccctggc cctgctcgtg 240
gccgtgacgg tgttccaacg cagtctcacc cctggtcagt ttctgcagga gcctccgcca 300
cccaccctgg agccacagaa ggcccagaag ccaaatggac agctggtgaa ccccaacaac 360
ttctggaaga acccgaaaga tgtggctgcg cccacgccca tggcctctca ggggccccag 420
gcctgggacg tgaccaccac taactgctca gccaatatca acttgaccca ccagccctgg 480
ttccaggtcc tggagccgca gttccggcag tttctcttct accgccactg ccgctacttc 540
cccatgctgc tgaaccaccc ggagaagtgc aggggcgatg tctacctgct ggtggttgtc 600
aagtcggtca tcacgcagca cgaccgccgc gaggccatcc gccagacctg gggccgcgag 660
cggcagtccg cgggtggggg ccgaggcgcc gtgcgcaccc tcttcctgct gggcacggcc 720
tccaagcagg aggagcgcac gcactaccag cagctgctgg cctacgaaga ccgcctctac 780

ggcgacatcc tgcagtgggg ctttctcgac accttcttca acctgaccct caaggagatc 840

cacttcctca agtggctgga catctactgc ccccacgtcc ccttcatttt caaaggcgac 900

gatgacgtct tcgtcaaccc caccaacctg ctagaatttc tggctgaccg gcagccacag 960

gaaaacctgt tcgtgggcga tgtcctgcag cacgctcggc ccattcgcag gaaagacaac 1020

aaatactaca tcccgggggc cctgtacggc aaggccagct atccgccgta tgcaggcggc 1080

ggtggcttcc tcatggccgg cagcctggcc cggcgcctgc accatgcctg cgacaccctg 1140

gagctctacc cgatcgacga cgtctttctg ggcatgtgcc tggaggtgct gggcgtgcag 1200

cccacggccc acgagggctt caagactttc ggcatctccc ggaaccgcaa cagccgcatg 1260

aacaaggagc cgtgcttttt ccgcgccatg ctcgtggtgc acaagctgct gccccctgag 1320

ctgctcgcca tgtgggggct ggtgcacagc aatctcacct gctcccgcaa gctccaggtg 1380

ctctgacccc agccgggcta ctaggacagg ccagggcact tgctcctgag cccc 1434

<210> 19
<211> 1894
<212> DNA
<213> Homo sapiens

<400> 19
tcttcctcca gctgttccca caggctccat tattcaaact ttgggggagg aaatcagggc 60

tggacagatc atcaactgct gctgctgaca gactgtgttc ctgccatgat ggggacaggc 120

ctgccccaaa ccaggctgtc tcaccagaaa ccagccctaa cacgccagag ccccatctct 180

caggtcggcc tcctgccctc ctccctccct ccctccctcc ctctctcctg cctcagcccc 240

gccccggccc ctggggccct ccctgctccc gctccactcc cctccccgcc cctctggact 300

ggctgggcag ggctgggccc cgccggtcga caacacaggg gctggggcag gcagatcaga 360

gggagctgag ggaggcctga cctgaggccg gcacccggag ctggcgggag ccagacccag 420

agctcccgcg gccgcccctt ccctgggccg ggtcatgcgc tgccccaagt gccttctctg 480

cctgtcagca ctgctcacac tcctgggcct caaagtgtac atcgagtgga catccgagtc 540

ccggctcagc aaggcctacc ccagccctcg gggcaccccg ccaagcccca cgccagccaa 600

ccctgagccc accctacctg ccaacctctc cacccgcctg ggccagacta tcccgctgcc 660

ctttgcttac tggaaccagc agcagtggcg gctggggtcc ctgcccagtg gggacagcac 720

tgaaacgggg ggctgccagg cttggggggc cgccgccgcc accgagatcc ctgacttcgc 780

ctcctacccc aaggacctcc gccgcttctt gctgtcagca gcctgccgga gcttcccaca 840

gtggctgcct ggaggtggtg gcagccaagt ctccagctgc tcagatactg atgtccccta 900

cctgctgttg gccgtcaagt cagaaccagg gcgctttgca gaacgacagg ccgtgagaga 960

gacgtggggc agtccagctc cagggatccg gctgctcttc ctgctagggt ctccggtggg 1020

tgaggcgggg cctgacctag actcactagt ggcctgggag agccgtcgct acagtgacct 1080

gctgctctgg gacttcctcg acgtcccatt caaccagacg ctcaaagacc tgctgctgct 1140

ggcctggctg ggccgccact gccccaccgt gagttttgtc ttgcgagctc aggacgatgc 1200

ctttgtacac acccctgccc tgctggctca cctgcgggcc ctgccacctg cctcggcccg 1260

aagcctctac ctgggtgagg tctttaccca ggccatgcct ctccggaagc caggaggacc 1320

cttctatgtg cccgagtcct tcttcgaagg tggctaccca gcctatgcaa gcgggggtgg 1380

ctacgtcatt gccgggcgcc tggcaccctg gctgctgcgg gcggcagccc gtgtggcacc 1440

cttccccttt gaggacgtct acactggcct ttgcatccga gccctgggcc tggtgcccca 1500

ggcccaccca ggcttcctca cagcctggcc agcagaccgc actgcggacc actgtgcttt 1560

ccgcaacctg ctgctggtac ggcccctggg cccccaggcc agcattcggc tctggaaaca 1620

actgcaagac ccaaggctcc agtgctgact ctcattgggg agggcggagg tgctgacctg 1680

gcctgggcct ctggggccgg cccctggctc agcccctcct tccaggtctt gatgggaggg 1740

aggagggccc agaagctgga caacttaagc cactccttgg cctcccccag ccaggggcct 1800

gggcaggaaa gatggggtgg tggactgttt ttgcctactt tttgtttttg aaaaacatgc 1860

actccccact ctgaaaaaaa aaaaaaaaaa aaaa 1894

<210> 20
<211> 1383
<212> DNA
<213> Homo sapiens

<400> 20
gccgaagcgg cgggatgagc ggagccggcg tgggcggggc gtccgaggag agccaggcca 60

tgcaggccca cgtgtctatt atcctcccag tccacaacgc tgaaccgtgg ctggacgaat 120

gtttgaggtc tgttttgcaa caggactttg aaggtaccat ggagctgtct gttttcaatg 180

atgccagtaa ggacaagtct ggggctatca ttgaaaaatg gagagtgaag ctggaagatt 240

ctggtgtcca cgtgatcatt ggggggcacg attctccctc tcctagaggc gtcggatacg 300

ctaaaaatca agcagttgcc cagagctcag ggtcttacct ttgctttttg gattcggatg 360

acgtcatgat gccccagcgg gtgaggctgc aacacgaggc tgccgttcag cacccgtcga 420

gcatcattgg ttgcagagtg aggagagatc cccctaactc caccgaacga tacacacgtt 480

ggatcaacca gctgacgccg gagcagctcc taacccaggt tttcacctca aatggcccca 540

cggtgatcat gcccacctgg ttctgctcgc gagcgtggtt ctcccacgtg ggccccttta 600

acgagggagg tcagggcgtc ccggaggacc tgctgttctt ctacgagcac ctcaggaagg 660

gcggcggcgt catccgcgtg gaccagagtc tcctgctgta tcgccaccac ccacaggcgg 720

ccacgcactg cgtcctcgag acgaccatct ggacccaccg cgtccgcttc ctggaagagc 780

aggccctgcc ccgctgggcg gccttcacca tctggaacgc tggcaagcag gggcgccggc 840

tgtaccgcag cttgactgcc ggcagccagc gcaaggtggt ggcattctgt gacgtggacg 900

agaacaagat caggaaaggc ttctattgcc acgaggactc tcaggaaaga cccaagcccc 960

gaatccccat cctgcacttc cgagccgccc ggccaccctt cgtcatctgc gtgaagctgg 1020

acctcacagg gggcgccttt gaggacaacc tgaggtcact gcacttgcag gagggccagg 1080

acttccttca cttcagctga cggacccatg gcagctgctc ccagtgcatc ccaggccagc 1140

aggagctttg tgagctgcag gcatggcgat ggtgcgcctg ttccacaccc agcaggcgca 1200

accagagtct cgtgtgtgcc gaccacagga gccaagcctt ttccactgtg tggactcatg 1260

tggccaaggc taggcctggt cacccaggac cctcaccacg tgaccccagc caatcgggac 1320

agttcaagga ggaggagacc cctattacac aggttggaat aaaatattta aatctcgtaa 1380

aaa 1383

<210> 21
<211> 3010
<212> DNA
<213> Homo sapiens

<400> 21
cgcattcccc gcgcggagcc gaagcagccg caacgagccg ggagctgagc cgcgctgcgc 60

tgcggtgcga agagccgggc ggcggccaga gccctccccg cgctgccagt gggacgcgga 120

gccagggatc ccccggcttg ccccggggct ggggtgcaac agactcttaa cttggtcctc 180

gcgccccgcc aaccgccccc gggcaggaaa cgcccagaac caccaccgcg ccgcgctcca 240

caaaagcccc gggagttcca agacgggagg gatcgggcgc gctccagagg caggagggtc 300

ccccacaccc tcaggctcat gcccagctcc cccatcgggc agcccccagc cccatgagga 360

ccctcaggcc ggggcgagag cccggcacag cccggaccga aattttgccg ctgccttaga 420

gcgttagaca ggatgtggct gggccgccgg gccctgtgcg ctctggtcct tctgctcgcc 480

tgcgcctcgc tggggctcct gtacgcgagc acccgggacg cgcccggcct ccggctacct 540

cttgcgccgt gggcgccccc gcaaagcccc cgcaggcccg agctgccaga tcttgctcct 600

gagccccgct acgcacacat cccggtcagg atcaaggagc aagtagtggg gctgctggct 660

tggaacaact gcagttgtga gtccagtggg gggggcctcc ccctcccctt ccagaaacaa 720

gtccgagcta ttgacctcac caaggccttt gaccctgcag agctgagggc tgcctctgcc 780

acaagagagc aggagttcca ggcctttctg tcgaggagcc agtccccagc tgaccagctg 840

ctcatagccc ctgccaactc cccgctccag taccccctac agggtgtgga agttcagccc 900

ctcaggagca tcttggtgcc agggctgagc cttcaggcag cttctggtca ggaggtatac 960

caggtgaacc tgactgcctc cctaggcacc tgggacgtgg caggggaagt gactggagtt 1020

actctcactg gagagggtca ggcagatctc acccttgtca gcccagggct ggaccaactc 1080

aacaggcaac tacaactggt cacttacagc agccgaagct accagaccaa cacagcagac 1140

acagtccggt tctccaccga gggacatgag gctgctttca ctatccgcat aagacacccg 1200

cccaaccctc ggctgtaccc acctgggtct ctaccccagg gagcccagta caacatcagc 1260

gctctagtca cgattgccac caagaccttc ctccgttatg atcggctacg ggctctcatc 1320

accagtatcc gccgcttcta cccaacggtt accgtggtca tcgctgacga cagcgacaag 1380

ccagagcgcg ttagtggccc ctacgtggaa cactatctca tgcccttcgg caagggctgg 1440

ttcgcaggcc ggaacctggc cgtgtctcaa gtaaccacca agtacgtgct gtgggtggac 1500

gacgacttcg tcttcacggc gcggacgcgg ctggagaggc ttgtggacgt gctggagcgg 1560
acgccgctgg acctggtggg gggcgcggtg cgcgagatct ccggctttgc caccacttat 1620
cggcagctgc tgagcgtgga gcccggcgcc ccaggcctcg ggaactgcct ccggcaaagg 1680
cgcggcttcc accacgagct cgtcggcttc ccaggctgcg tggtcaccga cggcgtggtt 1740
aacttcttcc tggcgcggac tgacaaggtg cgcgaggtcg gtttcgaccc ccgcctcagc 1800
cgcgtggctc atctggaatt cttcttggat gggcttggtt cccttcgggt tggctcctgc 1860
tccgacgtcg tggtggatca tgcatccaaa ctgaagctgc cttggacatc aagggatgcc 1920
ggagcagaga cttacgcccg gtaccgttac ccaggatcac tggacgagag ccagatggcc 1980
aaacaccggc tgctcttctt caaacaccgg ctgcagtgca tgacctccca gtgatggccc 2040
gctggggatt tctgactgtc aggctgggcc tgcctccttg tccctgccag gaatttccaa 2100
caaaccccac caccctgtga gcactctact ggctgtccct gagcctctag ttcctcactc 2160
ttccttttca gaacctgatg cccagtaggg gttgtcctgg tgacacccct cctttttcca 2220
gtgcccagag gcctggtgga gccataacct ctcccacagc cagtgccaag tcctccccct 2280
gcccattctc atggggcagg aaatgggggg atcactttcc aagtgccaaa gagcccagag 2340
ggactctaag aacctaaggt ggaaacactg tcctctcatc ttgggaccga gggggtgggg 2400
aagttcccca acacataatc ccaagactgt gcccctcatc tgcatcttca gatccagtac 2460
tctgtgtacc tgctccagcc ccacccccac agagagaact tgtggctctg gggctggggt 2520
gagggctggt ggttggtgaa agccattctt agttgtgtct ctgcaatgct gtgggcacaa 2580
aagaaggggc accagagtcc ctgtgcaaac acctagactc acttcatgga ttccaaagct 2640
ctcagcttca ttttattagt tacgttaggt aagggggttc aagggtcatg gtcctcatca 2700
cacacatgtc atcagggccc tctgcactcc acatgatgag gtcagaccca cacggtgcaa 2760
atctttgggt cagtgagctc ctggagaaga gaggagacat gtcaggaata gattaggcac 2820
ccctcttcct taatgaaatg tggcagtcct ctcaggggta ccccacctac ttagggatct 2880
gagttcttca gttcctcacc catagctcag cccgggctcc ctgatttggt cttcaatcta 2940
ctattcattc cgtccccacc cttttcttct tgatcttcct cagacccttt cccaaacccc 3000
tctccctggg 3010

<210> 22
<211> 1792
<212> DNA
<213> Homo sapiens

<400> 22
ataggtggct gcagaggcga ggtgacggcg cgtgcggaac gaactctgca cccccaggaa 60
tggggagcgc tggcttttcc gtgggaaaat tccacgtgga ggtggcctct cgcggccggg 120
aatgtgtctc ggggacgccc gagtgtggga atcggctcgg gagtgcgggc ttcggggatc 180
tctgcttgga actcagaggc gctgacccag cctggggccc gtttgctgcc cacgggagga 240

gccgccgtca gggctcgaga tttctgtggc tcctcaagat attggtcata atcctggtac 300

ttggcattgt tggatttatg ttcggaagca tgttccttca agcagtgttc agcagcccca 360

agccagaact cccaagtcct gccccgggtg tccagaagct gaagcttctg cctgaggaac 420

gtctcaggaa cctcttttcc tacgatggaa tctggctgtt cccgaaaaat cagtgcaaat 480

gtgaagccaa caaagagcag ggaggttaca actttcagga tgcctatggc cagagcgacc 540

tcccagcggt gaaagcgagg agacaggctg aatttgaaca ctttcagagg agagaagggc 600

tgccccgccc actgcccctg ctggtccagc ccaacctccc ctttgggtac ccagtccacg 660

gagtggaggt gatgcccctg cacacggttc ccatcccagg cctccagttt gaaggacccg 720

atgcccccgt ctatgaggtc accctgacag cttctctggg gacactgaac acccttgctg 780

atgtcccaga cagtgtggtg cagggcagag gccagaagca gctgatcatt tctaccagtg 840

accggaagct gttgaagttc attcttcagc acgtgacata caccagcacg gggtaccagc 900

accagaaggt agacatagtg agtctggagt ccaggtcctc agtggccaag tttccagtga 960

ccatccgcca tcctgtcata cccaagctat acgaccctgg accagagagg aagctcagaa 1020

acctggttac cattgctacc aagactttcc tccgccccca caagctcatg atcatgctcc 1080

ggagtattcg agagtattac ccagacttga ccgtaatagt ggctgatgac agccagaagc 1140

ccctggaaat taaagacaac cacgtggagt attacactat gccctttggg aagggttggt 1200

ttgctggtag gaacctggcc atatctcagg tcaccaccaa atacgttctc tgggtggacg 1260

atgattttct cttcaacgag gagaccaaga ttgaggtgct ggtggatgtc ctggagaaaa 1320

cagaactgga cgtggtaggc ggcagtgtgc tgggaaatgt gttccagttt aagttgttgc 1380

tggaacagag tgagaatggg gcctgccttc acaagaggat gggatttttc caacccctgg 1440

atggcttccc cagctgcgtg gtgaccagtg gcgtggtcaa cttcttcctg gcccacacgg 1500

agcgactcca aagagttggc tttgatcccc gcctgcaacg agtggctcac tcagaattct 1560

tcattgatgg gctagggacc ctactcgtgg ggtcatgccc agaagtgatt ataggtcacc 1620

agtctcggtc tccagtggtg gactcagaac tggctgccct agagaagacc tacaatacat 1680

accggtccaa caccctcacc cgggtccagt tcaagctggc cctccactac ttcaagaacc 1740

atctccaatg tgccgcataa aggtgtgagg gcataggaga aacactaggc tg 1792

<210> 23
<211> 3438
<212> DNA
<213> Homo sapiens

<400> 23
atggggagcc cccgggccgc gcggcccccg ctgctcctgc gcccggtgaa gctgctgcgg 60

aggcgcttcc ggctgctgct ggcgctcgcc gtggtgtctg tggggctctg gactctgtat 120

ctggaactgg tggcgtcggc ccaggtcggc gggaaccccc tgaaccggag gtacggcagc 180

tggagagaac tggccaaggc tctggccagc aggaacattc cagctgtgga tccacacctc 240

cagttctacc atccccagag gctgagcctc gaggaccacg acattgacca aggggtgagc 300

agtaacagca gctacttgaa gtggaacaag cctgtcccct ggctctcaga gttccggggc 360
cgtgccaacc tgcatgtgtt tgaagactgg tgtggcagct ctatccagca gctcaggagg 420
aacctgcatt tcccactgta cccccatatt cgcacaaccc tgaggaagct tgctgtgtcc 480
cccaaatgga ccaactatgg cctccgcatc tttggctacc tgcacccctt tactgatggg 540
aaaatccagt ttgccattgc tgcagatgac aacgcggagt tctggctgag cctcgatgac 600
caggtctcag gcctccagct gctggccagt gtgggcaaga ctggaaagga gtggaccgcc 660
ccgggagagt ttgggaaatt tcggagccaa atttccaagc cggtgagcct gtcagcctcc 720
cacaggtact acttcgaggt gctgcacaag cagaatgagg agggcaccga ccacgtggaa 780
gttgcatggc gacggaacga ccctggagcc aagttcacca tcattgactc cctctccctg 840
tccctcttca caaatgagac gttcctacag atggatgagg tgggccacat cccacagaca 900
gcagccagcc acgtggactc ctccaacgct cttcccaggg atgagcagcc gcccgctgac 960
atgcttcggc ctgacccccg ggacaccctc tatcgagtgc ctctgatccc caagtcgcat 1020
ctccgccacg tcctgcctga ctgtccctac aaacccagct atctggtgga tgggcttcct 1080
ctgcagcgct accagggact ccggtttgtt catctgtctt ttgtttaccc caatgactat 1140
acccgcctga gccacatgga gacccacaat aaatgtttct accaggaaaa cgcctactac 1200
caagaccggt tcagctttca ggagtacatc aagattgacc agcctgagaa gcaggggctg 1260
gagcagccag gttttgagga aaaccttcta gaagagtccc agtatgggga agtggcagag 1320
gagacccctg cctccaacaa ccagaatgcc aggatgcttg agggaagaca gacacctgcc 1380
tccaccctgg agcaagatgc cactgactac cgcctccgaa gcctgcggaa actcctggct 1440
cagccccggg agggcctgct ggcccccttc tccaagcgga actccacagc gtccttccca 1500
gggaggacca gccacattcc agtgcagcag ccagagaaga ggaagcaaaa acccagccct 1560
gagcccagcc aagattcacc tcattccgac aagtggcctc ctgggcaccc tgtgaagaac 1620
ctgcctcaga tgagggggcc caggcccagg cccgctggtg acagccccag gaagactcag 1680
tggctgaacc aggtggagtc gtacatcgca gagcagagac ggggtgacag gatgcggcct 1740
caggcccctg gaaggggctg gcatggggag gaggaagtgg tggcggccgc aggccaggaa 1800
ggacaagtgg agggagagga agagggggaa gaagaggagg aggaagagga tatgagtgag 1860
gtgttcgagt acgtacctgt gtttgacccg gtagtaaact gggaccagac cttcagtgcc 1920
cggaatctcg acttccaagc cctgaggact gactggatcg atctgagctg taacacatct 1980
ggcaacctgc tgcttccaga gcaggaagct ctggaggtca cgcgagtctt cttgaagaag 2040
ctcaaccaga ggagccgggg gaggtaccag ctacagcgca ttgtgaacgt ggaaaagcgt 2100
caggaccagc tacgtggggg tcgctacctc ctggagcttg aactgttgga acaaggccag 2160
cgcgtggtgc ggctctcgga gtatgtgtct gcacgaggct ggcagggcat cgatccagct 2220
ggtggggagg aggtcgaggc ccggaacctg caaggcctgg tctgggaccc acacaaccgt 2280
aggagacagg tcctgaatac ccgggcccaa gagcccaagc tgtgctggcc tcagggtttc 2340

tcctggagtc accgagccgt ggtccacttc gtcgtgcctg tgaagaacca ggcacgctgg 2400
gtacagcaat tcatcaaaga catggaaaac ctgttccagg tcaccggtga cccacacttc 2460
aacatcgtca tcactgacta tagcagtgag gacatggatg ttgagatggc actgaagagg 2520
tccaagctgc ggagctacca gtacgtgaag ctaagtggaa actttgaacg ctcagctgga 2580
cttcaggctg gcatagacct cgtgaaggac ccgcacagca tcatcttcct ctgtgacctc 2640
cacatccact tcccagctgg agtcatcgat gccattcgga agcactgtgt ggagggaaag 2700
atggcctttg cccccatggt gatgaggctg cattgtgggg ccacccccca gtggcctgag 2760
ggctactggg aggtgaatgg gttcgggctg cttggcatct acaagtctga cctggacagg 2820
attgggggca tgaacaccaa ggagttccga gaccgctggg gcggggaaga ctgggagctg 2880
ctggacagga tactccaagc gggcctggac gtggagcgtc tctccctcag gaatttcttc 2940
catcatttcc attccaagcg aggcatgtgg agccgtcgcc agatgaagac gctgtagccg 3000
gagggtgtcc gcggggccca gcactccccg ctctggacta gcagtggctc cccagggccc 3060
tgctactgtt cagggatggg gagtggggtg acggctggac cccaagaggc ctcgaagctg 3120
acggcccact ccacctggag ctgtcccctc acagaggcag gttccggggc tcctgtctct 3180
gcctcctggg ccttcagaag ggaggacttt gagagagagg ccaggaggga ccacttgctc 3240
agtcgagaac gggaagagct cctgagaagg acgggtcagg aaggagagat ctgactgagc 3300
gacaccatcc tcatccatga aggtgcacgc ccacatcccc caagcgctcc tcacgccaag 3360
cgctgaccac cagaccctct tctagtggac atattttatt gggggcgggg gattttaaaa 3420
acagaaaacc gaaactgc 3438

<210> 24
<211> 3120
<212> DNA
<213> Homo sapiens

<400> 24
atgccgcggc tcccggtgaa gaagatccgt aagcagatga agctgctgct gctgctgctg 60
ctgctgagct gcgccgcgtg gctcacctac gtgcacctgg gcctggtgcg ccagggacgc 120
gcgctgcgcc agcgcctggg ctacgggcga gatggtgaga agctgaccag tgagaccgac 180
ggccgggggg tccacgctgc gccatccaca cagagggctg aggactccag tgagagccgt 240
gaagaggagc aagcgcccga aggtcgggac ctagacatgc tgtttcctgg gggggctggg 300
aggctgccac tgaacttcac ccatcagaca cccccatggc gggaggagta caaggggcag 360
gtgaacctgc acgtgtttga ggactggtgt gggggcgccg tgggccacct gaggaggaac 420
ctgcacttcc cgctgttccc tcatacgcgc accaccgtga agaagttggc cgtgtccccc 480
aagtggaaga actatggact ccgtattttt ggtttcatcc acccggcgag ggacggagac 540
gtccagtttt ctgtggcctc agacgacaac tcggagttct ggctgagtct ggacgagagc 600
cctgctgctg cccagcttgt ggcctttgtg ggcaagactg gctccgagtg gacagcgcct 660

ggagaattca ccaagttcag ctcccaggtg tccaagccca ggcggctcat ggcctcccgg 720
aggtactact ttgagttgct gcacaagcag gacgaccgcg gctcggacca cgtggaagtg 780
ggctggcgag ctttcctgcc cggcctgaag ttcgaggtca tcagctctgc tcacatctcc 840
ctgtacacag atgagtcagc cttgaagatg gaccacgtgg cgcacgtccc ccagtctcca 900
gccagccacg tgggggggcg tccgccgcag gaggagacca gcgcagacat gctgcggcca 960
gatcccaggg ataccttttt cctcactcca cgcatggaat cttcgagcct ggagaacgtg 1020
ctggagccct gcgcctacgc ccccacctac gtggtcaagg acttcccgat cgccagatac 1080
cagggcctgc aatttgtgta cctgtccttc gtttatccca acgactacac tcgcctcacc 1140
cacatggaga cggacaacaa gtgcttctac cgcgagtctc cgctgtatct ggagaggttt 1200
gggttctata aatacatgaa gatggacaag gaggaggggg atgaggatga agaagacgag 1260
gtgcagcgcc gagccttcct cttcctcaac ccggacgact tcctggacga cgaggacgag 1320
ggggagctgc tcgacagcct ggagcccacc gaggcggccc cgcccaggag cggcccccag 1380
tcccccgccc cagcagcccc cgcccagccc ggagccaccc tcgccccgcc gacccctccc 1440
cgcccccggg acggggggac ccccaggcac tcccgggccc tgagctgggc cgccagggcc 1500
gcccgccctt tgccgctctt cttgggccga gctccgcccc cgcgccctgc agtggagcag 1560
ccgcccccaa aggtgtacgt gaccagggtg cggccgggac agcgggcatc cccccgggcc 1620
ccagcgccgc gtgcgccctg gccgcccttc cctggcgtct tcctgcaccc caggcctctg 1680
cccagagtgc agctgcgggc gcccccacgc ccaccccggc cccacggccg caggaccggc 1740
ggcccccagg ccacacagcc gaggccccca gcccgggcgc aggccaccca agggggccgg 1800
gagggccagg cgcgcacgct gggacctgcg gcgcccacag tggactcaaa cttgtcctcc 1860
gaagcgcggc ccgtgacctc cttcctgagc ttgtcccagg tgtccgggcc gcagctgccc 1920
ggggagggcg aagaggagga ggaaggggag gacgatgggg ccccgggcga cgaggccgcg 1980
tcggaggaca gcgaggaggc cgcgggcccg gcgctcggac gctggcgtga ggacgccatc 2040
gactggcagc gcacgttcag cgtgggcgcc gtggacttcg agctgctgcg ctcggactgg 2100
aacgacctgc gatgcaacgt ttcggggaac ctgcagctgc cggaggcgga ggccgtggac 2160
gtgaccgctc agtacatgga gcggctgaac gcgcgccacg gcgggcgctt cgcgcttctg 2220
cgcatcgtga acgtggagaa gcgccgggac tcggcgcgag ggagtcgctt cctgctggag 2280
ctggagctgc aggagcgcgg gggcggccgc ctgcgactgt ccgagtacgt cttcctgcgg 2340
ctgccgggag cccgcgtagg ggatgcagac ggagaaagtc ccgaacccgc tcccgccgcc 2400
tccgtgcgcc ccgacggccg ccccgagctc tgccggccac tgcgcctggc ctggcgccag 2460
gacgtgatgg ttcacttcat cgtgccagtg aaaaaccagg cacggtgggt ggcacagttc 2520
ctggcggaca tggctgcgct gcacgcgcgc accggggact cgcgtttcag cgtcgtcctg 2580
gtggatttcg agagcgagga tatggacgtg gagcgggccc tgcgcgccgc gcgcctgccc 2640
cggtaccagt acctgagacg aaccgggaac ttcgagcgct ccgccgggct gcaggcggga 2700

gtggacgcgg tagaggacgc cagcagcatc gtgttcctct gcgacctgca catccacttc 2760

ccacccaaca tcctggacgg catccgcaag cactgcgtgg agggcaggct ggccttcgcg 2820

cccgtggtca tgcgcctgag ctgcgggagc tcgccccggg acccccacgg ttactgggag 2880

gtgaacggct ttggcctttt tgggatctac aagtcggact ttgaccgggt tggaggaatg 2940

aacacggagg agttccgaga ccagtggggg ggtgaagact gggagctcct ggacagggtc 3000

ctgcaggcag ggctggaggt ggagcggctc cgactgcgga atttctatca ccactaccac 3060

tccaagaggg gcatgtggag cgtccgcagc aggaagggct ctcgcacggg ggcgtcttga 3120

<210> 25
<211> 4080
<212> DNA
<213> Homo sapiens

<400> 25
gcgcctgcgg cgccgcgggc gggtcgcctc ccctcctgta gcccacaccc ttcttaaagc 60

ggcggcggga agatgaggct tcgggagccg ctcctgagcg gcagcgccgc gatgccaggc 120

gcgtccctac agcgggcctg ccgcctgctc gtggccgtct gcgctctgca ccttggcgtc 180

accctcgttt actacctggc tggccgcgac ctgagccgcc tgccccaact ggtcggagtc 240

tccacaccgc tgcagggcgg ctcgaacagt gccgccgcca tcgggcagtc ctccggggag 300

ctccggaccg gaggggcccg gccgccgcct cctctaggcg cctcctccca gccgcgcccg 360

ggtggcgact ccagcccagt cgtggattct ggccctggcc ccgctagcaa cttgacctcg 420

gtcccagtgc cccacaccac cgcactgtcg ctgcccgcct gccctgagga gtccccgctg 480

cttgtgggcc ccatgctgat tgagtttaac atgcctgtgg acctggagct cgtggcaaag 540

cagaacccaa atgtgaagat gggcggccgc tatgccccca gggactgcgt ctctcctcac 600

aaggtggcca tcatcattcc attccgcaac cggcaggagc acctcaagta ctggctatat 660

tatttgcacc cagtcctgca gcgccagcag ctggactatg gcatctatgt tatcaaccag 720

gcgggagaca ctatattcaa tcgtgctaag ctcctcaatg ttggctttca agaagccttg 780

aaggactatg actacacctg ctttgtgttt agtgacgtgg acctcattcc aatgaatgac 840

cataatgcgt acaggtgttt ttcacagcca cggcacattt ccgttgcaat ggataagttt 900

ggattcagcc taccttatgt tcagtatttt ggaggtgtct ctgctctaag taaacaacag 960

tttctaacca tcaatggatt tcctaataat tattggggct ggggaggaga agatgatgac 1020

atttttaaca gattagtttt tagaggcatg tctatatctc gcccaaatgc tgtggtcggg 1080

aggtgtcgca tgatccgcca ctcaagagac aagaaaaatg aacccaatcc tcagaggttt 1140

gaccgaattg cacacacaaa ggagacaatg ctctctgatg gtttgaactc actcacctac 1200

caggtgctgg atgtacagag atacccattg tatacccaaa tcacagtgga catcgggaca 1260

ccgagctagc gttttggtac acggataaga gacctgaaat tagccaggga cctctgctgt 1320

gtgtctctgc caatctgctg ggctggtccc tctcattttt accagtctga gtgacagctc 1380

cccttggctc atcattcaga tggctttcca gatgaccagg acaggtggga tattttgccc 1440

ccaacttggc tcggcatgtg aattcttagc tctgcaaggt gtttatgcct ttgcgggttt 1500
cttgatgtgt tcgcagtgtc acccaagagt cagaactgta gacatcccaa aatttggtgg 1560
ccgtggaaca cattcccggt gatagaattg ctaaattgtc gtgaaatagg ttagaatttt 1620
tctttaaatt atggttttct tattcgcgaa aattcggaga gtgctgctaa aattggattg 1680
gtgtcatctt tttggtagtt gtaatttaac agaaaaacac aaaatttcaa ccattcttaa 1740
tgttacgtcc tccccccacc cccttctttc agtggtatgc aaccactgca atcaatgtgt 1800
catatgtctt ttcttagcaa aaggatttaa aacttgagcc ctggaccttt tgcctatgtg 1860
tgtggattcc agggcaactc tagcatcaga gcaaaagcct tgggtttctc gcattcagtg 1920
gcctatctcc agattgtctg atttctgaat gtaaagttgt tgtgtttttt tttaaatagt 1980
aggtttgtag tattttaaag aaagaacaga tcgagttcta attatgatct agcttgattt 2040
tgtgttgatc caaatttgca tagctgttta atgttaagtc atgacaattt atttttcttg 2100
gcatgctatg taaacttgaa tttcctaagt atttttattc tggtgtttta aatatgggga 2160
ggggtattga gcatttttta gggagaaaaa taaatatatg ctgtagtggc cacaaatagg 2220
cctatgattt agctggcagg ccaggttttc tcaagagcaa aatcaccctc tggccccttg 2280
gcaggtaagg cctcccggtc agcattatcc tgccagacct cggggaggat acctgggaga 2340
cagaagcctc tgcacctact gtgcagaact ctccacttcc ccaaccctcc ccaggtgggc 2400
agggcggagg gagcctcagc ctccttagac tgacccctca ggcccctagg ctggggggtt 2460
gtaaataaca gcagtcaggt tgtttaccag ccctttgcac ctccccaggc agagggagcc 2520
tctgttctgg tgggggccac ctccctcaga ggctctgcta gccacactcc gtggcccacc 2580
ctttgttacc agttcttcct ccttcctctt ttcccctgcc tttctcattc cttccttcgt 2640
ctcccttttt gttcctttgc ctcttgcctg tcccctaaaa cttgactgtg gcactcaggg 2700
tcaaacagac tatccattcc ccagcatgaa tgtgcctttt aattagtgat ctagaaagaa 2760
gttcagccgc acccacaccc caactccctc ccaagaactt cggtcctaaa gcctcctgtt 2820
ccacctcagg ttttcacagg tgctcacacc acagttgagg ctcacacaca ggtctgtctg 2880
tcacaaaccc acctctgttg ggagctattg agccacctgg gatgagatga cacaagacac 2940
tcctaccact gagcgccttt gtccaggtgc cagcctgggc tcaggttcca agactcagct 3000
gcctaatccc agggttgagc cttgtgctcg tgtcggaccc caaaccactg ccctcctggt 3060
accagccctc agtgtggagg ctgagctggt gcctggcccc agtcttatct gtgcctttac 3120
tgctttgcgc atctcagatg ctaacttggt tctttttcca gaaggctttg tattggttaa 3180
aaattatttt ctattgcaga gagcagctgt gactcatgca aaaagtattt tctctgtcag 3240
atccccactc tataccaagg atattattaa aactagaaat gactgcattg agagggagtt 3300
gtgggaaata agaagaatga aagcctctct ttctgtccgc agatcctgac ttttccaaag 3360
tgccttaaaa gaaatcagac aaatgccctg agtggtaact tctgtgttat tttactctta 3420
aaaccaaact ctaccttttc ttgttttttt tttttttttt tttttttttt ttggttacct 3480

tctcattcat gtcaagtatg tggttcattc ttagaaccaa gggaaatact gctcccccca 3540

tttgctgacg tagtgctctc atgggctcac ctgggcccaa ggcacagcca gggcacagtt 3600

aggcctggat gtttgcctgg tccgtgagat gccgcgggtc ctgtttcctt actggggatt 3660

tcagggctgg gggttcaggg agcatttcct tttcctggga gttatgtacc gcgaagtgtg 3720

tcatgtgccg tgcccttttc tgtttctgtg tatcctattg ctggtgactc tgtgtgaact 3780

ggcctttggg aaagatcaga gaggcagagg tggcacagga cagtaaagga gatgctgtgc 3840

tgcctacagc ctggacaggg tctctgctgt actgccaggg gcgggggctc tgcatagcca 3900

ggatgacgcc tttcatgtcc cagagacctg ttgtgctgtg tattttgatt tcctgtgtat 3960

gcaaatgtgt gtatttacca ttgtgtaggg ggctgtgtct gatcttggtg ttcaaaacag 4020

aactgtattt ttgcctttaa aattaaataa tataacgtga ataaatgacc ctaactttgt 4080

<210> 26
<211> 2227
<212> DNA
<213> Homo sapiens

<400> 26
cgcgcgggcc gggcctgccc ctccgaggcg ccgtagcgcg ggagggaggc ggcggcgctg 60

tggtccgtgg gtccgccggt ccgtgggtct gcccggccgc ccggccccgc cctgcccccc 120

ggggcggctc ggctccatgg cccgcggcgg cggcggcggc ggcggcggcg ggaggcgacc 180

cgggggccgc ggtggccgct gaccgggcgg cgggaggcgg cggggccggg ccatggggga 240

cggaaccgtc cgcagccgcc ggagccggga gccctgccca agtcggagcg gcgtcccctg 300

ctgagccccg agcgccgggc cagcagccgg atgcccgggc ccactgggcg ggccagtggc 360

cgcctgcggg atgagcagac tgctgggggg gacgctggag cgcgtctgca aggctgtgct 420

ccttctctgc ctgctgcact tcctcgtggc cgtcatcctc tactttgacg tctacgccca 480

gcacctggcc ttcttcagcc gcttcagtgc ccgaggccct gcccatgccc tccacccagc 540

tgctagcagc agcagcagca gcagcaactg ctcccggccc aacgccaccg cctctagctc 600

cgggctccct gaggtcccca gtgccctgcc cggtcccacg gctcccacgc tgccaccctg 660

tcctgactcg ccacctggtc ttgtgggcag actgctgatc gagttcacct cacccatgcc 720

cctggagcgg gtgcagaggg agaacccagg cgtgctcatg ggcggccgat acacaccgcc 780

cgactgcacc ccagcccaga cggtggcggt catcatcccc tttagacacc gggaacacca 840

cctgcgctac tggctccact atctacaccc catcttgagg cggcagcggc tgcgctacgg 900

cgtctatgtc atcaaccagc atggtgagga caccttcaac cgggccaagc tgcttaacgt 960

gggcttccta gaggcgctga aggaggatgc cgcctatgac tgcttcatct tcagcgatgt 1020

ggacctggtc cccatggatg accgcaacct ataccgctgc ggcgaccaac cccgccactt 1080

tgccattgcc atggacaagt ttggcttccg gcttccctat gctggctact ttggaggtgt 1140

gtcaggcctg agtaaggctc agtttctgag aatcaatggc ttccccaatg agtactgggg 1200

ctggggtggc gaggatgatg acatcttcaa ccggatctcc ctgactggga tgaagatctc 1260

acgcccagac atccgaatcg gccgctaccg catgatcaag cacgaccgcg acaagcataa 1320

cgaacctaac cctcagaggt ttaccaagat tcaaaacacg aagctgacca tgaagcggga 1380

cggcattggg tcagtgcggt accaggtctt ggaggtgtct cggcaaccac tcttcaccaa 1440

tatcacagtg gacattgggc ggcctccgtc gtggccccct cggggctgac actaatggac 1500

agaggctctc ggtgccgaag attgcctgcc agaggactga ccacagcctg gctggcagct 1560

gctctgtgga ggacctccag gactgagact gggctctgtt ttccaagggt cttcactagg 1620

ccccctagct acacctggaa gtttcagaac ccactttggg gggcctcctg cctgggcagg 1680

ctcttcaagt gtggccctct ttggagtcaa ccctccttcc cgaccccctc cccctagccc 1740

agccccagtc actgtcaggg tcgggccagc ccctgcactg cctcgcagag tggcctgggc 1800

taggtcactc cacctctctg tgcctcagtt tccccccctt gagtccccta gggcctggaa 1860

gggtgggagg tatgtctagg gggcagtgtc tcttccaggg ggaattctca gctcttggga 1920

acccccttgc tcccagggga ggggaaacct ttttcattca acattgtagg gggcaagctt 1980

tggtgcgccc cctgctgagg agcagcccca ggaggggacc agaggggatg ctgtgtcgct 2040

gcctgggatc ttggggttgg cctttgcatg ggaggcaggt ggggcttgga tcagtaagtc 2100

tggttcccgc ctccctgtct gagagaggag gcaggagccc cagggccggc ttgtgtttgt 2160

acattgcaca gaaacttgtg tgggtgcttt agtaaaaaac gtgaatggaa aaaaaaaaaa 2220

aaaaaaa 2227

<210> 27
<211> 1949
<212> DNA
<213> Homo sapiens

<400> 27
atgacgcgag accccgcccc cgcagcgccc gcttccaaga tggcggcagc gatgcctgcc 60

cggctgttgg ggtggcggtg acgacaggca gcaaaagacc agctggtccc agattcgctg 120

ctggagtgct ggatggagcc tttctctgcc ctctgtgaca tttccaattt tagataatgc 180

ctcacatctc tgtcccccg ggacccctg gagcccccat gatccctaag aagacagctt 240

gaacctagat ctcaccccca ggatgttgcg gaggctgctg gagcggcctt gcacgctggc 300

cctgcttgtg ggctcccagc tggctgtcat gatgtacctg tcactggggg gcttccgaag 360

tctcagtgcc ctatttggcc gagatcaggg accgacattt gactattctc accctcgtga 420

tgtctacagt aacctcagtc acctgcctgg ggccccaggg ggtcctccag ctcctcaagg 480

tctgccctac tgtccagaac gatctcctct cttagtgggt cctgtgtcgg tgtcctttag 540

cccagtgcca tcactggcag agattgtgga gcggaatccc cgggtagaac cagggggccg 600

gtaccgccct gcaggttgtg agccccgctc ccgaacagcc atcattgtgc ctcatcgtgc 660

ccgggagcac cacctgcgcc tgctgctcta ccacctgcac cccttcttgc agcgccagca 720

gcttgcttat ggcatctatg tcatccacca ggctggaaat ggaacattta acagggcaaa 780

actgttgaac gttggggtgc gagaggccct gcgtgatgaa gagtgggact gcctgttctt 840

gcacgatgtg gacctcttgc cagaaaatga ccacaatctg tatgtgtgtg acccccgggg 900

accccgccat gttgccgttg ctatgaacaa gtttggatac agcctcccgt acccccagta 960

cttcggagga gtctcagcac ttactcctga ccagtacctg aagatgaatg gcttccccaa 1020

tgaatactgg ggctggggtg gtgaggatga cgacattgct accagggtgc gcctggctgg 1080

gatgaagatc tctcggcccc ccacatctgt aggacactat aagatggtga agcaccgagg 1140

agataagggc aatgaggaaa atccccacag atttgacctc ctggtccgta cccagaattc 1200

ctggacgcaa gatgggatga actcactgac ataccagttg ctggctcgag agctggggcc 1260

tctttatacc aacatcacag cagacattgg gactgaccct cggggtcctc gggctccttc 1320

tgggccacgt tacccacctg gttcctccca agccttccgt caagagatgc tgcaacgccg 1380

gcccccagcc aggcctgggc ctctatctac tgccaaccac acagccctcc gaggttcaca 1440

ctgactcctc cttcctgtct accttaatca tgaaaccgaa ttcatggggt tgtattctcc 1500

ccaccctcag ctcctcactg ttctcagagg gatgtgaggg aactgaactc tggtgccgtg 1560

ctagggggta ggggcctctc cctcactgct ggactggagc tgggctcctg tagacctgag 1620

gggtccctct ctctagggtc tcctgtaggg cttatgactg tgaatccttg atgtcatgat 1680

tttatgtgac gattcctagg agtccctgcc cctagagtag gagcagggct ggaccccaag 1740

cccctccctc ttccatggag agaagagtga tctggcttct cctcggacct ctgtgaatat 1800

ttattctatt tatggttccc gggaagttgt ttggtgaagg aagcccctcc ctgggcattt 1860

tctgcctatg ctggaatagc tccctcttct ggtcctggct cagggggctg ggattttgat 1920

atattttcta ataaaggact ttgtctcgc 1949

<210> 28
<211> 2283
<212> DNA
<213> Homo sapiens

<400> 28
tttccgcgtt ctcccctctc cgcgggcagt gctagctcgc cgcggccgcc tccggggacc 60

acctggcttc atgtgtggat ttccacggct cttgcccaga ggcgggtaca ctgtgttcca 120

atgtgccacg gaactcacgc agtggcactt tgtggcttca tgaaggaaga ggcaggccac 180

gcaacacttc ctccccaagc caaggagaag tatcactttt agaggcagag gagcggaagg 240

cagtgggtgt gaccaaaagt gccatttgtt aaagactgtt ggagcagaac tactgagaaa 300

aaccaggcat tgtatcttca gttgtcatca agttcgcaat cagattggaa aagctcaact 360

tgaagctttc ttgcctgcag tgaagcagag agatagatat tattcacgta ataaaaaaca 420

tgggcttcaa cctgactttc cacctttcct acaaattccg attactgttg ctgttgactt 480

tgtgcctgac agtggttggg tgggccacca gtaactactt cgtgggtgcc attcaagaga 540

ttcctaaagc aaaggagttc atggctaatt tccataagac cctcattttg gggaagggaa 600

aaactctgac taatgaagca tccacgaaga aggtagaact tgacaactgc ccttctgtgt 660
ctccttacct cagaggccag agcaagctca ttttcaaacc agatctcact ttggaagagg 720
tacaggcaga aaatcccaaa gtgtccagag gccggtatcg ccctcaggaa tgtaaagctt 780
tacagagggt cgccatcctc gttccccacc ggaacagaga gaaacacctg atgtacctgc 840
tggaacatct gcatcccttc ctgcagaggc agcagctgga ttatggcatc tacgtcatcc 900
accaggctga aggtaaaaag tttaatcgag ccaaactctt gaatgtgggc tatctagaag 960
ccctcaagga agaaaattgg gactgcttta tattccacga tgtggacctg gtacccgaga 1020
atgactttaa cctttacaag tgtgaggagc atcccaagca tctggtggtt ggcaggaaca 1080
gcactgggta caggttacgt tacagtggat attttggggg tgttactgcc ctaagcagag 1140
agcagttttt caaggtgaat ggattctcta acaactactg gggatgggga ggcgaagacg 1200
atgacctcag actcagggtt gagctccaaa gaatgaaaat ttcccggccc ctgcctgaag 1260
tgggtaaata tacaatggtc ttccacacta gagacaaagg caatgaggtg aacgcagaac 1320
ggatgaagct cttacaccaa gtgtcacgag tctggagaac agatgggttg agtagttgtt 1380
cttataaatt agtatctgtg gaacacaatc ctttatatat caacatcaca gtggatttct 1440
ggtttggtgc atgaccctgg gtcttttggt gatgtttgga agaactgatt ctttgtttgc 1500
aataattttg gcctagagac ttcaaatagt agcacacatt aagaacctgt tacagctcat 1560
tgttgagctg aatttttcct ttttgtattt tcttagcaga gctcctggtg atgtagagta 1620
taaaacagtt gtaacaagac agctttctta gtcattttga tcatgagggt taaatattgt 1680
aatatggata cttgaaggac tttatataaa aggatgactc aaaggataaa atgaacgcta 1740
tttgaggact ctggttgaag gagatttatt taaatttgaa gtaatatatt atgggataaa 1800
aggccacagg aaataagact gctgaatgtc tgagagaacc agagttgttc tcgtccaagg 1860
tagaaaggta cgaagataca atactgttat tcatttatcc tgtacaatca tctgtgaagt 1920
ggtggtgtca ggtgagaagg cgtccacaaa agaggggaga aaaggcgacg aatcaggaca 1980
cagtgaactt gggaatgaag aggtagcagg agggtggagt gtcggctgca aaggcagcag 2040
tagctgagct ggttgcagct gctgatagcc ttcaggggag gacctgccca ggtatgcctt 2100
ccagtgatgc ccaccagaga atacattctc tattagtttt taaagagttt ttgtaaaatg 2160
attttgtaca agtaggatat gaattagcag tttacaagtt tacatattaa ctaataataa 2220
atatgtctat caaatacctc tgtagtaaaa tgtgaaaaag caaaaaaaaa aaaaaaaaaa 2280
aaa 2283

<210> 29
<211> 4646
<212> DNA
<213> Homo sapiens

<400> 29
gggaggcggt ggccgaggcc caggcggtgg cggcggcggc ccaggaggcg gcggacgggg 60
agctgcggga gcaggcccgg gcctggctct ctagcggccg cctggctgca gcatgcgcgc 120

ccgccggggg ctgctgcggc tgccgcgccg ctcgctgctc gccgcgctct tcttcttttc 180
tctctcgtcc tcgctgctgt acttcgtcta tgtggcgccc ggcatagtga acacctacct 240
cttcatgatg caagcccaag gcattctgat ccgggacaac gtgagaacaa tcggtgctca 300
ggtttatgag caggtgcttc ggagtgctta tgccaagagg aacagcagtg taaatgactc 360
agattatcct cttgacttga accacagtga aaccttcctg caaactacaa catttcttcc 420
tgaagacttc acctactttg caaaccatac ctgccctgaa agactccctt ccatgaaggg 480
cccaatagac ataaacatga gtgaaattgg aatggattac attcatgaac tcttctccaa 540
agacccaacc atcaagctcg gaggtcactg gaagccttct gattgcatgc ctcggtggaa 600
ggtggcgatc cttatcccct tccggaaccg ccacgagcac ctcccagtcc tgttcagaca 660
cctgcttccc atgctccagc gccagcgctt gcagtttgca ttttatgtgg ttgaacaagt 720
tggtacccaa ccctttaatc gagccatgct tttcaacgtt ggctttcaag aggcaatgaa 780
agacttggat tgggactgtt tgatttttca tgatgtagat cacataccgg aaagtgatcg 840
caactattat ggatgtggac agatgccgag gcattttgca accaaattgg ataagtatat 900
gtatctgctt ccttataccg agttctttgg cggagtgagt ggcttaacag tggaacaatt 960
tcggaaaatc aatggctttc ctaatgcttt ctggggttgg ggtggagaag atgacgacct 1020
ctggaacaga gtacagaatg caggctattc tgtgagccgg ccagagggtg acacaggaaa 1080
gtacaagtcc attcctcatc accatcgagg agaagtccag tttcttggaa ggtatgctct 1140
gctgaggaag tcaaaagaac ggcaagggct ggatggcctc aacaacctga actactttgc 1200
aaacatcaca tacgacgcct tgtataaaaa cataactgtc aacctgacac ccgagctggc 1260
tcaggtgaac gagtactgag aggagagaat gtacgtttgc tttacccacc gccaccaaga 1320
aagcagtccg atgagatttt tttttggagg ggggagggtc tacacagcaa gagaacagaa 1380
atactgtgtc tcatgaagga tcacagagtt cagggggaaa atgtgacagc acacgcacaa 1440
acgccttcac tggatcagcc gctggaactg agggagtgag cttggggact tccttcgtca 1500
gcactggctt tctgttttca caagacagac gtctgtcccg ctgctctctc cccatctcct 1560
accccacatc ctgtcttagc cgcagtctcc agaacccatg atgaactgtg atctgccgtg 1620
gtcctgccgt ggtcctgccg tggagcctgt ccctacacat gaccttggag cctcttggcc 1680
ttcagagcag aggcaaaccc accacagggc agctgcgttt taggaagagc aaatgaaact 1740
ccacaccatt cttctagatc tctggtgttc tctttggttt cattttttta aaaaattacc 1800
ttctttgggt ggggattgag ggtggagggg agggtgtttg ggaaagataa atagacataa 1860
atatataaca atcacttctt gaagaagtat aattgtaaat aagccatgta aaatgccttt 1920
ttaaaattta attttctagc tggctccaat tcaaattgag gatttatgta ttaggccact 1980
tacttggttg gcaagtgcag gaactcagtt aaaatgcagt tgaagaatgt catctcccga 2040
attgctgtca ctttggcgag ggagtggata tagggcatgt cacaaaagaa caaaataacc 2100
cgacctttat tgctgggagc tggcttctgt ccctttcttc ccccccccac gagtcttgcc 2160

cttgacttct gctctggatt cactcttccc tgtcggccgc gcatgtgctc atcccactct 2220
ccgctaagcg ggaggctgct gttagagcag gctgcttcct gcctaaagca ggcccttcgg 2280
ggctcgctgc acacacatct ctggctctcc aggcttcgtg ttctgtcttt tcatcagcat 2340
ggcggggcgg ggggcggggg gcgggggtgt gtatgggaat ccctccccct cttacttttt 2400
ctcttgtgga acttggccac agtttctgaa caatgtgcct acattaccag ctggcttcag 2460
tgattcctct gtgtcccttt ttggtttctg gaaagattct ttgtcaacat tagtaactga 2520
tacatagaac caaggagcac tcaaataggg agccaggagc cagggagctg gtgacacttg 2580
tgtgctgtgg ggcagctggg atccaggtaa gaccggattg aagctttgaa attagactaa 2640
caaagctcca gacagcaaga gcccaggtgc actgctcaca cccccacctg cattttgaag 2700
tcatattatt ttttgttttg ttttttaaga cggtctggct ctgtcgccta agctggagtg 2760
tggtggcacg atcacagctc actgcagcct ccatctccta ggctcaagcc attttcccac 2820
ctcagcctcc cgagtagctg ggactacagg tgcacaccac cacacctggc taattttttg 2880
tatttttagt agagacaggg gtttcttcca tgttgcccag gctggtctcg aactcctgga 2940
ctcaagcaat ccgcccacct tgacttccca aagtgctggg attatgggcg ggtgtgagcc 3000
attgcgccca gccttgaagt catgttctaa attgtatttg aatttgtgcc tctttgtttt 3060
tccccaaacc aaagccctca aattgtagtc tctgtcggct tctgcagaat tctggaaaat 3120
gccagttttc ctcccccgcc cttgttttcc ataaaacata tttatatatt gtgatgagga 3180
gtactttctg aagagtactt cgtatttttt tttaattgcc ttgtttgcct tcaacttcct 3240
tgattttcat agtttacatg ggtgtgtgta ggggtgtgtg tgtgtatgtg tgtgggttag 3300
ggcttttttc gttgcatgtg atggttctgt ggacatatga tccccacaaa ctgtgggagt 3360
gattggccag gccttgtttt gtttgtttgt ttgtttgtgt tttgttcttt ttgaagaata 3420
gagtggtatt tagaaaataa attgcattgc aaagctctta tcggctcata tgagagagca 3480
ggttcctgcc cttgaaaatg ccggtaagct atagcatatg ttttttaaga cttaagcatt 3540
tcatgcttta aaataccttc acaagtgaac attacacaca gaagttcatt tggttttcct 3600
ttgttttatg gtgcatatag caataaagac ccccctccac cctgcaaccc ccatccccca 3660
ccgggccttt gtccctgcct tggcttttct ccccttctca ttctcctctc ccctttcctc 3720
actgaaggct gtgagttgct ttcaatgtga caacactatg atgtcatttg gaaggatttg 3780
ccaggacaga ctgattctga gtcctgggtg ccgtatgtgt atgcggcagt gttgtcaggc 3840
gatcttgttt gaagctctat gttgccataa ttaccatcaa gtacacactg ttggcaaaag 3900
gctaacacct gactttagaa aatgctgatt tgagaacaaa aggaaaggtc ttttttcact 3960
gcttaaagtg gggtcacttt gatacctttg cggtcatgtc tgtgtctgat gagtgtagaa 4020
tctctggatg tgcactgtca gtcatgtgtc caccaggcct cgaatatcat atgggaaatg 4080
tcatagttaa aaacgtacag ccaggcccgt gtgctgttaa tagtgtgaaa ttgtcatgtt 4140
aaaaaaaaaa acaggaacca aatgtgacct tgtgcatata ttggtagctg aaaatcttca 4200

aggctactga tgggtggccc cttaatcttg tctttgattg ctgtgtgcag ggaaaggtgt 4260

ccccgtttgt tcatgctgtt ttggggggtg ggggggtatt tgcaagaata ctcattttga 4320

cataataggt cctcttgtca gagatcctct accacagaca ttaatagctg agcaggagcc 4380

acatggattg attgtatcca ctcaccattg acgatggcat tgagcgtagc tagcttattt 4440

ccatcactac gtgtttttga gcttgctctt acgttttaag aggtgccagg ggtacatttt 4500

tgcactgaaa tctaaagatg ttttaaaaaa cacttttcac aaaaatagtc ctttgtcatt 4560

acattattta ctcatgtgtt tgtacatttt tgtatgttaa tttatgaatg attttttcag 4620

taaaaaatac atattcaaga accaaa 4646

<210> 30
<211> 3830
<212> DNA
<213> Homo sapiens

<400> 30
tccacctctt gtccctcggc ctccgcgcct cccctccccc gactctctgg cctcccattc 60

attccgtccg ctccctccca tttcctcttt tccgctcggg ccctctcccc agagcctccc 120

cgcgcggacc ccgctgcagt gtcggggttc tcagcacatt tatggaagtt tagggcctgg 180

acagtggccc cgagtccggc ctgagagcgc agcctgggct gctggcaggg aagaggaaga 240

tgtctgtgct caggcggatg atgcgggttt ccaatcgctc tctcctcgcc ttcatcttct 300

ttttctccct ctcttcgtcc tgtctgtact tcatctatgt ggccccaggc atcgccaaca 360

catatctctt tatggtacaa gctcgaggta taatgttgag agaaaatgtg aaaacaatag 420

gtcatatgat caggctgtac acaaataaaa acagtacgct caacggtaca gattatcccg 480

aaggcaataa ttcaagtgat tatcttgttc aaacaacaac gtatctcccg gaaaacttca 540

catactcacc atacctcccc tgtccagaaa agctgcctta tatgcgagga ttcctcaatg 600

tcaatgtaag cgaagtcagt tttgatgaaa ttcatcaact cttctccaag gatttagata 660

ttgagccagg gggtcattgg aggccaaaag actgtaaacc cagatggaag gtggcagttc 720

tcattccttt ccgtaatcgc catgaacatc ttccaatttt tttcttacat ctgattccaa 780

tgctccagaa gcagcggctg gaatttgcgt tttatgtcgt tgaacagact ggcacacaac 840

cttttaaccg tgcgatgctt ttcaatgtgg gcttcaaaga ggccatgaaa gacagtgtct 900

gggactgtgt aatcttccac gatgtggatc atctacctga aaatgaccgg aactattacg 960

gatgtggaga aatgccacgt cattttgctg caaagctgga taaatacatg tatattcttc 1020

catataaaga attttttggt ggtgtaagtg ggctgacagt ggaacaattt agaaagatca 1080

atggttttcc taatgccttc tggggatggg gaggagaaga tgatgacctt tggaacagag 1140

ttcactatgc tggatataat gtaaccagac cagagggaga cttaggaaaa tacaagtcaa 1200

ttcctcatca ccatagaggt gaagtccagt ttttaggacg gtataaatta ctaaggtatt 1260

ccaaggagcg tcagtacatc gatggactga acaatttaat atataggcca aaaatactgg 1320

ttgataggtt gtatacaaac atatctgtaa acctcatgcc agagttagct ccaatcgaag 1380
actattaaaa gaagtggctg tcgtggcaag gtagaccaca atgctggatc ataaacttgg 1440
agcgcgctct tagtggaggt cagtgattgg ctgtgtcaca gtgccctttt ctctgagaag 1500
agccagcagt ccacagtgtt tacagagtgg gactatatac agtcaccctt ctctcacccg 1560
tcctccctgc tctgagacac acccctgtgg cgagcaccgc agaaatggga agccactact 1620
taagatcgga agttaagaga gctccctccg aagagaaaat ttttatacta aaatctataa 1680
tttaattcaa gagaatgctt ttatttccgt ttaaacatat tttgtatata tgtgatataa 1740
attaatgtgt gcaaattgtt taaaaattag atgtgttgca gttttgcatg taatcggtta 1800
tacctttatt ggacttttat agacattttt tatttgcatg aaaaaaactc actaaattta 1860
catcactaaa caaaggttaa cccttgtgtg aaatgaagga actgtcaata attgacagcc 1920
aactaataca gtaaactgtt atactagttt tgagctttag acctcagcct tttgtgtgga 1980
agaagtcaca gctttcttag gctttaaagg aaaagaagga aggacttaaa tagcttttct 2040
tcctaccggg attacctatg tttttccttg cttgcaatct catctgattt tgctagaaat 2100
cacaaccata ttgtttatgc atattgcatg agtattacca agaaaaatct taaaagttgt 2160
gatgtgacat gatataaagg atctctttat gttaaatgtc tttccatgta cctctggtgt 2220
gtcagggatt ttgtgcctca aaaaatgttt ccaaggttgt gtgtttatac tgtgtatttt 2280
ttttaaattc acggtgaaca gcacttttat tatttccagt tcagaagagc caaaaaaaag 2340
tatcttcatt taaaaagaaa tcaagtcagt ttttttaaaa gaatgatcta tggaagaaaa 2400
cccaatagta cttataatat acaaatgaat tatactatta gataagaatt aaatattcta 2460
tttttataaa gataaatact tagtagataa aaacaaattt taagtttagt tttcagacat 2520
agaatttcac ttttggaggg gacccttgaa tgttagtttc attcaaacta tattttgttt 2580
ttttcacttt taagagatta tatctctggt gttaattact aaaatttctt ttctaaaagt 2640
ataatttaat agaattatct taaaatacat gatcagtatt cagtgcgctg atattataat 2700
gtcttctata tgtagtatct aaaagcataa tttaatagca ttgtcttaaa atacatgatg 2760
agttatttgg tgcactgatg ccataatgtc ttctatatgt aatatcttac atgtttgcat 2820
gagtgtcagg gctttgtcca gttatcttca tatgactgtt ctctgagaaa aggtcttact 2880
acctgatatc aagtacatat cttaattttg gatgaatttt tccacagtaa agccgaacag 2940
tattcttttt tgaaaattag caattataaa gattgtttaa aatatcttga tattttcaca 3000
aattttaaac aaaaaattaa atatctataa agtgaatgtt caacaacaat gatgagcaat 3060
agaaatctat atatgagaaa cgtttggata atatttgcct caaggattgt ttacctaaaa 3120
cagaactatg gttatttatc tcagattgta agttttattt tgaagtgtca tttttatggc 3180
aatattgata acacaaatga ccacaaacta aatcataaaa taattgaatc cttactttga 3240
gaaaatggat agcttctgtg ttatgatacc aaacaaaata ttttcatatt tatttcttag 3300
aactatttcc aaaatattta tatttcagtt tttttatggt actcgaaaaa tcaaaccttc 3360

tgaaaattat gatatttgta cttcatttgt gtattagttc attttctgtt tctattacat 3420

agtttttaaa gtcaatgaaa gagattgaga atgttaacat ttgttgatca atgtggagct 3480

gtccacattc ggtatctttt catttttata tatattttac agtttcaaga aaacttagta 3540

ctactagtat ctgccttatt ttcttaagtc tgatgttaat ttttgtttca ggagtgagat 3600

ataaacattt tgcttttttt cagttttgaa atatagtttg tcatcctgtg aaaaccacct 3660

cgtttgcttt ttgtttgttt gttttgcttt tttagtgtag acctaaaatt atttttgcct 3720

ataaacagtg aacttaatat gtttctgtga gagtgacaga gctgtgcccg tttatcttca 3780

ttgtattaat tattacataa agattaacaa atacattgca aatgcaaatt 3830

<210> 31
<211> 1669
<212> DNA
<213> Homo sapiens

<400> 31
ctgcgagcgc ctgccccatg cgccgccgcc tctccgcacg atgttcccct cgcggaggaa 60

agcggcgcag ctgccctggg aggacggcag gtccgggttg ctctccggcg gcctccctcg 120

gaagtgttcc gtcttccacc tgttcgtggc ctgcctctcg ctgggcttct tctccctact 180

ctggctgcag ctcagctgct ctggggacgt ggcccgggca gtcaggggac aagggcagga 240

gacctcgggc cctccccgcg cctgcccccc agagccgccc cctgagcact gggaagaaga 300

cgcatcctgg ggcccccacc gcctggcagt gctggtgccc ttccgcgaac gcttcgagga 360

gctcctggtc ttcgtgcccc acatgcgccg cttcctgagc aggaagaaga tccggcacca 420

catctacgtg ctcaaccagg tggaccactt caggttcaac cgggcagcgc tcatcaacgt 480

gggcttcctg gagagcagca acagcacgga ctacattgcc atgcacgacg ttgacctgct 540

ccctctcaac gaggagctgg actatggctt tcctgaggct gggcccttcc acgtggcctc 600

cccggagctc caccctctct accactacaa gacctatgtc ggcggcatcc tgctgctctc 660

caagcagcac taccggctgt gcaatgggat gtccaaccgc ttctggggct ggggccgcga 720

ggacgacgag ttctaccggc gcattaaggg agctgggctc cagcttttcc gcccctcggg 780

aatcacaact gggtacaaga catttcgcca cctgcacgac ccagcctggc ggaagaggga 840

ccagaagcgc atcgcagctc aaaaacagga gcagttcaag gtggacaggg agggaggcct 900

gaacactgtg aagtaccatg tggcttcccg cactgccctg tctgtgggcg ggcccccctg 960

cactgtcctc aacatcatgt tggactgtga caagaccgcc acaccctggt gcacattcag 1020

ctgagctgga tggacagtga ggaagcctgt acctacaggc catattgctc aggctcagga 1080

caaggcctca ggtcgtgggc ccagctctga caggatgtgg agtggccagg accaagacag 1140

caagctacgc aattgcagcc acccggccgc caaggcaggc ttgggctggg ccaggacacg 1200

tggggtgcct gggacgctgc ttgccatgca cagtgatcag agagaggctg gggtgtgtcc 1260

tgtccgggac cccccctgcc ttcctgctca ccctactctg acctccttca cgtgcccagg 1320

cctgtgggta gtggggaggg ctgaacagga caacctctca tcacccccac ttttgttcct 1380

tcctgctggg ctgcctcgtg cagagacaca gtgtaggggc catgcagctg gcgtaggtgg 1440
cagttgggcc tggtgagggt taggacttca gaaaccagag cacaagcccc acagaggggg 1500
aacagccagc accgctctag ctggttgttg ccatgccgga atgtgggcct agtgttgcca 1560
gatcttctga tttttcgaaa gaaactagaa tgctggattc ttaagtgata tcttctgatt 1620
ttttaaatga tagcacctaa atgaaacttt caaaaagtaa aaaaaaaaa 1669

<210> 32
<211> 1794
<212> DNA
<213> Homo sapiens

<400> 32
tacagctttg attttataac atcctgctaa tttttgttct tacagaaata cactttcggg 60
aaatggcctc taaatcctgg ctgaattttt taaccttcct ctgtggatca gcaataggat 120
ttcttttatg ttctcagcta tttagtattt tgttgggaga aaaggttgac acccagccta 180
atgttcttca taatgatcct catgcaaggc attcagatga taatggacag aatcatctag 240
aaggacaaat gaacttcaat gcagattcta gccaacataa agatgagaac acagacattg 300
ctgaaaacct ctatcagaaa gttagaattc tttgctgggt tatgaccggc cctcaaaacc 360
tagagaaaaa ggccaaacac gtcaaagcta cttgggccca gcgttgtaac aaagtgttgt 420
ttatgagttc agaagaaaat aaagacttcc ctgctgtggg actgaaaacc aaagaaggca 480
gagatcaact atactggaaa acaattaaag cttttcagta tgttcatgaa cattatttag 540
aagatgctga ttggtttttg aaagcagatg atgacacgta tgtcatacta gacaatttga 600
ggtggcttct ttcaaaatac gaccctgaag aacccattta ctttgggaga agatttaagc 660
cttatgtaaa gcagggctac atgagtggag gagcaggata tgtactaagc aaagaagcct 720
tgaaaagatt tgttgatgca tttaaaacag acaagtgtac acatagttcc tccattgaag 780
acttagcact ggggagatgc atggaaatta tgaatgtaga agcaggagat tccagagata 840
ccattggaaa agaaactttt catccctttg tgccagaaca ccatttaatt aaaggttatc 900
tacctagaac gttttggtac tggaattaca actattatcc tcctgtagag ggtcctggtt 960
gctgctctga tcttgcagtt tcttttcact atgttgattc tacaaccatg tatgagttag 1020
aatacctcgt ttatcatctt cgtccatatg gttatttata cagatatcaa cctaccttac 1080
ctgaacgtat actaaaggaa attagtcaag caaacaaaaa tgaagataca aaagtgaagt 1140
taggaaatcc ttgaaagaaa atcatgaatg aacaaaggta atatgtctag cactgcactg 1200
aaaaaggact tctgcatttc tgacatagaa cactggaatc ccagtgagga attctaagtg 1260
aacattcctt atagaaacct ttcacatgaa tgactataaa ctgaagcttt aaatgagctg 1320
tgaagtgtgt taaaatgtgt tttgatacag taatatataa atatgtctat atatatgagg 1380
aacttgtgtt ttttaaatgg tggccaggta gaggaactag aaaagagatt ttgttgcctg 1440
ttttctgacc atctgtgtta ttgtcactga gaaactaaaa tagtaaattt actaaaacta 1500

cactgcacca tgttagtaat aaacagatct gccttaaaga aaagaaaatt ttagaaagaa 1560

atattgttgc tcagtgttgt taatatagct caagaattga gtttatattt gcagtatgct 1620

ataaatgata cccccctacc acacccacac acacagtttt tgtctaatga aaatgttgct 1680

gtgattattt ataattggta gtatttcttc cagaagaagc taaaataaga ctggcactta 1740

ccctgaagtg cattaataaa accacacttt aaaattaaaa aaaaaaaaaa aaaa 1794

<210> 33
<211> 1838
<212> DNA
<213> Homo sapiens

<400> 33
gccttcgcgg cgctcgtttc ctggcgctcc ccacctttag cgagaccaac gagagaacac 60

cgcctgcagc tagaacagcc tggtcaggag cgtaacggag tggtgcgcca acgtgagagg 120

aaacccgtgc gcggctgcgc tttcctgtcc ccaagccgtt ctagacgcgg atgaagtgca 180

aaacaaactt ctccatagag gagttgttgc aaagttccag tttataccaa acagtaatca 240

gattccattg gaagctaaag attttgagag ccttttgtac tatatgcaac taacttgatt 300

tcaagcttgg gaacctttta aaaaaaacat taaaagcaaa atgaaaaatg ctttctgaaa 360

gcagctcctt tttgaagggt gtgatgcttg gaagcatttt ctgtgctttg atcactatgc 420

taggacacat taggattggt catggaaata gaatgcacca ccatgagcat catcacctac 480

aagctcctaa caaagaagat atcttgaaaa tttcagagga tgagcgcatg gagctcagta 540

agagctttcg agtatactgt attatccttg taaaacccaa agatgtgagt ctttgggctg 600

cagtaaagga gacttggacc aaacactgtg acaaagcaga gttcttcagt tctgaaaatg 660

ttaaagtgtt tgagtcaatt aatatggaca caaatgacat gtggttaatg atgagaaaag 720

cttacaaata cgcctttgat aagtatagag accaatacaa ctggttcttc cttgcacgcc 780

ccactacgtt tgctatcatt gaaaacctaa agtatttttt gttaaaaaag gatccatcac 840

agcctttcta tctaggccac actataaaat ctggagacct tgaatatgtg ggtatggaag 900

gaggaattgt cttaagtgta gaatcaatga aaagacttaa cagccttctc aatatcccag 960

aaaagtgtcc tgaacaggga gggatgattt ggaagatatc tgaagataaa cagctagcag 1020

tttgcctgaa atatgctgga gtatttgcag aaaatgcaga agatgctgat ggaaaagatg 1080

tatttaatac caaatctgtt gggctttcta ttaaagaggc aatgacttat caccccaacc 1140

aggtagtaga aggctgttgt tcagatatgg ctgttacttt taatggactg actccaaatc 1200

agatgcatgt gatgatgtat ggggtatacc gccttagggc atttgggcat attttcaatg 1260

atgcattggt tttcttacct ccaaatggtt ctgacaatga ctgagaagtg gtagaaaagc 1320

gtgaatatga tctttgtata ggacgtgtgt tgtcattatt tgtagtagta actacatatc 1380

caatacagct gtatgtttct ttttcttttc taatttggtg gcactggtat aaccacacat 1440

taaagtcagt agtacatttt taaatgaggg tggttttttt ctttaaaaca catgaacatt 1500

gtaaatgtgt tggaaagaag tgttttaaga ataataattt tgcaaataaa ctattaataa 1560

atattatatg tgataaattc taaattatga acattagaaa tctgtggggc acatattttt 1620

gctgattggt taaaaaattt taacaggtct ttagcgttct aagatatgca aatgatatct 1680

ctagttgtga atttgtgatt aaagtaaaac ttttagctgt gtgttccctt tacttctaat 1740

actgatttat gttctaagcc tccccaagtt ccaatggatt tgccttctca aaatgtacaa 1800

ctaagcaact aaagaaaatt aaagtgaaag ttgaaaaa 1838

<210> 34
<211> 3260
<212> DNA
<213> Homo sapiens

<400> 34
ccatcccata gtgagggaag acacgcggaa acaggcttgc acccagacac gacaccatgc 60

atctcctcgg cccctggctc ctgctcctgg ttctagaata cttggctttc tctgactcaa 120

gtaaatgggt ttttgagcac cctgaaaccc tctacgcctg ggagggggcc tgcgtctgga 180

tcccctgcac ctacagagcc ctagatggtg acctggaaag cttcatcctg ttccacaatc 240

ctgagtataa caagaacacc tcgaagtttg atgggacaag actctatgaa agcacaaagg 300

atgggaaggt tccttctgag cagaaaaggg tgcaattcct gggagacaag aataagaact 360

gcacactgag tatccacccg gtgcacctca atgacagtgg tcagctgggg ctgaggatgg 420

agtccaagac tgagaaatgg atggaacgaa tacacctcaa tgtctctgaa aggccttttc 480

cacctcatat ccagctccct ccagaaattc aagagtccca ggaagtcact ctgacctgct 540

tgctgaattt ctcctgctat gggtatccga tccaattgca gtggctccta gagggggttc 600

caatgaggca ggctgctgtc acctcgacct ccttgaccat caagtctgtc ttcacccgga 660

gcgagctcaa gttctcccca cagtggagtc accatgggaa gattgtgacc tgccagcttc 720

aggatgcaga tgggaagttc ctctccaatg acacggtgca gctgaacgtg aagcacaccc 780

cgaagttgga gatcaaggtc actcccagtg atgccatagt gagggagggg gactctgtga 840

ccatgacctg cgaggtcagc agcagcaacc cggagtacac gacggtatcc tggctcaagg 900

atgggacctc gctgaagaag cagaatacat tcacgctaaa cctgcgcgaa gtgaccaagg 960

accagagtgg gaagtactgc tgtcaggtct ccaatgacgt gggcccggga aggtcggaag 1020

aagtgttcct gcaagtgcag tatgccccgg aaccttccac ggttcagatc ctccactcac 1080

cggctgtgga gggaagtcaa gtcgagtttc tttgcatgtc actggccaat cctcttccaa 1140

caaattacac gtggtaccac aatgggaaag aaatgcaggg aaggacagag gagaaagtcc 1200

acatcccaaa gatcctcccc tggcacgctg ggacttattc ctgtgtggca gaaaacattc 1260

ttggtactgg acagaggggc ccgggagctg agctggatgt ccagtatcct cccaagaagg 1320

tgaccacagt gattcaaaac cccatgccga ttcgagaagg agacacagtg accctttcct 1380

gtaactacaa ttccagtaac cccagtgtta cccggtatga atggaaaccc catggcgcct 1440

gggaggagcc atcgcttggg gtgctgaaga tccaaaacgt tggctgggac aacacaacca 1500

tcgcctgcgc acgttgtaat agttggtgct cgtgggcctc ccctgtcgcc ctgaatgtcc 1560
agtatgcccc ccgagacgtg agggtccgga aaatcaagcc cctttccgag attcactctg 1620
gaaactcggt cagcctccaa tgtgacttct caagcagcca ccccaaagaa gtccagttct 1680
tctgggagaa aaatggcagg cttctgggga aagaaagcca gctgaatttt gactccatct 1740
ccccagaaga tgctgggagt tacagctgct gggtgaacaa ctccatagga cagacagcgt 1800
ccaaggcctg gacacttgaa gtgctgtatg cacccaggag gctgcgtgtg tccatgagcc 1860
cgggggacca agtgatggag gggaagagtg caaccctgac ctgtgagagt gacgccaacc 1920
ctcccgtctc ccactacacc tggtttgact ggaataacca aagcctcccc caccacagcc 1980
agaagctgag attggagccg gtgaaggtcc agcactcggg tgcctactgg tgccagggga 2040
ccaacagtgt gggcaagggc cgttcgcctc tcagcaccct tactgtctac tatagcccgg 2100
agaccatcgg caggcgagtg gctgtgggac tcgggtcctg cctcgccatc ctcatcctgg 2160
caatctgtgg gctcaagctc cagcgacgtt ggaagaggac acagagccag caggggcttc 2220
aggagaattc cagcggccag agcttctttg tgaggaataa aaaggttaga agggcccccc 2280
tctctgaagg cccccactcc ctgggatgct acaatccaat gatggaagat ggcattagct 2340
acaccaccct gcgctttccc gagatgaaca taccacgaac tggagatgca gagtcctcag 2400
agatgcagag acctccccgg acctgcgatg acacggtcac ttattcagca ttgcacaagc 2460
gccaagtggg cgactatgag aacgtcattc cagattttcc agaagatgag gggattcatt 2520
actcagagct gatccagttt ggggtcgggg agcggcctca ggcacaagaa aatgtggact 2580
atgtgatcct caaacattga cactggatgg gctgcagcag aggcactggg ggcagcgggg 2640
gccagggaag tccccgagtt tccccagaca ccgccacatg gcttcctcct gcgtgcatgt 2700
gcgcacacac acacacacac gcacacacac acacacacac tcactgcgga gaaccttgtg 2760
cctggctcag agccagtctt tttggtgagg gtaaccccaa acctccaaaa ctcctgcccc 2820
tgttctcttc cactctcctt gctacccaga aatcatctaa atacctgccc tgacatgcac 2880
acctcccctg ccccaccagc ccactggcca tctccacccg gagctgctgt gtcctctgga 2940
tctgctcgtc attttccttc ccttctccat ctctctggcc ctctacccct gatctgacat 3000
ccccactcac gaatattatg cccagtttct gcctctgagg gaaagcccag aaaaggacag 3060
aaacgaagta gaaaggggcc cagtcctggc ctggcttctc ctttggaagt gaggcattgc 3120
acggggagac gtacgtatca gcggcccctt gactctgggg actccgggtt tgagatggac 3180
acactggtgt ggattaacct gccagggaga cagagctcac aataaaaatg gctcagatgc 3240
cacttcaaag aaaaaaaaaa 3260

<210> 35
<211> 1466
<212> DNA
<213> Homo sapiens

<400> 35
tctgctcaca caggaagccc tggaagctgc ttcctcagac atgccgctgc tgctactgct 60

gcccctgctg tgggcagggg ccctggctat ggatccaaat ttctggctgc aagtgcagga 120

gtcagtgacg gtacaggagg gtttgtgcgt cctcgtgccc tgcactttct tccatcccat 180

accctactac gacaagaact ccccagttca tggttactgg ttccgggaag gagccattat 240

atccagggac tctccagtgg ccacaaacaa gctagatcaa gaagtacagg aggagactca 300

gggcagattc cgcctccttg gggatcccag taggaacaac tgctccctga gcatcgtaga 360

cgccaggagg agggataatg gttcatactt ctttcggatg gagagaggaa gtaccaaata 420

cagttacaaa tctccccagc tctctgtgca tgtgacagac ttgacccaca ggcccaaaat 480

cctcatccct ggcactctag aacccggcca ctccaaaaac ctgacctgct ctgtgtcctg 540

ggcctgtgag cagggaacac ccccgatctt ctcctggttg tcagctgccc ccacctccct 600

gggccccagg actactcact cctcggtgct cataatcacc ccacggcccc aggaccacgg 660

caccaacctg acctgtcagg tgaagttcgc tggagctggt gtgactacgg agagaaccat 720

ccagctcaac gtcacctatg ttccacagaa cccaacaact ggtatctttc caggagatgg 780

ctcagggaaa caagagacca gagcaggagt ggttcatggg gccattggag gagctggtgt 840

tacagccctg ctcgctcttt gtctctgcct catcttcttc atagtgaaga cccacaggag 900

gaaagcagcc aggacagcag tgggcaggaa tgacacccac cctaccacag ggtcagcctc 960

cccgaaacac cagaagaagt ccaagttaca tggccccact gaaacctcaa gctgttcagg 1020

tgccgcccct actgtggaga tggatgagga gctgcattat gcttccctca actttcatgg 1080

gatgaatcct tccaaggaca cctccaccga atactcagag gtcaggaccc agtgaggaac 1140

ccacaagagc atcaggctca gctagaagat ccacatcctc tacaggtcgg ggaccaaagg 1200

ctgattcttg gagatttaac accccacagg caatgggttt atagacatta tgtgagtttc 1260

ctgctatatt aacatcatct tagactttgc aagcagagag tcgtggaatc aaatctgtgc 1320

tctttcattt gctaagtgta tgatgtcaca caagctcctt aaccttccat gtctccattt 1380

tcttctctgt gaagtaggta taagaagtcc tatctcatag ggatgctgtg agcattaaat 1440

aaaggtacac atggaaaaca ccagtc 1466

<210> 36
<211> 1702
<212> DNA
<213> Homo sapiens

<400> 36
agactcaaca agagctccag caaagacttt cactgtagct tgacttgacc tgagattaac 60

tagggaatct tgagaataaa gatgagctct gaaaattgtt tcgtagcaga gaacagctct 120

ttgcatccgg agagtggaca agaaaatgat gccaccagtc cccatttctc aacacgtcat 180

gaagggtcct tccaagttcc tgtcctgtgt gctgtaatga atgtggtctt catcaccatt 240

ttaatcatag ctctcattgc cttatcagtg ggccaataca attgtccagg ccaatacaca 300

ttctcaatgc catcagacag ccatgtttct tcatgctctg aggactgggt tggctaccag 360

aggaaatgct actttatttc tactgtgaag aggagctgga cttcagccca aaatgcttgt 420

tctgaacatg gtgctactct tgctgtcatt gattctgaaa aggacatgaa ctttctaaaa 480

cgatacgcag gtagagagga acactgggtt ggactgaaaa aggaacctgg tcacccatgg 540

aagtggtcaa atggcaaaga atttaacaac tggttcaacg ttacagggtc tgacaagtgt 600

gtttttctga aaaacacaga ggtcagcagc atggaatgtg agaagaattt atactggata 660

tgtaacaaac cttacaaata ataaggaaac atgttcactt attgactatt atagaatgga 720

actcaaggaa atctgtgtca gtggatgctg ctctgtggtc cgaagtcttc catagagact 780

ttgtgaaaaa aaattttata gtgtcttggg aattttcttc caaacagaac tatggaaaaa 840

aaggaagaaa ttccaggaaa atctgcactg tgggctttta ttgccatgag ctagaagcat 900

cacaggttga ccaataacca tgcccaagaa tgagaagaat gactatgcaa cctttggatg 960

cactttatat tattttgaat ccagaaataa tgaaataact aggcgtggac ttactattta 1020

ttgctgaatg actaccaaca gtgagagccc ttcatgcatt tgcactactg gaaggagtta 1080

gatgttggta ctagatactg aatgtaaaca aaggaattat ggctggtaac ataggttttt 1140

agtctaattg aatcccttaa actcagggag catttataaa tggacaaatg cttatgaaac 1200

taagatttgt aatatttctc tctttttaga gaaatttgcc aatttacttt gttatttttc 1260

cccaaaaaga atgggatgat cgtgtattta tttttttact tcctcagctg tagacaggtc 1320

cttttcgatg gtacatattt ctttgccttt ataatctttt atacagtgtc ttacagagaa 1380

aagacataag caaagactat gaggaatatt tgcaagacat agaatagtgt tggaaaatgt 1440

gcaatatgtg atgtggcaaa tctctattag gaaatattct gtaatcttca gacctagaat 1500

aatactagtc ttataatagg tttgtgactt tcctaaatca attctattac gtgcaatact 1560

tcaatacttc atttaaaata tttttatgtg caataaaatg tatttgtttg tattttgtgt 1620

tcagtacaat tataagctgt tttatatatt gtgaaataaa agtagaataa acacaaaaaa 1680

aaaaaaaaaa aaaaaaaaaa aa 1702

<210> 37
<211> 2500
<212> DNA
<213> Homo sapiens

<400> 37
atacaggaag tgacgatact tttggcgcgc gcggttgctg tttcttctct ggctccggga 60

ccggcggcgg cggcggcggc acgggcggcg gcgtagggtg ttttaactca aatgggtgat 120

gaaaaggact cttggaaagt gaaaacttta gatgaaattc ttcaggaaaa gaaacgaagg 180

aaggaacaag aggagaaagc agagataaaa cgcttaaaaa ataacaacgc ttcttcggtg 240

aagttctttt gtacttccaa atgtcgcagt ctgatgaccg ggattccaag cgggattccc 300

ttgaggaggg ggagctgaga gatcaccgca tggagatcac aataaggaac tccccgtata 360

gaagagaaga ctctatggaa gacagaggag aagaagatga ttctttggcc atcaaaccac 420

cccagcaaat gtctcggaaa gaaaaagttc atcacagaaa agatgaaaag agaaaagaga 480

aaaagcatgc tagagtgaaa gaaaaagaaa gagagcacga acgtcggaaa cgacatcgag 540
aagaacagga taaagctcgc cgggaatggg aaagacagaa gagaagggaa atggcaaggg 600
agcattccag gagagaaagg gaccgcttgg agcagttaga aaggaagcgg gagcgggagc 660
gcaagatgcg ggagcagcag aaggagcagc gggagcagaa ggagcgcgag cggcgggcgg 720
aggagcggcg caaggagcgg gaggcccgca gggaagtgtc tgcacatcac cgaacgatga 780
gagaggacta cagcgacaaa gtgaaagcca gccactggag tcgcagcccg cctcggccgc 840
cgcgggagcg gttcgagttg ggagacggcc ggaagccagt aaaagaagag aaaatggaag 900
aaagggacct gctgtccgac ttacaggaca tcagcgacag cgagaggaag accagctcgg 960
ccgagtcctc gtcagcagaa tcaggctcag gttctgagga agaagaggag gaggaggaag 1020
aggaggagga ggaagggagc accagtgaag aatcagagga ggaggaggag gaagaggaag 1080
aggaggagga ggagaccggc agcaactctg aggaggcatc agagcagtct gccgaagaag 1140
taagtgagga agaaatgagt gaagatgaag aacgagaaaa tgaaaaccac ctcttggttg 1200
ttccagagtc acggttcgac cgagattccg gggagagtga agaagcagag gaagaagtgg 1260
gtgagggaac gccgcagagc agcgccctga cagagggcga ctatgtgccc gactcccctg 1320
ccctgtcgcc catcgagctc aagcaggagc tgcccaagta cctgccggcc ctgcagggct 1380
gccggagcgt cgaggagttc cagtgcctga acaggatcga ggagggcacc tatggagtgg 1440
tctacagagc aaaagacaag aaaacagatg aaattgtggc tctaaagcgg ctgaagatgg 1500
agaaggagaa ggagggcttc ccgatcacgt cgctgaggga gatcaacacc atcctcaagg 1560
cccagcatcc caacatcgtc accgttagag agattgtggt gggcagcaac atggacaaga 1620
tctacatcgt gatgaactat gtggagcacg acctcaagag cctgatggag accatgaaac 1680
agcccttcct gccaggggag gtgaagaccc tgatgatcca gctgctgcgt ggggtgaaac 1740
acctgcacga caactggatc ctgcaccgtg acctcaagac gtccaacctg ctgctgagcc 1800
acgccggcat cctcaaggtg ggtgacttcg ggctggcgcg ggagtacgga tcccctctga 1860
aggcctacac cccggtcgtg gtgaccctgt ggtaccgcgc cccagagctg ctgcttggtg 1920
ccaaggaata ctccacggcc gtggacatgt ggtcagtggg ttgcatcttc ggggagctgc 1980
tgactcagaa gcctctgttc cccgggaagt cagaaatcga tcagatcaac aaggtgttca 2040
aggatctggg gacccctagt gagaaaatct ggcccggcta cagcgagctc ccagcagtca 2100
agaagatgac cttcagcgag cacccctaca acaacctccg caagcgcttc ggggctctgc 2160
tctcagacca gggcttcgac ctcatgaaca agttcctgac ctacttcccc gggaggagga 2220
tcagcgctga ggacggcctc aagcatgagt atttccgcga gacccccctc cccatcgacc 2280
cctccatgtt ccccacgtgg cccgccaaga gcgagcagca gcgtgtgaag cggggcacca 2340
gcccgaggcc ccctgaggga ggcctgggct acagccagct gggtgacgac gacctgaagg 2400
agacgggctt ccaccttacc accacgaacc agggggcctc tgccgcgggc cccggcttca 2460
gcctcaagtt ctgaaggtca gagtggaccc cgtcatgggg 2500

<210> 38
<211> 2500
<212> DNA
<213> Homo sapiens

<400> 38
atacaggaag tgacgatact tttggcgcgc gcggttgctg tttcttctct ggctccggga 60

ccggcggcgg cggcggcggc acgggcggcg gcgtagggtg ttttaactca aatgggtgat 120

gaaaaggact cttggaaagt gaaaacttta gatgaaattc ttcaggaaaa gaaacgaagg 180

aaggaacaag aggagaaagc agagataaaa cgcttaaaaa ataacaacgc ttcttcggtg 240

aagttctttt gtacttccaa atgtcgcagt ctgatgaccg ggattccaag cgggattccc 300

ttgaggaggg ggagctgaga gatcaccgca tggagatcac aataaggaac tccccgtata 360

gaagagaaga ctctatggaa gacagaggag aagaagatga ttctttggcc atcaaaccac 420

cccagcaaat gtctcggaaa gaaaaagttc atcacagaaa agatgaaaag agaaaagaga 480

aaaagcatgc tagagtgaaa gaaaaagaaa gagagcacga acgtcggaaa cgacatcgag 540

aagaacagga taaagctcgc cgggaatggg aaagacagaa gagaagggaa atggcaaggg 600

agcattccag gagagaaagg gaccgcttgg agcagttaga aaggaagcgg gagcgggagc 660

gcaagatgcg ggagcagcag aaggagcagc gggagcagaa ggagcgcgag cggcgggcgg 720

aggagcggcg caaggagcgg gaggcccgca gggaagtgtc tgcacatcac cgaacgatga 780

gagaggacta cagcgacaaa gtgaaagcca gccactggag tcgcagcccg cctcggccgc 840

cgcgggagcg gttcgagttg ggagacggcc ggaagccagt aaaagaagag aaaatggaag 900

aaaggaccct gctgtccgac ttacaggaca tcagcgacag cgagaggaag accagctcgg 960

ccgagtcctc gtcagcagaa tcaggctcag gttctgagga agaagaggag gaggaggaag 1020

aggaggagga ggaagggagc accagtgaag aatcagagga ggaggaggag gaagaggaag 1080

aggaggagga ggagaccggc agcaactctg aggaggcatc agagcagtct gccgaagaag 1140

taagtgagga agaaatgagt gaagatgaag aacgagaaaa tgaaaaccac ctcttggttg 1200

ttccagagtc acggttcgac cgagattccg gggagagtga agaagcagag gaagaagtgg 1260

gtgagggaac gccgcagagc agcgccctga cagagggcga ctatgtgccc gactcccctg 1320

ccctgtcgcc catcgagctc aagcaggagc tgcccaagta cctgccggcc ctgcagggct 1380

gccggagcgt cgaggagttc cagtgcctga acaggatcga ggagggcacc tatggagtgg 1440

tctacagagc aaaagacaag aaaacagatg aaattgtggc tctaaagcgg ctgaagatgg 1500

agaaggagaa ggagggcttc ccgatcacgt cgctgaggga gatcaacacc atcctcaagg 1560

cccagcatcc caacatcgtc accgttagag agattgtggt gggcagcaac atggacaaga 1620

tctacatcgt gatgaactat gtggagcacg acctcaagag cctgatggag accatgaaac 1680

agcccttcct gccaggggag gtgaagaccc tgatgatcca gctgctgcgt ggggtgaaac 1740

acctgcacga caactggatc ctgcaccgtg acctcaagac gtccaacctg ctgctgagcc 1800

acgccggcat cctcaaggtg ggtgacttcg ggctggcgcg ggagtacgga tcccctctga 1860

aggcctacac cccggtcgtg gtgaccctgt ggtaccgcgc cccagagctg ctgcttggtg 1920

ccaaggaata ctccacggcc gtggacatgt ggtcagtggg ttgcatcttc ggggagctgc 1980

tgactcagaa gcctctgttc cccgggaagt cagaaatcga tcagatcaac aaggtgttca 2040

aggatctggg gacccctagt gagaaaatct ggcccggcta cagcgagctc ccagcagtca 2100

agaagatgac cttcagcgag cacccctaca acaacctccg caagcgcttc ggggctctgc 2160

tctcagacca gggcttcgac ctcatgaaca agttcctgac ctacttcccc gggaggagga 2220

tcagcgctga ggacggcctc aagcatgagt atttccgcga gacccccctc cccatcgacc 2280

cctccatgtt ccccacgtgg cccgccaaga gcgagcagca gcgtgtgaag cggggcacca 2340

gcccgaggcc ccctgaggga ggcctgggct acagccagct gggtgacgac gacctgaagg 2400

agacgggctt ccaccttacc accacgaacc agggggcctc tgccgcgggc cccggcttca 2460

gcctcaagtt ctgaaggtca gagtggaccc cgtcatgggg 2500

<210> 39
<211> 3697
<212> DNA
<213> Homo sapiens

<400> 39
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420

gctaatggaa ttatagtgga gggtccagtc cctatcacca tagaagtgaa ggacatcaac 480

gacaatcgac ccacgtttct ccagtcaaag tacgaaggct cagtaaggca gaactctcgc 540

ccaggaaagc ccttcttgta tgtcaatgcc acagacctgg atgatccggc cactcccaat 600

ggccagcttt attaccagat tgtcatccag cttcccatga tcaacaatgt catgtacttt 660

cagatcaaca acaaaacggg agccatctct cttacccgag agggatctca ggaattgaat 720

cctgctaaga atccttccta taatctggtg atctcagtga aggacatggg aggccagagt 780

gagaattcct tcagtgatac cacatctgtg gatatcatag tgacagagaa tatttggaaa 840

gcaccaaaac ctgtggagat ggtggaaaac tcaactgatc ctcaccccat caaaatcact 900

caggtgcggt ggaatgatcc cggtgcacaa tattccttag ttgacaaaga gaagctgcca 960

agattcccat tttcaattga ccaggaagga gatatttacg tgactcagcc cttggaccga 1020

gaagaaaagg atgcatatgt tttttatgca gttgcaaagg atgagtacgg aaaaccactt 1080

tcatatccgc tggaaattca tgtaaaagtt aaagatatta atgataatcc acctacatgt 1140

ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag accctttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccaccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa ttttttaaac agatattccc tcttgtcctt taatatttgc taaatatttc 2760
ttttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120
cttgtgtttt tccctttggg ggcaagacag actcattaaa tattctgtac attttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 40
<211> 2032
<212> DNA
<213> Homo sapiens

<400> 40
ggctcgaggc caggatgcct ccagtctggg ggaaaatgct ttcctcattg cttctcccag 60

cccacctcaa gcagtctccc caccccttga gtctcagcag tgttaaagct gttactttca 120

cagcttcctg ggagcgagtg ctttctcaag cccgtcttgc aaggtcttcc acttcagcac 180

aatgctactg cctaaaaaaa tgaagctcct gctgtttctg gtttcccaga tggccatctt 240

ggctctattc ttccacatgt acagccacaa catcagctcc ctgtctatga aggcacagcc 300

cgagcgcatg cacgtgctgg ttctgtcttc ctggcgctct ggctcttctt ttgtggggca 360

gctttttggg cagcacccag atgttttcta cctgatggag cccgcctggc acgtgtggat 420

gaccttcaag cagagcaccg cctggatgct gcacatggct gtgcgggatc tgatacgggc 480

cgtcttcttg tgcgacatga gcgtctttga tgcctacatg gaacctggtc cccggagaca 540

gtccagcctc tttcagtggg agaacagccg ggccctgtgt tctgcacctg cctgtgacat 600

catcccacaa gatgaaatca tcccccgggc tcactgcagg ctcctgtgca gtcaacagcc 660

ctttgaggtg gtggagaagg cctgccgctc ctacagccac gtggtgctca aggaggtgcg 720

cttcttcaac ctgcagtccc tctacccgct gctgaaagac ccctccctca acctgcatat 780

cgtgcacctg gtccgggacc cccgggccgt gttccgttcc cgagaacgca caaagggaga 840

tctcatgatt gacagtcgca ttgtgatggg gcagcatgag cagaaactca agaaggagga 900

ccaaccctac tatgtgatgc aggtcatctg ccaaagccag ctggagatct acaagaccat 960

ccagtccttg cccaaggccc tgcaggaacg ctacctgctt gtgcgctatg aggacctggc 1020

tcgagcccct gtggcccaga cttcccgaat gtatgaattc gtgggattgg aattcttgcc 1080

ccatcttcag acctgggtgc ataacatcac ccgaggcaag ggcatgggtg accacgcttt 1140

ccacacaaat gccagggatg cccttaatgt ctcccaggct tggcgctggt ctttgcccta 1200

tgaaaaggtt tctcgacttc agaaagcctg tggcgatgcc atgaatttgc tgggctaccg 1260

ccacgtcaga tctgaacaag aacagagaaa cctgttgctg gatcttctgt ctacctggac 1320

tgtccctgag caaatccact aagagggttg agaaggcttt gctgccacct ggtgtcagcc 1380

tcagtcactt tctctgaatg cttctgagcc ttgcctacat ctctgagcct taactacatg 1440

tctgtgggta tcacactgag tgtgagttgt gtccacacgt gctcaagcag aaggactttt 1500

gtgtccatgc ttgtgtctag aaaacagact ggggaacctt atgtgagcag cacatcccac 1560

cagtgaaaca gggtattgct cttcttcttt tcttgatctt cctgtctggg cagacttcag 1620

agactttgtg gcctggaggc ctattaagca cgacacagta tcagtggaat tgatccataa 1680

acctccctgt ccacatcttg cccaatgggg aatggatctt tcaccaaaga gctcaccagc 1740

attttccaca gagatgcaaa ttctgagccc ttggagttcc cagtggattc aaggaaggaa 1800

gtgggaacaa ggttggatgc ctacttatga gcttgaccat cacagctatc ggtaatcaga 1860

aatatgaaac aaaatctctg cacaaaagag caagctctta agttcacagg gtgcctgggc 1920

tgcatttgaa tatcacttcc cctctgcatt ttcccatcac atagaagact ttgacctgtg 1980

aagctgccat ctgttaatac taaaattccc aaataagaaa aaaaaaaaaa aa 2032

<210> 41
<211> 6885
<212> DNA
<213> Homo sapiens

<400> 41
gacgacgttt gggagccttt gctgagtcca gggagagagg cgtcccccac cgtgccgctg 60

cagctcgggc agagccgcca agctttgggg tgctgaggaa cctctaatca tctcccatgg 120

atttgtgatc agcgttgcag ctctcccagc agccctggac agtggccccc agcagtcagc 180

atgtggctgc cgcgcgtctc cagcacagca gtgaccgcgc tcctcctggc gcagaccttc 240

ctcctcctct ttctggtttc ccggccaggg ccctcgtccc cagcaggcgg cgaggcgcgc 300

gtgcatgtgc tggtgctgtc ctcgtggcgc tcgggctcgt ccttcgtggg ccaactcttc 360

aaccagcacc ccgacgtctt ctacctaatg gagcccgcgt ggcacgtgtg gaccaccctg 420

tcgcagggca gcgccgcaac gctgcacatg gctgtgcgcg acctggtgcg ctccgtcttc 480

ctgtgcgaca tggacgtgtt tgatgcctat ctgccttggc gccgcaacct gtccgacctc 540

ttccagtggg ccgtgagccg tgcactgtgc tcgccacccg cctgcagtgc ctttccccga 600

ggcgccatca gcagcgaggc cgtgtgcaag ccactgtgcg cgcggcagtc cttcaccctg 660

gcccgggagg cctgccgctc ctacagccac gtggtgctca aggaggtgcg cttcttcaac 720

ctgcaggtgc tctacccgct gctcagcgac cccgcgctca acctacgcat cgtgcacctg 780

gtgcgcgacc cgcgggccgt gctgcgctcc cgggagcaga cagccaaggc tctggcgcgt 840

gacaacggca tcgtgctggg caccaacggc acgtgggtgg aggccgaccc cggcctgcgc 900

gtggtgcgcg aggtgtgccg tagccacgta cgcatcgccg aggccgccac actcaagccg 960

ccaccctttc tgcgcggccg ctaccgcctg gtgcgcttcg aggacctggc gcgggagccg 1020

ctggcagaaa tccgtgcgct ctacgccttc actgggctca gtctcacgcc acagctcgag 1080

gcctggatcc ataacatcac ccacggatct ggacctggtg cgcgccgcga agccttcaag 1140
acttcgtcca ggaatgcgct caacgtctcc caggcctggc gccatgcgct gccctttgcc 1200
aagatccgcc gcgtgcagga actgtgcgct ggtgcgctgc agctgctggg ctaccggcct 1260
gtgtactctg aggacgagca gcgcaacctc gcccttgatc tggtgctgcc acgaggcctg 1320
aacggcttca cttgggcatc atccaccgcc tcgcaccccc gaaattagtg gaggccacag 1380
ttgtagcagg cgctaggccc gggaggagag tgcatggtgc agagggggct ggggcgcacg 1440
gagaagcagg tccctatatt gaccaaggag tttgtggtac gacccctccc cctccccaag 1500
taggcaagga ctgcacgttt ctttctctct tgattcttgg ttttcctttg agtcctctgg 1560
agctgccttc tcatcaggtg cactcttcat ggaaagcaac tcttgcccct cctcctctgg 1620
gcacagggtg tgcgttcaga tgacttggct cctactcaag ggctttcttc ccctttaact 1680
ctctccttct ggtgacacat cctgcagcag ctgagggggt gccctggcac tggctgggag 1740
tggagaggca ctgtggtgaa atggctccag aggtctgtac atcacataca tatgcacaca 1800
ggcacacatg gcaaaactcg gaagtgaaag gacttgtctg aaatcacatg gtgagaagga 1860
ggatgaaggg aggagagagc ttttgctctg ggtctccagt ggataggaga ggacctgcct 1920
cctgggtgag aagggtcaga ttttcctatt ttaattgctt tagggaagag caagcagagt 1980
catgaccagg gacacagctg agagatagag gaggctgtga atgctgagac cagagtttat 2040
catgctggac aagcctggaa ggaggcaata agtgggaaag gtaggaggag agaaggctgg 2100
ggagggctgg gcagcaagcc aggcacagtg agtggcagag caagaggggg aaagcaggat 2160
cagtgcctgg aaggcaggtg tgcccgtcag cggggagtgg aactcatcag gcttgccaag 2220
aggttggaag ggaaatggct ctgggctgga actgtcttcc cttggtcctt ctggtccagg 2280
ccttggagga aagcagagga tgatccctgc ctgtgagcca cacctcctag ctctgggggc 2340
aaaggggctt agtaaaggaa tgctggatgt gtagagggtt tagtcccgag ctcaggaaat 2400
gagagcctat aagtgcccag tacatgttta aaagaagagc tcatggaacc tctggaaagg 2460
acagggaagt tgagttagcc acataaatga acccaagtca cattggaaca cagagctggt 2520
ctgggaactg tgttggctgc caacagaact tctgaccctg ttacctgtga aatgaggcag 2580
tttccctcac gttgccatca gctaccagga gcgatgctgg tggtcactag cttctgatcc 2640
tcatcctggg tgtggccaca gattggggga acctggattg tggagtcaca tcctccctgc 2700
aaagcaagca gggcaaggga gatctggcat tttctgcttt acgtggaggg agaacaggca 2760
cattagcctt gaagctgaag ctcattttag gttccttcca ggtttagaag cttcaaccaa 2820
atgaaacttg aatctgtccc tcgtgacaat tataggagga aggtatttaa aaccccagat 2880
ttatgaatgt gtactacatg gcttagagaa tgtctttgtt cttgttcagg tggttataac 2940
aaaatacctt aagagtgggt aacttggctg gatgcagtgg ctcatgcctg taatcccagc 3000
actgtgggag gccgaggggg atgaatcacc tgaggtcagg cattcaagaa cagcctggcc 3060
aacatggcga agcccctcct ctactaaaaa tacaaaatta gcgaggcatg gtcgcacata 3120

cctgtaatcc cagctcctcg ggaagctgag gcaggagaat cgcttgaacc caggaggcgg 3180
aggttgcagt gagccaagat cacgccattg cactccagcc tgggtgacag agcaagactc 3240
catctcaaaa aaaaaaaaaa gactgggtaa cttataaaca aatgttcttc tcacaagtct 3300
ggagactggg aagtccaaga tcaagccacc agtgctgtct gatgagggcc cactttttca 3360
aagacagtgc cttctagctg tgtcctctta tcgtagaaga tgggagacag ctctccaggg 3420
ccattttttt tttttttttt ttttttttga gatggagtct ggctctgtcg cccaggctgg 3480
agtgcagtgg cacaatctcg gctcactgca acctctgcct ctctctgcct cctgagttca 3540
agcaattctc ctgcctcagc ctcctgagga gctgggacta cagggatgca ccaccatgcc 3600
cagctaattt ttgtattttt gtagacactg ggtttcacca tattggccag gttggtctca 3660
aactcctgac ctcaagtgat ctgcccacct cagcctccca aagtgctgag attacaggca 3720
tgagccactg tacccagtct ccagggcctt ttaaagaatg tcactaatcc cattcttgag 3780
gtctccacct tcattatcta atcacctccc aaaggctcca catcccaaca ccatcatatt 3840
gtgggttaag atttcaacca caagccaggc gtggtggctc atgcctgtaa tcccagcatt 3900
ttggaaggct gaggcaggtg gatcacttga ggtcaggagt ttgagaccag cctggccaac 3960
atggtgaaac cccatctcta ctaaaaataa aaaaaattag ccgggtgtgg tggtgcacac 4020
ctgtaattcc agctactcag gaggctgagg caggagaatc ccttgaatcc aggaggcgga 4080
cagtgcagtg agccgagata atgccactgc actccagcct ggatgacaga gcaagactcc 4140
atctcatgcc cagccagcat gcccaacaag cttcatttgc ccctgtttag gtcacaaatt 4200
ttattgatgg ctgcaattaa tggcctcttg gtatccaagt cctttgttgt atgacccatc 4260
cattctcccc tgactcccaa ggtgtcagga catgcttgac tggctcctga atttgctctc 4320
tgcgcatggg cagtacagtc aagcctcaca gtgaacccag gtcagctttc aggacaaaga 4380
aagtggcctg gctgactagg cacagtaaag ccagggctgg gtaggtacat acttgtgctg 4440
atcacgtatg tcttatatct ctgtgagagt gcagtcccaa caggaaggtt taatcactgg 4500
ggactgccca atgctgtgac agggcacaga gctctgggtt gctgtggggg tgactgcatt 4560
gaccactgtt agtggtttgc tgtgttgaca ctctgtgctg tgtgaccatg gctcctgcca 4620
tcaagaagta gagtctgttt ctccacctct gaatccaggc tggtcctgtg acttgctttg 4680
tcctgtagac aagtgtagtg caacttcctg tgagccagtt tgaagcatag gccttggaag 4740
caaaacttta cctccacctg tcttaggttt tcagctgggg ctctgctgtg atttgattgt 4800
gtctcccaaa gttggaacct tgatccccag tgttgtgagg tgaggcttga tggaaagtaa 4860
attacgccgt gcgggttatg cccttgtgaa tgggtagaga acattatttc tgggcgcagg 4920
catggtagct catgtctgta atcccagcat tttgggaggt tgaggtgtgc ggattacttg 4980
aggtcaggag tttgagacca gcctggccaa caaggtaaaa caccatttct agttaaaata 5040
caaaaattag ccaggtgtgg tggcacatgc ctgtaaggcc agctacttgg gaggctgaga 5100
caggagaatc gcttgaaccc aggaggcaga gattgcagtg agctgagatc gcaccactgt 5160

actccagcct gggcaacaaa gcgagagtct gtcttaaaaa aaaccaccat tatttcagga 5220
gtgagttggt tatcctgaga gtggtgcctt ttaaatgaag gagttcattc tttgtctttc 5280
tctcgccctc actttgccct tctgccatat gatgccttcc atcatgctag gacacagcaa 5340
gaaggctctc gctagatgct ggctccttga tcttgggctt cccagcctcc agaactgtaa 5400
gccaatacac ttctatttat tatatatgac ccttgctggg ttcagtggct cacgcctgta 5460
atcccaatac tttgcaaggc tgaggcagga ggatcacttg agaccaggca ctcaagacca 5520
gcctgggcaa catagtgaga ccccatctct acaaagttaa aaaaaaatta gcagggcatg 5580
gtgtcgtgca cctgtagtcc tagctacttg ggaggctgag ttgggaggac tgcttgaccc 5640
tgggaggttg aggctacatt gaaccatgat catgccagtg cactccagcc tgagtgacag 5700
agcaagacac ctatctctaa ataaatgacc ccatctgtgg tattgttata gcaaaacaaa 5760
acagattaag agagactttt taatgaaaag acagattcac aaagaaaaac aatgtttttg 5820
tttctgtttt tttgaggcag agtcttgctc ttgtccccca ggctggagtg cagtggcgcc 5880
atcttggctc actgcaacct ccgcctccca gtttcaagcg attctcctgc ctcagcctcc 5940
cgagtagctg ggattacaga tgtacaccac cacgcccggc taattttttt tgtattttta 6000
gtagagatgg ggtttcacca tgtcgatcag gctgggctgg aactcctgac ctcaggtgat 6060
ccacctgcct tggcctccca atgtgctagg attacaggca tgagccactg tacctggcga 6120
aaaacagttt gttaacacag gcagccaaca tcactcagga taagcctcaa tgaaaagtaa 6180
caaagtgatg gcttggaaca ctgtcttaca cagcattttt aaaaaataca ataaatttgt 6240
agagatagga tgaccaagga caacagtttt aggcttccaa aggtggtaaa ctatgggatg 6300
gtaaatatcc gagaggaagc tgatgcaaca ggatttgtct gcagcagcct ctggtaccac 6360
ctctgagtca agggttgtgt ccagtgatgg agagtttata tcgtgccttt aggcagaaaa 6420
ggggagggaa acctgaactt ttcctgcact ttctgcttct taattgcctt cagctgaaaa 6480
tcatttttta tgtgaaaaag gcatagtctg agctgacgcc tctgctttcc tccacctgaa 6540
gagaacctgc gtgctgctcc tttgcttcgg acctccgcct ctgcccggga gaaagcccag 6600
gccagcctgc tggacaagca gagaccatga gaaggagagt tcaggggtcc caaaccaggc 6660
catcctagac cagccagctc cagctgatcc gcacgcagcc acttcggcta ccttctactg 6720
gccaaaggga gtcccagggc tcacccagat tcagaggtgg ggaaactgag tccaccactt 6780
gagaagagta gctataaaga catatgagcg aggccagctg agcccagcac tgcggccaag 6840
tcgaagactt taggagcaat aaaagtgctt attgtgtttc agtca 6885

<210> 42
<211> 2496
<212> DNA
<213> Homo sapiens

<400> 42
gggactcgtc ccagagtttg ctgccgccgc cgccgctgcc attagagcgg ttttaattag 60

ctgcaggatt tttaatgagc tgaaatcaag aagagcccca tctcagaagt aagcgagagc 120
cgcgcgcagg gaagaactag tctgacaaaa cagaaggagg tggtgctctc gggggactgg 180
gctgtaggct cccaccgatc gatcggcgac gagcagatga gttcccgagg ccaggaggct 240
gaggagggaa gccagggctc agagaccagc gcccggtgca gagtccccgc tgctgccacc 300
gcggcagaag gcacggtgcc ggattcaagg tgacggcagt gcctggctcc attctcctca 360
agttgccata gcagcctctc tcccaacaag cagcttctcc gggagccctc agccgggagg 420
gacgtcattg ttctctcctg cccgtgtggt gcaaggagac ccaccggaca gagaactttc 480
ccgagcccgg ggtccgctga ccacgtgggt gcacccatgc tgcctggagc caggcacagg 540
gacaggtccc cacggccaca gggaatcctc tgggcagctg aggggagcgt ccaggcccag 600
aagcagctgc agccaagcgt gtccttggag ccgtccatgc gtctgtccgc ctgctgccgg 660
tcgccactgg agatctcggc ctgatggacg cctggtgtgg acgatgaggg aagaacgtgc 720
cccccacacc caagaggtga cccctgagcc agccccggat gaccctgcga cctggaacaa 780
tgcggctggc ctgcatgttc tcttccatcc tgctgttcgg agctgcaggc ctcctcctct 840
tcatcagcct gcaggaccct acggagctcg ccccccagca ggtgccagga ataaagttca 900
acatcaggcc aaggcagccc caccacgacc tcccaccagg cggctcccag gatggtgact 960
tgaaggaacc cacagagagg gtcactcggg acttatccag tggggccccg aggggccgca 1020
acctgccagc gcctgaccag cctcaacccc cgctgcagag gggaacccgt ctgcggctcc 1080
gccagcgccg tcgccgtctg ctcatcaaga aaatgccagc tgcggcgacc atcccggcca 1140
acagctcgga cgcgcccttc atccggccgg gacccgggac gctggatggc cgctgggtca 1200
gcctgcaccg gagccagcag gagcgcaagc gggtgatgca ggaggcctgc gccaagtacc 1260
gggcgagcag cagccgccgg gccgtcacgc cccgccacgt gtcccgtatc ttcgtggagg 1320
accgccaccg cgtgctctac tgcgaggtgc ccaaggccgg ctgctccaat tggaagcggg 1380
tgctcatggt gctggccggc ctggcctcgt ccactgccga catccagcac aacaccgtcc 1440
actatggcag cgctctcaag cgcctggaca ccttcgaccg ccagggtatc ttgcaccgtc 1500
tcagcaccta caccaagatg ctctttgtcc gcgagccctt cgagaggctg gtgtccgcct 1560
tccgcgacaa gtttgagcac cccaacagct actatcaccc ggtcttcggc aaggccatcc 1620
tggcccggta ccgcgccaat gcctctcggg aggccctgcg gaccggctct ggggtgcgtt 1680
ttcccgagtt cgtccagtac ctgctggacg tgcaccggcc cgtggggatg gacattcact 1740
gggaccatgt cagccggctc tgcagcccct gcctcatcga ctacgatttc gtaggcaagt 1800
tcgagagcat ggaggacgat gccaacttct tcctgagcct catccgcgcg ccgcggaacc 1860
tgaccttccc ccggttcaag gaccggcact cgcaggaggc gcggaccaca gcgaggatcg 1920
cccaccagta cttcgcccaa ctctcggccc tgcaaaggca gcgcacctac gacttctact 1980
acatggatta cctgatgttc aactattcca agccctttgc agatctgtac tgaggggcgc 2040
cgcagctggc cggggccgcc ctgccccggt cactcacctg tgctcccggg catcctcctg 2100

tccctggctc ctcatcctgg gagcaacagg gctctgagga cgtgaggagc catcgctgtg 2160
ggaggcagca ggccccgggt ggggggcaga ggcgcccagc cttggatggg gaccccagcc 2220
cctggcctgt acctgtttcc tcattccttg gctgagggag aggctgagaa ctgggcagac 2280
acccctggag ctcagccgac agttttgatg agcagggaag tctgaggccc agaggacggg 2340
gggcccagcg gtaagggatg tcccgcactc ccttagccat tgccttggac caaaccacgt 2400
ggtttgcagc ttttctacga gccagggggg aggttccctt ggattaaggt tccaaataaa 2460
gcacatggtt tccagagcaa aaaaaaaaaa aaaaaa 2496

<210> 43
<211> 2219
<212> DNA
<213> Homo sapiens

<400> 43
gtcagggcca gggtgagcgc ccgactccga gctgtccccg ctcccggcgc ggcgctccgc 60
tctcagccac ctcacggctg ccaggagtgc gcgggagttt gccccggagc gcggggaagt 120
ttcctccgaa gctgcgctcc tggaacagca gcacctgcaa gcgcccggca gcggcccgcg 180
aggttacttt atggaattgg gctcttagag aacaagaaaa gactgaagtt ttacgggaaa 240
acaaatcatg tggtcttcag attctgaaat aaggagaaat gcagccatct gaaatggtca 300
tgaaccccaa acaagtcttc ctctctgtgc tgatatttgg agtagctggg ctactcctct 360
tcatgtattt gcaagtctgg attgaagaac aacatacagg gagagtggag aagagaagag 420
aacaaaaagt aacttcagga tggggaccag tgaagtactt gcggcctgta cccagaatca 480
tgagtacaga aaaaatccag gaacatatca ccaaccagaa ccccaagttt cacatgcctg 540
aggatgtacg agaaaaaaag gaaaatcttc tactcaattc tgagagatct actaggctct 600
taacaaagac cagtcattca caaggagggg atcaagcttt aagtaagtcc acagggtcac 660
caacagagaa gttgattgaa aaacgtcaag gagctaagac tgtttttaac aagttcagca 720
acatgaattg gccagtggac attcaccctt taaacaaaag tttagtcaaa gataataaat 780
ggaagaaaac tgaggagacc caagagaaac gaaggtcttt ccttcaggag ttttgcaaga 840
aatacggtgg ggtgagtcat catcagtcac atctttttca tacagtatcc agaatctatg 900
tagaagataa acacaaaatc ttatattgtg aggtacctaa ggctggctgt tccaattgga 960
aaagaattct gatggtacta aatggattgg cttcctctgc atacaacatc tcccacaatg 1020
ctgtccacta cgggaagcat ttgaagaagc tagatagctt tgacctaaaa gggatatata 1080
cccgcttaaa tacttacacc aaagctgtgt ttgttcgtga tcccatggaa agattagtat 1140
cagcctttag ggacaaattt gaacacccca atagttatta ccatccagta ttcggaaagg 1200
caattatcaa gaaatatcga ccaaatgcct gtgaagaagc attaattaat ggatctggag 1260
tcaagttcaa agagtttatc cactacttgc tggattccca ccgtccagta ggaatggaca 1320
ttcactggga aaaggtcagc aaactctgct atccgtgttt gatcaactat gattttgtag 1380
ggaaatttga gactttggaa gaagatgcca attacttttt acagatgatc ggtgctccaa 1440

aggagctgaa atttcccaac tttaaggata ggcactcttc cgatgaaaga accaatgctc 1500
aagtcgtgag acagtattta aaggatctga ctagaactga gagacaatta atctatgact 1560
tttattactt ggactattta atgtttaatt atacaactcc atttttgtag tttgcattca 1620
ttttctaaaa ccctgtatat acttaatgat gataagttca aatcagctgt aatttttcta 1680
taattctctg tatgacagaa atttaaccaa gtgcagttgt cttgatttaa tgtagatttt 1740
taccaaatag tatgacacca attggcacaa agttatagga aaatcaccta caggagatgt 1800
aaacaacttg agttgctcta aaatgtttgg aaaagagctg cttttgcatt atgaattata 1860
ttgttgaagc aataacctag ccagctgttg cattagctaa agcagcctct tgcaatggta 1920
ggaaaaaagg atctcaaata gcatgagtgt atgtctatat cctgaaattt attgtctaaa 1980
atgcatgaat atatttttag cagtctgtgg catattaatc aaactgttga attgttttct 2040
tacaccctgg aaatctttct atcaactata atgataaatc cattttgaag tgatattttg 2100
gacttaggca ttttacttta gattggaagg cattatgtga tttacaatat gagaatatag 2160
cagaaaaacc agatgaggct gtggcttttt atattcaaca gccaataaaa aatgcacaa 2219

<210> 44
<211> 1741
<212> DNA
<213> Homo sapiens

<400> 44
gatcgggcgg cgccgagctg aggtggtgag ggactagctc ccggatgtgg agaagctggg 60
gagaaggcgt gggaggaaga tggactcggt ggagaagggg gccgccacct ccgtctccaa 120
cccgcggggg cgaccgtccc ggggccggcc gccgaagctg cagcgcaact ctcgcggcgg 180
ccagggccga ggtgtggaga agcccccgca cctggcagcc ctaattctgg cccggggagg 240
cagcaaaggc atccccctga agaacattaa gcacctggcg ggggtcccgc tcattggctg 300
ggtcctgcgt gcggccctgg attcaggggc cttccagagt gtatgggttt cgacagacca 360
tgatgaaatt gagaatgtgg ccaaacaatt tggtgcacaa gttcatcgaa gaagttctga 420
agtttcaaaa gacagctcta cctcactaga tgccatcata gaatttctta attatcataa 480
tgaggttgac attgtaggaa atattcaagc tacttctcca tgtttacatc ctactgatct 540
tcaaaaagtt gcagaaatga ttcgagaaga aggatatgat tctgttttct ctgttgtgag 600
acgccatcag tttcgatgga gtgaaattca gaaaggagtt cgtgaagtga ccgaacctct 660
gaatttaaat ccagctaaac ggcctcgtcg acaagactgg gatggagaat tatatgaaaa 720
tggctcattt tattttgcta aaagacattt gatagagatg ggttacttgc agggtggaaa 780
aatggcatac tacgaaatgc gagctgaaca tagtgtggat atagatgtgg atattgattg 840
gcctattgca gagcaaagag tattaagata tggctatttt ggcaaagaga agcttaagga 900
aataaaactt ttggtttgca atattgatgg atgtctcacc aatggccaca tttatgtatc 960
aggagaccaa aaagaaataa tatcttatga tgtaaaagat gctattggga taagtttatt 1020

aaagaaaagt ggtattgagg tgaggctaat ctcagaaagg gcctgttcaa agcagacgct 1080
gtcttcttta aaactggatt gcaaaatgga agtcagtgta tcagacaagc tagcagttgt 1140
agatgaatgg agaaaagaaa tgggcctgtg ctggaaagaa gtggcatatc ttggaaatga 1200
agtgtctgat gaagagtgct tgaagagagt gggcctaagt ggcgctcctg ctgatgcctg 1260
ttctactgcc cagaaggctg ttggatacat ttgcaaatgt aatggtggcc gtggtgccat 1320
ccgagaattt gcagagcaca tttgcctact aatggaaaag gttaataatt catgccaaaa 1380
atagaaatta gcgtaatatt gagaaaaaaa tgatacagcc ttcttcagcc agtttgcttt 1440
tatttttgat taagtaaatt ccatgttgta atgttacaga gagtgtgatt tggtttgtga 1500
tatatatata ttgtgctcta cttttctctt tacgcaagat aattatttag agactgatta 1560
cagtctttct cagattttta gtaaatgcaa gtaagaacat catcaaagtt cactttgtat 1620
tgtaccctgt aaaactgtgt gtttgtgtgc tttcaaagat gttgggattt tatttatctg 1680
gggacagtgt gtatggtaag acatgccctt ctattaataa aactacattt ctcaaacttg 1740
a 1741

<210> 45
<211> 2492
<212> DNA
<213> Homo sapiens

<400> 45
gggtgcagag gtgccgccgc cgccgcgagc cagtcgggag cgcgcgaggc gcggggagcc 60
tgggaccagg agcgagagcc gcctacctgc agccgccgcc cacggcacgg cagccaccat 120
ggcgctcctg ctgtgcttcg tgctcctgtg cggagtagtg gatttcgcca gaagtttgag 180
tatcactact cctgaagaga tgattgaaaa agccaaaggg gaaactgcct atctgccatg 240
caaatttacg cttagtcccg aagaccaggg accgctggac atcgagtggc tgatatcacc 300
agctgataat cagaaggtgg atcaagtgat tattttatat tctggagaca aaatttatga 360
tgactactat ccagatctga aaggccgagt acattttacg agtaatgatc tcaaatctgg 420
tgatgcatca ataaatgtaa cgaatttaca actgtcagat attggcacat atcagtgcaa 480
agtgaaaaaa gctcctggtg ttgcaaataa gaagattcat ctggtagttc ttgttaagcc 540
ttcaggtgcg agatgttacg ttgatggatc tgaagaaatt ggaagtgact ttaagataaa 600
atgtgaacca aaagaaggtt cacttccatt acagtatgag tggcaaaaat tgtctgactc 660
acagaaaatg cccacttcat ggttagcaga aatgacttca tctgttatat ctgtaaaaaa 720
tgcctcttct gagtactctg ggacatacag ctgtacagtc agaaacagag tgggctctga 780
tcagtgcctg ttgcgtctaa acgttgtccc tccttcaaat aaagctggac taattgcagg 840
agccattata ggaactttgc ttgctctagc gctcattggt cttatcatct tttgctgtcg 900
taaaaagcgc agagaagaaa aatatgaaaa ggaagttcat cacgatatca gggaagatgt 960
gccacctcca aagagccgta cgtccactgc cagaagctac atcggcagta atcattcatc 1020
cctggggtcc atgtctcctt ccaacatgga aggatattcc aagactcagt ataaccaagt 1080

accaagtgaa gactttgaac gcactcctca gagtccgact ctcccacctg ctaaggtagc 1140
tgcccctaat ctaagtcgaa tgggtgcgat tcctgtgatg attccagcac agagcaagga 1200
tgggtctata gtatagagcc tccatatgtc tcatctgtgc tctccgtgtt cctttccttt 1260
ttttgatata tgaaaaccta ttctggtcta aattgtgtta ctagcctcaa aatacatcaa 1320
aaaataagtt aatcaggaac tgtacggaat atatttttaa aaatttttgt ttggttatat 1380
cgaaatagtt acaggcacta aagttagtaa agaaaagttt accatctgaa aaagctggat 1440
tttctttaag aggttgatta taaagttttc taaatttatc agtacctaag taagatgtag 1500
cgctttgaat atgaaatcat aggtgaagac atgggtgaac ttacttgcat accaagttga 1560
tacttgaata accatctgaa agtggtactt gatcattttt accattattt ttaggatgtg 1620
tatttcattt atttatggcc caccagtctc ccccaaatta gtacagaaat atccatgaca 1680
aaattactta cgtatgtttg tacttggttt tacagctcct ttgaaaactc tgtgtttgga 1740
atatctctaa aaacatagaa aacactacag tggtttagaa attactaatt ttacttctaa 1800
gtcattcata aaccttgtct atgaaatgac ttcttaaata tttagttgat agactgctac 1860
aggtaatagg gacttagcaa gctcttttat atgctaaagg agcatctatc agattaagtt 1920
agaacatttg ctgtcagcca catattgaga tgacactagg tgcaatagca gggatagatt 1980
ttgttggtga gtagtctcat gccttgagat ctgtggtggt cttcaaaatg gtggccagcc 2040
agatcaagga tgtagtatct catagttccc aggtgatatt tttcttatta gaaaaatatt 2100
ataactcatt tgttgtttga cacttataga ttgaaatttc ctaatttatt ctaaatttta 2160
agtggttctt tggttccagt gctttatgtt gttgttgttt ttggatggtg ttacatatta 2220
tatgttctag aaacatgtaa tcctaaattt accctcttga atataatccc tggatgatat 2280
tttttatcat aaatgcagaa taatcaaata cattttaagc aagttaagtg tcctccatca 2340
attctgtatt ccagacttgg gaggatgtac agttgctgtt gtgtgatcaa acatgtctct 2400
gtgtagttcc agcaaatcaa gctgagcttt gaaaaagttt gtcttagttt tgtgaaggtg 2460
atttattctt aaaaaaaaaa aaaaaaaaaa aa 2492

<210> 46
<211> 2038
<212> DNA
<213> Homo sapiens

<400> 46
aagtatccgt tcttggctgc ctttctttaa ttgcgtttcc agtactctct cggtgattct 60
actcttgaac ataggatgaa atttggaatc acacttctct tccacttcca tccccaccct 120
ctaatgccca tattaaaaat ggcggccgcc gccttccgca gtaatggttg ttcagcgaac 180
aagatccggg cggaaacagt agataggcgg gtgcagcggg gcagaacata ggttgcctta 240
gagaggttcc ccggtgtccc gacggcggct caagtcagag ttgctgggtt ttgctcagat 300
tggtgtggga agagcctgcc tgtggggagc ggccactcca tactgctgag gcctcaggac 360

tgctgctcag cttgcccgtt acctgaagag gcggcggagc cgggcccctg accggtcacc 420
atgtgggcct tctcggaatt gcccatgccg ctgctgatca atttgatcgt ctcgctgctg 480
ggatttgtgg ccacagtcac cctcatcccg gccttccggg gccacttcat tgctgcgcgc 540
ctctgtggtc aggacctcaa caaaaccagc cgacagcaga ttttgtggcc ctgataggtg 600
ccctccttgc catctgctgc atgatcttcc tgggctttgc ggatgatgta ctgaatctgc 660
gctggcgcca taagctgctg ctacctacag ctgcctcact acctctcctc atggtctatt 720
tcaccaactt tggcaacacg accattgtgg tgcccaagcc cttccgcccg atacttggcc 780
tgcatctgga cttgggaatc ctgtactatg tctacatggg gctgctggca gtgttctgta 840
ccaatgccat caatatccta gcaggaatta acggcctaga ggctggccag tcactagtca 900
tttctgcttc catcattgtc ttcaacctgg tagagttgga aggtgattgt cgggatgatc 960
atgtcttttc cctctacttc atgataccct tttttttcac cactttggga ttgctctacc 1020
acaactggta cccatcacgg gtgtttgtgg gagatacctt ctgttacttt gctggcatga 1080
cctttgccgt ggtgggcatc ttgggacact tcagcaagac catgctacta ttcttcatgc 1140
cccaggtgtt caacttcctc tactcactgc ctcagctcct gcatatcatc ccctgccctc 1200
gccaccgcat acccagactc aatatcaaga caggcaaact ggagatgagc tattccaagt 1260
tcaagaccaa gagcctctct ttcttgggca cctttatttt aaaggtggca gagagcctcc 1320
agctggtgac agtacaccag agtgagactg aagatggtga attcactgaa tgtaacaaca 1380
tgaccctcat caacttgcta cttaaagtcc ttgggcccat acatgagaga aacctcacat 1440
tgctcctgct gctgctgcag atcctgggca gtgccatcac cttctccatt cgatatcagc 1500
tcgttcgact cttctatgat gtctgagtcc cttgatcatt gtcctttacc tcacagtctc 1560
taggattcct gactcaggct gacctctctc tctggtccca gactgcctcc ttgcccaggc 1620
ctctctcact cttcatactc ctccagattt tgttctcagc attttccttt ctctgtgatc 1680
attggcatcc tgggcgtttc ttgccctctg ctgactactg attggatttt acctatggct 1740
ttctgcaact tgctactctc tccctctcca tcccatcttt gcagcctcat agggtgggat 1800
acagcagctt tttttgcagt tatccacact cacatttcag agtcctgact ctcaaggaac 1860
cactggtttt tgggatagaa cttgggccag ggctaggaac acaggctcca cggtgacatg 1920
tcatttgatt gtaaattaag tgttctgatt agtaagaact aagcaggggg ccacatgctc 1980
tcaatggaga caataaagtg ttgtcttttt cttattgttt aaaaaaaaaa aaaaaaaa 2038

<210> 47
<211> 9730
<212> DNA
<213> Homo sapiens

<400> 47
ccgaccaaca ccaacaccca gctccgacgc agctcctctg cgcccttgcc gccctccgag 60
ccacagcttt cctcccgctc ctgcccccgg cccgtcgccg tctccgcgct cgcagcggcc 120
tcgggagggc ccaggtagcg agcagcgacc tcgcgagcct tccgcactcc cgcccggttc 180

cccggccgtc cgcctatcct tggccccctc cgctttctcc gcgccggccc gcctcgctta 240
tgcctcggcg ctgagccgct ctcccgattg cccgccgaca tgagctgcaa cggaggctcc 300
cacccgcgga tcaacactct gggccgcatg atccgcgccg agtctggccc ggacctgcgc 360
tacgaggtga ccagcggcgg cgggggcacc agcaggatgt actattctcg gcgcggcgtg 420
atcaccgacc agaactcgga cggctactgt caaaccggca cgatgtccag gcaccagaac 480
cagaacacca tccaggagct gctgcagaac tgctccgact gcttgatgcg agcagagctc 540
atcgtgcagc ctgaattgaa gtatggagat ggaatacaac tgactcggag tcgagaattg 600
gatgagtgtt ttgcccaggc caatgaccaa atggaaatcc tcgacagctt gatcagagag 660
atgcggcaga tgggccagcc ctgtgatgct taccagaaaa ggcttcttca gctccaagag 720
caaatgcgag ccctttataa agccatcagt gtccctcgag tccgcagggc cagctccaag 780
ggtggtggag gctacacttg tcagagtggc tctggctggg atgagttcac caaacatgtc 840
accagtgaat gtttggggtg gatgaggcag caaagggcgg agatggacat ggtggcctgg 900
ggtgtggacc tggcctcagt ggagcagcac attaacagcc accggggcat ccacaactcc 960
atcggcgact atcgctggca gctggacaaa atcaaagccg acctgcgcga gaaatctgcg 1020
atctaccagt tggaggagga gtatgaaaac ctgctgaaag cgtcctttga gaggatggat 1080
cacctgcgac agctgcagaa catcattcag gccacgtcca gggagatcat gtggatcaat 1140
gactgcgagg aggaggagct gctgtacgac tggagcgaca agaacaccaa catcgctcag 1200
aaacaggagg ccttctccat acgcatgagt caactggaag ttaaagaaaa agagctcaat 1260
aagctgaaac aagaaagtga ccaacttgtc ctcaatcagc atccagcttc agacaaaatt 1320
gaggcctata tggacactct gcagacgcag tggagttgga ttcttcagat caccaagtgc 1380
attgatgttc atctgaaaga aaatgctgcc tactttcagt tttttgaaga ggcgcagtct 1440
actgaagcat acctgaaggg gctccaggac tccatcagga agaagtaccc ctgcgacaag 1500
aacatgcccc tgcagcacct gctggaacag atcaaggagc tggagaaaga acgagagaaa 1560
atccttgaat acaagcgtca ggtgcagaac ttggtaaaca agtctaagaa gattgtacag 1620
ctgaagcctc gtaacccaga ctacagaagc aataaaccca ttattctcag agctctctgt 1680
gactacaaac aagatcagaa aatcgtgcat aagggggatg agtgtatcct gaaggacaac 1740
aacgagcgca gcaagtggta cgtgacgggc ccgggaggcg ttgacatgct tgttccctct 1800
gtggggctga tcatccctcc tccgaaccca ctggccgtgg acctctcttg caagattgag 1860
cagtactacg aagccatctt ggctctgtgg aaccagctct acatcaacat gaagagcctg 1920
gtgtcctggc actactgcat gattgacata gagaagatca gggccatgac aatcgccaag 1980
ctgaaaacaa tgcggcagga agattacatg aagacgatag ccgaccttga gttacattac 2040
caagagttca tcagaaatag ccaaggctca gagatgtttg gagatgatga caagcggaaa 2100
atacagtctc agttcaccga tgcccagaag cattaccaga ccctggtcat tcagctccct 2160
ggctatcccc agcaccagac agtgaccaca actgaaatca ctcatcatgg aacctgccaa 2220

gatgtcaacc ataataaagt aattgaaacc aacagagaaa atgacaagca agaaacatgg 2280
atgctgatgg agctgcagaa gattcgcagg cagatagagc actgcgaggg caggatgact 2340
ctcaaaaacc tccctctagc agaccagggg tcttctcacc acatcacagt gaaaattaac 2400
gagcttaaga gtgtgcagaa tgattcacaa gcaattgctg aggttctcaa ccagcttaaa 2460
gatatgcttg ccaacttcag aggttctgaa aagtactgct atttacagaa tgaagtattt 2520
ggactatttc agaaactgga aaatatcaat ggtgttacag atggctactt aaatagctta 2580
tgcacagtaa gggcactgct ccaggctatt ctccaaacag aagacatgtt aaaggtttat 2640
gaagccaggc tcactgagga ggaaactgtc tgcctggacc tggataaagt ggaagcttac 2700
cgctgtggac tgaagaaaat aaaaaatgac ttgaacttga agaagtcgtt gttggccact 2760
atgaagacag aactacagaa agcccagcag atccactctc agacttcaca gcagtatcca 2820
ctttatgatc tggacttggg caagttcggt gaaaaagtca cacagctgac agaccgctgg 2880
caaaggatag ataaacagat cgactttagg ttatgggacc tggagaaaca aatcaagcaa 2940
ttgaggaatt atcgtgataa ctatcaggct ttctgcaagt ggctctatga tgctaaacgc 3000
cgccaggatt ccttagaatc catgaaattt ggagattcca acacagtcat gcggtttttg 3060
aatgagcaga agaacttgca cagtgaaata tctggcaaac gagacaaatc agaggaagta 3120
caaaaaattg ctgaactttg cgccaattca attaaggatt atgagctcca gctggcctca 3180
tacacctcag gactggaaac tctgctgaac atacctatca agaggaccat gattcagtcc 3240
ccttctgggg tgattctgca agaggctgca gatgttcatg ctcggtacat tgaactactt 3300
acaagatctg gagactatta caggttctta agtgagatgc tgaagagttt ggaagatctg 3360
aagctgaaaa ataccaagat cgaagttttg gaagaggagc tcagactggc ccgagatgcc 3420
aactcggaaa actgtaataa gaacaaattc ctggatcaga acctgcagaa ataccaggca 3480
gagtgttccc agttcaaagc gaagcttgcg agcctggagg agctgaagag acaggctgag 3540
ctggatggga agtcggctaa gcaaaatcta gacaagtgct acggccaaat aaaagaactc 3600
aatgagaaga tcacccgact gacttatgag attgaagatg aaaagagaag aagaaaatct 3660
gtggaagaca gatttgacca acagaagaat gactatgacc aactgcagaa agcaaggcaa 3720
tgtgaaaagg agaaccttgg ttggcagaaa ttagagtctg agaaagccat caaggagaag 3780
gagtacgaga ttgaaaggtt gagggttcta ctgcaggaag aaggcacccg gaagagagaa 3840
tatgaaaatg agctggcaaa ggtaagaaac cactataatg aggagatgag taatttaagg 3900
aacaagtatg aaacagagat taacattacg aagaccacca tcaaggagat atccatgcaa 3960
aaagaggatg attccaaaaa tcttagaaac cagcttgata gactttcaag ggaaaatcga 4020
gatctgaagg atgaaattgt caggctcaat gacagcatct tgcaggccac tgagcagcga 4080
aggcgagctg aagaaaacgc ccttcagcaa aaggcctgtg gctctgagat aatgcagaag 4140
aagcagcatc tggagataga actgaagcag gtcatgcagc agcgctctga ggacaatgcc 4200
cggcacaagc agtccctgga ggaggctgcc aagaccattc aggacaaaaa taaggagatc 4260

gagagactca aagctgagtt tcaggaggag gccaagcgcc gctgggaata tgaaaatgaa 4320
ctgagtaagg taagaaacaa ttatgatgag gagatcatta gcttaaaaaa tcagtttgag 4380
accgagatca acatcaccaa gaccaccatc caccagctca ccatgcagaa ggaagaggat 4440
accagtggct accgggctca gatagacaat ctcacccgag aaaacaggag cttatctgaa 4500
gaaataaaga ggctgaagaa cactctaacc cagaccacag agaatctcag gagggtggaa 4560
gaagacatcc aacagcaaaa ggccactggc tctgaggtgt ctcagaggaa acagcagctg 4620
gaggttgagc tgagacaagt cactcagatg cgaacagagg agagcgtaag atataagcaa 4680
tctcttgatg atgctgccaa aaccatccag gataaaaaca aggagataga aaggttaaaa 4740
caactgatcg acaaagaaac aaatgaccgg aaatgcctgg aagatgaaaa cgcgagatta 4800
caaagggtcc agtatgacct gcagaaagca aacagtagtg cgacggagac aataaacaaa 4860
ctgaaggttc aggagcaaga actgacacgc ctgaggatcg actatgaaag ggtttcccag 4920
gagaggactg tgaaggacca ggatatcacg cggttccaga actctctgaa agagctgcag 4980
ctgcagaagc agaaggtgga agaggagctg aatcggctga agaggaccgc gtcagaagac 5040
tcctgcaaga ggaagaagct ggaggaagag ctggaaggca tgaggaggtc gctgaaggag 5100
caagccatca aaatcaccaa cctgacccag cagctggagc aggcatccat tgttaagaag 5160
aggagtgagg atgacctccg gcagcagagg gacgtgctgg atggccacct gagggaaaag 5220
cagaggaccc aggaagagct gaggaggctc tcttctgagg tcgaggccct gaggcggcag 5280
ttactccagg aacaggaaag tgtcaaacaa gctcacttga ggaatgagca tttccagaag 5340
gcgatagaag ataaaagcag aagcttaaat gaaagcaaaa tagaaattga gaggctgcag 5400
tctctcacag agaacctgac caaggagcac ttgatgttag aagaagaact gcggaacctg 5460
aggctggagt acgatgacct gaggagagga cgaagcgaag cggacagtga taaaaatgca 5520
accatcttgg aactaaggag ccagctgcag atcagcaaca accggaccct ggaactgcag 5580
gggctgatta atgatttaca gagagagagg gaaaatttga gacaggaaat tgagaaattc 5640
caaaagcagg ctttagaggc atctaatagg attcaggaat caaagaatca gtgtactcag 5700
gtggtacagg aaagagagag ccttctggtg aaaatcaaag tcctggagca agacaaggca 5760
aggctgcaga ggctggagga tgagctgaat cgtgcaaaat caactctaga ggcagaaacc 5820
agggtgaaac agcgcctgga gtgtgagaaa cagcaaattc agaatgacct gaatcagtgg 5880
aagactcaat attcccgcaa ggaggaggct attaggaaga tagaatcgga aagagaaaag 5940
agtgagagag agaagaacag tcttaggagt gagatcgaaa gactccaagc agagatcaag 6000
agaattgaag agaggtgcag gcgtaagctg gaggattcta ccagggagac acagtcacag 6060
ttagaaacag aacgctcccg atatcagagg gagattgata aactcagaca gcgcccatat 6120
gggtcccatc gagagaccca gactgagtgt gagtggaccg ttgacacctc caagctggtg 6180
tttgatgggc tgaggaagaa ggtgacagca atgcagctct atgagtgtca gctgatcgac 6240
aaaacaacct tggacaaact attgaagggg aagaagtcag tggaagaagt tgcttctgaa 6300

atccagccat tccttcgggg tgcaggatct atcgctggag catctgcttc tcctaaggaa 6360
aaatactctt tggtagaggc caagagaaag aaattaatca gcccagaatc cacagtcatg 6420
cttctggagg cccaggcagc tacaggtggt ataattgatc cccatcggaa tgagaagctg 6480
actgtcgaca gtgccatagc tcgggacctc attgacttcg atgaccgtca gcagatatat 6540
gcagcagaaa aagctatcac tggttttgat gatccatttt caggcaagac agtatctgtt 6600
tcagaagcca tcaagaaaaa tttgattgat agagaaaccg gaatgcgcct gctggaagcc 6660
cagattgctt cagggggtgt agtagaccct gtgaacagtg tctttttgcc aaaagatgtc 6720
gccttggccc gggggctgat tgatagagat ttgtatcgat ccctgaatga tccccgagat 6780
agtcagaaaa actttgtgga tccagtcacc aaaaagaagg tcagttacgt gcagctgaag 6840
gaacggtgca gaatcgaacc acatactggt ctgctcttgc tttcagtaca gaagagaagc 6900
atgtccttcc aaggaatcag acaacctgtg accgtcactg agctagtaga ttctggtata 6960
ttgagaccgt ccactgtcaa tgaactggaa tctggtcaga tttcttatga cgaggttggt 7020
gagagaatta aggacttcct ccagggttca agctgcatag caggcatata caatgagacc 7080
acaaaacaga agcttggcat ttatgaggcc atgaaaattg gcttagtccg acctggtact 7140
gctctggagt tgctggaagc ccaagcagct actggcttta tagtggatcc tgttagcaac 7200
ttgaggttac cagtggagga agcctacaag agaggtctgg tgggcattga gttcaaagag 7260
aagctcctgt ctgcagaacg agctgtcact gggtataatg atcctgaaac aggaaacatc 7320
atctctttgt tccaagccat gaataaggaa ctcatcgaaa agggccacgg tattcgctta 7380
ttagaagcac agatcgcaac cggggggatc attgacccaa aggagagcca tcgtttacca 7440
gttgacatag catataagag gggctatttc aatgaggaac tcagtgagat tctctcagat 7500
ccaagtgatg ataccaaagg attttttgac cccaacactg aagaaaatct tacctatctg 7560
caactaaaag aaagatgcat taaggatgag gaaacagggc tctgtcttct gcctctgaaa 7620
gaaaagaaga aacaggtgca gacatcacaa aagaataccc tcaggaagcg tagagtggtc 7680
atagttgacc cagaaaccaa taaagaaatg tctgttcagg aggcctacaa gaagggccta 7740
attgattatg aaaccttcaa agaactgtgt gagcaggaat gtgaatggga agaaataacc 7800
atcacgggat cagatggctc caccagggtg gtcctggtag atagaaagac aggcagtcag 7860
tatgatattc aagatgctat tgacaagggc cttgttgaca ggaagttctt tgatcagtac 7920
cgatccggca gcctcagcct cactcaattt gctgacatga tctccttgaa aaatggtgtc 7980
ggcaccagca gcagcatggg cagtggtgtc agcgatgatg tttttagcag ctcccgacat 8040
gaatcagtaa gtaagatttc caccatatcc agcgtcagga atttaaccat aaggagcagc 8100
tctttttcag acaccctgga agaatcgagc cccattgcag ccatctttga cacagaaaac 8160
ctggagaaaa tctccattac agaaggtata gagcggggca tcgttgacag catcacgggt 8220
cagaggcttc tggaggctca ggcctgcaca ggtggcatca tccacccaac cacgggccag 8280
aagctgtcac ttcaggacgc agtctcccag ggtgtgattg accaagacat ggccaccagg 8340

ctgaagcctg ctcagaaagc cttcataggc ttcgagggtg tgaagggaaa gaagaagatg 8400

tcagcagcag aggcagtgaa agaaaaatgg ctcccgtatg aggctggcca gcgcttcctg 8460

gagttccagt acctcacggg aggtcttgtt gacccggaag tgcatgggag gataagcacc 8520

gaagaagcca tccggaaggg gttcatagat ggccgcgccg cacagaggct gcaagacacc 8580

agcagctatg ccaaaatcct gacctgcccc aaaaccaaat taaaaatatc ctataaggat 8640

gccataaatc gctccatggt agaagatatc actgggctgc gccttctgga agccgcctcc 8700

gtgtcgtcca agggcttacc cagcccttac aacatgtctt cggctccggg gtcccgctcc 8760

ggctcccgct cgggatctcg ctccggatct cgctccgggt cccgcagtgg gtcccggaga 8820

ggaagctttg acgccacagg gaattcttcc tactcttatt cctactcatt tagcagtagt 8880

tctattgggc actagtagtc agttgggagt ggttgctata ccttgacttc atttatatga 8940

atttccactt tattaaataa tagaaaagaa aatcccggtg cttgcagtag agtgatagga 9000

cattctatgc ttacagaaaa tatagccatg attgaaatca aatagtaaag gctgttctgg 9060

ctttttatct tcttagctca tcttaaataa gcagtacact tggatgcagt gcgtctgaag 9120

tgctaatcag ttgtaacaat agcacaaatc gaacttagga tttgtttctt ctcttctgtg 9180

tttcgatttt tgatcaattc tttaattttg gaagcctata atacagtttt ctattcttgg 9240

agataaaaat taaatggatc actgatattt tagtcattct gcttctcatc taaatatttc 9300

catattctgt attaggagaa aattaccctc ccagcaccag cccccctctc aaacccccaa 9360

cccaaaacca agcattttgg aatgagtctc ctttagtttc agagtgtgga ttgtataacc 9420

catatactct tcgatgtact tgtttggttt ggtattaatt tgactgtgca tgacagcggc 9480

aatcttttct ttggtcaaag ttttctgttt attttgcttg tcatattcga tgtactttaa 9540

ggtgtcttta tgaagtttgc tattctggca ataaactttt agacttttga agtgtttgtg 9600

ttttaattta atatgtttat aagcatgtat aaacatttag catattttta tcataggtct 9660

aaaaatattt gtttactaaa tacctgtgaa gaaataccat taaaaaacta tttggttctg 9720

aattcttact 9730

<210> 48
<211> 933
<212> DNA
<213> Homo sapiens

<400> 48
gcagagaggg gcgcgggggg cgggggtggt ggggcttctg gactgagccg ctgagggtgc 60

gggctgaccc tgtaagtggc tgcggcggga agatggcgga gctgcgcgtg ctcgtagctg 120

tcaagagggt catcgactac gccgtgaaga tccgagtgaa gcctgacagg accggtgtgg 180

tcacggatgg tgtgaagcac tccatgaacc ccttctgtga gatcgcggtg gaggaggctg 240

tgcggctcaa ggagaagaag ctggtgaagg aggtcatcgc cgtcagctgt gggcctgcac 300

agtgccagga gacgattcgt accgccctgg ccatgggtgc agaccgaggt atccacgtgg 360

aggtgccccc agcagaagca gaacgcttgg gtcccctgca ggtggctcgg gtcctggcca 420

agctggcaga gaaggagaag gtggacctgg tgctgctggg caaacaggcc atcgatgatg 480

actgtaacca gacagggcag atgacagctg gatttcttga ctggccacag ggcacattcg 540

cctcccaggt gacgctggag ggggacaagt tgaaagtgga gcgggagatc gatgggggcc 600

tggagaccct gcgcctgaag ctgccagctg tggtgacagc tgacctgagg ctcaacgagc 660

cccgctacgc cacgctgccc aacatcatga aagccaagaa gaagaagatc gaggtgatca 720

agcctgggga cctgggtgtg gacctgacct ccaagctctc tgtgatcagt gtggaggacc 780

cgccccagcg cacggccggc gtcaaggtgg agaccactga ggacctggtg gccaagctga 840

aggagattgg gcggatttga gcccctccca gagatggcaa taaaactgac tctcaacatc 900

tatctctgtt aacttaaaaa aaaaaaaaaa aaa 933

<210> 49
<211> 3426
<212> DNA
<213> Homo sapiens

<400> 49
gcgtgctgtg cggcgcggtc tcagggaagg tggggctatg gcagctgcta gggaccctcc 60

ggaagtatcg ctgcgagaag ccacccagcg aaaattgcgg aggttttccg agctaagagg 120

caaacttgta gcacgtggag aattctggga catagttgca ataacagcgg ctgatgaaaa 180

acaggaactt gcttacaacc aacagctgtc agaaaagctg aaaagaaagg agttacccct 240

tggagttcaa tatcacgttt ttgtggatcc tgctggagcc aaaattggaa atggaggatc 300

aacactttgt gcccttcaat gtttggaaaa gctatatgga gataaatgga attcttttac 360

catcttatta attcactctg gtggctacag tcaacgactt ccaaatgcaa gtgctctggg 420

aaaaattttc actgctttac ctcttggtaa ccccatttat cagatgctag aattaaaact 480

agccatgtac attgatttcc ccttaaatat gaatcctgga attctggtta cctgtgcaga 540

tgatattgaa ctttatagta ttggagaatt tgagtttatt aggtttgaca aacctggctt 600

tactgcttta gctcatcctt ctagtttgac gataggtacc acacatggag tatttgtctt 660

agatcctttt gatgatttaa aacatagaga ccttgaatac aggtcttgcc atcgtttcct 720

tcataagccc agcatagaaa agatgtatca gtttaatgct gtgtgtagac ctggaaattt 780

ttgtcaacag gactttgctg ggggtgacat tgccgatctt aaattagact ctgactatgt 840

ctacacagat agcctatttt atatggatca taaatcagca aaaatgttac ttgcttttta 900

tgaaaaaata ggcacactga gctgtgaaat agatgcctat ggtgactttc tgcaggcttt 960

gggacctgga gcaactgtgg agtacaccag aaacacatca aatgtcatta aagaagagtc 1020

agagttggta gaaatgaggc agagaatatt tcatcttctt aaaggaacat cactaaatgt 1080

tgttgttctt aataactcca aattttatca cattggaaca accgaagaat atttgtttta 1140

ctttacctca gataacagtt taaagtcaga gctcggctta cagtccataa cttttagtat 1200

ctttccagat ataccagaat gctctggcaa aacatcctgt atcattcaaa gcatactgga 1260

ttcaagatgt tctgtggcac ctggctcagt tgtggagtat tccagattgg ggcctgatgt 1320
ttcagttggg gaaaactgca ttattagtgg ttcttacatc ctaacaaaag ctgccctccc 1380
cgcacattct tttgtatgtt ccttaagctt aaagatgaat agatgcttaa agtatgcaac 1440
tatggcattt ggagtgcaag acaacttgaa aaagagtgtg aaaacattgt cagatataaa 1500
gttacttcaa ttctttggag tctgtttcct gtcatgctta gatgtttgga atcttaaagt 1560
tacagaggaa ctgttctctg gtaacaagac atgtctgagt ttgtggactg cacgcatttt 1620
cccagtttgt tcttctttga gtgactcagt tataacatcc ctaaagatgt taaatgctgt 1680
taagaacaag tcagcattca gcctgaatag ctataagttg ctgtccattg aagaaatgct 1740
tatctacaaa gatgtagaag atatgataac ttacagggaa caaatttttc tagaaatcag 1800
tttaaaaagc agtttgatgt agagatattt taaatattgt acactttgcc tttttgagta 1860
acattccaga gataggtatt ttttgtaggc tgtttcactg aactcagtta atgaaaactg 1920
tattaacata attgttgtag cataatatta atagtgcaaa agtacatata agtcattttg 1980
atgaaaaata ttccaagact aagttgagaa aagagatact attttggatg tgtatcagta 2040
tttttgtttt taataatgat tgatttgtgg agcattgttt tttcacataa ttagttttaa 2100
aggtaatttt ctaagcatac ctttggaatt tttccatctt ttttgaggct tttggtccag 2160
tgaagttcta agtattcact ggcacttctc tcctcaactg taattctatt tttaataata 2220
aaaatggcat actgtagggt cttcagagta gtgtaggaat actgtagaaa tactttttca 2280
gaaacgaatc catagctgac aaattcactc agtgcccaat atattgtgat tattttcgtt 2340
gataaagaac tagatacaaa gacctctgaa attgatgata aaatttgtat ctcattaatt 2400
ttatcaaaat gaaactaaaa gtacatatgt attatacact tgaacatgtg tttgtatatc 2460
tttaaaattt tcctttatgt tccattccat aggaaaacac acatatgcac acaaaactca 2520
gttatctgtg gggaaagtgg tagtaataat tgagattcat caataatcat ataatttcac 2580
tatagacatt acttgcatta tctcctatca gtccttctaa caaaacttca attaccagat 2640
gaactgactt tagttttcat cttatttttt gtccacatgc tggtgatgct gagaaacaat 2700
aattggccca aatgcagaca tcaagaagca ggcaggaaaa tggaaaaact gagataatac 2760
acaggtgata aacagtttcg agaaaataaa aattggctag aatctcaagt tatgtgtttc 2820
tatattggta taagcatata agtgaaaagt cttataagtg taatagagaa aagatttgtc 2880
agtgtgtttt tttaaatgaa ataactagtc tgtgctactt tatgtcaata taaaaattgg 2940
taaactagaa gtaacttgtc cacaaccctc agttatgata cttatgtgcg tgtttttttt 3000
tccaaagttt ttcccaagga gaagatacaa atatatggta gctattgttt aaaaatgatt 3060
gatttacttg cagatttttc agaggatgtt atgtctttgt cattcattaa atgttcaaaa 3120
ttgtagtttt accaaatctg aagtgccatc agttataaga atcaccatta ttttatgttc 3180
cattaagaaa aacacaataa aacatgacac tgcttttttt gttacttgaa attttcatat 3240
ttattggaga gctcttttag atgtttttaa atacagattt ttatttaacc ctcttgcata 3300

tacatataaa ataacaaaaa aataaaatat tgagatatca tcaagaaatt tctacagctt 3360

cacattcaaa ctcttcagca ctttcagagc tattgatatg catatgtcca cctagtacca 3420

tcttct 3426

<210> 50
<211> 2095
<212> DNA
<213> Homo sapiens

<400> 50
gggcacctgc gcgttaagag tgggccgcgt cgctgagggg tagcgatgcg ggctccgggg 60

atgaggtcgc ggccggcggg tcccgcgctg ttgctgctgc tgctcttcct cggagcggcc 120

gagtcggtgc gtcgggccca gcctccgcgc cgctacaccc cagactggcc gagcctggat 180

tctcggccgc tgccggcctg gttcgacgaa gccaagttcg gggtgttcat ccactggggc 240

gtgttctcgg tgcccgcctg gggcagcgag tggttctggt ggcactggca gggcgagggg 300

cggccgcagt accagcgctt catgcgcgac aactacccgc ccggcttcag ctacgccgac 360

ttcggaccgc agttcactgc gcgcttcttc cacccggagg agtgggccga cctcttccag 420

gccgcgggcg ccaagtatgt agttttgacg acaaagcatc acgaaggctt cacaaactgg 480

ccgagtcctg tgtcttggaa ctggaactcc aaagacgtgg ggcctcatcg ggatttggtt 540

ggtgaattgg gaacagctct ccggaagagg aacatccgct atggactata ccactcactc 600

ttagagtggt tccatccact ctatctactt gataagaaaa atggcttcaa aacacagcat 660

tttgtcagtg caaaaacaat gccagagctg tacgaccttg ttaacagcta taaacctgat 720

ctgatctggt ctgatgggga gtgggaatgt cctgatactt actggaactc cacaaatttt 780

ctttcatggc tctacaatga cagccctgtc aaggatgagg tggtagtaaa tgaccgatgg 840

ggtcagaact gttcctgtca ccatggagga tactataact gtgaagataa attcaagcca 900

cagagcttgc cagatcacaa gtgggagatg tgcaccagca ttgacaagtt ttcctggggc 960

tatcgtcgtg acatggcatt gtctgatgtt acagaagaat ctgaaatcat ttcggaactg 1020

gttcagacag taagtttggg aggcaactat cttctgaaca ttggaccaac taaagatgga 1080

ctgattgttc ccatcttcca agaaaggctt cttgctgttg ggaaatggct gagcatcaat 1140

ggggaggcta tctatgcctc caaaccatgg cgggtgcaat gggaaaagaa cacaacatct 1200

gtatggtata cctcaaaggg atcggctgtt tatgccattt ttctgcactg gccagaaaat 1260

ggagtcttaa accttgaatc ccccataact acctcaacta caaagataac aatgctggga 1320

attcaaggag atctgaagtg gtccacagat ccagataaag gtctcttcat ctctctaccc 1380

cagttgccac cctctgctgt ccccgcagag tttgcttgga ctataaagct gacaggagtg 1440

aagtaatcat ttgagtgcaa gaagaaagag gcgctgctca ctgttttcct gcttcagttt 1500

ttctcttata gtaccatcac tataatcaac gaacttctct tctccaccca gagatggctt 1560

ttccaacaca ttttaattaa aggaactgag tacattaccc tgatgtctaa atggaccaaa 1620

gatctgagat ccattgtgat tatatctgta tcaggtcagc agaagaagga actgagcagt 1680
tgaactctga gttcatcaat tgtaatattt ggaaattatc tacaatggaa tcttccctct 1740
gttctctgat aacctacttg cttactcaat gcctttaagc caagtcaccc tgttgcctat 1800
gggaggaggt ggaaggattt ggcaagctca accacatgct atttagttag catcagttgt 1860
caccaacagt ctttctgcaa agggcaggag agctttgggg gaaaggaaaa ggcttaccag 1920
gctgctatgg tcaactcttc agaaattttc agagcaatct aaaagcgcca aaattcgcta 1980
tgtttacagt gatactatta agaaaatgaa tgtgattctg ctctgtcttt ttaagtatga 2040
tcaaataaaa aatttgtaca tcacaatcat ttctaccaaa aaaaaaaaaa aaaaa 2095

<210> 51
<211> 3923
<212> DNA
<213> Homo sapiens

<400> 51
aaagagcccg ctccgccgag cgccgggcga cggcaggaag cccctccgct tggccagaat 60
ggagcagccg aagggagttg attggacagt catcatcctg acctgccagt acaaggacag 120
tgtccaggtc tttcagagag aactggaagt gcggcagaag cgggagcaga tccctgctgg 180
gacgctgtta ctggccgtgg aggacccaga gaagcgtgtg ggcagcggag gagccaccct 240
caacgccctg ctggtggctg ctgaacacct gagtgcccgg gcaggcttca ctgtggtcac 300
atccgatgtc ctgcactcgg cctggatcct cattctgcac atgggtcgag acttcccctt 360
tgatgactgt ggcagggctt tcacctgcct ccccgtggag aaccccgagg cccccgtgga 420
agccttggtc tgcaacctgg actgcctgct ggacatcatg acctatcggc tgggcccggg 480
ctccccgcca ggcgtgtggg tctgcagcac cgacatgctg ctgtctgttc ctgcaaatcc 540
tggtatcagc tgggacagct tccggggagc cagagtgatc gccctcccag ggagcccggc 600
ctacgctcag aatcatggcg tctacctaac tgacccccag ggccttgttt tggacattta 660
ctaccagggc actgaggcag agattcagcg gtgtgtcagg cctgatgggc gggtgccact 720
ggtctctggg gttgtcttct tctctgtgga gactgccgag cgcctcctag ccacccacgt 780
gagcccgccc ctggatgcct gcacctacct aggcttggac tccggagccc ggcctgtcca 840
gctgtctctg ttttttgaca ttctccactg catggctgag aacgtgacca gggaggactt 900
cctggtgggg aggcccccag agttggggca aggcgatgca gatgtagcgg gttatctgca 960
gagcgcccgg gcccagctgt ggagggagct tcgcgatcag ccccttacca tggcctatgt 1020
ctccagcggc agctacagct acatgacctc ctcagccagt gagttcctgc tcagcctcac 1080
actccccggg gctcctgggg cccagattgt gcactcccag gtggaggagc agcagcttct 1140
ggcggccggg agctctgtgg tcagctgcct gctggagggc cctgtccagc tgggtcctgg 1200
gagcgtcctg cagcactgcc acctgcaggg ccccattcac ataggcgctg gctgcttggt 1260
gactggcctg gatacagccc actccaaggc cctgcatggc cgggagctgc gtgaccttgt 1320
cctgcaggga caccacacgc ggctacacgg ctccccgggc cacgccttca ccctcgttgg 1380

ccgtctggac agctgggaga gacagggggc aggcacatat ctcaacgtgc cctggagtga 1440
attcttcaag aggacaggtg ttcgagcctg ggacctgtgg gaccctgaga cgctgcccgc 1500
agagtactgc cttcccagcg cccgcctctt tcctgtgctc cacccctcga gggagctggg 1560
accccaggac ctgctgtgga tgctggacca ccaggaggat gggggcgagg ccctgcgagc 1620
ctggcgggcc tcctggcgcc tgtcctggga gcagctgcag ccgtgcctgg atcgggctgc 1680
cacgctggcc tctcgccggg acctgttctt ccgccaggcc ctgcataagg cgcggcacgt 1740
gctggaggcc cggcaggacc tcagcctgcg cccgctgatc tgggctgctg tccgcgaggg 1800
ctgccccggg cccctgctgg ccacgctgga ccaggttgca gctggggcag gagaccctgg 1860
tgtggcggca cgggcactgg cctgtgtggc ggacgtcctg ggctgcatgg cagagggccg 1920
tgggggcttg cggagcgggc cagctgccaa ccctgagtgg atgcggccct tctcatacct 1980
ggagtgtgga gacctggcag cgggcgtgga ggcgcttgcc caggagaggg acaagtggct 2040
aagcaggcca gccttgctgg tgcgagcggc ccgccactat gagggggctg gtcagatcct 2100
gatccgccag gctgtgatgt cagcccagca ctttgtctcc acagagcagg tggaactgcc 2160
gggacctggg cagtgggtgg tggctgagtg cccggcccgt gtggatttct ctgggggctg 2220
gagtgacacg ccaccccttg cctatgagct tggcggggct gtgctgggcc tggctgtgcg 2280
agtggacggc cgccggccca tcggagccag ggcacgccgc atcccggagc ctgagctgtg 2340
gctggcggtg gggcctcggc aggatgagat gactgtgaag atagtgtgcc ggtgcctggc 2400
tgacctgcgg gactactgcc agcctcatgc cccaggggcc ctgctgaagg cggccttcat 2460
ctgtgcaggg atcgtgcatg tccactcgga actccagctg agtgagcagc tgctccgcac 2520
cttcgggggc ggctttgagc tgcacacctg gtctgagctg ccccacggct ctggcctggg 2580
caccagcagc atcctggcag gcactgccct ggctgccttg cagcgagccg caggccgggt 2640
ggtgggcacg gaagccctga tccacgcagt gctgcacctg gagcaggtgc tcaccactgg 2700
aggtggctgg caggaccaag taggtggcct aatgcctggc atcaaggtgg ggcgctcccg 2760
ggctcagctg ccactgaagg tggaggtaga agaggtcacg gtgcctgagg gctttgtcca 2820
gaagctcaat gaccacctgc tcttggtgta cactggcaag acccgcctgg ctcggaacct 2880
gctgcaggat gtgctgagga gctggtatgc ccgacttcct gctgtggtgc agaatgccca 2940
cagcctggta cggcaaactg aggagtgtgc tgaaggcttc cgccaaggaa gcctgcctct 3000
gctgggccag tgcctgacct cgtactggga gcagaagaag ctcatggctc caggctgtga 3060
gcccctgact gtgcggcgta tgatggatgt cctggccccc cacgtgcatg gccagagcct 3120
ggctggggca ggcggtggag gctttctcta tctgttgacc aaggagccac agcaaaagga 3180
ggccttggag gcggtgctgg ccaagaccga gggccttggg aattacagca tccacctggt 3240
tgaagtggac actcagggcc tgagcctgaa gctgctgggg accgaggcct caacctgttg 3300
ccctttccca tgaagctggc ttctctctgc aacaggagaa aacctggagc tacagtgtcc 3360
cccaccttcc ttgccccatg ggaacctcca cctcctactc cccacccacc tctgcgaatc 3420

tgctcccaaa ggaagctgac cagagcaaga tctgggcaag cagagagtgc ctgggacagg 3480
actgtgacct ggtggacagg ggcctagatg tagcctctgt tcctcctgga cataggaagg 3540
tcccaagctt agtatcccac gtggccttta caaatcctat ggctggcctt ctcattccac 3600
aagggccctg gaaagggttg acagccagcc ttggcatatg gctgggagtc ccttagcaag 3660
gccaaccctg aagaggccct ttgaggcatt ccctatggct tagagttgta gacttacact 3720
caaccctcat gtgagcgtgg gagtgagggt ggcggtccct tgccaagttg gtagcagtga 3780
cccagtgatt cactgccatc ccaggcctta actagcaaaa ctacggagcg tgccaagtga 3840
cctggtgcct gtgggaagtg ggttctcagg actggcattc ttggaataaa ttcactctgt 3900
ccttgcaaaa aaaaaaaaaa aaa 3923

<210> 52
<211> 4244
<212> DNA
<213> Homo sapiens

<400> 52
gaaagtccct gactggagtt ggcagccaag ccaggccctg gagtgggcac ccagagggaa 60
gacaggttgg ctaatttcct ggagccccta agggtgcaag ggggaagaac acccacccgc 120
aaagccccgt agctggaccc tacgttagcc tgccctgctc ggggttggcg atgctggagg 180
tgggccttgg accagagaaa atgctttaat taggtgacaa gcgggcagag gcctttgtct 240
ctggagccgg cagccacggc ccccgctgac ggcgtgggaa acagaccctg ttccactccg 300
gtctccagcc ttggaatggt tgccttcgtg cagtgcagga ggacgcggca gagggcggac 360
gatcgctcca ctcgccggga ccaggtgcgg gggccctgcc cagccgctgg ggcgtggcca 420
ggctcgaagc acccaggtgt cgggggccga ctctaagccc tggcaccgga agagagaggg 480
cggcggattg gacctcccgg ctccagcatt gcaactgggc gctccgtctc ctggtccacg 540
caatgatggt gcggctgctc agaagccagg tagcctgccc tgggtgaagc cttcgcgcag 600
gtcaatgacg gggcggaggg gcagggcgcg gtcccctgca tccccgatct ggggagcggt 660
gggcccaggg gccatcgcct tagcccctgg cgctggggct cggcgccaag tgacgggcgg 720
ggctccacct tccagccatc cgcccggccc gggagggcgg acgctgcgag actcccggcc 780
gcgccctctc cttcctctcc tccccaagcc ctcgctgcca gtccggacag gctgcgcgga 840
ggggagggct gccgggccgg atagccggac gcctggcgtt ccaggggcgg ccggatgtgg 900
cctgcctttg cggagggtgc gctccggcca cgaaaagcgg actgtggatc tgccacctgc 960
aagcagctcg gccatgtggc tccggagcca tcgtcagctc tgcctggcct tcctgctagt 1020
ctgtgtcctc tctgtaatct tcttcctcca tatccatcaa gacagctttc cacatggcct 1080
aggcctgtcg atcctgtgtc cagaccgccg cctggtgaca cccccagtgg ccatcttctg 1140
cctgccgggt actgcgatgg gccccaacgc ctcctcttcc tgtccccagc accctgcttc 1200
cctctccggc acctggactg tctaccccaa tggccggttt ggtaatcaga tgggacagta 1260

tgccacgctg ctggctctgg cccagctcaa cggccgccgg gcctttatcc tgcctgccat 1320
gcatgccgcc ctggccccgg tattccgcat caccctgccc gtgctggccc cagaagtgga 1380
cagccgcacg ccgtggcggg agctgcagct tcacgactgg atgtcggagg agtacgcgga 1440
cttgagagat cctttcctga agctctctgg cttcccctgc tcttggactt tcttccacca 1500
tctccgggaa cagatccgca gagagttcac cctgcacgac caccttcggg aagaggcgca 1560
gagtgtgctg ggtcagctcc gcctgggccg cacaggggac cgcccgcgca cctttgtcgg 1620
cgtccacgtg cgccgtgggg actatctgca ggttatgcct cagcgctgga agggtgtggt 1680
gggcgacagc gcctacctcc ggcaggccat ggactggttc cgggcacggc acgaagcccc 1740
cgttttcgtg gtcaccagca acggcatgga gtggtgtaaa gaaaacatcg acacctccca 1800
gggcgatgtg acgtttgctg gcgatggaca ggaggctaca ccgtggaaag actttgccct 1860
gctcacacag tgcaaccaca ccattatgac cattggcacc ttcggcttct gggctgccta 1920
cctggctggc ggagacactg tctacctggc caacttcacc ctgccagact ctgagttcct 1980
gaagatcttt aagccggagg cggccttcct gcccgagtgg gtgggcatta atgcagactt 2040
gtctccactc tggacattgg ctaagccttg agagccaggg agactttctg aagtagcctg 2100
atctttctag agccagcagt acgtggcttc agaggcctgg catcttctgg agaagcttgt 2160
ggtgttcctg aagcaaatgg gtgcccgtat ccagagtgat tctagttggg agagttggag 2220
agaaggggga cgtttctgga actgtctgaa tattctagaa ctagcaaaac atcttttcct 2280
gatggctggc aggcagttct agaagccaca gtgcccacct gctcttccca gcccatatct 2340
acagtacttc cagatggctg cccccaggaa tggggaactc tccctctggt ctactctaga 2400
agaggggtta cttctcccct gggtcctcca aagactgaag gagcatatga ttgctccaga 2460
gcaagcattc accaagtccc cttctgtgtt tctggagtga ttctagaggg agacttgttc 2520
tagagaggac caggtttgat gcctgtgaag aaccctgcag ggcccttatg gacaggatgg 2580
ggttctggaa atccagataa ctaaggtgaa gaatcttttt agtttttttt tttttttttt 2640
ggagacaggg tctcgctctg ttgcccaggc tggagtgcag tggcgtgatc ttggctcact 2700
gcaacttccg cctcctgtgt tcaagcgatt ctcctgtctc agcctcctga gtagatggga 2760
ctacaggcac aggccattat gcctggctaa tttttgtatt tttagtagag acagggtttc 2820
accatgttgg ccaggatggt ctcgatctcc tgaccttgtc atccacctgt cttggcctcc 2880
caaagtgctg ggattactgg catgagccac tgtgcccagc ccggatattt tttttttaat 2940
tatttattta tttatttatt tattgagacg gagtcttgct ctgtagccca ggccagagtg 3000
cagtggcgcg atctcagctc actgcaagct ctgcctcccg ggttcatgcc attctgcctc 3060
agcctcctga gtagctggga ctacaggcgc ccgccaccac gcccggctaa ttttttttgt 3120
atttttagta gagacggggt ttcatcgtgt taaccaggat ggtctcgatc tcctgacctc 3180
gtgatctgcc cacctcggcc tcccacagtg ctgggattac cggcgtgagc caccatgcct 3240
ggcccggata atttttttta atttttgtag agacgaggtc ttgtgatatt gcccaggctg 3300

ttcttcaact cctgggctca agcagtcctc ccaccttggc ctcccagaat gctgggttta 3360
tagatgtgag ccagcacacc gggccaagtg aagaatctaa tgaatgtgca acctaattgt 3420
agcatctaat gaatgttcca ccattgctgg aaaaattgag atggaaaaca aaccatctct 3480
agttggccag cgtcttgctc tgttcacagt ctctggaaaa gctggggtag ttggtgagca 3540
gagcgggact ctgtccaaca agccccacag cccctcaaag actttttttt gtttgttttg 3600
agcagacagg ctaaaatgtg aacgtggggt gagggatcac tgccaaaatg gtacagcttc 3660
tggagcagaa ctttccaggg atccagggac actttttttt aaagctcata aactgccaag 3720
agctccatat attgggtgtg agttcaggtt gcctctcaca atgaaggaag ttggtctttg 3780
tctgcaggtg ggctgctgag ggtctgggat ctgttttctg gaagtgtgca ggtataaaca 3840
caccctctgt gcttgtgaca aactggcagg taccgtgctc attgctaacc actgtctgtc 3900
cctgaactcc cagaaccact acatctggct ttgggcaggt ctgagataaa acgatctaaa 3960
ggtaggcaga ccctggaccc agcctcagat ccaggcagga gcacgaggtc tggccaaggt 4020
ggacggggtt gtcgagatct caggagcccc ttgctgtttt ttggagggtg aaagaagaaa 4080
ccttaaacat agtcagctct gatcacatcc cctgtctact catccagacc ccatgcctgt 4140
aggcttatca gggagttaca gttacaattg ttacagtact gttcccaact cagctgccac 4200
gggtgagaga gcaggaggta tgaattaaaa gtctacagca ctaa 4244

<210> 53
<211> 3549
<212> DNA
<213> Homo sapiens

<400> 53
   gtcttcccgg cgcccaggct tgggctcata gggagaaagg aagctgcgct gcattctgcg 60
   ggacgaaccc tgctccgcgc gagaattttt ttgattcctt cttatttgga gaaatctcca 120
   gctgctctga tgatagccta agaagactgc atgctgcttc ctctcgatgc caagccagac 180
   cctctcacaa cctcggatct cagtccttca tggagacctg gtcccagcag gaatggcagt 240
   gcaggaaatt ggcgcccaga tggttcttcc atgtgaagtt gtctcgggct ctgggctgac 300
   gagagaacac ctggtaacca ggttagccct ctgtcagtca cccagggcag ggcagcatgg 360
   tgcggattca gaggaggaag cttttggcat cttgcctgtg cgtcacagcc accgtctttc 420
   tgcttgtcac actccaggtc atggttgagc tggggaagtt tgaaaggaag gagtttaaaa 480
   gttccagttt gcaagatgga catacaaaaa tggaggaagc acctacgcat cttaattcat 540
   ttcttaagaa agaaggattg accttcaaca ggaaaagaaa atgggaattg gacagctacc 600
   ccattatgct ctggtggtcc ccgctgacgg gggagactgg gaggttaggc caatgtggag 660
   cagatgcttg tttcttcacc atcaaccgga cctacctcca tcatcacatg accaaagcat 720
   tcctcttcta tggtactgac tttaacatag atagcttacc tctgcctcgg aaagcccatc 780
   atgactgggc tgtttttcat gaagagtccc cgaaaaacaa ttataagctc tttcataaac 840
   cagtgatcac cttgttcaac tacactgcca cgttcagcag gcattcccac ttgccactaa 900
   ctacccaata cttggagagc attgaagtcc tgaagtcact ccgataccta gttcctttgc 960
   agtccaaaaa caagcttaga aaaagacttg ctccgctggt gtatgtacag tcagactgtg 1020
   acccaccatc agacagggac agctatgttc gcgagctgat gacttacatc gaggtcgatt 1080
   cctatggtga atgtttacga aacaaagacc tccctcagca gctgaaaaat ccagcctcta 1140
   tggatgccga tggcttttat aggatcattg cacagtataa gtttatccta gcttttgaga 1200
   atgcagtttg tgatgactac atcactgaga agttctggag gccactgaaa ctgggggtag 1260
   tccctgtata ttacggatcc cccagcatca cagactggct tccaagtaac aaaagtgcta 1320
   ttcttgtatc agaattttct caccccaggg aactggcaag ttacatcaga cgactggatt 1380
   ctgatgacag attgtatgag gcctatgtag aatggaagct gaagggtgag atctctaacc 1440
   agcgacttct gacagctctc agggaacgga aatggggagt gcaagacgtc aaccaggaca 1500
   attacatcga tgcatttgag tgtatggtgt gcaccaaggt gtgggctaat atcaggcttc 1560
   aggaaaaggg cttaccaccc aaaagatggg aggcagaaga tacccacctg agttgcccag 1620
   agcccacagt gtttgctttc tcaccactcc ggactccacc tttgagctct ttgcgagaga 1680
   tgtggatttc cagctttgaa caatccaaga aagaagccca ggcactaagg tggctggttg 1740
   ataggaatca aaacttttca tctcaagagt tttggggcct agtattcaag gactgatttc 1800
   aaaaatgatc agaatgaaac agactagagc cttcttgagg tttattttct aggttgcctt 1860
   aatatttgaa cataatagct attctgttga ctatccatca ggataataat tagttgctgc 1920
   agtactcata atgagccctt tcaaggaata gatgtaaatt acccttaggg gtagccacta 1980
   tattttttat actaaaaaag gaaatatttc ttatggacac tggttatctt ccatgtccgt 2040
   gtctgtggac ccacatactt actgcctttt gtggaaattc acctagagtg agtactttcc 2100
   ttgtgcaatg gagggagacg atctagctat agattcttga tactcacctc actctcaaca 2160
   gcttattggt atttggcaaa cctggtatcc tgcagccgag catgaacatg aaatacatta 2220
   gaacttggca atgagagatc ccatctgttg gtttccaggg atgtaagtga gtaataaaag 2280
   ctgtttcaag tttatccact atccactctt caagtgaaaa gaacttatgt gtgtctacct 2340
   tcttctccat accctgagcc acctctctca ctttctttag ttgtgccttg ccctatcgcc 2400
   tcccattttc ccttcaaagt agaatgtctt gtaaagacac agtaactcct gtgtgttttg 2460
   attagtgaga tgttaccact taccccttct gctgcgcaga ataatattaa gaattgctat 2520
   gcttatgatt caggctggac agcctggaca ttttgagcag cgcgagagta gaagtgaata 2580
   cccccaggaa ctccaattgt gctgaagctg atttgtctgt tcgtaggatg atgtgcagcc 2640
   tgtcacagtg gcagccacag gcattcagtt tgtgggcatt tcctgtcatt tcggatcctg 2700
   ctatagggaa ggttcagctc tcctgtctgg ggcatctact ggagcacctc agaagacaag 2760
   gaggaaaaca ctggctcatg ttttctttcc atgtgaggga ggggaggttt ctagatgata 2820
   ctaaaagcct gttgccactg ttgtgtcatg atttccattc ttcatcatgg aaagccacaa 2880
   gcctcagaag gcagccagag gacaggaaga gtctgtgatg ctcatgtctg tgtcatggca 2940
   tgagggggaa ttgacgagtg cttgcttagc atcagctctc attgtattga aggctgtgcc 3000
   cttggtgtgc ttagcagtga gcagcgtctt cattaggtag ctctggctgg gctttgtcag 3060
   ccagcgtgtg agaaaggaga accaaaacca atacctcctt atagaagcca gaaggatcac 3120
   attggatgag agaaggtgtc tataactggg ttcttcccaa tcccagctct ttcattaact 3180
   aaatatgatc ttggacatat ctcttaacct ccatcggcct tagtttttgc atccaaaaag 3240
   aagtgattgg gctggtcttg gaaggttttt ttcttttttt tcccctgtgt aacattcttc 3300
   attgttcatc aacatttaag aaatttaatt ttgtagatgt ttttactaaa gagggtaaca 3360
   aactattttg gaactgctgt atgttattca agggaagcac gctgtttgga ggaaggagca 3420
   tacttaatgg gtagtattga ggatgaaagg gaatttctct ctctgccatt ttatttgata 3480
   atattaaaga aataaagact ggaaaggggg attccttgtt aaaggtcaga ttttaaattg 3540
   catataaaa 3549

<210> 54
<211> 2069
<212> DNA
<213> Homo sapiens

<400> 54
ggggtcggtg ctggccgagg gggcgccggc tgccggagtg gacatggcgg ccggccccat 60
tagggtggtg ttggtccttc taggggtgct cagtgtctgt gcagccagcg gccatgggtc 120
cgtagcggag agggaggccg gcggggaggc ggagtgggcg gaaccgtggg atggcgcggt 180
tttccggccg ccctcggcgc tgggcgcagt gggggtgacg cgcagctctg ggacgccgcg 240
gccagggagg gaggaggcgg gggacttgcc ggtactgctg tggtggagcc cagggctatt 300
cccccacttc ccgggagact cggagcgcat cgagtgtgcg cgcggcgcgt gcgtggcgtc 360
ccggaaccgc cgagcgctga gggactcgcg gacgcgcgcg ctgctcttct acggcacaga 420
cttccgcgcg tcggccgccc cgctgccgcg cctggcgcac cagagctggg cgctcctcca 480
cgaggagtcg cccctcaaca acttcttgct gagccacggc ccgggcatcc gcctcttcaa 540
tcttacctcc accttcagtc gccactcgga ttacccgctg tcgctgcagt ggctgcccgg 600
gaccgcctat ctgcgccgcc cggtgcctcc gcccatggaa cgcgcggagt ggcgccgccg 660
cggctacgcg ccgctgctct atctgcagtc acactgcgac gtgccagcgg accgggaccg 720
ctacgtgcgc gagctcatgc gccacatccc ggtagactcc tacgggaaat gcctgcagaa 780
tcgggagctg cctaccgcgc ggctacagga cacagccacg gccaccaccg aggatccaga 840
gctcttggct ttcttgtccc gctataagtt ccacttggcc ctggaaaatg ccatctgtaa 900
cgactacatg acagaaaaac tgtggcgtcc catgcacctg ggcgctgtgc ccgtgtaccg 960
cggttctccc tctgtgaggg actggatgcc gaacaatcac tccgtcatcc tgattgatga 1020
ttttgagtct cctcagaagc tggcagagtt tattgacttt ctggacaaga atgatgagga 1080
gtatatgaaa tacctggcat acaagcaacc tgggggcatc accaaccaat ttcttctgga 1140

tagtctgaag catcgggagt ggggagtgaa tgatcctttg ctgcctaact acctcaacgg 1200
cttcgagtgt ttcgtctgtg actacgaact ggctcggctg gatgccgaga aagcccacgc 1260
ggcctctccc ggggacagcc ccgtctttga gccccacatt gcccagccct cacacatgga 1320
ctgcccagtg cccacacctg gctttggcaa tgtggaagag attcctgaga atgacagttg 1380
gaaagagatg tggctgcaag attattggca aggtctggac cagggggaag ctctcactgc 1440
catgatccac aacaatgaaa cagagcagac gaaattttgg gattacctac atgaaatctt 1500
catgaagagg caacatctct aagtgccctt gcaagagcct ttaacttggc ggagctaagg 1560
agatcttatt ctaccatggg acataaggag catccactgc acaaaccctt aatgaacact 1620
gtcttttcat ggattcaagg aattccagtt ttatctatta agattttatc ttaatgatga 1680
gtagccaagg tctaacatag ggcctctcct caaggagaga tggagggata caattcttgg 1740
ttcagtggga aacagaaccc taaaacatcc atttgattca aggtgctggt ccaacagagt 1800
ttttaaacta ctcacttctt tatttcatcc tttcgactgt acttgattac cagtgaagta 1860
agatgggtca ggttacgact tacaactttt gttctattcc ccagactcct cattattcag 1920
tacatttccc aataatctct ttttctcatc tcttgcttta taaattgtta cgttggtgga 1980
gaagcaaaac atttggtgag ttgtattctg gttttccgga gttggatttt tttatattat 2040
atactttcat gtcaaaaaaa aaaaaaaaa 2069

<210> 55
<211> 3088
<212> DNA
<213> Homo sapiens

<400> 55
gctggaaggg tttctttggc cctgagtgaa gagagaccca gagggaacac tgaggtgcct 60
gcccaaccac tctgtcccgg tttccttcag caggaccagg tgagagaagc catgctggtc 120
gttcagatgc ctttctcctt tcccatggcc cacttcatcc tctttgtctt tacggtttcc 180
actatatttc acgttcagca gcggctagcg aagattcaag ccatgtggga gttaccggtg 240
cagataccag tgctagcctc aacatcaaag gcactgggac ccagccagct cagggggatg 300
tggacgatca atgcaatagg ccgcctgggg aaccagatgg gcgagtacgc cacactgtac 360
gccctggcca agatgaacgg gcggcccgcc ttcatcccgg cccagatgca cagcaccctg 420
gcccccatct tcagaatcac cctgccggtg ctgcacagcg ccacggccag caggatcccc 480
tggcagaact accacctgaa cgactggatg gaggaggaat accgccactt cccgggggag 540
tacgtccgct tcaccggcta cccctgctcc tggaccttct accaccacct ccgccaggag 600
atcctccagg agttcaccct gcacgaccac gtgcgggagg aggcccagaa gttcctgcgg 660
ggcctgcagg tgaacgggag ccggccgggc acctttgtag gggtccatgt tcgccgaggg 720
gactatgtcc atgtcatgcc aaaagtgtgg aagggggtgg tggccgaccg gcgataccta 780
cagcaggccc tggactggtt ccgagctcgc tacagctccc tcatcttcgt ggtcaccagt 840
aatggcatgg cctggtgtcg ggagaacatt gacacctccc acggtgatgt ggtgtttgct 900

ggcgatggca ttgagggctc acctgccaaa gattttgctc tactcacaca gtgtaaccac 960
accatcatga ccattgggac gttcgggatc tgggccgcat acctcacggg cggagacacc 1020
atctacctgg ccaattacac cctccccgac tcccctttcc tcaaaatctt taagccagag 1080
gcagccttcc tgccggagtg gacagggatt gccgcagacc tgtccccctt actcaagcac 1140
taatgctggc ccattctttg agaccttttc tccttctctg cctccctcaa gatgagtgcc 1200
cgggcatgag aagcacatgg ttccatgagc aggacccatc tctcttctgt gaagatgcgt 1260
tgggctgcaa gtaacagaaa tctcagtgaa cagtggcctg gcgtggtggc tcatgcctgt 1320
aatgctcgca ctttgggagg ccagggtggg tggatcactt gaggtcagga gttcaagact 1380
agcctggcca acatggtgaa accccatctc gactaaaaat acaaaaatta gccaggcgtg 1440
gtggtgcaca cttgtaatcc cagctactcg ggaggctgag gcaagagaat cacttgaacc 1500
caggaggcgg aggttgcagt gagccaagat ggtgccgctg cactccagcc tgggtgacac 1560
agcaagactc catctcaaaa aaaaaaaaag aaaaagaaat gaacgggttc aaagaccata 1620
atcatgcata tcacataaga ccagaagtgg cccaggtcca gggtcagtta atttagcagc 1680
tccacaaagt catcagtcac ctgagctcca tccatcttca catgctgtgc taccatttct 1740
tagctgtatc atcccatggt cccaaaaggg ctgctacaca tccagccatc acatgcagat 1800
aattcctttc aaaaacagca gaaagaggct cgttcttgtc ttggtccctt ttgaagaatg 1860
aatgaaacct tcctaagcct tccagcaatt tccccccaac tccgatgggt aggaattgtc 1920
acatacccat gtgacccgat aggaggcaaa agaaatgaga cttctgggat tagtttagcc 1980
tcagattctg cagctgagaa gttgatcagc cacctctgaa ggacatgcag cttgcagaaa 2040
attagggtgg tgttaccaag gtgaaaaggg gaaatggctt tagagtagac aacagagatg 2100
ccctgagggg ttgtgtaggt tgttcactgc aggaagtccc ctggttaaga aggcaagtgg 2160
ggtttaaaca gacccacagt ctactcatca aaccaggtgt ccttggcatt gtgtccaccc 2220
agagagctca ctgttttctt ttctttttct tttctttttt tttttttgag atggagtctt 2280
gctgcatccc ccaggctgga gtgcagtggc atgatcttgg ctcactgcag cctccgcctc 2340
ccaggttcag gcgattctcc tgcctcagcc tcccgagtgg ctgggattgc aggtgcgtgc 2400
caccacgccc agctaatttt gtacgtttag tggaaatgga gtttcaccat gttggtcagg 2460
ctggtctcaa actcctgacc tcatgatccg ccttcctcgg cctcccaagg tgctgggatt 2520
acaggtgtta gccactgcgc ccggccctag agctcactgt tttctagtta gtccatctgg 2580
aagtggagcc tttttccagt ttgcacaaat gtgccatatt ggcttgtagc tggcatgcat 2640
ccaagtccat aggtcctgcc tcttcaatcc tggctttcta gggcctggga tgatcattgc 2700
tagaactgag agaccagcct ggctgagtga acttcagggc gttccgttca ttctttcagt 2760
aaatggttgc agcacatgtt ttacatgtca ggcagtgaaa ccccccacag cagccttccc 2820
tctcagagga tacatttgta accattacac agtcatcaaa ggaataattt tttttaatca 2880
ccagtgtgca tacagtcatg gagctgggta ttcccagcta ccagggaggc tgaggtggga 2940

ggattgcttg atgccaggag ttagggaata tagtgcaccg tgattggact tgcgaatagc 3000

cactgcactg cggcctggac gacgtagtga taccctgact cttataaata aataaatgaa 3060

taaacacaat tatgactttg cggatggg 3088

<210> 56
<211> 2581
<212> DNA
<213> Homo sapiens

<400> 56
gttcccactt cctcccgccc caggaaacct gccatggcct cctggtgagc tgtcctcatc 60

cactgctcgc tgcctctcca gatcttcagt tgcttcaggc cactttgaat gtatatgagc 120

cggtcgtagg ggatatcgat ggcttagctt gggctcagag gcctgaaaat cgcccccacc 180

aatcacctgt ttcccccaat ctaccctcct gaaggtcact gacaaagact tcattgtctc 240

ctaggagagg ctgccatata tcagggctga cgtaattcca tcttaatatc agttacatta 300

taaaaattta cctcgtgcct gaggccccag agcccaaggg tgcaaagcag taattagtca 360

aagttcaact tcccctccca ctctgggctc aggctgtccc tgagggcctg tgttttgagt 420

ctctttccag aaccttggtg tgaacttagg tcttggcgtc gggatccctt ttcgtcacac 480

tcaggtgacc tacaggctcc gctcgacact gcaaggctta gaccagttcg gtccaacaga 540

gaaagcaggc aaccaccatg tcatttgaaa acagtttcat cgggatataa ttcgcaaccc 600

atacagtgaa tccatttaag atactctgac ccatggatcc cctgggtgca gccaagccac 660

aatggccatg gcgccgctgt ctggccgcac tgctatttca gctgctggtg gctgtgtgtt 720

tcttctccta cctgcgtgtg tcccgagacg atgccactgg atcccctagg gctcccagtg 780

ggtcctcccg acaggacacc actcccaccc gccccaccct cctgatcctg ctatggacat 840

ggcctttcca catccctgtg gctctgtccc gctgttcaga gatggtgccc ggcacagccg 900

actgccacat cactgccgac cgcaaggtgt acccacaggc agacacggtc atcgtgcacc 960

actgggatat catgtccaac cctaagtcac gcctcccacc ttccccgagg ccgcaggggc 1020

agcgctggat ctggttcaac ttggagccac cccctaactg ccagcacctg gaagccctgg 1080

acagatactt caatctcacc atgtcctacc gcagcgactc cgacatcttc acgccctacg 1140

gctggctgga gccgtggtcc ggccagcctg cccacccacc gctcaacctc tcggccaaga 1200

ccgagctggt ggcctgggcg gtgtccaact ggaagccgga ctcagccagg gtgcgctact 1260

accagagcct gcaggctcat ctcaaggtgg acgtgtacgg acgctcccac aagcccctgc 1320

ccaaggggac catgatggag acgctgtccc ggtacaagtt ctacctggcc ttcgagaact 1380

ccttgcaccc cgactacatc accgagaagc tgtggaggaa cgccctggag gcctgggccg 1440

tgcccgtggt gctgggcccc agcagaagca actacgagag gttcctgcca cccgacgcct 1500

tcatccacgt ggacgacttc cagagcccca aggacctggc ccggtacctg caggagctgg 1560

acaaggacca cgcccgctac ctgagctact ttcgctggcg ggagacgctg cggcctcgct 1620

ccttcagctg ggcactggat ttctgcaagg cctgctggaa actgcagcag gaatccaggt 1680
accagacggt gcgcagcata gcggcttggt tcacctgaga ggccggcatg gtgcctgggc 1740
tgccgggaac ctcatctgcc tggggcctca cctgctggag tcctttgtgg ccaaccctct 1800
ctcttacctg ggacctcaca cgctgggctt cacggctgcc aggagcctct cccctccaga 1860
agacttgcct gctagggacc tcgcctgctg gggacctcgc ctgttgggga cctcacctgc 1920
tggggacctc acctgctggg gaccttggct gctggaggct gcacctactg aggatgtcgg 1980
cggtcgggga ctttacctgc tgggacctgc tcccagagac cttgccacac tgaatctcac 2040
ctgctgggga cctcaccctg gagggccctg ggccctgggg aactggctta cttggggccc 2100
cacccgggag tgatggttct ggctgatttg tttgtgatgt tgttagccgc ctgtgagggg 2160
tgcagagaga tcatcacggc acggtttcca gatgtaatac tgcaaggaaa aatgatgacg 2220
tgtctcctca ctctagaggg gttggtccca tgggttaaga gctcacccca ggttctcacc 2280
tcaggggtta agagctcaga gttcagacag gtccaagttc aagcccagga ccaccactta 2340
tagggtacag gtgggatcga ctgtaaatga ggacttctgg aacattccaa atattctggg 2400
gttgagggaa attgctgctg tctacaaaat gccaagggtg gacaggcgct gtggctcacg 2460
cctgtaattc cagcactttg ggaggctgag gtaggaggat tgattgaggc caagagttaa 2520
agaccagcct ggtcaatata gcaagaccac gtctctaaat aaaaaataat aggccggcca 2580
g 2581

<210> 57
<211> 5937
<212> DNA
<213> Homo sapiens

<400> 57
ctgctcctgc gcggcagctg ctttagaagg tctcgagcct cctgtacctt cccagggatg 60
aaccgggcct tccctctgga aggcgagggt tcgggccaca gtgagcgagg gccagggcgg 120
tgggcgcgcg cagagggaaa ccggatcagt tgagagagaa tcaagagtag cggatgaggc 180
gcttgtgggg cgcggcccgg aagccctcgg gcgcgggctg ggagaaggag tgggcggagg 240
cgccgcagga ggctcccggg gcctggtcgg gccggctggg ccccgggcgc agtggaagaa 300
agggacgggc ggtgcccggt tgggcgtcct ggccagctca ccttgccctg gcggctcgcc 360
ccgcccggca cttgggagga gcagggcagg gcccgcggcc tttgcattct gggaccgccc 420
ccttccattc ccgggccagc ggcgagcggc agcgacggct ggagccgcag ctacagcatg 480
agagccggtg ccgctcctcc acgcctgcgg acgcgtggcg agcggaggca gcgctgcctg 540
ttcgcgccat gggggcaccg tggggctcgc cgacggcggc ggcgggcggg cggcgcgggt 600
ggcgccgagg ccgggggctg ccatggaccg tctgtgtgct ggcggccgcc ggcttgacgt 660
gtacggcgct gatcacctac gcttgctggg ggcagctgcc gccgctgccc tgggcgtcgc 720
caaccccgtc gcgaccggtg ggcgtgctgc tgtggtggga gcccttcggg gggcgcgata 780
gcgccccgag gccgccccct gactgccggc tgcgcttcaa catcagcggc tgccgcctgc 840

tcaccgaccg cgcgtcctac ggagaggctc aggccgtgct tttccaccac cgcgacctcg 900
tgaaggggcc ccccgactgg cccccgccct ggggcatcca ggcgcacact gccgaggagg 960
tggatctgcg cgtgttggac tacgaggagg cagcggcggc ggcagaagcc ctggcgacct 1020
ccagccccag gcccccgggc cagcgctggg tttggatgaa cttcgagtcg ccctcgcact 1080
ccccggggct gcgaagcctg gcaagtaacc tcttcaactg gacgctctcc taccgggcgg 1140
actcggacgt ctttgtgcct tatggctacc tctaccccag aagccacccc ggcgacccgc 1200
cctcaggcct ggccccgcca ctgtccagga aacaggggct ggtggcatgg gtggtgagcc 1260
actgggacga gcgccaggcc cgggtccgct actaccacca actgagccaa catgtgaccg 1320
tggacgtgtt cggccggggc gggccggggc agccggtgcc cgaaattggg ctcctgcaca 1380
cagtggcccg ctacaagttc tacctggctt tcgagaactc gcagcacctg gattatatca 1440
ccgagaagct ctggcgcaac gcgttgctcg ctggggcggt gccggtggtg ctgggcccag 1500
accgtgccaa ctacgagcgc tttgtgcccc gcggcgcctt catccacgtg gacgacttcc 1560
caagtgcctc ctccctggcc tcgtacctgc ttttcctcga ccgcaacccc gcggtctatc 1620
gccgctactt ccactggcgc cggagctacg ctgtccacat cacctccttc tgggacgagc 1680
cttggtgccg ggtgtgccag gctgtacaga gggctgggga ccggcccaag agcatacgga 1740
acttggccag ctggttcgag cggtgaagcc gcgctcccct ggaagcgacc caggggaggc 1800
caagttgtca gctttttgat cctctactgt gcatctcctt gactgccgca tcatgggagt 1860
aagttcttca aacacccatt tttgctctat gggaaaaaaa cgatttacca attaatatta 1920
ctcagcacag agatgggggc ccggtttcca tattttttgc acagctagca attgggctcc 1980
ctttgctgct gatgggcatc attgtttagg ggtgaaggag ggggttcttc ctcaccttgt 2040
aaccagtgca gaaatgaaat agcttagcgg caagaagccg ttgaggcggt ttcctgaatt 2100
tccccatctg ccacaggcca tatttgtggc ccgtgcagct tccaaatctc atacacaact 2160
gttcccgatt cacgtttttc tggaccaagg tgaagcaaat ttgtggttgt agaaggagcc 2220
ttgttggtgg agagtggaag gactgtggct gcaggtggga ctttgttgtt tggattcctc 2280
acagccttgg ctcctgagaa aggtgaggag ggcagtccaa gaggggccgc tgacttcttt 2340
cacaagtact atctgttccc ctgtcctgtg aatggaagca aagtgctgga ttgtccttgg 2400
aggaaactta agatgaatac atgcgtgtac ctcactttac ataagaaatg tattcctgaa 2460
aagctgcatt taaatcaagt cccaaattca ttgacttagg ggagttcagt atttaatgaa 2520
accctatgga gaatttatcc ctttacaatg tgaatagtca tctcctaatt tgtttcttct 2580
gtctttatgt ttttctataa cctggatttt ttaaatcata ttaaaattac agatgtgaaa 2640
ataaagcaga agcaaccttt ttccctcttc ccagaaaacc agtctgtgtt tacagacaga 2700
agagaaggaa gccatagtgt cacttccaca caattattta tttcatgtct ttactggacc 2760
tgaaatttaa actgcaatgc cagtcctgca ggagtgctgg cattaccctc tgcagaacag 2820
tgaaaggtat tgcactacat tatggaatca tgcaaaagga aaaaaagttt catgatatct 2880

gttgttggca gtttttgttt atctctgaca gtttttagtt aaatgtttag atcctcagaa 2940
ctacattagt gcctactatt aacttactct gtctcttgtt aaaggctaaa tctgcgcttc 3000
tccctggtgc cagcaggttc ccctcacagt caatgcagtg gtatagcata tcctcacatt 3060
tctagtgccc ttgagactgt gctatggaac caatcttgaa catacatgca ttgacttgac 3120
aagttactga gtaagcagca tattcagcag gtgccactac atgcctactc tgccagacac 3180
tgagcttggg gccctaggga agatagagaa ttatacaagg caaagtcctt ctctttaggg 3240
ctcttacaat ctatcacttc caaaaagtaa atggtgactg ataaaacaat tggcagaacc 3300
tgtttgatta ctgtgacagt cttaatgata ccataaatca atattagaaa gctagttgac 3360
ttaaagcctg aaataatggg agttttctcc tccacttatt agaataagga ccctcagtga 3420
ctaattattg tgggtagggt caagattaac tagttttata cagagttctg ctgtaaatag 3480
tcattttgca tttgattagt gcagttctct gaatcataaa gcaagtttta cctctctgta 3540
catgtttttg cagacatact tgaaaagctc acttaaatct aggtgcttca attcactttc 3600
ttgagaggac aaatgaaaag ctgtggagaa aatgtcctca ttaaagtatt aaagtgtggg 3660
cagaattaca attacaaagt gccagccacc gaataaagat aaaagttcag ttcttaaaat 3720
gagtttttat gagataacag tcagtgatct tggtgttacc gggattccac atggggcagt 3780
gggaaagagt tcaggttttg aaggtaacct agtttagatt tgaattccag ctatgtgaca 3840
ttgggtaaat tagtagtagt cctgagcctc agcgtcctca tctataaaat gactggcgaa 3900
aatacttcac aagctcattt tgagcacttt aggaagtaag tgaaagtacc taaaatagca 3960
ggcacccaat tgatgatttt atatcttcct tctttgcttg cagtgatttc aggatgtcct 4020
catatctatt tataggtcta aaattatatc ttaaggtatg ttgtagaata aattaaaagg 4080
ataatctaaa tcaccattta gattaagctt gacttgcaaa ctaggaagaa gcacctaggc 4140
tttctttgaa aatatttttt tggttcgttt tggtaaagct ctataaattg gtatctatta 4200
ttttaccaat ttttttttag tattaagtcc atttagaact aaccatatta tttatggaat 4260
aattagcatg aggaaggtat aattgcattt tttttttttt tagacggagc ttgcactgta 4320
gccccagctg gactgcagtg gcgtgatctt ggctcactgc aacctccgcc tcccaggttc 4380
aagcgattct cctgcctcag cctcccgagc agctgagact acaggcgcct gccaccacgc 4440
ctggccaatt ttttgtattt ttagtagaga ctgcgtttca ccatgttggg caggctggtc 4500
ttgaactcct gaccttgtga tccacctgcc tcggcctctc agagagctgg gattacaggt 4560
gtgagccgcc gtgcccagcc attgcatttt tattcacata cacattgtta atgtggaaca 4620
atttaacact aatctcatca gagagcgaga tgaatgtggc aattgctcat tttattttgc 4680
atatattaaa ttgagtaggt tcagctctaa cataccttaa gaaaaatgca tatcggtgca 4740
ctgtatgtat ttcaaaatgc ctttcctatg attgtcatgt cctcctttaa ggcttttccc 4800
tcaaatttat tacaaattta gtatttttag tacttgatga ctctaattac atgaatgcac 4860
ctggaatgac atttgtaaca gaagacggtc tgacttgctt tcagtattca caagttcttt 4920

ccagtttcca agtcttttcc tagcagtaat ttaggggaga cagaggagtt tcatgtaaag 4980

agcatgcagt ttggagtcag aacctgggta tgactctgtg gccttgatga agcaagttac 5040

ttaaactctt gagttttagc tttctccttt acaatgcatg aatgcctatc cccctacaaa 5100

acaaagatta aatgtgatga tgtatgccaa ggtgctttgt atattgtaaa gtgctatata 5160

attataagat gttctaaatt ttcaaggatc taaaccaggg attggcaaac gtttttccag 5220

ggagtaaata ttttacgctt tgcatatata atttatggag gtgttgagag gatagattag 5280

acacttgaag tactcaggat agtgcctggc atgtaggaag cacctggaaa atattcgctg 5340

tgattaccat cagtccattt taccgaggaa ggagccaagg tccaggccca ctgaaggact 5400

tgcataacat tacaatagca gtggcagaac cagccatgct tctgcaaatc acaacctctt 5460

tgagcctctg tcacctgaac tgcaaaatga gtgggttaga caaaatcatc tgttgggacc 5520

tcctagttcc acgtgctatc attctactaa ctggcaccct aaggttgaaa gtgcttatct 5580

gctttccaat gtggcttcct tacagtctgg aactgacaat atgcaggagc agtaaactgg 5640

cagaaaacca ggaatcagag aaagaaaata taatttaact ttaaagatgt aaattatata 5700

tatagtatat tatatatatt tttaaagctt tatatgcctc aaatatcagg gaaaggagcc 5760

aagtccttgg tatttagttt ggtgaatact tgcattgaat acatgtcaag atgtcaagtc 5820

atttttgaat gtgtctcagg gatttctatg ctacacattc ttttaacaaa tcaagtattt 5880

atgtacacat gttcagattt tttgacaaaa tgattaaaat aatgagatgg aaaatga 5937

<210> 58
<211> 1988
<212> DNA
<213> Homo sapiens

<400> 58
   gattacagac atgagccacc tcgcccagcc ttttcatggc tgagtgatat tccattgtgt 60
   ggatggatca cactagctac tctgacccat ggatcccctg ggcccagcca agccacagtg 120
   gctgtggcgc cgctgtctgg ccgggctgct gtttcagctg ctggtggctg tgtgtttctt 180
   ctcctacctg cgtgtgtccc gagacgatgc cactggatcc cctaggccag ggcttatggc 240
   agtggaacct gtcaccgggg ctcccaatgg gtcccgctgc caggacagca tggcgacccc 300
   tgcccacccc accctactga tcctgctgtg gacgtggcct tttaacacac ccgtggctct 360
   gccccgctgc tcagagatgg tgcccggcgc ggccgactgc aacatcactg ccgactccag 420
   tgtgtaccca caggcagacg cggtcatcgt gcaccactgg gatatcatgt acaaccccag 480
   tgccaacctc ccgcccccca ccaggccgca ggggcagcgc tggatctggt tcagcatgga 540
   gtcccccagc aactgccggc acctggaagc cctggacgga tacttcaatc tcaccatgtc 600
   ctaccgcagc gactccgaca tcttcacgcc ctacggctgg ctggagccgt ggtccggcca 660
   gcctgcccac ccaccgctca acctctcggc caagaccgag ctggtggcct gggcggtgtc 720
   caactggaag ccggactcgg ccagggtgcg ctactaccag agcctgcagg ctcatctcaa 780
   ggtggacgtg tacggacgct cccacaagcc cctgcccaag gggaccatga tggagacgct 840
   gtcccggtac aagttctatc tggccttcga gaactccttg caccccgact acatcaccga 900
   gaagctgtgg aggaacgccc tggaggcctg ggccgtgccc gtggtgctgg gccccagcag 960
   aagcaactac gagaggttcc tgccacccga cgccttcatc cacgtggacg acttccagag 1020
   ccccaaggac ctggcccggt acctgcagga gctggacaag gaccacgccc gctacctgag 1080
   ctactttcac tggcgggaga cgctgcggcc tcgctccttc agctgggcac tggctttctg 1140
   caaggcctgc tggaagctgc agcaggaatc taggtaccag acggtgcgca gcatagcggc 1200
   ttggttcacc tgagaggccg gcatggggcc tgggctgccg ggacctcact ttcccagggc 1260
   ctcacctacc tagggtctca ctagtcgggg gatttaccta cctggggcct cggctgcctg 1320
   gggcctcggc tgcctggggt ctcacctgcc tggggcctca cctgctggag tctttggtgg 1380
   ccaggcatgt gacttacctg ggatttcact tgccgggctt cactgccagg agcctcccct 1440
   gctggggacc ttgccagctg gggctgggga tggtgcctac tggggacctt gctttctgga 1500
   ggctgcacct actgaggatc tcggctgttg gggactttac ctgctgggac ctgctcccag 1560
   agaccttgcc acactgaatg tcacctgcta ggagcctcac ccgctgggag gcacagggcc 1620
   caagggagct ggatgtgttg cccaaggtgt gcagggcagg tcagggaagg gcagggtccc 1680
   ctaaggagga gggcgaaggg tatgtgtgtg accatcagca gtggtgtgca catggctggg 1740
   ggacactcgg tttgaccgcc agcggatggg tgtcacaaat gcatcactat gggtgtgacc 1800
   tcggcgtgac tctgatagtg cctgtggatg tgttgcgatg cctcaccctg gagggcactg 1860
   ggccctgaga acgggtgccc ttgaggccct gcccccgggc gatggtgcag gctggtttgc 1920
   ttgtggtttt attgctgttg ttaaccaccc atgaggggtg caaacagata atgctgttaa 1980
   cattttca 1988

<210> 59
<211> 2598
<212> DNA
<213> Homo sapiens

<400> 59
ccaggctcca ggccctggct tagagggagc ggggagtctg gacttcaggc tggatccctt 60

cctcttcctg gagcgggtgc tggcccccaa cccgcttgcg tcagggacaa aaggactcct 120

tccctttcca gcctggaaag cccctctgct gcaggctgga ggaagggacc ctgggcccag 180

cctatagtca gcggtgtcta tgggcatgga tctggacggg gaaaaggaca aagcagcctc 240

catccacagt tcattccggg accaggccct tgcaggcacg cgctgggctc ctgtgggaag 300

acactaaggg ccccaggaca gacctcctct ccgggcatct gggttcctag atggcagagg 360

tggcagagtg gggtgggatg gcccaattgg gagctttagc ttccggcaaa gagctgagca 420

cagtacatct tcattgtgta agattctcct gggagaccag ggcccagctg gtggtgagct 480

gggggaagtg ggtgatactg ccgtgggagg agccacctgg ccctctgggg aagtgcactc 540

gctgtctgca gcgcccaggc ctgggtagct gggtgggggc tgggggccca tctgtgctca 600

gggtgcctgc acctgggcct tctctgccct gggccaagcc tgcccgagcc tctctgtcct 660
ctgcctgccc agctggacat ctctgggcct ctctggagac cagtggggtg ggctgtgggg 720
gcgtcatatt gccctggctt ggcatccctc ttgtggctgt acccctccca gcagccccag 780
gactagcaag tccccgagat gggggtgggg acagtggttg atgccaaagg ttgtgggggc 840
aggggcgggg caggagcagg aaggtcccct gagttccctc accttgggca gagataaaag 900
gagcacagtt ccaggcgggg ctgagctagg gcgtagctgt gatttcaggg gcacctctgg 960
cggctgccgt gatttgagaa tctcgggtct cttggctgac tgatcctggg agactgtgga 1020
tgaataatgc tgggcacggc cccacccgga ggctgcgagg cttgggggtc ctggccgggg 1080
tggctctgct cgctgccctc tggctcctgt ggctgctggg gtcagcccct cggggtaccc 1140
cggcacccca gcccacgatc accatccttg tctggcactg gcccttcact gaccagcccc 1200
cagagctgcc cagcgacacc tgcacccgct acggcatcgc ccgctgccac ctgagtgcca 1260
accgaagcct gctggccagc gccgacgccg tggtcttcca ccaccgcgag ctgcagaccc 1320
ggcggtccca cctgcccctg gcccagcggc cgcgagggca gccctgggtg tgggcctcca 1380
tggagtctcc tagccacacc cacggcctca gccacctccg aggcatcttc aactgggtgc 1440
tgagctaccg gcgcgactcg gacatctttg tgccctatgg ccgcctggag ccccactggg 1500
ggccctcgcc accgctgcca gccaagagca gggtggccgc ctgggtggtc agcaacttcc 1560
aggagcggca gctgcgtgcc aggctgtacc ggcagctggc gcctcatctg cgggtggatg 1620
tctttggccg tgccaatgga cggccactgt gcgccagctg cctggtgccc accgtggccc 1680
agtaccgctt ctacctgtcc tttgagaact ctcagcaccg cgactacatt acggagaaat 1740
tctggcgcaa cgcactggtg gctggcactg tgccagtggt gctggggccc ccacgggcca 1800
cctatgaggc cttcgtgccg gctgacgcct tcgtgcatgt ggatgacttt ggctcagccc 1860
gagagctggc ggctttcctc actggcatga atgagagccg ataccaacgc ttctttgcct 1920
ggcgtgacag gctccgcgtg cgactgttca ccgactggcg ggaacgtttc tgtgccatct 1980
gtgaccgcta cccacaccta ccccgcagcc aagtctatga ggaccttgag ggttggtttc 2040
aggcctgaga tccgctggcc gggggaggtg ggtgtgggtg gaagggctgg gtgtcgaaat 2100
caaaccacca ggcatccggc ccttaccggc aagcagcggg ctaacgggag gctgggcaca 2160
gaggtcagga agcaggggtg gggggtgcag gtgggcactg gagcatgcag aggaggtgag 2220
agtgggaggg aggtaacggg tgcctgctgc ggcagacggg aggggaaagg ctgccgagga 2280
ccctccccac cctgaacaaa tcttgggtgg gtgaaggcct ggctggaaga gggtgaaagg 2340
cagggccctt ggggctgggg ggcaccccag cctgaagttt gtgggggcca aacctgggac 2400
cccgagcttc ctcggtagca gaggccctgt ggtccccgag acacaggcac gggtccctgc 2460
cacgtccata gttctgaggt ccctgtgtgt aggctggggc ggggcccagg agaccacggg 2520
gagcaaacca gcttgttctg ggctcaggga gggagggcgg tggacaataa acgtctgagc 2580
agtgaaaaaa aaaaaaaa 2598

<210>  60
<211>  3127
<212>  DNA
<213>  Homo sapiens

<400>  60
gtttgcatag ctccctggac ttctgctttg cactgccctg caggagtggg tggggaaagg      60

aagtggcttt gaggcacaca gaggggcttg ttgaggccac cggaggaagc ttctgccacc     120

aatatgggac ctgtgcccag cctaccagaa gagagcatct gaaaacatgt atcgacatgg     180

taacccctct gcttgaagcc tcacatggct ccctattgcc ttggtgctga acaccctatg     240

gctgaccgtg gcccagcctc tgcaacagct ctgcctcctc tccagtggtg aagacccagc     300

ctgctgagac tcctcctgca gttcctcaac atgcctgcat ttctgctgcc gtcagggcct     360

ttgcgaaggt tgttccttgt aactggaatg cccttccatc ccttttttta ttcaaaaggc     420

tgcaatttta attgaagaaa gttcccttcc aaggttcatg agttgcctga cttgcccacc     480

ggtttcctgc aagatccctt ggcctggcac ttagtgctca ggaaatattt ggtgatgggc     540

caactgagtg agaaggtggg atctggtggg aaggaaaggc ggaaggtaga aattctgctc     600

acttcctcat tcccacctcc caaggaaccc ctggtgtccc tgtggaaccc gctttgggaa     660

ccggtggttc aggtcagcct tttcactttg tactcaaagc cacatcgcat tgaagccaca     720

ggtggggcaa ggtcatgcat gactgagtct ccaaatccct tcaccctgtt tggttctgca     780

acggggatta ggggagcccc acgatttgtt ttcaaaggat gtccgggctc caggacagga     840

tgccctgggt cacctgatga caggtgtggt ggttggaaag ggccgggttt cagctccggg     900

tacacttcct ccttccttct gctgcgtggt gtggcctctt ccacgtcctc agaatccagc     960

tgttactcgt ccgcggcctc tcagctctag ggccctctgc acactggccc ccccagtgtc    1020

acgggcatcc agacgggatc cagtgcatcc tcttttagaa gaaaggcctg tctccaggtc    1080

cccgagtccc tctagcatct cccagaaggt gtcaagacgc agcagtgtcc aggagcggca    1140

gagactctga cccatggatc ccctgggccc ggccaagcca cagtggtcgt ggcgctgctg    1200

tctgaccacg ctgctgtttc agctgctgat ggctgtgtgt ttcttctcct atctgcgtgt    1260

gtctcaagac gatcccactg tgtaccctaa tgggtcccgc ttcccagaca gcacagggac    1320

ccccgcccac tccatccccc tgatcctgct gtggacgtgg ccttttaaca aacccatagc    1380

tctgccccgc tgctcagaga tggtgcctgg cacggctgac tgcaacatca ctgccgaccg    1440

caaggtgtat ccacaggcag acgcggtcat cgtgcaccac cgagaggtca tgtacaaccc    1500

cagtgcccag ctcccacgct ccccgaggcg gcaggggcag cgatggatct ggttcagcat    1560

ggagtcccca agccactgct ggcagctgaa agccatggac ggatacttca atctcaccat    1620

gtcctaccgc agcgactccg acatcttcac gccctacggc tggctggagc cgtggtccgg    1680

ccagcctgcc cacccaccgc tcaacctctc ggccaagacc gagctggtgg cctgggcagt    1740

gtccaactgg gggccaaact ccgccagggt gcgctactac cagagcctgc aggcccatct    1800

caaggtggac gtgtacggac gctcccacaa gcccctgccc cagggaacca tgatggagac 1860
gctgtcccgg tacaagttct atctggcctt cgagaactcc ttgcaccccg actacatcac 1920
cgagaagctg tggaggaacg ccctggaggc ctgggccgtg cccgtggtgc tgggccccag 1980
cagaagcaac tacgagaggt tcctgccacc cgacgccttc atccacgtgg acgacttcca 2040
gagccccaag gacctggccc ggtacctgca ggagctggac aaggaccacg cccgctacct 2100
gagctacttt cgctggcggg agacgctgcg gcctcgctcc ttcagctggg cactcgcttt 2160
ctgcaaggcc tgctggaaac tgcaggagga atccaggtac cagacacgcg gcatagcggc 2220
ttggttcacc tgagaggctg gtgtggggcc tgggctgcca ggaacctcat tttcctgggg 2280
cctcacctga gtgggggcct catctaccta aggactcgtt tgcctgaagc ttcacctgcc 2340
tgaggactca cctgcctggg acggtcacct gttgcagctt cacctgcctg gggattcacc 2400
tacctgggtc ctcactttcc tggggcctca cctgctggag tcttcggtgg ccaggtatgt 2460
cccttacctg ggatttcaca tgctggcttc caggagcgtc ccctgcggaa gcctggcctg 2520
ctggggatgt ctcctgggga ctttgcctac tggggacctc ggctgttggg gactttacct 2580
gctgggacct gctcccagag accttccaca ctgaatctca cctgctagga gcctcacctg 2640
ctggggacct caccctggag gcactgggcc ctgggaactg gcacccatgg gcccacccat 2700
gagtgatggt tctggctgat ttgtttgtga tgttgttagc cgcctgtgag gggtgcagag 2760
agataatcac cgcaccgttt ccagatgtaa tactgcaaag aaaaccaatg atgaggccgg 2820
gtgcggtggc tcacacctgt aatcccagca ctttgggagg ccgaggcagg cggatcacaa 2880
ggtcaggaga tcgagaccat cctggccaat atggtgaaac ccgtctctat taaaaaatac 2940
aaaaattagt ggggcgtggt ctcaggctcc tgcagtccca gctacttggg aggctgaggc 3000
aggagaatgg tgtgaacctg tgaggtggag cttgcagtga gccaagatcg cgccattgca 3060
ctccaacctg gacgacagag caagactcca tctcaaaaaa ataaaataaa ggccatatgt 3120
ttaatca 3127

<210> 61
<211> 2963
<212> DNA
<213> Homo sapiens

<400> 61
ggccgacccg agcagccggt tccctcctct ccaggccccc tccccatccc acccccgccg 60
cctggcccca gccgacccgt cccttcgtct ccccgcggaa tggggccggc actgctcagg 120
gtcgcgcgcc ctggacccag ctcgctctcg gtctcgcgct gtcagcgact gcccggctcg 180
cgccgcctcg cgctctgcct cagtcagtgg cgccgaaggc tccgttaagc ggcggcggcg 240
gttcctgttt ccgtttcttc ctctccgttc ggtcgggagt agcatcctcc actcagccac 300
ccttcccact cccccatcgt ggggcagctg cggctgaggg ctgtggcttt ggcagctgcg 360
acggggagcg gcggagaccg cctctgctcc cgcctggggt tgctgctttt gctcagagga 420
catccatgac cctaatggtc tttttgttca agataaagtg attttttgcc tttgttgatt 480

aactggacaa attcaggata ccagaaggcc ctattgatca ggggccagct ataggaagag 540
tacgcgtttt agaagagcag cttgttaagg ccaaagaaca gattgaaaat tacaagaaac 600
agaccagaaa tggtctgggg aaggatcatg aaatcctgag gaggaggatt gaaaatggag 660
ctaaagagct ctggtttttc ctacagagtg aattgaagaa attaaagaac ttagaaggaa 720
atgaactcca aagacatgca gatgaatttc ttttggattt aggacatcat gaaaggattc 780
tgatggcaat tactgtctca ttagtgaaca ataaaagaaa aattgttgta ttagcacaac 840
ctactactgt gaagaggaaa agaattaccc catacaagtc tataatgacg gatctatact 900
acctcagtca gacagatgga gcaggtgatt ggcgggaaaa agaggccaaa gatctgacag 960
aactggttca gcggagaata acatatcttc agaatcccaa ggactgcagc aaagccaaaa 1020
agctggtgtg taatatcaac aaaggctgtg gctatggctg tcagctccat catgtggtct 1080
actgcttcat gattgcatat ggcacccagc gaacactcat cttggaatct cagaattggc 1140
gctatgctac tggtggatgg gagactgtat ttaggcctgt aagtgagaca tgcacagaca 1200
gatctggcat ctccactgga cactggtcag gtgaagtgaa ggacaaaaat gttcaagtgg 1260
tcgagcttcc cattgtagac agtcttcatc cccgtcctcc atatttaccc ttggctgtac 1320
cagaagacct cgcagatcga cttgtacgag tgcatggtga ccctgcagtg tggtgggtgt 1380
ctcagtttgt caaatacttg atccgcccac agccttggct agaaaaagaa atagaagaag 1440
ccaccaagaa gcttggcttc aaacatccag ttattggagt ccatgtcaga cgcacagaca 1500
aagtgggaac agaagctgcc ttccatccca ttgaagagta catggtgcat gttgaagaac 1560
attttcagct tcttgcacgc agaatgcaag tggacaaaaa aagagtgtat ttggccacag 1620
atgacccttc tttattaaag gaggcaaaaa caaagtaccc caattatgaa tttattagtg 1680
ataactctat ttcctggtca gctggactgc acaatcgata cacagaaaat tcacttcgtg 1740
gagtgatcct ggatatacat tttctctctc aggcagactt cctagtgtgt actttttcat 1800
cccaggtctg tcgagttgct tatgaaatta tgcaaacact acatcctgat gcctctgcaa 1860
acttccattc tttagatgac atctactatt ttgggggcca gaatgcccac aatcaaattg 1920
ccatttatgc tcaccaaccc cgaactgcag atgaaattcc catggaacct ggagatatca 1980
ttggtgtggc tggaaatcat tgggatggct attctaaagg tgtcaacagg aaattgggaa 2040
ggacgggcct atatccctcc tacaaagttc gagagaagat agaaacggtc aagtacccca 2100
catatcctga ggctgagaaa taaagctcag atggaagaga taaacgacca aactcagttc 2160
gaccaaactc agttcaaacc atttcagcca aactgtagat gaagagggct ctgatctaac 2220
aaaataaggt tatatgagta gatactctca gcaccaagag cagctgggaa ctgacatagg 2280
cttcaattgg tggaattcct ctttaacaag ggctgcaatg ccctcatacc catgcacagt 2340
acaataatgt actcacatat aacatgcaaa caggttgttt tctactttgc ccctttcagt 2400
atgtccccat aagacaaaca ctgccatatt gtgtaattta agtgacacag acattttgtg 2460
tgagacttaa aacatggtgc ctatatctga gagacctgtg tgaactattg agaagatcgg 2520

aacagctcct tactctgagg aagttgattc ttatttgatg gtggtattgt gaccactgaa 2580

ttcactccag tcaacagatt cagaatgaga atggacgttt ggtttttttt tgtttttgtt 2640

tttgtttttt cctttataag gttgtctgtt tttttttttt taaataattg catcagttca 2700

ttgacctcat cattaataag tgaagaatac atcagaaaat aaaatattca ctctccatta 2760

gaaaattttg taaaacaatg ccatgaacaa attctttagt actcaatgtt tctggacatt 2820

ctctttgata acaaaaaata aattttaaaa aggaattttg taaagtttct agaattttat 2880

atcattggat gatatgttga tcagccttat gtggaagaac tgtgataaaa agaggagctt 2940

tttagttttt cagcttaaaa aaa 2963

<210> 62
<211> 12815
<212> DNA
<213> Homo sapiens

<400> 62
gcggcagtag ctgtagcagc ttcagcgaag ccggagatgg gcagagagcg cgcgcggcgc 60

agcagctcca gattcactgc tctcccctgc agctccccgc gcccccgccg ctgtcgctgc 120

ctcggtgtcc cccagcccca gtcgcgctct taggacagcg ccgccaccgc cgcctggccc 180

tgcctgcctc ctgcgccgcg cagccctcgc gagcgccccg gatggcgctt tacccctagg 240

accgatttag aatgtaataa ctcaaggatt tgataataca gtgaagtagt ataacaactg 300

tctacgtgct tcccatgata tgttctctat attgaaaaat tatgacatca acatccaaag 360

gaattcttcg cccattttta attgtctgca ttatcctggg ctgtttcatg gcatgtcttc 420

tcatttacat caaacctacc aacagctgga tcttcagtcc aatggaatca gccagctctg 480

tgctgaaaat gaaaaacttc ttttccacca aaactgatta ttttaatgaa actactattc 540

tggtgtgggt gtggccattt gggcagacct ttgaccttac atcctgccaa gcaatgttca 600

acatccaagg atgccatctc acaacggacc gttcactgta caacaaatcc catgcagttc 660

tgatccatca ccgagacatc agttgggatc tgacaaattt acctcagcaa gctaggccac 720

ccttccagaa atggatttgg atgaatttgg aatcaccaac tcacactccc caaaagagtg 780

gcattgagca cttgtttaac ctgactctga cttaccgccg tgattcagat atccaagtgc 840

cttatggctt cttgacggta agcacaaatc ccttcgtgtt tgaagtgcca agcaaagaga 900

aattggtgtg ctgggttgtg agtaactgga accctgagca tgccagagtc aagtattaca 960

atgagctaag caaaagcatt gaaatccata cctacgggca agcatttgga gaatatgtca 1020

atgataaaaa tttgattcct accatatcta cttgtaaatt ttatctttcc tttgaaaatt 1080

caatccacaa ggattacatc acggaaaagc tatacaatgc ttttctggct ggctctgtac 1140

ctgttgttct gggaccatct agggaaaact atgagaatta tattccagca gattcattca 1200

ttcatgtgga agattataac tctcccagtg agctagcaaa gtatctgaag gaagtcgaca 1260

aaaacaataa gttatacctt agttacttta actggaggaa ggatttcact gtaaatcttc 1320

cacgattttg ggaatcacat gcatgtttgg cttgcgatca tgtgaaaagg catcaagaat 1380
ataagtctgt tggtaattta gagaaatggt tttggaatta aaatttttca tcacttgcac 1440
acttgataaa tattttgatg agatatcatc caagtattga ggataagaag agatgcaaca 1500
tactactttt gtgtcacaat ttatttttat caccctctct agggtaacgt gtatattttg 1560
gtggagattt ttaaaagctc agcatgagca atcattccat tcggttttaa attatcctgt 1620
atatacctaa ttatgtgcac tggagagtaa tttattcttc attatcattt gtaaacattg 1680
ttttttcaca tttttgtagt tgtccataat gtaagcttgt ggtttgatta ttgtttccac 1740
actgatcagc tgtttaatct atttgggaaa tgaagatgca catcttaaag tatgaaaaat 1800
tttcactaag tattacaatg tctagttcca actttgcata ctataacaga ggaagaacat 1860
gttgcgattg aattctaacc tctttgactc cgaagatgaa tgaagtgtat aactgtctct 1920
atttgatcta ttttttttac ctgtttatca catttgtgaa ggtgaaatta ttcatggagt 1980
gaataagaaa gatatgaagc agaactgttc tattcgggaa gctattagac ttctcattta 2040
ttttcattaa gctgatttgc agctacttat tctcatggtc ttaaattaaa ttattcaagt 2100
atttttaaat atccaatttg ttgtgatttt cagcacctgg gaagtaatcc caataatact 2160
ttagaaaatc taagacagtt ctttctgcta ctgatgacac tcattgtcat aataaaacaa 2220
ataatttcct caaataacaa agaaaaatga tacctataaa tatatttata aatggtgtca 2280
tttatgaaca atgtttaatt atgtatcaat ttaagatttt tttctgaagc cctaatattt 2340
aaaatggtct tattttacca tatggatata agatttggct cataatgatg agccctatca 2400
tttgatttga gttctatcat ttaagagagc ctaaataaaa ttatcatcaa ggtattaaat 2460
ataagacgtt aaatataata aagtggggat atatagaaaa cacacagtgt tagcacacag 2520
taagatctca atgcacattt gttggatgaa taaataaatg caattgaatt cccagaaaaa 2580
tgattgtttc aaggaagtga cagttctact ttagaagtac taattggaga tgacttttat 2640
atcccatttt ggtaattatt catacatagc acatatgacc atgatgttca gggctttata 2700
gaaccaaata aacctaccat tacatgaaaa ttttgaagag tatattcctg aacttgcagc 2760
tgcctatagc attctcctcc ataaggctag aggagagctg gatataagca gagatcgtag 2820
gtgaaaaaag atgggccatt aaggattctt caaggaaaac attcctcaaa atgatcttta 2880
gtgcctttat tcctttcagg ccaataatcc acccaaaaat tagaatgttc tgagacatcc 2940
atttgggtct atttgtgact tattcgtatt acaaattgac atcagcaaaa ttcaaaaaga 3000
attcagtaaa aagggcatag atccagctat ccagactatc ttgtgagaga ataagctcac 3060
cccagcacca gaaagatttg agcagaggaa tattcctgta atgaatagag gattgtttga 3120
ttctcaggta tatttcttaa gccaagattg aatgcagtgt caggcactgc cttaacaccc 3180
acagagataa gatgaacaag gtactatcac tgcctaaaat gagcttacaa ttaagtggat 3240
agacatataa aagcttcctt tcaattcagc ctgagactgt ttatatgcat gtgctactcc 3300
gaatgacgtt tgtatgggtc aatgctgatt taatggggaa aaaaagtaga aaatttcccc 3360

aattgcttag ctgtttgaca tgtttctgag ctgcaaactt tgtcaactct tttgacccaa 3420
catattattc tctccgtatt atctcttcta accacaatgc attaagaaat aatattttta 3480
tgtggatgtc ctcctcaata agtaaagatc tccatccata tttaattaat tggtaagaca 3540
gtcaaattca tacatttaca tttactttgt caaatttttt ggtctataaa aagcatagag 3600
atctaaagag gatacactgc taacaagttt agaaagcacc tgcttttttt tattattatt 3660
atttttgtga tagagtcttg ctctgtcgcc caggccggag tgcagtggtg cagtctcggc 3720
tcactgcaac ctcggcctcc caggttcaag tgattctcct gtatcagcct cccgagtagc 3780
taggattaca ggcgtgtacc actatgcctg gctagttttt gtatttttag tagagatggg 3840
tttcactgtg ttggccaggc tggtcttgaa ctcctgacct caaatgatcc acttgcctca 3900
gcctcccaaa gtgctgggat tacaggtatg agccactgtg tctggcccac ctgttttttt 3960
tgaaggcaga aaaacattca atattaaaag cattcaatat tatttcaacc tgccttggct 4020
ctagtgaatg ttcatgattt gaaagaactg aaagacctaa aggcagtgtc tctattgttg 4080
actacaaatg taggctcttt gttggactat gactataatc acacactgat aatatgacgc 4140
aagtagaggc agtactacta agtatctagc aggtgctttg aaaccccatt actaagtccc 4200
agaagactgt ttcctaaatt tgaagtggaa gaattaaatg actcaatgga accctttagc 4260
tgtgggtcca ttgatgtgtg agttttagag ctggaaggaa ttataatttt gttgtttata 4320
attgctataa tatagaaatt ataattgctt ttctttctac ttatgagtta actagatcca 4380
gagatattta ttcaagacac acattcacac acacatacac acacacacac acacacccca 4440
cagctagtga caatagttaa gatttggatt taaggatcct atctttcttt atcccacttt 4500
cttttcaatc gcattcataa cttattatag aattatgtct caatttttac aaatgtgaat 4560
gtcttattgg tatttttagg gcctatagaa tggtacacct catgtagaag aacttcgact 4620
tttgaaggta tacaataaat aaaacatcaa acttgttagc attcgttctg gtttaaatat 4680
tttaggagtt gagttcagca gaaggaaatg ggctatcact acatttaagt gaaatacaac 4740
cctcaagaaa gtgtatggtt cacagccact cctttaataa atcctgtggg ttgcaattct 4800
ttgtaagaaa ctcatgaaaa agatatattg attcaacaaa attggtttgc tgttgattat 4860
acaaaccaat aattatgcct agaattaaat tgtggacaca aaaaagggaa cagaccattt 4920
aaaatgtttt atttcaaaat atcgaaaagc tattaccttc aatactctaa atatttaaat 4980
attaattatt gtagacatca gtaagaataa ataaatgtta atcattatag accttactaa 5040
taagaatata tactgaattg tccacatcca gagtattaca ccctgaacaa gaaagacatg 5100
agagcctgac agtcatatat attatgacag ctgtatatct gacaatatga agtagaaact 5160
gaaaaaaaaa atcatcttag tgggaaacaa atacatgctt aaaattgtta tatttactgt 5220
aaaactcctg ataatcttag attgtgcaga aatgtttctt attttgaggg gcttagaaag 5280
ctcagcgcat ttgatgtata aagcaacaga atatttagga attatttgcg gaggctttaa 5340
attcttacct ccaattggag gttaaatatt aaaagaatag gaaactatgc ctctgatatt 5400

ttttttgtca gcctattttt aaactagatt ccatgagtct taaggtaata tcctagaatt 5460
ttttttttgc gtgaattaaa gttaccacaa atttcaggtc cttaccttat gactgtgttt 5520
gatcatatct ttgagttccc ttgaactctg atctcatttt gagaaacatt ttgagaaact 5580
tcttaacaat tacaatcgag ctgttaaata taacaaacat cagaatctaa aggataagca 5640
cagtgcctaa tgtacaggag ggtcatatca aatacttgtt gaataaatac atgaatgtga 5700
taagtggtcc tgtaaaatat tttttaagag tagtgacaat gtttattggt accaattgga 5760
gatgatattt tatatttatt ttgttagttt gtcacctgga tgcaaaagga aggcatttta 5820
attcctttct atcttacaac attgtaaagc atcaaaagaa gtaacatctc aagaaagatt 5880
agaaaggatt tggatcacgt taacattcta tgcaaatatt agtaacatat caactgtttc 5940
taaaaggaaa gaaaattagt atttaaggag agtataaagt gcgagcactg tgaagggcag 6000
atatctatta ccaaaccaca tggagagaaa tatagtctct ctcctaactt cattccattg 6060
tattgcaatc agtcaattaa ggtgattttt tttttctgga tgtctcaatc tagagatttt 6120
gtagcatttt gcaaatgggt atgcttcact actatccaca gtgttctccc ctttagtaat 6180
tcatattgaa tatatccata ttcatatgca ttttataaaa cagttaacag aatctaaata 6240
agatatagcc ggctaaatct ggaatgtgag tgctacccta ggttattagt tcaacaaact 6300
aattttgaga gagccactta acctctctgg accttgtttg taagctttct tctagcacta 6360
ttactcttat ctaagatcga aattgatata tgcatgaaat tcctaatgga atttcaatgt 6420
ccatctcaaa gtaatttttt aggatattcc atacagattt ccttctcatt tctctgtgta 6480
gatatttaaa gtatgaaatt gattatttaa actaatatgt ggaaagaacc taatccagag 6540
cttggaatat agaaagtgct caattaactg acattcctcc tgtatttcct ccctcccctt 6600
agttgtctat gacttttgaa ctcactgctc actgacatct taattgcctg cagaaaaatc 6660
aatcatattt taggcattat tttgaaatat tttagagttt tctgtactat tcaaactctt 6720
attacaaaaa tctaaagata tttattgatg caaaaaattt agggagtcat tggaaatacc 6780
tgaggcaatc ccaggctaac aataaaatag cataatttta agtaagctag attgagttca 6840
tttgaacatt tagttatttt ttagctcata gttaacatat gtccatgcat ttgatttact 6900
ttaaaaatat tttatttaaa aataacctct caaacaccag ttgctttctg tcagttttac 6960
tcaaagcatg cttcctaggt cacctgacct agaatcacca gaagtgttaa caataccgat 7020
ttctgggctt tgagccatga aattagaatt tcttcaaata atccatgccc ccccgtcacc 7080
caaaaaaaga gcatgctttt aatacatttc caaaattatc ctgtttctgc tataactggt 7140
gaaccatcct gtcctaaact agcagttctc aaacatgcat atgcctttga aacttcctag 7200
atcatctaca taaaaatcat ttgaaatatt gttaataata cacagcttct gaatctctgg 7260
gtgtgagatg ggaccaggaa tcttatttta aaaatctccc cagatggttc tggtgtcctg 7320
ctagttttga ggttttcatc ccacctgttt agatgggtag tattctatta aatatgaggt 7380
attacctttc tttagttcac ctggaatctt cttaaatata tcggtaaagt attctacttc 7440

aaaatccaag acattttgta tacctaatta tttcacacaa gagcgtggaa ctcaccagtc 7500
ttgtgacttg gggcaggtta tttatagtct ccagacactt tttccagttg cgagttgctt 7560
atagtagttc gcgaacctga atgcacatta gaaccatctg gagaggcttt aaaattgcag 7620
atgtccaaac cccacctcca gagagctgat ctatttagtt tgaggaagag cccagaagtc 7680
taacaggtgt aaatgttcca caggtaatac taatgcaggc atacctcagg aaatcaccag 7740
ttctcgtctt ggaggaacta ttaggaaaat aaaagattat atatcttgaa aaaaatctct 7800
aatttgcttt ctaggttcta actgcatgtt tatattttgc aacaggaaag actggtctag 7860
acaattacta ggatatatgc ttctgtgatc tgcttttcta ttgtatgtgt ttaagagacc 7920
acattcttgg gttccaaagt ctccttgtct gataggtaat ttgcaattga gatatggcaa 7980
agaattgtag catccaaatt tttgacaact acagatttag aattgaatcc aaggtctgcc 8040
attcactagc tatgtgcaag ttactcaact tctcttatcc tcagattttg cagctgtaaa 8100
gcagaggtag catatttctg agaaattata ttcaggtgac tagatatgtg ttcaagtgtt 8160
atcttttatt cttataaaag ataattgtga ggaatccagt gctattttca caattttaat 8220
tctaaaactc tgaagacatt aaacaggcag tttgtaggaa tcattgaaat tttatacaag 8280
ccgaaggaac aacattcagc aaaacggaaa caaataaata cagcatggaa gcatatattc 8340
atgtttccca taattactaa gttacggaag gggtgtgagg agctggcagt ggtggtgaat 8400
aggcattgtt tgcattacta aatatatata ccatttctta agataattgg aactcaagca 8460
gtcctaattt tctctccctg ttatgagcaa tcagtaaatt tgtagtcaat ttaacttatt 8520
atcagttggt atagcgcatg tcacagaata ggatagcaag aagattctca gagatcattt 8580
tctggtaaag aaaaaggcct agcaatattt aacagtttga cttaatccat cacttcagct 8640
ggtccactag ttttgacaca ggactagtta atgatttgtt aggaagtctc catctactct 8700
taaactaatt taaaaaaaag ctaaaagttg cttttaatat gttagggtat aacttcaata 8760
aacaggagta tttatctagt ggttcagaaa taaaataatt gttagccagc ctgtgattaa 8820
ggctaccata attgcataat aaatagcttt gacgtgaata atctcagttg ttcaagtgta 8880
caaatttaaa gctgtactat gttagaataa aaaagtaggc tcaaggaaaa tttctatttt 8940
tctatatgaa aaagagaatg atattcaaaa tagtattttt aagtagtttc taattgtgtt 9000
cctttaaaga gtattagaaa tgtctgttat gtcagtaaca ttagaaaact tcaaaaactg 9060
atttattttt ctgagttctc tggaaaccaa acaattttga attagttgtg taagtaaagt 9120
aagttaagtt atagcttgct ggaattttat tgtggaaaac aaatatagat tttatctgaa 9180
aaattttaag ggccaattct acagaaagtt atgctaacat gctaactttc cacacatggc 9240
tgcattccat tttgattaat gagggttcag gaaatagaaa ggcagtctct gcagaagcag 9300
tatccaaagg ctaaattaac aacccaggac taccactcat tatgtgagaa atatgggcca 9360
gagttacaat atcacatctc cagtctcaga ttggcctcat tagccacctt cagttcctta 9420
aatgagataa tcctgaagat gccatccttg tccaaggtga gattgccaga ggatatgagc 9480

tcatctagga tggaatacat gtctccaggg actgtaatga gatcaggctt ttcatttact 9540
ggttttctgc ctcaagagtc cttaagcaaa tgaagattat ctgattgtct atgtaaacct 9600
ggaagtagtc tactttttag ataaaagata atctatcttg aatatttcat atgtgatttt 9660
aaaataatta atcaaaaaac aaagactatc atatttgaga acaagatttt acttagaagg 9720
gaaaaaaaag aactttcagc ttaatcgggg ttaagggagt aattgtgaca atataaaatg 9780
gcattgttat agaatcccta aaaggtaaat aatgtatgag atgaaggtag gaagtaatgt 9840
gtgcttgcag aaatgcaact ggtcaaaatt acttgaatga aattgttgag gttaaacatc 9900
ttcattataa taatatgtct tattctgatt ccaagacaat acgaatgtag agaagagatt 9960
tttaaatgga aaaagtaagg agctaacaag tctgtaccta gattcaagtt cacaaagcgg 10020
caaaaaggag aaaagtgtca acaagaaaca agaagaggag tagtaaacta aggatcaaag 10080
cccaagttcc agttttcaca atctgctgta taattcacta acatttctga gccatagatt 10140
tttaatctgt aaaaggagtg tgatggactg tacattagaa gtacctgaga agctcttaat 10200
aaatcttgat gaattggtta gaaattctaa tttaattagc ctggcacagt gcacaaactg 10260
ccatatattc gtgcagatgt tttcttcttt tcttactgcg catttccatg tggccatgtc 10320
actacaaggg gtggggaaga agttaatgtg taattgcttc aggagctctg aataaaaggt 10380
tgctgaagca aaatactgca atgaagttct gaaaatgaag agttaggact ttacatgatt 10440
tctcttgcct cattccctca agaatgcaca tatagacaca catatgccaa tatctttttt 10500
atgtagttca agtataactt tgaataaaat gacttacatt caaggaaggt caaaactatt 10560
attctgaaac aaaatgtgac aattctagcc attatgtgaa ttgtaagggt cagtaatcca 10620
taaattattt ggatggtaat tgtatgtttt attaatgtga tgtaattttg cactattgta 10680
ttgtcatatt taatcaaact cttaaaaata ccctgaaaca taatgccaaa tgtacagttt 10740
ataggttcgt gttatttctg ataagaattt aattgcattg tgctaatagt tgtttattgc 10800
tattaacttt ctgtgtatgt attattgcga tgcagaccca atagggttat tttaagcaca 10860
ctaattagtc atctggattt ttatagataa aatcacatgt tgatcagaaa tcatttttct 10920
acttattttt ttgtagagta tttttccaca cttgaaaaaa acatgattat tttatatcaa 10980
acatcaactt ttctttaaat cttattttag tagctaatgc atgtttgaaa taagatgtgt 11040
tagaaattat atgactatat taaggttgtg tttatattag gtgaaaaatt gcatgcaatt 11100
ggtgaatcat gcagctttgg ggacatttac actgccactc acacacaggc tctgtctcaa 11160
tccgtatgca tgattcacgt cctttcaaaa gtcccttttc aacatttggt aaagtgaaaa 11220
tgttattcat agtgctaatg agccaataaa caatgctgcg tttctttttt ctaactgcag 11280
catattgtgg taaattttct cccaaaactt tatttttgga gttattgttg gcaataaata 11340
caacaaatta tttgttggca agtgatacaa tttactgtat aacaacttaa tagtacaaag 11400
catgaagatg tgaaaatatc atcttaacca gtttcattct atgaacataa tattctggca 11460
gctttttcta taactgagaa tggtatatct ttttatacac tgcctaaatc agtactactg 11520

ccagtcacct gaggtcaggt ctgcacaaca ctaaattggg caataacata gaacatctag 11580
gcagtcttga cagtcaacca gtgtaatcac taggggaaga aaaagtaggc ctaccctttt 11640
acttattaac ttaagtaata aaaattgtat aaaaatatga atgttggctg cagaggagcc 11700
tttacatgca gataatttga agcagtcttt gaaaataaca aaaattattc catttaatga 11760
agggtttgtt ttgtttagct tttctctttt attcagaaaa catacctgtg cttttgaaag 11820
ggcttaatcc caaacaggta atatgtgtgg atcaatcatc tctcctccca tgaaattaat 11880
cattcatggt aatatattaa ggctggaacg tagctcttag tgacttaaaa catgacagta 11940
agcatttaca ctgttggaag gtaatttcat tgctatgtta ttaaaatgat gggaatccta 12000
tttatacatt tatttattta tttatttaca gaagattggt tccttccagt tcaatttaac 12060
agcttcagtg aagttagtat aatgataaga aaaattgact gtagctatta ttccaagtga 12120
aaatcatgca gctgagtcct gctgcatcct gggagcaaag cattaattca aatgaggagt 12180
agtcagtcct agcactgtag acgccgactt taccaaccaa gatagtgtat gtgtgtgaca 12240
ttcagctaac attgatctag gcacttagtt tgctaccaca ttgttccctt cattgattga 12300
aactgtaaat aacataacac tttaaggcag ctaagcaaat attttaataa gccatgaaag 12360
gcaagatgcc agagaaaatc tgtatattca gctatttgga gaactcgtgt tttccacaaa 12420
ttaaactgga gatgtcattt gaaattttct tcccttaaac atgctgtcac aacatggatt 12480
ccttctcatg gatgtctttc taggcttata aatatatggt gtgattgcta taattttgtg 12540
aaattttatt cagcaattaa tagtgatttc agcaatatgt actaagattc caaggcagaa 12600
ataaatgtat aaaggatttg agcctgtatg tgtaagaaga aactctctct tcagtcatat 12660
ttcctaaatt cagtgtaagt acctcgctga tttagcactg gagttattcc ttgaatgtgt 12720
aaataatgat gttctattct gacctaatga attcctgtaa tgtgaatatt taaaataaaa 12780
gaattcaatt taaatgtaaa aaaaaaaaaa aaaaa 12815

<210> 63
<211> 4331
<212> DNA
<213> Homo sapiens

<400> 63
atgtcgctgc tgcagtctgc gctcgacttc ttggcgggtc caggctccct gggcggtgct 60
tccggccgcg accagagtga cttcgtgggg cagacggtgg aactgggcga gctgcggctg 120
cgggtgcggc gggtcctggc cgaaggaggg tttgcatttg tgtatgaagc tcaagatgtg 180
gggagtggca gagagtatgc attaaagagg ctattatcca atgaagagga aaagaacaga 240
gccatcattc aagaagtttg cttcatgaaa aagctttccg gccacccgaa cattgtccag 300
ttttgttctg cagcgtctat aggaaaagag gagtcagaca cggggcaggc tgagttcctc 360
ttgctcacag agctctgtaa agggcagctg gtggaatttt tgaagaaaat ggaatctcga 420
ggcccccttt cgtgcgacac ggttctgaag atcttctacc agacgtgccg cgccgtgcag 480
cacatgcacc ggcagaagcc gcccatcatc cacagggacc tcaaggttga gaacttgttg 540

cttagtaacc aagggaccat taagctgtgt gactttggca gtgccacgac catctcgcac 600
taccctgact acagctggag cgcccagagg cgagccctgg tggaggaaga gatcacgagg 660
aatacaacac caatgtatag aacaccagaa atcatagact tgtattccaa cttcccgatc 720
ggcgagaagc aggatatctg ggccctgggc tgcatcttgt acctgctgtg cttccggcag 780
cacccttttg aggatggagc gaaacttcga atagtcaatg ggaagtactc gatccccccg 840
cacgacacgc agtacacggt cttccacagc ctcatccgcg ccatgctgca ggtgaacccg 900
gaggagcggc tgtccatcgc cgaggtggtg caccagctgc aggagatcgc ggccgcccgc 960
aacgtgaacc ccaagtctcc catcacagag ctcctggagc agaatggagg ctacgggagc 1020
gccacactgt cccgagggcc accccctccc gtgggccccg ctggcagtgg ctacagtgga 1080
ggcctggcgc tggcggagta cgaccagccg tatggcggct tcctggacat tctgcggggt 1140
gggacagagc ggctcttcac caacctcaag gacacctcct ccaaggtcat ccagtccgtc 1200
gctaattatg caaagggtga cctggacata tcttacatca catccagaat tgcagtgatg 1260
tcattcccag cagaaggtgt ggagtcagcg ctcaaaaaca acatcgaaga tgtgcggttg 1320
ttcctggact ccaagcaccc agggcactat gccgtctaca acctgtcccc gaggacctac 1380
cggccctcca ggttccacaa ccgggtctcc gagtgtggct gggcagcacg gcgggcccca 1440
cacctgcaca ccctgtacaa catctgcagg aacatgcacg cctggctgcg gcaggaccac 1500
aagaacgtct gcgtcgtgca ctgcatggac gggagagccg cgtctgctgt ggccgtctgc 1560
tccttcctgt gcttctgccg tctcttcagc accgcggagg ccgccgtgta catgttcagc 1620
atgaagcgct gcccaccagg catctggcca tcccacaaaa ggtacatcga gtacatgtgt 1680
gacatggtgg cggaggagcc catcacaccc cacagcaagc ccatcctggt gagggccgtg 1740
gtcatgacac ccgtgccgct gttcagcaag cagaggagcg gctgcaggcc cttctgcgag 1800
gtctacgtgg gggacgagcg tgtggccagc acctcccagg agtacgacaa gatgcgggac 1860
tttaagattg aagatggcaa agcggtgatt cccctgggcg tcacggtgca aggagacgtg 1920
ctcatcgtca tctatcacgc ccggtccact ctgggcggcc ggctgcaggc caagatggca 1980
tccatgaaga tgttccagat tcagttccac acggggtttg tgcctcggaa cgccaccact 2040
gtgaaatttg ccaagtatga cctggacgcg tgtgacattc aagaaaaata cccggattta 2100
tttcaagtga acctggaagt ggaggtggag cccagggaca ggccgagccg ggaagcccca 2160
ccatgggaga actcgagcat gagggggctg aaccccaaaa tcctgttttc cagccgggag 2220
gagcagcaag acattctgtc taagtttggg aagccggagc ttccccggca gcctggctcc 2280
acggctcagt atgatgctgg ggcagggtcc ccggaagccg aacccacaga ctctgactca 2340
ccgccaagca gcagcgcgga cgccagtcgc ttcctgcaca cgctggactg gcaggaagag 2400
aaggaggcag agactggtgc agaaaatgcc tcttccaagg agagcgagtc tgccctgatg 2460
gaggacagag acgagagtga ggtgtcagat gaagggggat ccccgatctc cagcgagggc 2520
caggaaccca gggccgaccc agagcccccc ggcctggcag cagggctggt gcagcaggac 2580

ttggtttttg aggtggagac accggctgtg ctgccagagc ctgtgccaca ggaagacggg 2640
gtcgacctcc tgggcctgca ctccgaggtg ggcgcagggc cagctgtacc cccgcaggcc 2700
tgcaaggccc cctccagcaa caccgacctg ctcagctgcc tccttgggcc ccctgaggcc 2760
gcctcccagg ggcccccgga ggatctgctc agcgaggacc cgctgctcct ggcaagcccg 2820
gcccctcccc tgagcgtgca gagcacccca agaggagggc cccctgccgc tgctgacccc 2880
tttggcccgc ttctgccgtc ttcaggcaac aactcccagc cctgctccaa tcctgatctc 2940
ttcggcgaat ttctcaattc ggactctgtg accgtcccac catccttccc gtctgcccac 3000
agcgctccgc ccccatcctg cagcgccgac ttcctgcacc tgggggatct gccaggagag 3060
cccagcaaga tgacagcctc gtccagcaac ccagacctgc tgggaggatg ggctgcctgg 3120
accgagactg cagcgtcggc agtggccccc acgccagcca cagaaggccc cctcttctct 3180
cctggaggtc agccggcccc ttgtggctct caggccagct ggaccaagtc tcagaacccg 3240
gacccatttg ctgaccttgg cgacctcagc tccggcctcc aaggctcacc agctggattt 3300
cctcctgggg gcttcattcc caaaacggcc accacggcca aaggcagcag ctcctggcag 3360
acaagtcggc cgccagccca gggcgcctca tggccccctc aggccaagcc gccccccaaa 3420
gcctgcacac agccaaggcc taactatgcc tcgaacttca gtgtgatcgg ggcgcgggag 3480
gagcgggggg tccgcgcacc cagctttgct caaaagccaa aagtctctga gaacgacttt 3540
gaagatctgt tgtccaatca aggcttctcc tccaggtctg acaagaaagg gccaaagacc 3600
attgcagaga tgaggaagca ggacctggct aaagacacgg acccactcaa gctgaagctc 3660
ctggactgga ttgagggcaa ggagcggaac atccgggccc tgctgtccac gctgcacaca 3720
gtgctgtggg acggggagag ccgctggacg cccgtgggca tggccgacct ggtggctccg 3780
gagcaagtga agaagcacta tcgccgcgcg gtgctggccg tgcaccccga caaggctgcg 3840
gggcagccgt acgagcagca cgccaagatg atcttcatgg agctgaatga cgcctggtcg 3900
gagtttgaga accagggctc ccggcccctc ttctgaggcc gcagtggtgg tggctgcgca 3960
cacagctcca caggttggga gccgtcgtgg gacctgggtc cccaccgtga ggaccccgtg 4020
ggcgacagca ggtgtggcca gggtggggct ccgagccccg ggtcaccgcc cgcccagcgt 4080
tccaggcaca tgaagagaaa gcattccaaa gcctctgatt gttgtttcct ttttctcctc 4140
ccgaaggaac agctgattca tgctcctccc gcaattgtca cgtctgtgat ttatttggtg 4200
tttcgggcgt ggcctctgga gccccggcac gtggtgggcc acgctgctgg cgctcatggg 4260
ccctggtgtt tgcaccgcac tttgtaatca gtcccgtggt tgtctgtaca gaattaaact 4320
attttccgat g 4331

<210> 64
<211> 1449
<212> DNA
<213> Homo sapiens

<400> 64

tcacctgccc cacagccgca ccctgcctgt gcctgcaccc tggggagccc agagccggca 60

ggggccgagg cggtgggacc tcgggggagc tcaagcctcg actgtcccct cgctggaggc 120

cagaggccaa gatgatgtcc atgctgggcg gcttgcagag atacttccgg gtcatcctcc 180

tcctcctcct ggccctgact ctgctcctgc tggccggatt cctgcactcg gacttagagc 240

tggacacacc cctgtttggg ggccaggctg aggggccgcc ggtcaccaac atcatgttcc 300

tgaagacgca caagacggcc agcagcacgg tgctcaacat cctctaccgc ttcgccgaga 360

cccacaacct gtccgtggcg ctgcccgccg gctcacgcgt ccacctgggc tacccctggc 420

tcttcctggc gcgctacgtg gaaggcgtgg ggtcgcagca gcgcttcaac atcatgtgca 480

accacctgag gttcaacctg cctcaggtgc agaaagtcat gcccaacgac accttctact 540

tctccatcct gaggaacccc gtgttccagc tggagtcctc cttcatctac tacaaaacct 600

acgcccccgc cttccggggc gccccgagcc tggacgcgtt cctggcctcg ccgcggacgt 660

tctacaacga cagccgccac ctcaggaacg tctacgccaa gaacaacatg tggttcgact 720

tcggcttcga ccccaacgcg cagtgcgagg agggctacgt gcgcgcgcgc atcgccgagg 780

tggagcggcg cttccggctg gtgctcatcg ccgagcacct ggacgagtcc ctggtgctgc 840

tgcggcgccg gctgcgctgg gcgctggacg acgtggtggc cttcaggctc aactcccgca 900

gcgcgcgctc cgtggcccgc ctgtcgcccg agacccggga gcgcgcgcgg agctggtgcg 960

cgctggactg gcgcctgtac gagcatttca accgcaccct ctgggcgcag ctgcgcgccg 1020

agctggggcc gcggcggctg cgcggggagg tggagcggct gcgcgcccgg aggcgcgaac 1080

tcgcgagcct gtgcctgcag gacggcggcg cgctcaagaa ccacacgcag atcagagacc 1140

cgcgcctgcg cccctaccag tccggcaagg ccgacatcct gggttacaac ctccggccgg 1200

gcctggacaa ccagacgctg ggcgtgtgcc agaggcttgt gatgcctgag ctccagtaca 1260

tggcccgcct gtacgccctg cagttcccgg agaagcccct caagaacatc ccgttcctgg 1320

gggcgtagag gggccgggcc ggggacgagg cctcctgcgg acaccagctc ctctctccgc 1380

cgtcaccggg gaggccgggg atccttgcag ggcttctggg gcgttgggaa acccaggccc 1440

gccggccac 1449

<210> 65
<211> 1585
<212> DNA
<213> Homo sapiens

<400> 65
gccggagtgg gccgtcagca cttaaagggc ccgcggctcg ggcgtaggag gcggtgcctc 60

tgcagcaagc gtggggcgcg ggaacccgag caggactctc cagtcctcag tcaccttgga 120

caaagaagtg tggatcctca gattccatct tttccaactc caaggtgcca tggcagagaa 180

ggtgctggta acaggtgggg ctggctacat tggcagccac acggtgctgg agctgctgga 240

ggctggctac ttgcctgtgg tcatcgataa cttccataat gccttccgtg gagggggctc 300

cctgcctgag agcctgcggc gggtccagga gctgacaggc cgctctgtgg agtttgagga 360

gatggacatt ttggaccagg gagccctaca gcgtctcttc aaaaagtaca gctttatggc 420

ggtcatccac tttgcggggc tcaaggccgt gggcgagtcg gtgcagaagc ctctggatta 480

ttacagagtt aacctgaccg ggaccatcca gcttctggag atcatgaagg cccacggggt 540

gaagaacctg gtgttcagca gctcagccac tgtgtacggg aacccccagt acctgcccct 600

tgatgaggcc caccccacgg gtggttgtac caacccttac ggcaagtcca agttcttcat 660

cgaggaaatg atccgggacc tgtgccaggc agacaagact tggaacgcag tgctgctgcg 720

ctatttcaac cccacaggtg cccatgcctc tggctgcatt ggtgaggatc cccagggcat 780

acccaacaac ctcatgcctt atgtctccca ggtggcgatc gggcgacggg aggccctgaa 840

tgtctttggc aatgactatg acacagagga tggcacaggt gtccgggatt acatccatgt 900

cgtggatctg gccaagggcc acattgcagc cttaaggaag ctgaaagaac agtgtggctg 960

ccggatctac aacctgggca cgggcacagg ctattcagtg ctgcagatgg tccaggctat 1020

ggagaaggcc tctgggaaga agatcccgta caaggtggtg gcacggcggg aaggtgatgt 1080

ggcagcctgt tacgccaacc ccagcctggc ccaagaggag ctggggtgga cagcagcctt 1140

agggctggac aggatgtgtg aggatctctg gcgctggcag aagcagaatc cttcaggctt 1200

tggcacgcaa gcctgaggac cctcccctac caaggaccag gaaaagcagc agctgcctgc 1260

tctccagcct ctggcaggaa ctcagggccc tggagctgct ggggccaagc caaggcctcc 1320

cctacctcaa accccagctg ggcccgctta gcccaccagg catgaggcca agggctccac 1380

tgaccaggag gccgaggtct ctaactctta tcttccacag ggtccaagag ttcatcagga 1440

cccccaagag tgagtgaggg ggcaaggctc tggcacaaaa cctcctcctc ccaggcactc 1500

atttatattg ctctgaaaga gctttccaaa gtatttaaaa ataaaaacaa gttttcttac 1560

actgggaaaa aaaaaaaaaa aaaaa 1585

<210> 66
<211> 3778
<212> DNA
<213> Homo sapiens

<400> 66
tttttaaatt ttgcatttga cttaaagtgc catgagaaaa tttgcatact gcaaggtggt 60

cctagccacc tccttgattt gggtactctt ggatatgttc ctgctgcttt acttcagtga 120

atgcaacaaa tgtgatgaaa aaaaggagag aggacttcct gctggagatg ttctagagcc 180

agtacaaaag cctcatgaag gtcctggaga aatggggaaa ccagtcgtca ttcctaaaga 240

ggatcaagaa aagatgaaag agatgtttaa aatcaatcag ttcaatttaa tggcaagtga 300

gatgattgca ctcaacagat ctttaccaga tgttaggtta gaagggtgta aaacaaaggt 360

gtatccagat aatcttccta caacaagtgt ggtgattgtt ttccacaatg aggcttggag 420

cacacttctg cgaactgtcc atagtgtcat taatcgctca ccaagacaca tgatagaaga 480

aattgttcta gtagatgatg ccagtgaaag agactttttg aaaaggcctt tagagagtta 540

tgtgaaaaaa ctaaaagtac cagttcatgt aattcgaatg gaacaacgtt ctggattgat 600
cagagctaga ttaaaaggag ctgctgtgtc taaaggccaa gtgatcacct tcctggatgc 660
ccattgtgag tgtacagtgg gatggctgga gcctctcttg gccaggatca aacatgacag 720
gagaacagtg gtgtgtccca tcatcgatgt gatcagtgat gatacttttg agtacatggc 780
aggctctgat atgacctatg gtgggttcaa ctggaagctc aattttcgct ggtatcctgt 840
tccccaaaga gaaatggaca gaaggaaagg tgatcggact cttcctgtca ggacacctac 900
catggcagga ggcctttttt caatagacag agattacttt caggaaattg gaacatatga 960
tgctggaatg gatatttggg gaggagaaaa cctagaaatt tcctttagga tttggcagtg 1020
tggaggaact ttggaaattg ttacatgctc acatgttgga catgtgtttc ggaaagctac 1080
accttacacg tttccaggag gcacagggca gattatcaat aaaaataaca gacgacttgc 1140
agaagtgtgg atggatgaat tcaagaattt cttctatata atttctccag gtgttacaaa 1200
ggtagattat ggagatatat cgtcaagagt tggtctaaga cacaaactac aatgcaaacc 1260
tttttcctgg tacctagaga atatatatcc tgattctcaa attccacgtc actatttctc 1320
attgggagag atacgaaatg tggaaacgaa tcagtgtcta gataacatgg ctagaaaaga 1380
gaatgaaaaa gttggaattt ttaattgcca tggtatgggg ggtaatcagg ttttctctta 1440
tactgccaac aaagaaatta gaacagatga cctttgcttg gatgtttcca aacttaatgg 1500
cccagttaca atgctcaaat gccaccacct aaaaggcaac caactctggg agtatgaccc 1560
agtgaaatta accctgcagc atgtgaacag taatcagtgc ctggataaag ccacagaaga 1620
ggatagccag gtgcccagca ttagagactg caatggaagt cggtcccagc agtggcttct 1680
tcgaaacgtc accctgccag aaatattctg agaccaaatt tacaaaaaaa cgaaaaaaat 1740
aaggattgac tgggctacct cagcatacat ttctgccaca ttcttaagta gcaaaaaagg 1800
aaaagtgctt tcctcctctg caggatgtaa ggtttatcag ccattaaaac ttagacttct 1860
ctagcttttc actagctgtg aaccagcctt cctgtccatg gacgtgaaac tgcatagtaa 1920
tgagactgtg cacactgatg tttacaagat tgaaagagtc tttctccgaa aatcatggta 1980
aagaatactg agacaatgaa aaaaaatcaa caaaatatgc tttctggaga actgtacctt 2040
ctatggtttg cttgcacatc agtagtttct gctgaacgtg ctgtcataat gaagagattt 2100
ccaagatttt ttttcctgat tagaacgggt agccagtata ttaaatattg atagaaaaat 2160
aaaagaactg gaaccagatt cagaatcttg aaaacaacat tttttacaac aaacaaaaaa 2220
actatattaa acagggttta aaggaaaatt aaaacagaac tatgaagaag tacaatttgt 2280
tatagtatag tatcaaattt ctatatagat tttatacctc agtggggaaa aataactgat 2340
tccaatgaca ttcattttgt tttcatctgt gatagtcatg gatgctttta ttttccttgg 2400
ggtgctgaaa ttgagctgaa aaaaaaaggc tctttgaata tagttttaat ttctctctac 2460
agtttttttt gtttggtttg tgggctgttg gaattgtaat ttttaattgc cttctaaaaa 2520
atggaaattt aacaatgtct gatctcagct gaacaaatta gatgtttcag ttgctcttgg 2580

gtcaactggc ttacagattt acatgtgcac acacacacaa atttcttatc acattttcga 2640
cttcttcact tgacctaact gattatgcga aatacccaag attcatgcta ctgttccaca 2700
tttgttttca cagcaataaa tcttcagttc tgttgtttat gattccactt aacaaggggc 2760
ctgcaaatgt gatttattat ttgggtattt ggagataata catttgaggg ttttttggaa 2820
aacctttttc actccatact caaatatgct tcattgtcaa atgcatattt aaattaaatt 2880
attgaattgt aatgtttatc tgctgctttt tttaaataaa atttgactga aaatgtttaa 2940
ttggcatttt ttaatgactt acccaagaaa agtgcagcta ttattccata ttaataggct 3000
tgcatttctt ttcctaaatc ttatttaggc taaatcagtt ttattgtcct ctgatttttt 3060
ttaataccac agaaatcacc tgagtgtcaa ttgaaaagtt gtcaattaaa aggtaacctt 3120
ttaactctcg taggaggaat ctcattaaga catttttcct gatatgtaga gcagtctgtt 3180
ggcaaaaatg catatatttt ctttcatatt tgtaaaatta tatttaatgg aattcttttc 3240
tttgattatc aaggactttc actgcaggca gtgctatttc ttgtgcctaa gaatgtttcc 3300
aaaagtcgca tcgctaatga tatttgccaa gttgagtgta cacaaagttt ctcatatcct 3360
gttcaagtta atcaacatca aacacatggg gatgctttag ggtgagtcta taatacaaaa 3420
tgcataaacc atgtccccag gaaatttgaa aggaagcaag tgctgaatgg aatttttttc 3480
cttttccatg agctgtgtta attctatctc cagtaggcct aatgcttgaa ataagcaaga 3540
tgtctaatca ataaattatt ttcatgctca gaatttcagg tttttgtact ccagcatagc 3600
ttggtcttat ttcttactgt atgaaagctt aacagcaatg tgatttaagg ttttgtttta 3660
aatgggagat gtaagtgatt taattcatgg gtacttttag aacctgatag ataatcccat 3720
tgcctttatt tttctaatta aagaatccta aatactttga aaatacaaaa tattcctg 3778

<210> 67
<211> 4361
<212> DNA
<213> Homo sapiens

<400> 67
cacatttctt agcttctctg agcctccatt tcagcatctg taaaatgtta ctacctactt 60
tataggtttt ctgatgaggc ttaagtgtgg ttgtgcatgt gaccatctta gcatagtgtg 120
tggcaatggt aagcactcag tgctgtcagc tgtttttacc gctgttatta tattactgtg 180
ttttcccatg tcattaggtc tgtgatccag gcttcagcag acaatccagg ctgatgcttt 240
tgctgagtgc actaagggat gctggcctgg cgggatggtg aactggaagc agaaacgtca 300
tcctctctct tccttcttgc catgcaggtg tggatgtgtg ggggccgcat ggaggacatc 360
ccctgctcca gggtgggcca tatctacagg aagtatgtgc cctacaaggt cccggccgga 420
gtcagcctgg cccggaacct taagcgggtg gccgaagtgt ggatggatga gtacgcagag 480
tacatttacc agcgccggcc tgaataccgc cacctctccg ctggggatgt cgcagtccag 540
aaaaagctcc gcagctccct taactgcaag agtttcaagt ggtttatgac gaagatagcc 600
tgggacctgc ccaaattcta cccacccgtg gagcccccgg ctgcagcttg gggggagatc 660

cgaaatgtgg gcacagggct gtgtgcagac acaaagcacg gggccttggg ctccccacta 720
aggctagagg gctgcgtccg aggccgtggg gaggctgcct ggaacaacat gcaggtattc 780
accttcacct ggagagagga catccggcct ggagaccccc agcacaccaa gaagttctgc 840
tttgatgcca tttcccacac cagccctgtc acgctgtacg actgccacag catgaagggc 900
aaccagctgt ggaaataccg caaagacaag accctgtacc accctgtcag tggcagctgc 960
atggactgca gtgaaagtga ccataggatc ttcatgaaca cctgcaaccc atcctctctc 1020
acccagcagt ggctgtttga acacaccaac tcaacagtct tggaaaaatt caataggaac 1080
tgagccctca tgtccccttg gcaggccccc cagggtctgg cactcactgc agacttcctc 1140
tttcaaggga ggcagggccc ctgtgggcac taggtgtaaa aggtgctggc caaatggttc 1200
agggtgaaga gggctcttga ttcaggggct ggggtctgcc tggtccttga gcccctgagt 1260
tgtgggggta gggtgaagag catatcccac aagaggcccc acagggagca gagactgctt 1320
taatccctgc tgacatcacg gaaaagcaac agagcctttt caactttgtc actatgtccc 1380
cttgaacatt atgtgggaga acaccaaggt agcctaggcc acccaaaagt gagtcctgcg 1440
aggttgccca gccctcagat ggctctccta catgatggtg ctttagaaac aaaggtaaaa 1500
tttgcctgtt tggggcagct tttagtatcg atgccactca tctgcagcag aagagaaaga 1560
agtcctcttg gggcttttta gtttctgccg tcctgggggg aacattgcag ttactgcaca 1620
gcttctgttc tctgtcacaa ccccaggtga tttggtccgg tcaaaggcca tacttggggc 1680
cctaagagtg ttcagtattg aatgctgatc agctgccagg tgaggagtca gaagagggag 1740
cccccctaga catttctttg cagctatgga catgcgggat atctccccct gctctctggg 1800
tatttgaaat gtcaatttta gcactctcca ggcacaagga cagcccagca ccagctttac 1860
agggcagtgt ttcagatggc cctgagccca cggaaaaggc caggtagacc tccaaactag 1920
aaatgctggc tgatttgccc tgatccatgc ttccatttcc ctgtctctct tccccaggca 1980
attactggcc tcaaaagagg aacagaggtg ctgcgaggtg ctcacctcac agagtctgga 2040
ggcctccagg atcaactgtg ggcaaagtgc ctgcctctga cctcatcatg gttctagttc 2100
tcatacagaa ctccagaatt tttaaagaac tctataattg gattgcaaac taggatgcta 2160
cataggattc tggtattcca catccaatat ggatttctag aatgctgtga ttaaaggagc 2220
cagccaggtg taatacagtc aaggcagccc ccagcctaga gacaatctgt gaaatccaaa 2280
gttggtggtg ttgggaaagc agggggacat gtgtccctca gctcagcaga ggctgtggta 2340
caacatggtc cttggtgaag acctgcaccc ctggaacctc ccaccatcat cacaactgta 2400
gtctcatttg cagtggagaa aagaacccga cgtcccacag ccagatatac acccagctcc 2460
atgccagccc ttcatgttta ccttttgctt tgttaattac atgtcagact cctagagggc 2520
ctccagacta ataggaagca tttctgtaac caacctgcca cccactgatt cagaaatgga 2580
aatcacattc cacaatctat ggcttccacc agctagccca ggaaatactt gaaatcagca 2640
ttccaattag tgttgagtct cttgattgtg tcatttacca attaaataac tgagacctaa 2700

gtctgggaac agagccacga atctgccttt gagatgctgg cagatctcaa ggccatcaat 2760
tattggggga gggagggaca aacactccca atcatccacc agtcagactg aatgtgtagc 2820
tggcgaggaa ttacttccac ttctggccca gcacaagccc tgctttggcc acctgtctgc 2880
aagagaggcg gcccctgtgc ttgcaacgct tacgtgttga tcccagtgtc cttttccaaa 2940
tgagtgctgt agctttagaa gtggccctct atagaaagaa gtcaaaagat gaggcccctt 3000
ctagaatcta ggataacaag agtgttgaca gtttgaggag tcgaattgag attcatcatc 3060
aaagagcaat gcagcgtcgt taaaataaaa actgtgcctt ttaaaaagaa aaatgcaaat 3120
atagagcaaa tccctaaact tgaaccattt cctagtgcct tgctagacaa acataaaggg 3180
gcagggttgt ggggagggga acgtttttgg tggtgtgtgc agttcctact ggatgagtgt 3240
gtgtttctta atgtctcatt tcaagcagga gatgttgggt ctggagcaag atctgagact 3300
gagatgtccc ccaagggtga caggtcagat ttatttcagt cagtgcaagt cacactccaa 3360
actaaaggct gggcagtgcg tttagtctgt ggcctagaag acctcatcag cccccgagag 3420
gaggccttgc attacctcac tgggacctgt ttgggggcca tccctgcggt ccaccctctg 3480
aacccccagc atgtttgtcc tgtttctcac cattgggtta aggggtgccg agcactagct 3540
agacttccac atagtcccac cccaagtggg cggcagtgtt cctggcatga ccaggattcc 3600
tgtgaaagca ggagcagcag caagccgtcc cgggcctgcc tttcccatcc tttggttctc 3660
ctttccagtc tgggttcaca ccctgaaaag ggatgggtgt gtcctctgag cactctggca 3720
tttgtcattg ctgagcccat atcagtaggt cctgggactt gacaaagtat gtgggagggg 3780
aaggaaggac agaacttgac tggctccatt tcatggacca actgatcaat tgccagtact 3840
aagccactca ttgttatggc ttcctttttg aaaaccacct gtctcaagga ttcaggtttc 3900
tgctcacatc cccagctgat gctcaataaa actcagccag gagcatatgg agatgctgac 3960
agccaggtaa tgggtgagac tgtggggtcc cttttcctct aaagccttta ctctgaggat 4020
aaggtcacaa agtagggtgt gacttgaaag tatcgtgacc atgtggccca ccagcagcct 4080
ttccaaaggg gaactccaga gacatttcat aatgcaaaca gcactgttgc tttaagaaca 4140
aggcctttgg aggtggctaa acttgcacgc acgtgtgtga aaagccatcc catcttctgc 4200
ctccagttgg agctttcagc tgttaaaaca gtcagaaact attgattctt ccctttagga 4260
aaaaatgctc aggcagaacg gggtacatcc acaggaggaa tcggacgaga ggatgtgaca 4320
ttcggtccag gagagaaaga gcagtttctg ttaaagatgt a 4361

<210> 68
<211> 2591
<212> DNA
<213> Homo sapiens

<400> 68
agttcagccc gcgccgctcc tgcgggtcgg actggggctg tggcggaaat tgacaatggc 60
ccttcagcta tgctaggtct ataatgggaa gtgtcacagt tcggtatttc tgttatgggt 120

gcctttttac atctgcgacc tggacagttt tgctttttgt ttatttcaac ttcagtgaag 180
tgactcagcc acttaagaat gtgcccgtca aggggtctgg gccccacgga ccatctccaa 240
aaaaattcta tccccgtttc actcgaggcc caagtcgagt gctcgagcca cagttcaaag 300
caaacaaaat tgacgatgtg atagacagtc gtgttgaaga tccagaagaa ggccacttga 360
aattctcttc tgaattaggt atgattttta atgaacgcga tcaagagttg agagacttgg 420
gctatcagaa acatgctttt aatatgctta tcagtgaccg cttgggctac cacagagatg 480
tgccagacac aaggaatgca gcatgtaaag aaaagttcta cccacctgac ctgccagctg 540
ctagtgttgt tatctgtttc tataatgaag cgttttctgc cttgcttcgg acagtgcaca 600
gtgtcataga ccgcacgcca gcacacctgc ttcatgagat catccttgtg gatgatgata 660
gtgactttga tgatttgaaa ggagaactag atgaatatgt ccaaaaatac ctccctggaa 720
aaattaaagt cataagaaat acaaagcgtg aggggttgat tcgagggaga atgattggcg 780
cggcccacgc gacaggagaa gtccttgtgt tcctggacag ccactgtgaa gtgaatgtga 840
tgtggctgca gcccttgctg gccgccatcc gtgaggaccg gcacaccgtg gtgtgcccag 900
tgattgacat catcagcgcc gacacgctgg cctacagctc gtcccctgtc gtccgcggag 960
ggttcaactg gggactgcac ttcaaatggg atcttgtccc cctttctgag ctaggacgag 1020
cggagggagc cactgcacca ataaagtcac caacaatggc tggaggtttg tttgccatga 1080
acagacagta tttccatgaa cttggacagt atgatagtgg catggatatc tggggaggag 1140
aaaatttgga aatatcattt cggatctgga tgtgtggcgg taagctcttc atcatccctt 1200
gctctagagt aggacacatt ttccgaaaaa ggcgaccata tggatctccc gaaggccagg 1260
acaccatgac acacaactct ttgcggctgg cacatgtctg gttggatgaa tacaaggagc 1320
agtatttttc cttaagacct gacctgaaga cgaaaagcta tggcaatatc agtgagcgtg 1380
tggaactgag aaagaagttg ggctgtaaat catttaaatg gtatttggat aatgtatacc 1440
cagagatgca gatatctggg tcccacgcca aaccccaaca acccattttt gtcaatagag 1500
ggccaaaacg acccaaagtc cttcaacgtg gaaggctcta tcacctccag accaacaaat 1560
gcctggtggc ccagggccgc ccaagtcaga agggaggtct cgtggtgctt aaggcctgtg 1620
actacagtga cccaaatcag atctggatct ataatgaaga gcatgaattg gttttaaata 1680
gtctcctttg tctagatatg tcagagactc gctcatcaga cccgccacgg ctcgtgaaat 1740
gccacgggtc aggaggatcc cagcagtgga cctttgggaa aaacaatcgg ctataccagg 1800
tgtcggttgg actgtgcctg agagcagtgg atcccctggg tcagaagggc tctgtcgcca 1860
tggcgatctg cgatggctcc tcttcacagc agtggcattt ggaaggttaa ggtggatgct 1920
gtggcgggaa cgttgcttca tcaggcgttg cctccggtgt ggagtttggg gctttaggaa 1980
agcctgggtt gggtggagca gaaccatctt ggagaagatg acagttccct gtcctcccgg 2040
agatgcctgg gtgtgttagc agaggtgaca cgtgtctgac agagacggga gctctgagtg 2100
tccacgggtg aagaagtgag tgtccacggg tgaagaagtg agtatgtttc acctggacat 2160

taaggtgatg tttgagctgc tgttaaggaa tttcttgctt atagaggcaa accacagtat 2220

cattttaact ctagaattgg gcttgtacag aaggataaaa cccaggaaaa tggatatttc 2280

tattcagatt tatttatgcc tctttttaat cccctttaat gatgcagtgg tttttatctg 2340

atcaggaact tgtcatgatt tcctttctta gacttcatag gagatagtgc tttaaaaaaa 2400

aaaaaaaact tctattattt gtttagtatg ttgtaagtag atcattttaa aaaactgaat 2460

ctatattatg tttaacttca gaaggcatca tttataagac agtatggcag ttaattataa 2520

aattattttg atgaattatg atacaatcta cataataaag aatccttttg attaaaaaaa 2580

aaaaaaaaaa a 2591

<210> 69
<211> 2770
<212> DNA
<213> Homo sapiens

<400> 69
atgtgggggc gcacggcgcg gcggcgctgc ccgcgggaac tgcggcgcgg ccgggaggcg 60

ctgttggtgc tcctggcgct actggcgttg gccgggctgg gctcggtgct gcgggcgcag 120

cgtggggccg gggccggggc tgccgagccg ggacccccgc gcaccccgcg ccccgggcgg 180

cgcgagccgg tcatgccgcg gccgccggtg ccggcgaacg cgctgggcgc gcggggcgag 240

gcggtgcggc tgcagctgca gggcgaggag ctgcggctgc aggaggagag cgtgcggctg 300

caccagatta acatctacct cagcgaccgc atctcactgc accgccgcct gcccgagcgc 360

tggaacccgc tgtgcaaaga gaagaaatat gattatgata atttgcccag gacatctgtt 420

atcatagcat tttataatga agcctggtca actctccttc ggacagttta cagtgtcctt 480

gagacatccc cggatatcct gctagaagaa gtgatccttg tagatgacta cagtgataga 540

gagcacctga aggagcgctt ggccaatgag ctttcgggac tgcccaaggt gcgcctgatc 600

cgcgccaaca agagagaggg cctggtgcga gcccggctgc tgggggcgtc tgcggcgagg 660

ggcgatgttc tgaccttcct ggactgtcac tgtgagtgcc acgaagggtg gctggagccg 720

ctgctgcaga ggatccatga agaggagtcg gcagtggtgt gcccggtgat tgatgtgatc 780

gactggaaca ccttcgaata cctggggaac tccggggagc cccagatcgg cggtttcgac 840

tggaggctgg tgttcacgtg gcacacagtt cctgagaggg agaggatacg gatgcaatcc 900

cccgtcgatg tcatcaggtc tccaacaatg gctggtgggc tgtttgctgt gagtaagaaa 960

tattttgaat atctggggtc ttatgataca ggaatggaag tttggggagg agaaaacctc 1020

gaattttcct ttaggatctg gcagtgtggt ggggttctgg aaacacaccc atgttcccat 1080

gttggccatg ttttccccaa gcaagctccc tactcccgca acaaggctct ggccaacagt 1140

gttcgtgcag ctgaagtatg gatggatgaa tttaaagagc tctactacca tcgcaacccc 1200

cgtgcccgct tggaaccttt tggggatgtg acagagagga agcagctccg ggacaagctc 1260

cagtgtaaag acttcaagtg gttcttggag actgtgtatc cagaactgca tgtgcctgag 1320

gacaggcctg gcttcttcgg gatgctccag aacaaaggac taacagacta ctgctttgac 1380

tataaccctc ccgatgaaaa ccagattgtg ggacaccagg tcattctgta cctctgtcat 1440
gggatgggcc agaatcagtt tttcgagtac acgtcccaga aagaaatacg ctataacacc 1500
caccagcctg agggctgcat tgctgtggaa gcaggaatgg atacccttat catgcatctc 1560
tgcgaagaaa ctgccccaga gaatcagaag ttcatcttgc aggaggatgg atctttattt 1620
cacgaacagt ccaagaaatg tgtccaggct gcgaggaagg agtcgagtga cagtttcgtt 1680
ccactcttac gagactgcac caactcggat catcagaaat ggttcttcaa agagcgcatg 1740
ttatgaagcc tcgtgtatca aggagcccat cgaaggagac tgtggagcca ggactctgcc 1800
caacaaagac ttagctaagc agtgaccaga acccaccaaa aactaggctg cattgctttg 1860
aagaggcaat cattttgcca tttgtgaaag ttgtgttgga tttagtaaaa atgtgaataa 1920
gctttgtact tattttgaga actttttaaa tgttccaaaa taccctattt tcaaagggta 1980
atcgtaagat gttaaccctt ggtatttaga aaattaaaac cttataatat ttttctatca 2040
agatgtatat tttacagtcg tgccttttac tctcattagc aaaaaagata aagattttat 2100
tttggtattt acaagaattc ccaggtacga agatatctgc atgggtggaa atcaggttca 2160
agcaacgtac tttgcattaa ctgataatac ctcagctgcg gggttaaagt tttcccagta 2220
tagagagact gtcactagga acattgtatt gatttattca ggtcattgag atcttctaga 2280
tgtattttaa aaagaatgct ttttggttat gtgttgctac cacagttaac actccataat 2340
gttcatgtca gccaaagagg actaaccaaa gctgaaatct cagagaacaa tttgctttac 2400
taagctgagt caacttgaga gcgaacttct aacaatgccg cactgtagtg tggctggttc 2460
taccactatg actttaaaac atgtttatat catttttaat ttttatgata cggtagtgtc 2520
agggagaaat gtaatgttct atatgaaatt cctttttcaa gtttgttcat taataacagt 2580
tattaattta aatcagcgtt agagtttgtg ctgctgcaac tgctgtgaaa atttctctga 2640
gtaattctga tttgtgaatg atcccagacc aaccctgaga ttttgtcaac ctgattaagt 2700
caatatgaat gattaaaaag atgtgagaac aaaaaaaaaa aaaaaaaaaa aaaaaaaaaa 2760
aaaaaaaaaa 2770

<210> 70
<211> 4447
<212> DNA
<213> Homo sapiens

<400> 70
atgcggcggc gctcgcggat gctgctctgc ttcgccttcc tgtgggtgct gggcatcgcc 60
tactacatgt actcgggggg cggctctgcg ctggccgggg gcgcgggcgg cggcgccggc 120
aggaaggagg actggaatga aattgacccc attaaaaaga aagaccttca tcacagcaat 180
ggagaagaga aagcacaaag catggagacc ctccctccag ggaaagtacg gtggccagac 240
tttaaccagg aagcttatgt tggagggacg atggtccgct ccgggcagga cccttacgcc 300
cgcaacaagt tcaaccaggt ggagagtgat aagcttcgaa tggacagagc catccctgac 360

acccggcatg accagtgtca gcggaagcag tggcgggtgg atctgccggc caccagcgtg 420
gtgatcacgt ttcacaatga agccaggtcg gccctactca ggaccgtggt cagcgtgctt 480
aagaaaagcc cgccccatct cataaaagaa atcatcttgg tggatgacta cagcaatgat 540
cctgaggacg gggctctctt ggggaaaatt gagaaagtgc gagttcttag aaatgatcga 600
cgagaaggcc tcatgcgctc acgggttcgg ggggccgatg ctgcccaagc caaggtcctg 660
accttcctgg acagtcactg cgagtgtaat gagcactggc tggagcccct cctggaaagg 720
gtggcggagg acaggactcg ggttgtgtca cccatcatcg atgtcattaa tatggacaac 780
tttcagtatg tgggggcatc tgctgacttg aagggcggtt ttgattggaa cttggtattc 840
aagtgggatt acatgacgcc tgagcagaga aggtcccggc aggggaaccc agtcgcccct 900
ataaaaaccc ccatgattgc tggtgggctg tttgtgatgg ataagttcta ttttgaagaa 960
ctggggaagt acgacatgat gatggatgtg tggggaggag agaacctaga gatctcgttc 1020
cgcgtgtggc agtgtggtgg cagcctggag atcatcccgt gcagccgtgt gggacacgtg 1080
ttccggaagc agcaccccta cacgttcccg ggtggcagtg gcactgtctt tgcccgaaac 1140
acccgccggg cagcagaggt ctggatggat gaatacaaaa atttctatta tgcagcagtg 1200
ccttctgcta gaaacgttcc ttatggaaat attcagagca gattggagct taggaagaaa 1260
ctcagctgca agcctttcaa atggtacctt gaaaatgtct atccagagtt aagggttcca 1320
gaccatcagg atatagcttt tggggccttg cagcagggaa ctaactgcct cgacactttg 1380
ggacactttg ctgatggtgt ggttggagtt tatgaatgtc acaatgctgg gggaaaccag 1440
gaatgggcct tgacgaagga gaagtcggtg aagcacatgg atttgtgcct tactgtggtg 1500
gaccgggcac cgggctctct tataaagctg cagggctgcc gagaaaatga cagcagacag 1560
aaatgggaac agatcgaggg caactccaag ctgaggcacg tgggcagcaa cctgtgcctg 1620
gacagtcgca cggccaagag cgggggccta agcgtggagg tgtgtggccc ggccctttcg 1680
cagcagtgga agttcacgct caacctgcag cagtaggagg gtccgggagg ccctgccgtc 1740
ctgtctcctg caccattggg tggagtctgg tgatcgcatt attgattatg tttcttaaac 1800
tttctgcgaa actaatatac ctcagtattc catcatggtc tgaaagtcaa acttcggcaa 1860
ggcacggacg actgtgcaga cacatcagcg gcaagaagcg agaactgccc tccccctcct 1920
ctcggtgcag ccagccgggc ccccttcccc aggccggagc gcccctcttc cttccagctt 1980
tcacttctgc cggtccgcaa ctgagtgaca cccagcgaca accgactggg gagtggtaga 2040
agcaactgaa cggatgcgtg cgagctgagg acagggcggg aggaggggca cacatgcccc 2100
aggggagcga ggagaactct tgaaatctcc attttcaatc ccttcgaaat cacgtatggt 2160
ttccacaaag ccgagtcgtg tcacgtggca ggtttacgtc aatagtccct ctctctgctc 2220
ctccattcgc aagtgtcttc ctgggccaga ctcccctccc cctcatgtac ttgctatatt 2280
gaggatgaag ttttctatgg tgggacacta aatataaagc tatatagaga aagaatgtac 2340
ggtcagttcc ctatggtttc tgtagatcat cgtcatcttg tatattcccc acaaagccgt 2400

tcgcagcttc cgggagaagg ggccagagcc cggtatgggg caagttctct aacagaggga 2460
ggaggtggcc tttgtgtccc ctggaggccc ccggatccag ccagttggtg ctactgctgt 2520
ggccagctgg ggggcttcct ccagaccacc ggcctcggcc ccggcatccc tgttgggcgt 2580
cagcctgaga gtccctactg tgcgtcagaa tccaccttgc gtgctgtgcg tatctgtgaa 2640
cctgggagac ggttacttat tttgacagat atcactttgg gtctttttac attaaatttc 2700
ttttctctaa ggaatataag acatacccca tagctctgtg tgagccagca ataccgctgc 2760
cccctggcga cagggcagac caatgatgcc aggcagctgt cacacgctag tattggcttc 2820
attgtgatct gagccctgca cgctgggcct tcagaattaa tggccagcag tgtcagggat 2880
gagcccgtca gccagggcac aggcctggct cacagtcctg cacacctgct ggcctggggg 2940
agctccagcc aggcagcgag tcctgccccg cccgcagctc cctcccacac cccgcctggc 3000
caagatgact gcttcagggg gctttgggga aagaattagg aagggtcaga accaaacaat 3060
acctgctcat ttacactgag gattcagggc gggagacagg agccttgggg tcctgttaaa 3120
ccacagacag ttatgaactg aaagtcataa cggggagagg tgcctggctt ctacctgggt 3180
gctcaggaat gttcctcctc acccctgcca ctctgtggtc ggtgccctgc ttcctcctcc 3240
actcctggcc gccttctcca gcgccgcaca cacagatgct cagtctcaga gaggctggca 3300
cggcctggca gtctgagaaa atcgtcagtt agccactgca ggccctcggt gggacagcgg 3360
cggccttgga ttaggagcca cctgggagtg tgccggtgcc atgctcctcc ctgtgtcttg 3420
tatgaaaggg ccactgtgtg tcttcctccc cggcgggagc ccacatgttg cactgtagga 3480
cagcggcccg aagtggaagc ctggctggag gctgccctat aggtctctct ccgcctcccc 3540
tgccatgcaa ccagatgtgt gtgaatgggc cacgtgcccc cacgctggat actccgccac 3600
gcttctgtct ttcacggtgg gcgctcgggg ggagctgaga aaacccctta gtactgtgcg 3660
agctgtggag tgcaggccag agcagggtgt gcgtagcccc cagcacccag gttcttctgt 3720
cagaccctgt gacctgcgag ctgctactac tgtaaggagg gaaatggatg aatctggctc 3780
gttttaaaat cacgttttct gacgaatcct ttgccccttc acctttaccc cgcccgcacc 3840
cctaggccct ctcagccttc ctatcatccc acgtgtctac ccagaccctt gtgcggccca 3900
tgccctgggg gcggcgtcct gtccctgagc tgggaggcgg ctttggatgg tccgggcgtc 3960
aagagcaggg gtgggccggg gaggggtcct ttgcggtgag ctatgtttac attgacacag 4020
tgtgccaaag tgacttactg cggttgcgtt agtttctgtc atcaggacac tatctcaccc 4080
tcccactcct gtttttaaaa ctcagaattc tttcctaaga gcccttcgaa gcaaagcgtg 4140
ccgaagttag ttgtcttctc tgtgctggtc ctttcttatg tcctcataaa agctcagatg 4200
atggtatctg tgagtatgtt ttgcaaattc aaaatatagt ttggtaattt tttttgccag 4260
ttgattttta aaaagaactg ctgtacagag ctagtacttt gtccatttta tgaatggaaa 4320
ccatccttga aaattgttta acttaaataa agagaagata ctttctagat accgctcttt 4380
gctggccaga gtctgtcctg gtgtgtgaag gctgggtggg gtgggtgtgg ggactgggat 4440

tggggtc 4447

<210> 71
<211> 3874
<212> DNA
<213> Homo sapiens

<400> 71
   atggctcacc taaagcgact agtaaaatta cacattaaaa gacattacca taaaaagttc 60
   tggaagcttg gtgcagtaat ttttttcttt ataatagttt tggttttaat gcaaagagaa 120
   gtaagtgttc aatattccaa agaggaatca aggatggaaa ggaacatgaa aaacaaaaac 180
   aagatgttgg atttaatgct agaagctgta aacaatatta aggatgccat gccaaaaatg 240
   caaataggag cacctgtcag gcaaaacatt gatgctggtg agagaccttg tttgcaagga 300
   tattatacag cagcagaatt gaagcctgtc cttgaccgtc cacctcagga ttcaaatgca 360
   cctggtgctt ctggtaaagc attcaagaca accaatttaa gtgttgaaga gcaaaaggaa 420
   aaggaacgtg gggaagctaa acactgcttt aatgctttcg caagtgacag gatttctttg 480
   caccgagatc ttggaccaga cactcgacct cctgaatgta ttgaacaaaa atttaagcgc 540
   tgccctcccc tgcccaccac cagtgtcata atagtttttc ataatgaagc gtggtccacg 600
   ttgcttagaa ctgtccacag tgtgctctat tcttcacctg caatactgct gaaggaaatc 660
   attttggtgg atgatgctag tgtagatgag tacttacatg ataaactaga tgaatatgta 720
   aaacaatttt ctatagtaaa aatagtcaga caaagagaaa gaaaaggtct gatcactgct 780
   cggttgctag gagcaacagt cgcaacagct gaaacgctca catttttaga tgctcactgt 840
   gagtgtttct atggttggct agaacctctg ttggccagaa tagctgagaa ctacacggct 900
   gtcgtaagtc cagatattgc atccatagat ctgaacacgt ttgaattcaa caaaccttct 960
   ccttatggaa gtaaccataa ccgtggaaat tttgactgga gtctttcatt tggctgggag 1020
   tcgcttcctg atcatgagaa gcaaagaagg aaagatgaaa cctacccaat taaaacaccc 1080
   acttttgcag gaggactttt ttccatatca aaagaatatt ttgagtatat tggaagctat 1140
   gatgaagaaa tggaaatctg gggaggtgaa aatatagaaa tgtctttcag agtatggcaa 1200
   tgtggtgggc agttggagat tatgccttgc tctgttgttg gacatgtttt tcgcagcaaa 1260
   agccctcata gctttccaaa aggcactcag gtgattgcta gaaaccaagt tcgccttgca 1320
   gaagtctgga tggatgaata caaggaaata ttttatagga gaaatacaga tgcagcaaaa 1380
   attgttaaac aaaaagcatt tggtgatctt tcaaaaagat ttgaaataaa acaccgtctt 1440
   cggtgtaaaa attttacatg gtatctgaac aacatttatc cagaggtgta tgtgccagac 1500
   cttaatcctg ttatatctgg atacattaaa agcgttggtc agcctctatg tctggatgtt 1560
   ggagaaaaca atcaaggagg caaaccatta attatgtata catgtcatgg acttggggga 1620
   aaccagtact ttgaatactc tgctcaacat gaaattcggc acaacatcca gaaggaatta 1680
   tgtcttcatg ctgctcaagg tctcgttcag ctgaaggcat gtacctacaa aggtcacaag 1740
   acagttgtca ctggagagca gatatgggag atccagaagg atcaacttct atacaatcca 1800
   ttcttaaaaa tgtgcctttc agcaaatgga gagcatccaa gtttagtgtc atgcaaccca 1860
   tcagatccac tccaaaaatg gatacttagc caaaatgatt aagtgttcct taaaattaag 1920
   ttgaaaaagg aaatattctt tctcataaaa ctgtgactag gcatacactg tagtttttga 1980
   aaattatgca aaagcagcta aatgtaactt attccaagtg catttttctt atttatatct 2040
   ttatgtagca ctactacaga aattctgcaa gtttctgttt caaagcacaa taactagtaa 2100
   taccaaagac tatttcaaaa tgtccagatg taggggaaga gatgtttaca gtatgatgaa 2160
   aataattttc caagtaaagt gatgtttgtg tgttttgtac acttagggat atatatatat 2220
   agctacattc acacactcac aatttaaaat atttccccta gtttttgggg gggataggaa 2280
   gaaagatttg ttactgtatt tttttaacta cataaaaata gatcaataaa tgtcagcatt 2340
   ggcctctgtg tacaaaccaa gagcttttac agatccagaa tttattagtt taaaatgcag 2400
   gtgaactttt ttttgcgttt ggtttacttg tctgtcaaat gtttccttaa acatgaaact 2460
   gaataaggag aagagtattt ttaacactta aatttcttgg caaattttaa aacatttttt 2520
   agtctgtaat acactccact tgaagcactt aagtcttcct taaatgactt ttcttaagta 2580
   atgatactgt gtgttttccc aaagcacttt taaaaaaatt tttataaatt actatctgtt 2640
   gaaaaggtgt ccttttcctt tcttctagta ttttttttct taccaaaatt cactaatctt 2700
   gaatgtttgt gatattaaat ttcaaatgca gaatacttga ctcatttaaa gctaaatttt 2760
   gttactgatt caattataat tgtaatggat ttttgacttt gtaatggatt cttttcatca 2820
   aaaagcctta ttatttttta tctatgtgga aaacacaata aaaaatcctc aacactattg 2880
   taatcatttg gttaagtgct tattcctctt ttgggtaaaa tctgtaattg ataataggtg 2940
   ggggaaaatg aattttgtat gctgaatttc taagcgccta ttgtttgtaa aaccatcaga 3000
   tatttcttat ggcacaaaaa atgaggaata gcaaaattcc tgtgttcaat atttagaaaa 3060
   ttttgtatta atttctgata aagttcctta agcatctgat agaatgatgt tttaaaaaaa 3120
   tttgacgctt gcttaggaga tttaccactt tttttttttg tttttcgtca ttttatattt 3180
   agatctcctg tattcttgtt cccgaagtaa aatacgatcg gtttcatatt ttaaatctgg 3240
   cagagcctca gctgtacgaa aaagagcata tactggttat tgaccctatc ttctcattgt 3300
   ttgtttgtaa gtttgaattt gtattaaaaa gcctgcattc tgagctggac atggtggctc 3360
   agcttctaat cccagcactt tggtaggcaa aggtgggagg atcatttgag ctcaggagtt 3420
   ccagaccagc ctgggcaaca tagcaaaatc tcatctctac aaaaagtaaa aattaaaaaa 3480
   tgaaattaaa aataaaatta cctaggtgtg gtggcacgca tctgtagttc cagctataca 3540
   ggaaggtgag gcagaagcat tgcttgagct tgggagatcg aggctacagt gagctatgat 3600
   tacaccactg cacttcagtc tgtgtgactg agcaagactc tttcaaaaaa aaaaaaaagc 3660
   ctacattctc cagttgatta tttccaacta atgtgtatta tgtgcctaat tttctatcag 3720
   aagttgtatt aagcccgttt tcacactgct gttaaagaca tacctgagac tgggtaattt 3780
   ataaagaaaa ataggttcaa tggacccaca ggtccgcgtg gctggggaag cttcacaatc 3840
   atggcggaag gtgaaagcat gtcttacgtg gaag 3874

<210> 72
<211> 5360
<212> DNA
<213> Homo sapiens

<400> 72
gggagagagg agttgggctg tgccggaggc cgaggaccga gagggctcag gtgacccctg 60
gaaagcctgg gtggctggaa aggagcctag cgcctgcatg aaaggaagaa cctgctggga 120
agtacctgag ctcgagctgt gggttccgcc gcccttcccc tgcgtggtgg cttggtggcc 180
gcgtctgcgc ctcagccctg agaatccgga tggcggtgag gtggacttgg gcaggcaaga 240
gctgcctgct gctggcgttt ttaacagtgg cctatatctt cgtggagctc ttggtctcta 300
cttttcatgc ctccgcagga gccggccgtg ccagggagct ggggtcaaga aggctctcag 360
acctccagaa aaatacggag gatttgtctc gaccgcttta taagaagccc cctgcagatt 420
cccgtgcact tggggagtgg gggaaagcca gcaaactcca gctcaacgag gatgaactga 480
agcagcaaga agaactcatt gagagatacg ccatcaatat ttacctcagt gacaggattt 540
ccctgcatcg acacatagag gataaaagaa tgtatgagtg taagtcccag aagttcaact 600
ataggacact tcctaccacc tctgttatca ttgctttcta taacgaagcc tggtcgactt 660
tgctccgtac cattcacagt gttttagaaa cttctcctgc agttcttttg aaagagatca 720
tcttggtgga tgacttgagt gacagagttt atttgaagac acaacttgaa acttacatca 780
gcaatcttga tagagtacgc ttgattagga ccaataagcg agaggggctg gttagggccc 840
gtctgattgg ggccactttc gccactgggg acgtcctcac tttcctggat tgtcactgtg 900
agtgtaattc cggttggctg gaaccgcttt tggaaaggat tgggagagat gaaacagcag 960
ttgtgtgtcc tgttatagac acaattgatt ggaatacttt tgaattctat atgcagatag 1020
gggagcccat gattggtggg tttgactggc gtttaacatt tcagtggcat tctgtcccca 1080
aacaggaaag ggacaggcgg atatcaagaa ttgaccccat cagatcacct accatggctg 1140
gaggactgtt tgctgtcagc aagaaatatt ttcagtacct tggaacgtat gacacaggaa 1200
tggaagtgtg gggaggtgaa aaccttgagc tgtcttttag ggtgtggcag tgtggtggca 1260
aattggagat ccacccgtgt tcccacgtgg gccatgtgtt ccccaagcgg gcaccatatg 1320
ctcgccccaa tttcctacag aatactgctc gggcagcaga agtttggatg gatgaataca 1380
aagagcactt ctacaataga aaccctccag caagaaaaga agcttatggt gatatttctg 1440
aaagaaaatt actacgagag cggttgagat gcaagagctt tgactggtat ttgaaaaacg 1500
tttttcctaa tttacatgtt ccagaggata gaccaggctg gcatggggct attcgcagta 1560
gagggatctc gtctgaatgt ttagattata attctcctga caacaacccc acaggtgcta 1620
acctttcact gtttggatgc catggtcaag gaggcaatca attctttgaa tatacttcaa 1680
acaaagaaat aaggtttaat tctgtgacag agttatgtgc agaggtacct gagcaaaaaa 1740

attatgtggg aatgcaaaat tgtcccaaag atgggttccc tgtaccagca aacattattt 1800
ggcattttaa agaagatgga actatttttc acccacactc aggactgtgt cttagtgctt 1860
atcggacacc ggagggccga cctgatgtac aaatgagaac ttgtgatgct ctagataaaa 1920
atcaaatttg gagttttgag aaatagagca caacagcact ttcgtcatga gctgacagta 1980
gtgtcaagaa agtcaaagag ccttaagagc ctcagtgaag attgtatttt attttatcaa 2040
aagccaccta gcagtcatct gtggagcact ggaaagctgg ggttcatttt ggtatatcac 2100
actgaaactg ggtacccaga gtgctgctgt ttaatatttc acaatgcctt acttattggt 2160
tgttttatat aagagttttg tcaatatggt ctcttcttaa aagaagttga ctatgaattg 2220
aaacacacaa aacatttaag tgccagactt aatattaaag aatgtaaagg tccaagtaaa 2280
atgaggtatg atttatgttg atgtgtaagt tcaccgcaca tcccactttt taacaaaact 2340
catgaatgtg cagtttgagc cattgctatt ttgattacat agaatttgta tttctttttt 2400
agccagcaca ttaaatttta gattttattt tttaatctaa tttttttcta atcaaaaaga 2460
aaattgagct taaggcaaaa ggcctggttt tagagatatg tgtaattgga agagggcatt 2520
tgtttgagtg tgagtttgga ggccttttta acatgcagac atacccatat ttaaatgaaa 2580
tggggagata tttacattcc gtactttgta aacttgagct attggacttc actgatgtat 2640
atattaatac ctcagattcc tctgattttg taagctgtct tctctgtgaa cgtgtttgtg 2700
tgtgtagggc attttctgat tgcacttcct taagttatga atgtactaga aagggactca 2760
tccagaatac tatgcctccc tttgttaatg cttaatcatt taaagtaaac acaattgaag 2820
cctctctgaa gttaaaccca actatgttta ttaaaatgtg tgaaactgaa agtgggctag 2880
gttctaccaa ggctgtggaa ctctcctacg agttctgctg atcaggaaat ttaagaattt 2940
atcttaaaaa tgcaaggaaa aaagactgcc ttggcaattg tgaatggtgc tttcaatctc 3000
ctagcaccga gcctggcact taggcagctt tcagtaagtg ggtgaatgaa tgactgaatg 3060
aatgaatgaa tggctcagct gaggaatgta actttggtca agttattatg atgtgtttgg 3120
gcttagtttt ctcattggta aaatgtgggt gctggattgg atcttaaaga tcccttccag 3180
ctctgaaatg ctgattgtac agtatattct tcccagattg actcactgtg caatctttac 3240
aatacttttt atcttttcac ttttgacata ggtaatgttg ttgagcagtt gagcaatgtt 3300
cagtccagtt gtgaagctgg agaagagaaa tgggttttaa aaattaagtg aggggaggcc 3360
gggtgcggtg gctcacgcat gtaatcccag cactttggga ggccaaggca ggtggatcac 3420
gaggtcagga gatccagacc atcctggcta acatggtgaa acctcgtctc tactaaaaat 3480
acaaaaaatt agccagctgt ggtggcgggc gcctgtagtc ccagctactc aggaggctga 3540
ggcaggagaa tggcgtgaac ctgtgaggca gagcttacag tgagccgaga tcgtgccact 3600
gcactccagc ctgggcgaca gagcaagact ctgtctcaaa aaaaaaaaat aataataaaa 3660
taagtgagct gaactcacct gaagtggttt acttctgtgg gttaagaagt tctagtcagt 3720
gttcatagtc gtttcgtttt gataattgtt gaaccaattt tgtttttaaa acctttagac 3780

tctgaaagta atattttgac taagaatgta aatatttcca aactaaatta ctcgggaagt 3840
aaacgctttt tttaaaagta tttttactgg ttttatacca atattatatg cagaaatcac 3900
aggatgaatt tagaattaaa tctcaattag ttcactttgg cctagattta tgaaaaatgc 3960
atgcctcgta aagagtccac tgtattcacg agtaaagttg cttttagtgt tcacttgatg 4020
acttggagag taggaatttt gcaaaatctg aatttaagga aattctttag gataaccatt 4080
tcaaaaaata aaattgctat gcaatcttga atattttctc ttttgcctcg taaaatgaaa 4140
atgcattcac agtttctgta aattatttag cagccttaaa gtttatcaaa aaattgtcca 4200
gattccacgt gcagcatgct tggccctgca tttaatttaa gaaggattaa taataatgct 4260
ctgaattttt cgaaagggat tctcctaaac ccacccactt ctcttgccca ggctgctttt 4320
taaaaatatt tttttatttt ttacttattt ttaaattttc tctttttatt tatttttggt 4380
tttcttgtta gccacctgtt atatgggaga acgaaaattg ttatattttg aaagtactta 4440
ttacattatt tttattttag tatcttgatg ctcctgtcaa aagggaaatg aggcttttaa 4500
aaataaagta ccttaattct ttattgactt tttgccctaa attgctaggt gtgacccagc 4560
aatcttttag gaagagattt tacagtggtg ctttatttat atcaataatc cagtatagtt 4620
aggctgttca ttcctcataa tagagtacat aacagaaaag tgggactttc acattttcat 4680
atttaggcac gttccaattt aattccaaaa atactctgta attctacatc taaaaaaacc 4740
gattccctaa ttcgaattta ttggtaccaa agctctcttt ggctatagac aattaagagt 4800
tgacctttta agttaatgta tatgcttaaa aacagtttta ggaaaatatt tggtagacaa 4860
agagtttcaa ctttaaatgt tcactatgtc atttagtgtc caactttacg gataggttga 4920
ctatctaaat aggcattttt agtcattaaa aaaaatctag tcaccaggag gatccctata 4980
actcaaaata acttgtttgt aaaagaaaat ttgtttactt acccattagt aagttcctgc 5040
atattcatta taagatggca aatcaaactt ttctaggatg aagacagctt atttttaagt 5100
tgtatagtct tagttggttt agggtctcaa ttttaattaa taaaatactt ggtttttatt 5160
tgcttgtcct tttgaattcc tgttttaata attttaaaat gagcacaaag aacgttgaag 5220
ttcagattaa tctcttctga atgatgtttt tttcctctgt gatgagttgt ttctgacttt 5280
tttccttttg tatttgtaat gttgattaag atgtaaaata aaaagtgtgc ctgattattt 5340
ttgcaaaaaa aaaaaaaaaa 5360

<210> 73
<211> 4520
<212> DNA
<213> Homo sapiens

<400> 73
ccaggctggt ccctcctgcc ttcgctctct gctggtcgcg gtggcctgta gctcctccct 60
cggtaggacc cccctccacc ttggcgctta cgagatgaat gaggagccca gacctctgga 120
acttggaggg ttgttcagat tcccaggctg acaaaaagga gaaacagctt ttacttgcat 180
ccagggccaa tcatcgcaga cccagacgtc tgcagagggc tgaggcccca gcttgtggcc 240

accacaacgt atcaagctat ctccagggtt gggctcagga ctcagagctg acgcagctgg 300
ggtgcccctt ggttctggag gatgaggctc ctccgcagac gccacatgcc cctgcgcctg 360
gccatggtgg gctgcgcctt tgtgctcttc ctcttcctcc tgcataggga tgtgagcagc 420
agagaggagg ccacagagaa gccgtggctg aagtccctgg tgagccggaa ggatcacgtc 480
ctggacctca tgctggaggc catgaacaac cttagagatt caatgcccaa gctccaaatc 540
agggctccag aagcccagca gactctgttc tccataaacc agtcctgcct ccctgggttc 600
tataccccag ctgaactgaa gcccttctgg gaacggccac cacaggaccc caatgcccct 660
ggggcagatg gaaaagcatt tcagaagagc aagtggaccc ccctggagac ccaggaaaag 720
gaagaaggct ataagaagca ctgtttcaat gcctttgcca gcgaccggat ctccctgcag 780
aggtccctgg ggccagacac ccgaccacct gagtgtgtgg accagaagtt ccggcgctgc 840
cccccactgg ccaccaccag cgtgatcatt gtgttccaca acgaagcctg gtccacactg 900
ctgcgaacag tgtacagcgt cctacacacc acccctgcca tcttgctcaa ggagatcata 960
ctggtggatg atgccagcac agaggagcac ctaaaggaga agctggagca gtacgtgaag 1020
cagctgcagg tggtgagggt ggtgcggcag gaggagcgga aggggctgat caccgcccgg 1080
ctgctggggg ccagcgtggc acaggcggag gtgctcacgt tcctggatgc ccactgtgag 1140
tgcttccacg gctggctgga gcccctcctg gctcgaatcg ctgaggacaa gacagtggtg 1200
gtgagcccag acatcgtcac catcgacctt aatacttttg agttcgccaa gcccgtccag 1260
aggggcagag tccatagccg aggcaacttt gactggagcc tgaccttcgg ctgggaaaca 1320
cttcctccac atgagaagca gaggcgcaag gatgaaacct accccatcaa atccccgacg 1380
tttgctggtg gcctcttctc catctccaag tcctactttg agcacatcgg tacctatgat 1440
aatcagatgg agatctgggg aggggagaac gtggaaatgt ccttccgggt gtggcagtgt 1500
gggggccagc tggagatcat cccctgctct gtcgtaggcc atgtgttccg gaccaagagc 1560
ccccacacct tccccaaggg cactagtgtc attgctcgca atcaagtgcg cctggcagag 1620
gtctggatgg acagctacaa gaagattttc tataggagaa atctgcaggc agcaaagatg 1680
gcccaagaga aatccttcgg tgacatttcg gaacgactgc agctgaggga acaactgcac 1740
tgtcacaact tttcctggta cctgcacaat gtctacccag agatgtttgt tcctgacctg 1800
acgcccacct tctatggtgc catcaagaac ctcggcacca accaatgcct ggatgtgggt 1860
gagaacaacc gcggggggaa gcccctcatc atgtactcct gccacggcct tggcggcaac 1920
cagtactttg agtacacaac tcagagggac cttcgccaca acatcgcaaa gcagctgtgt 1980
ctacatgtca gcaagggtgc tctgggcctt gggagctgtc acttcactgg caagaatagc 2040
caggtcccca aggacgagga atgggaattg gcccaggatc agctcatcag gaactcagga 2100
tctggtacct gcctgacatc ccaggacaaa aagccagcca tggccccctg caatcccagt 2160
gacccccatc agttgtggct ctttgtctag gacccagatc atccccagag agagccccca 2220
caagctcctc aggaaacagg attgctgatg tctgggaacc tgatcaccag cttctctgga 2280

ggccgtaaag atggatttct aaacccactg ggtggcaagg caggaccttc ctaatccttg 2340
caacaacatt gggcccattt tctttccttc acaccgatgg aagagaccat taggacatat 2400
atttagccta gcgttttcct gttctagaaa tagaggctcc caaagtaggg aaggcagctg 2460
ggggagggtt cagggcagca atgctgagtt caagaaaagt acttcaggct gggcacagtg 2520
gctcatgcct gaaatcctag cactttggga agacaatgtg ggagaatggc ttgagcccag 2580
gagttcaaga ccggcctgag caacatagtg aggatcccat ctctacgccc accctccccc 2640
cggcaaaaaa aaaaagctgg gtatggtggc ttatgcctgt agtcgcagct actcagaagg 2700
ctgaggtggg aggattgctt gttccccgga ggttgaagct acagtgagcc ttgattgtgt 2760
cactgcactc cagcctgggc aacaggtaag actctgtctc aaaaaaaaac aaaaaagaag 2820
aagaaaagta cttctacagc catgtcctat tccttgatca tccaaagcac ctgcagagtc 2880
cagtgaaatg atatattctg gctgggcaca gtggctcaca cctgtaatcc tagcactttg 2940
ggaggccaag gcaggtggat cacctgaggt cagaagtttg aaaccagcct ggactacatg 3000
gtgaaactcc atctctacta aaagtacaaa aattagctgg gcatgatggc acgcacctgc 3060
agtcccagct acttgggagg ctgaggcagg agaatcactc gaacccagga ggcagaggtt 3120
gcagtgagcc aagacagcac cattgcaccc cagcctgagc aacaagagcg aaactccatc 3180
tcaggaaaaa aaaaaaaaaa aaaagtatat tctaacagac agatcagagg tctaagagat 3240
cctcccttgc tattattacc tgaagtctgt agaactgttt acagatatct ccttgacagg 3300
tgtcctttat cttactttat ctgtacagta atcctgtgag aaagacagga cagaaaccac 3360
tgtgcctatt ttacagatac gaaaactgag acacaggtaa aggggcttgt ctgtagtccc 3420
atagctagca gatggctgga gccaagactg aggctcgttc ttcaatgctg agccagggct 3480
ccttccgctg caccacaaga acgctagacc actcgccacc agccttctca ttccctcttc 3540
ctccattcta atcatttcta gctggctggc ctccacagag cataggaaaa cagccagggc 3600
cgggcacggt ggctcatgcc tgtaatctca acactctggg aggccgagcc gggtggataa 3660
cctgaggtca ggaattcgag accagcctgg ccaacatggt aaaaccccat ctctactaaa 3720
aatataaaaa ttagccaggc atggtggcgc acacctgtaa tcccagctac tcaagaggct 3780
gaggcaggag aattgcttaa atctgggagg cggaagttgc agtgagccaa gatcgcgcca 3840
ctgaactcca gcctaggcaa caagagcaaa actccatctc caaaaaaaag aaaggaaaaa 3900
cagggccagg tagccattgt ggagagagca cacttaggaa tcctgggatg ttagtgttaa 3960
aagaaagctc ctggagccag tgattctcag gtttgtccca gaaccctttt ttctaagccc 4020
catataaaag gtagattaaa aaaacaaagt agcatgagtg aaattgagag agggacaggt 4080
aatgccttcc agcccctaac ttctaacaat ctggaagcac aacgtgaaaa tcacgtagcc 4140
caaccctatc attttcatat tatgaaactg agtccaggta agtgaatctg tccaaggtca 4200
cccagcaagg tatcagtagc cctgagggta aggactctga taaggctcgg gagggtcctg 4260
gaaagcctga ggcggcagga agagtgtgca gagttgagcg tgtctggaag gctgatccac 4320

tgctgggccc acatcaaagc ccccatgggg agcagacccg actgcacatg gctcttttgc 4380
tggaagaaga gcatggctgc gcagaggact aaaatttcat ctgggaaggc ttcttttgac 4440
tgtcagtagc aggatgtcac cagatgaggg tgctatggga ccacagctgt ctttgttccc 4500
attgcaactc aaccctgcgg 4520

<210> 74
<211> 4266
<212> DNA
<213> Homo sapiens

<400> 74
ccgggctgtg agtctctcgc cgccggagga agatgaggct gaagattggg ttcatcttac 60
gcagtttgct ggtggtggga agcttcctgg ggctagtggt cctctggtct tccctgaccc 120
cgcggccgga cgacccaagc ccgctgagca ggatgaggga agacagagat gtcaatgacc 180
ccatgcccaa ccgaggcggc aatggactag ctcctgggga ggacagattc aaacctgtgg 240
taccatggcc tcatgttgaa ggagtagaag tggacttaga gtctattaga agaataaaca 300
aggccaaaaa tgaacaagag caccatgctg gaggagattc ccagaaagat atcatgcaga 360
ggcagtatct cacatttaag cctcagacat tcacctacca tgatcctgtg cttcgcccag 420
ggatcctcgg taactttgaa cccaaagaac ctgagcctcc tggagtggtt ggtggccctg 480
gagagaaagc caagccattg gttttgggac cagaattcaa acaagcaatt caagccagca 540
ttaaagagtt tggatttaac atggtggcaa gtgacatgat ctcactggac cgcaacgtca 600
atgacttacg ccaagaagaa tgcaagtatt ggcattatga tgaaaacttg ctcacttcga 660
gcgttgtcat tgtcttccat aatgaaggat ggtcaaccct catgagaaca gtccacagtg 720
taattaaaag gactccaagg aaatatttag cagaaattgt gttaattgac gatttcagta 780
ataaagaaca cttaaaagaa aaactggatg aatatattaa gctgtggaat ggcctagtga 840
aggtatttcg aaatgaaaga agggaaggtt taattcaagc acgaagtatt ggtgctcaga 900
aggctaaact tggacaggtt ttgatatacc ttgatgccca ctgtgaagtg gcagttaact 960
ggtatgcacc acttgtagct cccatatcta aggacagaac catttgcact gtgccgctta 1020
tagatgtcat aaatggcaac acatatgaaa ttatacccca agggggtggt gatgaagatg 1080
ggtatgcccg aggagcatgg gattggagta tgctctggaa acgggtgcct ctgacccctc 1140
aagagaagag actgagaaaa acaaaaactg aaccgtatcg gtccccagcc atggctgggg 1200
gattatgtgc cattgaacga gagttcttct ttgaattggg tctctatgat ccaagtctcc 1260
agatttgggg tggtgaaaac tttgagatct catacaagat atggcagtgt ggtggcaaat 1320
tattatttgt tccttgttct cgtgttggac atatctaccg tcttgagggc tggcaaggaa 1380
atcctccgcc catttatgtt gggtcttctc caactctgaa gaattatgtt agagttgtgg 1440
aggtttggtg ggatgaatat aaagactact tctatgctag tcgtcctgaa tcgcaggcat 1500
taccatatgg ggatatatcg gagctgaaaa aatttcgaga agatcacaac tgccaaagtt 1560

ttaagtggtt catggaagaa atagcttatg atatcacctc acactaccct ttgccaccca 1620
aaaatgttga ctggggagaa atcagaggct tcgaaactgc ttactgcatt gatagcatgg 1680
gaaaaacaaa tggaggcttt gttgaactag gaccctgcca caggatggga gggaatcagc 1740
ttttcagaat caatgaagca aatcaactca tgcagtatga ccagtgtttg acaaagggag 1800
ctgatggatc aaaagttatg attacacact gtaatctaaa tgaatttaag gaatggcagt 1860
acttcaagaa cctgcacaga tttactcata ttccttcagg aaagtgttta gatcgctcag 1920
aggtcctgca tcaagtattc atctccaatt gtgactccag taaaacgact caaaaatggg 1980
aaatgaataa catccatagt gtttagagag aaaaaaataa accaataacc tacctactga 2040
caagtaaatt tatacaggac tgaaaaccgc ctggaaacct gctgcaacta ttgttattaa 2100
ctctgtatag ctccaaacct ggaacctcct gatcagtttg aaggacattg ataaactgtg 2160
attttacaat aacattatca tctgcagtta ctgtttacaa gactgctttt taccttaaac 2220
tttgtagatg tttacatctt ttttgttgtg ttttaaagat gatgttggta atttgtgcct 2280
tttagctctg ttttattaaa cagagttaaa gcatgttgtc ttctttggga ttacactcag 2340
gggttctgaa aggcagtttg atttttattt ttaacacact tgaaaaaagg ttggagtaac 2400
cagactttca tatataactt ggtgattatc aacctgttgt gtctttattt aattttacat 2460
ctttttgaag cactgccaca ggttattagc caaggtggcc ttccttcaca gtcatgctgc 2520
ttttttgaaa ggtgaatttc aacacattta gtgcctcttt catttctcag tatatatttc 2580
aagagcttgt gatgaaatct ataggatggt aatgatggac ttgtcacctg tatggggaat 2640
acttttacta ctcagaaatg aatttatgtg ctgccatttg ctataaagtt gaactttgta 2700
tggcttgaaa aagaaatgac catatggaac atcccaaggc tgtcccataa gggttggaag 2760
ttgtgtagca ttcactccct tacctactgg cattcccagt gccctctgtc catacctact 2820
tctaggattg caaagggagt cttccaacta gaagaaaaat tgtccactga catttgggat 2880
ttacttttct ccaatacctg ccaatacaga aaactattat cagttgttat tgttatccct 2940
tgaaaagcga gggtgacaaa aacaacaaaa caccgttata aacacatcaa aggttcattc 3000
tgactgaggt aagactttcc aagcccttgt tagattaggc cttataaaac ttgtgtgcat 3060
tataacctaa gctgtgcaac ctggtgaagc caagagtgaa ctgatgtttc atttatattt 3120
tcatccaaat gacattatct gcacgttttt aaaatttaaa aacaaaggac tatttaaaaa 3180
tacagtttat taacaaacgt gaaactactt tctgttacat taggtgttcc ctagtgtttc 3240
ttaatttctt tttagaaagt gtatttttat tagtattttt ccggtgaaca gaagatttgt 3300
ttggatttaa acatttacta agacagtacc tattaggaaa accaaatatt gcaaatggtc 3360
aattcgattt taatttctca aaagatactc tgttatccag aagattaaaa tgcctacatt 3420
gagtgcttaa aaaaaaaaac aactgtgatg atgtgagcag aatggcaagt aagttaagca 3480
tttttgatcc tgtaatcatg gtatcattac aatgaaagga attcacaaac tactgccaga 3540
ggaagtttgt tttttaattt aagagggaaa tataacctat aaatttgttt cttccaagct 3600

tagctcttaa atttgggaga ctcaaagtta aacatcctca acagagtttt atttataatt 3660

ttgaattgtc aatttgtatt ttgctactga tctgtgatca accattttaa ctttcatctc 3720

tagggatgtt taacatttat aattgcaaaa taaaccaact ataaaaaaag aaactaagag 3780

agaattggta ctttaattac ttgtgtgttt gcaaataggc tccattttcc atgttgagta 3840

gattataacc ttattaacta ggcataggcc taagaaaggt ggcaatgaac tgtgcatgta 3900

aattttaaat gggtactttg tgcaattcgt taaaagaaga tactctatga atatgattct 3960

atatattgaa atcagaaaac ctaccaaaca aaaacatcag aagctgctgc cataatgact 4020

attttctact gtaggctgct ttggaaataa ttcccatatc cttgctttgt aagttggtaa 4080

tatcactatg catttctaca cattttataa atttgattta tgcagatttt gatacactgt 4140

atgtttctgt agaaattgta taaatattca aaattttatt aggataaatt tgagaaactt 4200

acgtatatct taattctggg ttgcttgttt tttaggtgac aaaaataaaa tattgtattt 4260

taattc 4266

<210> 75
<211> 2135
<212> DNA
<213> Homo sapiens

<400> 75
ttcccacaaa agctaggctg gttctgattc ttaacctgct ccagcagtga cacactcagt 60

cccacaggga gtggacgacc cccaggaaga agatgatgtt ttggaggaaa ctccccaaag 120

ccctcttcat tgggctgact ctggccattg ctgtcaatct ccttctggta ttttctagca 180

aggggacttt acaaaacctg tttacgggtg gtctccacag ggagcttcct ttacatctga 240

ataaacgcta cggggcagtg ataaagagac tctcccactt ggaggtggaa ttgcaggatc 300

tgaaagaaag tatgaaatta gctctgaggc aacaagaaaa tgtgaacagc acactgaaga 360

gggcgaaaga tgaagtacgc cctcttctaa aggcaatgga aaccaaggtg aatgagacaa 420

agaagcacaa aacccaaatg aaactcttcc cacactcaca gcttttcagg caatggggcg 480

aggatctttc tgaggcccag cagaaggcgg cccaggacct cttccggaag tttggttaca 540

acgcgtacct cagcaaccag ctgcctctca atcgcaccat ccccgacacg cgagactaca 600

gatgtcttcg gaagacatat ccttcccaac tcccatccct cagtgtcatt ctcatattcg 660

tgaatgaagc tctgtccatt atacaacggg ccatcaccag tatcatcaac cggacgccct 720

ctcgattgtt gaaggaaatc atcttggtgg atgatttcag ctcaaatgga gaactaaagg 780

tacacttgga tgagaagatt aagctttaca accagaagta tccaggacta ctgaaaataa 840

tacggcatcc tgaaaggaaa ggtcttgctc aagcccgcaa cactggctgg gaagctgcca 900

cagcagacgt ggtcgccatc ttggatgctc acattgaagt caatgttggg tgggcagagc 960

caatcttggc tcggattcag gaggaccgca ctgtgattgt gtctcctgtg tttgacaaca 1020

ttcgttttga caccttcaaa ctggataagt atgaactggc agttgatggg tttaactggg 1080

aactctggtg ccgctacgat gcactgccac aagcctggat tgatctgcat gatgtcactg 1140

ccccagtgaa gagtccttca atcatgggca tcctggctgc taacaggcac ttcctgggag 1200
agatcgggtc tctggatggt ggaatgctca tctatggagg agagaacgtg gagcttagcc 1260
tgagggtgtg gcagtgtgga gggaaggtcg agattttgcc ctgttcccgg attgcccacc 1320
tagagagaca ccacaagccc tacgccttgg atctcaccgc tgccttgaag cgcaatgctc 1380
tgcgagtggc cgaaatctgg atggatgagc acaaacacat ggtctacttg gcctggaaca 1440
tacctctcca gaactctgga atagattttg gagacgtttc ttccagaatg gcactccggg 1500
aaaaactgaa atgtaaaact tttgactggt acctgaaaaa tgtttatcca ctcttgaagc 1560
cactccacac catcgtgggc tatggaagaa tgaaaaacct attggatgaa aatgtctgct 1620
tggatcaggg acccgttcca ggcaacaccc ccatcatgta ttactgccat gaattcagct 1680
cacagaatgt ctactatcac ctaactgggg agctctatgt gggacaactg attgcagagg 1740
ccagtgctag tgatcgctgc ctgacagacc ctggcaaggc ggagaagccc accttagaac 1800
catgctccaa ggcagctaag aatagactgc atatatattg ggattttaaa ccgggaggag 1860
ctgtcataaa cagagatacc aagcggtgtc tggagatgaa gaaggatctt ttgggtagcc 1920
acgtgcttgt gctccagacc tgtagcacgc aagtgtggga aatccagcac actgtcagag 1980
actggggtca gaccaacagc cagtgatcct cagatggtgc tggatctggg tcatcaattc 2040
ttgcaagtgg aatggcttct actcctaaca ctcccagctt ctttctcaat gagaaagaaa 2100
gcatgtgtat gtctgtttat ggcgacttca ggtgg 2135

<210> 76
<211> 1741
<212> DNA
<213> Homo sapiens

<400> 76
ggcgcttctg aagacgggtg ggcagacgat ggaggacgag gctctgtccc gcccctgcag 60
cttccttcgt tccggcccct tccagcgaag ggcaggctcc gagcagagac aacagacaga 120
aacccagaaa ccacctgcca cgatgtgaac ctgatgcaga aacgcggttc gctccctcgg 180
gatgcaaagg ccaacttcag cctggcacgg cggcgccgcg gactccacca tcccggggaa 240
ggagccggac ccctgtgtgg cagtgtggcg gcagcatgga ggtgctgccc tgctcccgcg 300
tggcccacat cgagcgcacc aggaagccct acaacaacga cattgactac tatgccaagc 360
gcaacgccct gcgcgccgcc gaggtgtgga tggatgactt caagtcccac gtgtacatgg 420
cctggaacat ccccatgtcg aacccagggg tggacttcgg ggacgtgtct gagaggctgg 480
ccctgcgtca gaggctgaag tgtcgcagct tcaagtggta cctggagaac gtgtacccgg 540
agatgagggt ctacaacaac accctcacgt acggagaggt gagaaacagc aaagccagtg 600
cctactgtct ggaccaggga gcggaggacg gcgaccgggc gatcctctac ccctgccacg 660
ggatgtcctc ccagctggtg cggtacagcg ctgatggact gctgcagctg gggcctctgg 720
gctccacagc cttcttgcct gactccaagt gtctggtgga tgacggcacg ggccgcatgc 780

ccaccctgaa gaagtgtgag gatgtggcgc ggccaacaca gcggctgtgg gacttcaccc 840
agagtggccc cattgtgagc cgggccacgg gccgctgcct ggaggtggag atgtccaaag 900
atgccaactt tgggctccgg ctggtggtac agaggtgctc ggggcagaag tggatgatca 960
gaaactggat caaacacgca cggcactgac cccacctccg cccggacccc cacagacctc 1020
gggaaggcgc tgggccgagc cagtgtggct gagtgaccgg ggtgtgcccg gcagacacag 1080
caggacaggg ctctatgtgc ggccaggaca gcagaggctg aggggccggg gtgtggctga 1140
gtgaccaggg tgtcacccac tgcatctgga gtacagcttc tcctaggaca ggcggctcta 1200
cccgagggag ggcgtctggg gacagtgatg ccaactcaaa cacgtgcctt ctccacggta 1260
tctcctggcc aggctgctgg gacagccgcc gcctctgcat gtaccacagc cccccacgcc 1320
ccatagggag gccaagcccc ggaccatgca ccaggctgca ccctggtgtc ttccacccgc 1380
aggcctccca tgctccaagc agcctccccc agcacttgcg gccgctcagc cctccaacca 1440
agacctaagt cacacatcat aatgcagctc cttgtggtct ggtaagaggg gtgctggagg 1500
acatggacag cgacaaacct gaggccgtgt ctaggaggct ggcacgactg cttcagcaga 1560
gaccaaataa agagaggatt aggcagcagc actgtgtgtg tcttcctggg agccgagctg 1620
ggggttgggg cccggtccca cctctttccc aggcacctgg gccctgccgc agcccagccc 1680
cttgcctgtt ttaataaata cagttttatt ggcgcccagc cacaaaaaaa aaaaaaaaaa 1740
a 1741

<210> 77
<211> 3435
<212> DNA
<213> Homo sapiens

<400> 77
tcaatgcact gttcaaggat gtttgagaga ttttctgtct tgcctgaata caaaacactg 60
tagggaacct cttgtgcttg actttgcaca cacacgggat cttcagggtg ggtacattta 120
aaactgacaa gtattgccaa attgcccccc aacgtagttt ttacacccca cctactactc 180
tcagtgaaaa atcaggccct ttttatttta agggctaaat tgctaatccc tgtgtgaaaa 240
tgggtgttaa caaaataaaa aataaggaaa atatgcaggc ttacccaatt ttgttatcta 300
tacattttac agtctctaat acttagcata gctcctttgg gtgtctgccc accacccttc 360
tttctgaacg tggggttccc tgggctttcc ttgtttgact tcagacctgg atctaatagg 420
gatctggagg tagaatcaag acgaatgttc cctttggcct taggtgagtc ctttgcatct 480
ctgaggccct gttgatgaca tccagttctc tagacctctg cccaaaccag agcaatccct 540
cctcaaatat gctaagcaga aatgcagaca atataagaca cactatattt atattttagg 600
agtttacatt accttccctt ctgtttggaa accaacatct ctcaaaatta ggtatagtta 660
acacagaata ccccaaaata ccaccattct tcataattcc atttccttat ttctggattc 720
tttcaagggt aaaaaccttg tttcattcat ctttggggca gcatttgcct agtatgtggt 780
aggcgcccag tagattttgg ataaactgac ctgattttct gttaatattc caactggatt 840

atttctttct cctcttacag aatgaagata ttcaaatgtt attttaaaca taccctacag 900
cagaaagttt tcatcctgtt tttaacccta tggctgctct ctttgttaaa gcttctaaat 960
gtgagacgac tctttccgca aaaagacatt tacttggttg agtactccct aagtacctcg 1020
ccttttgtaa gaaacagata cactcatgtt aaggatgaag tcaggtatga agttaactgt 1080
tcgggtatct atgaacagga gcctttggaa attggaaaga gtctggaaat aagaagaagg 1140
gacatcattg acttggagga tgatgatgtt gtggcaatga ccagtgattg tgacatttat 1200
cagactctaa gaggttatgc tcaaaagctt gtctcaaagg aggagaaaag cttcccaata 1260
gcctattctt tggttgtcca caaagatgca attatggttg aaaggcttat ccatgctata 1320
tacaaccagc acaatattta ctgcatccat tatgatcgta aggcacctga taccttcaaa 1380
gttgccatga acaatttagc taagtgcttc tccaatattt tcattgcttc caaattagag 1440
gctgtggaat atgcccacat ttccagactc caggctgatt taaattgctt gtcggacctt 1500
ctgaagtctt caatccagtg gaaatatgtt atcaacttgt gtgggcaaga ttttcccctg 1560
aagtcaaatt ttgaattggt gtcagagttg aaaaaactca atggagcaaa tatgttggag 1620
acggtgaaac ccccaaacag taaattggaa agattcactt accatcatga acttagacgg 1680
gtgccttatg aatatgtgaa gctaccaata aggacaaaca tctccaagga agcacccccc 1740
cataacattc agatatttgt tggcagtgct tattttgttt taagtcaagc atttgttaaa 1800
tatattttca acaactccat cgttcaagac ttttttgcct ggtctaaaga cacatactct 1860
cctgatgagc acttttgggc taccttgatt cgggttccag gaatacctgg ggagatttcc 1920
agatcagccc aggatgtgtc tgatctgcag agtaagactc gccttgtcaa gtggaattac 1980
tatgaaggct ttttctatcc cagttgtact ggatctcacc ttcgaagcgt gtgtatttat 2040
ggagctgcag aattaaggtg gcttatcaaa gatggacatt ggtttgctaa taaatttgat 2100
tctaaggtgg accctatctt gattaaatgc ttggcagaaa agcttgaaga acagcagaga 2160
gactggatca ctttgccctc agaaaagtta tttatggata gaaatctcac taccacatca 2220
tgatagtaaa atcaggatgg aaataagagg gtgcctgata aatggagtca gtgtggaatt 2280
gaataccata ctatgcccaa tactgtttaa actcagtcct cccatatttt aaaaggtgtc 2340
caaaattcca tacacaaggg aaagtgatct agcctttgat gttattagcc tgcagttggc 2400
taggtttttt taatatttgt ttttgcttgt aatctcactg agccaaatca gagatcttaa 2460
acattcagtc agtcatcaaa cattattgag cacctaacta tatgacaggc acttttttag 2520
agactgcggc ttatcctcat catagcaacc tcggtatctt taagttctcc acataacagt 2580
caggattcta ctgaagaagc ttttgaagtt tgtggtaatc gtctgatcat ataaaccacc 2640
catttcagag tagtgtttaa gtactgtgac caacactcca cttgtctctt aactcagctt 2700
tcaagacatt ccttaaccat cagagcagag gaggaaagac tcactacctc agaaaaatct 2760
caaagaatag tccaatttcc tgcttgccaa agcataatct gccttttggt gcattacttg 2820
gtcaattcag ggttgaggag actgttgggg ggcatttata atgtatgaaa gttaaggaag 2880

ggggtgagga tgtgggttgg gacaggtagt acctaaagag gagcgaaggg atttatacaa 2940

catttttatc atgttacaaa acagtatcat gaatggcctc ctttttagtt caactgtttc 3000

tttaaaatgc atttactgat taaaaataag aactgaccaa tagctccaag tgtcacacac 3060

cagaacatta agcctaagtc ctcaattcat aagttatcat tctagacaag tttctttaca 3120

atggaatact ttctatggaa tcattataat tctgttgtgt ggaactatca agctaagagt 3180

cactaaactt tcttgaaaag gttgtgtgaa atatgacagc tttctaaatt aatttgtata 3240

gtcatttaaa atttttcttt ctctggcaac tgtccaactg gaatccagat ttaaggtgat 3300

aaaagctcta agtttcttgc agtctttttc tcagcttagt tccagagaga aaaaagctaa 3360

ttttcctaag gacacagcaa gaatattcat taaggatatt ttctaaaacc cacacttgag 3420

aaaaccaccc aatga 3435

<210> 78
<211> 1552
<212> DNA
<213> Homo sapiens

<400> 78
acttaatggg ctcaaggtca cctacctgac ctaccagcag ctcaatggcc ttcccaaaga 60

agaaacttca gggtcttgtg gctgcaacca tcacgccaat gactgagaat ggagaaatca 120

acttttcagt aattggtcag tatgtggatt atcttgtgaa agaacaggga gtgaagaaca 180

tttttgtgaa tggcacaaca ggagaaggcc tgtccctgag cgtctcagag cgtcgccagg 240

ttgcagagga gtgggtgaca aaagggaagg acaagctgga tcaggtgata attcacgtag 300

gagcactgag cttgaaggag tcacaggaac tggcccaaca tgcagcagaa ataggagctg 360

atggcatcgc tgtcattgca ccgttcttcc tcaagccatg gaccaaagat atcctgatta 420

atttcctaaa ggaagtggct gctgccgccc ctgccctgcc attttattac tatcacattc 480

ctgccttgac aggggtaaag attcgtgctg aggagttgtt ggatgggatt ctggataaga 540

tccccacctt ccaagggctg aaattcagtg atacagatct cttagacttc gggcaatgtg 600

ttgatcagaa tcgccagcaa cagtttgctt tcctttttgg ggtggatgag caactgttga 660

gtgctctggt gatgggagca actggagcag tgggcagtac ctataactac ctgggaaaaa 720

agacaaacca gatgttggag gcttttgaac aaaaggactt ctctttagcc ctgaactatc 780

agttttgtat ccagagattt atcaactttg ttgtcaaact aggttttgga gtgtcacaga 840

ccaaagccat catgactctg gtctctggga ttccaatggg cccaccccgg cttccactgc 900

agaaagcctc cagggagttt actgatagtg ctgaagctaa actgaagagc ctggatttcc 960

tttctttcac tgatttaaag gatggaaact tggaagctgg tagctagtgc ctctctatca 1020

aatcagggtt tgcaccttga gacataatct accttaaata gtgcattttt ttctcaggga 1080

attttagatg aacttgaata aactctccta gcaaatgaaa tctcacaata agcattgagg 1140

taccttttgt gagccttaaa aagtcttatt ttgtgaaggg gcaaaaactc taggagtcac 1200

aactctcagt cattcatttc acagattttt ttgtggagaa atttctgttt atatggatga 1260

aatggaatca agaggaaaat tgtaattgat taattccatc tgtctttagg agctctcatt 1320

atctcggtct ctggttccta atcctatttt aaagttgtct aattttaaac cactataata 1380

tgtcttcatt ttaataaata ttcatttgga atctaggaaa actctgagct actgcattta 1440

ggcaggcact ttaataccaa actgtaacat gtctcaactg tatacaactc aaaatacacc 1500

agctcatttg gctgctcagt ctaactctag aatggatgct tttgaattca tt 1552

<210> 79
<211> 1535
<212> DNA
<213> Homo sapiens

<400> 79
gggcgtttac aggcaggcag gtcagtgatg tgtcctaagg gtccgaccga cctagatacc 60

cctctttgat tcctcctctt gggattagtg tccatctctg gaagcaggat ccaggaggac 120

gggaggggcc gctgcggacc gcagtcgctc cacctggagg agacaccaga aggaagacag 180

cctgagggac gcagccatcc ccggctccta ccggcgcccc gccccgcgca tgcgcacgcg 240

cacagggagt cagctggctg cgcgggaggt cacgggaagt ggggcggtgc ccagacagct 300

ggagggaagg aggtgtcagg cggggagaga cgcaaacggc gggaccagca gcgacggtag 360

cagcagcatg gccgcgatct atgggggtgt agagggggga ggcacacgat ccgaggtcct 420

tttagtctca gaggatggga agatcctggc agaagcagat ggactgagca caaaccactg 480

gctgatcggg acagacaagt gtgtggagag gatcaatgag atggtgaaca gggccaaacg 540

gaaagcaggg gtggatcctc tggtaccgct gcgaagcttg ggcctatctc tgagcggtgg 600

ggaccaggag gacgcgggga ggatcctgat cgaggagctg agggaccgat ttccctacct 660

gagtgaaagc tacttaatca ccaccgatgc cgccggctcc atcgccacag ctacaccgga 720

tggtggagtt gtgctcatat ctggaacagg ctccaactgc aggctcatca accctgatgg 780

ctccgagagt ggctgcggcg gctggggcca tatgatgggt gatgagggtt cagcctactg 840

gatcgcacac caagcagtga aaatagtgtt tgactccatt gacaacctag aggcggctcc 900

tcatgatatc ggctacgtca aacaggccat gttccactat ttccaggtgc cagatcggct 960

agggatactc actcacctgt atagggactt tgataaatgc aggtttgctg ggttttgccg 1020

gaaaattgca gaaggtgctc agcagggaga ccccctttcc cgctatatct tcaggaaggc 1080

tggggagatg ctgggcagac acatcgtagc agtgttgccc gagattgacc cggtcttgtt 1140

ccagggcaag attggactcc ccatcctgtg cgtgggctct gtgtggaaga gctgggagct 1200

gctgaaggaa ggttttcttc tggcgctgac ccagggcaga gagatccagg ctcagaactt 1260

cttctccagc ttcaccctga tgaagctgag gcactcctcc gctctgggtg gggccagcct 1320

aggggccagg cacatcgggc acctcctccc catggactat agcgccaatg ccattgcctt 1380

ctattcctac accttttcct agggggctgg tcccggctcc accccctcca agctcagtgg 1440

acactgggtc tgaaaggaag gagtcttttg cttcctttct cctttttaca aaaacaaaca 1500

tagaagaaaa taaatgcact ttatccactc cccaa 1535

<210> 80
<211> 2013
<212> DNA
<213> Homo sapiens

<400> 80
ctccgctgcg tgtaggtgaa gggggcttcc tgaccgagac atggatttag gtgctattac 60
aaaatactca gcattacacg ccaagcccaa tggactgatc cttcaatacg ggactgctgg 120
atttcgaacg aaggcagaac atcttgatca tgtcatgttt cgcatgggat tattagctgt 180
cctgaggtca aaacagacaa aatccactat aggagtcatg gtaacagcgt cccacaatcc 240
tgaggaagac aatggtgtaa aattggttga tcctttgggt gaaatgttgg caccatcctg 300
ggaggaacat gccacctgtt tagcaaatgc tgaggaacaa gatatgcaga gagtgcttat 360
tgacatcagc gagaaagaag ctgtgaatct gcaacaagat gcctttgtag ttattggtag 420
agataccagg cccagcagtg agaaactttc acaatctgta atagatggtg tgactgttct 480
aggaggtcaa ttccatgatt atggcttgtt aacaacaccc cagctgcact acatggtgta 540
ttgtcgaaac acgggtggcc gatatggaaa ggcaactata gaaggttact accagaaact 600
ctctaaggct tttgtggaac tcaccaaaca ggcttcttgc agtggagatg aatacagatc 660
acttaaggtt gactgtgcaa atggcatagg ggccctgaag ctaagggaaa tggaacacta 720
cttctcacag ggcctgtcag ttcagctgtt taatgatggg tccaagggca aactcaatca 780
tttatgtgga gctgactttg tgaaaagtca tcagaaacct ccacagggaa tggaaattaa 840
gtccaatgaa agatgctgtt cttttgatgg agatgcagac agaattgttt attactacca 900
tgatgcagat ggccactttc atctcataga tggagacaag atagcaacgt taattagcag 960
tttccttaaa gagctcctgg tggagattgg agaaagtttg aatattggtg ttgtacaaac 1020
tgcatatgca aatggaagtt caacacggta tcttgaagaa gttatgaagg tacctgtcta 1080
ttgcactaag actggtgtaa aacatttgca ccacaaggct caagagtttg acattggagt 1140
ttattttgaa gcaaatgggc atggcactgc actgtttagt acagctgttg aaatgaagat 1200
aaaacaatca gcagaacaac tggaagataa gaaaagaaaa gctgctaaga tgcttgaaaa 1260
cattattgac ttgtttaacc aggcagctgg tgatgctatt tctgacatgc tggtgattga 1320
agcaatcttg gctctgaagg gcttgactgt acaacagtgg gatgctctct atacagatct 1380
tccaaacaga caacttaaag ttcaggttgc agacaggaga gttattagca ctaccgatgc 1440
tgaaagacaa gcagttacac ccccaggatt acaggaggca atcaatgacc tggtgaagaa 1500
gtacaagctt tctcgagctt ttgtccggcc ctctggtaca gaagatgtcg tccgagtata 1560
tgcagaagca gactcacaag aaagtgcaga tcaccttgca catgaagtga gcttggcagt 1620
atttcagctg gctggaggaa ttggagaaag gccccaacca ggtttctgaa gataattttc 1680
atattcctga gaaactggac tttttacaag tctttacaaa actgtcaata ataatggcag 1740

tactaagaga tttataatca taatgtttac aatgcagcct actggattgt ctctagatct 1800

gtttttctta aacactaaca gaataattct ttataaatag gtaagcctta cacttgttaa 1860

agaaatttac ctctaatttc agtctcacta atgtaaaata ctgggactta agtatacaat 1920

tcagtcacta actgtacagt tttatgtggg gaacaattca tgcaggctac tggaaaatta 1980

aatcttatta ccaaaaaaaa aaaaaaaaaa aaa 2013

<210> 81
<211> 1472
<212> DNA
<213> Homo sapiens

<400> 81
gtggagggtc gcggcaggaa caagtgcggg ggcggggcct caaagccccg cccccaggcc 60

cagcggaacc ctgaagagga gggggagggt gagccgggcg cggctgtgtc agctgagtga 120

cagtcacggg accgcgacca actctgtctg gcttcctaga ggagcaggag ggcggggcaa 180

gggtctccca gcgcgacagg acatggagaa agagcgagag actctgcagg cctggaagga 240

gcgcgtgggg caggagctgg accgcgtggt ggctttctgg atggagcact cccacgacca 300

ggagcacggg ggcttcttca cgtgccttgg ccgcgagggg cgggtgtatg atgacctcaa 360

gtatgtgtgg ctgcagggga ggcaggtatg gatgtattgt cgcctgtacc gcactttcga 420

gcgcttccgc catgctcagc ttctggacgc agcaaaagca ggtggtgagt tcttgctgcg 480

gtatgcccgg gtggcacctc ctggcaagaa gtgtgccttt gtgctgactc gggacggccg 540

cccggtcaag gtgcagcgaa ccatcttcag tgagtgtttc tacaccatgg ccatgaacga 600

gctgtggaga gccacagggg aagtgcggta ccagacggaa gcggtggaga tgatggatca 660

gatcgtccac tgggtgcagg aggacgcgtc gggactgggc cggccccagc tccagggggc 720

cccggctgcg gagcccatgg cggtgcccat gatgctactg aacctggtgg agcagctcgg 780

ggaggcagat gaggagctgg cgggcaaata cgcagagctg ggggactggt gcgcccggag 840

gattctgcag cacgtgcaga gggatggaca agctgtgctg gagaatgtgt cagagggtgg 900

caaggaactt cctggctgcc tggggagaca gcagaaccca ggccacacgc tggaagccgg 960

ctggtttctg ctccgtcatt gcattcggaa aggcgacccc gaacttcgag cccacgtgat 1020

tgacaagttc ctattgttgc ccttccactc cggatgggac cctgaccacg gaggcctctt 1080

ttacttccag gatgctgata acttctgccc cacccagctg gagtgggcca tgaagctctg 1140

gtggccacac agtgaagcca tgattgcctt cctcatgggt tacagtgaca gtggggaccc 1200

tgtgctgctg cgcctcttct accaagtggc tgagtacacc ttccgccagt ttcgcgatcc 1260

cgagtacggg gaatggtttg gctacctgag ccgagagggc aaggtggccc tctccatcaa 1320

gggaggtcct ttcaaaggct gcttccacgt gccgcggtgc ctagccatgt gcgaggagat 1380

gctgggcgcc ctgctgagcc gccccgcccc cgccccctcc cccgccccca cccccgcctg 1440

ccgaggcgcg gaataaaggc tgagtccgct cc 1472

<210> 82
<211> 2753
<212> DNA
<213> Homo sapiens

<400> 82
gggccaggcc ggggcggggc cgcaagcggc atggaggagg cggaggccgc ggcgagccgg 60

gccgagcagt gagggcccta gcggggcccg agcggggccc ggggccccta agccattcct 120

gaagtcatgg gctggccagg acattggtga cccgccaatc cggtatggac gactggaagc 180

ccagccccct catcaagccc tttggggctc ggaagaagcg gagctggtac cttacctgga 240

agtataaact gacaaaccag cgggccctgc ggagattctg tcagacaggg gccgtgcttt 300

tcctgctggt gactgtcatt gtcaatatca agttgatcct ggacactcgg cgagccatca 360

gtgaagccaa tgaagaccca gagccagagc aagactatga tgaggcccta ggccgcctgg 420

agcccccacg gcgcagaggc agtggtcccc ggcgggtcct ggacgtagag gtgtattcaa 480

gtcgcagcaa agtatatgtg gcagtggatg gcaccacggt gctggaggat gaggcccggg 540

agcagggccg gggcatccat gtcattgtcc tcaaccaggc cacgggccac gtgatggcaa 600

aacgtgtgtt tgacacgtac tcacctcatg aggatgaggc catggtgcta ttcctcaaca 660

tggtagcgcc cggccgagtg ctcatctgca ctgtcaagga tgagggctcc ttccacctca 720

aggacacagc caaggctctg ctgaggagcc tgggcagcca ggctggccct gccctgggct 780

ggagggacac atgggccttc gtgggacgaa aaggaggtcc tgtcttcggg gagaaacatt 840

ctaaatcacc tgccctctct tcctgggggg acccagtcct gctgaagaca gatgtgccat 900

tgagctcagc agaagaggca gagtgccact gggcagacac agagctgaac cgtcgccgcc 960

ggcgcttctg cagcaaagtt gagggctatg gaagtgtatg cagctgcaag gaccccacac 1020

ccatcgagtt cagccctgac ccactcccag acaacaaggt cctcaatgtg cctgtggctg 1080

tcattgcagg gaaccgaccc aattacctgt acaggatgct gcgctctctg ctttcagccc 1140

agggggtgtc tcctcagatg ataacagttt tcattgacgg ctactatgag gaacccatgg 1200

atgtggtggc actgtttggt ctgaggggca tccagcatac tcccatcagc atcaagaatg 1260

cccgcgtgtc tcagcactac aaggccagcc tcactgccac tttcaacctg tttccggagg 1320

ccaagtttgc tgtggttctg gaagaggacc tggacattgc tgtggatttt ttcagtttcc 1380

tgagccaatc catccaccta ctggaggagg atgacagcct gtactgcatc tctgcctgga 1440

atgaccaggg gtatgaacac acggctgagg acccagcact actgtaccgt gtggagacca 1500

tgcctgggct gggctgggtg ctcaggaggt ccttgtacaa ggaggagctt gagcccaagt 1560

ggcctacacc ggaaaagctc tgggattggg acatgtggat gcggatgcct gaacaacgcc 1620

ggggccgaga gtgcatcatc cctgacgttt cccgatccta ccactttggc atcgtcggcc 1680

tcaacatgaa tggctacttt cacgaggcct acttcaagaa gcacaagttc aacacggttc 1740

caggtgtcca gctcaggaat gtggacagtc tgaagaaaga agcttatgaa gtggaagttc 1800

acaggctgct cagtgaggct gaggttctgg accacagcaa gaacccttgt gaagactctt 1860

tcctgccaga cacagagggc cacacctacg tggcctttat tcgaatggag aaagatgatg 1920
acttcaccac ctggacccag cttgccaagt gcctccatat ctgggacctg gatgtgcgtg 1980
gcaaccatcg gggcctgtgg agattgtttc ggaagaagaa ccacttcctg atggtggggg 2040
tcccggcttc cccctactca gtgaagaagc caccctcagt caccccaatt ttcctggagc 2100
cacccccaaa ggaggaggga gccccaggag ccccagaaca gacatgagac ctcctccagg 2160
accctgcggg gctgggtact gtgtaccccc aggctggcta gcccttccct ccatcctgta 2220
ggattttgta gatgctggta ggggctgggg ctaccttgtt tttaacatga gacttaatta 2280
ctaactccaa ggggagggtt cccctgctcc aacaccccgt tcctgagtta aaagtctatt 2340
tatttacttc cttgttggag aagggcagga gagtacctgg gaatcattac gatccctagc 2400
agctcatcct gccctttgaa taccctcact ttccaggcct ggctcagaat ctaacctatt 2460
tattgactgt cctgagggcc ttgaaaacag gccgaacctg gagggcctgg atttcttttt 2520
gggctggaat gctgccctga gggtggggct ggctcttact caggaaactg ctgtgcccaa 2580
cccatggaca ggcccagctg gggcccacat gctgacacag actcactcag agacccttag 2640
acactggacc aggcctcctc tcagccttct ctttgtccag atttccaaag ctggataagt 2700
tggtcattga ttaaaaaagg agaagccctc tggggaaaaa aaaaaaaaaa aaa 2753

<210> 83
<211> 2519
<212> DNA
<213> Homo sapiens

<400> 83
gctgttctcc tagcagcgcg ggccggtcgg accgccaagg cagccggcgc tggcgatggg 60
aagcggcgtg gccgccgaca caggcagtgg caaagtttcc cagacgtaca catctggacg 120
cgcggctgcc ggctacccgt gacccctcta ggaagggttc agggattttt aatttggaaa 180
aaaatccacc tggtttcctt tgtcaaggtc tctccgggtg gccagcggca ggagctgcaa 240
acttgggcac ggcggctaca ccggcagcgg accgggcttt ggagaacctc gggactcagg 300
tgctgaggtg cccagcggct ccggacgtgc tacggggtgc gagcgcgggg gagttcgggg 360
cgcacgacaa ggaagggccc ccgggagctc tatatggagg aaggagccca gaatggtgtg 420
caccaggaag accaaaactt tggtgtccac ttgcgtgatc ctgagcggca tgactaacat 480
catctgcctg ctctacgtgg gctgggtcac caactacatc gccagcgtgt atgtgcgggg 540
gcaggagccg gcgcccgaca agaagctgga ggaagacaaa ggggacactc tgaagattat 600
tgagcggctg gaccacctgg agaatgtcat caagcagcac attcaagagg ctcctgccaa 660
gcctgaggag gcagaggccg agcccttcac agactcctct ctgtttgcac actggggcca 720
ggagctcagc cccgaaggcc ggcgcgtggc cctgaagcaa ttccagtact acggctacaa 780
cgcctacctc agcgaccgcc tgcccctggg ccggcccctg cctgacctca gacccagtgg 840
gtgccgtaac ctctcatttc ctgacagcct gccagaggtg agcatcgtgt tcatcttcgt 900
caatgaagcg ctttcagtgc tgctgcgctc catccactcg gccatggaac gcacgccccc 960

acatctgctc aaggagatca ttctggtgga tgacaacagc agtaacgagg aactgaagga 1020

gaagctgacc gaatatgtgg acaaggtgaa cagccagaag ccaggcttca tcaaagtcgt 1080

gcgtcacagc aagcaggaag gcctcatccg ctccagggtc agtggctgga gggcggccac 1140

tgcccctgtg gtggcactct ttgatgccca cgtggagttc aatgtgggct gggctgaacc 1200

tgtactcacc cgcatcaagg agaaccggaa gcggatcatc tcgccatcct ttgataacat 1260

caaatatgac aactttgaga tagaagagta cccgctggct gcccagggct ttgactggga 1320

gctgtggtgc cgctacctaa atccccccaa ggcctggtgg aagctggaga actccacagc 1380

gccaatcagg agccctgccc tcattggctg cttcattgtg gaccggcagt acttccagga 1440

gatcggcctg ctggacgaag gcatggaagt ctacgggggc gagaatgtgg agcttgggat 1500

cagggtgtgg cagtgtggcg ggagtgtgga ggtcctgccc tgctcacgga ttgcccacat 1560

tgagcgagcc cacaagccct acacagagga cctcaccgcc catgtccgca ggaacgctct 1620

cagggtggct gaagtctgga tggatgaatt taaaagccac gtctacatgg catggaacat 1680

accgcaggag gactcaggaa ttgacattgg ggacatcact gcaaggaagg ctctcaggaa 1740

acagctgcag tgcaagacct tccggtggta cctggtcagc gtgtacccag agatgaggat 1800

gtactccgac atcattgcct atggagtgct gcagaattct ctgaagactg atttgtgtct 1860

tgaccagggg ccagatacag agaatgtccc catcatgtac atctgccatg ggatgacgcc 1920

tcagaacgtg tactacacga gcagtcagca gatccatgtg ggcattctga gccccaccgt 1980

ggatgatgat gacaaccgat gcctggtgga cgtcaacagc cggccccggc tcaccgaatg 2040

cagctacgcc aaagccaaga ggatgaagct tcactggcag ttctctcagg gaggacccat 2100

ccagaaccgc aagtctaagc gctgtctgga gctgcaggag aatagcgacc tggagttcgg 2160

cttccagctg gtgttgcaga agtgctcggg ccagcactgg agcatcacca acgtcctgag 2220

gagcctcgcg tcctgaccca ccggggccac ttccggctgc ctctttgcta ctgtgtagca 2280

cctgctgcaa cgttgcctgc tgtccacgtg gggttgtttg gagtctgggg aaccaggtta 2340

gtgggccccc aagaagagct ttttatttcc tattcaattt tcatggagtt tatagaaaga 2400

tgctgattgg taggtgatgg tatgatatca aactattttg cagttgtaaa taggggacag 2460

atggaaaata tttataactg acaataaaat attattaaga aaagaaaaaa aaaaaaaaa 2519

<210> 84
<211> 5536
<212> DNA
<213> Homo sapiens

<400> 84
gcgcggggcg ggagcttggt ggagcaggag cggctgggca tcctcctgag actccggggt 60

cagacgccca ctccagcgct ttctgcgcag cacaggcacc ctcgcctgtc acgcctccct 120

ctcctcttcc cgtctcccag cgtggatgag ggcgcctgca gatcccggct agggcacgga 180

ccgcctaggc cgagatttct ttaaagcctc gtgcagcaca tcattatcgc tggatgcccg 240

gacatgtaat acacctgaca gcatgtgaag tgctcagaat ggggcaggat gtcacctgga 300
atcagcacta agtgattcag actttcctta cttttaaatg tgctgctctt catttcaaga 360
tgccgttgca gctctgataa atgcaaactg acaaccttca aggccacgac ggagggaaaa 420
tcattggtgc ttggagcata gaagactgcc cttcacaaag gaaatccctg attattgttt 480
gaaatgctga ggacgttgct gcgaaggaga ctttttcttt atcccaccaa atactacttt 540
atggttcttg ttttatccct aatcaccttc tccgttttaa ggattcatca aaagcctgaa 600
tttgtaagtg tcagacactt ggagcttgct ggggagaatc ctagtagtga tattaattgc 660
accaaagttt tacagggtga tgtaaatgaa atccaaaagg taaagcttga gatcctaaca 720
gtgaaattta aaaagcgccc tcggtggaca cctgacgact atataaacat gaccagtgac 780
tgttcttctt tcatcaagag acgcaaatat attgtagaac cccttagtaa agaagaggcg 840
gagtttccaa tagcatattc tatagtggtt catcacaaga ttgaaatgct tgacaggctg 900
ctgagggcca tctatatgcc tcagaatttc tattgcattc atgtggacac aaaatccgag 960
gattcctatt tagctgcagt gatgggcatc gcttcctgtt ttagtaatgt ctttgtggcc 1020
agccgattgg agagtgtggt ttatgcatcg tggagccggg ttcaggctga cctcaactgc 1080
atgaaggatc tctatgcaat gagtgcaaac tggaagtact tgataaatct ttgtggtatg 1140
gatttcccca ttaaaaccaa cctagaaatt gtcaggaagc tcaagttgtt aatgggagaa 1200
aacaacctgg aaacggagag gatgccatcc cataaagaag aaaggtggaa gaagcggtat 1260
gaggtcgtta atggaaagct gacaaacaca gggactgtca aaatgcttcc tccactcgaa 1320
acacctctct tttctggcag tgcctacttc gtggtcagta gggagtatgt ggggtatgta 1380
ctacagaatg aaaaaatcca aaagttgatg gagtgggcac aagacacata cagccctgat 1440
gagtatctct gggccaccat ccaaaggatt cctgaagtcc cgggctcact ccctgccagc 1500
cataagtatg atctgtctga catgcaagca gttgccaggt ttgtcaagtg gcagtacttt 1560
gagggtgatg tttccaaggg tgctccctac ccgccctgcg atggagtcca tgtgcgctca 1620
gtgtgcattt tcggagctgg tgacttgaac tggatgctgc gcaaacacca cttgtttgcc 1680
aataagtttg acgtggatgt tgacctcttt gccatccagt gtttggatga gcatttgaga 1740
cacaaagctt tggagacatt aaaacactga ccattacggg caattttatg aacaagaaga 1800
aggatacaca aaacgtaccc ttatctgttt ccccttcctt gtcagcatcg ggaagatggt 1860
atgaagtcct ctttggggca gggactctag tagatcttct tgtcagagaa gctgcatggt 1920
ttctgcagag cacagttagc tagaaaggtg atagcattaa atgttcatct agagttaata 1980
gtgggaggag taaaggtagc cttgaggcca gagcaggtag caaggcattg tggaaagagg 2040
ggaccagggt ggctggggaa gaggccgatg cataaagtca gcctgttcaa agtgctcagg 2100
gacttagcaa aatgagaaga tgtgacctgt gccaaaacta ttttgagaat tttaaatgtg 2160
accatttttc tggtatgaat aaacttacag caacaaataa tcaaagatac aattaatctg 2220
atattatatt tgttgaaata gaaatttgat tgtactataa atgatttttg taaataattt 2280

atattctgct ctaatactgt actgtgtagt gtgtctccgt atgtcatctc agggagctta 2340
aaatgggctt gatttaacat tgaaaaaaaa ttttttgtgt tatttttgct tgaaacaacg 2400
cacacatttt caacaaccaa aaaatgacaa tttctagttt agttaatttc tacaaatcat 2460
cttatgttat tagcaaggtt aagacatctt ttttaaaaaa attatagctt ctaccaagag 2520
aaacactcaa tttttctaga gatttgcctc tatcttcctt tcctcagtct tcccagactg 2580
ctatcaagct gtgtaaaaat ttactttcac tggaccctaa attattgtct ctgctatctg 2640
actgccagta attagtgcag aaaactaaga caggatgata caggtttgag gggctgggga 2700
gtgggagggg ggagaaaagg aatgtattta aacaatttcc gatgcccatg atgagtttaa 2760
aaaccagcat tgacaccatc cccaaaatta aggctgtcgc ttattgaatc cacttgtgtc 2820
caacctccca ggattgtttt atcctaatgt cacctgtata ttcatttgaa aggacttggc 2880
cctgttcttg ggtcttcccg ttacctgccc cctgggtggt aagtttcctc ctttctcaac 2940
cttccacgag gaggaaagaa gtgtgcagtc attccacatg gcctgttgga aggcctgggg 3000
agggaacttt gggtttggga cagatttttt tttttgtttt tggtatcatt cacagcatac 3060
gatttttact ctctccatct tcaccataag acagataatt tggggttgct ataatgctgt 3120
cacacatctc aaagtacatt caaatcttaa aaagaaattc tcgtactttt gccatgttga 3180
tactgttcag caaacaagct accaggaact gtgaggcttt gtcatttagc attagacttt 3240
aaacaagaat taaaatcatg tgctgtattt ttaaaatcta gccaaattaa atagtacatg 3300
agaaattcag agtattagac agttttaagg cattcaactg agaaaacttt atttgtcaaa 3360
gtcagaaaac attttcatct tattgagaga tatgttttta aacttttatc atcatttgta 3420
aatgtggaag ttggtggatt gctgtgtttt tgcatgatta gcatgggagt ctgttggagc 3480
aagaggaaca tgcttgtttt gaaaactcga gatgatgagg gtggtacatg cagtgtgttc 3540
tctctttatt ggcttctaaa ccagttttgt cctttaatgc atgtcaaata tttctcccat 3600
gcttctctta gcagaaaagt ttttacctat aagacagggc accttttaac tctaaaacta 3660
gtgatactca gtgacataga ctttgtctta taaacatttt ttcatttttt attttgaaaa 3720
attgcaaatc tacagcaaaa gtaaaacagt agagtgaaca ccatgtaacc ctcacctggt 3780
gttaacattg taccctattt gctttaagtt gtatgtattt ctgaacttgg caaaattgga 3840
aattaaaatt tttaaaaatt acaaatatac aaagttattt atcttagcac atttattatg 3900
tgtgactgca tctgatttat atttaaattg gcaggttttg agggattttt ttcttcatca 3960
taaatgtaaa cataggattt tagagtctat ttccccaagc gccacattat aactgtaaac 4020
ttaccatctt ctatgtagct gtgatatctc atctttctaa aatggaactt gttaaaaagt 4080
gttcaaacac tatccctaat gcctgcggca gaatttatat acgatccatt cattggggct 4140
caaagtatct tttagacttt taaggacaat ttacagcaaa tgaaatttat gatgctgtga 4200
caagaaattt aaagaatcaa aacgatggtt tgaaaaggaa acctatgata catgcaggag 4260
aagcaaaacc caagtgattt gtgagaaata tagaaattat ttaaattact ttatagtaat 4320

tattaaacac taatttttgt actgtcattg aaggtgtttt atagagaaat ctgagaaatc 4380

acattcacaa attagaagtc aaacatggcc aggcacagtg gctcacacct gtaatcttca 4440

ggatttcaag accagcctag ccaacatgac gaaaccccat ctctactgaa aatagaaaaa 4500

aaaattagcc aggcttgcac ctgtaatccc agttacctgg gaggctgagg caggagaatc 4560

tcttgagccc aggaggcgga ggttgcagtg agctgagatg ccaccaccac accagcctgg 4620

gtgacagagt gaaactcgta tctccaaaca aacaaacaaa aagtccttaa acatatgtga 4680

acaaaaattt tgtgatggaa ggattctagt taatgagtat tgcatcaaga tttacatctt 4740

tcttactaag gaaaagagtt aataaaaatt gttctttatt ttacaggcag ttactgaggc 4800

tcttcccaga tctcagtaaa cagccactca gccttgaaaa tggagtgttg ttgtttctaa 4860

acatatattt atgtcattta ttaagtacag ttcacttaaa taacataagt agattttctc 4920

ttgtagtgat ttgggtagga agaggccatg tttcagttcg ttttctctgt agggtcgatt 4980

gaattggacc ttttcagttg ttcagaaaaa taaaaataat ttctcatatt aaatacagac 5040

gctcctcaac ttatgatgtg ggtaggtccc agtaaaccca ttataatttg aaaatatcat 5100

attgaaaatg catttaatat ctcttacctg aaatcataac ttagccaagc ctaccttaaa 5160

tgttctcaga acatttagcc tgcagttggg caaaaccatt taacacaaag cctattttat 5220

aatgaagtgt tgaatagctc atgttattta ctgaatactg ttgtgaaagt gaaaaacaat 5280

gattgtatgg gtactcaaag tataatttct actgaatgca tatcacttgt gcactgttgt 5340

aaagctgaaa aaccgttaag cctctacgat ttttaagtaa gttggggacc atcagtttaa 5400

aataaatgca atactatttc atgataaaca tggtcactgt aagttttact cttttgaatg 5460

agggtgcgac agaatgcagt tagaatcagt tcatatcacc attaaaatca tccattcaga 5520

aaccaaaaaa aaaaaa 5536

<210> 85
<211> 2128
<212> DNA
<213> Homo sapiens

<400> 85
cttgagaatg tttctgcatg atttctctgg gcttgctgtc ttacctgaga gcaggaagtc 60

aaaaggagag tgatggagcc acctgctgcc ctctactttg cagatattaa agaggagcct 120

gaaactgttc cttggacatc ttatgaatgt cagaaaatac cttttggagg gttagaagat 180

caggggacat ggttgttcac atttgctgcc acggaacacc gccagtcttc acttggaaac 240

agaatcacgc cttgtgaaga gatcatccct aagcaggaga gaagctacta aaggattgtg 300

tcctcctcca ccttccctgt gctcggtctc cacctgtctc ccattctgtg acgatggttc 360

aatggaagag actctgccag ctgcattact tgtgggctct gggctgctat atgctgctgg 420

ccactgtggc tctgaaactt tctttcaggt tgaagtgtga ctctgaccac ttgggtctgg 480

agtccaggga atctcaaagc cagtactgta ggaatatctt gtataatttc ctgaaacttc 540

cagcaaagag gtctatcaac tgttcagggg tcacccgagg ggaccaagag gcagtgcttc 600

aggctattct gaataacctg gaggtcaaga agaagcgaga gcctttcaca gacacccact 660

acctctccct caccagagac tgtgagcact tcaaggctga aaggaagttc atacagttcc 720

cactgagcaa agaagaggtg gagttcccta ttgcatactc tatggtgatt catgagaaga 780

ttgaaaactt tgaaaggcta ctgcgagctg tgtatgcccc tcagaacata tactgtgtcc 840

atgtggatga gaagtcccca gaaactttca aagaggcggt caaagcaatt atttcttgct 900

tcccaaatgt cttcatagcc agtaagctgg ttcgggtggt ttatgcctcc tggtccaggg 960

tgcaagctga cctcaactgc atggaagact tgctccagag ctcagtgccg tggaaatact 1020

tcctgaatac atgtgggacg gactttccta taaagagcaa tgcagagatg gtccaggctc 1080

tcaagatgtt gaatgggagg aatagcatgg agtcagaggt acctcctaag cacaaagaaa 1140

cccgctggaa atatcacttt gaggtagtga gagacacatt acacctaacc aacaagaaga 1200

aggatcctcc cccttataat ttaactatgt ttacagggaa tgcgtacatt gtggcttccc 1260

gagatttcgt ccaacatgtt ttgaagaacc ctaaatccca acaactgatt gaatgggtaa 1320

aagacactta tagcccagat gaacacctct gggccaccct tcagcgtgca cggtggatgc 1380

ctggctctgt tcccaaccac cccaagtacg acatctcaga catgacttct attgccaggc 1440

tggtcaagtg gcagggtcat gagggagaca tcgataaggg tgctccttat gctccctgct 1500

ctggaatcca ccagcgggct atctgcgttt atggggctgg ggacttgaat tggatgcttc 1560

aaaaccatca cctgttggcc aacaagtttg acccaaaggt agatgataat gctcttcagt 1620

gcttagaaga atacctacgt tataaggcca tctatgggac tgaactttga gacacactat 1680

gagagcgttg ctacctgtgg ggcaagagca tgtacaaaca tgctcagaac ttgctgggac 1740

agtgtgggtg ggagaccagg gctttgcaat tcgtggcatc ctttaggata agagggctgc 1800

tattagattg tgggtaagta gatcttttgc cttgcaaatt gctgcctggg tgaatgctgc 1860

ttgttctctc acccctaacc ctagtagttc ctccactaac tttctcacta agtgagaatg 1920

agaactgctg tgatagggag agtgaaggag ggatatgtgg tagagcactt gatttcagtt 1980

gaatgcctgc tggtagcttt tccattctgt ggagctgccg ttcctaataa ttccaggttt 2040

ggtaacgtgg aggagaactt tgatggaaag agaacttcct tctgtactgt taacttaaaa 2100

ataaatactc ctgatcaaaa aaaaaaaa 2128

<210> 86
<211> 2588
<212> DNA
<213> Homo sapiens

<400> 86
aggcgcgagg gctggctgct gggccgacga ggggcggtgc caggccgtgg gtccttagtc 60

aagtgacgcg aagcggccgg cctgggcgcc gactgcagag ccgggaggct ggtggtcatg 120

ccggggttcc tggttcgcat cctccctctg ttgctggttc tgctgcttct gggccctacg 180

cgcggcttgc gcaatgccac ccagaggatg tttgaaattg actatagccg ggactccttc 240

ctcaaggatg gccagccatt tcgctacatc tcaggaagca ttcactactc ccgtgtgccc 300
cgcttctact ggaaggaccg gctgctgaag atgaagatgg ctgggctgaa cgccatccag 360
acgtatgtgc cctggaactt tcatgagccc tggccaggac agtaccagtt ttctgaggac 420
catgatgtgg aatattttct tcggctggct catgagctgg gactgctggt tatcctgagg 480
cccgggccct acatctgtgc agagtgggaa atgggaggat tacctgcttg gctgctagag 540
aaagagtcta ttcttctccg ctcctccgac ccagattacc tggcagctgt ggacaagtgg 600
ttgggagtcc ttctgcccaa gatgaagcct ctcctctatc agaatggagg gccagttata 660
acagtgcagg ttgaaaatga atatggcagc tactttgcct gtgattttga ctacctgcgc 720
ttcctgcaga agcgctttcg ccaccatctg ggggatgatg tggttctgtt taccactgat 780
ggagcacata aaacattcct gaaatgtggg gccctgcagg gcctctacac cacggtggac 840
tttggaacag gcagcaacat cacagatgct ttcctaagcc agaggaagtg tgagcccaaa 900
ggacccttga tcaattctga attctatact ggctggctag atcactgggg ccaacctcac 960
tccacaatca agaccgaagc agtggcttcc tccctctatg atatacttgc ccgtggggcg 1020
agtgtgaact tgtacatgtt tataggtggg accaattttg cctattggaa tggggccaac 1080
tcaccctatg cagcacagcc caccagctac gactatgatg ccccactgag tgaggctggg 1140
gacctcactg agaagtattt tgctctgcga aacatcatcc agaagtttga aaaagtacca 1200
gaaggtccta tccctccatc tacaccaaag tttgcatatg gaaaggtcac tttggaaaag 1260
ttaaagacag tgggagcagc tctggacatt ctgtgtccct ctgggcccat caaaagcctt 1320
tatcccttga catttatcca ggtgaaacag cattatgggt ttgtgctgta ccggacaaca 1380
cttcctcaag attgcagcaa cccagcacct ctctcttcac ccctcaatgg agtccacgat 1440
cgagcatatg ttgctgtgga tgggatcccc cagggagtcc ttgagcgaaa caatgtgatc 1500
actctgaaca taacagggaa agctggagcc actctggacc ttctggtaga gaacatggga 1560
cgtgtgaact atggtgcata tatcaacgat tttaagggtt tggtttctaa cctgactctc 1620
agttccaata tcctcacgga ctggacgatc tttccactgg acactgagga tgcagtgcgc 1680
agccacctgg ggggctgggg acaccgtgac agtggccacc atgatgaagc ctgggcccac 1740
aactcatcca actacacgct cccggccttt tatatgggga acttctccat tcccagtggg 1800
atcccagact tgccccagga cacctttatc cagtttcctg gatggaccaa gggccaggtc 1860
tggattaatg gctttaacct tggccgctat tggccagccc ggggccctca gttgaccttg 1920
tttgtgcccc agcacatcct gatgacctcg gccccaaaca ccatcaccgt gctggaactg 1980
gagtgggcac cctgcagcag tgatgatcca gaactatgtg ctgtgacgtt cgtggacagg 2040
ccagttattg gctcatctgt gacctacgat catccctcca aacctgttga aaaaagactc 2100
atgcccccac ccccgcaaaa aaacaaagat tcatggctgg accatgtatg atgatgaaag 2160
cctgtgtctt tgagggattc taccctgaac atacctcaca gatcctccct gtcatgccac 2220
atttcactga ttggaatgtg gaaatggaaa aggaatttag gatgtgcatt ttcacctgag 2280

gtttccctgc atccctgcag tgccaaagcc ccaccttcag ggaccacctg gaatgtgtga 2340

ggggctgaca gcacagtaac gtgcatacat atctgcaggg ctggaatgga agctttaaag 2400

gtggtagtga tttttatttt ggaagaatca tgttaccttt ttgttaaata aaatttgtac 2460

tcaaatgatg atgtcactgt ttttaatgtg caggtattga attatatggt ctgacttaaa 2520

tcataactag acttgagtgg gctgaataaa ccacttcact aacttgaagt tcaaaaggat 2580

ggaaaata 2588

<210> 87
<211> 3760
<212> DNA
<213> Homo sapiens

<400> 87
gctctgcctg cttcgtggcg cttggttcgt ccctcgcccg aggagcgcgg tggcggcgtg 60

ggagggagcc tctgacggcg tctggaactc tattttaaga acctctcaaa acgaaacaag 120

caaatcatgg agaagaatgg aaataaccga aagctgcggg tttgtgttgc tacttgtaac 180

cgtgcagatt attctaaact tgccccgatc atgtttggca ttaaaaccga acctgagttc 240

tttgaacttg atgttgtggt acttggctct cacctgatag atgactatgg aaatacatat 300

cgaatgattg aacaagatga ctttgacatt aacaccaggc tacacacaat tgtgagggga 360

gaagatgagg cagccatggt ggagtcagta ggcctggccc tagtgaagct gccagatgtc 420

cttaatcgcc tgaagcctga tatcatgatt gttcatggag acaggtttga tgccctggct 480

ctggccacat ctgctgcctt gatgaacatc cgaatccttc acattgaagg tggggaagtc 540

agtgggacca ttgatgactc tatcagacat gccataacaa aactggctca ttatcatgtg 600

tgctgcaccc gcagtgcaga gcagcacctg atatccatgt gtgaggacca tgatcgcatc 660

cttttggcag gctgcccttc ctatgacaaa cttctctcag ccaagaacaa agactacatg 720

agcatcattc gcatgtggct aggtgatgat gtaaaatcta aagattacat tgttgcacta 780

cagcaccctg tgaccactga cattaagcat tccataaaaa tgtttgaatt aacattggat 840

gcacttatct catttaacaa gcggacccta gtcctgtttc caaatattga cgcagggagc 900

aaagagatgg ttcgagtgat gcggaagaag ggcattgagc atcatcccaa ctttcgtgca 960

gttaaacacg tcccatttga ccagtttata cagttggttg cccatgctgg ctgtatgatt 1020

gggaacagca gctgtggggt tcgagaagtt ggagcttttg gaacacctgt gatcaacctg 1080

ggaacacgtc agattggaag agaaacaggg gagaatgttc ttcatgtccg ggatgctgac 1140

acccaagaca aaatattgca agcactgcac cttcagtttg gtaaacagta cccttgttca 1200

aagatatatg gggatggaaa tgctgttcca aggattttga agtttctcaa atctatcgat 1260

cttcaagagc cactgcaaaa gaaattctgc tttcctcctg tgaaggagaa tatctctcaa 1320

gatattgacc atattcttga aactctaagt gccttggccg ttgatcttgg cgggacgaac 1380

ctccgagttg caatagtcag catgaagggt gaaatagtta agaagtatac tcagttcaat 1440

cctaaaacct atgaagagag gattaattta atcctacaga tgtgtgtgga agctgcagca 1500

gaagctgtaa aactgaactg cagaattttg ggagtaggca tttccacagg tggccgtgta 1560
aatcctcggg aaggaattgt gctgcattca accaaactga tccaagagtg gaactctgtg 1620
gaccttagga cccccctttc tgacactttg catctccctg tgtgggtaga caatgatggc 1680
aactgtgctg ccctggcgga aaggaaattt ggccaaggaa agggactgga aaactttgtt 1740
acacttatca caggcacagg aatcggtggt ggaattatcc atcagcatga attgatccac 1800
ggaagctcct tctgtgctgc agaactgggc caccttgttg tgtctctgga tgggcctgat 1860
tgttcctgtg gaagccatgg gtgcattgaa gcatacgcct ctggaatggc cttgcagagg 1920
gaggcaaaaa agctccatga tgaggacctg ctcttggtgg aagggatgtc agtgccaaaa 1980
gatgaggctg tgggtgcgct ccatctcatc caagctgcga aacttggcaa tgcgaaggcc 2040
cagagcatcc taagaacagc tggaacagct ttgggtcttg gggttgtgaa catcctccat 2100
accatgaatc cctcccttgt gatcctctcc ggagtcctgg ccagtcacta tatccacatt 2160
gtcaaagacg tcattcgcca gcaggccttg tcctccgtgc aggacgtgga tgtggtggtt 2220
tcggatttgg ttgaccccgc cctgctgggt gctgccagca tggttctgga ctacacaaca 2280
cgcaggatct actagacctc caggaacaga catggacctt ctctccagag ctcctgagtg 2340
gaatcaagtt cttgtcttta ggatgaccgt ttcttaacaa tcaaatctgg tattgaactg 2400
caggtgactt tggcagagaa atgttttcac ttttggtctc ctcttccaga gtcacctttc 2460
cccactccta tttttgtaga tgctattctt tctgatgtct tcttactagg ggtcatttta 2520
gctcaaaccc tgtaagttac agtcacaatt ttctgtgcca aagcagctac aataatagag 2580
aggaagcctt cttagaactc tgcttactaa tgtattaata ccactgagac cttcaggcct 2640
tcgctgggat atcacttcat cctgaagttt gcattaataa tccttccagg ccgggcacag 2700
tggctcacgc ctgtaatccc agcactttgg gaggccgagg cgggcggatc acgaggtcag 2760
gagatcgaga ccgccctggc taacatggtg aaacatggtg aaaccccgtc tctactaaaa 2820
atacaaaaaa ttagctgggt gtggtggcgg gtcccagcta ctcgggaggc tgaggcagga 2880
gaatggcatg aaccccaggc tggagtgcag tggctcaatg caacctctgc cccccaagtt 2940
caagtgattc tcctgcctca gcctcttgag tagctgggat tacaggcaca tgcaccacgc 3000
ccggctgatt tttttttgta tttttagtag acagggtttc accatgttgg ccaggctggt 3060
cttgaactcc tgacctcagg tgatccgccc gcctctgcct cccaaagtgc tgggattaca 3120
ggtgtaagcc atcacacctg gccctagtga caggttttta tgggtacttt tagatgatct 3180
aagaaatcat gtgcatatat ctttcagatt tctattttgg gaaaatgaag gtttctacaa 3240
catattgttt cagtgttcaa ataaactgaa ggactcaaca ttacatttga actatatcct 3300
tcctagtggg ttagtgtgaa aaagagtttg gctgattcct aaaactctgc cagccctgca 3360
gtaatctcca gggcctggtt attgttcaga cattccatgg tgattcctgg gaaggaagct 3420
tggctgctca gtttctgagt ctggggtgag ataatgttct gggaagggac atctgttctt 3480
tggtgtaatc tctcatggtg aaatctgctc tgtacatcag acaattgcat tgctaccaag 3540

tttcatacca aatatttgaa aggatggtat tgaatctaaa accaaatatt agtttttatt 3600

aaactcatgg gaaggctaat atattccaac gtaaattatt acatatggtt aagtaattgc 3660

atgttaattt attttaatgt aaatattttt gttactgttc tgagccaaat tctaaagaaa 3720

aaataaatac atttccttgt tgaaaaaaaa aaaaaaaaaa 3760

<210> 88
<211> 1228
<212> DNA
<213> Homo sapiens

<400> 88
acttcacgtg accgcgcggc ggccgctgcg gcgcgatggc ggcggggctg gcgcggctcc 60

tgttgctcct cgggctctcg gccggcgggc ccgcgccggc aggtgcagcg aagatgaagg 120

tggtggagga gcccaacgcg tttggggtga acaacccgtt cttgcctcag gccagtcgcc 180

tccaggccaa gagggatcct tcacccgtgt ctggacccgt gcatctcttc cgactctcgg 240

gcaagtgctt cagcctggtg gagtccacgt acaagtatga gttctgcccg ttccacaacg 300

tgacccagca cgagcagacc ttccgctgga acgcctacag tgggatcctc ggcatctggc 360

acgagtggga gatcgccaac aacaccttca cgggcatgtg gatgagggac ggtgacgcct 420

gccgttcccg gagccggcag agcaaggtgg agctggcgtg tggaaaaagc aaccggctgg 480

cccatgtgtc cgagccgagc acctgcgtct acgcgctgac gttcgagacc cccctcgtct 540

gccaccccca cgccttgcta gtgtacccaa ccctgccaga ggccctgcag cggcagtggg 600

accaggtaga gcaggacctg gccgatgagc tgatcacccc ccagggccat gagaagttgc 660

tgaggacact ttttgaggat gctggctact taaagacccc agaagaaaat gaacccaccc 720

agctggaggg aggtcctgac agcttggggt ttgagaccct ggaaaactgc aggaaggctc 780

ataaagaact ctcaaaggag atcaaaaggc tgaaaggttt gctcacccag cacggcatcc 840

cctacacgag gcccacagaa acttccaact tggagcactt gggccacgag acgcccagag 900

ccaagtctcc agagcagctg cggggtgacc caggactgcg tgggagtttg tgaccttgtg 960

gtgggagagc agaggtggac gcggccgaga gccctacaga gaagctggct ggtaggaccc 1020

gcagggacca gctgaccagg cttgtgctca gagaagcaga caaaacaaag attcaaggtt 1080

ttaattaatt cccatactga taaaaataac tccatgaatt ctgtaaacca ttgcataaat 1140

gctatagtgt aaaaaaattt aaacaagtgt taactttaaa cagttcgcta caagtaaatg 1200

attataaata ctaaaaaaaa aaaaaaaa 1228

<210> 89
<211> 2105
<212> DNA
<213> Homo sapiens

<400> 89
cggaagtgcg ggcgagcgcg ggtccccggg tctgacagga gcagcctgtg ggcaccgcgg 60

cggtagttgg aggcgggaga gggtccgtag ccgcgccgcc ctgccccgcc atgggcctcc 120

tgtcggaccc ggttcgccgg cgcgcgctcg cccgcctagt gctgcgcctc aacgcgccgt 180
tgtgcgtgct gagctacgtg gcgggcatcg cctggttctt ggcgctggtt ttcccgccgc 240
tgacccagcg cacttacatg tcggagaacg ccatgggctc caccatggtg gaggagcagt 300
ttgcgggcgg agaccgtgcc cgggcttttg cccgggactt cgccgcccac cgcaagaagt 360
cgggggctct gccagtggcc tggcttgaac ggacgatgcg gtcagtaggg ctggaggtct 420
acacgcagag tttctcccgg aaactgccct tcccagatga gacccacgag cgctatatgg 480
tgtcgggcac caacgtgtac ggcatcctgc gggccccgcg tgctgccagc accgagtcgc 540
ttgtgctcac cgtgccctgt ggctctgact ctaccaacag ccaggctgtg gggctgctgc 600
tggcactggc tgcccacttc cgggggcaga tttattgggc caaagatatc gtcttcctgg 660
taacagaaca tgaccttctg ggcactgagg cttggcttga agcctaccac gatgtcaatg 720
tcactggcat gcagtcgtct cccctgcagg gccgagctgg ggccattcag gcagccgtgg 780
ccctggagct gagcagtgat gtggtcacca gcctcgatgt ggccgtggag gggcttaacg 840
ggcagctgcc caaccttgac ctgctcaatc tcttccagac cttctgccag aaagggggcc 900
tgttgtgcac gcttcagggc aagctgcagc ccgaggactg gacatcattg gatggaccgc 960
tgcagggcct gcagacactg ctgctcatgg ttctgcggca ggcctccggc cgcccccacg 1020
gctcccatgg cctcttcctg cgctaccgtg tggaggccct aaccctgcgt ggcatcaata 1080
gcttccgcca gtacaagtat gacctggtgg cagtgggcaa ggctttggag ggcatgttcc 1140
gcaagctcaa ccacctcctg gagcgcctgc accagtcctt cttcctctac ttgctccccg 1200
gcctctcccg cttcgtctcc atcggcctct acatgcccgc tgtcggcttc ttgctcctgg 1260
tccttggtct caaggctctg gaactgtgga tgcagctgca tgaggctgga atgggccttg 1320
aggagcccgg gggtgcccct ggccccagtg taccccttcc cccatcacag ggtgtggggc 1380
tggcctcgct cgtggcacct ctgctgatct cacaggccat gggactggcc ctctatgtcc 1440
tgccagtgct gggccaacac gttgccaccc agcacttccc agtggcagag gctgaggctg 1500
tggtgctgac actgctggcg atttatgcag ctggcctggc cctgccccac aatacccacc 1560
gggtggtaag cacacaggcc ccagacaggg gctggatggc actgaagctg gtagccctga 1620
tctacctagc actgcagctg ggctgcatcg ccctcaccaa cttctcactg ggcttcctgc 1680
tggccaccac catggtgccc actgctgcgc ttgccaagcc tcatgggccc cggaccctct 1740
atgctgccct gctggtgctg accagcccgg cagccacgct ccttggcagc ctgttcctgt 1800
ggcgggagct gcaggaggcg ccactgtcac tggccgaggg ctggcagctc ttcctggcag 1860
cgctagccca gggtgtgctg gagcaccaca cctacggcgc cctgctcttc ccactgctgt 1920
ccctgggcct ctacccctgc tggctgcttt tctggaatgt gctcttctgg aagtgagatc 1980
tgcctgtccg ggctgggaca gagactcccc aaggacccca ttctgcctcc ttctggggaa 2040
ataaatgagt gtctgtttca gcagctaaaa aaaaaaaaaa aaaaaaaaaa aaaaaaaaaa 2100
aaaaa 2105

<210>  90
<211>  2091
<212>  DNA
<213>  Homo sapiens

<400>  90
gagcggagcc gcgggcggga gggcggacgg accgactgac ggtagggacg ggaggcgagc      60

aagatggcgc agacgcaggg cacccggagg aaagtctgtt actactacga cggggatgtt     120

ggaaattact attatggaca aggccaccca atgaagcctc accgaatccg catgactcat     180

aatttgctgc tcaactatgg tctctaccga aaaatggaaa tctatcgccc tcacaaagcc     240

aatgctgagg agatgaccaa gtaccacagc gatgactaca ttaaattctt gcgctccatc     300

cgtccagata acatgtcgga gtacagcaag cagatgcaga gattcaacgt tggtgaggac     360

tgtccagtat tcgatggcct gtttgagttc tgtcagttgt ctactggtgg ttctgtggca     420

agtgctgtga aacttaataa gcagcagacg gacatcgctg tgaattgggc tgggggcctg     480

caccatgcaa agaagtccga ggcatctggc ttctgttacg tcaatgatat cgtcttggcc     540

atcctggaac tgctaaagta tcaccagagg gtgctgtaca ttgacattga tattcaccat     600

ggtgacggcg tggaagaggc cttctacacc acggaccggg tcatgactgt gtcctttcat     660

aagtatggag agtacttccc aggaactggg gacctacggg atatcggggc tggcaaaggc     720

aagtattatg ctgttaacta cccgctccga gacgggattg atgacgagtc ctatgaggcc     780

attttcaagc cggtcatgtc caaagtaatg gagatgttcc agcctagtgc ggtggtctta     840

cagtgtggct cagactccct atctggggat cggttaggtt gcttcaatct aactatcaaa     900

ggacacgcca agtgtgtgga atttgtcaag agctttaacc tgcctatgct gatgctggga     960

ggcggtggtt acaccattcg taacgttgcc cggtgctgga catatgagac agctgtggcc    1020

ctggatacgg agatccctaa tgagcttcca tacaatgact actttgaata ctttggacca    1080

gatttcaagc tccacatcag tccttccaat atgactaacc agaacacgaa tgagtacctg    1140

gagaagatca aacagcgact gtttgagaac cttagaatgc tgccgcacgc acctggggtc    1200

caaatgcagg cgattcctga ggacgccatc cctgaggaga gtggcgatga ggacgaagac    1260

gaccctgaca agcgcatctc gatctgctcc tctgacaaac gaattgcctg tgaggaagag    1320

ttctccgatt ctgaagagga gggagagggg ggccgcaaga actcttccaa cttcaaaaaa    1380

gccaagagag tcaaaacaga ggatgaaaaa gagaaagacc cagaggagaa gaaagaagtc    1440

accgaagagg agaaaaccaa ggaggagaag ccagaagcca aaggggtcaa ggaggaggtc    1500

aagttggcct gaatggacct ctccagctct ggcttcctgc tgagtccctc acgtttcttc    1560

cccaacccct cagattttat attttctatt tctctgtgta tttatataaa aatttattaa    1620

atataaatat ccccagggac agaaaccaag gccccgagct cagggcagct gtgctgggtg    1680

agctcttcca ggagccacct tgccacccat tcttcccgtt cttaactttg aaccataaag    1740

ggtgccaggt ctgggtgaaa gggatacttt tatgcaacca taagacaaac tcctgaaatg    1800

ccaagtgcct gcttagtagc tttggaaagg tgcccttatt gaacattcta gaaggggtgg 1860

ctgggtcttc aaggatctcc tgtttttttc aggctcctaa agtaacatca gccattttta 1920

gattggttct gttttcgtac cttcccactg gcctcaagtg agccaagaaa cactgcctgc 1980

cctctgtctg tcttctccta attctgcagg tggaggttgc tagtctagtt tcctttttga 2040

gatactattt tcatttttgt gagcctcttt gtaataaaat ggtacatttc t 2091

<210> 91
<211> 1209
<212> DNA
<213> Homo sapiens

<400> 91
aggagcgttt ttggagaaag ctgcactctg ttgagctcca gggcgcagtg gagggaggga 60

gtgaaggagc tctctgtacc caaggaaagt gcagctgaga ctcagacaag attacaatga 120

accaactcag cttcctgctg tttctcatag cgaccaccag aggatggagt acagatgagg 180

ctaatactta cttcaaggaa tggacctgtt cttcgtctcc atctctgccc agaagctgca 240

aggaaatcaa agacgaatgt cctagtgcat ttgatggcct gtattttctc cgcactgaga 300

atggtgttat ctaccagacc ttctgtgaca tgacctctgg gggtggcggc tggaccctgg 360

tggccagcgt gcacgagaat gacatgcgtg ggaagtgcac ggtgggcgat cgctggtcca 420

gtcagcaggg cagcaaagca gtctacccag agggggacgg caactgggcc aactacaaca 480

cctttggatc tgcagaggcg gccacgagcg atgactacaa gaaccctggc tactacgaca 540

tccaggccaa ggacctgggc atctggcacg tgcccaataa gtcccccatg cagcactgga 600

gaaacagctc cctgctgagg taccgcacgg acactggctt cctccagaca ctgggacata 660

atctgtttgg catctaccag aaatatccag tgaaatatgg agaaggaaag tgttggactg 720

acaacggccc ggtgatccct gtggtctatg attttggcga cgcccagaaa acagcatctt 780

attactcacc ctatggccag cgggaattca ctgcgggatt tgttcagttc agggtattta 840

ataacgagag agcagccaac gccttgtgtg ctggaatgag ggtcaccgga tgtaacactg 900

agcaccactg cattggtgga ggaggatact ttccagaggc cagtccccag cagtgtggag 960

atttttctgg ttttgattgg agtggatatg gaactcatgt tggttacagc agcagccgtg 1020

agataactga ggcagctgtg cttctattct atcgttgaga gttttgtggg agggaaccca 1080

gacctctcct cccaaccatg agatcccaag gatggagaac aacttaccca gtagctagaa 1140

tgttaatggc agaagagaaa acaataaatc atattgactc aaaaaaaaaa aaaaaaaaaa 1200

aaaaaaaaa 1209

<210> 92
<211> 1150
<212> DNA
<213> Homo sapiens

<400> 92
gcaggggagc tccgagtgtc cacaggaagg gaactatcag ctcctggcat ctgtaaggat 60

gctgtccatg ctgaggacaa tgaccagact ctgcttcctg ttattcttct ctgtggccac 120

cagtgggtgc agtgcagcag cagcctcttc tcttgagatg ctctcgaggg aattcgaaac 180

ctgtgccttc tccttttctt ccctgcctag aagctgcaaa gaaatcaagg aacgctgcca 240

tagtgcaggt gatggcctgt attttctccg caccaagaat ggtgttgtct accagacctt 300

ctgtgacatg acttctgggg gtggcggctg gaccctggtg gccagcgtgc acgagaatga 360

catgcgtggg aagtgcacgg tgggtgatcg ctggtccagt cagcagggca acaaagcaga 420

ctacccagag ggggatggca actgggccaa ctacaacacc tttggatctg cagaggcggc 480

cacgagcgat gactacaaga accctggcta ctacgacatc caggccaagg acctgggcat 540

ctggcatgtg cccaacaagt cccccatgca gcattggaga aacagcgccc tgctgaggta 600

ccgcaccaac actggcttcc tccagagact gggacataat ctgtttggca tctaccagaa 660

atacccagtg aaatacagat cagggaaatg ttggaatgac aatggcccag ccatacctgt 720

ggtctatgac tttggtgatg ctaagaagac tgcatcttat tactcaccgt atggtcaacg 780

ggaatttgtt gcaggattcg ttcagttccg ggtgtttaat aacgagagag cagccaacgc 840

cctttgtgct gggataaaag ttactggctg taacactgag catcactgca tcggtggagg 900

agggttcttc ccacagggca aaccccgtca gtgtggggac ttctccgcct ttgactggga 960

tggatatgga actcacgtta agagcagctg cagtcgggag ataacggagg cggctgtact 1020

cttgttctat agatgagaca gagctctgcg gtgtcagggc gagaacccat cttccaaccc 1080

cggctatttg gagacggaaa aactggaatt ctaacaagga ggagaggaga ctaaatcaca 1140

tcaatttgca 1150

<210> 93
<211> 1575
<212> DNA
<213> Homo sapiens

<400> 93
   ctcttcccgg ctccagctcc gccgccagct ccagcctttg ctccccctcc caaagtcccc 60
   tccccggagc ggagcgcacc tagggtccct cttccgtccc cccagcccag ctacccgttc 120
   agaccagcag cctcgggggg cacccccccg ccagcctgcc tccctcccgc tcagccctgc 180
   cagggttccc cagccatgaa tctcttccga ttcctgggag acctctccca cctcctcgcc 240
   atcatcttgc tactgctcaa aatctggaag tcccgctcgt gcgccggaat ttcagggaag 300
   agccaggtcc tgtttgctgt ggtgttcact gcccgatatc tggacctctt caccaactac 360
   atctcactct acaacacgtg tatgaaggtg gtctacatag cctgctcctt caccacggtc 420
   tggttgattt atagcaagtt caaagctact tacgatggga accatgacac gttcagagtg 480
   gagttcctgg tcgttcccac agccattctg gcgttcctgg tcaatcatga cttcacccct 540
   ctggagatcc tctggacctt ctccatctac ctggagtcag tggccatctt gccgcagctg 600
   ttcatggtga gcaagaccgg cgaggcggag accatcacca gccactactt gtttgcgcta 660
   ggcgtttacc gcacgctcta tctcttcaac tggatctggc gctaccattt cgagggcttc 720
   ttcgacctca tcgccattgt ggcaggcctg gtccagacag tcctctactg cgatttcttc 780
   tacctctata tcaccaaagt cctaaagggg aagaagttga gtttgccggc atagccccgg 840
   tcctctccat ctctctcctc ggcagcagcg ggaggcagag gaaggcggca gaagatgaag 900
   agctttccca tccaggggtg acttttttaa gaacccacct cttgtgctcc ccatcccgcc 960
   tcctgccggg tttcaggggg acagtggagg atccaggtct tggggagctc aggacttggg 1020
   ctgtttgtag ttttttgcct tttagacaag aaaaaaaaat ctttccactc tttagttttt 1080
   gattctgatg actcgttttt cttctactct gtggccccaa tttttataaa gtgtttttga 1140
   gtgtcctatg ggccggggca gggtccaaga tcttttccct tccccaggcc cctcggctcc 1200
   ctcccagatc ccacccccag ccccactggt tgccaaacac taaatctgcc gacacccatc 1260
   tgccccacct cctgccatgg ccatgaaccg cgacccccac taaatttcta gattggggat 1320
   agggagaaag ggaggcccag gaaggtctcc cctgattttt tttcatagta atttttttcc 1380
   ccagagtttg aattttttgg tcttctcctg gttttttggc aaattagggg ggcccggggc 1440
   tcaagtgcgg gaagggggct ggcccgagga tcccatggct ctcacaccat gtttttgtac 1500
   agaactgatg gttgaatctt tgttctcttg aaataaacag aagaaaatga aacctttaaa 1560
   aaaaaaaaaa aaaaa 1575

<210> 94
<211> 1153
<212> DNA
<213> Homo sapiens

<400> 94
gccgccgccg ccatgaacat tttccggctg actggggacc tgtcccacct ggcggccatc 60

gtcatcctgc tgctgaagat ctggaagacg cgctcctgcg ccggtatttc tgggaaaagc 120

cagcttctgt ttgcactggt cttcacaact cgttacctgg atctttttac ttcatttatt 180

tcattgtata acacatctat gaaggttatc taccttgcct gctcctatgc cacagtgtac 240

ctgatctacc tgaaatttaa ggcaacctac gatggaaatc atgatacctt ccgagtggag 300

tttctggtgg tccctgtggg aggcctctca tttttagtta atcacgattt ctctcctctt 360

gagatcctct ggaccttctc catctacctg gagtccgtgg ctatccttcc gcagctgttt 420

atgatcagca agactgggga ggccgagacc atcaccaccc actacctgtt cttcctgggc 480

ctctatcgtg ctttgtatct tgtcaactgg atctggcgct tctactttga gggcttcttt 540

gacctcattg ctgtggtggc cggcgtagtc cagaccatcc tatactgtga cttcttctac 600

ttgtacatta caaaagtact caagggaaag aagctcagtt tgccagcata agtgccaaag 660

accatcacca gcatctgtcc ttcagggtgc tcggacagaa ttcttaccac agcaaaggca 720

taagatgctt gatacggaaa atcagaaact taactctttt gttgcagata gtcatcagtg 780

gctctgtaaa aacgcagagg aaaagagcca gaaggtttct gtttaatgca tcttgcctta 840

tcttttttta ttactgtgta caaagatttt tttacacaaa gaaacttaat gctgtattaa 900

taaattcagt gtgtagcttc aattgggata gttccaaaag tgaagatttt gtgaggaata 960
agtgcaaatt tttttttat tttaaaaaat tctttgaaac tcttaagtct ttgtgtctgc 1020
aatgaaattg tactccttga cagttgatag attatgtatt cttccatccc tcaaacttgc 1080
attccactat atttattttt tggcaaaaga tgagctgtat ttgtttgaaa tctgagacac 1140
tatgttcaat tgg 1153

<210> 95
<211> 2377
<212> DNA
<213> Homo sapiens

<400> 95
gaaccctgaa gtcggtgtct gctgcgttca cggcaggatt cggttaggag gaacagcaca 60
gcatgctggg ctctggattt aaagctgagc gcttaagagt gaatttgaga ttagtcataa 120
atcgccttaa actattggag aaaaagaaaa cggaactggc ccagaaagca aggaaggaga 180
ttgctgacta tctggctgct gggaaagatg aacgagctcg gatccgtgtg gagcacatta 240
tccgggaaga ctacctcgtg gaggccatgg agatcctgga gctgtactgt gacctgctgc 300
tggctcggtt tggccttatc cagtctatga aggaactaga ttctggtctg gctgaatctg 360
tgtctacatt gatctgggct gctcctcgac tccagtcaga agtggctgag ttgaaaatag 420
ttgctgatca gctctgtgcc aagtatagca aggaatatgg caagctatgt aggaccaacc 480
agattggaac tgtgaatgac aggctaatgc acaagctgag tgtggaagcc ccacccaaaa 540
tcctggtgga gagatacctg attgaaattg caaagaatta caacgtaccc tatgaacctg 600
actctgtggt catggcagaa gctcctcctg gggtagagac agatcttatt gatgttggat 660
tcacagatga tgtgaagaaa ggaggccctg gaagaggagg gagtggtggc ttcacagcac 720
cagttggtgg acctgatgga acggtgccaa tgcccatgcc catgcccatg cctatgccat 780
ctgcaaatac gcctttctca tatccactgc caaagggacc atcagatttc aatggactgc 840
caatggggac ttatcaggcc tttcccaata ttcatccacc tcagatacca gcaactcccc 900
catcgtatga atctatgaca ttaatgctga taagaatatc tcttctgcac agattgttgg 960
tcctggaccc aagccagaag cctctgcaaa gcttccttcc agacctgcag ataactatga 1020
caactttgtc ctaccagagt tgccatctgt gccagacaca ctaccaactg catctgctgg 1080
tgccagcacc tcagcatctg aagacattga ctttgatgat ctttcccgga ggtttgaaga 1140
gctgaaaaag aaaacatagg tctcttaaac caggcaactt tcacgttttg ggagttgaga 1200
ctgagcaatt tctccttgta acaaagaatc tccatgaaat tctgtttcat ctgttaaccg 1260
tcactcagca caacactccc tctgggctct cttcctgctc ctccagattc tgctgctttc 1320
cagttctctg ttgatcctga gactaacaat tggagactga ggccagagca actggctcct 1380
ggcagctgtg cttgtccgtt tcctgtcaga gtgatcccag gtttcctcct ggcccgtccc 1440
atggtccctc cacaggagtg tgagaggatg ggggaagcac tgtgggaaga ccaccaaaga 1500
tggctggaca gtgggagaga gcacgttgtg aagcatccca gcctcgtgtt gaggttccag 1560

acttagaaac agacccctct gtacaggggg attgtggtga gtgagaatca aggccacctt 1620

gtgtgttttc tcactctcga atgcaagtgg gagagggaaa atgactcggg acgccattgt 1680

aacggttcct ggaagctggg ccctctcatt ggcatataca gtactcctcg ctgcagggca 1740

ctgtcccacc gggatccagt tgcaaagttt gtcttgacag ttgaaggcct cgcttagttg 1800

tactggattc tcagggagcc ctctgtggcc ttttgctttg cgtgctgttt cccttgtacc 1860

agagggcggc accgtggaaa ttctgttttc cctgtagcat attgtgttgg attgcattac 1920

tggcagagaa aggacaaggt gccattcaag tcctagggtg ggcttccagc tgccttaata 1980

gaagtactca agtcttttgg gtagtgagct ggaaagccta caggaaaaga ggggtacctg 2040

ttttcatttg aaaactttga ttcatggaac ctttaaaact aatctcagaa aaatttttgg 2100

tgcccatgca gctgtagttg ttcactgctt tcctggatgg atgggactct tatgtcataa 2160

cttctgttac tcctttggcc catagctaag gtcatccttc cccacagggg tggctttggg 2220

attggatgat acagcttttg cttctgtgta gtatacctgt acatacttgt ttcaggcagc 2280

ctttctttaa tgttttcagt tggtttgtat tctgtagctc agtagctgct aataaagtta 2340

aagatcctga aaaaaaaaaa aaaaaaaaaa aaaaaaa 2377

<210> 96
<211> 586
<212> DNA
<213> Homo sapiens

<400> 96
agttaaaagg gtgggagcgt ccgggggccc atctctctcg ggtggagtct tctgacagct 60

ggtgcgcctg cccgggaaca tcctcctgga ctcaatcatg gcttgtggtc tggtcgccag 120

caacctgaat ctcaaacctg gagagtgcct tcgagtgcga ggcgaggtgg ctcctgacgc 180

taagagcttc gtgctgaacc tgggcaaaga cagcaacaac ctgtgcctgc acttcaaccc 240

tcgcttcaac gcccacggcg acgccaacac catcgtgtgc aacagcaagg acggcggggc 300

ctgggggacc gagcagcggg aggctgtctt tcccttccag cctggaagtg ttgcagaggt 360

gtgcatcacc ttcgaccagg ccaacctgac cgtcaagctg ccagatggat acgaattcaa 420

gttccccaac cgcctcaacc tggaggccat caactacatg gcagctgacg gtgacttcaa 480

gatcaaatgt gtggcctttg actgaaatca gccagcccat ggcccccaat aaaggcagct 540

gcctctgctc cctctgaaaa aaaaaaaaaa aaaaaaaaaa aaaaaa 586

<210> 97
<211> 1696
<212> DNA
<213> Homo sapiens

<400> 97
acaaaccctc gttggcccca cagggagcca gcctctggct tctctctgca atggccatgt 60

gctgcagacc cggagtgggt agttagttgg ttaatgccag tcttcctccc ctggacactg 120

agttctgctg acagcccccg cccagccaga gctctgctgt ataccaccgg gagtggggct 180

ggtgtggagc ctggaggtcg cccgctgccc tcctagggct gctccagaca gcattaaaac 240

gctgcaggtc gcaggtgaga ctaacagctg ggagagctgc tccaggcatt taggaccctg 300

actggggcag atgagtcagc ccagtggggg cagggctcct ggaacgagga tctacagttg 360

gagttgcccc actgtcatgt cacctggaga aaaactggac ccaattcctg acagcttcat 420

tctgcaacca ccagtcttcc acccggtggt tccttatgtc acgacgattt ttggaggcct 480

gcatgcaggc aagatggtca tgctgcaagg agtggtccct ctagatgcac acaggtttca 540

ggtggacttc cagtgtggct gcagcctgtg tccccggcca gatatcgcct tccacttcaa 600

ccctcgcttc cataccacca agccccatgt catctgcaac accctgcatg gtggacgctg 660

gcaaagggag gcccggtggc cccacctggc cctgcgaaga ggctccagct tcctcatcct 720

ctttctcttc gggaatgagg aagtgaaggt gagtgtgaat ggacagcact ttctccactt 780

ccgctaccgg ctcccactgt ctcatgtgga cacgctgggt atatttggtg acatcctggt 840

agaggctgtt ggattcctga acatcaatcc atttgtggag ggcagcagag agtacccagc 900

tggacatcct ttcctgctga tgagccccag gctggaggtg ccctgctcac atgctcttcc 960

ccagggtctc tcgcctgggc aggtcatcat agtacgggga ctggtcttgc aagagccgaa 1020

gcattttact gtgagcctga gggaccaggc tgcccatgct cctgtgacac tcagggcctc 1080

cttcgcagac agaactctgg cctggatctc ccgctggggg cagaagaaac tgatctcagc 1140

ccccttcctc ttttaccccc agagattctt tgaggtgctg ctcctgttcc aggagggagg 1200

gctgaagctg gcgctcaatg ggcaggggct gggggccacc agcatgaacc agcaggccct 1260

ggagcagctg cgggagctcc ggatcagtgg aagtgtccag ctctactgtg tccactcctg 1320

aggatggttc caggggaaat accgccagaa aacaagaagg tcagcccact cccagggccc 1380

cactctcctc ccctcattaa accatccacc tgacaccagc acatcaggcc tggttcacct 1440

ctggggtcac gagactgagt ctacaggagc tttgggcctg agggaaggca caagagtgca 1500

aaggttcctc gaactctgca ccttcctcca ccaggagcct gggatatggc tccatctgcc 1560

ttcagggcct ggactgcact cacagaggca agtgttgtag actaacaaag atactccaaa 1620

atacaatggc ttaaagaatg tggtcattta ttctttatta tttatttatt tgtggtcaaa 1680

taaataaata aggtta 1696

<210> 98
<211> 610
<212> DNA
<213> Homo sapiens

<400> 98
actggactca attctgaagg tcgccaagaa ggagagaaca atgtcttctt tacccgtgcc 60

atacaaactg cctgtgtctt tgtctgttgg ttcctgcgtg ataatcaaag ggacaccaat 120

ccactctttt atcaatgacc cacagctgca ggtggatttc tacactgaca tggatgagga 180

ttcagatatt gccttccgtt tccgagtgca ctttggcaat catgtggtca tgaacaggcg 240

tgagtttggg atatggatgt tggaggagac aacagactac gtgccctttg aggatggcaa 300

acaatttgag ctgtgcatct acgtacatta caatgagtat gagataaagg tcaatggcat 360

acgcatttac ggctttgtcc atcgaatccc gccatcattt gtgaagatgg tgcaagtgtc 420

gagagatatc tccctgacct cagtgtgtgt ctgcaattga gggagatgat cacactcctc 480

attgttgagg aatccctctt tctacctgac catgggattc ccagaacctg ctaacagaat 540

aatccctgct cacattttcc cctacacttt gtcattaaaa cagcacgaaa actcaaaaaa 600

aaaaaaaaaa 610

<210> 99
<211> 794
<212> DNA
<213> Homo sapiens

<400> 99
gctgcattac agacacagac ctgcaaacat ctatggttgt gacagagttt ctttctgaca 60

cctgagtctt tctcctgctg cacggaaagc ttgctgggag gggcttggaa tctggcatga 120

agccaaaggg catctctgag ttgcagcatt taaatgatcc cactcagaga ttcacacaga 180

agactggaca caattccgaa gagctgccca gaaggagaga acaatgtcat cactacccgt 240

accatacaca ctgcctgttt ccttgcctgt tggttcgtgc gtgataatca cagggacacc 300

gatcctcact tttgtcaagg acccacagct ggaggtgaat ttctacactg ggatggatga 360

ggactcagat attgctttcc aattccgact gcactttggt catcctgcaa tcatgaacag 420

ttgtgtgttt ggcatatgga gatatgagga gaaatgctac tatttaccct ttgaagatgg 480

caaaccattt gagctgtgca tctatgtgcg tcacaaggaa tacaaggtaa tggtaaatgg 540

ccaacgcatt tacaactttg cccatcgatt cccgccagca tctgtgaaga tgctgcaagt 600

cttcagagat atctccctga ccagagtgct tatcagcgat tgagggagat gatcagactc 660

ctcattgttg aggaatccct ctttctacct gaccatggga ttcccagagc ctactaacag 720

aataatccct cctcacccct tcccctacac ttgatcatta aaacagcacc aaacttcaaa 780

aaaaaaaaaa aaaa 794

<210> 100
<211> 543
<212> DNA
<213> Homo sapiens

<400> 100
gaccttgagg gagttaatgt gtaatattct aggatataag cttgaccacg agttgagacc 60

ctgagcacag gcctccagga gccgctggga gctgccgcca ggagctgtca ccatgacggg 120

ggaacttgag gttaagaaca tggacatgaa gccggggtca accctgaaga tcacaggcag 180

catcgccgat ggcactgatg gctttgtaat taatctgggc caggggacag acaagctgaa 240

cctgcatttc aaccctcgct tcagcgaatc caccattgtc tgcaactcat tggacggcag 300

caactggggg caagaacaac gggaagatca cctgtgcttc agcccagggt cagaggtcaa 360

gttcacagtg acctttgaga gtgacaaatt caaggtgaag ctgccagatg ggcacgagct 420

gacttttccc aacaggctgg gtcacagcca cctgagctac ctgagcgtaa ggggcgggtt 480

caacatgtcc tctttcaagt taaaagaata aaagacttcc agccgagaaa aaaaaaaaaa 540

aaa 543

<210> 101
<211> 1080
<212> DNA
<213> Homo sapiens

<400> 101
ggagaggact ggctgggcag gggcgccgcc ccgcctcggg agaggcgggc cgggcggggc 60

tgggagtatt tgaggctcgg agccaccgcc ccgccggcgc ccgcagcacc tcctcgccag 120

cagccgtccg gagccagcca acgagcggaa aatggcagac aatttttcgc tccatgatgc 180

gttatctggg tctggaaacc caaaccctca aggatggcct ggcgcatggg ggaaccagcc 240

tgctggggca gggggctacc caggggcttc ctatcctggg gcctaccccg ggcaggcacc 300

cccaggggct tatcctggac aggcacctcc aggcgcctac cctggagcac ctggagctta 360

tcccggagca cctgcacctg gagtctaccc agggccaccc agcggccctg gggcctaccc 420

atcttctgga cagccaagtg ccaccggagc ctaccctgcc actggcccct atggcgcccc 480

tgctgggcca ctgattgtgc cttataacct gcctttgcct gggggagtgg tgcctcgcat 540

gctgataaca attctgggca cggtgaagcc caatgcaaac agaattgctt tagatttcca 600

aagagggaat gatgttgcct tccactttaa cccacgcttc aatgagaaca acaggagagt 660

cattgtttgc aatacaaagc tggataataa ctggggaagg gaagaaagac agtcggtttt 720

cccatttgaa agtgggaaac cattcaaaat acaagtactg gttgaacctg accacttcaa 780

ggttgcagtg aatgatgctc acttgttgca gtacaatcat cgggttaaaa aactcaatga 840

aatcagcaaa ctgggaattt ctggtgacat agacctcacc agtgcttcat ataccatgat 900

ataatctgaa aggggcagat taaaaaaaaa aaaagaatct aaaccttaca tgtgtaaagg 960

tttcatgttc actgtgagtg aaaattttta cattcatcaa tatccctctt gtaagtcatc 1020

tacttaataa atattacagt gaattacctg tctcaatatg tcaaaaaaaa aaaaaaaaaa 1080

<210> 102
<211> 2254
<212> DNA
<213> Homo sapiens

<400> 102
aatcgaaagt agactctttt ctgaagcatt tcctgggatc agcctgacca cgctccatac 60

tgggagaggc ttctgggtca aaggaccagt ctgcagaggg atcctgtggc tggaagcgag 120

gaggctccac acggccgttg cagctaccgc agccaggatc tgggcatcca ggcacggcca 180

tgacccctcc gaggctcttc tgggtgtggc tgctggttgc aggaacccaa ggcgtgaacg 240

atggtgacat gcggctggcc gatgggggcg ccaccaacca gggccgcgtg gagatcttct 300

acagaggcca gtggggcact gtgtgtgaca acctgtggga cctgactgat gccagcgtcg 360
tctgccgggc cctgggcttc gagaacgcca cccaggctct gggcagagct gccttcgggc 420
aaggatcagg ccccatcatg ctggacgagg tccagtgcac gggaaccgag gcctcactgg 480
ccgactgcaa gtccctgggc tggctgaaga gcaactgcag gcacgagaga gacgctggtg 540
tggtctgcac caatgaaacc aggagcaccc acaccctgga cctctccagg gagctctcgg 600
aggcccttgg ccagatcttt gacagccagc ggggctgcga cctgtccatc agcgtgaatg 660
tgcagggcga ggacgccctg ggcttctgtg gccacacggt catcctgact gccaacctgg 720
aggcccaggc cctgtggaag gagccgggca gcaatgtcac catgagtgtg gatgctgagt 780
gtgtgcccat ggtcagggac cttctcaggt acttctactc ccgaaggatt gacatcaccc 840
tgtcgtcagt caagtgcttc cacaagctgg cctctgccta tggggccagg cagctgcagg 900
gctactgcgc aagcctcttt gccatcctcc tcccccagga cccctcgttc cagatgcccc 960
tggacctgta tgcctatgca gtggccacag gggacgccct gctggagaag ctctgcctac 1020
agttcctggc ctggaacttc gaggccttga cgcaggccga ggcctggccc agtgtcccca 1080
cagacctgct ccaactgctg ctgcccagga gcgacctggc ggtgcccagc gagctggccc 1140
tactgaaggc cgtggacacc tggagctggg gggagcgtgc ctcccatgag gaggtggagg 1200
gcttggtgga gaagatccgc ttccccatga tgctccctga ggagctcttt gagctgcagt 1260
tcaacctgtc cctgtactgg agccacgagg ccctgttcca gaagaagact ctgcaggccc 1320
tggaattcca cactgtgccc ttccagttgc tggcccggta caaaggcctg aacctcaccg 1380
aggataccta caagccccgg atttacacct cgcccacctg gagtgccttt gtgacagaca 1440
gttcctggag tgcacggaag tcacaactgg tctatcagtc cagacggggg cctttggtca 1500
aatattcttc tgattacttc caagccccct ctgactacag atactacccc taccagtcct 1560
tccagactcc acaacacccc agcttcctct tccaggacaa gagggtgtcc tggtccctgg 1620
tctacctccc caccatccag agctgctgga actacggctt ctcctgctcc tcggacgagc 1680
tccctgtcct gggcctcacc aagtctggcg gctcagatcg caccattgcc tacgaaaaca 1740
aagccctgat gctctgcgaa gggctcttcg tggcagacgt caccgatttc gagggctgga 1800
aggctgcgat tcccagtgcc ctggacacca acagctcgaa gagcacctcc tccttcccct 1860
gcccggcagg gcacttcaac ggcttccgca cggtcatccg ccccttctac ctgaccaact 1920
cctcaggtgt ggactagacg cgtggccaag ggtggtgaga accggagaac cccaggacgc 1980
cctcactgca ggctcccctc ctcggcttcc ttcctctctg caatgacctt caacaaccgg 2040
ccaccagatg tcgccctact cacctgaggc tcagcttcaa gaaattactg gaaggcttcc 2100
actagggtcc accaggagtt ctcccaccac ctcaccagtt tccaggtggt aagcaccagg 2160
aggccctcga ggttgctctg gatcccccca cagcccctgg tcagtctgcc cttgtcactg 2220
gtctgaggtc attaaaatta cattgaggtt ccta 2254

<210> 103

<211>  1117
<212>  DNA
<213>  Homo sapiens

<400>  103
atctcccact cctgcagctc ttctcacagg accagccact agcgcagcct cgagcgatgg      60

cctatgtccc cgcaccgggc taccagccca cctacaaccc gacgctgcct tactaccagc     120

ccatcccggg cgggctcaac gtgggaatgt ctgtttacat ccaaggagtg gccagcgagc     180

acatgaagcg gttcttcgtg aactttgtgg ttgggcagga tccgggctca gacgtcgcct     240

tccacttcaa tccgcggttt gacggctggg acaaggtggt cttcaacacg ttgcagggcg     300

ggaagtgggg cagcgaggag aggaagagga gcatgccctt caaaaagggt gccgcctttg     360

agctggtctt catagtcctg gctgagcact acaaggtggt ggtaaatgga aatcccttct     420

atgagtacgg gcaccggctt cccctacaga tggtcaccca cctgcaagtg gatggggatc     480

tgcaacttca atcaatcaac ttcatcggag gccagcccct ccggccccag ggacccccga     540

tgatgccacc ttaccctggt cccggacatt gccatcaaca gctgaacagc ctgcccacca     600

tggaaggacc cccaaccttc aacccgcctg tgccatattt cgggaggctg caaggagggc     660

tcacagctcg aagaaccatc atcatcaagg gctatgtgcc tcccacaggc aagagctttg     720

ctatcaactt caaggtgggc tcctcagggg acatagctct gcacattaat ccccgcatgg     780

gcaacggtac cgtggtccgg aacagccttc tgaatggctc gtggggatcc gaggagaaga     840

agatcaccca caacccattt ggtcccggac agttctttga tctgtccatt cgctgtggct     900

tggatcgctt caaggtttac gccaatggcc agcacctctt tgactttgcc catcgcctct     960

cggccttcca gagggtggac acattggaaa tccagggtga tgtcaccttg tcctatgtcc    1020

agatctaatc tattcctggg gccataactc atgggaaaac agaattatcc cctaggactc    1080

ctttctaagc ccctaataaa atgtctgagg gtgtctc                             1117

<210>  104
<211>  498
<212>  DNA
<213>  Homo sapiens

<400>  104
ttaaagcaaa gaattccccg gtcccagcca tgtccaacgt cccccacaag tcctcgctgc      60

ccgagggcat ccgccctggc acggtgctga gaattcgcgg cttggttcct cccaatgcca     120

gcaggttcca tgtaaacctg ctgtgcgggg aggagcaggg ctccgatgcc gccctgcatt     180

tcaacccccg gctggacacg tcggaggtgg tcttcaacag caaggagcaa ggctcctggg     240

gccgcgagga gcgcgggccg ggcgttcctt tccagcgcgg gcagcccttc gaggtgctca     300

tcatcgcgtc agacgacggc ttcaaggccg tggttgggga cgcccagtac caccacttcc     360

gccaccgcct gccgctggcg cgcgtgcgcc tggtggaggt gggcggggac gtgcagctgg     420

actccgtgag gatcttctga gcagaagccc aggcggcccg gggccttggc tggcaaataa     480

agcgttagcc cgcagcgc                                                   498

<210> 105
<211> 2996
<212> DNA
<213> Homo sapiens

<400> 105
tggacttgga tccgaggcag acgaggaagc tgagaaaacc ctggcgttga ccccgtggac 60

ctgggcgccc cgggaaggcc agcgcttggt ccaggcaggc ggggcctgtg cggtgaccac 120

cctggtcctg aaaagtccca gccccgagcg ccctccctcc tagacctgga ggcctggaac 180

agccagccgc ccacggacgc cagagccggg aaccctgacg gcacttagct gctgacaaac 240

aacctgctcc gtggagcgcc tgaaacacca gtctttgggg ccagtgcctc agtttcaatc 300

caggtaacct ttaaatgaaa cttgcctaaa atcttaggtc atacacagaa gagactccaa 360

tcgacaagaa gctggaaaag aatgatgttg tccttaaaca acctacagaa tatcatctat 420

aacccggtaa tcccgtttgt tggcaccatt cctgatcagc tggatcctgg aactttgatt 480

gtgatacgtg ggcatgttcc tagtgacgca gacagattcc aggtggatct gcagaatggc 540

agcagcatga aacctcgagc cgatgtggcc tttcatttca atcctcgttt caaaagggcc 600

ggctgcattg tttgcaatac tttgataaat gaaaaatggg gacgggaaga gatcacctat 660

gacacgcctt tcaaaagaga aaagtctttt gagatcgtga ttatggtgct gaaggacaaa 720

ttccaggtgg ctgtaaatgg aaaacatact ctgctctatg gccacaggat cggcccagag 780

aaaatagaca ctctgggcat ttatggcaaa gtgaatattc actcaattgg ttttagcttc 840

agctcggact tacaaagtac ccaagcatct agtctggaac tgacagagat aagtagagaa 900

aatgttccaa agtctggcac gccccagctt cctagtaata gaggaggaga catttctaaa 960

atcgcaccca gaactgtcta caccaagagc aaagattcga ctgtcaatca cactttgact 1020

tgcaccaaaa taccacctat gaactatgtg tcaaagaggc tgccattcgc tgcaaggttg 1080

aacaccccca tgggccctgg acgaactgtc gtcgttaaag gagaagtgaa tgcaaatgcc 1140

aaaagcttta atgttgacct actagcagga aaatcaaagg atattgctct acacttgaac 1200

ccacgcctga atattaaagc atttgtaaga aattcttttc ttcaggagtc ctggggagaa 1260

gaagagagaa atattacctc tttcccattt agtcctggga tgtactttga gatgataatt 1320

tattgtgatg ttagagaatt caaggttgca gtaaatggcg tacacagcct ggagtacaaa 1380

cacagattta aagagctcag cagtattgac acgctggaaa ttaatggaga catccactta 1440

ctggaagtaa ggagctggta gcctacctac acagctgcta caaaaaccaa aatacagaat 1500

ggcttctgtg atactggcct tgctgaaacg catctcactg tcattctatt gtttatattg 1560

ttaaaatgag cttgtgcacc attaggtcct gctgggtgtt ctcagtcctt gccatgaagt 1620

atggtggtgt ctagcactga atggggaaac tgggggcagc aacacttata gccagttaaa 1680

gccactctgc cctctctcct actttggctg actcttcaag aatgccattc aacaagtatt 1740

tatggagtac ctactataat acagtagcta acatgtattg agcacagatt ttttttggta 1800

aaactgtgaa gagctaggat atatacttgg tgaaacaaac cagtatgttc cctgttctct 1860

tgagcttcga ctcttctgtg cgctactgct gcgcactgct ttttctacag gcattacatc 1920

aactcctaag gggtcctctg ggattagtta tgcagatatt aaatcacccg aagacactaa 1980

cttacagaag acacaactcc ttccccagtg atcactgtca taaccagtgc tctaccgtat 2040

cccatcactg aggactgatg ttgactgaca tcattttctt tatcgtaata aacatgtggc 2100

tctattagct gcaagcttta ccaagtaatt ggcatgacat ctgagcacag aaattaaggc 2160

aaaaaaccaa agcaaaacaa atacatggtg ctgaaattaa cttgatgcca agcccaaggc 2220

agctgatttc tgtgtatttg aacttagggc aaatcagagt ctacacagac gcctacagaa 2280

agtttcagga agaggcaaga tgcattcaat ttgaaagata tttatgggca acaaagtaag 2340

gtcaggatta gacttcaggc attcataagg caggcactat cagaaagtgt acgccaacta 2400

agggacccac aaagcaggca gaggtaatgc agaaatctgt tttgttccca tgaaatcacc 2460

aatcaaggcc tccgttcttc taaagattag tccatcatca ttagcaactg agatcaaagc 2520

actcttccac tttacgtgat taaaatcaaa cctgtatcag caagttaaat ggttccattt 2580

ctgtgatttt tctattattt gaggggagtt ggcagaagtt ccatgtatat gggatcttta 2640

caggtcagat cttgttacag gaaatttcaa aggtttggga gtggggaggg aaaaaagctc 2700

agtcagtgag gatcatttta tcacattaga ctggggcaga actctgccag gatttaggaa 2760

tattttcaga acagatttta gatattattt ctatccatat attgaaaaga ataccattgt 2820

caatcttatt tttttaaaag tactcagtgt agaaatcgct agcccttaat tcttttccag 2880

cttttcatat taatgtatgc agagtctcac caagctcaaa gacactggtt gggggtggag 2940

ggtgccacag ggaaagctgt agaaggcaag aagactcgag aatcccccag agttat 2996

<210> 106
<211> 1739
<212> DNA
<213> Homo sapiens

<400> 106
tccccattga ataacagcca agttgctttg gtttctattt ctttgttaag tcgttccctc 60

tacaaaggac ttcctagtgg gtgtgaaagg cagcggtggc cacagaggcg gcggagagat 120

ggccttcagc ggttcccagg ctccctacct gagtccagct gtcccctttt ctgggactat 180

tcaaggaggt ctccaggacg gacttcagat cactgtcaat gggaccgttc tcagctccag 240

tggaaccagg tttgctgtga actttcagac tggcttcagt ggaaatgaca ttgccttcca 300

cttcaaccct cggtttgaag atggagggta cgtggtgtgc aacacgaggc agaacggaag 360

ctgggggccc gaggagagga agacacacat gcctttccag aaggggatgc cctttgacct 420

ctgcttcctg gtgcagagct cagatttcaa ggtgatggtg aacgggatcc tcttcgtgca 480

gtacttccac cgcgtgccct tccaccgtgt ggacaccatc tccgtcaatg gctctgtgca 540

gctgtcctac atcagcttcc agcctcccgg cgtgtggcct gccaacccgg ctcccattac 600

ccagacagtc atccacacag tgcagagcgc ccctggacag atgttctcta ctcccgccat 660

cccacctatg atgtaccccc accccgccta tccgatgcct ttcatcacca ccattctggg 720
agggctgtac ccatccaagt ccatcctcct gtcaggcact gtcctgccca gtgctcagag 780
gttccacatc aacctgtgct ctgggaacca catcgccttc cacctgaacc cccgttttga 840
tgagaatgct gtggtccgca acacccagat cgacaactcc tgggggtctg aggagcgaag 900
tctgccccga aaaatgccct tcgtccgtgg ccagagcttc tcagtgtgga tcttgtgtga 960
agctcactgc ctcaaggtgg ccgtggatgg tcagcacctg tttgaatact accatcgcct 1020
gaggaacctg cccaccatca acagactgga agtggggggc gacatccagc tgacccatgt 1080
gcagacatag gcggcttcct ggccctgggg ccgggggctg gggtgtgggg cagtctgggt 1140
cctctcatca tccccacttc ccaggcccag cctttccaac cctgcctggg atctgggctt 1200
taatgcagag gccatgtcct tgtctggtcc tgcttctggc tacagccacc ctggaacgga 1260
gaaggcagct gacggggatt gccttcctca gccgcagcag cacctggggc tccagctgct 1320
ggaatcctac catcccagga ggcaggcaca gccagggaga ggggaggagt gggcagtgaa 1380
gatgaagccc catgctcagt cccctcccat cccccacgca gctccacccc agtcccaagc 1440
caccagctgt ctgctcctgg tgggaggtgg cctcctcagc ccctcctctc tgacctttaa 1500
cctcactctc accttgcacc gtgcaccaac ccttcacccc tcctggaaag caggcctgat 1560
ggcttcccac tggcctccac cacctgacca gagtgttctc ttcagaggac tggctccttt 1620
cccagtgtcc ttaaaataaa gaaatgaaaa tgcttgttgg cacattcaaa aaaaaaaaaa 1680
aaaaaaaaaa aaaaaaaaaa aaaaaaaaaa aaaaaaaaaa aaaaaaaaaa aaaaaaaaa 1739

<210> 107
<211> 3547
<212> DNA
<213> Homo sapiens

<400> 107
ggaggtgagc aggaaggaga cggccgccca gcagcccgtg ggcaggcgcg gcggagcgag 60
cggggccggc ggcgggcgcc gagggacgcc gaggcctcgg gcgggggctg gcccggggtt 120
ccaggtctcc agtgggggct gcagactaag caaaatgagg cggttcctga ggccagggca 180
tgaccctgtg cgggagaggc tcaagcggga cctgttccag tttaacaaga cggtggagca 240
tggcttcccg caccagccca gcgccctcgg ctacagcccg tccctgcgca tcctggccat 300
cggcacccgt tctggagcca tcaagctcta cggagcccca ggcgtggagt tcatggggct 360
gcaccaggag aacaacgctg tgacgcagat ccacctcctg cccggccagt gccagctggt 420
caccctgctg gatgacaaca gcctgcacct ttggagcctg aaggtcaagg gcggggcatc 480
ggagctgcag gaggatgaga gcttcacact gcgtggaccc ccaggggctg cccccagtgc 540
cacacagatc accgtggtcc tgccacattc ctcctgcgag ctgctctacc tgggcaccga 600
gagtggcaac gtgtttgtgg tgcagctgcc agcttttcgt gcgctggagg accggaccat 660
cagctcggac gcggtgctgc agcggttgcc agaggaggcc cgccaccggc gtgtgttcga 720
gatggtggag gcactgcagg agcaccctcg agaccccaac cagatcctga tcggctacag 780

ccgaggcctc gttgtcatct gggacctaca gggcagccgc gtgctctacc acttcctcag 840
cagccagcaa ctggagaaca tctggtggca gcgggacggc cgcctgctcg tcagctgtca 900
ctctgacggc agctactgcc agtggcccgt gtccagcgaa gcccagcaac cagagcccct 960
ccgcagcctc gtgccttacg gtccctttcc ttgcaaagcg attaccagaa tcctctggct 1020
gaccactagg caggggttgc ccttcaccat cttccagggt ggcatgccac gggccagcta 1080
cggggaccgc cactgcatct cagtgatcca cgatggccag cagacggcct tcgacttcac 1140
ctcccgtgtc atcggcttca ctgtcctcac agaggcagac cctgcagcca cctttgacga 1200
cccctatgcc ctggtggtgc tggctgagga ggagctggtg gtgattgacc tgcagacagc 1260
aggctggcca ccggtccagc tgccctacct ggcttctctg cactgttccg ccatcacctg 1320
ctctcaccac gtctccaaca tcccgctgaa gctgtgggag cggatcattg ccgccggcag 1380
ccggcagaac gcacacttct ccaccatgga gtggccaatt gatggtggca ccagcctgac 1440
cccagcccca ccccagaggg acctgctgct cacagggcac gaggacggca cggtgcggtt 1500
ctgggatgcc tcgggtgtct gcctgcggct gctctacaaa ctcagcactg tgcgcgtgtt 1560
cctcaccgac acggacccca acgagaactt cagtgcccag ggcgaggacg agtggccccc 1620
actccgcaag gtgggctcct ttgaccccta cagtgatgac ccccggctgg gcatccagaa 1680
gatcttcctc tgcaagtaca gcggctacct ggctgtggca ggcacggcag ggcaggtgct 1740
ggtactggaa ctgaatgacg aggcagcgga gcaggctgtg gagcaggtgg aggccgacct 1800
gctgcaggac caagagggct accgctggaa ggggcacgag cgcctggcag cccgctcagg 1860
gcccgtgcgc tttgagcctg gctttcagcc cttcgtgttg gtgcagtgtc agcccccggc 1920
tgtggtcacc tccttggccc tgcactctga gtggcggctc gtggccttcg gcaccagcca 1980
tggctttggc ctctttgacc accagcagcg gcggcaggtc tttgttaagt gcacactgca 2040
ccccagtgac cagctggcct tggagggccc actctcccgc gtcaagtccc tcaagaagtc 2100
cttgcgtcag tcattccgcc ggatgcgtcg gagccgggtg tccagccgga agcggcaccc 2160
ggctggcccc ccaggagagg cacaggaggg gagtgccaag gctgagcggc caggcctcca 2220
gaacatggag ctggcgcctg tgcagcgcaa gatcgaggct cgctcggcag aggactcctt 2280
cacaggcttc gtccggaccc tgtactttgc tgacacctac ctgaaggaca gctcccggca 2340
ctgcccctcg ctgtgggctg gcaccaatgg gggcaccatc tatgccttct ccctgcgtgt 2400
gcctcccgcc gagcggagaa tggatgagcc tgtgcgggca gagcaggcca aggagatcca 2460
gctgatgcac cgggcgccgg tggtgggcat cctggtgctc gacggacaca gcgtacccct 2520
tcccgagccc ctcgaagtgg cccatgatct gtcgaagagc cctgacatgc agggaagcca 2580
ccagctgctc gtcgtatcag aggagcagtt caaggtgttc acgctgccca aggtgagtgc 2640
caagctgaag ttgaagctga cggccctgga gggctcaaga gtgcggcggg tcagcgtggc 2700
ccacttcggc agtcgtcgag ccgaggacta cggggagcac cacctggcag tccttaccaa 2760
cctgggcgac atccaggtgg tctcgctgcc cctgctcaag ccccaggtgc gctacagctg 2820

catccgccgg gaggacgtca gtggcatcgc ctcctgcgtc ttcaccaaat atggccaagg 2880

cttctacctg atctcaccct cggagtttga gcgcttctct ctctccacca agtggctggt 2940

ggagccccgg tgtctggtgg attcagcaga aaccaagaac caccgccctg gtaacggtgc 3000

gggccccaag aaggccccga gccgagccag gaactcaggg actcagagtg atggcgagga 3060

gaagcagccc ggcctggtga tggagcgcgc tctgctcagt gatgagagag cggcaactgg 3120

cgttcacatc gagccgccgt ggggtgcagc ctcagcaatg gcggagcaga gtgagtggct 3180

gagcgtccag gctgcgcgat gagcacacac tactactgat ggcctttcgg gggtccctgc 3240

cccaaccgga gaggccggtg cacagggccc cgccaggggc tgggggcatc ccggcttcca 3300

caatgcagct gctctgggcc tcgggagagg agagacccca gtcccctggg ctgcccttcc 3360

cgggcctcgt ctgtctgggt cctttggtca atgttgcaca gtttttattg ctcccatccc 3420

tttttgtagt gggctgggtt ttaagttata aatgttaact gcctctgggt gaaaaagttt 3480

ttaataaaca cctattacct cttgaaaaaa aaaaaaaaaa aaaaaaaaaa aaaaaaaaaa 3540

aaaaaaa 3547

<210> 108
<211> 1648
<212> DNA
<213> Homo sapiens

<400> 108
ggaagttccg gcgggggcgg ccgaggggga agagtgtgtc tgcgggagaa agaggagaat 60

cgcccaagcg gcctcggaag tcccagggag tggaggcccc cgccgtggag ccgtgtggtg 120

tatgtgtggt aacaccatgt ctgtgcccct gctcaccgat gctgccaccg tgtctggagc 180

tgagcgggaa acggccgcgg ttattttttt acatggactt ggagacacag ggcacagctg 240

ggctgacgcc ctctccacca tccggctccc tcacgtcaag tacatctgtc cccatgcgcc 300

taggatccct gtgaccctca acatgaagat ggtgatgccc tcctggtttg acctgatggg 360

gctgagtcca gatgccccag aggacgaggc tggcatcaag aaggcagcag agaacatcaa 420

ggccttgatt gagcatgaaa tgaagaacgg gatccctgcc aatcgaatcg tcctgggagg 480

cttttcacag ggcggggccc tgtccctcta cacggccctc acctgccccc accctctggc 540

tggcatcgtg gcgttgagct gctggctgcc tctgcaccgg gccttccccc aggcagctaa 600

tggcagtgcc aaggacctgg ccatactcca gtgccatggg gagctggacc ccatggtgcc 660

cgtacggttt ggggccctga cggctgagaa gctccggtct gttgtcacac ctgccagggt 720

ccagttcaag acatacccgg gtgtcatgca cagctcctgt cctcaggaga tggcagctgt 780

gaaggaattt cttgagaagc tgctgcctcc tgtctaacta gtcgctggcc ccagtgcagt 840

accccagctc atgggggact cagcaagcaa gcgtggcacc atcttggatc tgagccggtc 900

gagcccctgt ccccaccctt cctgacctgt ccttttccca caggcctctg ggggcaggtg 960

gcaaggcctg gccgggcctt ccttcctggc cttagccacc tggctctgtc tgcagcaggg 1020

gcaggctgct ttcttatcca tttccctgga ggcgggcccc cctggcagca gtattggagg 1080

ggctacaggc agctggagaa aggggcccag ccgctgaccc actcactcag gacctcactc 1140

actagccccg ctttgggccc cctcctgtga cctcagggtt tggcccatgg ggccccccca 1200

ggcccctgcc ccaactgatt ctgcccagat aatcgtgtct cctgcctcca ctcagctgct 1260

tctcagtcat gaatgtggcc atggccccgg ggtccccttg ctgctgtggg ctccctgtcc 1320

ctgggcagga gtgctggtga ggaggtggag ccttttgagg ggggccttcc ctcagctgtt 1380

tccccacact ggggggctgg gccctgcctc cccgttaccc tccttccctg caggcctgga 1440

gcctgtaggg ctggactgag gttcaggtct ccccccagct gtctcacccc cactttgtcc 1500

ccactctaga gcagggaggc agtgggggag gagttgtgtc tcgtcttctg tctccatgtg 1560

gtttttgggt gtttttcttg ttgtgtcctg gattccgata aaattaaaga aattgcttcc 1620

tcaaaaaaaa aaaaaaaaaa aaaaaaaa 1648

<210> 109
<211> 3608
<212> DNA
<213> Homo sapiens

<400> 109
gcggccgctt ccccccggcc gggcccccgc cgccccgcgg tccccagagc gccaggcccc 60

cggggggagg gagggagggc gccgggccgg tgggagccag cggcgcgcgg tgggacccac 120

ggagccccgc gacccgccga gcctggagcc gggccgggtc ggggaagccg gctccagccc 180

ggagcgaact tcgcagcccg tcgggggcgg gcggggaggg ggcccggagc cggaggaggg 240

ggcggccgcg ggcacccccg cctgtgcccc ggcgtccccg ggcaccatgc tgtccaactc 300

ccagggccag agcccgccgg tgccgttccc cgccccggcc ccgccgccgc agccccccac 360

ccctgccctg ccgcaccccc cggcgcagcc gccgccgccg cccccgcagc agttcccgca 420

gttccacgtc aagtccggcc tgcagatcaa gaagaacgcc atcatcgatg actacaaggt 480

caccagccag gtcctggggc tgggcatcaa cggcaaagtt ttgcagatct tcaacaagag 540

gacccaggag aaattcgccc tcaaaatgct tcaggactgc cccaaggccc gcagggaggt 600

ggagctgcac tggcgggcct cccagtgccc gcacatcgta cggatcgtgg atgtgtacga 660

gaatctgtac gcagggagga agtgcctgct gattgtcatg gaatgtttgg acggtggaga 720

actctttagc cgaatccagg atcgaggaga ccaggcattc acagaaagag aagcatccga 780

aatcatgaag agcatcggtg aggccatcca gtatctgcat tcaatcaaca ttgcccatcg 840

ggatgtcaag cctgagaatc tcttatacac ctccaaaagg cccaacgcca tcctgaaact 900

cactgacttt ggctttgcca aggaaaccac cagccacaac tctttgacca ctccttgtta 960

tacaccgtac tatgtggctc cagaagtgct gggtccagag aagtatgaca agtcctgtga 1020

catgtggtcc ctgggtgtca tcatgtacat cctgctgtgt gggtatcccc ccttctactc 1080

caaccacggc cttgccatct ctccgggcat gaagactcgc atccgaatgg gccagtatga 1140

atttcccaac ccagaatggt cagaagtatc agaggaagtg aagatgctca ttcggaatct 1200

gctgaaaaca gagcccaccc agagaatgac catcaccgag tttatgaacc acccttggat 1260
catgcaatca acaaaggtcc ctcaaacccc actgcacacc agccgggtcc tgaaggagga 1320
caaggagcgg tgggaggatg tcaaggggtg tcttcatgac aagaacagcg accaggccac 1380
ttggctgacc aggttgtgag cagaggattc tgtgttcctg tccaaactca gtgctgtttc 1440
ttagaatcct tttattccct gggtctctaa tgggacctta aagaccatct ggtatcatct 1500
tctcattttg cagaagagaa actgaggccc agaggcggag ggcagtctgc tcaaggtcac 1560
gcagctggtg actggttggg gcagaccgga cccaggtttc ctgactcctg gcccaagtct 1620
cttcctccta tcctgcggga tcactggggg gctctcaggg aacagcagca gtgccatagc 1680
caggctctct gctgcccagc gctggggtga ggctgccgtt gtcagcgtgg accactaacc 1740
agcccgtctt ctctctctgc tcccacccct gccgccctca ccctgccctt gttgtctctg 1800
tctctcacgt ctctcttctg ctgtctctcc tacctgtctt ctggctctct ctgtaccctt 1860
cctggtgctg ccgtgccccc aggaggagat gaccagtgcc ttggccacaa tgcgcgttga 1920
ctacgagcag atcaagataa aaaagattga agatgcatcc aaccctctgc tgctgaagag 1980
gcggaagaaa gctcgggccc tggaggctgc ggctctggcc cactgagcca ccgcgccctc 2040
ctgcccacgg gaggacaagc aataactctc tacaggaata tattttttaa acgaagagac 2100
agaactgtcc acatctgcct cctctcctcc tcagctgcat ggagcctgga actgcatcag 2160
tgactgaatt ctgccttggt tctggccacc ccagagtggg agaggctggg aggttgggag 2220
gctgtggaga gaagtgagca aggtgctctt gaacctgtgc tcattttgca attttatcag 2280
taatttgact tagagttttt acgaaacctc ttttgttgtc cttgccccac tcctctccac 2340
cagacgcctt cctctctgga tactgcaaag gcttgtggtt tgttagaggg tatttgtgga 2400
aactgtcata gggattgtcc ctgtgttgtc ccatctgccc tccctgtttc tccacaacag 2460
cctggggttg tccccgctgg ctcacgcgtt ctgggagctc aaggccacct tggaggagga 2520
tgccacgcac ttcctctctc ggagccctca gacatctcca gtgtgccaga caaataggag 2580
tgagtgtatg tgtgtgtgtg tgtgtgtgtg tgtgcacacg tgtgtatgag tgcgcagatc 2640
tgtgcctggg atcgtgcatt tgaggggcca ggggcaggca gggctgcaga gggagacggc 2700
cctgctgggg cttaggaacc ttctcccttc ttgggtctgc cctgcccata ctgagcctgc 2760
caaagtgcct gggaagccca cccagattct gaaacaggcc ctctgtggcc tgtctctatt 2820
agctgggttc cgggaggcag agaggagtga ccgggcactg gcactgcgat caggaagact 2880
ggacccccag cccccagggc ccccctcccc ccacttagtg ctggtcctag gtcctctgag 2940
gcactcatct actgaatgac ctctctactt ccccttcttg ccattattaa cccatttttg 3000
tttattttcc ttaaattttt agccatttct ccatgggcca ccgcccagct catgtaggtg 3060
agcctgggca gcttctgttg gcagagcttt tgcatttcct gtgtttgtcc tgggttctgg 3120
ggcatcagcc agctacccct tgtgggcaaa ggcagggcca cttttgaagt cttccctcag 3180
atttccattg tgtggcctgg tgggtcaggg ggagtctttg caccaaagat gtcctgactt 3240

tgcccccttg cccatcagcc atttgccatc accccaaaca actcagcttc ggggccggtg 3300
aggggagggg cctcccccag cacagatgag gagcagctgg ggtaggctgt ctgtgccatg 3360
gccccccact cccccttccc ttggagggag aggtggcagg aatacttcac ctttcctctc 3420
cctcaggggc aggtggtgga ggggcgccca gggtcgtctt tgtgtatggg ggaaggcgct 3480
gggtgcctgc agcgcctccc ttgtctcaga tggtgtgtcc agcactcgat tgttgtaaac 3540
tgttgttttg tatgagcgaa attgtcttta ctaaacagat ttaatagtta aaaaaaaaaa 3600
aaaaaaaa 3608

<210> 110
<211> 2937
<212> DNA
<213> Homo sapiens

<400> 110
gggttgcgcc agggagccgg aaagccgact cccgaagttg gggtcctggg aaaacttggg 60
tcctgggttg actgagaagc ggcggggaaa ggaggcgggc caggaggagg gggcctggcg 120
gacgccggcc ggggggcggg gcgcggcggg gctgtcggtc acgcccctca gtccgccccg 180
ccccgccccg cctgccgggg aagggccacg ttgcccggcc cggccgtccg gccccggcgc 240
gccgcagaaa gggctggcga gtcgaaaggc gaggcggccg cggcagcgct tgggacgcgc 300
ctgggcaccg ggctcgctcc ctgcgccccg gagcaggcca agttcggggc caggacgtcg 360
ggaggacctg gtgcatggct gcctcctaat cccatagtcc agaggaggca tccctaggac 420
tgcgggcaag ggagccgggc aagcccaggg cagccttgaa ccgtcccctg gcctgccctc 480
cccggtgggg gccaggatgc tgaagaagca gtctgcaggg cttgtgctgt ggggcgctat 540
cctctttgtg gcctggaatg ccctgctgct cctcttcttc tggacgcgcc cagcacctgg 600
caggccaccc tcagtcagcg ctctcgatgg cgaccccgcc agcctcaccc gggaagtgat 660
tcgcctggcc caagacgccg aggtggagct ggagcggcag cgtgggctgc tgcagcagat 720
cggggatgcc ctgtcgagcc agcgggggag ggtgcccacc gcggcccctc ccgcccagcc 780
gcgtgtgcct gtgacccccg cgccggcggt gattcccatc ctggtcatcg cctgtgaccg 840
cagcactgtt cggcgctgcc tggacaagct gctgcattat cggccctcgg ctgagctctt 900
ccccatcatc gttagccagg actgcgggca cgaggagacg gcccaggcca tcgcctccta 960
cggcagcgcg gtcacgcaca tccggcagcc cgacctgagc agcattgcgg tgccgccgga 1020
ccaccgcaag ttccagggct actacaagat cgcgcgccac taccgctggg cgctgggcca 1080
ggtcttccgg cagtttcgct tccccgcggc cgtggtggtg gaggatgacc tggaggtggc 1140
cccggacttc ttcgagtact ttcgggccac ctatccgctg ctgaaggccg acccctccct 1200
gtggtgcgtc tcggcctgga atgacaacgg caaggagcag atggtggacg ccagcaggcc 1260
tgagctgctc taccgcaccg actttttccc tggcctgggc tggctgctgt tggccgagct 1320
ctgggctgag ctggagccca agtggccaaa ggccttctgg gacgactgga tgcggcggcc 1380

ggagcagcgg caggggcggg cctgcatacg ccctgagatc tcaagaacga tgacctttgg 1440
ccgcaagggt gtgagccacg ggcagttctt tgaccagcac ctcaagttta tcaagctgaa 1500
ccagcagttt gtgcacttca cccagctgga cctgtcttac ctgcagcggg aggcctatga 1560
ccgagatttc ctcgcccgcg tctacggtgc tccccagctg caggtggaga aagtgaggac 1620
caatgaccgg aaggagctgg gggaggtgcg ggtgcagtat acgggcaggg acagcttcaa 1680
ggctttcgcc aaggctctgg gtgtcatgga tgaccttaag tcgggggttc cgagagctgg 1740
ctaccggggt attgtcacct tccagttccg gggccgccgt gtccacctgg cgcccccacc 1800
gacgtgggag ggctatgatc ctagctggaa ttagcacctg cctgtccttc ctgggcccct 1860
ccttgccaca tcatgagctg aggtgggacc acagtcccca ggctgcatcg gcctgcctgt 1920
gtttccctct taggtgcatt tatctttttg atttttccga gtggcattta agtgcacaaa 1980
tgataacaag aggattattc tcccgttctc aagggagtca gatcagggga actattctag 2040
ggtatgttgc ggggtattaa gcaggaaacc actgtgtggt ggggggcact gggcttgttg 2100
gggccagaaa tgtccacgtc ctgagctttc tcctggagca tgtgcagaga gtttggcaac 2160
gttcgctctc ttgaccagac cccttctccc tgacctggct cttccagcca gggcacgagc 2220
cctccttcta tacctgctcc ccttccccca gtggggactg agttatggga gaaggggaca 2280
tatttgtggc caaaatgata ctaaccaaag gggcttcctt gtcagggcct ggtggagttg 2340
gtgggtcatc ggggctcact gcctcctgcc cttctctcct gtctgacccc cacttagccc 2400
ttctctcctt gcagcctagc agtttatagt tctgagatgg aaagttgaag ggggcaagca 2460
agacctctcc tcagcccatg cccagctgtc aggagagagg tgcagggagg aaggccttgt 2520
gctgggacaa cctctctctt gccttacctc agagagggac tatgccctga cccctccttt 2580
ctgaaaatca gtgccctccc tgttgctcta ggaggctcct gctggcttgg tagaagacag 2640
aattcgatct gcctgtccct tttccccctg gggtttgaca cacaggctcc tctcagcatg 2700
aggtggagca gtgaccaggt ggagcagtga ccaggacgcc tctggcccag tgctgcccag 2760
cctccccgcc cgctcccagg cgccccatgt cctcacaggc caggacgcca tggcaggatg 2820
gagaggactt ggtggatttt tgtttcttgc ctgacctcag tttcatgaaa gaaagtggaa 2880
gctacagaat tattttctaa aataaaggct gaattgtctg aaaaaaaaaa aaaaaaa 2937

<210> 111
<211> 2327
<212> DNA
<213> Homo sapiens

<400> 111
   gagcggccgc gccgggtccc cgggacgggg tggaagtggg ggtgggggga ggggatcggg 60
   gccgggccgg ggccgcgctg cctgcgatgc cgggcgcccg ccgcagccgc tgccgccgga 120
   gcccgggatg gggcgagagg ctgcggcgga cgccagcatc tccccgccgg ggaccccggg 180
   ggccgcggag ccgccgccgc cgctgctgcc gccgttgctg agacccagcg ggcgatgggg 240
   atgaagatga gacgctacaa gctctttctc atgttctgta tggccggcct gtgcctcatc 300
   tccttcctgc acttcttcaa gaccctgtcc tatgtcacct tcccccgaga actggcctcc 360
   ctcagcccta acctggtgtc cagctttttc tggaacaatg ccccggtcac gccccaggcc 420
   agccccgagc caggaggccc tgacctgctg cgtaccccac tctactccca ctcgcccctg 480
   ctgcagccgc tgccgcccag caaggcggcc gaggagctcc accgggtgga cttggtgctg 540
   cccgaggaca ccaccgagta tttcgtgcgc accaaggccg gcggcgtctg cttcaaaccc 600
   ggcaccaaga tgctggagag gccgcccccg ggacggccgg aggagaagcc tgagggggcc 660
   aacggctcct cggcccggcg gccaccccgg tacctcctga gcgcccggga gcgcacgggg 720
   ggccgaggcg cccggcgcaa gtgggtggag tgcgtgtgcc tgcccggctg gcacggaccc 780
   agctgcggcg tgcccactgt ggtgcagtac tccaacctgc ccaccaagga gcggctggtg 840
   cccagggagg tgccgcgccg cgtcatcaac gccatcaacg tcaaccacga gttcgacctg 900
   ctggacgtgc gcttccacga gctgggcgac gtggtggacg cctttgtggt gtgcgagtcc 960
   aacttcacgg cttatgggga gccgcggccg ctcaagttcc gggagatgct gaccaatggc 1020
   accttcgagt acatccgcca caaggtgctc tatgtcttcc tggaccactt cccgcccggc 1080
   ggccggcagg acggctggat cgccgacgac tacctgcgca ccttcctcac ccaggacggc 1140
   gtctcgcggc tgcgcaacct gcggcccgac gacgtcttca tcattgacga tgcggacgag 1200
   atcccggccc gtgacggcgt ccttttcctc aagctctacg atggctggac cgagcccttc 1260
   gccttccaca tgcgcaagtc gctctacggc ttcttctgga agcagccggg caccctggag 1320
   gtggtgtcag gctgcacggt ggacatgctg caggcagtgt atgggctgga cggcatccgc 1380
   ctgcgccgcc gccagtacta caccatgccc aacttcagac agtatgagaa ccgcaccggc 1440
   cacatcctgg tgcagtggtc gctgggcagc cccctgcact tcgccggctg gcactgctcc 1500
   tggtgcttca cgcccgaggg catctacttc aagctcgtgt ccgcccagaa tggcgacttc 1560
   ccacgctggg gtgactacga ggacaagcgg gacctgaact acatccgcgg cctgatccgc 1620
   accgggggct ggttcgacgg cacgcagcag gagtacccgc ctgcagaccc cagcgagcac 1680
   atgtatgcgc ccaagtacct gctgaagaac tacgaccggt tccactacct gctggacaac 1740
   ccctaccagg agcccaggag cacggcggcg ggcgggtggc gccacagggg tcccgaggga 1800
   aggccgcccg cccggggcaa actggacgag gcggaagtct agagctgcat gatctgatag 1860
   ggtttgtgac agggcggggg tggcggcggc ccctagcgct atctccctgc ctcctgccgg 1920
   ctccttggtt cttgagggga ccaggagtgg gtggggagtg ggggtggggg tagggtttcc 1980
   ctactgaagc ccttgtgaat caagggtcag gcctttgagc tcagaaaata tccctcctgt 2040
   tgggagaggg cgcaggccgt gacgtctggg tggcccttat gactgccaag actgctgtgg 2100
   ccaggaggtg ccactggagt gtgcgtggtg gtccctgggt agcgggggag ggtaggcagg 2160
   attggggaag agagcctgca ggatctcacc aggcagcctc tggggggtgg ccaggccggg 2220
   aaaaagccca ccatttggca tccctgggcc ttgggctccg tgtgggagac cggcctgcca 2280
   ggaggaccca gggctctgta agtagatgca tttgggtcca ggaggaa 2327

<210> 112
<211> 3592
<212> DNA
<213> Homo sapiens

<400> 112
gtgactgaac tttgcctgat catgaccttg atcatgcaca gggctgctgg tggggtgagg 60

ccatcccacg caactctggg gtctgtgaag ccaccaagga tcaaggaatg gaagagctgc 120

ctgagtgtag agggagttcg gtcaggcttc ttaaacttgg agacttggaa acagaaggcc 180

ttgtaggcca cataggtttt cagagactcg cctggcagag gcctggagcg tcctgctgcg 240

tggactgtgg gcaccgagcc accaggaggt tgttgatctc attcttgctc actggtccct 300

gccagactgg tggtgcctct ctactgtcgg gcagccccac ctctgtgctg ccccatccac 360

ctacagccct catgcctggg cccagctgga ggttctgaca ggggccccct ggggtggcat 420

gcggaccctt cagggtgccc caggcgcagc acaggtctcc agagacacag gcaccagggc 480

gttcagagct agtcccctcg ctgctgttga ctgtccttga gcagccccag ggccggtttc 540

tcaacctcat ttgctcacct gagcaatgaa gtgaggtggg ctcctggaac tgcagcagcc 600

accccccggg gccatcgtga ggctaagaat ccagtgcagg gtgagcaacc agcgaccgct 660

acaaggacag tgaagagcta gcaccaggac cttgggtagc ccacccgctg gagggagcgt 720

gtgctgcaaa aagcaagagt gggctttgga ggccaacgga tggagcggat tcagtctgag 780

gctgttccag cccttgctta ggatccacct acctaggtct ggaaatcgta tttccacttc 840

agatgccttc tcagaggata aaataacccc cggtggggga gagtactgga agagggctaa 900

ttcccctggt tttctcccca tgagcattag tgtcagtgta attttagagt gaccccagct 960

atgtcacgtg tggcaggcac aagaacagat gtcaatgagc tattgcagag gtggacaccc 1020

agatgtgtgc gctggcacac tggaggggcc cgtagggtgg ctctagaccg ccccctcgtg 1080

acggcttgcc taccacctgc aggcgacgtt gtggacgttt accagcggga gttcctggcg 1140

ctgcgcgatc ggttgcacgc agctgagcag gagagcctca agcgctccaa ggagctcaac 1200

ctggtgctgg acgagatcaa gagggccgtg tcagaaaggc aggcgctgcg agacggagac 1260

ggcaatcgca cctggggccg cctaacagag gacccccgat tgaagccgtg gaacggctca 1320

caccggcacg tgctgcacct gcccaccgtc ttccatcacc tgccacacct gctggccaag 1380

gagagcagtc tgcagcccgc ggtgcgcgtg ggccagggcc gcaccggagt gtcggtggtg 1440

atgggcatcc cgagcgtgcg gcgcgaggtg cactcgtacc tgactgacac tctgcactcg 1500

ctcatctccg agctgagccc gcaggagaag gaggactcgg tcatcgtggt gctgatcgcc 1560

gagactgact cacagtacac ttcggcagtg acagagaaca tcaaggcctt gttccccacg 1620

gagatccatt ctgggctcct ggaggtcatc tcaccctccc cccacttcta ccctgacttc 1680

tcccgcctcc gagagtcctt tggggacccc aaggagagag tcaggtggag gaccaaacag 1740

aacctcgatt actgcttcct catgatgtac gcgcagtcca aaggcatcta ctacgtgcag 1800

ctggaggatg acatcgtggc caagcccaac tacctgagca ccatgaagaa ctttgcactg 1860
cagcagcctt cagaggactg gatgatcctg gagttctccc agctgggctt cattggtaag 1920
atgttcaagt cgctggacct gagcctgatt gtagagttca ttctcatgtt ctaccgggac 1980
aagcccatcg actggctcct ggaccatatt ctgtgggtga aagtctgcaa ccccgagaag 2040
gatgcgaagc actgtgaccg gcagaaagcc aacctgcgga tccgcttcaa accgtccctc 2100
ttccagcacg tgggcactca ctcctcgctg gctggcaaga tccagaaact gaaggacaaa 2160
gactttggaa agcaggcgct gcggaaggag catgtgaacc cgccagcaga ggtgagcacg 2220
agcctgaaga cataccagca cttcaccctg gagaaagcct acctgcgcga ggacttcttc 2280
tgggccttca cccctgccgc gggggacttc atccgcttcc gcttcttcca acctctaaga 2340
ctggagcggt tcttcttccg cagtgggaac atcgagcacc cggaggacaa gctcttcaac 2400
acgtctgtgg aggtgctgcc cttcgacaac cctcagtcag acaaggaggc cctgcaggag 2460
ggccgcaccg ccaccctccg gtaccctcgg agccccgacg gctacctcca gatcggctcc 2520
ttctacaagg gagtggcaga gggagaggtg gacccagcct tcggccctct ggaagcactg 2580
cgcctctcga tccagacgga ctcccctgtg tgggtgattc tgagcgagat cttcctgaaa 2640
aaggccgact aagctgcggg cttctgaggg taccctgtgg ccagccctga agcccacatt 2700
tctgggggtg tcgtcactgc cgtccccgga gggccagata cggccccgcc caaagggttc 2760
tgcctggcgt cgggcttggg ccggcctggg gtccgccgct ggcccggagg ccctaggagc 2820
tggtgctgcc cccgcccgcc gggccgcgga ggaggcaggc ggcccccaca ctgtgcctga 2880
ggcccggaac cgttcgcacc cggcctgccc cagtcaggcc gttttagaag agcttttact 2940
tgggcgcccg ccgtctctgg cgcgaacact ggaatgcata tactacttta tgtgctgtgt 3000
tttttattct tggatacatt tgattttttc acgtaagtcc acatatactt ctataagagc 3060
gtgacttgta ataaagggtt aatgaagtgt gtgcctcaaa tgtgagcgcc ctgggcatct 3120
ccctctgccc tgcggttggc ttcatttggt ctgtgcccca acatgccaac taagcctggc 3180
actgggcctc cccagccagc atcagcagcg acctttccct gctaagtcaa ggagccagcc 3240
tggcctgcag gcgcggggtc cagcccctgg ctgtgcagct gcagtgccac ctcggcgcca 3300
gcgtaccccc cttcctagga tcaggccgca aagtctgtca ctggtgaaac cagctcagaa 3360
tctgggacca caatgcctat agcaggtccc ccgaatgtct cccccatgtc caaggcctgc 3420
gatgaggcct tcccttagct cctcaagctg aaggcatttc ccagcctcct tgttcccctc 3480
cgtgtgtctc agcttggtcc tcatcctggg tccccgacac tcctctggca cattagggtc 3540
tcccgctcac ctcagggcct ttgcacacac tgttgcccct ccttggaaag ct 3592

<210> 113
<211> 2421
<212> DNA
<213> Homo sapiens

<400> 113
cagcagaatg gaagtgagga aaggcaacca gctgacacag gagccagagt gagaccagca 60

gactctcaca ctcaacctac accatgaatt tgtgtctatc ttctacgcgt taagagccaa 120
ggacaggtga agttgccaga gagcaatggc tctcttcact ccgtggaagt tgtcctctca 180
gaagctgggc tttttcctgg tgacttttgg cttcatttgg ggtatgatgc ttctgcactt 240
taccatccag cagcgaactc agcctgaaag cagctccatg ctgcgcgagc agatcctgga 300
cctcagcaaa aggtacatca aggcactggc agaagaaaac aggaatgtgg tggatgggcc 360
atacgctgga gtcatgacag cttatgatct gaagaaaacc cttgctgtgt tattagataa 420
cattttgcag cgcattggca agttggagtc gaaggtggac aatcttgttg tcaatggcac 480
cggaacaaac tcaaccaact ccactacagc tgttcccagc ttggttgcac ttgagaaaat 540
taatgtggca gatatcatta acggagctca agaaaaatgt gtattgcctc ctatggacgg 600
ctaccctcac tgtgagggaa agatcaagtg gatgaaagac atgtggcgtt cagatccctg 660
ctacgcagac tatggagtgg atggatccac ctgctctttt tttatttacc tcagtgaggt 720
tgaaaattgg tgtcctcatt taccttggag agcaaaaaat ccctacgaag aagctgatca 780
taattcattg gcggaaattc gtacagattt taatattctc tacagtatga tgaaaaagca 840
tgaagaattc cggtggatga gactacggat ccggcgaatg gctgacgcat ggatccaagc 900
aatcaagtcc ctggcagaaa agcagaacct tgaaaagaga aagcggaaga aagtcctcgt 960
tcacctggga ctcctgacca aggaatctgg atttaagatt gcagagacag ctttcagtgg 1020
tggccctctt ggtgaattag ttcaatggag tgatttaatt acatctctgt acttactggg 1080
ccatgacatt aggatttcag cttcactggc tgagctcaag gaaatcatga agaaggttgt 1140
aggaaaccga tctggctgcc caactgtagg agacagaatt gttgagctca tttacattga 1200
tattgtagga cttgctcaat tcaagaaaac tcttggacca tcctgggttc attaccagtg 1260
catgctccga gtccttgatt catttggtac tgaacccgaa tttaatcatg caaattatgc 1320
ccaatcgaaa ggccacaaga ccccttgggg aaaatggaat ctgaaccctc agcagtttta 1380
taccatgttc cctcataccc cagacaacag ctttctgggg tttgtggttg agcagcacct 1440
gaactccagt gatatccacc acattaatga aatcaaaagg cagaaccagt cccttgtgta 1500
tggcaaagtg gatagcttct ggaagaataa gaagatctac ttggacatta ttcacacata 1560
catggaagtg catgcaactg tttatggctc cagcacaaag aatattccca gttacgtgaa 1620
aaaccatggt atcctcagtg gacgggacct gcagttcctt cttcgagaaa ccaagttgtt 1680
tgttggactt gggttccctt acgagggccc agctcccctg gaagctatcg caaatggatg 1740
tgcttttctg aatcccaagt tcaacccacc caaaagcagc aaaaacacag actttttcat 1800
tggcaagcca actctgagag agctgacatc ccagcatcct tacgctgaag ttttcatcgg 1860
gcggccacat gtgtggactg ttgacctcaa caatcaggag gaagtagagg atgcagtgaa 1920
agcaatttta aatcagaaga ttgagccata catgccatat gaatttacgt gcgaggggat 1980
gctacagaga atcaatgctt tcattgaaaa acaggacttc tgccatgggc aagtgatgtg 2040
gccacccctc agcgccctac aggtcaagct tgctgagccc gggcagtcct gcaagcaggt 2100

gtgccaggag agccagctca tctgcgagcc ttctttcttc cagcacctca acaaggacaa 2160

ggacatgctg aagtacaagg tgacctgcca aagctcagag ctggccaagg acatcctggt 2220

gccctccttt gaccctaaga ataagcactg tgtgtttcaa ggtgacctcc tgctcttcag 2280

ctgtgcaggc gcccacccca ggcaccagag ggtctgcccc tgccgggact tcatcaaggg 2340

ccaggtggct ctctgcaaag actgcctata gcagctacct gctcagccct gcaccatgct 2400

gctggggaag acagtggccc c 2421

<210> 114
<211> 3941
<212> DNA
<213> Homo sapiens

<400> 114
tggacgagcg tccgtgcggc ctggtccggg ccatgtccgc gtgaggaccc cgccgctgtc 60

gccgctcccg ttccggccct ggcccctctg cccggcagcg cggcgcacca tgggctccat 120

tctcagccgc cgcatcgcgg gggtggagga catcgacatc caggcgaact cggcctatcg 180

ctaccctccg aagtccggaa actactttgc ttcgcacttt ttcatgggag gagagaaatt 240

cgacaccccc caccctgaag gttacctctt tggagagaac atggatctga acttcctggg 300

cagccgcccg gtccagtttc cctacgtcac tcctgccccc cacgagcccg tgaagacgct 360

gcggagcctg gtgaacatcc gcaaagactc cctgcggctg gtgaggtaca aagacgatgc 420

cgacagcccc accgaggacg gcgacaagcc ccgggtgctc tacagcctgg agttcacctt 480

cgacgccgat gcccgcgtgg ccatcaccat ctactgccag gcatcggagg agttcctgaa 540

cggcagggca gtatacagcc ccaagagccc ctcgctacag tccgagaccg tccactacaa 600

gagaggggtg agccagcagt tctccctgcc ctccttcaag attgacttct cggaatggaa 660

ggatgacgag ctgaactttg acctggaccg gggcgtgttt ccagtagtca tccaggctgt 720

ggtggacgaa ggagatgtgg tggaagtgac tggccacgcc cacgtgctct tggctgcctt 780

tgaaaagcac atggacggca gcttctctgt gaagccttta aagcagaagc aaattgtgga 840

ccgggtcagc tacctcctgc aggagatcta tggcattgag aacaagaaca accaggagac 900

caagccctcg gacgacgaga acagcgacaa cagcaacgag tgtgtggtgt gcctgtccga 960

cctgcgggac acgctgatcc tgccctgccg ccacctgtgc ctctgtacct cctgcgccga 1020

cacgctgcgc taccaggcca acaactgccc catctgccgg ctgcctttcc gggccctcct 1080

gcagatccgg gcggtgcgga agaagccagg agccctgtcc cccgtgtcct tcagccccgt 1140

cctggcccag agcctggagc atgatgagca ctcttgtccc tttaaaaaat caaagccgca 1200

ccccgcctcc ctggccagca agaaacctaa aagggaaaca aactctgaca gcgtcccacc 1260

tggctacgag cccatctcgc tgctcgaggc gctcaacggc ctccgggctg tctccccggc 1320

catcccctcg gcccctcttt atgaagaaat cacctattca ggcatctcgg acggcctgtc 1380

ccaggccagc tgtcccctcg cggctatcga ccacatcctg gacagcagcc gccagaaggg 1440

caggccgcag agcaaggccc ccgacagcac cctacggtcc ccgtcttccc ccatccacga 1500
agaggatgag gagaagctct ccgaggacgt ggacgcccct cccccactgg gtggcgcaga 1560
gctggccctg cgggaaagca gctcccctga gagtttcata acagaagagg ttgatgagtc 1620
gtcgtcacca cagcaaggga cccgagcagc ttccattgag aatgtcctgc aggacagcag 1680
ccccgagcac tgtggccgag gcccacctgc tgacatctac ctgccaggac ggcccacctc 1740
catggagacg gcccacggcc tcgccaccac cagccccacc tggcctccac ttggtggccc 1800
cagccccgat cccagcgccg ccgagctgac cccactctga gagcctggcc gagctggcag 1860
catggagccc tcggctcccc agactttgcc gaggggctgc tccggacccc gttgtgagcc 1920
ggcctcctgt ctgcatgccc cctgtggcca ccaggctccg aggggccgtg gtgactcttg 1980
atcaaagagc acagtgaact gtcccttctg agtctccctt ttctacagtt gatatatttg 2040
taactggtac aagatgaagg acagcagctt tccatcccta gttcagagcc cccgttcccc 2100
agggtcctgt gggctgagcg gctggggctg gggctgccca cgtgtggcct ccgctggctc 2160
tgcctgctcc tgcaacagtg cggtccctgc ccggagaact caggaggcct gcagaagaga 2220
actgattggt ggtcgaagca ccatcttcac agatgttcag gggcagtggg gggctccagg 2280
cacggtcaat gaaggaaaca gtgcctgtcc acccaccctg cgtgtcactg tggcggcctg 2340
gctgtcgctg ctttttgtcc tctgacgtgt ttgcgcggcc tcagtgccct ccctggtgcg 2400
tctgcgctgg ggccctcagt gctcggggcc ttggggtgca tgggcgccgc cctgggcagc 2460
tagagtgtct cagcccggtg ctgggcctgg ccgaggggcg gaggcacagc tgcttccagc 2520
agccagcatt cagtggcctt gtcaccaagc tccacacctc ctcctggtgc tggctttggt 2580
gacatcacaa ggcccctcca ggtgcagggg cttctgtttg gcaggcccct gccagggagg 2640
acctggtggc ctcctcattc tcttttgcca ttggaatgtc cccttgcagt tctcttctct 2700
tttttttttt ttgagatgga gtttcactct tgctgcccag gctggagtgc agtggctcaa 2760
tctcgggtca ctgcaacctc cgcctcccgg gttcaagtga tcgtcctgcc ttaggctcct 2820
gagtagctgg ggattacagg tgcctaccag catgctcggc taattttttt gtatttttag 2880
tagagaaggg atttcaccat gttggccggg ctggtctcaa actcctaagg tcatccacct 2940
gcctcggcct cccagagtgc tgagattaca ggcgtgagcc tccgcgcccg gcccccttgc 3000
agttctctct gatttggttt gttctgtctc aggcttctgt ggcaggactg gcccagggag 3060
gaggaagcca gcagcacacc tggggaatgg ggtcccggcc gggaggcttg gcctctgggc 3120
gacctcgtcc tgttttgttt gtttgtttgt ttgttttttt aaaggtaaac ctcctgggcc 3180
gcagatggca aagggagtgc ctgggcctgg tgacccaggg ctggatccac ccctgcggag 3240
ccctgggcca ggcaggtgtc tgctgctcac ctggctctgg agggctgccc tgcagctggg 3300
cctggggaca ggtcggctgt ggggcagctc agtaccctcc ctgaggctca cggtggctcc 3360
gagcatgagc tctgcctcct gggcgagacc cagcagtgga cagcacggtc ctcacaccca 3420
gctccctgca cacccaggcc agccacccct cccgctcgtg cacaggcacg cagatgcgct 3480

cacacgtaca cacacacaaa tgcacgccca cttgcacatg ctcacgcaca cgttcacaca 3540

tgcacactca cgctcacaca tgctgtcacg catacacaca cgcacatact cctgcacatg 3600

ttcccatgca tgtgtgtgca ctcggaccga gcatctccca cgcacctcta ccccacccca 3660

agcacctctc tccccccatg cacctctccc caacaacaca cacagccccc tgcaccgccc 3720

gccccccgcc cccaccaagg ccccagcctc tggccatcag tcctggtgcc agagctttgc 3780

gtgaagttcg ggccgcagag tggcccgctg ggactcccat gtgctgccgt ctgatgtgct 3840

cagatgggct catcgttggt tcgtttttac tgtatattta tagtaataaa atcatgcagc 3900

aaaaaaaaaa aaaaaaaaaa aaaaaaaaaa aaaaaaaaaa a 3941

<210> 115
<211> 1170
<212> DNA
<213> Homo sapiens

<400> 115
agagtagagg cggcggcggc ggcggccgga cccagactgg tagtgaggct ttggaccccg 60

agccgctgca atgccgctgg agctggagct gtgtcccggg cgctgggtgg gcgggcaaca 120

cccgtgcttc atcattgccg agatcggcca gaaccaccag ggcgacctgg acgtagccaa 180

gcgcatgatc cgcatggcca aggagtgtgg ggctgattgt gctaagttcc agaagagtga 240

gctagaattc aagtttaatc ggaaagcctt ggagaggcca tacacctcga agcattcctg 300

ggggaagacg tacggggagc acaaacgaca tctggagttc agccatgacc agtacaggga 360

gctgcagagg tacgccgagg aggttgggat cttcttcact gcctctggca tggatgagat 420

ggcagttgaa ttcctgcatg aactgaatgt tccatttttc aaagttggat ctggagacac 480

taataatttt ccttatctgg aaaagacagc caaaaaaggt cgcccaatgg tgatctccag 540

tgggatgcag tcaatggaca ccatgaagca agtttatcag atcgtgaagc ccctcaaccc 600

caacttctgc ttcttgcagt gtaccagcgc atacccgctc cagcctgagg acgtcaacct 660

gcgggtcatc tcggaatatc agaagctctt tcctgacatt cccatagggt attctgggca 720

tgaaacaggc atagcgatat ctgtggccgc agtggctctg ggggccaagg tgttggaacg 780

tcacataact ttggacaaga cctggaaggg gagtgaccac tcggcctcgc tggagcctgg 840

agaactggcc gagctggtgc ggtcagtgcg tcttgtggag cgtgccctgg gctccccaac 900

caagcagctg ctgccctgtg agatggcctg caatgagaag ctgggcaagt ctgtggtggc 960

caaagtgaaa attccggaag gcaccattct aacaatggac atgctcaccg tgaaggtggg 1020

tgagcccaaa ggctatcctc ctgaagacat ctttaatcta gtgggcaaga aggtcctggt 1080

cactgttgaa gaggatgaca ccatcatgga agaattggta gataatcatg gcaaaaaaat 1140

caagtcttaa aaataaagtg ccattctctg 1170

<210> 116
<211> 7913
<212> DNA
<213> Homo sapiens

<400> 116
ccttagcccc gtgggcccca cggagtgagc ggcatgcagc acccccgggc ctgtccagtg 60
tcctctggcc tgctgccctg cacccccaga aggccctgac gccctggggc acttctgctc 120
tgcacaggac cacgcggggg tttgccatgg tgacataaag gggcgcggag gaaggaagga 180
gcgtgaccag cctgtggact gcgcccctgg ctgggaggaa ggactggggg cccagatcct 240
ccactcccag tgccccacaa gggcgtcgct tcctaagtct ctgtgaattt gttggtcagt 300
ggacgattct cgtgtctcct cctgtgtggg gccttggggt agccagggca ggccgggcct 360
ccggtggcca aggtctcgga ggccaggatg cctgccctgg catgcctccg gaggctgtgt 420
cggcacgtgt ccccgcaggc tgtccttttc ctgctgttca tcttctgcct gttcagcgtt 480
ttcatctcgg cctactacct atatggctgg aagcgaggcc tggagccctc ggcggatgcc 540
cccgagcctg actgcgggga cccgccgcct gtggccccca gtcgcctgct gccactcaag 600
cctgtgcagg cagccacccc ttcccgcaca gacccgttgg tgctggtctt tgtggagagc 660
ctctactcgc aactgggcca ggaggtggtg gccatcctgg agtccagccg cttcaaatac 720
cgcacagaga ttgcgccggg caagggtgac atgcccacgc tcactgacaa gggccgtggc 780
cgcttcgccc tcatcatcta tgagaacatc ctcaagtatg tcaacctgga cgcctggaac 840
cgggagctgc tggacaagta ctgtgtggcc tacggcgtgg gcatcattgg cttcttcaag 900
gccaatgaga acagcctgct gagtgcgcag ctcaagggct tccccctgtt cctgcactca 960
aacctgggcc tgaaggactg cagcatcaac cccaagtccc cgctgctcta cgtgacgcga 1020
cctagcgagg tggagaaagg tgtgctcccc ggcgaggact ggacggtttt ccagtcaaat 1080
cactccacct atgagccagt gctgctggcc aagacgcgct cgtctgagtc catcccacac 1140
ctgggcgcag acgccggcct gcatgctgca ctgcacgcca ctgtggtcca ggacctgggc 1200
ctgcacgacg gcatccagcg cgtgctgttt ggcaacaacc tgaacttctg gctgcacaag 1260
cttgtcttcg tggatgccgt ggccttcctc acggggaagc gcctctccct gccattggac 1320
cgctacatcc tggtggacat tgatgacatc ttcgtgggca aggagggcac acgcatgaag 1380
gtggaggacg tgaaggccct gtttgacaca cagaacgaac tacgcgcaca catcccaaac 1440
ttcaccttca acctgggcta ctcagggaaa ttcttccaca caggtaccaa tgctgaggac 1500
gctggggatg atctgctgct gtcgtatgtg aaggagttct ggtggttccc ccacatgtgg 1560
agccacatgc agccccacct tttccacaac cagtccgtgt tggccgagca gatggccttg 1620
aacaagaagt tcgctgtcga gcatggcatt cccacagaca tggggtatgc agtggcgccc 1680
caccactcgg gcgtgtaccc cgtgcacgtg cagctgtacg aggcttggaa gcaggtgtgg 1740
agcatccgcg tgaccagcac ggaggagtac ccccacctga agccagcccg ctaccgccgt 1800
ggcttcatcc acaatggcat catggttctc ccacggcaga cctgcggcct cttcacacac 1860
accatcttct acaacgagta ccctggcggc tccagtgagc tggacaagat catcaacggg 1920
ggcgagctct tcctcaccgt gctcctcaat cctatcagca tcttcatgac gcacctgtcc 1980

aactatggga atgaccgcct gggcctgtac accttcaagc acctggtgcg cttcctgcac 2040
tcctggacga acctccggct gcagacactg ccccctgtgc agttggcgca gaagtacttc 2100
cagatcttct ccgaggagaa ggacccgctc tggcaggacc cctgcgagga caaacgtcac 2160
aaagacatct ggtccaagga gaagacgtgt gaccgcttcc caaagctcct catcatcggc 2220
ccccagaaaa caggcaccac tgccctctac ctgttcctgg gcatgcaccc tgacctaagc 2280
agcaactacc ccagctctga gacctttgag gagatccagt tttttaatgg ccacaactat 2340
cacaaaggca tcgactggta catggagttc ttccccatcc cttccaacac cacctccgac 2400
ttctactttg agaaaagcgc caactacttt gattcagaag tggcgccccg gcgggcagca 2460
gccctcttgc ccaaagccaa ggtcctgacc atcctcatca accccgcgga ccgggcctat 2520
tcctggtacc agcaccagcg agcccatgac gacccagtgg ccctaaagta caccttccat 2580
gaggtgatta ccgccggctc tgacgcatcc tcgaagctgc gtgccctcca gaaccgctgc 2640
ctggtccctg gctggtacgc cacccacatc gagcgctggc tcagtgccta tcacgccaac 2700
cagattctgg tcttggatgg caaactgctt cgcacagaac ctgccaaagt gatggacatg 2760
gtgcagaagt tccttggggt gaccaacacc attgactacc acaaaacctt ggcgtttgat 2820
ccaaagaaag gattttggtg ccaactgctt gaaggaggaa aaaccaagtg tctgggcaaa 2880
agcaagggcc ggaaatatcc cgagatggac ttggattccc gagccttcct gaaggactat 2940
taccgggacc acaacatcga gctctccaag ctgctgtata agatgggcca gacacttccc 3000
acttggctac gagaggacct ccagaacacc aggtagccgt ggccaccaca gccagactga 3060
acgtttgtga aagctgggac atcccaccac acgctgagcc agacctgcag agtgggaagc 3120
tggaccaggg cagctgcgca cttatgagca atactctgtg gaggtctggt ggggctgggg 3180
gagcacccag gcggatctgc aagcacctcg gagcacccac cgctgggtct gcggcctaag 3240
ggacctccct cgccagcaga ggtccattcc gttcccagct gctcctgggg aggccgcttc 3300
ctggtaggag ggagtccacg agactctttt ctgtccctca ctgtgttccg ccgactgtcc 3360
cctctcgtca cccatcactc cctgcttccg cagggcgccc ctcagtattc gctgccatat 3420
gtccctgtcc tccaggctgt aggggaggag agcctggccg ggggagacag actggacatt 3480
tccctgtttc gagccaggct cttccaaggg gccagctggg tccccggagt cagtcctagg 3540
ctggatggga gggtggcccc ctcaagagga ctcccagcct ccacatctgg ttcctacctt 3600
cacatctcac cctcccgttc tggggaagaa tttctggttc ctacagtatc cactccatcc 3660
tcaaggcttc ccgcagggcc ttggggcact gccttgccat cgggcccagt tctccgggcc 3720
ccacctgcac ccctttcttc cccctgggat atgatgtgtg gtgtttcctg tggtaaaaga 3780
ctgaggcagg ccaggggtct gccagtaaca tgttcccatg tacagacacg gtccccacac 3840
cctcccagcc tcaggcccag gcagacatgg gcgagctggt gagactgcca gccacggctt 3900
tgcttagcca cctgtggccg agggctctca gagaccccct taacctccca aatactaaga 3960
agctaaaata ttttaatatt ttgttttttt ttttcttggt gccagagttt ataccctggg 4020

tgctggggtc gcactgtgtt atatatatat atatatatat ataatgtgta tatatatata 4080
ttctattttt tttggttggg ttcgttttta attcagtcct acaaaatggt ggtaagcccc 4140
agtctcaggg gtcaccctgt cttagagcta gagatgaggt ggcagggagg gaggctgtgt 4200
cctcagtggc ctggggctga gcagacctta ggaagggacc ctccccagtg ccgtggctgc 4260
tctggggcta ccccaggtga acagaggtgg tgagatgcgc agggaagacc agctctctcc 4320
aggacatcgg gctgcgtgaa gtccagtgac cctgagggga ttttggccaa aggaaacagc 4380
cagggctggg ctaggagagc tagggtcaaa agctgcccca cccactctcc ctcacatggt 4440
gctaggctgg gagctgccct gagagctggg atgcccacca aggcccacag aactgtctcc 4500
aggcccttgt agacagtggg atctctgggc tgatttctgg ttggggtctg ctcctacccc 4560
accccattta acaatgtgaa gtgactgagg ctggggtgcc tctccctggc cccctccaac 4620
tgccaaattc tgcttccctc tgaaccccag gaaatacttt ctggagaggc ttgtcttgtt 4680
cagaagactc tagcatcttg gggctggaag gtcctttgac gggtatgaaa caggtccaga 4740
aaggggaggt ggcctgcttg gccgacgtca tggagccagc tgggagcagg gctgagcgtt 4800
gaacccgagg ctcccggttc caagtcccat cgtgctcctc ttccaatgcc acactgcctc 4860
tgagcccatc cttggaccct actctgtgcc aggttcctgt cccttcctcc gtcacagcat 4920
ggtcattcca ggatatggga caaaccactc cttcccagcc agggtgcctt tgagccttct 4980
cattccgagg tctgcgtttg catccctgtc gtgttctccc tcgaggggtc cttccccagc 5040
ctgtgggggc ctgggtctgt tctgagctga ttcccttttc tccagttctc tgctgggatg 5100
aagaaagttg gacactcagt gggaccagct gcaggtggtt tcactgtgta cacagcagtg 5160
tccccatcat gcctctcaat ggggcctgca agcctgggaa cttgggcatc gttgggccag 5220
caggatgagc agcaaagagt ctgaggacag ggtggcatgt ctgcttgtgt gctcatgaag 5280
acatgcccac gccatggagc caggggctta cagagagggg cagtgacttc cagagtccca 5340
tccgtcagtg aggggactgg gctgcgggat gggtgaggat cttacagctc ccaggttcgg 5400
aaatctaccc tctaggcctg gaggcctctc tcgcagagca tggaaggcag gagaggtggg 5460
ctggcctagg gcaggagctc cacttctctc tgcctctggc tatgggaagg agcaggcggt 5520
tggaaatcct gtgacaacag agatcttctg gatttttgcc tggacccttc tgcaagcctg 5580
gagactggac ctgccacatg ttccaagtct gtctgcctag gtcactgctg ccacttctcc 5640
tcccactaac agacctttct tgaagaccca ctaggtccag acactgccgg gacttgcagg 5700
tatggggggt agaaggagag gcagataagc tgcctggggg gctgttagct aatgggtaca 5760
aggacacccc ctcccgagaa gagaagccac agatgagtgg gccagcgagg ccagtcttca 5820
atgtccacta attcactcta agcatttggc caaaaaaagg agagagaaat aactgaaatt 5880
gttcaggtgg ctccatcgtc tattctgtgg tcccctccat gagcctttgc cccagggtgg 5940
gtgcaggtgc gagtgtcacc tgcagctgcc tcagtcattc agagttccag agccttctgt 6000
ggtggagcat cctgctgctt ggaggatgag tctgaagttt aaaaatgggc cttttctgag 6060

cccccctgat gagcagtcag tgcttctggg cccagctaag gaagccatct gttcccagca 6120
gaggagagct gctgcgctga cctgcagagg ttggtggtcc cagcacagag ctgtgcccat 6180
gggcttgtta gtgggaaaag caaatacaac aatgttttac caaacattta attatgaatg 6240
cccactcccc agcatctaga tctaaaatta ttaacatttt gccacattct ttttctctct 6300
ctgtgtatgt atatgttttc tctgtcctaa tttaaaagta cattgcaaac atcatgaaac 6360
ttgatccctg aaagcttcag cctgcatctc ctaagagtta gggcattgtc ctacacgttg 6420
gcccagtatc acagccaaga cgagaagtca tttcccagcg atgtctgcta tctaatctgt 6480
attcaaatcc ccccaattgt ctcccaggta acttatgtgt ctggttttgg aatcagcatc 6540
cagtcacggt gcacacattg cattttctaa ctatgttttt aaaacctctt tgaatgctga 6600
aaggtcttcc ctcagctcac tttgagtttt taaatgacat tgtttttgag gccctaggcc 6660
tgctgtcttt ttgaagcccc acattctgaa tttgtcagat tgtttgctca tggtgtcgca 6720
taaccacttc ttctatcccc tgtactttca aggataattt aggctacatg agattttgcc 6780
ttttgtgctg acttgacagc ttcacttctg cctaagatgc gcctccactg tcacctgtac 6840
caagtgccaa aacagcagag gggccagtta gagcaagagc tctggggggg ccggaaagtc 6900
tccctggaga aggccgtaat atctgggcct tggaaggaag gacacagggc tctggaagga 6960
ggaggctgca tgcctgaagg ctgggccttc gaccctgggg atgggccaca gggcaccaaa 7020
cacatcatat gtctctctct ttctattttg gggtcccagg gttttcattt cctggagacc 7080
cgtggtctgt gtgcactgga gcagagccag gctctcagtt gcagcaggga aaagcctaca 7140
tggggaactg cctcccgcaa gctgtggtgt gagaagagcc ctggctggca gcaggagctg 7200
tgaagtttca tcccagccgg gtgcatttag tagctgcctg accatcttcc tcccaggccc 7260
tcagtttctt catctgtaga atgagcagat aggggccaca gcagggattt tcacacatca 7320
aaggaggcag cctctgctct agttgatgtg aggatgaggg ggcggcctag accccctgct 7380
gcccaggccg ttgaggcaac actgggttcc tggcttgaca gctgtgggct ggctgccctt 7440
gccagctcag acggctggcg agttttcagc ttccctaagg gactggcata gggttttgtg 7500
gggtcaatgc caggaacctg ggggaaactg gatgcgtttt atcctaagaa gttcatccct 7560
cctgctctta ctgtttccgg ccctggagga gaatgaggga ggagagggtt ttctatgact 7620
gtgttagctt ttattattag caagagggct ccttttgtaa tttgtgcatg aaattcatgt 7680
catttcctgg gagaggagag ggccgactgg agagggtggg gggcacactg gggaagcagg 7740
cagaggtttc cttcccttgt aggggggtgg ggggagtggg gagagggggg gctatccagt 7800
tcgcctgatg agggctctcg aaccagccgt tttgggttgt gttaaaggtg agaccttcct 7860
ccattaatgt acaatctcga actaactgct aataaagtgg ggttctgttt gta 7913

<210> 117
<211> 1943
<212> DNA
<213> Homo sapiens

<400> 117
cgggaagcgc ggcggggcct ccagaccggg gcgggcttaa gggtgacatc tgcgctttaa 60
agggtccggg tcagctgact cccgactctg tggagtctag ctgccagggt cgcggcagct 120
gcggggagag atgactgggg agcgacccag cacggcgctc ccggacagac gctgggggcc 180
gcggattctg ggcttctggg gaggctgtag ggtttgggtg tttgccgcga tcttcctgct 240
gctgtctctg gcagcctcct ggtccaaggc tgagaacgac ttcggtctgg tgcagccgct 300
ggtgaccatg gagcaactgc tgtgggtgag cgggagacag atcggctcag tggacacctt 360
ccgcatcccg ctcatcacag ccactccgcg gggcactctt ctcgcctttg ctgaggcgag 420
gaaaatgtcc tcatccgatg agggggccaa gttcatcgcc ctgcggaggt ccatggacca 480
gggcagcaca tggtctccta cagcgttcat tgtcaatgat ggggatgtcc ccgatgggct 540
gaaccttggg gcagtagtga gcgatgttga gacaggagta gtatttcttt tctactccct 600
ttgtgctcac aaggccggct gccaggtggc ctctaccatg ttggtatgga gcaaggatga 660
tggtgtttcc tggagcacac cccggaatct ctccctggat attggcactg aagtgtttgc 720
ccctggaccg ggctctggta ttcagaaaca gcgggagcca cggaagggcc gcctcatcgt 780
gtgtggccat gggacgctgg agcgggacgg agtcttctgt ctcctcagcg atgatcatgg 840
tgcctcctgg cgctacggaa gtggggtcag cggcatcccc tacggtcagc ccaagcagga 900
aaatgatttc aatcctgatg aatgccagcc ctatgagctc ccagatggct cagtcgtcat 960
caatgcccga aaccagaaca actaccactg ccactgccga attgtcctcc gcagctatga 1020
tgcctgtgat acactaaggc cccgtgatgt gaccttcgac cctgagctcg tggaccctgt 1080
ggtagctgca ggagctgtag tcaccagctc cggcattgtc ttcttctcca acccagcaca 1140
tccagagttc cgagtgaacc tgaccctgcg atggagcttc agcaatggta cctcatggcg 1200
gaaagagaca gtccagctat ggccaggccc cagtggctat tcatccctgg caaccctgga 1260
gggcagcatg gatggagagg agcaggcccc ccagctctac gtcctgtatg agaaaggccg 1320
gaaccactac acagagagca tctccgtggc caaaatcagt gtctatggga cactctgagc 1380
tgtgccactg ccacaggggt attctgcctt caggactctg ccttcaggaa cacgggtctg 1440
tagagggtct gctggagacg cctgaaagac agttccatct tcctttagac tccagccttg 1500
gcaaaatcac cttcccttta ccagggaaat cacttccttt aggactgaaa gctaggcgtc 1560
ctctcccaca aaaaagtcct gccctcatct gagaatactg tctttccata tggctaagtg 1620
tggccccacc accctctctg ccctcccggg acattgattg gtcctgtctt gggcaggtct 1680
agtgagctgt agaattgaat caatgtgaac tcagggaact ggggaaggct gagcctcctc 1740
tttggtgttg cggtaagata accgacaggg ctggtgaaag tccccagatg gcaggatatt 1800
tggtttcaga gtaaggacta ggtgcaccac catgactgac tatcaatcaa aatgtttgta 1860
acttaaaatt tttaatgaag gataatgaat atttgtagag tctctatggt tctgtcaatg 1920
caaaaaaaaa aaaaaaaaaa aaa 1943

<210> 118
<211> 1143
<212> DNA
<213> Homo sapiens

<400> 118
atggcgtccc ttcctgtcct gcagaaggag agcgtgttcc agtcgggagc ccatgcctac 60

agaatccctg ccctgctcta cctgcctggg cagcagtccc tgctggcctt cgcggaacag 120

cgggcaagca agaaggatga gcacgcagag ctgattgtcc tgcgcagagg agactacgac 180

gcacccaccc accaggttca gtggcaagct caggaggtgg tggcccaggc ccggctggat 240

ggccaccggt ccatgaaccc atgccccttg tatgacgcgc agacggggac cctcttcctc 300

ttcttcattg ccatccctgg gcaagtcacg gagcaacagc agctgcagac cagggccaat 360

gtgacgcggc tgtgccaagt caccagcact gaccacggga ggacctggag ctcccccaga 420

gacctcactg atgcggccat cggcccagcc taccgggagt ggtccacctt tgcagtgggc 480

ccggggcatt gtttgcagct taacgacagg gcccggagcc tggtggtgcc cgcctacgcc 540

taccggaaac ttcaccccat ccaaaggccg atcccctctg ccttctgctt cctcagccat 600

gaccatgggc gcacgtgggc gcgagggcac tttgtggccc aggacaccct ggagtgccag 660

gtggccgaag tcgagactgg ggagcagagg gtggtgaccc tcaacgcgag aagccacctc 720

cgagccaggg tccaggccca gagcaccaat gacgggcttg atttccagga gtctcagctg 780

gtgaagaagc tggtggagcc gccgccccag ggctgccagg ggagcgtcat cagcttcccc 840

agcccccgct cggggcctgg ctccccagcc cagtggctgc tctacactca ccccacacac 900

tcctggcaga gggccgacct gggtgcctac ctcaacccgc gacctccagc ccctgaggcc 960

tggtcagagc cggtactgct ggccaagggc agctgtgcct actcagacct ccagagcatg 1020

ggcaccggcc ctgatgggtc ccccttgttt gggtgtctgt acgaagccaa tgattacgag 1080

gagattgtct ttctcatgtt caccctgaag caagccttcc cagctgagta cctgcctcag 1140

tga 1143

<210> 119
<211> 2748
<212> DNA
<213> Homo sapiens

<400> 119
cttttcgttg ccgttacctg tttccggcag tcgacacgct cttcgcttct cggggcttgt 60

ctccgtgtcc tccgtctcag ttgtttctcc ctctctatcc tcctctgtct cagtctcccc 120

agccttgggg ccggtgcctc ttccgggctt cggcgaatga gacctgcgga cctgcccccg 180

cgccccatgg aagaatcccc ggcgtccagc tctgccccga cagagacgga ggagccgggg 240

tccagtgcag aggtcatgga agaagtgaca acatgctcct tcaacagccc tctgttccgg 300

caggaagatg acagagggat tacctaccgg atcccagccc tgctctacat accccccacc 360

cacaccttcc tggcctttgc agagaagcgt tctacgagga gagatgagga tgctctccac 420

ctggtgctga ggcgagggtt gaggattggg cagttggtac agtgggggcc cctgaagcca 480

ctgatggaag ccacactacc ggggcatcgg accatgaacc cctgtcctgt atgggagcag 540
aagagtggtt gtgtgttcct gttcttcatc tgtgtgcggg gccatgtcac agagcgtcaa 600
cagattgtgt caggcaggaa tgctgcccgc ctttgcttca tctacagtca ggatgctgga 660
tgttcatgga gtgaggtgag ggacttgact gaggaggtca ttggctcaga gctgaagcac 720
tgggccacat ttgctgtggg cccaggtcat ggcatccagc tgcagtcagg gagactggtc 780
atccctgcgt atacctacta catcccttcc tggttctttt gcttccagct accatgtaaa 840
accaggcctc attctctgat gatctacagt gatgacctag gggtcacatg gcaccatggt 900
agactcatta ggcccatggt tacagtagaa tgtgaagtgg cagaggtgac tgggagggct 960
ggccaccctg tgctatattg cagtgcccgg acaccaaaca ggtgccgggc agaggcgctc 1020
agcactgacc atggtgaagg ctttcagaga ctggccctga gtcgacagct ctgtgagccc 1080
ccacatggtt gccaagggag tgtggtaagt ttccggcccc tggagatccc acataggtgc 1140
caggactcta gcagcaaaga tgcacccacc attcagcaga gctctccagg cagttcactg 1200
aggctggagg aggaagctgg aacaccgtca gaatcatggc tcttgtactc acacccaacc 1260
agtaggaaac agagggttga cctaggtatc tatctcaacc agaccccctt ggaggctgcc 1320
tgctggtccc gcccctggat cttgcactgt gggccctgtg gctactctga tctggctgct 1380
ctggaggagg agggcttgtt tgggtgtttg tttgaatgtg ggaccaagca agagtgtgag 1440
cagattgcct tccgcctgtt tacacaccgg gagatcctga gtcacctgca gggggactgc 1500
accagccctg gtaggaaccc aagccaattc aaaagcaatt aattggctta ggacccaatt 1560
tccatagatg caaatggcag ttacagacag gttaacagaa gctactgaag tctacagata 1620
atcaaaaaac ttaatattct gttccctacc ttttttcact tttcctcctc caaagagcaa 1680
aatgaaaatt ttgccttagc tactgcagtg gaaagagcac tgaactagga gttggaagac 1740
aaggatgtgg tcctggctct gccactggct tgcttttgga ccttggatgt gtcacctgaa 1800
ctctctggac ctcaggtttc catctgtaaa atgagagtat tggttctaag atttctcatc 1860
ttctcatccc taggacaagc atagtgcctg catgcttcat gatcagtaag tcctggctgc 1920
ataaaggact ctgatgtcaa aatggaaacc aggggactta ccttttcaca tgacttaccc 1980
ctcatccgag tgtgaggtta caagcaggtg tcatggcagg aaggaagacc agatctgtat 2040
gatttgttcc atttttaata acaaaaatat ccacaccctt ttaataatgc tcagttctgt 2100
aggctctcta tcctagagga attgagcaaa acagaagaat catgaagtct cctaccttct 2160
acagccttgt agttctgctt accttctctt cctcatccag aaagcatcat tttctaggga 2220
gaacaatgag aatctcaatg ccagtagtac tggataatag tgcgtattgc ttctggtggc 2280
attaccctga tgatgggctg aagttcattt attagggtgg ttcctgatgg gaaaaggaca 2340
tggattagga ctttaaaaca ctggacagaa tttcccacag tctttgccct caaggagttc 2400
accagtttat ggggctagaa gagcgagaaa attcaagaaa ataaatgtag ctggtgggag 2460
actttgtaga tgttgggcta tatgttgggg tgatggtagc tcctgatgta attttcttag 2520

ttgcatcttc aatatgcctg gagtcgtctg tccaaggctt gtccaggctt ctgggtttct 2580

ctcaagtttg tttttctcag gatattgtcc tggcccagct actcctttac ctgtgagaag 2640

atcttcacca ttaggaagat ctctagaccc ccagatctca gaatcaggcc tatttgtgta 2700

ggcccatgga aatcactact tgtgaagtag agatgccttt ttgtctaa 2748

<210> 120
<211> 2276
<212> DNA
<213> Homo sapiens

<400> 120
gaacgtggta taaaaggggc gggaggccag gctcgtgccg ttttgcagac gccaccgccg 60

aggaaaaccg tgtactatta gccatggtca accccaccgt gttcttcgac attgccgtcg 120

acggcgagcc cttgggccgc gtctcctttg agctgtttgc agacaaggtc ccaaagacag 180

cagaaaattt tcgtgctctg agcactggag agaaaggatt tggttataag ggttcctgct 240

ttcacagaat tattccaggg tttatgtgtc agggtggtga cttcacacgc cataatggca 300

ctggtggcaa gtccatctat ggggagaaat ttgaagatga gaacttcatc ctaaagcata 360

cgggtcctgg catcttgtcc atggcaaatg ctggacccaa cacaaatggt tcccagtttt 420

tcatctgcac tgccaagact gagtggttgg atggcaagca tgtggtgttt ggcaaagtga 480

aagaaggcat gaatattgtg gaggccatgg agcgctttgg gtccaggaat ggcaagacca 540

gcaagaagat caccattgct gactgtggac aactcgaata agtttgactt gtgttttatc 600

ttaaccacca gatcattcct tctgtagctc aggagagcac ccctccaccc catttgctcg 660

cagtatccta gaatctttgt gctctcgctg cagttccctt tgggttccat gttttccttg 720

ttccctccca tgcctagctg gattgcagag ttaagtttat gattatgaaa taaaaactaa 780

ataacaattg tcctcgtttg agttaagagt gttgatgtag gctttatttt aagcagtaat 840

gggttacttc tgaaacatca cttgtttgct taattctaca cagtacttag atttttttta 900

ctttccagtc ccaggaagtg tcaatgtttg ttgagtggaa tattgaaaat gtaggcagca 960

actgggcatg gtggctcact gtctgtaatg tattacctga ggcagaagac cacctgaggg 1020

taggagtcaa gatcagcctg ggcaacatag tgagacgctg tctctacaaa aaataattag 1080

cctggcctgg tggtgcatgc ctagtcctag ctgatctgga ggctgacgtg ggaggattgc 1140

ttgagcctag agtgagctat tatcatgcca ctgtacagcc tgggtgttca cagatcttgt 1200

gtctcaaagg taggcagagg caggaaaagc aaggagccag aattaagagg ttgggtcagt 1260

ctgcagtgag ttcatgcatt tagaggtgtt cttcaagatg actaatgtca aaaattgaga 1320

catctgttgc ggtttttttt tttttttttt cccctggaat gcagtggcgt gatctcagct 1380

cactgcagcc tccgcctcct gggttcaagt gattctagtg cctcagcctc ctgagtagct 1440

gggataatgg gcgtgtgcca ccatgcccag ctaatttttg tatttttagt atagatgggg 1500

tttcatcatt ttgaccaggc tggtctcaaa ctcttgacct cagctgatgc gcctgccttg 1560

gcctcccaaa ctgctgagat tacagatgtg agccaccgca ccctacctca ttttctgtaa 1620
caaagctaag cttgaacact gttgatgttc ttgagggaag catattgggc tttaggctgt 1680
aggtcaagtt tatacatctt aattatggtg gaattcctat gtagagtcta aaaagccagg 1740
tacttggtgc tacagtcagt ctccctgcag agggttaagg cgcagactac ctgcagtgag 1800
gaggtactgc ttgtagcata tagagcctct ccctagcttt ggttatggag gctttgaggt 1860
tttgcaaacc tgaccaattt aagccataag atctggtcaa agggataccc ttcccactaa 1920
ggacttggtt tctcaggaaa ttatatgtac agtgcttgct ggcagttaga tgtcaggaca 1980
atctaagctg agaaaacccc ttctctgccc accttaacag acctctaggg ttcttaaccc 2040
agcaatcaag tttgcctatc ctagaggtgg cggatttgat catttggtgt gttgggcaat 2100
ttttgtttta ctgtctggtt ccttctgcgt gaattaccac caccaccact tgtgcatctc 2160
agtcttgtgt gttgtctggt tacgtattcc ctgggtgata ccattcaatg tcttaatgta 2220
cttgtggctc agacctgagt gcaaggtgga aataaacatc aaacatcttt tcatta 2276

<210> 121
<211> 2940
<212> DNA
<213> Homo sapiens

<400> 121
gcggcccttc tcccctcccc tctcagttcc ctccgccctc ctcgggctcc agcggtggcg 60
gagcgaacgg gaccggcccg gcttcagagc gcgaggtgga gggtggaacg cgggcgcctg 120
aaggagttgt tgtctcggca gcgcccgcgg agacgtgaag aggtggttgt gggaagagaa 180
gtttgcagaa ctgaaatgga ggtcagagct tcattacaga aggttagtgg atcatctgat 240
tctgtggcta caatgaacag tgaagaattt gttttggttc ctcagtatgc agatgataat 300
tctacaaaac atgaagaaaa acctcaactg aagatagttt ctaatggtga tgaacaattg 360
gaaaaagcca tggaagagat tttgagagat tccgagaaaa ggccaagcag tcttcttgtt 420
gattgtcaaa gttccagtga gatttcagac cattcgtttg gagatattcc agccagccaa 480
acaaataagc catctcttca gttaattttg gatccgtcta acacagaaat ttctacaccc 540
agaccatctt ctccaggtgg actacctgaa gaagatagtg ttttatttaa taaactgacc 600
tacttaggat gtatgaaggt ttcttcccca cgtaatgaag tagaggcttt acgggcaatg 660
gcaaccatga aatcttccag tcaatacccc tttcctgtta ccctgtatgt accaaatgtt 720
ccagaaggtt ctgtgagaat tatagaccaa tccagcaatg tggagatagc atcttttcca 780
atctataagg tgttattctg tgcacgtgga catgacggaa caacagagag caattgcttt 840
gcatttacag agagttccca tggttcggaa gaatttcaga tacatgtttt ctcctgtgaa 900
attaaagagg cagtaagcag aattttgtac agtttctgta cagcattcaa acgttcttcc 960
agacaagtgt ctgatgttaa agactcagtt attcctaccc ccgacagtga tgtgtttacc 1020
ttcagtgtct ccttggaggt aaaagaagac gatggaaaag gaaactttag ccctgtgcct 1080
aaggatagag ataaatttta tttcaaatta aagcaaggaa tagagaagaa ggttgtgatt 1140

acagtgcagc aactttctaa caaagaatta gctattgaaa gatgttttgg aatgttatta 1200
agcccaggtc gaaacgtgaa gaacagtgac atgcatttac tggatatgga atccatggga 1260
aagagctatg atgggagagc ttatgtcatc actggcatgt ggaaccccaa tgcaccagta 1320
tttctggcac ttaacgagga aaccccaaaa gataagcaag tatacatgac tgtggcagtg 1380
gatatggtag tcacagaggt ggtggagcct gttcgctttc tcctggagac agtagtccgt 1440
gtgtaccctg caaatgagcg attttggtat ttcagcagaa agactttcac agagactttc 1500
ttcatgagat tgaaacagtc tgagggaaaa ggccatacca atgctggaga tgcaatatat 1560
gaggtggtga gtctacagcg agagtctgac aaggaggaac cagtcactcc tactagtgga 1620
gggggtccaa tgtcacccca ggatgatgaa gcagaagagg agagtgataa tgaactctca 1680
agtggaacag gtgatgtgtc taaggattgt cctgagaaga tcctgtattc ttggggagag 1740
ttgctaggaa aatggcacag taaccttggt gcacgaccga aagggctgtc tactctggtg 1800
aagagtggtg tccctgaagc attgagggca gaggtatggc agttattggc aggctgccat 1860
gacaaccagg caatgctgga tagataccga attcttatca caaaggactc agcccaggag 1920
agtgttatta ctcgagatat tcatcgtaca tttcccgcac atgattactt taaagatact 1980
ggaggagatg gtcaagaatc gctctataag atctgcaagg cctactctgt gtatgatgaa 2040
gacattgggt actgtcaagg gcagtctttt cttgctgctg tattactgct gcatatgcca 2100
gaggaacaag cattctgtgt tttggtgaaa atcatgtacg actatggttt gagagacctc 2160
tacagaaaca acttcgaaga tcttcattgc aaattctacc agttggagag actaatgcag 2220
gaacagctac cggacctgca tagccatttt tctgatctga acctggaagc tcatatgtat 2280
gcatcccagt ggtttctcac tctttttact gccaagttcc cactctgcat ggtgttccac 2340
atcattgact tactgctttg tgagggtttg aacataatct ttcatgtagc tttggctctc 2400
ctaaagacct caaaggaaga ccttctgcag gctgattttg aaggtgcttt aaagttcttt 2460
agagttcagc ttccaaagag atacagggca gaggaaaatg caagaagact gatggagcag 2520
gcttgcaata ttaaagtacc aaccaagaag ctgaagaaat atgagaaaga atatcagaca 2580
atgcgagaga gtcagctgca acaggaagac ccaatggata gatacaagtt tgtatatttg 2640
taggtaactc cagctgttgc atttatactg ggaatcttca taagaagctg agagaaagag 2700
aggggaaaaa gaaagtggct ttctactttc aaaaatgaaa caaaaaggaa aaatggcaaa 2760
gtactgtttt agctgtgcat gtcatatcca caaagacttt tagcaggtga actgttccaa 2820
gactgacaca aggatgtttc aaacttgcct ctgtctgtag aaaatgttaa aaataccaac 2880
tcacttggaa ggaaaaataa aaatcacaaa ggtatattga gcacagtaaa aaaaaaaaaa 2940

<210> 122
<211> 8207
<212> DNA
<213> Homo sapiens

<400> 122

agacgctcgc gagcaaccct ccgcgctccg gagcctcctc agcctcccgc acaccccgag 60
acgctcgccc cgctgggcct cggtccccgc ctcctccccc gtccttttcc cctctctcgc 120
tccccacatt gtctctgcct cattttctct cctagttgag gattaaaaaa aaaaaaaaat 180
caccatcatc ctcagggagc cccctcgccc tcggaaaaca aaaccagaca tgatcctggg 240
ctcctgcttc tgaaagatct gcccgaccga ggaggaggga gcctaggttc aagtcgcagg 300
aaaggtcacg gcagggaagc cccctcagcc cctccgcgtc gcctccccgc gggccgcccc 360
tctcggccct gcgccccgcg gccccgcagc ctccgcgcgg cctcccgccc catccccgcc 420
actttccccg gagctgggcc gcgaacacct cactattgat ggtgtagcgc tttaggggaa 480
ggaggtgatt gtctaggagt aactgtatgt gcgctcagca ccgggtcaca accaccgtgg 540
gatccactta cagagaagat ttgtggagag cttcagcctg tttatggtgt tactgagaaa 600
ggaattgtgg ccatttgata taattctgga tggaagacct cttatttgta ttacattgaa 660
ttagagcatt tttgaaacgt tggggttgtt ggagtggttg gattttccct ggaattgagt 720
gagaaattca gaagactgaa gcccaggctt actgtctacc tttcacggag gcctagccgt 780
gagaggacag aagaaggcac gtggcgaatc atgacagcgg acaaagacaa agacaaagac 840
aaagagaagg accgggaccg agaccgggac cgagagagag agaaaagaga caaagcaaga 900
gagagtgaga attcaaggcc acgccggagc tgtaccttgg aaggaggagc caaaaattat 960
gctgagagtg atcacagtga agacgaggac aatgacaaca atagtgccac cgcagaggag 1020
tccacgaaga agaataagaa gaaaccaccg aaaaaaaagt ctcgttatga aaggacagat 1080
accggtgaga taacatccta catcactgaa gatgatgtgg tctacagacc aggagactgt 1140
gtgtatatcg agagtcggag gccaaacaca ccgtatttca tctgtagcat tcaagacttc 1200
aaactggtcc acaactccca ggcctgttgc agatctccaa ctcctgcttt gtgtgacccc 1260
ccagcatgct ctctgccggt ggcatcacag ccaccacagc atctttctga agccgggaga 1320
gggcctgtag ggagtaagag ggaccatctc ctcatgaacg tcaaatggta ctaccgtcaa 1380
tctgaggttc cagattctgt gtatcagcat ttggttcagg atcgacataa tgaaaatgac 1440
tctggaagag aacttgtcat tacagaccca gttatcaaga accgagagct cttcatttct 1500
gattacgttg acacttacca tgctgctgcc cttagaggga agtgtaacat ctcccatttt 1560
tctgacatat ttgctgctag agagtttaaa gcccgagtgg attcattttt ctacatatta 1620
ggatataacc ctgagacaag gaggctgaac agtacccagg gggagattcg tgtcggtcct 1680
agtcatcagg ccaaacttcc agatctgcaa ccatttcctt ctccagatgg tgatacagtg 1740
acccaacatg aggaactggt ctggatgcct ggagttaacg actgtgacct ccttatgtac 1800
ttgagggcag caaggagcat ggcggcattt gcaggaatgt gtgatggagg ctctacagag 1860
gacggctgtg tcgcagcctc tcgggatgac accactctga atgcactgaa cacactgcat 1920
gaaagcggtt acgatgctgg caaagccctg cagcgcctgg tgaagaagcc tgtgcccaag 1980
ctcatcgaga agtgctggac cgaggacgaa gtgaaacgct tcgttaaggg actcaggcag 2040

tacgggaaga acttcttcag aattagaaag gagctgcttc ccaataagga aacaggggag 2100
ctgatcacct tctattacta ttggaagaag acccccgaag cagccagctc ccgagcccat 2160
cgtaggcacc gcaggcaggc cgtgttcagg aggattaaga ctcgcaccgc gtccacaccc 2220
gtcaacacac cctccagacc cccgtccagt gaattcttgg acctaagttc agccagtgaa 2280
gatgacttcg acagtgagga cagtgagcag gagctgaagg ggtacgcctg ccgccactgc 2340
ttcaccacca cctccaaaga ttggcaccac ggaggccggg agaacatcct gctttgcacc 2400
gactgtcgca tccacttcaa gaaatacggt gagctcccgc ccattgagaa gcccgtggac 2460
ccgccaccgt ttatgttcaa acccgtcaag gaagaggatg atgggctcag tgggaagcat 2520
agcatgagga cacggcggag tcggggctcg atgtcgacac tacgcagtgg tcggaagaag 2580
cagccagcca gccctgatgg tcgcacctca cccatcaatg aagacatccg ctccagcggc 2640
cggaactccc ccagcgctgc cagtacctcc agcaatgaca gtaaagcaga gacagtgaag 2700
aagtcggcca agaaggtgaa ggaggaagcc tcttcccctc ttaagagtaa caaacgccag 2760
cgggagaagg tggcctctga tacggaggag gctgacagga ccagctccaa gaagacaaaa 2820
acgcaggaga tcagcaggcc caactcgcca tctgaaggtg agggagagag ttcagacagt 2880
cgcagcgtca acgatgaggg tagcagtgac cccaaagaca tcgaccagga caatcgcagc 2940
acgtccccga gcatccccag cccccaggac aatgagagtg actcggactc gtcagcccag 3000
cagcagatgc tgcaggccca gcccccagcc ttgcaggctc ccactggggt caccccagct 3060
ccctcctcag ctcctccagg gacccctcag ctgcccacgc cagggcccac gccctctgcc 3120
actgcagttc ccccacaggg ctcccccacg gcctcccagg cccctaacca gccacaggct 3180
cccacagcgc ctgttcccca cacccacatc caacaggcac cggccttgca cccccagcgg 3240
ccgccctcac cgcatccccc gccgcatccc tcgccacatc ccccgctgca gcctctgact 3300
gggtcggcgg gccagccttc tgcaccctct catgcccagc ccccactgca cggtcagggc 3360
ccacccggcc ctcacagcct gcaggctggg cccctgctgc agcacccagg ccccccacag 3420
ccctttggcc tccctcccca ggcctcccaa ggccaggccc ctctggggac ctccccagca 3480
gcagcgtacc ctcacacctc cctgcagctg ccagcctctc agtcagcgct gcagtcccaa 3540
cagcctccac gggagcagcc cctgccacca gcgcccttgg ccatgcccca catcaagccc 3600
ccgcctacca ctcccatccc ccagctgccg gcgccacagg cccacaagca ccctccccac 3660
ctctcggggc cctcaccctt ctccatgaat gccaacctgc ctccccctcc agccctgaag 3720
cccctgagct ccctgtccac acatcacccc ccgtcggctc acccccaccc cctgcaactc 3780
atgcctcaga gccagccatt gccctcctcg cccgcccagc cccccgggct gacccagagc 3840
cagaacctgc ccccgccccc tgcctcccac ccccctacag gcctccacca ggtggccccc 3900
caacccccgt ttgctcagca cccctttgtc cctggaggcc ctcctcccat cacccctccg 3960
acctgcccct ccacctctac cccaccggcg ggacctggca cctcggccca gccaccctgc 4020
tctggtgcgg cggcttcagg aggcagcata gcgggggggt cgtcctgccc actccccacc 4080

gtccagatca aggaggaggc tctggacgac gctgaggagc ctgagagccc ccctccccca 4140
ccaaggagcc cgtccccgga gcccactgtg gtggacaccc ccagtcacgc cagccagtca 4200
gctaggttct acaaacacct ggaccggggc tacaactcgt gtgcccggac agacctgtac 4260
ttcatgcctc tggccgggtc caagctggcc aagaagaggg aggaggccat tgagaaggcc 4320
aagcgcgagg ctgagcagaa agcccgagag gagcgagagc gggagaagga gaaggagaag 4380
gagcgggagc gggagcgaga gcgggagcgc gaggcagagc gggcggctaa ggcgtccagc 4440
tcagcgcatg aaggtcgcct cagtgaccca cagctcagtg gtcctggcca catgcggcca 4500
tccttcgagc caccaccaac caccattgct gctgtgcccc cctacatcgg gcccgacaca 4560
cctgcccttc ggactctgag cgagtacgcc cggccccacg tcatgtcgcc caccaaccgc 4620
aaccacccct tctacatgcc ccttaacccc acggaccccc tgctggccta ccacatgcct 4680
ggcctctaca acgtcgaccc caccatccgc gagcgggagc tccgggagcg ggagatccga 4740
gagcgggaga tccgagagcg ggagctgcgg gagaggatga agccgggctt cgaggtgaag 4800
cccccagagc tggaccccct gcacccagcc gccaacccca tggagcactt tgcccggcac 4860
agcgccctca ccatcccccc gaccgccggg ccccaccctt ttgcttcttt ccacccgggc 4920
ctgaacccct tggagaggga gagactggcc ctggcgggcc cccagctgcg gcccgagatg 4980
agctaccctg acagactggc agccgagcgt atccacgcag agcgcatggc atcgctgacc 5040
agcgatcccc tggcccgact gcagatgttc aacgtgactc cgcaccatca ccagcactct 5100
cacattcact cccacctcca cctccaccag caggaccccc tccaccaagg ttcagcaggc 5160
cccgttcacc cgctggtcga ccccctgact gccggtcccc acctggctcg cttcccctac 5220
ccgcctggca ctctccccaa ccctctgctt ggacagcccc cacacgagca cgagatgctt 5280
cgccacccag ttttcggcac cccctacccc cgtgacctgc ctggggccat cccacccccc 5340
atgtcagcag cccaccagct gcaggccatg catgcccagt cggccgagct gcagagactg 5400
gccatggagc agcagtggct gcatggacac ccccacatgc atggtggcca cctaccaagt 5460
caggaagatt attacagtcg actgaagaaa gaaggtgaca agcagttata agttatttat 5520
ttgttaacgc tggctgtgga aaccccagtt cttgggggga gaaacaggac tttttacata 5580
aaataggagc tgcaaaagca aaaagaatat cttctaaaga tttctttata tatttaaaaa 5640
cccacaacta aaaatgtatc cacatagtag tgttcgtttg tcgagaggat ttcctgagac 5700
tggtttggat ctccctgcat gacagtcccc cagaaactta gtgagtcctg gactggactg 5760
aacatccaga aagcttccct gcaatcttgg ggtttggctt tagttttctt ttccttgatt 5820
tctcagtagg tgctagaatc cagttcacac ccttcactgt gcgtgcagac acactgacac 5880
actccgccac gagtgctcca gagcccacga ggcttgcaga tcgggggcat aggaatttgg 5940
aatccaagag ctataatttt taaaaaaaaa aatcttttat tttaatacat tgtaggaaat 6000
cttcataatt ggagaaagtt ctgcagcatg gctttttacg tctgtaaata aataatttta 6060
gaacagcctt tttttcttcc ataaactact attgtgatct attttttcca gccatgtcac 6120

taattgtgaa ttcctaccaa ctattgacag aatacagagt tgatttttta ataaaaagtt 6180
atatataatt atccctttaa ttaaagggag caaaggggcg ttccacatgg acagaggctt 6240
ggaccgaggc ctggtcacag cagcgagcat ccagggtttg cagggacgat gttacagact 6300
ctgttttctg cctggcgttt cacttgtgtc tgctcctagc ctgtgctctg ccagcagcac 6360
agacatctgc tccatcagac ctcttccatt ttgcacaggg agtgcaggag gtgaatgttc 6420
actttctgtt ctccagtgtc actgttctgt ttccacggga tggaaagcgc atgggcctgt 6480
gtccattgta gatttccttc tagatttctg tgtacacaca cttgattgtt ctggatgaat 6540
gtctttttta atactccgaa aatttcatca tctaagaaaa tgattccata caaataactc 6600
agcacacaag tgacccagga catatgcctg ccaaagggat gtgttagaag gctgccttct 6660
catgcgcatt gtcacttgga tcttgtggtg aggacggccc catctttctt gccacagatt 6720
gaggccactt ttgagcaagg gagatcctgg agttaagaca ggtgttgggg gcagcctgta 6780
ttttacccta ggggcaggtc tgcatggtga ccccacattg cactggtaaa ccatttgagt 6840
cccactcttc atcctggaag tgggaactgg agtcccaccc acagtgcatt cagaaagcat 6900
gctgtgtggg ggctgcttct caggaggcca ggcccttctg agcggaaccg tcctggagag 6960
agcctgccct cgtttccagg ctgcagccgt aacgcacttt ctcccaggct gagggcgggt 7020
gttctggggt gtctgccctc tgtcggccct gcttcctgcc aggacgtggc ctcttccgat 7080
ccttttctct cagacactgg aggtctcttc tgccattgtg ctggtcccat cccaagaatt 7140
gtaggacaga gaccacactg ggtcggcgga cacaaagtcc atccaggacc caggccgcag 7200
agggagcagg aagagatgct gatagtttga tctagaaacc agcagctact ggctcaaatt 7260
caggttctgg cgtcaaatag cgacatttcc agtttctctt aaaaaccgtg tttggtttca 7320
gttgggatag gcttgttttg tctgttgaaa atgtttctag ttttttttct ttcatttttc 7380
tctcattcca tttctgcctt aactttagtt tgttcacagg gaggcaaagc tgacatgaac 7440
cttttgtcgt gggacttcag gccacattgg cttgaaggca ttcgtttcct tctggggtgg 7500
ggacaggccc tcatggcagg cttgttcccg tggctctgag cgaggcctct tcctgctggg 7560
ctcccagact cctgcatcca ggcccccacc ttctcggctt ctggtttttc tttctttttg 7620
gtagaacaca acatctacca ttcagttaaa ccttctttat ctcctccttt ggcatccatt 7680
tttccaaaga agagtcgagt cctctgaggt ctgtgcttga aagccgtccg aaggcattct 7740
tgttagcttt gcttttctcc ccatatccca aggcgaagcg ctgagattct tccatctaaa 7800
aaaccctcga cccgaaaccc tcaccagata aactacagtt tgtttaggag gccctgacct 7860
tcatggtgtc tttgaagccc aaccactcgg tttccttcgg attttcctcc ctttgttcgg 7920
ggtttggttt ggctcctctg tgtgtgtccg tatcttgttc ggtgtcctcg aggttgagct 7980
tcactccact gcggcagagg cagcgtgcac actcggattt gctacgtttc tatatatctt 8040
gaagctaaat gtatatatga gtagtttgcc atgagataac acagtgtaaa cagtagacac 8100
ccagaaatcg tgacttctgt gttctctcca tttgagtatt ttgtaatttt tttgaaatat 8160

ttgtggacat aaataaaacc aagctacact acaaaaaaaa aaaaaaa 8207

<210> 123
<211> 1805
<212> DNA
<213> Homo sapiens

<400> 123
gcagatggtg ctgtcgacca gtgtcaagac caagtttcat gggttcctgg acgctcagcc 60

aggggtagtc tctgaagggc cgcctggcaa ttgggagcag aagccagttt cccgccatct 120

gctctccgtg gaggcagtgc tctcgcggct gctgcccgtt ggggtccagc tgaggaagga 180

gcgcggatcc caggctcgtt ctttgcctgg gctgcccctg cggcccctgg ggcgggtgct 240

gctgcggcgc cagctccaag ggcggatcca ggcgggaggg gcctcctcgg agaagcgggg 300

cgcggtccca actacgcaga ggctggcacg ccgaccctcc acacctcacc acgcccccat 360

ctccgtccgt gtacacacac tcacacaagg acgccaaccc cacctagatg caaagcagga 420

ttcaaaagaa catctttgcg ttttctaccg gctccccatc atcgtactag ggaggaagaa 480

gcgggtgaga aacaaaactt ctttccattg tcctgcccgt ttctgcggac ttgttctgag 540

gccgaggcac ctctaagata ctgatggctc tgcagaggac ccattcattg cttctgcttt 600

tgctgctgac cctgctgggg ctggggctgg tccagccctc ctatggccag gatggcatgt 660

accagcgatt cctgcggcaa cacgtgcacc ctgaggagac aggtggcagt gatcgctact 720

gcaacttgat gatgcaaaga cggaagatga ctttgtatca ctgcaagcgc ttcaacacct 780

tcatccatga agatatctgg aacattcgta gtatctgcag caccaccaat atccaatgca 840

agaacggcaa gatgaactgc catgagggtg tagtgaaggt cacagattgc agggacacag 900

gaagttccag ggcacccaac tgcagatatc gggccatagc gagcactaga cgtgttgtca 960

ttgcctgtga gggtaaccca caggtgcctg tgcactttga cggttagatg ccaccatgta 1020

gggattatcg cgagtggttg accttacact tactccttaa atagcagtga gtaatgcatt 1080

tgagctgtcc caggctctgt ctcctcagct catttcctac tctttttctc tatataactc 1140

attctattaa atacattgca ccaaagagat atggagacat aaacctgtaa tgaatgaggc 1200

tgggcttttc tgtaataagc ttccttttat aatactggtc agcttagctc tctcagatcc 1260

tatcctgtgg aatttagtta ttatgtgtat ttatgtagta tttcaaacat ttcaaaatgc 1320

tttcatctat gtttatcaca ttttaatacc acagcactta taatgatgtc actacatata 1380

gaagctcaaa gttaagggat ttgctgaaga ctgtaaagtt aatggaagaa ttgagacaaa 1440

aatccagtgt agctggccac ttatccaggg ctttttctac ttcatcacaa ggaatgtttt 1500

gaaagtgtct gcttttttta tccttaaaat tcacctgtca gggaggcatt aaaaatttgg 1560

aaatgtatgc cagcaaaatg tgagctctgt attttttggc attcttatgt ttgggtttaa 1620

taagattaag aaaatgatac tgggaatttt ctttttcctg aaactttgaa tcaccctagt 1680

aagtcaaagt actaaaaaat gtactagatc attaagactt atgtgctctt actgattgaa 1740

agatttttta tgttttcctt gtaataaagg acctaaaccg aaggtacctg aaaaaaaaaa 1800

aaaaa 1805

<210> 124
<211> 1142
<212> DNA
<213> Homo sapiens

<400> 124
cttttccaag cggctgccga agatggcgga ggtgcaggtc ctggtgcttg atggtcgagg 60

ccatctcctg ggccgcctgg cggccatcgt ggctaaacag gtactgctgg gccggaaggt 120

ggtggtcgta cgctgtgaag gcatcaacat ttctggcaat ttctacagaa acaagttgaa 180

gtacctggct ttcctccgca agcggatgaa caccaaccct tcccgaggcc cctaccactt 240

ccgggccccc agccgcatct tctggcggac cgtgcgaggt atgctgcccc acaaaaccaa 300

gcgaggccag gccgctctgg accgtctcaa ggtgtttgac ggcatcccac cgccctacga 360

caagaaaaag cggatggtgg ttcctgctgc cctcaaggtc gtgcgtctga agcctacaag 420

aaagtttgcc tatctggggc gcctggctca cgaggttggc tggaagtacc aggcagtgac 480

agccaccctg gaggagaaga ggaaagagaa agccaagatc cactaccgga agaagaaaca 540

gctcatgagg ctacggaaac aggccgagaa gaacgtggag aagaaaattg acaaatacac 600

agaggtcctc aagacccacg gactcctggt ctgagcccaa taaagactgt taattcctca 660

tgcgttgcct gcccttcctc cattgttgcc ctggaatgta cgggacccag gggcagcagc 720

agtccaggtg ccacaggcag ccctgggaca taggaagctg ggagcaagga aagggtctta 780

gtcactgcct cccgaagttg cttgaaagca ctcggagaat tgtgcaggtg tcatttatct 840

atgaccaata ggaagagcaa ccagttacta tgagtgaaag ggagccagaa gactgattgg 900

agggccctat cttgtgagtg gggcatctgt tggactttcc acctggtcat atactctgca 960

gctgttagaa tgtgcaagca cttggggaca gcatgagctt gctgttgtac acagggtatt 1020

tctagaagca gaaatagact gggaagatgc acaaccaagg ggttacaggc atcgcccatg 1080

ctcctcacct gtattttgta atcagaaata aattgctttt aaagaaaaaa aaaaaaaaaa 1140

aa 1142

<210> 125
<211> 3834
<212> DNA
<213> Homo sapiens

<400> 125
cctgagacag aggcagcagt gatacccacc tgagagatcc tgtgtttgaa caactgcttc 60

ccaaaacgga aagtatttca agcctaaacc tttgggtgaa aagaactctt gaagtcatga 120

ttgcttcaca gtttctctca gctctcactt tggtgcttct cattaaagag agtggagcct 180

ggtcttacaa cacctccacg gaagctatga cttatgatga ggccagtgct tattgtcagc 240

aaaggtacac acacctggtt gcaattcaaa acaaagaaga gattgagtac ctaaactcca 300

tattgagcta ttcaccaagt tattactgga ttggaatcag aaaagtcaac aatgtgtggg 360

tctgggtagg aacccagaaa cctctgacag aagaagccaa gaactgggct ccaggtgaac 420
ccaacaatag gcaaaaagat gaggactgcg tggagatcta catcaagaga gaaaaagatg 480
tgggcatgtg gaatgatgag aggtgcagca agaagaagct tgccctatgc tacacagctg 540
cctgtaccaa tacatcctgc agtggccacg gtgaatgtgt agagaccatc aataattaca 600
cttgcaagtg tgaccctggc ttcagtggac tcaagtgtga gcaaattgtg aactgtacag 660
ccctggaatc ccctgagcat ggaagcctgg tttgcagtca cccactggga aacttcagct 720
acaattcttc ctgctctatc agctgtgata ggggttacct gccaagcagc atggagacca 780
tgcagtgtat gtcctctgga gaatggagtg ctcctattcc agcctgcaat gtggttgagt 840
gtgatgctgt gacaaatcca gccaatgggt tcgtggaatg tttccaaaac cctggaagct 900
tcccatggaa cacaacctgt acatttgact gtgaagaagg atttgaacta atgggagccc 960
agagccttca gtgtacctca tctgggaatt gggacaacga gaagccaacg tgtaaagctg 1020
tgacatgcag ggccgtccgc cagcctcaga atggctctgt gaggtgcagc cattcccctg 1080
ctggagagtt caccttcaaa tcatcctgca acttcacctg tgaggaaggc ttcatgttgc 1140
agggaccagc ccaggttgaa tgcaccactc aagggcagtg gacacagcaa atcccagttt 1200
gtgaagcttt ccagtgcaca gccttgtcca accccgagcg aggctacatg aattgtcttc 1260
ctagtgcttc tggcagtttc cgttatgggt ccagctgtga gttctcctgt gagcagggtt 1320
ttgtgttgaa gggatccaaa aggctccaat gtggccccac aggggagtgg gacaacgaga 1380
agcccacatg tgaagctgtg agatgcgatg ctgtccacca gcccccgaag ggtttggtga 1440
ggtgtgctca ttcccctatt ggagaattca cctacaagtc ctcttgtgcc ttcagctgtg 1500
aggagggatt tgaattatat ggatcaactc aacttgagtg cacatctcag ggacaatgga 1560
cagaagaggt tccttcctgc caagtggtaa aatgttcaag cctggcagtt ccgggaaaga 1620
tcaacatgag ctgcagtggg gagcccgtgt ttggcactgt gtgcaagttc gcctgtcctg 1680
aaggatggac gctcaatggc tctgcagctc ggacatgtgg agccacagga cactggtctg 1740
gcctgctacc tacctgtgaa gctcccactg agtccaacat tcccttggta gctggacttt 1800
ctgctgctgg actctccctc ctgacattag caccatttct cctctggctt cggaaatgct 1860
tacggaaagc aaagaaattt gttcctgcca gcagctgcca aagccttgaa tcagacggaa 1920
gctaccaaaa gccttcttac atcctttaag ttcaaaagaa tcagaaacag gtgcatctgg 1980
ggaactagag ggatacactg aagttaacag agacagataa ctctcctcgg gtctctggcc 2040
cttcttgcct actatgccag atgcctttat ggctgaaacc gcaacaccca tcaccacttc 2100
aatagatcaa agtccagcag gcaaggacgg ccttcaactg aaaagactca gtgttccctt 2160
tcctactctc aggatcaaga aagtgttggc taatgaaggg aaaggatatt ttcttccaag 2220
caaaggtgaa gagaccaaga ctctgaaatc tcagaattcc ttttctaact ctcccttgct 2280
cgctgtaaaa tcttggcaca gaaacacaat attttgtggc tttctttctt ttgcccttca 2340
cagtgtttcg acagctgatt acacagttgc tgtcataaga atgaataata attatccaga 2400

gtttagagga aaaaaatgac taaaaatatt ataacttaaa aaaatgacag atgttgaatg 2460
cccacaggca aatgcatgga gggttgttaa tggtgcaaat cctactgaat gctctgtgcg 2520
agggttacta tgcacaattt aatcactttc atccctatgg gattcagtgc ttcttaaaga 2580
gttcttaagg attgtgatat ttttacttgc attgaatata ttataatctt ccatacttct 2640
tcattcaata caagtgtggt agggacttaa aaaacttgta aatgctgtca actatgatat 2700
ggtaaaagtt acttattcta gattaccccc tcattgttta ttaacaaatt atgttacatc 2760
tgttttaaat ttatttcaaa aagggaaact attgtcccct agcaaggcat gatgttaacc 2820
agaataaagt tctgagtgtt tttactacag ttgttttttg aaaacatggt agaattggag 2880
agtaaaaact gaatggaagg tttgtatatt gtcagatatt ttttcagaaa tatgtggttt 2940
ccacgatgaa aaacttccat gaggccaaac gttttgaact aataaaagca taaatgcaaa 3000
cacacaaagg tataatttta tgaatgtctt tgttggaaaa gaatacagaa agatggatgt 3060
gctttgcatt cctacaaaga tgtttgtcag atgtgatatg taaacataat tcttgtatat 3120
tatggaagat tttaaattca caatagaaac tcaccatgta aaagagtcat ctggtagatt 3180
tttaacgaat gaagatgtct aatagttatt ccctatttgt tttcttctgt atgttagggt 3240
gctctggaag agaggaatgc ctgtgtgagc aagcatttat gtttatttat aagcagattt 3300
aacaattcca aaggaatctc cagttttcag ttgatcactg gcaatgaaaa attctcagtc 3360
agtaattgcc aaagctgctc tagccttgag gagtgtgaga atcaaaactc tcctacactt 3420
ccattaactt agcatgtgtt gaaaaaaaaa gtttcagaga agttctggct gaacactggc 3480
aacgacaaag ccaacagtca aaacagagat gtgataagga tcagaacagc agaggttctt 3540
ttaaaggggc agaaaaactc tgggaaataa gagagaacaa ctactgtgat caggctatgt 3600
atggaataca gtgttatttt ctttgaaatt gtttaagtgt tgtaaatatt tatgtaaact 3660
gcattagaaa ttagctgtgt gaaataccag tgtggtttgt gtttgagttt tattgagaat 3720
tttaaattat aacttaaaat attttataat ttttaaagta tatatttatt taagcttatg 3780
tcagacctat ttgacataac actataaagg ttgacaataa atgtgcttat gttt 3834

<210> 126
<211> 2448
<212> DNA
<213> Homo sapiens

<400> 126
   aaggggaggg aaaaggggag gaggaggagg atgtgagact gggttagaga aatgaaagaa 60
   agcaaggctt tctgttgaca ttcagtgcag tctacctgca gcacagcaca ctccctttgg 120
   gcaaggacct gagacccttg tgctaagtca agaggctcaa tgggctgcag aagaactaga 180
   gaaggaccaa gcaaagccat gatatttcca tggaaatgtc agagcaccca gagggactta 240
   tggaacatct tcaagttgtg ggggtggaca atgctctgtt gtgatttcct ggcacatcat 300
   ggaaccgact gctggactta ccattattct gaaaaaccca tgaactggca aagggctaga 360
   agattctgcc gagacaatta cacagattta gttgccatac aaaacaaggc ggaaattgag 420
   tatctggaga agactctgcc tttcagtcgt tcttactact ggataggaat ccggaagata 480
   ggaggaatat ggacgtgggt gggaaccaac aaatctctta ctgaagaagc agagaactgg 540
   ggagatggtg agcccaacaa caagaagaac aaggaggact gcgtggagat ctatatcaag 600
   agaaacaaag atgcaggcaa atggaacgat gacgcctgcc acaaactaaa ggcagccctc 660
   tgttacacag cttcttgcca gccctggtca tgcagtggcc atggagaatg tgtagaaatc 720
   atcaataatt acacctgcaa ctgtgatgtg gggtactatg ggccccagtg tcagtttgtg 780
   attcagtgtg agcctttgga ggccccagag ctgggtacca tggactgtac tcaccctttg 840
   ggaaacttca gcttcagctc acagtgtgcc ttcagctgct ctgaaggaac aaacttaact 900
   gggattgaag aaaccacctg tggaccattt ggaaactggt catctccaga accaacctgt 960
   caagtgattc agtgtgagcc tctatcagca ccagatttgg ggatcatgaa ctgtagccat 1020
   cccctggcca gcttcagctt tacctctgca tgtaccttca tctgctcaga aggaactgag 1080
   ttaattggga agaagaaaac catttgtgaa tcatctggaa tctggtcaaa tcctagtcca 1140
   atatgtcaaa aattggacaa aagtttctca atgattaagg agggtgatta taaccccctc 1200
   ttcattccag tggcagtcat ggttactgca ttctctgggt tggcatttat catttggctg 1260
   gcaaggagat taaaaaaagg caagaaatcc aagagaagta tgaatgaccc atattaaatc 1320
   gcccttggtg aaagaaaatt cttggaatac taaaaatcat gagatccttt aaatccttcc 1380
   atgaaacgtt ttgtgtggtg gcacctccta cgtcaaacat gaagtgtgtt tccttcagtg 1440
   catctgggaa gatttctacc tgaccaacag ttccttcagc ttccatttcg cccctcattt 1500
   atccctcaac ccccagccca caggtgttta tacagctcag ctttttgtct tttctgagga 1560
   gaaacaaata agaccataaa gggaaaggat tcatgtggaa tataaagatg gctgactttg 1620
   ctctttcttg actcttgttt tcagtttcaa ttcagtgctg tacttgatga cagacacttc 1680
   taaatgaagt gcaaatttga tacatatgtg aatatggact cagttttctt gcagatcaaa 1740
   tttcacgtcg tcttctgtat actgtggagg tacactctta tagaaagttc aaaaagtcta 1800
   cgctctcctt tctttctaac tccagtgaag taatggggtc ctgctcaagt tgaaagagtc 1860
   ctatttgcac tgtagcctcg ccgtctgtga attggaccat cctatttaac tggcttcagc 1920
   ctccccacct tcttcagcca cctctctttt tcagttggct gacttccaca cctagcatct 1980
   catgagtgcc aagcaaaagg agagaagaga gaaatagcct gcgctgtttt ttagtttggg 2040
   ggttttgctg tttcctttta tgagacccat tcctatttct tatagtcaat gtttctttta 2100
   tcacgatatt attagtaaga aaacatcact gaaatgctag ctgcaagtga catctctttg 2160
   atgtcatatg gaagagttaa aacaggtgga gaaattcctt gattcacaat gaaatgctct 2220
   cctttcccct gcccccagac cttttatcca cttacctaga ttctacatat tctttaaatt 2280
   tcatctcagg cctccctcaa ccccaccact tcttttataa ctagtccttt actaatccaa 2340
   cccatgatga gctcctcttc ctggcttctt actgaaaggt taccctgtaa catgcaattt 2400
   tgcatttgaa taaagcctgc tttttaagtg ttaaaaaaaa aaaaaaaa 2448

<210> 127
<211> 3199
<212> DNA
<213> Homo sapiens

<400> 127
gtttttctaa acagcctgac actgagggga ggcagtgaga ctgtaagcag tctgggttgg 60
gcagaaggca gaaaaccagc agagtcacag aggagatggc caactgccaa atagccatct 120
tgtaccagag attccagaga gtggtctttg gaatttccca actcctttgc ttcagtgccc 180
tgatctctga actaacaaac cagaaagaag tggcagcatg gacttatcat tacagcacaa 240
aagcatactc atggaatatt tcccgtaaat actgccagaa tcgctacaca gacttagtgg 300
ccatccagaa taaaaatgaa attgattacc tcaataaggt cctaccctac tacagctcct 360
actactggat tgggatccga aagaacaata agacatggac atgggtggga accaaaaagg 420
ctctcaccaa cgaggctgag aactgggctg ataatgaacc taacaacaaa aggaacaacg 480
aggactgcgt ggagatatac atcaagagtc cgtcagcccc tggcaagtgg aatgatgagc 540
actgcttgaa gaaaaagcac gcattgtgtt acacagcctc ctgccaggac atgtcctgca 600
gcaaacaagg agagtgcctc gagaccatcg ggaactacac ctgctcctgt taccctggat 660
tctatgggcc agaatgtgaa tacgtgagag agtgtggaga acttgagctc cctcaacacg 720
tgctcatgaa ctgcagccac cctctgggaa acttctcttt taactcgcag tgcagcttcc 780
actgcactga cgggtaccaa gtaaatgggc ccagcaagct ggaatgcttg gcttctggaa 840
tctggacaaa taagcctcca cagtgtttag ctgcccagtg cccacccctg aagattcctg 900
aacgaggaaa catgatctgc cttcattctg caaaagcatt ccagcatcag tctagctgca 960
gcttcagttg tgaagaggga tttgcattag ttggaccgga agtggtgcaa tgcacagcct 1020
cgggggtatg gacagcccca gccccagtgt gtaaagctgt gcagtgtcag cacctggaag 1080
cccccagtga aggaaccatg gactgtgttc atccgctcac tgcttttgcc tatggctcca 1140
gctgcaaatt tgagtgccag cccggctaca gagtgagggg cttggacatg ctccgctgca 1200
ttgactctgg acactggtct gcacccttgc caacctgtga ggctatttcg tgtgagccgc 1260
tggagagtcc tgtccacgga agcatggatt gctctccatc cttgagagcg tttcagtatg 1320
acaccaactg tagcttccgc tgtgctgaag gtttcatgct gagaggagcc gatatagttc 1380
ggtgtgataa cttgggacag tggacagcac cagccccagt ctgtcaagct ttgcagtgcc 1440
aggatctccc agttccaaat gaggcccggg tgaactgctc ccaccccttc ggtgccttta 1500
ggtaccagtc agtctgcagc ttcacctgca atgaaggctt gctcctggtg ggagcaagtg 1560
tgctacagtg cttggctact ggaaactgga attctgttcc tccagaatgc caagccattc 1620
cctgcacacc tttgctaagc cctcagaatg gaacaatgac ctgtgttcaa cctcttggaa 1680
gttccagtta taaatccaca tgtcaattca tctgtgacga gggatattct ttgtctggac 1740
cagaaagatt ggattgtact cgatcgggac gctggacaga ctccccacca atgtgtgaag 1800

ccatcaagtg cccagaactc tttgccccag agcagggcag cctggattgt tctgacactc 1860
gtggagaatt caatgttggc tccacctgtc atttctcttg taacaatggc tttaagctgg 1920
aggggcccaa taatgtggaa tgcacaactt ctggaagatg gtcagctact ccaccaacct 1980
gcaaaggcat agcatcactt cctactccag ggttgcaatg tccagccctc accactcctg 2040
ggcagggaac catgtactgt aggcatcatc cgggaacctt tggttttaat accacttgtt 2100
actttggctg caacgctgga ttcacactca taggagacag cactctcagc tgcagacctt 2160
caggacaatg gacagcagta actccagcat gcagagctgt gaaatgctca gaactacatg 2220
ttaataagcc aatagcgatg aactgctcca acctctgggg aaacttcagt tatggatcaa 2280
tctgctcttt ccattgtcta gagggccagt tacttaatgg ctctgcacaa acagcatgcc 2340
aagagaatgg ccactggtca actaccgtgc caacctgcca agcaggacca ttgactatcc 2400
aggaagccct gacttacttt ggtggagcgg tggcttctac aataggtctg ataatgggtg 2460
ggacgctcct ggctttgcta agaaagcgtt tcagacaaaa agatgatggg aaatgcccct 2520
tgaatcctca cagccaccta ggaacatatg gagtttttac aaacgctgca tttgacccga 2580
gtccttaagg tttccataaa cacccatgaa tcaaagacat ggaattacct tagattagct 2640
ctggaccagc ctgttggacc cgctctggac caaccctgtt tcctgagttt gggattgtgg 2700
tacaatctca aattctcaac ctaccacccc ttcctgtccc acctcttctc ttcctgtaac 2760
acaagccaca gaagccagga gcaaatgttt ctgcagtagt ctctgtgctt tgactcacct 2820
gttacttgaa ataccagtga accaaagaga ctggagcatc tgactcacaa gaagaccaga 2880
ctgtggagaa ataaaaatac ctctttattt tttgattgaa ggaaggtttt ctccactttg 2940
ttggaaagca ggtggcatct ctaattggaa gaaattcctg tagcatcttc tggagtctcc 3000
agtggttgct gttgatgagg cctcttggac ctctgctctg aggcttccag agagtcctct 3060
ggatggcacc agaggctgca gaaggccaag aatcaagcta gaaggccaca tgtcaccgtg 3120
gaccttcctg ccaccagtca ctgtccctca aatgacccaa agaccaatat tcaaatgcgt 3180
aattaaaaga attttcccc 3199

<210> 128
<211> 4452
<212> DNA
<213> Homo sapiens

<400> 128
   ctgtcactgc agactaatgg gacggagggg ggtgacttct cagggttcct cctgggcagg 60
   tgctccggaa ccttttctca gcacgctggc ctggggcacg gccgttcctc ctgcccagcc 120
   acgttggggt acagggtgaa gaagggctgg ggccagccca ggacagagga aggcgaggca 180
   ggcacgcagg aactgggctt tttaaaccct taagcccaag gaaatcgtag catcgcggga 240
   cagggaaaat gaaagacttt ggaagtcgtc aggaatttga ctctgtgagt tggtttccaa 300
   gagtctaagt taagcatctc caagtggata ttaaaaagga gcagcaagcc tcggggcggc 360
   gggggctgga ggaggtggag agaggaggct gccggaagcc gcactcggga cctctgcagc 420
   caccgaccag accgggcggc cgggactctg ggactctcgc aggcagaccc ggtggtctgc 480
   cggactcctc ggggcccact tcgggccctc tctcctgcct cctatttttg gatttctctc 540
   ctttgctccc tttttcctcc cgttttgaag agacaatgct acttcagttt ggagcacaaa 600
   catatgatca gcacatggaa atgtggtaat tcggatgcat tcgtgattgc aacagattga 660
   agaaattaga ccagacaaag agtgttttta gaggaggagg aggaggagga ggaggctgag 720
   agagggaggg cgacgggggt gagaaagggg aggccgcctc tgagcgggac gccgggactc 780
   ccgccgctgc taaatatatc cgtaggaatg gagagggacc ggatctcagc cttgaaaaga 840
   tcttttgagg tcgaggaggt cgagacaccc aactccaccc caccccggag ggtccagact 900
   cccctactcc gagccactgt ggccagctcc acccagaaat tccaggacct gggcgtgaag 960
   aactcagaac cctcggcccg ccatgtggac tccctaagcc aacgctcccc caaggcgtcc 1020
   ctgcggaggg tggagctctc gggccccaag gcggccgagc cggtgtcccg gcgcactgag 1080
   ctgtccattg acatctcgtc caagcaggtg gagaacgccg gggccatcgg cccgtcccgg 1140
   ttcgggctca agagggccga ggtgttgggc cacaagacgc cagaaccggc ccctcggagg 1200
   acggagatca ccatcgtcaa accccaggag tcagcccacc ggaggatgga gccccctgcc 1260
   tccaaggtcc ccgaggtgcc cactgcccct gccaccgacg cagcccccaa gagggtggag 1320
   atccagatgc ccaagcctgc tgaggcgccc accgccccca gcccagccca gaccttggag 1380
   aattcagagc ctgcccctgt gtctcagctg cagagcaggc tggagcccaa gccccagccc 1440
   cctgtggctg aggctacacc ccggagccag gaggccactg aggcggctcc cagctgcgtt 1500
   ggcgacatgg ccgacacccc cagagatgcc gggctcaagc aggcgcctgc atcacggaac 1560
   gagaaggccc cggtggactt cggctacgtg gggattgact ccatcctgga gcagatgcgc 1620
   cggaaggcca tgaagcaggg cttcgagttc aacatcatgg tggtcggcca gagcggcttg 1680
   ggtaaatcca ccttaatcaa caccctcttc aaatccaaaa tcagccggaa gtcggtgcag 1740
   cccacctcag aggagcgcat ccccaagacc atcgagatca agtccatcac gcacgatatt 1800
   gaggagaaag gcgtccggat gaagctgaca gtgattgaca caccagggtt cggggaccac 1860
   atcaacaacg agaactgctg gcagcccatc atgaagttca tcaatgacca gtacgagaaa 1920
   tacctgcagg aggaggtcaa catcaaccgc aagaagcgca tcccggacac ccgcgtccac 1980
   tgctgcctct acttcatccc cgccaccggc cactccctca ggcccctgga catcgagttt 2040
   atgaaacgcc tgagcaaggt ggtcaacatc gtccctgtca tcgccaaggc ggacacactc 2100
   accctggagg agagggtcca cttcaaacag cggatcaccg cagacctgct gtccaacggc 2160
   atcgacgtgt acccccagaa ggaatttgat gaggactcgg aggaccggct ggtgaacgag 2220
   aagttccggg agatgatccc atttgctgtg gtgggcagtg accacgagta ccaggtcaac 2280
   ggcaagagga tccttgggag gaagaccaag tggggtacca tcgaagttga aaacaccaca 2340
   cactgtgagt ttgcctacct gcgggacctt ctcatcagga cgcacatgca gaacatcaag 2400
   gacatcacca gcagcatcca cttcgaggcg taccgtgtga agcgcctcaa cgagggcagc 2460
   agcgccatgg ccaacggcat ggaggagaag gagccagaag ccccggagat gtagacgcca 2520
   ccctgcccac ccccgggatc ctgcccccaa gtcatttccg tcccccccca ggccctccca 2580
   ccaccccatt ttattttata tgattttctc catttgtcat cgttccccac cccttcgaca 2640
   tgctgccagg aaacaaggga aggggcctcc ctccgagtga gtcagtgatg aggccgcggc 2700
   ctccccgagg ttgtggggag gctgcactgg agccacaggc aggggtgaga gcacccactg 2760
   aattgacatg accctctgtc cccaggcctg gctccccgag ggctcagaag agcagcttcg 2820
   gtgtgcagat catccgtctg tgtggggttc tcagtgccgg aggccttggg gtgggggcca 2880
   ggcctcgcac ttgcagagga gcccagtggg ctgcacgctc ccctccatcc ccatcggccc 2940
   tgtcccctgg agtgtgtcag agcccagggg agaatgcagc ccaccaggag cacctggacc 3000
   ccctgcccgc cacatggtgt ggccatcact cagcccctac ccctgccctg ctcctaaggg 3060
   tagaaaactc cagggtcccc tgccaccgac tgcccagcca ctccaagccc cctggcagct 3120
   gcccctcctg gagcagaaag tgcctttatc tcagccatcc gcagactgct tggccagatg 3180
   cggggacagg ctggaatgag ggaggcgtct tcatctccct gccatccccc tctcacgcca 3240
   cccccgcccc caccgggctg caggtgctgc tgatgcgctg ggatctgatt gaggataaaa 3300
   aggaaggaga gatgacccct accccctcat cccccagttt tgaaaaggtc taagcaagtg 3360
   agtctggtgg aggagctgag ggagggagcc atggaaggtg ccagaaggaa ggttggcggg 3420
   ggcacgtgtg ggccgtggct tgggctggtc agagtggcgt gagctgcccg gcgcctgccc 3480
   tgcccaagtg accagggaag tgtgtgtgtg tccatgtgta tgcgtgtccg tctgtctgtc 3540
   tagtgtctgg gtttggccca agactgggct gtagttacat taatgcccag ccagccaccc 3600
   ctgccactca cccttcctgg cccaggcctt gctgactctc tgagctgggg aggtgggagg 3660
   ccaggcgagc ctgactctgt tgatctaccc gtgcctgggc ccctcccctc agagcccatg 3720
   gtaacgaacc cctagaaagg agagaacggg cgtcaggggt gcacagtcca cagctgaaga 3780
   gcaaggtttc gtggcagcac ggcccggccc ctcaccctct gtccccacga ggggacccat 3840
   gggggctgtc tttgcagggc acagatgacc aaagtccctt cctgcttcct gttacctgtc 3900
   ttgctcctgg ggagaaagag gggcctgatg agactccact caggtgcaca catcaccagg 3960
   tgcatctgca ggcaccgggc tggctgcttg cagccaggag aaggtcagcg agaaggagtg 4020
   tatgagtgtg agtgtgtgtg catggaagtt ggggcactgg gcgtctgact ccctccccac 4080
   ccaagagagg aaggacccct caccaccccc actggtgaga cagtttactt tgccaacttg 4140
   ccatgttttt gccaaaacca agattttgaa ggaaatgagt ggccagcgcc agggcccagg 4200
   ccatgtggcc tgcccagcct caatgtcact tggtggcggg gtggggtggg ggtgggcagc 4260
   agcatcccag ccttgagatg cttcactttc cttctctgta accagacttt gaaaaattgt 4320
   tcgtttcatc aggctctgtt cctcaatggc cttttgctac gtgcctcccg agaaatttgt 4380
   ctttttgtat aaatgacaaa gtgttgaaaa tgtatttcct gaaataaatg tttcaaatgc 4440
   agaaacccag aa                                                        4452

<210>  129
<211>  3794
<212>  DNA
<213>  Homo sapiens

<400>  129
tttgaatttc agataaattt attttgcttt ctggtataag tatatcccat gcagcatttc      60

ggacatactt ataccaaaaa acataaaata aaatacttta tattgtctgt gaaagcttta     120

accaggcatc ctgcatttta cctgacaaga ccagccccct ccagtaacgg tcccttcccc     180

tgtacccacc acctccacca ccagtggccc agataagggt agctcactgt ggacttctca     240

cttctgcttt tccgatgacc cagggcccag agggagagcc cccaccccac taccccaccc     300

ccagccaggc ccccaggata gacacaagcc tgccctctcc tcctacacag ggagctctgg     360

aaggaccagc tcaacaagcc caaggccagg ctgggccccc aggagaccca gaggacaact     420

gggcaaggtg ggccggagag tgtgggggaa ggcaaaggag ttctgtgagc tcagcgtctg     480

aagctcattt catgcatcag gccccagggc tcagcttccg ccttcggctt ccccttctgc     540

caagagccct gagccactca cagcacgacc agagaacagg cctgtctcag gcaggccctg     600

cgcctcctat gcggagatgc tactgccact gctgctgtcc tcgctgctgg gcgggtccca     660

ggctatggat gggagattct ggatacgagt gcaggagtca gtgatggtgc cggagggcct     720

gtgcatctct gtgccctgct ctttctccta cccccgacag gactggacag ggtctacccc     780

agcttatggc tactggttca aagcagtgac tgagacaacc aagggtgctc ctgtggccac     840

aaaccaccag agtcgagagg tggaaatgag cacccggggc cgattccagc tcactgggga     900

tcccgccaag gggaactgct ccttggtgat cagagacgcg cagatgcagg atgagtcaca     960

gtacttcttt cgggtggaga gaggaagcta tgtgagatat aatttcatga acgatgggtt    1020

ctttctaaaa gtaacagccc tgactcagaa gcctgatgtc tacatccccg agaccctgga    1080

gcccgggcag ccggtgacgg tcatctgtgt gtttaactgg gcctttgagg aatgtccacc    1140

cccttctttc tcctggacgg gggctgccct ctcctcccaa ggaaccaaac caacgacctc    1200

ccacttctca gtgctcagct tcacgcccag accccaggac cacaacaccg acctcacctg    1260

ccatgtggac ttctccagaa agggtgtgag cgcacagagg accgtccgac tccgtgtggc    1320

ctatgccccc agagaccttg ttatcagcat ttcacgtgac aacacgccag ccctggagcc    1380

ccagccccag ggaaatgtcc catacctgga agcccaaaaa ggccagttcc tgcggctcct    1440

ctgtgctgct gacagccagc cccctgccac actgagctgg gtcctgcaga acagagtcct    1500

ctcctcgtcc catccctggg gccctagacc cctggggctg gagctgcccg gggtgaaggc    1560

tggggattca gggcgctaca cctgccgagc ggagaacagg cttggctccc agcagcgagc    1620

cctggacctc tctgtgcagt atcctccaga gaacctgaga gtgatggttt cccaagcaaa    1680

caggacagtc ctggaaaacc ttgggaacgg cacgtctctc ccagtactgg agggccaaag    1740

cctgtgcctg gtctgtgtca cacacagcag ccccccagcc aggctgagct ggacccagag    1800

gggacaggtt ctgagcccct cccagccctc agaccccggg gtcctggagc tgcctcgggt 1860
tcaagtggag cacgaaggag agttcacctg ccacgctcgg cacccactgg gctcccagca 1920
cgtctctctc agcctctccg tgcactactc cccgaagctg ctgggcccct cctgctcctg 1980
ggaggctgag ggtctgcact gcagctgctc ctcccaggcc agcccggccc cctctctgcg 2040
ctggtggctt ggggaggagc tgctggaggg gaacagcagc caggactcct tcgaggtcac 2100
ccccagctca gccgggccct gggccaacag ctccctgagc ctccatggag ggctcagctc 2160
cggcctcagg ctccgctgtg aggcctggaa cgtccatggg gcccagagtg gatccatcct 2220
gcagctgcca gataagaagg gactcatctc aacggcattc tccaacggag cgtttctggg 2280
aatcggcatc acggctcttc ttttcctctg cctggccctg atcatcatga agattctacc 2340
gaagagacgg actcagacag aaaccccgag gcccaggttc tcccggcaca gcacgatcct 2400
ggattacatc aatgtggtcc cgacggctgg ccccctggct cagaagcgga atcagaaagc 2460
cacaccaaac agtcctcgga cccctcttcc accaggtgct ccctccccag aatcaaagaa 2520
gaaccagaaa aagcagtatc agttgcccag tttcccagaa cccaaatcat ccactcaagc 2580
cccagaatcc caggagagcc aagaggagct ccattatgcc acgctcaact tcccaggcgt 2640
cagacccagg cctgaggccc ggatgcccaa gggcacccag gcggattatg cagaagtcaa 2700
gttccaatga gggtctctta ggctttagga ctgggacttc ggctagggag gaaggtagag 2760
taagaggttg aagataacag agtgcaaagt ttccttctct ccctctctct ctctctttct 2820
ctctctctct ctctttctct ctcttttaaa aaaacatctg gccagggcac agtggctcac 2880
gcctgtaatc ccagcacttt gggaggttga ggtgggcaga tcgcctgagg tcgggagttc 2940
gagaccagcc tggccaactt ggtgaaaccc cgtctctact aaaaatacaa aaattagctg 3000
ggcatggtgg caggcgcctg taatcctacc tacttgggaa gctgaggcag gagaatcact 3060
tgaacctggg agacggaggt tgcagtgagc caagatcaca ccattgcacg ccagcctggg 3120
caacaaagcg agactccatc tcaaaaaaaa aatcctccaa atgggttggg tgtctgtaat 3180
cccagcactt tgggaggcta aggtgggtgg attgcttgag cccaggagtt cgagaccagc 3240
ctgggcaaca tggtgaaacc ccatctctac aaaaaataca aaacatagct gggcttggtg 3300
gtgtgtgcct gtagtcccag ctgtcagaca tttaaaccag agcaactcca tctggaatag 3360
gagctgaata aaatgaggct gagacctact gggctgcatt ctcagacagt ggaggcattc 3420
taagtcacag gatgagacag gaggtccgta caagatacag gtcataaaga ctttgctgat 3480
aaaacagatt gcagtaaaga agccaaccaa atcccaccaa aaccaagttg gccacgagag 3540
tgacctctgg tcgtcctcac tgctacactc ctgacagcac catgacagtt tacaaatgcc 3600
atggcaacat caggaagtta cccgatatgt cccaaaaggg ggaggaatga ataatccacc 3660
ccttgtttag caaataagca agaaataacc ataaaagtgg gcaaccagca gctctaggcg 3720
ctgctcttgt ctatggagta gccattcttt tgttccttta ctttcttaat aaacttgctt 3780
tcaccttaaa aaaa 3794

<210> 130
<211> 3183
<212> DNA
<213> Homo sapiens

<400> 130
cgaggctcct cctctgtgga tggtcactgc ccctccacca ggcttcctgc tggaggagtt 60

tccttcccag ccaggccggc ccagaagcca gatggtcccg ggacaggccc agccccagag 120

cccagagatg ctgctgctgc ccctgctgct gcccgtgctg ggggcggggt ccctgaacaa 180

ggatcccagt tacagtcttc aagtgcagag gcaggtgccg gtgccggagg gcctgtgtgt 240

catcgtgtct tgcaacctct cctacccccg ggatggctgg gacgagtcta ctgctgctta 300

tggctactgg ttcaaaggac ggaccagccc aaagacgggt gctcctgtgg ccactaacaa 360

ccagagtcga gaggtggaaa tgagcacccg ggaccgattc cagctcactg gggatcccgg 420

caaagggagc tgctccttgg tgatcagaga cgcgcagagg gaggatgagg catggtactt 480

ctttcgggtg gagagaggaa gccgtgtgag acatagtttc ctgagcaatg cgttctttct 540

aaaagtaaca gccctgacta agaagcctga tgtctacatc cccgagaccc tggagcccgg 600

gcagccggtg acggtcatct gtgtgtttaa ctgggctttc aagaaatgtc cagccccttc 660

tttctcctgg acgggggctg ccctctcccc tagaagaacc agaccaagca cctcccactt 720

ctcagtgctc agcttcacgc ccagccccca ggaccacgac accgacctca cctgccatgt 780

ggacttctcc agaaagggtg tgagcgcaca gaggaccgtc cgactccgtg tggcctatgc 840

ccccaaagac cttattatca gcatttcaca tgacaacacg tcagccctgg aactccaggg 900

aaacgtcata tatctggaag ttcagaaagg ccagttcctg cggctcctct gtgctgctga 960

cagccagccc cctgccacgc tgagctgggt cctgcaggac agagtcctct cctcgtccca 1020

cccctggggc cccagaaccc tggggctgga gctgcgtggg gtaagggccg gggattcagg 1080

gcgctacacc tgccgagcgg agaacaggct tggctcccag cagcaagccc tggacctctc 1140

tgtgcagtat cctccagaga acctgagagt gatggtttcc caagcaaaca ggacagtcct 1200

ggaaaacctc gggaacggca catccctccc ggtcctggag ggccaaagcc tgcgcctggt 1260

ctgtgtcacc cacagcagcc ccccagccag gctgagctgg acccggtggg gacagaccgt 1320

gggcccctcc cagccctcag accccggggt cctggagctg ccacccattc aaatggagca 1380

cgaaggagag ttcacctgcc acgctcagca ccctctgggc tcccagcacg tctctctcag 1440

cctctccgtg cactaccctc cacagctgct gggcccctcc tgctcctggg aggctgaggg 1500

tctgcactgc agctgctcct cccaggccag cccggccccc tctctgcgct ggtggcttgg 1560

ggaggagctg ctggagggga acagcagtca gggctccttc gaggtcaccc ccagctcagc 1620

cgggccctgg gccaacagct ccctgagcct ccatggaggg ctcagctccg gcctcaggct 1680

ccgctgtaag gcctggaacg tccacggggc ccagagtggc tctgtcttcc agctgctacc 1740

agggaagctg gagcatgggg gaggacttgg cctgggggct gccctgggag ctggcgtcgc 1800

tgccctgctc gctttctgtt cctgccttgt cgtcttcagg gtgaagatct gcaggaagga 1860

agctcgcaag agggcagcag ctgagcagga cgtgccctcc accctgggac ccatctccca 1920

gggtcaccag catgaatgct cggcaggcag ctcccaagac cacccgcccc caggtgcagc 1980

cacctacacc ccggggaagg gggaagagca ggagctccac tatgcctccc tcagcttcca 2040

gggcctgagg ctctgggagc ctgcggacca ggaggccccc agcaccaccg agtactcgga 2100

gatcaagatc cacacaggac agcccctgag gggcccaggc tttgggcttc aattggagag 2160

ggagatgtca gggatggttc caaagtgaag aggtctccat ggcaacagga caccagcaag 2220

tgtgtgggag tcgcactggt gtgacggcca gaactggact cagatttcag ccccatcccc 2280

aatgaagagc ttgagtttga agattatact ttttttgaga cagggtctga ctctgtcctc 2340

caggccggag tccagtggtg caatctcggc tcactgtagc ctcaacctgc caggttgaag 2400

tgagcctccc atttcagcct cccaagtggc tgggactaca attgtgagcc accatgccag 2460

gctcattgtt gtatttttgg tagagacagg gttttgccat gtttccctgg ctggtctcgg 2520

actcctgggc tcaagcaatc tgcccgcctc tgcctcccaa ggtgctggga ttgcagacgt 2580

gagccaccac agctggctga agattatact ttcaattcag agcgagtttg aagatgacac 2640

tttgaggcat cgtgtctatg gttcattact acagaagctt ctctggatgt gtaaagcaca 2700

ggaaaccagg cagaggaggc acagggtgct ctccagaacg agaagccagc tcctggagtt 2760

gtttgctgca actgccattc cccgttgatg accatgctct tccttcagaa gagggagagt 2820

gagaggacca agtccaagtg gttcccattt gaacatttaa aaaaaaaaaa aaggctgggc 2880

atggtggctc acgcctgtgg tctcagcact ttgggaggct gaagtgggtg gatcacaagt 2940

caggagttcg agaccagcct gggcaagatg gtgaaacccc atctctactg gaaatgcaaa 3000

ggttagccgg gcatggtggc gggcgcctaa aatcccagct actcgggaga ctaggcagag 3060

aattggttga acccgggagg tggaggttgc agtgagccga gatcgtccca ctgcactcca 3120

gcctgggcaa cagagtgaga ctctgtttct aaataaataa acgaaaaaaa aaaaaaaaaa 3180

aaa 3183

<210> 131
<211> 2126
<212> DNA
<213> Homo sapiens

<400> 131
gaagaaccct gaggaacaga cttacctcag caaccctggc acctccaacc cgacacatgc 60

tactgctgct gctactgctg ccacccctgc tctgtgggag agtgggggct aaggaacaga 120

aggattacct gctgacaatg cagaagtccg tgacggtgca ggagggcctg tgtgtctctg 180

tgctttgctc cttctcctac ccccaaaatg gctggactgc ctccgatcca gttcatggct 240

actggttccg ggcaggggac catgtaagcc ggaacattcc agtggccaca aacaacccag 300

ctcgagcagt gcaggaggag actcgggacc gattccacct ccttggggac ccacagaaca 360

aggattgtac cctgagcatc agagacacca gagagagtga tgcagggaca tacgtctttt 420

gtgtagagag aggaaatatg aaatggaatt ataaatatga ccagctctct gtgaatgtga 480
cagcgtccca ggacctactg tcaagataca ggctggaggt gccagagtcg gtgactgtgc 540
aggagggtct gtgtgtctct gtgccctgca gtgtccttta cccccattac aactggactg 600
cctctagccc tgtttatgga tcctggttca aggaaggggc cgatatacca tgggatattc 660
cagtggccac aaacacccca agtggaaaag tgcaagagga tacccacggt cgattcctcc 720
tccttgggga cccacagacc aacaactgct ccctgagcat cagagatgcc aggaaggggg 780
attcagggaa gtactacttc caggtggaga gaggaagcag gaaatggaac tacatatatg 840
acaagctctc tgtgcatgtg acagccctga ctcacatgcc caccttctcc atcccgggga 900
ccctggagtc tggccacccc aggaacctga cctgctctgt gccctgggcc tgtgaacagg 960
ggacgccccc cacgatcacc tggatggggg cctccgtgtc ctccctggac cccactatca 1020
ctcgctcctc gatgctcagc ctcatcccac agccccagga ccatggcacc agcctcacct 1080
gtcaggtgac cttgcctggg gccggcgtga ccatgaccag ggctgtccga ctcaacatat 1140
cctatcctcc tcagaacttg accatgactg tcttccaagg agatggcaca gcatccacaa 1200
ccttgaggaa tggctcggcc ctttcagtcc tggagggcca gtccctgcac cttgtctgtg 1260
ctgtcgacag caatccccct gccaggctga gctggacctg ggggagcctg accctgagcc 1320
cctcacagtc ctcgaacctt ggggtgctgg agctgcctcg agtgcatgtg aaggatgaag 1380
gggaattcac ctgccgagct cagaaccctc taggctccca gcacatttcc ctgagcctct 1440
ccctgcaaaa cgagtacaca ggcaaaatga ggcctatatc aggagtgacg ctaggggcat 1500
tcgggggagc tggagccaca gccctggtct tcctgtactt ctgcatcatc ttcgttgtag 1560
tgaggtcctg caggaagaaa tcggcaaggc cagcagtggg cgtgggggat acaggcatgg 1620
aggacgcaaa cgctgtcagg ggctcagcct ctcagggacc cctgattgaa tccccggcag 1680
atgacagccc cccacaccat gctccgccag ccctggccac cccctcccca gaggaaggag 1740
agatccagta tgcatccctc agcttccaca aagcgaggcc tcagtaccca caggaacagg 1800
aggccatcgg ctatgagtac tccgagatca acatccccaa gtgagaaact gcagagactc 1860
aggcctgttt gagggctcac gacccctgca gcaaagaagc ccgagactga ttcctttaga 1920
attaacagcc ctccatgctg tgcaacagga catcagaact tattcctctt gtcaaactga 1980
aaatgcgtgc ctgatgacca aactctccct ttctccatcc aatcggtcca cactccccgc 2040
ccccggcctc tggtacccac cattctcttc tctacttctc tgaggtcgac tattttaggt 2100
tccaaatata gtgagatcgt agagtg 2126

<210> 132
<211> 2130
<212> DNA
<213> Homo sapiens

<400> 132
cattgtgttg ggcacagctc tcactcaccc tccggcttcc tgtcggggct ttctcagccc 60

caccccacgt ttggacattt ggagcatttc cttccctgac agccggacct gggactgggc 120
tggggccctg gcggatggag acatgctgcc cctgctgctg ctgcccctgc tgtggggggg 180
gtccctgcag gagaagccag tgtacgagct gcaagtgcag aagtcggtga cggtgcagga 240
gggcctgtgc gtccttgtgc cctgctcctt ctcttacccc tggagatcct ggtattcctc 300
tcccccactc tacgtctact ggttccggga cggggagatc ccatactacg ctgaggttgt 360
ggccacaaac aacccagaca gaagagtgaa gccagagacc cagggccgat tccgcctcct 420
tggggatgtc cagaagaaga actgctccct gagcatcgga gatgccagaa tggaggacac 480
gggaagctat ttcttccgcg tggagagagg aagggatgta aaatatagct accaacagaa 540
taagctgaac ttggaggtga cagccctgat agagaaaccc gacatccact ttctggagcc 600
tctggagtcc ggccgcccca caaggctgag ctgcagcctt ccaggatcct gtgaagcggg 660
accacctctc acattctcct ggacggggaa tgccctcagc cccctggacc ccgagaccac 720
ccgctcctcg gagctcaccc tcacccccag gcccgaggac catggcacca acctcacctg 780
tcagatgaaa cgccaaggag ctcaggtgac cacggagaga actgtccagc tcaatgtctc 840
ctatgctcca cagaccatca ccatcttcag gaacggcata gccctagaga tcctgcaaaa 900
cacctcatac cttccggtcc tggagggcca ggctctgcgg ctgctctgtg atgctcccag 960
caacccccct gcacacctga gctggttcca gggctcccct gccctgaacg ccacccccat 1020
ctccaatacc gggatcttgg agcttcgtcg agtaaggtct gcagaagaag gaggcttcac 1080
ctgccgcgct cagcacccgc tgggcttcct gcaaattttt ctgaatctct cagtttactc 1140
cctcccacag ttgctgggcc cctcctgctc ctgggaggct gagggtctgc actgcagatg 1200
ctcctttcga gcccggccgg ccccctccct gtgctggcgg cttgaggaga agccgctgga 1260
ggggaacagc agccagggct cattcaaggt caactccagc tcagctgggc cctgggccaa 1320
cagctccctg atcctccacg gggggctcag ctccgacctc aaagtcagct gcaaggcctg 1380
gaacatctat gggtcccaga gcggctctgt cctgctgctg caagggagat cgaacctcgg 1440
gacaggagtg gttcctgcag cccttggtgg tgctggtgtc atggccctgc tctgtatctg 1500
tctgtgcctc atcttctttt taatagtgaa agcccgcagg aagcaagcag ctgggagacc 1560
agagaaaatg gatgatgaag accccattat gggtaccatc acctcgggtt ccaggaagaa 1620
gccctggcca gacagccccg gagatcaagc atctcctcct ggggatgccc ctcccttgga 1680
agaacaaaag gagctccatt atgcctcccc tagtttttct gagatgaagt cgagggagcc 1740
taaggaccag gaggccccaa gcaccacgga gtactcggag atcaagacaa gcaagtgagg 1800
atttgcccag agttcagtcc tggctggagg agccacagcc tgtctggggg aaaggacaag 1860
tcagggacca cttgctgaag cacgaagagc ccttgtggca atgttaacat taactgatgt 1920
ttaagtgctc caagcagagc agaaagaaaa cagatgatgg aattagagag gtgggctcaa 1980
atctaggccc tggcactgtc atcaagcaat tcactgcatc cctctgtgcc tcagtttccc 2040
attctgtaaa tcagagatca tgcatgctac ctcaaaggtt gttgtgaaca ttaaagaaat 2100

caacacatgg aaatcaaaaa aaaaaaaaaa                                    2130

<210>  133
<211>  1829
<212>  DNA
<213>  Homo sapiens

<400>  133
gggtctcaaa gtttccgtct gctctgtgca gagggagtgg agctccgagg gcttgtggct      60

tcgcagttcc tcttctgtga acagccgaga tcacgcgctc ctccccagcc acccgttcct     120

ccccgcagtc cttcccctcc actcccttcc ccttctctgc tcatgcaggg agcccaggaa     180

gcctccgcct cagagatgct accgctgctg ctgcccctgc tgtgggcagg ggccctggct     240

caggagcgga gattccagct ggaggggcca gagtcactga cggtgcagga gggtctgtgc     300

gtcctcgtac cctgcagatt gcccactacc cttccagcct cgtactatgg ttatggctac     360

tggttcctgg aaggggctga tgttccagtg gccacaaacg acccagacga agaagtgcag     420

gaggagaccc ggggccgatt ccacctcctc tgggatccca gaaggaagaa ctgctccctg     480

agcatcagag atgcccggag gagggacaat gctgcatact tctttcggtt gaagtccaaa     540

tggatgaaat acggttatac atcttccaag ctctctgtgc gtgtgatggc cctgacccac     600

aggcccaaca tctccatccc agggaccctg gagtctggcc atcccagcaa tctgacctgc     660

tctgtgccct gggtctgtga gcaggggacg ccccccatct tctcctggat gtcagctgcc     720

cccacctccc tgggccccag gaccacccag tcctcggtgc tcacaatcac cccacggccc     780

caggaccaca gcaccaacct cacctgtcag gtgacgttcc ctggagccgg tgtgaccatg     840

gagagaacca tccagctcaa tgtctcctcc ttcaaaatcc tgcaaaacac ctcgtccctc     900

cctgtcctgg agggccaggc tctgcggctg ctctgtgatg ctgacggcaa cccccctgca     960

cacctgagct ggttccaggg cttccccgcc ctgaacgcca cccccatctc caataccggg    1020

gtcctggagc tgcctcaagt agggtctgca gaagaaggag atttcacctg ccgtgctcag    1080

catcctctgg gctccctgca aatctctctg agtctctttg tgcattggaa accagaaggc    1140

agggctggtg gtgtcctggg agcagtctgg ggagctagca tcacaaccct ggttttcctc    1200

tgtgtttgct tcatcttcag agtgaagact agaaggaaga aagcagccca gccagtgcaa    1260

aacacggatg atgtgaaccc cgtcatggtc tcaggctcca ggggtcatca gcaccagttc    1320

cagacaggca tagtttcaga ccaccctgct gaggctggcc ccatctcaga agatgagcag    1380

gagctccact acgctgtcct acacttccac aaggtgcaac ctcaggaacc aaaggtcacc    1440

gacactgagt actcagaaat caagatacac aagtgaggaa ttgtccaaag ccataacctt    1500

gattggagag aacatggtac ctctcagtgt attggttact agggctgcca cagcaatgta    1560

ccacaaaccg agtgacataa acacagaact ttattttcgt atagtttcag atgttagagg    1620

tctgagaaca aggtgttatc agggttggtc ccttctaagg cctctcttgt tggcttgtag    1680

atggctgtct cctccttgtg tcttcacatg gtctttcctc tgagtgtgtt tgtgtcctaa    1740

tcttctcttc ttataaagac actagtcata ttggattagg gcctccccat gacctaattt    1800

aaataaatta actatttaaa gaccctcca 1829

<210> 134
<211> 1475
<212> DNA
<213> Homo sapiens

<400> 134
gcagttcctg agagaagaac cctgaggaac agacgttccc tcgcggccct ggcacctcca 60

accccagata tgctgctgct gctgctgctg cccctgctct gggggaggga gagggtggaa 120

ggacagaaga gtaaccggaa ggattactcg ctgacgatgc agagttccgt gaccgtgcaa 180

gagggcatgt gtgtccatgt gcgctgctcc ttctcctacc cagtggacag ccagactgac 240

tctgacccag ttcatggcta ctggttccgg gcagggaatg atataagctg gaaggctcca 300

gtggccacaa acaacccagc ttgggcagtg caggaggaaa ctcgggaccg attccacctc 360

cttggggacc cacagaccaa aaattgcacc ctgagcatca gagatgccag aatgagtgat 420

gcggggagat acttctttcg tatggagaaa ggaaatataa aatggaatta taaatatgac 480

cagctctctg tgaacgtgac agaccctcct cagaacttga ctgtgactgt cttccaagga 540

gaaggcacag catccacagc tctggggaac agctcatctc tttcagtcct agagggccag 600

tctctgcgct tggtctgtgc tgttgacagc aatccccctg ccaggctgag ctggacctgg 660

aggagtctga ccctgtaccc ctcacagccc tcaaaccctc tggtactgga gctgcaagtg 720

cacctggggg atgaagggga attcacctgt cgagctcaga actctctggg ttcccagcac 780

gtttccctga acctctccct gcaacaggag tacacaggca aaatgaggcc tgtatcagga 840

gtgttgctgg gggcggtcgg gggagctgga gccacagccc tggtcttcct ctccttctgt 900

gtcatcttca ttgtagtgag gtcctgcagg aagaaatcgg caaggccagc agcggacgtg 960

ggagacatag gcatgaagga tgcaaacacc atcaggggct cagcctctca gggtaacctg 1020

actgagtcct gggcagatga taacccccga caccatggcc tggctgccca ctcctcaggg 1080

gaggaaagag agatccagta tgcacccctc agctttcata agggggagcc tcaggaccta 1140

tcaggacaag aagccaccaa caatgagtac tcagagatca agatccccaa gtaagaaaat 1200

gcagaggctc gggcttgttt gagggttcac gacccctcca gcaaaggagt ctgaggctga 1260

ttccagtaga attagcagcc ctcaatgctg tgcaacaaga catcagaact tattcctctt 1320

gtctaactga aaatgcatgc ctgatgacca aactctccct ttccccatcc aatcggtcca 1380

cactccccgc cctggcctct ggtacccacc attctcctct gtacttctct aaggatgact 1440

actttagatt ccgaatatag tgagattgta acgtg 1475

<210> 135
<211> 2967
<212> DNA
<213> Homo sapiens

<400> 135
agtttctgag agaagaaccc tgaggaacag acgttccctg gcggccctgg cgccttcaaa 60

cccagacatg ctgctgctgc tgctgctgct gcccctgctc tgggggacaa aggggatgga 120
gggagacaga caatatgggg atggttactt gctgcaagtg caggagctgg tgacggtgca 180
ggagggcctg tgtgtccatg tgccctgctc cttctcctac ccccaggatg gctggactga 240
ctctgaccca gttcatggct actggttccg ggcaggagac agaccatacc aagacgctcc 300
agtggccaca aacaacccag acagagaagt gcaggcagag acccagggcc gattccaact 360
ccttggggac atttggagca acgactgctc cctgagcatc agagacgcca ggaagaggga 420
taaggggtca tatttctttc ggctagagag aggaagcatg aaatggagtt acaaatcaca 480
gttgaattac aaaactaagc agctgtctgt gtttgtgaca gccctgaccc ataggcctga 540
catcctcatc ctagggaccc tagagtctgg ccactccagg aacctgacct gctctgtgcc 600
ctgggcctgt aagcagggga caccccccat gatctcctgg attggggcct ccgtgtcctc 660
cccgggcccc actactgccc gctcctcagt gctcaccctt accccaaagc cccaggacca 720
cggcaccagc ctcacctgtc aggtgacctt gcctgggaca ggtgtgacca cgaccagtac 780
cgtccgcctc gatgtgtcct accctccttg gaacttgacc atgactgtct tccaaggaga 840
tgccacagca tccacagccc tgggaaatgg ctcatctctt tcagtccttg agggccagtc 900
tctgcgcctg gtctgtgctg tcaacagcaa tccccctgcc aggctgagct ggacccgggg 960
gagcctgacc ctgtgcccct cacggtcctc aaaccctggg ctgctggagc tgcctcgagt 1020
gcacgtgagg gatgaagggg aattcacctg ccgagctcag aacgctcagg gctcccagca 1080
catttccctg agcctctccc tgcagaatga gggcacaggc acctcaagac ctgtatcaca 1140
agtgacactg gcagcagtcg ggggagctgg agccacagcc ctggccttcc tgtccttctg 1200
catcatcttc atcatagtga ggtcctgcag gaagaaatcg gcaaggccag cagcgggcgt 1260
gggggataca ggcatggaag atgcaaaggc catcaggggc tcggcctctc agggacccct 1320
gactgaatcc tggaaagatg gcaaccccct gaagaagcct cccccagctg ttgccccctc 1380
gtcaggggag gaaggagagc tccattatgc aaccctcagc ttccataaag tgaagcctca 1440
ggacccgcag ggacaggagg ccactgacag tgaatactcg gagatcaaga tccacaagcg 1500
agaaactgca gagactcagg cctgtttgag gaatcacaac ccctccagca aagaagtcag 1560
aggctgattc tcatagaaca agaaccctct agagccccat gctatgcagt aggtcaccag 1620
ggctccctcc tcctgtctaa ccaaaacttg gaccaatgtc tcccctttcc ccggctacca 1680
gggacccatc cctgcctcta gcttctacta cccaccattc tcctctcgac ctctctgagg 1740
ttgactattt tagattccac atagagatga ggtcatgtgg tacttgcctc tctgtgtgtg 1800
gctcatttta cacaaaaaaa tatcccctag gttcatccat gttctctcaa atgacagaat 1860
caagcactga atattttttt ttctttgaga gatggagttt cgctctgttg cccaggctgg 1920
agtgcagtgg ttcaatctct gctcactgca acctccacct cctgggttca aacgattctc 1980
ctgcctcagc ttcccaagta gctggtacta caggcgtgtg tcaccacgcc cagctaattt 2040
ttgtattttt tagtagagac ggggtttcac tataagtggg ccaggctagt ctcaaactcc 2100

tgacctcaag tgatctgcct gccttggcct cccaaagtgc tgggatttca ggcatgagcc 2160

accgcaccca gcttgcattg aatattttca aggagctaaa agaagatttt aaatggtctc 2220

acaaaaacag ataaatattt gcacagatgg gtgtgctaat cattgtgcct tgatggttcc 2280

acgatgtatc cgggtgtgga aatctcactg ggtctctctc aaggccactc ggctactcag 2340

gacagggctg gaatttaaag cctgtccgat tctgaggtct cttctctcat ctagcactga 2400

gtcaagcaat cagcaggctg ggcacccctt agccataagt tttcaggaaa taaattcctt 2460

gagggcattg acttttacaa aagagggagc agcaatggcc tagagtctca ggaacaagac 2520

aggtgcactg aggagatgaa ggccgggacc ccctgcccaa cctgtatggc gggtctgtac 2580

ttattttgtt tacccccaat ttaaaacgtt tttttttatt gcaggttgtt tgtttgatat 2640

ggtttggctg tgtccccacc caaatcttat ctagaattgt aatcagaatt ataatcccca 2700

tgtgttgggg gagggacctg gtgggaggtg ataggatcat gggggtggtt cccccatgct 2760

gttctgatag tgagtgagtt atcacgagat ctgatggttt tgtaagtggt ggtttcccct 2820

gctcttctct cttgcctgcc accatgtaag atgtgcctgg ttccccttcc gccatgattg 2880

taagtttcct gaggcctccc ccgccatgtg gaactgtgag tcaattacac ctctttcatt 2940

tataaattaa aaaaaaaaaa aaaaaaa 2967

<210> 136
<211> 1709
<212> DNA
<213> Homo sapiens

<400> 136
agttcctgag agaagaaccc tgaggaacag acgttccctc gcggccctgg cacctctaac 60

cccagacatg ctgctgctgc tgctgcccct gctctggggg agggagaggg cggaaggaca 120

gacaagtaaa ctgctgacga tgcagagttc cgtgacggtg caggaaggcc tgtgtgtcca 180

tgtgccctgc tccttctcct acccctcgca tggctggatt taccctggcc cagtagttca 240

tggctactgg ttccgggaag gggccaatac agaccaggat gctccagtgg ccacaaacaa 300

cccagctcgg gcagtgtggg aggagactcg ggaccgattc cacctccttg gggacccaca 360

taccaagaat tgcaccctga gcatcagaga tgccagaaga agtgatgcgg ggagatactt 420

ctttcgtatg gagaaaggaa gtataaaatg gaattataaa catcaccggc tctctgtgaa 480

tgtgacagcc ttgacccaca ggcccaacat cctcatccca ggcaccctgg agtccggctg 540

cccccagaat ctgacctgct ctgtgccctg ggcctgtgag caggggacac cccctatgat 600

ctcctggata gggacctccg tgtcccccct ggacccctcc accacccgct cctcggtgct 660

caccctcatc ccacagcccc aggaccatgg caccagcctc acctgtcagg tgaccttccc 720

tggggccagc gtgaccacga acaagaccgt ccatctcaac gtgtcctacc cgcctcagaa 780

cttgaccatg actgtcttcc aaggagacgg cacagtatcc acagtcttgg gaaatggctc 840

atctctgtca ctcccagagg gccagtctct gcgcctggtc tgtgcagttg atgcagttga 900

cagcaatccc cctgccaggc tgagcctgag ctggagaggc ctgaccctgt gcccctcaca 960

gccctcaaac ccgggggtgc tggagctgcc ttgggtgcac ctgagggatg cagctgaatt 1020

cacctgcaga gctcagaacc ctctcggctc tcagcaggtc tacctgaacg tctccctgca 1080

gagcaaagcc acatcaggag tgactcaggg ggtggtcggg ggagctggag ccacagccct 1140

ggtcttcctg tccttctgcg tcatcttcgt tgtagtgagg tcctgcagga agaaatcggc 1200

aaggccagca gcgggcgtgg gagatacggg catagaggat gcaaacgctg tcaggggttc 1260

agcctctcag gggcccctga ctgaaccttg ggcagaagac agtcccccag accagcctcc 1320

cccagcttct gcccgctcct cagtggggga aggagagctc cagtatgcat ccctcagctt 1380

ccagatggtg aagccttggg actcgcgggg acaggaggcc actgacaccg agtactcgga 1440

gatcaagatc cacagatgag aaactgcaga gactcaccct gattgaggga tcacagcccc 1500

tccaggcaag ggagaagtca gaggctgatt cttgtagaat taacagccct caacgtgatg 1560

agctatgata acactatgaa ttatgtgcag agtgaaaagc acacaggctt tagagtcaaa 1620

gtatctcaaa cctgaatcca cactgtgccc tcccttttat ttttttaact aaaagacaga 1680

caaattccta aaaaaaaaaa aaaaaaaaa 1709

<210> 137
<211> 2856
<212> DNA
<213> Homo sapiens

<400> 137
tagtcgcggg tccccgagtg agcacgccag ggagcaggag accaaacgac gggggtcgga 60

gtcagagtcg cagtgggagt ccccggaccg gagcacgagc ctgagcggga gagcgccgct 120

cgcacgcccg tcgccacccg cgtacccggc gcagccagag ccaccagcgc agcgctgcca 180

tggagcccag cagcaagaag ctgacgggtc gcctcatgct ggctgtggga ggagcagtgc 240

ttggctccct gcagtttggc tacaacactg gagtcatcaa tgccccccag aaggtgatcg 300

aggagttcta caaccagaca tgggtccacc gctatgggga gagcatcctg cccaccacgc 360

tcaccacgct ctggtccctc tcagtggcca tcttttctgt tgggggcatg attggctcct 420

tctctgtggg ccttttcgtt aaccgctttg gccggcggaa ttcaatgctg atgatgaacc 480

tgctggcctt cgtgtccgcc gtgctcatgg gcttctcgaa actgggcaag tcctttgaga 540

tgctgatcct gggccgcttc atcatcggtg tgtactgcgg cctgaccaca ggcttcgtgc 600

ccatgtatgt gggtgaagtg tcacccacag cctttcgtgg ggccctgggc accctgcacc 660

agctgggcat cgtcgtcggc atcctcatcg cccaggtgtt cggcctggac tccatcatgg 720

gcaacaagga cctgtggccc ctgctgctga gcatcatctt catcccggcc ctgctgcagt 780

gcatcgtgct gcccttctgc cccgagagtc cccgcttcct gctcatcaac cgcaacgagg 840

agaaccgggc caagagtgtg ctaaagaagc tgcgcgggac agctgacgtg acccatgacc 900

tgcaggagat gaaggaagag agtcggcaga tgatgcggga gaagaaggtc accatcctgg 960

agctgttccg ctcccccgcc taccgccagc ccatcctcat cgctgtggtg ctgcagctgt 1020

cccagcagct gtctggcatc aacgctgtct tctattactc cacgagcatc ttcgagaagg 1080
cgggggtgca gcagcctgtg tatgccacca ttggctccgg tatcgtcaac acggccttca 1140
ctgtcgtgtc gctgtttgtg gtggagcgag caggccggcg gaccctgcac ctcataggcc 1200
tcgctggcat ggcgggttgt gccatactca tgaccatcgc gctagcactg ctggagcagc 1260
taccctggat gtcctatctg agcatcgtgg ccatctttgg ctttgtggcc ttctttgaag 1320
tgggtcctgg ccccatccca tggttcatcg tggctgaact cttcagccag ggtccacgtc 1380
cagctgccat tgccgttgca ggcttctcca actggacctc aaatttcatt gtgggcatgt 1440
gcttccagta tgtggagcaa ctgtgtggtc cctacgtctt catcatcttc actgtgctcc 1500
tggttctgtt cttcatcttc acctacttca aagttcctga gactaaaggc cggaccttcg 1560
atgagatcgc ttccggcttc cggcaggggg gagccagcca aagtgataag acacccgagg 1620
agctgttcca tcccctgggg gctgattccc aagtgtgagt cgccccagat caccagcccg 1680
gcctgctccc agcagcccta aggatctctc aggagcacag gcagctggat gagacttcca 1740
aacctgacag atgtcagccg agccgggcct ggggctcctt tctccagcca gcaatgatgt 1800
ccagaagaat attcaggact taacggctcc aggattttaa caaaagcaag actgttgctc 1860
aaatctattc agacaagcaa caggttttat aattttttta ttactgattt tgttattttt 1920
atatcagcct gagtctcctg tgcccacatc ccaggcttca ccctgaatgg ttccatgcct 1980
gagggtggag actaagccct gtcgagacac ttgccttctt cacccagcta atctgtaggg 2040
ctggacctat gtcctaagga cacactaatc gaactatgaa ctacaaagct tctatcccag 2100
gaggtggcta tggccacccg ttctgctggc ctggatctcc ccactctagg ggtcaggctc 2160
cattaggatt tgccccttcc catctcttcc tacccaacca ctcaaattaa tctttcttta 2220
cctgagacca gttgggagca ctggagtgca gggaggagag gggaagggcc agtctgggct 2280
gccgggttct agtctccttt gcactgaggg ccacactatt accatgagaa gagggcctgt 2340
gggagcctgc aaactcactg ctcaagaaga catggagact cctgccctgt tgtgtataga 2400
tgcaagatat ttatatatat ttttggttgt caatattaaa tacagacact aagttatagt 2460
atatctggac aagccaactt gtaaatacac cacctcactc ctgttactta cctaaacaga 2520
tataaatggc tggtttttag aaacatggtt ttgaaatgct tgtggattga gggtaggagg 2580
tttggatggg agtgagacag aagtaagtgg ggttgcaacc actgcaacgg cttagacttc 2640
gactcaggat ccagtccctt acacgtacct ctcatcagtg tcctcttgct caaaaatctg 2700
tttgatccct gttacccaga gaatatatac attctttatc ttgacattca aggcatttct 2760
atcacatatt tgatagttgg tgttcaaaaa aacactagtt ttgtgccagc cgtgatgctc 2820
aggcttgaaa tcgcattatt ttgaatgtga agggaa 2856

<210> 138
<211> 1882
<212> DNA
<213> Homo sapiens

<400> 138
agttccgcgc ggggctgtcg gggaaccatg gctgccccga gagacaatgt cactttatta 60

ttcaagttat actgcttggc agtgatgacc ctgatggctg cagtctatac catagcttta 120

agatacacaa ggacatcaga caaagaactc tacttttcaa ccacagccgt gtgtatcaca 180

gaagttataa agttattgct aagtgtggga attttagcta aagaaactgg tagtctgggt 240

agattcaaag catctttaag agaaaatgtc ttggggagcc ccaaggaact gttgaagtta 300

agtgtgccat cgttagtgta tgctgttcag aacaacatgg ctttcctagc tcttagcaat 360

ctggatgcag cagtgtacca ggtgacctac cagttgaaga ttccgtgtac tgctttatgc 420

actgttttaa tgttaaaccg gacactcagc aaattacagt gggtttcagt ttttatgctg 480

tgtgctggag ttacgcttgt acagtggaaa ccagcccaag ctacaaaagt ggtggtggaa 540

caaaatccat tattagggtt tggcgctata gctattgctg tattgtgctc aggatttgca 600

ggagtatatt ttgaaaaagt tttaaagagt tcagatactt ctctttgggt gagaaacatt 660

caaatgtatc tatcagggat tattgtgaca ttagctggcg tctacttgtc agatggagct 720

gaaattaaag aaaaaggatt tttctatggt tacacatatt atgtctggtt tgtcatcttt 780

cttgcaagtg ttggtggcct ctacacttct gttgtggtta agtacacaga caacatcatg 840

aaaggctttt ctgcagcagc ggccattgtc ctttccacca ttgcttcagt aatgctgttt 900

ggattacaga taacactcac ctttgccctg ggtactcttc ttgtatgtgt ttccatatat 960

ctctatggat tacccagaca agacactaca tccatccaac aaggagaaac agcttcaaag 1020

gagagagtta ttggtgtgtg attttagcct cacgtgagac tccttttaag actaaaccat 1080

ttgcattaaa ctagagcctt aagtcaatct cagaaggtag cataaacaaa taaaaattaa 1140

ctgtatggca tgatcagtgc ggttatgtgg aaacaacaac aaacaaacga agctatctga 1200

gtgaactgct aatacagaaa cttaatgtag acctgtttgg ggtctactat tgttttagaa 1260

tgaaggaatt gtattattgt gtgtatatat aatttgtaaa taaaaagtat ggagatgata 1320

cggtgttaaa aaaaatcatg gtgaggctac aatactcaag taacaaggtt tgggacaatg 1380

tctaagggtt aaagtgccaa agccatttct gtactaactg ttctcttgtt ccggtaccgg 1440

ggagaaggat gacccctcct tattctccaa ttcatgtaca gtattttgtc ctagcagcat 1500

aaagacctag ctcttttctt acaagaggca gaaacaagac aggctagttc ataaacaaac 1560

tgaatgtgta actaacttct caaaatgaat ctatttcata actcggacaa tttctgggtg 1620

gtgactgagt acccctttag tgagtacccc tttagtgcta tatttgtgcc attcattatc 1680

tggttcatat ttctttgctg ttagatgata cacatttctt caaaaaaatt tctaatgtca 1740

cttttgtact tttttaaata aagtatgttt aactgttggg ctctcaataa tttgtgaaat 1800

ttcagtgttt tctataatgt taatggggaa attcagcaat aaactttatt tgcaaaaaaa 1860

aaaaaaaaaa aaaaaaaaaa aa 1882

<210> 139

<211>  1451
<212>  DNA
<213>  Homo sapiens

<400>  139
caacatggca gcggttgggg ctggtggttc caccgcggcg cccgggccag gggcggtttc      60

cgcgggtgca ttggagccgg ggaccgccag tgcggctcac aggcgcctga agtacatatc     120

cctagctgtg ctggtggtcc agaatgcctc cctcatcctc agcatccgct acgcccgcac     180

gttgccaggg gaccgcttct ttgccaccac tgctgtggtc atggcggaag tgctcaaagg     240

tctcacctgc ctgctgctgc tcttcgcaca gaagaggggt aacgtgaagc acctggttct     300

cttcctccat gaggctgtcc tggtgcagta tgtggacacg ctcaagctcg cagtgccctc     360

tctcatctac accttgcaga ataacctcca gtatgttgcc atctctaacc taccagctgc     420

cactttccag gtgacatacc agctgaagat cctgaccaca gcgctgttct ccgtgctcat     480

gctgaatcgc agcctttccc ggctgcagtg ggcctccctg ctgctcctct tcactggcgt     540

cgccattgtc caggcacagc aagccggtgg gggaggccca cggccactgg atcagaaccc     600

tggggcaggc ctggcagccg tcgtggcctc ctgtctctcc tccggcttcg caggtgtcta     660

ctttgagaag atcctcaaag gcagctcagg ctccgtgtgg ctgcgcaacc tgcaactggg     720

cctcttcggc acagcactgg gcctggtggg gctctggtgg gctgagggta ccgccgtggc     780

cacccgtggt ttcttttttg ggtacacacc tgctgtctgg ggcgtggtgc tcaaccaggc     840

cttcggcggg ctactggtgg ctgtggttgt caagtacgct gacaatatcc tcaagggctt     900

tgccacctcc ctgtccattg tgctgtccac tgttgcctcc attcgcctct ttggcttcca     960

cgtggaccca ttatttgccc ttggcgctgg actcgtcatt ggtgctgtct acctctacag    1020

ccttccccga ggtgcagcca aagccatagc ctctgcctct gcctccgcct ccgggccctg    1080

cgttcaccag cagcctcccg ggcagccacc accaccgcag ctgtcttccc accgtggaga    1140

cctcatcacg gagccctttc tgccaaagtt gctcaccaag gtgaagggtt cctagccgct    1200

gggattgaag acattggcct ggcctcgttc tctcttcttg cccttggccc agctgggacc    1260

aaactctgat cagtattagg ggtagagtga ggtagacact ggactccctg tccccaccac    1320

ccctgcccca cccagggcca acatgactaa gctctctcat gacccacctc agctcagccc    1380

cccagcccct gccagctcca cactatctct tagctgagtt tttgcaaata aaatgtgttg    1440

tgtatcttgc c                                                         1451

<210>  140
<211>  2149
<212>  DNA
<213>  Homo sapiens

<400>  140
   ccgcgggaac ccggaaacgc ctgggctgca ccgagctggc ggtggctacg gcgacgggag      60
   ccggcggcgc tgcgggtcag cggtcgcgta ggacccagcg gactcggcag cctggggcgc     120
   ccggcggagc tgaaccgcgg cccccggtgg tgggctcagc cggtcgagct gcgcgggagg     180
   caaatgaaga taaaacaatg ttcgccaacc taaaatacgt ttccctggga attttggtct 240
   ttcagactac cagtttggtt ctaacaatgc gttattccag aactttaaaa gaagaaggac 300
   ctcgttatct atcttctaca gcagtggttg ttgctgaact tttgaagata atggcctgca 360
   ttttattggt ctacaaagac agcaaatgta gtctaagagc actgaatcga gtactacatg 420
   atgaaattct taataaacct atggaaacac ttaaacttgc tattccatca gggatctata 480
   ctcttcagaa taatttactg tatgtggcac tatcaaatct agatgcagct acttatcagg 540
   tcacgtatca gttaaaaatt cttacaacag cattattttc tgtgtctatg cttagtaaaa 600
   aattgggtgt ataccagtgg ctgtccctag taattttgat gacaggagtt gcttttgtac 660
   agtggccctc agattctcag cttgattcta aggaactttc agctggttct caatttgtag 720
   gactcatggc agttctcaca gcatgttttt caagtggctt tgctggggtt tactttgaga 780
   aaatcttaaa agaaacaaaa caatcagtgt ggataagaaa tattcagctt ggtttctttg 840
   gaagtatatt tggattaatg ggtgtataca tttatgatgg agaactggta tcaaagaatg 900
   gattttttca gggatataac cgactgacct ggatagtagt tgttcttcag gcacttggag 960
   gccttgtaat agctgctgtt attaagtatg cagataatat tttaaaagga tttgcaacct 1020
   ctttatcgat aatattatca acattgatct cctatttttg gcttcaagat tttgtgccaa 1080
   ccagtgtctt tttccttgga gccatccttg taataacagc tacttttttg tatggttatg 1140
   atcccaaacc tgcaggaaat cccactaaag catagttgta tactatcttt aactggtttt 1200
   tcacgatggg gcactaggaa tctcgacatt aatcttgcac agaggacttc tacagagtct 1260
   gagaagatat catcatgctg aatctgatca tactgttttt taaaagttta aggataagac 1320
   atgtgtatat gtaacaaaac acattgcatc tagaaatcaa aacttgaaag tatttccagg 1380
   gattaggatt agaaggaata ttagaggaaa cttgaaatct gagtttaaaa agattttacc 1440
   tttttgattg ctgcagaaat gtcctatgca ctctttgcaa gagcacacaa caaatgtcag 1500
   ataccaattt ttgcaaatta gatttaatct tattaaatgt ttttatctta ctctttctgt 1560
   acagatatat caaatcacat gaaatattta aagtttgaaa attataatta cctataaagc 1620
   tgtgaaaaat agaagtataa tttgaaaaaa catttcactt atcagagatt tttatattta 1680
   tacaaaagat tactaaatga aggattgcta aatgtttttg gttcaattac ataaaaatta 1740
   atattctggg tctgatctgt cagagaataa atatcaaatc taaatttaat gtagagatac 1800
   atactatttc tccatatgaa ttttaagata ttttagtgct tcaagactgc tgaaagcaat 1860
   ccagttgctc ctgtgctaga tggtagccag agaattttat agtaatggag gttagccctt 1920
   aatctcttca ttgcatttca tttctgtaaa tcagattaag tccttaatat tattttaaat 1980
   taaaatttgt gtgtaattgc cattaaattt tcaaaatgta atttaaaagg attaaatact 2040
   catttaataa tttaaaataa ttattgtata atatctacat ttggagaatt ttgaactatc 2100
   aagcatatac tgtatacagt tagaaagtta ttaaatgaac attttactc 2149

<210> 141

<211>  1272
<212>  DNA
<213>  Homo sapiens

<400>  141
cccgccgcca ggccaccgct gctgcggcag ccatggcgga agttcataga cgtcagcatg      60

ctcgggttaa aggagaagcc cccgcgaaat cctccacact ccgagatgag gaggagctgg     120

ggatggcgtc ggccgaaacg ctgaccgtgt ttctgaagct gctggccgcc ggcttttacg     180

gcgtgagctc cttcctgatc gtggtggtga ataagagcgt gctcaccaat tacagatttc     240

cctcctcact atgtgttgga cttggccaga tggtggccac agtggcagtt ctctgggtgg     300

gaaaggcgct cagagtagtc aagtttcctg accttgacag aaatgtacct cgaaagacgt     360

ttccactacc tctactatat tttgggaacc aaatcacggg actgttcagc acaaagaaac     420

tgaacttgcc aatgtttaca gttctgagaa ggttctccat cctgtttaca atgtttgctg     480

aaggagtttt actcaagaag actttttctt ggggtattaa aatgactgta tttgcaatga     540

ttattggagc ctttgtagct gccagctctg acttggcatt tgatctggaa ggatatgctt     600

ttattctgat aaacgatgtc ctaacagcag caaatggtgc atacgtaaaa caaaaattag     660

attcaaaaga gctgggaaaa tatggactgc tctattacaa tgcactgttc atgattctgc     720

ccaccctggc cattgcgtat ttcacaggag atgcacaaaa ggctgtggag tttgaaggct     780

gggctgacac cctctttctt ctgcagttca ccctctcctg tgtgatgggg tttatcttaa     840

tgtacgccac agtactctgc acgcagtata attctgctct tacaactaca atagttggct     900

gtattaagaa tatattaata acttatattg gaatggtctt tggtggagat tatattttca     960

cgtggacaaa cttcattggt ttaaatatca gcattgctgg gagcctggta tattcctata    1020

tcactttcac tgaagagcag ctgagcaaac agtcagaggc taataacaag ctggacatta    1080

aggggaaagg agcagtgtga ccagaggatt gcttcatctg atacgtaggc ctaagttttt    1140

aatcaactca gaacactggc agttcctaca gagacactga ggtgtcttga ttatggagga    1200

aataaccatt cctttgcact tgtacaatct gaggattgat tgctgccttt taaaatttta    1260

tgaagagaga aa                                                        1272

<210>  142
<211>  2911
<212>  DNA
<213>  Homo sapiens

<400>  142
ctccagcctc cgccggcgga gcccactatg ccagacagtt tcgacacttt gcaaagacaa      60

agagccctag accggaggga ggaggaggag gaagaagagc ggagagagaa ggaagaggcg     120

atgtgagctg ggaagggggc aagtgtccgg gacacccaca cccctgtatt ctcctccgaa     180

ccccttcatg cccaaatccc ggaaactcca gcgtgtctcc agccgtgttg gtaccatttt     240

cagatttcat cttcctaaac tggaaatgtc aatgagagga aattaacacc cccaagagct     300

gcagtgagca aatgcattga gcttgggtca ggacaattcc atttggggac cagagatgga     360

cggtcactca gcctatggag atgaagaaac tgaggttcag agaggttaag agactccact 420
gaggtcacac agccgatgac agacaacctt ctgtgccttc atcaagctgg ttgtgtaccc 480
accatgtccc tggcgacagg atgggaaaga aaaagcccta attaaggatc gtcagaaacc 540
acagttggag gaggacggca gagacagttt ccctccccgc tataccaaca cccttccttc 600
gaggtcctcg ctcctgaggg accctggact gtcacagaga ttaatgaccc cttatcttct 660
ttggatgtga aaggaaatca ctggttaaag cttgatcgag agacattatc agctctttaa 720
ggattgcaga agaataggct actttatttt ctgaaaaggt aaatatatgc aagcaaagcc 780
aacatgccac gaatggcgtt ggtctaccac acagccgtgt ctgggacaca gttgggggtc 840
atcccccagc aggagtgaag tcgagcttag cggcccttgt gtcctccctt ggaattcctg 900
ccatcccttt tgattgagcc tccacctctg ggatttttct tccatttttc tcctctctta 960
ggagggagtt cctgctaccc atcgtgggag gccaccatca ggactgcgaa gatggtgacc 1020
ctgcggaaga ggaccctgaa agtgctcacc ttcctcgtgc tcttcatctt cctcacctcc 1080
ttcttcctga actactccca caccatggtg gccaccacct ggttccccaa gcagatggtc 1140
ctggagctct ccgagaacct gaagagactg atcaagcaca ggccttgcac ctgcacccac 1200
tgcatcgggc agcgcaagct ctcggcctgg ttcgatgaga ggttcaacca gaccatgcag 1260
ccgctgctga ccgcccagaa cgcgctcttg gaggacgaca cctaccgatg gtggctgagg 1320
ctccagcggg agaagaagcc caataacttg aatgacacca tcaaggagct gttcagagtg 1380
gtgcctggga atgtggaccc tatgctggag aagaggtcgg tgggctgccg gcgctgcgcc 1440
gttgtgggca actcgggcaa cctgagggag tcttcttatg ggcctgagat agacagtcac 1500
gactttgtcc tcaggatgaa caaggcgccc acggcagggt ttgaagctga tgttgggacc 1560
aagaccaccc accatctggt gtaccctgag agcttccggg agctgggaga taatgtcagc 1620
atgatcctgg tgcccttcaa gaccatcgac ttggagtggg tggtgagcgc catcaccacg 1680
ggcaccattt cccacaccta catcccggtt cctgcaaaga tcagagtgaa acaggataag 1740
atcctgatct accacccagc cttcatcaag tatgtctttg acaactggct gcaagggcac 1800
gggcgatacc catctaccgg catcctctcg gtcatcttct caatgcatgt ctgcgatgag 1860
gtggacttgt acggcttcgg ggcagacagc aaagggaact ggcaccacta ctgggagaac 1920
aacccatccg cgggggcttt tcgcaagacg ggggtgcacg atgcagactt tgagtctaac 1980
gtgacggcca ccttggcctc catcaataaa atccggatct tcaaggggag atgacgcagt 2040
gaagggctga ggatggacgc actgtcacac ctctgcattt ccagccccag catcttgctg 2100
gagccgttcc atcccggagc ttggaggggc agcctcaggt gtgtgcctgg gcaccgctca 2160
cagcctcttg cacccagccg ttggcagcat ctactcagca aggtcactaa gctctgccag 2220
cgtggcagag catgtcttgg aacctgtctt gagtggggac aacgtccccc cactgctgcc 2280
ctagagctgg ggagacgctg ggaaaggttc aacctccaca cactaaaatc attttggctc 2340
ctggggcaag cttggggaat gaatgtggaa gatgcctata ttctgagaga caggacagtt 2400

tcccaggaag atgggcagag acttgagtgg cgattacctc cagcacagag acgtgccagg 2460

cggtgttggc gctcggggcg agatgctgcc cttctttgca cgaagcctgg cctcttgctt 2520

ggcgtgataa ccctgtcatc ttcccaaagc tcatttatga gccaccagag gctcctaccc 2580

caaagatttt cacagaaact tgaggccagg tgccgtggct cacacctgta atctgaacac 2640

tttgggaggc cgaggcggga ggatcacttg agcccaggag ttcaagacca gcctgggcaa 2700

catagtgaga ctcctgtctc tacaaaaata aaagatttaa aaaaattagc caggcacggt 2760

ggcacacact tgtagcccca gctactaggg aggctgagga gggaggatct cttgtgccta 2820

ggagttcgag gctgcagtgg gctgtgatca caccactgca ctccagcctg ggcaacagag 2880

tgagaccctg tctctgaaaa aaaaaaaaaa a 2911

<210> 143
<211> 2479
<212> DNA
<213> Homo sapiens

<400> 143
cgggcggacg agagagctgc gagctaacgg gtccggacga cggcggcggc ggcggcggcg 60

gcggcgacgg ctttctgcgc gggcggcggc ggcggccgga gcggcgagca tggcccgcgc 120

ggcgccgggc gctgactgag ggtcgtgctg cggcagcctg gcgcccgaca tgtgaagggg 180

aacctgccag gggattactg tgagctcaca gcaggatagg ctggccccgg gggagtgggg 240

atgggcagaa cctgggccag gagtattagg aaggagacct ggaccctggt agggagctca 300

gcacaagctc gcctgaccca gatggtattg gacacagtgc cccgctgaac agcagctgtt 360

cccagcgcca gaggacccca cagccacagt aggagctttg cgacccctac gcaactgcag 420

ctggggctgg ggccaccagg agaagggacc agcacctcag atgggaagca gaagggcaag 480

acctactgcg gaaaggatct cgatctctgc agcggagttt tcccacactc acagagcttc 540

ctgtttttgt cctctcaggg ttggggtctg gctcccctcc tctggcagag gttttgggcc 600

aggaggtggg acaacacttg catggaaccc aagtcccctg aagcccctgt gctctctgtt 660

gtgattggag ttctagggct tccccgctgc cgggaacgag gggccgcctc attgtctgct 720

catgaaccac aaggaccccg actgctccag actggattat ttcaagccgc caaagagggg 780

gacccctaga gctggcagcc agcaatccca agggactaga gggctgcaat ggactgacct 840

ccccctcacc agggtggcag gagaggcaga gcctctgtgg cctagctagt gacggagaga 900

cccgatgaag ccctaagcag gggccccgcc tgactcaggg acaggacagc cactcctgcc 960

aacgtgtgtt ctccctacat gagggagggc gtggcaaggg acccctgcca ctgtcccctg 1020

ctgcagcacg tgcccctatg ccctttgcat gtggtgccag aataggcagg ctacgccgtg 1080

gctggcccct cagcgggctg ggaaaagagt ggccacggtg accgtcaccc gcctgccggc 1140

accatgaagt gctccctgcg ggtgtggttc ctctccgtgg ccttcctgct ggtgttcatc 1200

atgtccctgc tcttcaccta ctcgcaccac agcatggcca cgctccccta cctggactca 1260

ggggccctgg atgggacgca ccgggtgaag ctggtgcccg gctatgccgg cctgcagcgc 1320

ctcagcaagg agaggctctc gggcaagagc tgtgcctgtc gccgctgcat gggcgatgcc 1380
ggtgcctccg actggtttga cagccacttt gacggtaaca tttcccccgt ctggacccga 1440
gagaacatgg atcttccacc ggacgtccag aggtggtgga tgatgctgca gccccagttc 1500
aagtcacaca acaccaatga ggtgctggag aagctgttcc agatagtgcc tggcgagaac 1560
ccctaccgct tccgggaccc ccaccagtgc cggcgctgtg ccgtggtggg gaactcgggc 1620
aacctgcggg gctctggcta tgggcaggac gtggacgggc acaacttcat catgaggatg 1680
aatcaggcgc caaccgtggg ctttgagcag gatgttggca gccgaaccac ccaccatttc 1740
atgtaccctg agagtgccaa gaacctgccc gccaacgtca gcttcgtgct ggtgcccttc 1800
aaggtcctgg accttctgtg gatcgccagc gccttgtcca cggggcagat ccgattcacc 1860
tacgccccag tgaagtcctt ccttcgagtg gataaagaaa aggtccagat ctacaaccca 1920
gccttcttca agtatatcca cgacaggtgg acagagcatc acgggcggta cccttccacg 1980
gggatgctgg tgcttttctt tgccctgcat gtgtgtgatg aggtgaacgt gtacgggttc 2040
ggggccgaca gccggggcaa ctggcaccac tactgggaga acaaccggta cgcgggcgag 2100
ttccggaaga ctggcgtgca cgacgcggac ttcgaggccc acatcatcga catgctggcc 2160
aaggccagca agatcgaagt ctaccggggc aactgagccg ggcctcgccg cgacccttcc 2220
ggcccatcta tcgggcaccg gggctccggc ccgggaccca ggaccagcaa cccgcgacca 2280
atcatgctgc agcccagggg cgtctgctgt gccccgccaa tcacgagact gggggaccgg 2340
ccgggcctgg caccaatctg cgctgcggtc gggcggagct tctgtttctc ccagccaatc 2400
atgtgactca aggaaaactt ccggcgctgt gtccagtctc ctccaatcaa tggccttcgg 2460
gggcgggcca gcggccgct 2479

<210> 144
<211> 2271
<212> DNA
<213> Homo sapiens

<400> 144
actatggcgg cgcccatgca gcccagcgcg ttgtgggctc ccgccggggt cccccgcggc 60
tgtcgccgcc gcctacgccg ctgcctccgc cttcctgccc cgcgtcgggc cgggcgccac 120
ctcccccctg cctccctctc cgctgtggtc atttaggaaa tcgtaaatca tgtgaagatg 180
ggactcttgg tatttgtgcg caatctgctg ctagccctct gcctctttct ggtactggga 240
tttttgtatt attctgcgtg gaagctacac ttactccagt gggaggagga ctccaattca 300
gtggttcttt cctttgactc cgctggacaa acactaggct cagagtatga tcggttgggc 360
ttcctcctga atctggactc taaactgcct gctgaattag ccaccaagta cgcaaacttt 420
tcagagggag cttgcaagcc tggctatgct tcagccttga tgacggccat cttcccccgg 480
ttctccaagc cagcacccat gttcctggat gactcctttc gcaagtgggc tagaatccgg 540
gagttcgtgc cgccttttgg gatcaaaggt caagacaatc tgatcaaagc catcttgtca 600

gtcaccaaag agtaccgcct gacccctgcc ttggacagcc tccgctgccg ccgctgcatc 660
atcgtgggca atggaggcgt tcttgccaac aagtctctgg ggtcacgaat tgacgactat 720
gacattgtgg tgagactgaa ttcagcacca gtgaaaggct ttgagaagga cgtgggcagc 780
aaaacgacac tgcgcatcac ctaccccgag ggcgccatgc agcggcctga gcagtacgag 840
cgcgattctc tctttgtcct cgccggcttc aagtggcagg actttaagtg gttgaaatac 900
atcgtctaca aggagagagt gagtgcatcg gatggcttct ggaaatctgt ggccactcga 960
gtgcccaagg agccccctga gattcgaatc ctcaacccat atttcatcca ggaggccgcc 1020
ttcaccctca ttggcctgcc cttcaacaat ggcctcatgg gccgggggaa catccctacc 1080
cttggcagtg tggcagtgac catggcacta cacggctgtg acgaggtggc agtcgcagga 1140
tttggctatg acatgagcac acccaacgca cccctgcact actatgagac cgttcgcatg 1200
gcagccatca aagagtcctg gacgcacaat atccagcgag agaaagagtt tctgcggaag 1260
ctggtgaaag ctcgcgtcat cactgatcta agcagtggca tctgagtggg cccagcacat 1320
ggccatagag gcccaggcac caccaggagc agcagccagc accacctaca caggagtctt 1380
cagacccaga gaaggacggt gccaagggcc ccaggggcag caaggccttg gtggagcagc 1440
cagagctgtg cctgctcagc agccagtctc agagaccagc actcagcctc attcagcatg 1500
ggtccttgat gccagagggc cagcaggctc ctggctgtgc ccagcaggcc cagcatgcag 1560
gtggtgggac actgggcagc aaggctgctg ccggaatcac ttctccaatc agtgtttggt 1620
gtattatcat tttgtgaatt tgggtagggg ggagggtagg gataatttat ttttaaataa 1680
ggttggagat gtcaagttgg gttcacttgc catgcaggaa gaggcccact agagggccca 1740
tcaggcagtg ttacctgtta gctccctgtg gggcaggagt gccaggacca gcctgtacct 1800
tgctgtgggg ctacaggatg gtgggcagga tctcaagcca gccccctcca gctcatgaca 1860
ctgtttggcc tttcttgggg agaaggcggg gtattcccac tcaccagccc tagctgtccc 1920
atggggaaac cctggagcca tcccttcgga gccaacaaga ccgccccagg gctatagcag 1980
aaagaacttt aaagctcagg agggtgacgc ccagctccgc ctgctgggaa gagctcccct 2040
ccacagctgc agctgatcca taggactacc gcaggcccgg actcaccaac ttgccacatg 2100
ttctaggttt cagcaacaag actgccaggt ggttgggttc tgcctttagc ctggaccaaa 2160
gggaagtgag gcccaaggag cttacccaag ctgtggcagc cgtcccaggc cacccccatg 2220
gaagcaataa agctcttccc tgaaaaaaaa aaaaaaaaaa aaaaaaaaaa a 2271

<210> 145
<211> 1766
<212> DNA
<213> Homo sapiens

<400> 145
cggtcaggtc cagcacttgg gagctgactg tgctggaggt gacaggcttt gcggggtccg 60
cctgtgtgca ggagtcgcaa ggtcgctgag caggacccaa aggtggcccg aggcagccgg 120
gatgacagct ctccccagga atcctgctgc ctgctgagaa acatggtcag caagtcccgc 180

tggaagctcc tggccatgtt ggctctggtc ctggtcgtca tggtgtggta ttccatctcc 240

cgggaagaca gtttttattt tcccatccca gagaagaagg agccgtgcct ccagggtgag 300

gcagagagca aggcctctaa gctctttggc aactactccc gggatcagcc catcttcctg 360

cggcttgagg attatttctg ggtcaagacg ccatctgctt acgagctgcc ctatgggacc 420

aaggggagtg aggatctgct cctccgggtg ctagccatca ccagctcctc catccccaag 480

aacatccaga gcctcaggtg ccgccgctgt gtggtcgtgg ggaacgggca ccggctgcgg 540

aacagctcac tgggagatgc catcaacaag tacgatgtgg tcatcagatt gaacaatgcc 600

ccagtggctg gctatgaggg tgacgtgggc tccaagacca ccatgcgtct cttctaccct 660

gaatctgccc acttcgaccc caaagtagaa aacaacccag acacactcct cgtcctggta 720

gctttcaagg caatggactt ccactggatt gagaccatcc tgagtgataa gaagcgggtg 780

cgaaagggtt tctggaaaca gcctcccctc atctgggatg tcaatcctaa acagattcgg 840

attctcaacc ccttcttcat ggagattgca gctgacaaac tgctgagcct gccaatgcaa 900

cagccacgga agattaagca gaagcccacc acgggcctgt tggccatcac gctggccctc 960

cacctctgtg acttggtgca cattgccggc tttggctacc cagacgccta caacaagaag 1020

cagaccattc actactatga gcagatcacg ctcaagtcca tggcggggtc aggccataat 1080

gtctcccaag aggccctggc cattaagcgg atgctggaga tgggagctat caagaacctc 1140

acgtccttct gacctgggca agagctgtag cctgtcggtt gcctactctg ctgtctgggt 1200

gacccccatg cgtggctgtg ggggtggctg gtgccagtat gacccacttg gactcacccc 1260

ctcttgggga gggagttctg ggcctggcca ggtctgagat gaggccatgc ccctggctgc 1320

tcttatggag ccgagatcca gtcagggtgg gggcgctgga gccgtgggag cccggccagg 1380

gcagggggct cgtcgctgtg gcaccccctc tctgccagca ccaagagatt atttaatggg 1440

ctatttaatt aaggggtagg aaggtgctgt gggctggtcc cacacatcca ggaaagaggc 1500

cagtagagaa ttctgcccac tttttataaa aacttacagc gatggcccca ccaaggccta 1560

gacacggcac tggcctccca ggagggcagg ggcattggga atgggtgggt gccctccaga 1620

gaggggctgc tacctcccag caggcatggg aagagcactg gtgtgggggt tccaccgaga 1680

aggggacctc atctagaaaa gaggttacaa acctaccatt aaactatttt tcctaaaacg 1740

gaaaaaaaaa aaaaaaaaaa aaaaaa 1766

<210> 146
<211> 2397
<212> DNA
<213> Homo sapiens

<400> 146
cacgcgcccc tcctccgcat ctgagcgggg gagcggcggc ccccagctga atgggcgcga 60

gagcggcgct gggggcgggt gggggcgcgg ggtaccgggc tggcggccgg ccggcgcccc 120

ctcattagta tgcggacgaa ggcggcgggc tgcgcggagc ggcgtcccct gcagccgcgg 180

accgaggcag cggcggcacc tgccggccga gcaatgccaa gtgagtacac ctatgtgaaa 240
ctgagaagtg attgctcgag gccttccctg caatggtaca cccgagctca aagcaagatg 300
agaaggccca gcttgttatt aaaagacatc ctcaaatgta cattgcttgt gtttggagtg 360
tggatccttt atatcctcaa gttaaattat actactgaag aatgtgacat gaaaaaaatg 420
cattatgtgg accctgacca tgtaaagaga gctcagaaat atgctcagca agtcttgcag 480
aaggaatgtc gtcccaagtt tgccaagaca tcaatggcgc tgttatttga gcacaggtat 540
agcgtggact tactcccttt tgtgcagaag gcccccaaag acagtgaagc tgagtccaag 600
tacgatcctc cttttgggtt ccggaagttc tccagtaaag tccagaccct cttggaactc 660
ttgccagagc acgacctccc tgaacacttg aaagccaaga cctgtcggcg ctgtgtggtt 720
attggaagcg gaggaatact gcacggatta gaactgggcc acaccctgaa ccagttcgat 780
gttgtgataa ggttaaacag tgcaccagtt gagggatatt cagaacatgt tggaaataaa 840
actactataa ggatgactta tccagagggc gcaccactgt ctgaccttga atattattcc 900
aatgacttat ttgttgctgt tttatttaag agtgttgatt tcaactggct tcaagcaatg 960
gtaaaaaagg aaaccctgcc attctgggta cgactcttct tttggaagca ggtggcagaa 1020
aaaatcccac tgcagccaaa acatttcagg attttgaatc cagttatcat caaagagact 1080
gcctttgaca tccttcagta ctcagagcct cagtcaaggt tctggggccg agataagaac 1140
gtccccacaa tcggtgtcat tgccgttgtc ttagccacac atctgtgcga tgaagtcagt 1200
ttggcgggtt ttggatatga cctcaatcaa cccagaacac ctttgcacta cttcgacagt 1260
caatgcatgg ctgctatgaa ctttcagacc atgcataatg tgacaacgga aaccaagttc 1320
ctcttaaagc tggtcaaaga gggagtggtg aaagatctca gtggaggcat tgatcgtgaa 1380
ttttgaacac agaaaacctc agttgaaaat gcaactctaa ctctgagagc tgtttttgac 1440
agccttcttg atgtatttct ccatcctgca gatactttga agtgcagctc atgtttttaa 1500
cttttaattt aaaaacacaa aaaaaatttt agctcttccc actttttttt tcctatttat 1560
ttgaggtcag tgtttgtttt tgcacaccat tttgtaaatg aaacttaaga attgaattgg 1620
aaagacttct caaagagaat tgtatgtaac gatgttgtat tgatttttaa gaaagtaatt 1680
taatttgtaa aacttctgct cgtttacact gcacattgaa tacaggtaac taattggaag 1740
gagaggggag gtcactcttt tgatggtggc cctgaacctc attctggttc cctgctgcgc 1800
tgcttggtgt gacccacgga ggatccactc ccaggatgac gtgctccgta gctctgctgc 1860
tgatactggg tctgcgatgc agcggcgtga ggcctgggct ggttggagaa ggtcacaacc 1920
cttctctgtt ggtctgcctt ctgctgaaag actcgagaac caaccaggga agctgtcctg 1980
gaggtccctg gtcggagagg gacatagaat ctgtgacctc tgacaactgt gaagccaccc 2040
tgggctacag aaaccacagt cttcccagca attattacaa ttcttgaatt ccttggggat 2100
tttttactgc cctttcaaag cacttaagtg ttagatctaa cgtgttccag tgtctgtctg 2160
aggtgactta aaaaatcaga acaaaacttc tattatccag agtcatggga gagtacaccc 2220

tttccaggaa taatgttttg ggaaacactg aaatgaaatc ttcccagtat tataaattgt 2280

gtatttaaaa aaaagaaact tttctgaatg cctacctggc ggtgtatacc aggcagtgtg 2340

ccagtttaaa aagatgaaaa agaataaaaa cttttgagga acaaaaaaaa aaaaaaa 2397

<210> 147
<211> 2107
<212> DNA
<213> Homo sapiens

<400> 147
gagcctgaga ctccgggcag ggctgctccc tcctctgctc ccccgccaga tccgcgggga 60

aggaatcgtg cccgcgccgc ccctggcccg cgccaccttc ctttggtttc tgccggcctc 120

gggcttctgc ggcccgatgt ggcaggcgcc gcgagagagg cagcagccgg ctggagcagc 180

ggcccctcag gtctcggagc ccggtgcgcc tctgcggtcg tcgctcctgg gcctcggcgg 240

gtcactcttg ccggccggct tcgctgcggg tttgcactgc ccggcatttg caagtgagga 300

agcagcacaa ccctgggttt tagataattt ctaacagaga ggccccagca attcagcagg 360

cagcgtcctg agtctttggc agcctggtcc ctctttcgca aattctccac ctttgcgaac 420

agagggtctt taggacacag attcttaaaa gtgcaggtga gccagccatg agagggtatc 480

ttgtggccat attcctgagt gctgtcttcc tctattatgt actgcattgc atattatggg 540

gaacgaatgt ctattgggtg gcacctgtgg aaatgaaacg gagaaataag atccagcctt 600

gtttatcaaa gccagctttt gcctctctgc tgaggtttca tcagtttcac ccttttctgt 660

gtgcggctga ttttagaaag attgcttcct tgtatggtag cgataagttt gatttgccct 720

atgggatgag aacatcagcg gaatattttc gacttgctct ttcaaaactg cagagttgtg 780

atctctttga tgagtttgac aacataccct gtaaaaagtg tgtggtggtt ggtaatggag 840

gagttttgaa gaataagaca ttaggagaaa aaatcgactc ctatgatgta ataataagaa 900

tgaataatgg tcctgtttta ggacatgaag aagaagttgg gagaaggaca accttccgac 960

ttttttatcc agaatctgtt ttttcagatc ctattcacaa tgaccctaat acgacagtga 1020

ttctcactgc ttttaagcca catgatttaa ggtggctgtt ggaattgttg atgggtgaca 1080

aaataaacac taatggtttt tggaagaaac cagccttaaa cctgatttat aaaccttatc 1140

aaatccgaat attagatcct ttcattatca gaacagcagc ttatgaactg cttcattttc 1200

caaaagtgtt tcccaaaaat cagaaaccta aacacccaac aacaggaatt attgccatca 1260

cattggcgtt ttacatatgt cacgaagttc acctagctgg ttttaaatac aacttttctg 1320

acctcaagag tcctttgcac tactatggga atgccaccat gtctttgatg aataagaacg 1380

cgtatcacaa tgtgactgca gagcagctct tttgaagga cattatagaa aaaaacctcg 1440

taatcaactt gactcaagat tgactctaca gactcagaag atgatgctaa cagtgttagt 1500

tttatttttg tactgcaatt tttagtttaa aatatgttgg atgcactcgt caaataatta 1560

tgtatactgt ctgttgctgc ctggtgattc ataaccacca gcttaatttc tgtgaatact 1620

gtatatttaa cttatgaaaa ccaagaaatg taaagataac aggaaaataa gttttgattg 1680

caatgttttt aaaataagct agttttctga ggtgttttca cacgtctttt tatagttact 1740

tcatcttaga tttttgaagg gatatgactt cctactaagg atttagttta ccacaacaat 1800

tctgactaca ataagacatt ttgaggagga tatttggcta ctgtaaacat ggctggtgga 1860

aaatcacgat tgtggcttga tgtggcaagc cgaaaccact tggctctgga aatctaagtt 1920

catactggtt taattaagct ctctcctgac aacccccaga attaaatgaa ccatgattgt 1980

gaagagtaat ttggtacaat gaaggcagtg tttgttttta agttaaagga aatgggctaa 2040

acataaagtt cttattagat aagtaaataa ctaaagaaag aatacaatta ctaaaaaaaa 2100

aaaaaaa 2107

<210> 148
<211> 4295
<212> DNA
<213> Homo sapiens

<400> 148
gaggaggttc ctgtagggcg gcacaaccag ggagggcgtg gaagctctgc atcccttctc 60

ccataccttg ctctacacat ctcttcatct gtatcctctg cagcatcctt gatgataaac 120

cagtaaatat gagttttgat catcctgaga aaaatgggcc ttggcctgca gacccaataa 180

accttccctc ccatggataa tagtgctaat tcctgaggac ctgaagggcc tgccgcccct 240

gggggattag ccagaagcag gcttgttttc ctgctcagaa caaagtgact tccctgaaca 300

catcttcatt atgattcaca ccaacctgaa gaaaaagttc agctgctgcg tcctggtctt 360

tcttctgttt gcagtcatct gtgtgtggaa ggaaaagaag aaagggagtt actatgattc 420

ctttaaattg caaaccaagg aattccaggt gttaaagagt ctggggaaat tggccatggg 480

gtctgattcc cagtctgtat cctcaagcag cacccaggac ccccacaggg gccgccagac 540

cctcggcagt ctcagaggcc tagccaaggc caaaccagag gcctccttcc aggtgtggaa 600

caaggacagc tcttccaaaa accttatccc taggctgcaa aagatctgga agaattacct 660

aagcatgaac aagtacaaag tgtcctacaa ggggccagga ccaggcatca agttcagtgc 720

agaggccctg cgctgccacc tccgggacca tgtgaatgta tccatggtag aggtcacaga 780

ttttcccttc aatacctctg aatgggaggg ttatctgccc aaggagagca ttaggaccaa 840

ggctgggcct tggggcaggt gtgctgttgt gtcgtcagcg ggatctctga agtcctccca 900

actaggcaga gaaatcgatg atcatgacgc agtcctgagg tttaatgggg cacccacagc 960

caacttccaa caagatgtgg gcacaaaaac taccattcgc ctgatgaact ctcagttggt 1020

taccacagag aagcgcttcc tcaaagacag tttgtacaat gaaggaatcc taattgtatg 1080

ggacccatct gtataccact cagatatccc aaagtggtac cagaatccgg attataattt 1140

ctttaacaac tacaagactt atcgtaagct gcaccccaat cagccctttt acatcctcaa 1200

gccccagatg ccttgggagc tatgggacat tcttcaagaa atctccccag aagagattca 1260

gccaaacccc ccatcctctg ggatgcttgg tatcatcatc atgatgacgc tgtgtgacca 1320

ggtggatatt tatgagttcc tcccatccaa gcgcaagact gacgtgtgct actactacca 1380
gaagttcttc gatagtgcct gcacgatggg tgcctaccac ccgctgctct atgagaagaa 1440
tttggtgaag catctcaacc agggcacaga tgaggacatc tacctgcttg gaaaagccac 1500
actgcctggc ttccggacca ttcactgcta agcacaggct cctcactctt ctccatcagg 1560
cattaaatga atggtctctt ggccacccca gcctgggaag aacattttcc tgaacaattc 1620
cagcctgctc cttttactct aggggcctct gtcagcaaga ccatggggac ttcaagagcc 1680
tgtggtcagg aaatcaggtc cagccttccc tgtagccaga cagtttatga gcccagagcc 1740
tcctgccaca cacatgcaca catatctagc attctttcca gacagcatcc tccccgcctt 1800
ccaccttggt agatgcaagg tctatctctc ccatcagggc tgccaaagct gggctttgtt 1860
tttcccagca gaatgatgcc attctcacaa accaatgctc tatattgctt gaagtctgca 1920
tctaaatatt gatttcacgt tttaaagaaa ttctcttaaa ttacaattgt gcccaatgca 1980
gggtggctct ggggggcaag taggtggtac aggggattgg aaacatgctc cgcgcctcca 2040
gagaaaagtt gctcccgagg tccatgcccc tggaacgtgt tcctatcact ctggctggtt 2100
gggctggtcc ttagactggg tgcttatgat taaagggtct tggttagccc actttccctc 2160
tccatgtgga gatggaaggt agagaaggat acagtgtcta tcctcaagtt gctacggttc 2220
agtgagagag gcagacatct gaacaggcag gtaggattca gtgtgctcag tgcactgggg 2280
atttggagag agatgggctt gctctctctg tgcacccagg agggccacgc acttaaaact 2340
gtgtttgtgg atcagagaag gctttatagc acagggggca ttcagatgag tcttagagga 2400
agagaagaaa catggcaagc agattacatc tgagccgttt gaattgtgtt tttctttctt 2460
cccatgttta ttttctaaga tctacctgaa cttagagact caagatattt ttttaggaaa 2520
cctcctaccc atgtctgagg tagcaagtgc agcctcacga cagataccag gcaatccaga 2580
gccacaaaac gtgattcctc caggctctgc ctggcctgac cctgtcctgt cagctgggtt 2640
tacataccag tcccattctt ccttttcaat acctaccccc aaatcttctc ctaaccacca 2700
tctgtttttt ttttttaaag cattttttgc tttaaaagca tcctgacccc aatttctttg 2760
agctcacggg ccttttgctg aaggtctctc agggtgtagt ggtgtggctc tctggactta 2820
acgtcactct cagaggtcag aaccttggag atcagaactg attctcacca ggtgtgagag 2880
gtgtggtagc agattgcaat gctctgcacc tcttccttgc aagtgagcaa cttcaggctc 2940
tctgggcaga ggctggccca ctgtagtttg cagacatgct ctccagatgg ttttactaag 3000
tcccctctcc ctgataggga atcctgctgg accagcgcag ccctggtgtg gagaggttaa 3060
aagacttgca caggatcacc aagtcatgct gtagagccag gattcctaga cccagggctc 3120
tgcactctca aggctggccc catgtgctca aggggatcta atgtttgggc tccaaactaa 3180
ccatctcgga gctgggctcc tcatttactg ccaaaccctc agcttatgta gctagaaagg 3240
gccctggagt gagaaagcct ggattttcaa attgatgctc ccctactgac tagctgtgcc 3300
actctgggca aatgctcttc cttgagcctg tttccacacc tgtaaagtgg ggatgatgat 3360

cctatctcac tgcttttgtg aggattacag gaaagcacct gtcctggctc tgtacctggc 3420
acgtagtagg tgctcagttc atgctggttt ccttcctgcc tttagtaggg acctgctctg 3480
tgctcacacc tcggctgcat gcaccctgct gtgacggagg ctagtgtgga agaggtcctg 3540
tcctcaggga attaactgtc ttattgggag acaacaactg tcctccttgg aacacccaag 3600
aaaccatgca aagcagtgga caacacagaa cacgccctcc tcctcgctgc ctgcagctcc 3660
aatctgattc tgcttgggaa tgggcggagc acgtgggctg cttaactgct gtataggaca 3720
agccccttac ccctctctgg gcccatgaat tcctggcttg gtttatgttc tgatttgaca 3780
cactgatttt aatcttcgaa tcatgacact gagtgcagag gaggtggcat tccgacagca 3840
ggacatacat gttggtgtga agactgggac gacactgggt agaatctagt ttttaattat 3900
tattaatata aaggatcaaa ttaatttaaa tatgattctg aagtctacag aacttttagt 3960
tctgtgctgt ctatgtggac actttggtaa aatgcaaatt atgatatgga cgttatcatt 4020
ggtctggtga gatgtttcat atttgtgaca gttaatttaa aaattatgag ttaatgctgc 4080
ctgtgtctat ggggttctgt cttctttgat agccatctat tcatctggat catgggaccc 4140
tctctaatcc ttccaccaat caaataagct attgctattg gtttggagtt gagatatcag 4200
tctcggaaac ttctgaaaaa tgctaataat tacccaagga ttatgtcaaa ttttaaaata 4260
aatgtgtgtg tgtttcttta aaaaaaaaaa aaaaa 4295

<210> 149
<211> 1793
<212> DNA
<213> Homo sapiens

<400> 149
ggcgccggga ctccctcctg gccgcccaca gcctgtgcgc attcctgcat tcctgccgcc 60
gcccgggacc cgagcccccg gaggtgtcca ggcgcggtgc caggcgggta ctgtgcaggt 120
tcattctgcc acccatctgc attaagacac aaggtgctga ccgcagagac ctgccatgaa 180
accacacttg aagcaatgga gacaacgaat gcttttcgga atattcgctt gggggctcct 240
ctttttgctg attttcatct acttcaccga cagcaacccc gctgagcctg tacccagctc 300
cctctccttc ctggagacca ggaggctcct gccggtgcag gggaagcagc gggccatcat 360
gggcgccgca catgagccct ccccgcctgg gggcctggac gcacgccagg cgctgccccg 420
cgcccaccca gccggttcct ttcatgcggg gcctggagac ctgcagaaat gggcccagtc 480
ccaagatggg tttgaacata aagagttttt ttcatcccag gtggggagaa aatctcaaag 540
tgctttctac ccggaggatg acgactactt ttttgctgct ggtcagccag ggtggcacag 600
ccacactcag ggacattggg gattcccttc ccccggggag ccaggcccac gggagggggc 660
ttttccggct gcacaggtcc agaggaggcg ggtgaagaag aggcaccgga ggcagagaag 720
gagccacgtg ttggaggagg gcgacgacgg cgacaggctg tactcctcca tgtccagggc 780
cttcctgtac cggctctgga aggggaacgt ctcttccaaa atgctgaacc cgcgcctgca 840
gaaggcgatg aaggattacc tgaccgccaa caagcacggg gtgcgcttcc gcgggaagcg 900

ggaggccggg ctgagcaggg cacagctgct gtgccagctg cggagccgcg cgcgcgtgcg 960
gacgctggac ggcaccgagg cgcccttttc tgcgctgggc tggcggcgcc tggtgcccgc 1020
cgtgcccctg agccagctgc acccccgcgg cctgcgcagc tgcgctgtcg tcatgtctgc 1080
aggcgcaatc ctcaactctt ccttgggcga ggaaatagat tctcatgatg cggttttgag 1140
atttaactct gctcctacac gtggttatga gaaagatgtt gggaataaaa ccaccatacg 1200
catcattaat tcgcagattc tgaccaaccc cagccatcac ttcattgaca gttcactgta 1260
taaagacgtc attttggtgg cctgggaccc tgccccatat tccgcaaatc ttaacctgtg 1320
gtacaaaaaa ccggattaca acctgttcac tccatatatt cagcatcgtc agagaaaccc 1380
aaatcagcca ttttacattc ttcatcctaa atttatatgg cagctctggg atattatcca 1440
ggagaacact aaagagaaga ttcaaccaaa cccaccatct tctggtttca ttggaatcct 1500
catcatgatg tccatgtgca gagaggtgca cgtgtatgaa tatatcccat ccgtgcggca 1560
gacggagctg tgccactacc acgagctgta ctacgacgca gcctgcaccc tcggggcgta 1620
ccacccacta ctctatgaga agctcctggt gcagcgcctg aacatgggca cgcaggggga 1680
tttgcatcgc aagggcaagg tggttcttcc tggcttccag gcggtgcact gccctgcacc 1740
aagtccagtc attccacact cttaaaaagg gtttcttggg aatcaatgtg caa 1793

<210> 150
<211> 2550
<212> DNA
<213> Homo sapiens

<400> 150
ggttcctccc ttcccaggca gagactgata aactcagcac ttgccggagt ggctcattgt 60
taagacaaag ggtgtgcact tcctggccag gaaacctgag cggtgagact cccagctgcc 120
tacatcaagg ccccaggaca tgcagaacct tcctctagaa cccgacccac caccatgagg 180
tcctgcctgt ggagatgcag gcacctgagc caaggcgtcc agtggtcctt gcttctggct 240
gtcctggtct tctttctctt cgccttgccc tcttttatta aggagcctca aacaaagcct 300
tccaggcatc aacgcacaga gaacattaaa gaaaggtctc tacagtccct ggcaaagcct 360
aagtcccagg cacccacaag ggcaaggagg acaaccatct atgcagagcc agtgccagag 420
aacaatgccc tcaacacaca aacccagccc aaggcccaca ccaccggaga cagaggaaag 480
gaggccaacc aggcaccgcc ggaggagcag gacaaggtgc cccacacagc acagagggca 540
gcatggaaga gcccagaaaa agagaaaacc atggtgaaca cactgtcacc cagagggcaa 600
gatgcaggga tggcctctgg caggacagag gcacaatcat ggaagagcca ggacacaaag 660
acgacccaag gaaatggggg ccagaccagg aagctgacgg cctccaggac ggtgtcagag 720
aagcaccagg gcaaagcggc aaccacagcc aagacgctca ttcccaaaag tcagcacaga 780
atgctggctc ccacaggagc agtgtcaaca aggacgagac agaaaggagt gaccacagca 840
gtcatcccac ctaaggagaa gaaacctcag gccaccccac cccctgcccc tttccagagc 900

cccacgacgc agagaaacca aagactgaag gccgccaact tcaaatctga gcctcggtgg 960

gattttgagg aaaaatacag cttcgaaata ggaggccttc agacgacttg ccctgactct 1020

gtgaagatca aagcctccaa gtcgctgtgg ctccagaaac tctttctgcc caacctcact 1080

ctcttcctgg actccagaca cttcaaccag agtgagtggg accgcctgga acactttgca 1140

ccaccctttg gcttcatgga gctcaactac tccttggtgc agaaggtcgt gacacgcttc 1200

cctccagtgc cccagcagca gctgctcctg gccagcctcc ccgctgggag cctccggtgc 1260

atcacctgtg ccgtggtggg caacgggggc atcctgaaca actcccacat gggccaggag 1320

atagacagtc acgactacgt gttccgattg agcggagctc tcattaaagg ctacgaacag 1380

gatgtgggga ctcggacatc cttctacggc tttaccgcct tctccctgac ccagtcactc 1440

cttatattgg gcaatcgggg tttcaagaac gtgcctcttg ggaaggacgt ccgctacttg 1500

cacttcctgg aaggcacccg ggactatgag tggctggaag cactgcttat gaatcagacg 1560

gtgatgtcaa aaaacctttt ctggttcagg cacagacccc aggaagcttt tcgggaagcc 1620

ctgcacatgg acaggtacct gttgctgcac ccagactttc tccgatacat gaagaacagg 1680

tttctgaggt ctaagaccct ggatggtgcc cactggagga tataccgccc caccactggg 1740

gccctcctgc tgctcactgc ccttcagctc tgtgaccagg tgagtgctta tggcttcatc 1800

actgagggcc atgagcgctt ttctgatcac tactatgata catcatggaa gcggctgatc 1860

ttttacataa accatgactt caagctggag agagaagtct ggaagcggct acacgatgaa 1920

gggataatcc ggctgtacca gcgtcctggt cccggaactg ccaaagccaa gaactgaccg 1980

gggccagggc tgccatggtc tccttgcctg ctccaaggca caggatacag tgggaatctt 2040

gagactcttt ggccatttcc catggctcag actaagctcc aagcccttca ggagttccaa 2100

gggaacactt gaaccatgga caagactctc tcaagatggc aaatggctaa ttgaggttct 2160

gaagttcttc agtacattgc tgtaggtcct gaggccaggg atttttaatt aaatggggtg 2220

atgggtggcc aataccacaa ttcctgctga aaaacactct tccagtccaa aagcttcttg 2280

atacagaaaa aagagcctgg atttacagaa acatatagat ctggtttgaa ttccagatcg 2340

agtttacagt tgtgaaatct tgaaggtatt acttaacttc actacagatt gtctagaaga 2400

cctttctagg agttatctga ttctagaagg gtctatactt gtccttgtct ttaagctatt 2460

tgacaactct acgtgttgta gaaaactgat aataatacaa atgattgttg tccatggaaa 2520

ggcaaataaa ttttctacag tgaaaaaaaa 2550

<210> 151
<211> 1908
<212> DNA
<213> Homo sapiens

<400> 151
   gggacgtcag cggacggggc gctcgcgggc cggggctgta tggggctccc gcgcgggtcg 60
   ttcttctggg tgctgctcct gctcacggct gcctgctcgg ggctcctctt tgccctgtac 120
   ttctcggcgg tgcagcggta cccggggcca gcggccggag ccagggacac cacatcattt 180
   gaagcattct ttcaatccaa ggcatcgaat tcttggacag gaaagggcca ggcctgccga 240
   cacctgcttc acctggccat tcagcggcac ccccacttcc gtggcctgtt caatctctcc 300
   attccagtgc tgctgtgggg ggacctcttc accccagcgc tctgggaccg cctgagccaa 360
   cacaaagccc cgtatggctg gcgggggctc tctcaccaag tcatcgcctc caccctgagc 420
   cttctgaacg gctcagagag tgccaagctg tttgccccgc ccagggacac ccctccaaag 480
   tgtatccggt gtgccgtggt gggcaacgga ggcattctga atgggtcccg ccagggtccc 540
   aacatcgatg cccatgacta tgtattcaga ctcaatggag ctgtgatcaa aggcttcgag 600
   cgcgatgtgg gcaccaagac ttccttctat ggtttcactg tgaacacgat gaagaactcc 660
   ctcgtctcct actggaatct gggcttcacc tccgtgccac aaggacagga cctgcagtat 720
   atcttcatcc cctcagacat ccgcgactat gtgatgctga gatcggccat tctgggcgtg 780
   cctgtccctg agggcctaga taaaggggac aggccgcacg cctattttgg accagaagcc 840
   tctgccagta aattcaagct gctacatccg gacttcatca gctacctgac agaaaggttc 900
   ttgaaatcaa agttgattaa cacacatttt ggagacctat atatgcctag taccggggct 960
   ctcatgctgc tgacagcttt gcatacctgt gaccaggtca gtgcctatgg attcatcaca 1020
   agcaactact ggaaattttc cgaccactat ttcgaacgaa aaatgaagcc attgatattt 1080
   tatgcaaacc acgatctgtc cctggaagct gccctgtgga gggacctgca caaggccggc 1140
   atccttcagc tgtaccagcg ctgaccccaa tgcactgagc gctttgcttc ttcaagagtt 1200
   gcggccctga tcctctcaag tggccaaaag cttttttaac ttttcaatct tcaccttccc 1260
   ttgccaacag agggcactgg ggtgaattca agattttcat cgaggtctgt tcaatatagg 1320
   acaccccagc ttgtccttgg ctcatccaag aactcttctg tatctaaaac aatacatctc 1380
   aatcttggcc aagggaaaat ggactgcttt gctggattgg cactgagcaa ctttaggaaa 1440
   tgtcggtgga gtgttcagca agatcagaca gcagtccagg tcaaaggcaa acacacacgc 1500
   tccagcccaa atcctcctgg tggcacatcc taccccagat gctaaagtga ttcaaggact 1560
   ccaggacacc tcttaagagc ctttctaaga acatgatagg cttacttctg ctccataata 1620
   aagtgggaga aaaaagccag aatataactt aagactagat aactgcgtac atgatggacc 1680
   attttttttt tttttggctg ggtagagaaa tcatataaaa cgcaggctgt ttagcatgga 1740
   gatgactctc agaacactgg gagggtctgg cacttgatgg gggttagttg cttggcagcc 1800
   tgcctgccac tgagggaagt cccattagag atgtatcacc accttgtcac caacaggatg 1860
   atgtcaccaa caggatgatg tcaccaggta ataaaccttc atcctcac 1908

<210> 152
<211> 3207
<212> DNA
<213> Homo sapiens

<400> 152
gcagcttaca ccgcggtccc cttatttgga tctgcgggaa tgtgggctgg agaggtcctg 60

ccgtggtacc agcctccagc ctgcccccag gactgcccct gacccaggcg cgcccgctgc 120
tcggtggcag gagggccggc ggagcgccat ggcctgcatc ctgaagagaa agtctgtgat 180
tgctgtgagc ttcatagcag cgttcctttt cctgctggtt gtgcgtcttg taaatgaagt 240
gaatttccca ttgctactaa actgctttgg acaacctggt acaaagtgga taccattctc 300
ctacacatac aggcggcccc ttcgaactca ctatggatac ataaatgtga agacacaaga 360
gcctttgcaa ctggactgtg acctttgtgc catagtgtca aactcaggtc agatggttgg 420
ccagaaggtg ggaaatgaga tagatcgatc ctcctgcatt tggagaatga acaatgcccc 480
caccaaaggt tatgaagaag atgtcggccg catgaccatg attcgagttg tgtcccatac 540
cagcgttcct cttttgctaa aaaaccctga ttattttttc aaggaagcga atactactat 600
ttatgttatt tggggacctt tccgcaatat gaggaaagat ggcaatggca tcatttacaa 660
catgttgaaa aagacagttg gtatctatcc gaatgcccaa atatacgtga ccacagagaa 720
gcgcatgagt tactgtgatg gagtttttaa gaaggaaact gggaaggaca gagtccagtc 780
tggctcatat ctcagcacag ggtggtttac cttccttctg gccatggacg cctgttatgg 840
cattcacgtc tacgggatga taaatgacac ctactgcaag acagaagggt atagaaaagt 900
cccctaccat tattatgaac aaggaagaga tgagtgtgat gaatattttc ttcatgaaca 960
tgccccatat gggggtcata ggtttatcac tgaaaagaaa gtgtttgcta aatgggccaa 1020
gaagcacagg ataatattta cacatccaaa ctggacattg tcttgataat ggttttcctg 1080
atcttgccgc atcacttaat gtgatcccca tactgcaact gtgatgctga tgatgctaat 1140
ggagatgatg gtaatgataa agacaacaac aatgattatc aagttcctgt acactctcag 1200
atgtggatgg tgactctgct agtaatttaa acttggcatt tcatggagga tggttgtgct 1260
catgatgttc tttctggagg ttcaacacta cgtaccgcac tttataattt aactggaatt 1320
gagatgaagg ccagtgacat gacaactgtg acctaagaaa tgggaagatt atctttcaag 1380
atagctgcct agaattgttc aacagtgagt aacttccaga agccaaaaga gtttggcttc 1440
atgaggcaag gtgattgact caatggcttg ttgaatctca gcagttccgc catcttgaag 1500
aatgattgat tgtcaaacac tgacccaaga actgctatca gggtgcaagt atcttactac 1560
ttagcctaag gatgggaaaa tatctcttcc taatatacca tctttctaca gtatttccta 1620
cagctaaagc ctcaggccat tgcctaatta acaatgaaga tttcatggac atcctacact 1680
tcaggattac ttgactatct caaacacata aatcaaaatg ggccaagtag caaatcttga 1740
tgaatttcca ctcttaccat ccattcttac taccaacagt ataactgaac atgtgattag 1800
aatgatctat attaatgttt agttattttt ggttgagtgc taaatatttc agaaggctac 1860
ttaacatgta agataccaac ttcaacactg taataacata tactgtgaaa acattcctaa 1920
aaagtaacca aagggtttgc taactctgct tcaacatcca acaacacaaa actatatgat 1980
ttttaaaatc atgaaataga gacagcaaag catattttta catcaatttg tatcctatca 2040
tacttcataa tatatatttg tatattcaaa tttctatttt ataggcccaa ggtgtgtttc 2100

tccaaacatt acattctcat tgccaagtgc cagctgttac acatggagag gaaatgattc 2160
cttatattaa acctgaccag agctttttgc cttctaggga tttatttttc ccatagtgta 2220
tcagttgtta tgccacaata acaacaataa taatgttctt aatgtccaaa gtgctttgtc 2280
atttagagtc ctttaacatc tgttatacca tgtgatgccc ttgaacaccc cattgggcta 2340
ttgtctgtgt attctgccct ctttagcaga tgatctgtaa tttggtcatt gttctgttat 2400
attccagatt gctctacttg ctaatatttg tatagaatag aatttatgta aactagtaac 2460
cagttgatct ctgtgccaaa tacattatat caggattaca caaaaagcat cacaaaggat 2520
ggtcttggcc atatgctagg gcccctgtga aaataagtag ttttgagaag tttttctata 2580
tacgtatata tacacgtgtg gttctattta ggtggggaaa aaatgaaaaa gcagggacaa 2640
caggaggaaa tgaaggcaga gaaatatagt ttcctttcct aggattgaga caattctatt 2700
tttcatatac tcgttgctca ttaaatagta aagggttgat ttgctgcaag agtagctgag 2760
aattcaaaaa catccttgca gaatgattca aaagacaaaa gccaggctca acttatagaa 2820
tgttttggaa actggaagta atgatacatt tcacttcaga agcacttgta ggtctttact 2880
agtgctaagt attatggtga gctatcatta aagtgtgcca tcttggataa aggccttttg 2940
cctactctct tctgcaattt tgacacctca atattcacac ccataaacat tactaagccc 3000
cagttctatc gttggaagga gttgttattc ttttgttgtt cccttatgca atttttaatt 3060
ctatgaatgt taagtttttt tgagctaatg atagatagaa atgcccactc aaagaaaaat 3120
aaacagagac aaatgttaaa attgccatac agactgtttt tccccctgtt gtttcacttt 3180
aaaataaaaa gaaatccttg attaatc 3207

<210> 153
<211> 2048
<212> DNA
<213> Homo sapiens

<400> 153
ctctgcaaca gccgcgcttc ccgggtcccg cggctcccgc gcgcgatctg ccgcggccgg 60
ctgctgggca aaaatcagag ccgcctccgc cccattaccc atcatggaaa ccctccagga 120
aaaagtggcc ccggacgcgc gagcctgagg attctgcaca aaagaggtgc ccaaaatgaa 180
gaccctgatg cgccatggtc tggcagtgtg tttagcgctc accaccatgt gcaccagctt 240
gttgctagtg tacagcagcc tcggcggcca gaaggagcgg cccccgcagc agcagcagca 300
gcagcagcaa cagcagcagc aggcgtcggc caccggcagc tcgcagccgg cggcggagag 360
cagcacccag cagcgccccg gggtccccgc gggaccgcgg ccactggacg gatacctcgg 420
agtggcggac cacaagcccc tgaaaatgca ctgcagggac tgtgccctgg tgaccagctc 480
agggcatctg ctgcacagtc ggcaaggctc ccagattgac cagacagagt gtgtcatccg 540
catgaatgac gcccccacac gcggctatgg gcgtgacgtg ggcaatcgca ccagcctgag 600
ggtcatcgcg cattccagca tccagaggat cctccgcaac cgccatgacc tgctcaacgt 660
gagccagggc accgtgttca tcttctgggg ccccagcagc tacatgcggc gggacggcaa 720

gggccaggtc tacaacaacc tgcatctcct gagccaggtg ctgccccggc tgaaggcctt 780
catgattact cgccacaaga tgctgcagtt tgatgagctc ttcaagcagg agactggcaa 840
agacaggaag atatccaaca cttggctcag cactggctgg tttacaatga caattgcact 900
ggagctctgt gacaggatca atgtttatgg catggtgccc ccagacttct gcagggatcc 960
caatcaccct tcagtacctt atcattatta tgaacctttt ggacctgatg aatgtacaat 1020
gtacctctcc catgagcgag gacgcaaggg cagtcatcac cgctttatca cagagaaacg 1080
agtctttaag aactgggcac ggacattcaa tattcacttt tttcaaccag actggaaacc 1140
agaatcactt gctataaatc atcctgagaa taaacctgtg ttctaaggaa tgagcatgcc 1200
agactgtaat cccaggtatt cactgcatca gacaccgaga cactgaactt cctgagccac 1260
cagacaggaa agggtagcag aaaacagctt cactcctcag gaagtaccat ggacagacgc 1320
ctaccagggg tgacaaagca gtgcagttgg attgtaagga aaaattccgg aattaatgca 1380
tcctaatgaa tgttgtcccc ttcaatggtg ttaccttagg agctgaacat tcaattcagt 1440
tacaccacta tgactaaaaa cagtttggat ctcttagtat tgcctttgaa actgcaacat 1500
aagcaactca acaatattag ttgcattcct ttatagacat accatgtcaa agacgttttt 1560
ctatcaagtt gtattctttc ctgttctata acctttgtca tctgttagac tctgtatgtg 1620
tgatttgtaa aaagcaggct gaaactatgg acatgatttc tgaagagcac atctccactg 1680
actttcataa agcaaatgtc caatatttat ttattgagag ttttttagtg caatctgggc 1740
cagtattttt atagattatg attatgtggt aatttatcct tcctaactct ttaatcctga 1800
atgatggttg gaaatggcct agaattaggt tactctgttc acaatgctca ttgttagcat 1860
gcaattggta tttgacttgg aagtgttgtg ttgtattttt tgaaccccta ggcttcagga 1920
aaactgctct tttgtaaaaa gaatagcgat gacattttct aatgtgcaga aatgttccaa 1980
aaggacaaaa ttgaaaacca aaaactatgt tattaaaaca aaaaaatgct aaaaaaaaaa 2040
aaaaaaaa 2048

<210> 154
<211> 2416
<212> DNA
<213> Homo sapiens

<400> 154
ggcctgagga gcgcggaccc cggcgctcgg ctcccggcgc catgtgaggg ggctcggggg 60
ccgcgggggg ccgggcgctc cccgccggag atgaggaaac tgaggctcag agaggtcaca 120
tggcttgctc gaggcccccc agccagtgtg aacccacatc cctgccccca gggccacctg 180
caggacgccg acacctaccc ctcagcagac gccggagaga aatgagtagc aacaaagagc 240
agcggtcagc agtgttcgtg atcctctttg ccctcatcac catcctcatc ctctacagct 300
ccaacagtgc caatgaggtc ttccattacg gctccctgcg gggccgtagc cgccgacctg 360
tcaacctcaa gaagtggagc atcactgacg gctatgtccc cattctcggc aacaagacac 420

tgccctctcg gtgccaccag tgtgtgattg tcagcagctc cagccacctg ctgggcacca 480
agctgggccc tgagatcgag cgggctgagt gtacaatccg catgaatgat gcacccacca 540
ctggctactc agctgatgtg ggcaacaaga ccacctaccg cgtcgtggcc cattccagtg 600
tgttccgcgt gctgaggagg ccccaggagt ttgtcaaccg gacccctgaa accgtgttca 660
tcttctgggg gcccccgagc aagatgcaga agccccaggg cagcctcgtg cgtgtgatcc 720
agcgagcggg cctggtgttc cccaacatgg aagcatatgc cgtctctccc ggccgcatgc 780
ggcaatttga cgacctcttc cggggtgaga cgggcaagga cagggagaag tctcattcgt 840
ggttgagcac aggctggttt accatggtga tcgcggtgga gttgtgtgac cacgtgcatg 900
tctatggcat ggtccccccc aactactgca gccagcggcc ccgcctccag cgcatgccct 960
accactacta cgagcccaag gggccggacg aatgtgtcac ctacatccag aatgagcaca 1020
gtcgcaaggg caaccaccac cgcttcatca ccgagaaaag ggtcttctca tcgtgggccc 1080
agctgtatgg catcaccttc tcccacccct cctggaccta ggccacccag cctgtgggac 1140
ctcaggaggg tcagaggaga agcagcctcc gcccagccgc taggccaggg accatcttct 1200
ggccaatcaa ggcttgctgg agtgtctccc agccaatcag ggccttgagg aggatgtatc 1260
ctccagccaa tcagggcctg gggaatctgt tggcgaatca gggatttggg agtctatgtg 1320
gttaatcagg ggtgtctttc ttgtgcagtc agggtctgcg cacagtcaat cagggtagag 1380
ggggtatttc tgagtcaatc tgaggctaag gacatgtcct ttcccatgag gccttggttc 1440
agagccccag gaatggaccc cccaatcact ccccactctg ctgggataat ggggtcctgt 1500
cccaaggagc tgggaacttg gtgttgcccc ctcaatttcc agcaccagaa agagagattg 1560
tgtgggggta gaagctgtct ggaggcccgg ccagagaatt tgtggggttg tggaggttgt 1620
gggggcggtg gggaggtccc agaggtggga ggctggcatc caggtcttgg ctctgccctg 1680
agaccttgga caaacccttc cccctctctg ggcacccttc tgcccacacc agtttccagt 1740
gcggagtctg agaccctttc cacctcccct acaagtgccc tcgggtctgt cctccccgtc 1800
tggaccctcc cagccactat cccttgctgg aaggctcagc tctttggggg gtctggggtg 1860
acctccccac ctcctggaaa actttagggt atttttgcgc aaactccttc agggttgggg 1920
gactctgaag gaaacgggac aaaaccttaa gctgttttct tagcccctca gccagctgcc 1980
attagcttgg ctcttaaagg gccaggcctc cttttctgcc ctctagcagg gaggttttcc 2040
aactgttgga ggcgcctttg gggctgcccc tttgtctgga gtcactgggg gcttccgagg 2100
gtctccctcg accctctgtc gtcctgggat ggctgtcggg agctgtatca cctgggttct 2160
gtcccctggc tctgtatcag gcactttatt aaagctgggc ctcagtgggg tgtgtttgtc 2220
tcctgctctt ctggagcctg gaaggaaagg gcttcaggag gaggctgtga ggctggaggg 2280
accagatgga ggaggccagc agctagccat tgcacactgg ggtgatgggt gggggcggtg 2340
actgccccag acttggtttt gtaatgattt gtacaggaat aaacacacct acgctccgaa 2400
aaaaaaaaaa aaaaaa 2416

<210> 155
<211> 2117
<212> DNA
<213> Homo sapiens

<400> 155
ggggggggtt gcctggcggc gcagcagcgc gggaggcggc gaagggcgca ggagcatcgc 60

tcggagggga caaggggacg ccacgggcca catgtttagg agggagccga gccttctccc 120

ggaccctcgc cgagggcgac cgtgatgctg cagaaccggc gggagcgact cgccgccgcc 180

gctctctgcg cactcggaga ccccagcgcc cgccttctgc aggggaagcg accatggcca 240

tagatcgtga cttcaccccc agccacttcc cctagaaaga aatccttgga aaagttgcat 300

ttgaaaaaat ccttgcgctg acctttgggg ccgacggggc cgaagaagcg tgcgtgcgtt 360

tgcaagtaag agaaccaaag gtgtgtgtgc atggggggct ggcggtgggg gaccctccgc 420

tgccacttcg cctagctttg tgctgaggcc ccggcccccg cccctgggac gccggggctg 480

cgatgagccc ctgcgggcgg gcccggcgac aaacgtccag aggggccatg gctgtactgg 540

cgtggaagtt cccgcggacc cggctgccca tgggagccag tgccctctgt gtcgtggtcc 600

tctgttggct ctacatcttc cccgtctacc ggctgcccaa cgagaaagag atcgtgcagg 660

gggtgctgca acagggcacg gcgtggagga ggaaccagac cgcggccaga gcgttcagga 720

aacaaatgga agactgctgc gaccctgccc atctctttgc tatgactaaa atgaattccc 780

ctatggggaa gagcatgtgg tatgacgggg agtttttata ctcattcacc attgacaatt 840

caacttactc tctcttccca caggcaaccc cattccagct gccattgaag aaatgcgcgg 900

tggtgggaaa tggtgggatt ctgaagaaga gtggctgtgg ccgtcaaata gatgaagcaa 960

attttgtcat gcgatgcaat ctccctcctt tgtcaagtga atacactaag gatgttggat 1020

ccaaaagtca gttagtgaca gctaatccca gcataattcg gcaaaggttt cagaaccttc 1080

tgtggtccag aaagacattt gtggacaaca tgaaaattta taaccacagt tacatctaca 1140

tgcctgcctt ttctatgaag acaggaacag agccatcttt gagggtttat tatacactgt 1200

cagatgttgg tgccaatcaa acagtgctgt ttgccaaccc caactttctg cgtagcattg 1260

gaaagttctg gaaaagtaga ggaatccatg ccaagcgcct gtccacagga ctttttctgg 1320

tgagcgcagc tctgggtctc tgtgaagagg tggccatcta tggcttctgg cccttctctg 1380

tgaatatgca tgagcagccc atcagccacc actactatga caacgtctta cccttttctg 1440

gcttccatgc catgcccgag gaatttctcc aactctggta tcttcataaa atcggtgcac 1500

tgagaatgca gctggaccca tgtgaagata cctcactcca gcccacttcc taggaacaat 1560

ggaagaagaa aggactgaac cagggtattt ttgttaggtt ttctatgtga ctccaagagg 1620

gaatggtcaa gttgtttcat gagtttgcat gggcccttgg aaaaacagga aaggagcaat 1680

gaagatccaa gcaaaacttt actttcagcg ttggcttgga ggacaaataa gaaatgaaac 1740

atcctatgaa atactttata gcacatggca gatttgcaac tagtaaaatg ctggtgaaat 1800

gctgttggta aagcacatgg ttcaaatcta gaagatgcag ttcaaaaaca agacagactc 1860

gagttgttag ggctgaggaa ccaatcaagg tagaacaaag aaaatgttgg ggtaaaagtg 1920

ttgctgattg tcaacacaaa ctggcttaat aatattaata agaacctgtc ttattaagac 1980

tggctttaga accgtaggtt tttttaaaaa attattattt atttttgccc tctttgggga 2040

agtgggtggg tagatttaaa aaatcccttc ctgagtaata aagatacaaa atgttactgc 2100

tgataaaaaa aaaaaaa 2117

<210> 156
<211> 5626
<212> DNA
<213> Homo sapiens

<400> 156
   cgctgccgct gccgccgccg gcccggactc gtccggagcg caggtgtctg gcccagctgc 60
   gcgcggcgcg cggaggctcc ggcgtccgcc gctgcgccct ccggcccctg ctcctcgcgc 120
   cggcccgcgt gggtcccggc gggcgcgaac ccaccatgca gctgcagttc cggagctgga 180
   tgctggccgc gctcacgctg ctcgtggtct tcctcatctt cgcagacatc tcagagatcg 240
   aagaagaaat cgggaattcg ggaggcagag gtacaatcag atcagctgtg aacagcttac 300
   atagcaaatc taatagagct gaagttgtaa taaacggctc ctcatcacca gctgttgttg 360
   acagaagtaa tgaaagcatc aagcacaaca tccagccagc ctcgtccaaa tggagacata 420
   accagacgct ctctctgagg atcaggaagc agattttaaa gttcttggat gctgaaaagg 480
   acatttctgt cctaaaggga accctgaagc ctggagatat tattcattac atcttcgatc 540
   gagacagcac catgaatgtg tcccagaacc tctacgagct cctccccagg acttcgccac 600
   tgaagaataa gcactttggg acttgtgcca tcgtgggcaa ctcgggggtc ttgctgaaca 660
   gcggctgtgg gcaggagatt gacgcccaca gcttcgtcat caggtgcaac ctggccccag 720
   tacaggagta tgcccgggat gtggggctca agacagacct ggtaaccatg aacccctcgg 780
   tcatccagcg ggcctttgag gacttggtca atgccacgtg gcgggagaag ctgctgcaac 840
   ggctgcacag cctcaatggc agcatcctgt ggatccctgc cttcatggcc cggggcggca 900
   aggagcgtgt tgagtgggtc aacgagctta tcctgaagca ccacgtcaac gtgcgcactg 960
   catacccctc gctgcgcctg ctgcacgccg ttcgcggata ctggctgacc aacaaagtcc 1020
   acatcaaaag acccaccacc ggcctcttga tgtataccct ggccacacgt ttctgcaaac 1080
   aaatctacct ctacggcttc tggccctttc cgctggatca gaaccagaac ccagtcaagt 1140
   accactatta tgacagcctc aagtatggct acacctccca ggccagcccg cataccatgc 1200
   ccttggagtt taaggccctc aagagcctac atgagcaggg ggctttgaaa ctgactgtcg 1260
   gccagtgcga tggggccacg tagggtgggc accccatggg actcagtggc tcacatttcc 1320
   tgccagctac accacaggca gatgggagtc ggggtggcac aaacaataga acaagcaggc 1380
   tagtggtttt ctttgttaaa gtgtaaaaca gtgaccagaa tatatatatc tatacctgca 1440
   tatatatatt gacctgagta tttataactg tgtggtgttc atctagcatt aggcagatag 1500
   gccacaggaa gaaggtgtgg agaacccacc tgaaccagat tgagactcaa tccatctttg 1560
   ggggtggaag gacttgacat gaaaagaagc cagtcccatg actttggatg acaaactgcc 1620
   tcctggcttg gagggatctt tgggctcatg gatgaatggc gagccacctg ttgtcagctg 1680
   ccccaagcct ctgggatatg tggattcgga gaaagctgag gcccagaggg gacactcgac 1740
   ccagtctaaa gtgggtgcca ttcacggagg agagggaggt tggagacttc aaccgggcaa 1800
   aggagcacaa ggaaaccaag cagcagaggc cccatcagca cagagggcct cgttccacga 1860
   acgtagagtt tgactgccta agggacagca cgaatgactc acatgtttca gacaggcctc 1920
   cacggctatg gagagaaggc tggaggagac acaatggatg gttcatgttc tggcttagca 1980
   agagccacca agaactttgg gtgaagcaag cagatgacaa tgaagaggag taatgttccg 2040
   aatcatgttt ttgaaaaatc aagacctcct gttaagggcc cacttgaccc tagaataagg 2100
   caggagagtg tctaaaatct agctgctttg ggtgtttttt aaagcaatgt gagtccactg 2160
   aggaggaaga gaccctggga tttcaccctc tgggttagtc tctgtctcac cctgttagaa 2220
   tgtcacgagg gactgtactg tcccctctgt caccagggaa gccacaatga gagcttgaaa 2280
   gggtgctgtc aagataaatg tcatccttct taaaatacat atgattgcct ttttgggtgc 2340
   ctcacagagc cccagagtgt gagacctgaa agaatgttca gcatctagct ccatgtccct 2400
   gcaaccactc tctgtttgcc ttctgcattc tgcactctgc ggggcctttg cagccagact 2460
   gctgtgtcaa tgttactttc ttgtcacttt cctaccctga tggtcacact agcccacccc 2520
   aagctccgac attgaggttt tcatctctgc agggagaggt tccaatgtcc accccacccc 2580
   caggtacccg cctcttccca cagaagcaat tttagaattt tgcaaaattc ttttcccctt 2640
   attgagatta atatttaaga ttaaaaaaaa aatacccaaa gccaaatgca gttccaaata 2700
   gtggacataa tctcccaggg aaatctgggc tcgaggccta caacttaggg gctggaatca 2760
   caggtctcac atagagaaat agccagcttg aatttgttag tcgtattcct tcacctgatg 2820
   aataacagtg ttttaaaaca tatttgaaag gtaagtaaaa aactaccatc ttgaaggtga 2880
   agggagttaa gaaaagtaat caagacctgc tcctaagcat atgagaccag atgtaattaa 2940
   tcaatggcaa tgcagaacag gaccttggct ctaaattaat ggttgaccgg aagggattgg 3000
   aggcggcaga accattaacc ctacacaaga acactccagc ccaaaaccgt gaggaaggtg 3060
   atatatctct gcattttccc ttgttgttgc ttgaagtctg tgattgggga tttgcgttat 3120
   aaaatgaata ttttaaaaag caacagccct aatagcctct gtgggaactg agtggtttcg 3180
   gactcctcac aggcggtgga aataattaat accatatttt atacatctcc agagcctttc 3240
   atcttgagga tctcaaagac cgtagaagac atttaatttc taaagcttga agtgccccga 3300
   ggagcaggta gaatttccag cagggccttg gtggattaac caaggccttg gattccagaa 3360
   tgtttcatgg taagcactgt ggagaaccta catgtcaggt tctggagttg gccctgagct 3420
   ctatccacca agccagcacc ccaccatccc tttgcagggg agtcaaaggg ccagaactcc 3480
   ttgggggacc agaacatggt tttccttctg cctatcttag tcatggctcc taaaattgaa 3540
   gctgatatga gctggagtgc aaggagtcct tccaccctgc cttcaggttc tctctggaga 3600
   aaggttagag accccaggac aatcaacatg tgcaccgttc ccctcctcgg cttgagtcct 3660
   gcactaccaa aatggcctgg ccatttgatg gcggagctcc aggggccttg gctttctctg 3720
   gctcattgtc cttctccatc tgggccaatt gtccttccct gcccattcag agttcaattt 3780
   gagcctccac tgcagttcct ggtgtgggcc agctggtgtt gggtggcccc agggagtcac 3840
   acagtctgta cccggccccg agatcctctg ctgtaactgc agggtcacag gcatcccggg 3900
   agaatgggcc tccaccctca gccagccctc actgctgaca ggcacccctc tgtcacctcc 3960
   tgccacctgt cagataccca gcctgcccgc catcagccac ccgagtggtt ccgacctcca 4020
   ttcacctcaa gccccaacag gcccctgtca ttccacttca tggaagagac ggagccttct 4080
   gaaatgagat ctaccctgaa aatctcttct ccttcccctt tctgagaaac gtcatcccca 4140
   ccagttaacg tttcctgtac accctcaccc tctctcgtcc atctctgttg cctctgcaca 4200
   tcagcacaca ctgactccac ctgggctggc tcctgccgcc tcttccctac ctcctccact 4260
   agccgcagga agggcctcca gcacccacaa ctgcccttcc tcttgatgcc cctcggcttc 4320
   cccccagcct ggcagcagcg tcccagcgcc tccacattgc ccctctcacc tcccagctct 4380
   ccacgccact tggctctgca gcgacccctc ctagacgctc cctggcccct cggcacccag 4440
   aatgccacat tcttgtgttt ttttcctcct tctttcccaa tccctctcaa ccatgtattt 4500
   atttatatat gtatgtattt ttttatttat tatttattta ttgaaacccc tactttgatt 4560
   gacaaaggaa tcaaggtaga tctgaccagt attcccctct cttctccttg tcccaccaca 4620
   tggacatgcc ttacactggc gtcctctcct gagtccctcc gcactctcca tcagcaacct 4680
   cacacctgcc ttcagacccg gctgcaactt cctaagctgc ccctgcccca gctcctctct 4740
   gcaacccagc cctctcctga gctctaggct gggattacac ccgctggagg gacactcacc 4800
   ccatctgctc ccacggcagc accatcaact caacaagtca gaagccaagt ggatcatctg 4860
   tgtccccttc ccccacaatc cctccttttt ctagtaaaag acccgtgatc ccgtcagcca 4920
   cccagtctga gatgctgacg ttatcttcga ctattcttcc ctgtcctgcc ttgctaattc 4980
   ttcccactag cactaatctc gactctttgt cacttggact atttcagcaa cagtctcatc 5040
   tcccagatta cctctgtgca cactgacacg tggtcccgtc catcctgaac atgcacagtg 5100
   attttcctct cccgttgcac acaaaccttt aaaagttccc cggcccgtga acaaatgtac 5160
   aattctctat tctgacatta cagaccaccg gataaggccc cattcttcct ttacaccctc 5220
   ccctggtcag cctaggagag ttgtttttcc tggaaagggt gcctgcgttt tactgtctct 5280
   gtgcctttgg tcttgctatc cccatcccct ggaatgctcc cttttcttcc cttcaaagcc 5340
   catgcggaaa ccacgtcctt catgaagccc tccctgatgt accaaccact gggatctctc 5400
   cctctgctga accaccgtag cactttattt gtacctttct catggcactt accatattct 5460
   gccttgtatt atagttattt gtgcacttgt cctttgactg ttcttagact gtaagcttgc 5520
   caagggcagg gactgtgttt tattcatctg tatccatcac agcactaggc gtggtgcata 5580
   gcacacagca gttgctcaat aaatgtttgt tggattaaat ccttaa 5626

<210> 157
<211> 10154
<212> DNA
<213> Homo sapiens

<400> 157
   ggcagcctcg ctggaaacgc gcgggggagc ctgagccggc ggccggggac gcacggcgct 60
   gcgcgctcct tcgccacgcc gccgcgcagc ccctccatct tcctgctcgg caccgggccc 120
   cgcgcgcccc tgcctacggg gtcccgctgc tctccggggc tcctgccagc cccaaccccc 180
   ggccccggtg gcctcccccc acccccgccc gggtccccct cctccgccac acgcgcgcgc 240
   gctcacacac acacacacac acacacacac acacacacac atatatacac gccagcgagc 300
   tgctggccgc tcaatggacc gatttccccg gtttccctga acccagccca gcccgggatg 360
   agaaactgca aaatggcccg ggtcgccagt gtgctggggc tggtcatgct cagcgtcgcc 420
   ctgctgattt tatcgctcat cagctacgtg tccctgaaaa aggagaacat cttcaccact 480
   cccaagtacg ccagcccggg ggcgccccga atgtacatgt tccacgcggg attccggtca 540
   caatttgcgc tgaagtttct agacccgtca ttcgtgccca ttacgaattc tctcacccag 600
   gaactccaag agaaaccttc taagtggaaa tttaatcgga cagcgttttt acatcaaagg 660
   caagaaattc ttcagcatgt cgatgtaata aaaaattttt ctttgaccaa gaatagtgtt 720
   cggattggac aactgatgca ctatgattat tccagccata aatatgtttt ctctattagc 780
   aataacttcc ggtcacttct tccagatgtg tcacccatta tgaacaagca ttataatatt 840
   tgtgctgtgg ttggaaatag tgggatcctg acagggagcc agtgtggaca agaaatagat 900
   aaatcagatt ttgttttccg ttgcaatttc gcccctacgg aggctttcca aagagatgtt 960
   ggaagaaaaa ccaatcttac caccttcaac cccagcatcc tggaaaaata ttacaacaat 1020
   ctcttgacta ttcaggaccg taacaacttt ttcctcagtt taaaaaagct tgacggggcc 1080
   attctttgga tccctgcatt tttcttccac acttcagcaa ctgtgaccag gacattagtt 1140
   gacttttttg ttgaacacag aggtcagtta aaagtccaac tggcttggcc gggaaatata 1200
   atgcaacatg tcaacaggta ctggaaaaac aaacatttgt cacctaaacg gctgagcaca 1260
   ggtattctta tgtacaccct tgcatcagca atatgtgaag agatccactt gtatggattt 1320
   tggccgtttg gatttgaccc caacacaagg gaagatcttc cataccatta ctatgacaaa 1380
   aaaggaacca aatttaccac caagtggcag gagtcccacc agctgcctgc tgagtttcag 1440
   ctgctgtacc gaatgcatgg ggaagggctc accaagctga ctctgtcaca ctgtgcctaa 1500
   gaactccaaa cggaaagcgc caaatggctg tttaaaaagt gccccaaatc aaattgaata 1560
   gccttcagaa tagaacccta gagaatgtct tataaggatt gtctgccatt taaaaggaaa 1620
   gatgtctttt ctcttttgca ctgctctttt aagagtttta gcagatttag caggacagat 1680
   gcattgaagc cacatggttt agacttgatt gataaaggga atgttgcatt tgggactatg 1740
   ctgctaacga aatggtttga agtattttca tgtttggatt ttaataataa actgcctctc 1800
   atttttatga ggactagagc tatagtttct gcagctctgc tcagatagtc ctcataatca 1860
   gaggcctctg gccaactggg gcaggacctg ttttgctggt gggatcagac tctgaaaaat 1920
   ggaaacgtaa aaaactggtt tgcatatctc atcttctatc tatctcccta tctccatctc 1980
   tatcaccatc tcactccttc cctctgcaac tactccctgc cctaccaccg tggagtttaa 2040
   taatttgcta ggaatcctat tgaattcgct ttgcttgtat atgttgcatt tgtacttgat 2100
   gtgtttaagg ttctgggtaa ctttgatgct tcatgacaaa ataggcaaat cacgatggac 2160
   tcccttgtga ttcctttttt aaagaaatgc ctctgatgca gtcctcacca gtccattcag 2220
   tcattctaca gcaagatggt ccctctgtgg tgagtgggat ataaggcagt gcatctttca 2280
   tgatcacaaa gaaagcctat gttgtggata gcattgcgtc tcttgatgta ggcattgtta 2340
   tggcaaataa tagcactgtg gccacatctg taaggccatt ccagcctaaa tttagtgtct 2400
   cccataatcc caacccatgc actaggctgg gtgttctctc agaatataga ggcaatgaat 2460
   cagcttaaga ccctgggaca cacaaaggaa aaggaagaag aatatttaga agttgttatg 2520
   gaatcctgtg taccctaaag tgtggtaacc atgttaccca ttccagctgg gttaaagtgg 2580
   aggaggatct tggccaggcc aggtgcccag gtgactagtg ctgaggtgcg atcgttttcc 2640
   gtatcattgt gtgcaccacc accaatccag aaccatttgc tctctttgag aacattgtag 2700
   cactggatat tccctctttc cactgaagtc agatctatac aactacaaat taagcatttc 2760
   aaaagaaaaa gccattgtgc agtggcaaga gttggaatcc aagattatgt agacctgcga 2820
   tgaaggaagt agctggccat tctcattttc tttatttcag ccatatgagt agaaatcaaa 2880
   tcattcccat ccctggcttt ttactgagca gcactttgtt cttccaattc agcccaacgt 2940
   ctggccaagt tgatgttaaa ttttaataag agtttctttc ttctttccaa atgccagtca 3000
   agcatatttg ttatgcattt gagtggggta gctcattctt ttcctgaaat ctcatctaga 3060
   ttgcttgcaa tgcgacaaat aactgtttaa aaaatgtgct gtagttcagt gatgagctca 3120
   ttgcccctct ttaggaaggt aagatcatgc ctaaaattaa accgaatatg gtaaggaaag 3180
   acactatcgt tatgctcacg ttctgcctgg tcctaaacct gccttattat tattttcaga 3240
   aagcaatcta attagagcaa cttgcataga tctagatatt aactaatgaa agaaacacca 3300
   agttcaaatg tgttaccaat tatgacacac attacaatat aattataagg ggagacatta 3360
   aaaacatttt gtaattcagt ggtataattt gatgggtatc acaaccacac cgtgtttgtt 3420
   ccctgtccct ttgaagagga ctgtactacc atcctcacac tgtgctctgt tgggatcact 3480
   gagagattga caactgacag aagtgtttag ttggctgttc ctgagcactt agttcatttg 3540
   gttatagcta agtgatatta atgccaatat tacttgggcc agattctgac tctggagttt 3600
   taatcccaat gtgcccattc attttcacta aatataaatg catagacact ttaaaccaag 3660
   aagattaaaa gaggaaaaga ccagaatctc taaaaaacag tttactgttc tgtagtggac 3720
   aatttaaatt ttatctcatc ataatgccaa ggatttttgt ttgtttttta agttcaggga 3780
   gattttttaa atctccttgc agtcttggct ccatcttaac cttagagtgg gaggactttc 3840
   ccatccccca ccaccgtgtt atctatcact ttaatgaaaa ctcaaaatag tgaggaaatg 3900
   gacttttctt cagaacttga ggggtttaga gatcaggctc atctctgctt ataaggagga 3960
   cttcctggag atgatcccaa tagatcaccc ttcacttttg aagaatagca gcttctagct 4020
   gaccatgcaa aatctctgtt tgtttttaaa gactgtttca aagattaaat ctatacaacc 4080
   acttttatga ccaaaaaagt tgttgtgtcc caagtgtcct aattagaaag gcagctggct 4140
   ctcctgcagc tcccaggctt gtgtagtcaa gtgcagtgaa ctgggcagta atcagctcag 4200
   gaccattggc tctttcctcc tctttcagct acagacacat gccctggctt ttgcattgac 4260
   cttgctttgt tagttctaga ctaatttatc cttttgtcag acactttccc ttttttagga 4320
   tactcacaac agagtccact ttcagctatt ggaatacatt ttaccataaa gcccccaagg 4380
   ctttctaccc ataacaccaa cacaaaaaac caattaagtt gcagaggggg gaaataatca 4440
   caaacttttg gcaacctgct taatgctaga cgctctgttt tcactggtta ttctgagtgt 4500
   catatgcaga tgcagatcca agggagacag ggctaaatgg tggtgtcaag aattcctccc 4560
   acaccccaaa aattctttcc aatatttgat tattaaaata tctatcattt ctccaccttg 4620
   tgtctgtgtc ttaagtgtct gtgaatattg taaaagtgct gtatgtttag tagtgttgtg 4680
   tgcctggcag tgctgactat gactactgtg ccatctgtct gtgaccttga tgtcaggtac 4740
   ctggccatgg ggctaccagc aaggatgtgc aaaggaagaa ccgctgcccc tgccctcagc 4800
   ttccttatgc ccgagccact acttatccgt gaatgtgagt gccaagagaa acctaatttg 4860
   gtggggaagc caaggaatgg gagaacgttt tttctgattt gagtcaaggc actaattatt 4920
   agacactctc agacaacaaa gcggtattga cctgagatca aaggaagcag ggacaatatt 4980
   gtagaatgct aggacactgg aaatgaagag gccttcagct tcgccagtct tgattcttga 5040
   ctttacaaat gagaaaatgg agaaacaggg aggctaggtg atttgcccca gatgacactg 5100
   tcaattagtg gagaaattat gattcctcct ctagtacttc ttggtcttac cagcatcaaa 5160
   tgaagctggg ctcacaggga aagtggtttt tggtctggga tctgcagagc acataaaata 5220
   agttagaaag tctataaaag aggaaaaaag gttctcctct ccctcccaaa aatgacttcc 5280
   caacacagaa caataaagga gatgcttgtg tgggtatgca actgtccacc aattatcacc 5340
   taccttctcc ctgagatgaa acaaaacacg cttggtaaat catgatttga taaataacaa 5400
   ttaagttact ggtggttcat gcttgacagc tgcaatctta cccaataagc tattcatctt 5460
   agtaaaacct tgttttagta atatacatgc ataatttaga agttgaaggc tgaaaattgt 5520
   tgtgttcttc tccatatata tacaactttc atcggggtcc cttgagttaa gaaatgcctt 5580
   ctattttata ggtaatctca acagcagact ttgaaatatt gtgtttggtt caatatgcta 5640
   agaagatata caaataataa taacttcgtg agtcttcatg tgcaaaatga caaactgagc 5700
   aattttctgt ataattctag attcttaaaa ccttttttcc cataattgtt tgaatgcact 5760
   aggcacaagt ttctgtttaa gcctctgctt ctccctgaat gtgtagaact gtcactttgc 5820
   ttacaaaggg gctgtgtgtt tgtggctggc agcccagata gagcactctg ggtgctgggg 5880
   attgagggca ccttggccag ctgattgacc tgtctggact ctgtggccca attaatcgag 5940
   gccacagatc agttggtact aagcatcaaa atgattcatt atgcctctct cacactccga 6000
   ttgctttgcc acatagataa cagcatttga aatgggaaac actttagaat gtcattattg 6060
   tattatatat tgtcatctta tgcagtattt ctgaaaattt atttttattg tgatgactga 6120
   ttgaagacat atgtgtatga atagatgttc ctcagtgaag cctattctct tgttgttgat 6180
   gacagtggca tcagagtgaa gagttgttgc agagtaactg acttcagttt aaggttaccc 6240
   tggggttctg gggttaggac ccggattatg cagctgatct cccagcaaac acagctgtat 6300
   gaaataagca actcttcaaa atacaattct tgccactcat tctggacctt tgattcataa 6360
   tgtcctggga taagtccaac ttacaaagat tcccaggtga ttcgtgtgac caccatttgg 6420
   aaaccagtaa tatatacatc cggagtcata tataccattt tgaacaaagt agctaaatgc 6480
   ttatctctcc ttgtgcgatt agtttcacat gttcgttgag gttatcatgt taaatggcaa 6540
   tatagacaca gtcttagtga ttcaacaatt cagctgtttc agccgaattg accagttgct 6600
   aatttataag caaattgtaa ctgatcaatt tagtcagaat ggatgcaagc ccaaagacaa 6660
   ggtggagagc aagctttaat atacactttg tctcaaggcc agctcttcat atttccctct 6720
   ttatatttca aactaagagg cagatctcag gtcactcatc tttagaattg gtgagaatga 6780
   gaaacaggga actgaagtga ttcattcaga ttcaatttgg aagtcattcc cagagcctaa 6840
   aatatttatc taaggggtct gatttatcaa cctcaggtag tctccaaaac acaaaggtat 6900
   tcaggctaaa ataaaaccaa tcaaaaaagg aaattcagga ggctccagtt ctaagactaa 6960
   cccagtataa caagggtgat cagttattac ttgtggcctt gtaccacctc tgttggataa 7020
   tgtgccaccg tcctaatgaa gctttggcca gacttctttc tatagctgtg cacatagaaa 7080
   ggctttagac aacagtgata ccaaagggtt cctttgattt tccttctgtg tgattctcag 7140
   acagttttag ctctttggtg tgctgccaaa tgtttaacaa ctggctctgg aaaacagtgg 7200
   aacccagatt tttagcacct accaatttct atggtgtaaa tactctcatc tcagccaatt 7260
   tcaggctact aacttaatgt caaccagctg aaaacacaac aatatgtttt caagagccag 7320
   tataagttgg ctccaataca gcatgagcca gctccagcat agcactgcct gaacaatttt 7380
   taagtcagtg agtaccaaat aggagaggct agcagaggct ttggagggac ttttaaatgt 7440
   cccctccacc aagggaagca tttccatgtt ggttagtttt actgattttt agtaaaagag 7500
   catattattg gatcccaagg tgttcaaata ttccacgtga ctcttaggaa cctctcaaca 7560
   gactccccag tatcttggga gattaccaac tgtggctttt ctaatttcta agggtgaccc 7620
   tccaggaaaa cattctttaa tttaactacc agttaaaacc atgtttggct gatctttttt 7680
   aaaaaatcta tacctatagt tcatcacttg tttccccaac tttgtcattt tccctatcca 7740
   cttgcccctc atccttctga cacaataaaa aaaatacagg tacccacaaa acagtgcctg 7800
   caaaggagca ggggaagaac aaggcaccca gtaagctcac ttggctctac atatctggaa 7860
   aggcactgag gttccaaggt ctctacccca ccaggggaca cccagtaaac atcacgctga 7920
   gagtttaagt tgcactcaac tggagttgat ttgcccttag aaaagtttcc aacggtgaag 7980
   aaataccact tggtaggaat atctctgggc aacttcattg ctgactgggc aaactttgta 8040
   ggataaggtg tcatgtgatg taaggctgga ccatgaaaaa caaaaatcta ctaatacaca 8100
   tttggcccct gcagtcccct ggcattgagc aatagagcaa ctgtcctttc tcaccactag 8160
   ttgtgagtgt acttccaact gagtgtttag aaatcaggta atctggtaat ccacagaaca 8220
   agctttgttt gcaaattgca aatttttctg gtagaagtca ttcttaggtg ggcttcatta 8280
   aatctttgga gaggcaatgc tgggaaaaaa tatgcctaat tcctaggaca cagtgagtgg 8340
   ccgtaagttg ttatctgcat gtactaaatc aaaattagat catgaaggac caaatctact 8400
   tccgttgcta taataaaata ccctacaggt tctgtaaggc atttctaaac tgtaaatgct 8460
   tgaaacgcaa gttggacatt ttctatacta tgtgtaaaat gccaggttat acctctattt 8520
   tctttcttcc taatggtcat tgaaatgagc ttgtttctct acattgagca agctacattt 8580
   tattttaaat gagtcaggtg catgttcaga tgtctgtatt cccaatacat tgcatgcagc 8640
   ctgagcacaa gtatgccttc accctctggc tcttttcctg acaccaaaca gagaagtcca 8700
   cagctacaag cacaaggggc ttgacaggta ggccttgttt tattatgaac aaattcacca 8760
   gaaaaccatt cttgaggaac cagccactcc catagcattt ttagtcttag ggaagaaaat 8820
   tggcagggaa gaaaaattgg cagacaatgg gaaagaggtt tggtcaacct gcataagtgg 8880
   aataaagtca ttgaagtact tgcaaaaaga agagggaggt ttccctctga aggaatctag 8940
   accatttttt aatatggaga ataactgtac cccatgttag aagtgctctt tagccatatt 9000
   tcaagatcca gatctgaacc catactgtgc agaaaagcac aagagcgctt acagccaaga 9060
   gccctgggag gagttgagta acaggctgag atccggtcac caagcatcat tagttagcac 9120
   tgaaattctc ccaggacaca aaacaaatgt atggaaacat cgcaatacac tatcatttac 9180
   tcacatggga caaccaccta agtccatagc aaaactttac agatctagat gttatctcaa 9240
   gtcaggattc cagatcaaca tcctccatgt ggagatgaga caactgaggt tcagaaagtt 9300
   ggagtgactt atttgcccag ctagctaact ggaattagag tctgggtctc ccaaccactg 9360
   attctgctac accacactgt gctgcttcca ttagttagat ggacattgac aattaattgt 9420
   gaaagcatta attagaagtc aagacaaaag aatgtttgtg atatcttctc tcgcttaaca 9480
   tactttgatt tcaccattgc atttcagcat tttaaagaat agtgtatcag tatagaagga 9540
   ggagcaaagc tctttaagag taatgatgaa accaaaaggt caaaaagtag aagaatagcg 9600
   aatgtaagtt attaaataaa tgagcaagaa gtatgtgtca gggaaaataa atgcttctta 9660
   tattgttaat accgtgattc cctgcccgct caccccgtca ctcaacactt gatgcatttg 9720
   ttttggaaag tgccatttta tgctgagata ctggtattga aaacttccct ctttcccaac 9780
   tctgtcagaa gttctcttgc tctttggaag aagccgaggg ccttaacaaa tcagactgac 9840
   ctcccttgtg acaggcagcc tcgcctgccc ttggaaacaa gctgccagaa tgtgagggcc 9900
   acagagaccc aagagaagcc agcactgcta gactgaggaa cttgtaaata tcttctgctt 9960
   tcttggtgaa caaggacaga ggcaattgcg tgtatattgt gactgtagtt tgtgaagaaa 10020
   atgcaaccat ttgcttcgac agctcctcaa atgtacttgt taagtgtgaa tgtgcctgcc 10080
   tcattgcctt gtgttccaaa cacagtactg aatgcgttgt ttttaaataa accatttcgt 10140
   tttgctttgg gaaa 10154

<210> 158
<211> 6051
<212> DNA
<213> Homo sapiens

<400> 158
gctgcagaag ctgccgccac ctccaatgca caaggtgtct catctgaaaa gaaacctgag 60

ccccagggag gcggcgcgga gcgaccctgg cagagctggc gcaaacaggg cgagaggtcg 120

ctgggcagcg ttcgaggacc agagggagct cggccacaga agaccccagt gatctgatcc 180

cgggatcccg gctccaagct ctcctcgcat tttacagatt tcacccccgc gactatctcc 240

ccaaaacgga gcctttatat caagagaagg tgcgggagct ggggcaacca ggactttctc 300

gggcacccaa gatgcgctcc attaggaaga ggtggacgat ctgcacaata agtctgctcc 360

tgatctttta taagacaaaa gaaatagcaa gaactgagga gcaccaggag acgcaactca 420

tcggagatgg tgaattgtct ttgagtcggt cacttgtcaa tagctctgat aaaatcattc 480

gaaaggctgg ctcttcaatc ttccagcaca atgtagaagg ttggaaaatc aattcctctt 540

tggtcctaga gataaggaag aacatacttc gtttcttaga tgcagaacga gatgtgtcag 600

tggtcaagag cagttttaag cctggtgatg tcatacacta tgtgcttgac aggcgccgga 660

cactaaacat ttctcatgat ctacatagcc tcctacctga agtttcacca atgaagaatc 720

gcaggtttaa gacctgtgca gttgttggaa attctggcat tctgttagac agtgaatgtg 780

gaaaggagat tgacagtcac aattttgtaa taaggtgtaa tctagctcct gtggtggagt 840

ttgctgcaga tgtgggaact aaatcagatt ttattaccat gaatccatca gttgtacaaa 900

gagcatttgg aggctttcga aatgagagtg acagagaaaa atttgtgcat agactttcca 960

tgctgaatga cagtgtcctt tggattcctg ctttcatggt caaaggagga gagaagcacg 1020

tggagtgggt taatgcatta atccttaaga ataaactgaa agtgcgaact gcctatccgt 1080

cattgagact tattcatgct gtcagaggtt actggctgac caacaaagtt cctatcaaaa 1140

gacccagcac aggtcttctc atgtatacac ttgccacaag attctgtgat gaaattcacc 1200

tgtatggatt ctggcccttc cctaaggatt taaatggaaa agcggtcaaa tatcattatt 1260

atgatgactt aaaatatagg tacttttcca atgcaagccc tcacagaatg ccattagaat 1320

tcaaaacatt aaatgtgcta cataatagag gagctctaaa actgacaaca ggaaagtgtg 1380

taaagcaata aagcacattt tgaaacaaac aatatgcact tcttttctga agatgcttcc 1440

gaagatttga aaataggatc caaaacacgg ctgggtttca gcatccacca atgaactgaa 1500

aggtgaataa aggacgttca tgagaaatcg actaccagct gatgaaatac ctgcaaagtg 1560

ctctaaaaat taaatatttt gactttaagg gtcctagtaa gtgccacttc cactaagaat 1620
acagtttgaa tgtataatca gtagtgttta caagatccaa cagtgcactc atcattagtt 1680
aacaaagcaa atatgttcat cactgtcagg ctgcccacag caacaccaag catattagaa 1740
gaggaacccc aggaacgcaa ctcagacctt gggaaattaa accatccttg tcagcagaag 1800
ccaagatgga agcagtttga gcaatgaaat ccgtaagatt aaacaactca agtaaatgct 1860
tcagtcagga ctctgagtct gatcatgaat tttatgtttt aatttatgtt ttttttttgt 1920
cttctggaat ctcttttggt ttggatattg ggatgcttag aaatcctttc tgagatgcat 1980
atgagtgagg aaataaactt taagtaatta tttttaaagt tcttatactt tttaaaagct 2040
atcacacaaa gacttttttt tttttttttg tctcgctctg ttgcccaggc tggagtacag 2100
tggcgcgatc tcagctcact gcaagctccg cctcccaagt tcactccatt ctcctgcctc 2160
agcctccgga gtagctggga ctgcaggcgc ctgccaccac gcctggctaa ttttttgtat 2220
ttttagtgga gacggatttt caccgtgtta gccaggatgg tctcaatctc ctgacctcgt 2280
gatccacccg ccttggcctc ccaaagtgct gggattacag gcgtgagcca ccgtgcctgg 2340
ccgacatttt taaaaaagtt ttattttgca cggctctaaa cctccatgtt attttccagt 2400
ggtgtagaag gtaccagcta aagtgaacca ctatgtaata ttaggccatt ctaaaggaaa 2460
gatgttccat gtcatcagag atggtaaaat aggcagggaa aaaaaaatct ttggtaccaa 2520
agattacact tgtgtttcta cacagcaaac catttttctt tcatgaaaat aatatattat 2580
taacatgaat atattatttt gctattaatg tgaaagttgt ctctaaatat tttttaattt 2640
tcaaactcat actttatttt catttgaaat gtttttcaca ccttttgcat tacataataa 2700
ttttgtggaa gcattttgcc ctttagaata aatattagat tgatatagct gaaatgtgac 2760
ttccagttct ttgatattcc ccttgttatt caaatagaaa tatggaaatg ctttatatat 2820
tactgttaaa tttcttagtg cagaaataac attattaata gagtattgtt ttcaaaacag 2880
atgattaatt tcaagaggtt taacagtgaa attgtgtcaa tattttgcat ttaaaatgaa 2940
tttaattgac cgatattttc tgtagttaaa tttagtcaca atatcacata tgttcttcaa 3000
gaaacacatg aaattattaa taaagtaatt aaaaaatttt taatgtataa cagaattgac 3060
caataggcca gttttctggt aacttatgat agtagattgt ttctttagaa actgggcaga 3120
agctctgcat tctcacttgt actttgattt cttatttctt gggcaggcaa tttgaggaaa 3180
gaagaaatgg catggggaat atatatgttt tgtttcttag ggaaaacagt ctgagaaatg 3240
aataaaaagc atgaagtacg tgtgtgtgtg tgtgtgttac catggaaaag gatattcaca 3300
gtagtacagt tctcaatatt tttaattaga tgtcatattt ttttaatata gtaaaacctt 3360
gggatataga atattacatc ttttgagaat gtatgtgtct ctaagtaagt aaaatctaat 3420
gcgtatagga gactgatagc taaaaatgaa tggaacatta atgtactttt ataattaaac 3480
ctcttatcta tcagaaattg taagagaata gatacatgtt ttgaatgtaa agttgaaaag 3540
tctggtttac ttaataaatt gaaagtgatt tataaaatct aaatttggac tacttgcaaa 3600

tgataagcta ttctagtagc ctttagttta aatccaacag aaatctagaa gtcacaagca 3660
aatatcttaa aggtaaaatc catctgggca ctcatttaaa gtatatctta aaaaagcagc 3720
agcaaggtac cttgccattt ttagcatatt ttcttccttt ttcttttttc tttttttttt 3780
ttttttgaga tggagtctca ctctgtcaca caggctggaa tgcagtgatg ccatctcagc 3840
tcactgcaac ctccacctcc tgggttcaag tgattctcgt gcctcagcct cccaagtagc 3900
tggggttaca ggcgcccacc accacactcg gctaattttg tgtttttagt agagacaaag 3960
tttcaccatg ttggccaggc tggtcttgaa cttcctgacc tcaggttatc cacccacctc 4020
agcctcccaa agtgctggga ttacaggtgt gagccaccgc agccggacca tttttagtat 4080
attttcagta aatacattta aacaatgtta aggccacagc acacatatct cagccattca 4140
ttgttctgtg cattgatgtt tatctcatag atgcattgag tagtgccttt ttagcttttt 4200
cacattactt tgtcaccata tcctttgtgt tctctaaata cattgcccac ttccaaaaat 4260
gttcagcatg aaaaaaaggg cttcagtgtc gattgagatt gcttttgttc atctcaggga 4320
tttcaatagt caagaatgaa ttcagttaaa ggtatttagg gttcaaagaa gacaaactgt 4380
acaagcccat ttcattcctt gttgtatacc tttccatctg ccctcccatt ttaactatct 4440
actgtggcct ttttatggaa acagagcaag atcaatgaag gctaatggca agaataagaa 4500
aaagagttga gatttaacca atagcggagc ataaaggatc atgacaaaat caaattataa 4560
aagcatactt gaaataggtg gagctttttc ttttgaaaat atatattcac aattttaata 4620
ttttaattta ttttttacta tttaaccctg tacttggcaa tgctcaggca gctgattgtg 4680
aaatattctt gtcctttaca gaacatggtt gttattgtgc tgttgacatg aatagaccat 4740
ggaaacattt tcatcattat tattcagcct gtgctgtagt taatgttaag ttgctgaaat 4800
aaaaagtgag caagtaatag attttcttgg caaatctaat gattcagccc acaggactgt 4860
tgaaactact gcggaagttt ttctatctga aagaaggtgc tgggcattca aatgtgttca 4920
tgtattgtat atcatatgaa ttgtatatca attactaatg ggaatttcta catatatgct 4980
tacaaaagca atttatttaa gtaatgctag gggtagtgta cataccaatt agttattcag 5040
ctactataca gaaaaaggat gaacaaatta atttatttct aattgagcca gttagacata 5100
atgcatataa cgtgatattt ggttcatgaa agagttgttt tcatgtggtt attgtaggga 5160
gtatatataa ttgtggaagg ggtatgggaa gagttgtgta tagttagttg ttatctctac 5220
aagtttgaaa gttttcccat caaacattat caatatacca atgttttaaa aattgagtga 5280
gggttattat ttgtatttga tgaaagaaaa tccaaataaa gcccacctag aaatagatat 5340
tttattatat atgtgctata gatataccta tatagtacaa atagacatgt gtgatgcata 5400
tatacaatgt tatatatgtg tatatgtctg tatacacact gagtctgtaa tatgtataca 5460
ctaaatttgt gttatgctaa catcttcagg gtctgcactg tgaactcccc tggagataag 5520
taagtccact ttagaataaa gaagttcttt tgagacttca gttactaacg tgctttaaga 5580
ggtatctact ttataactga attctatgtc gttcatacgt agagttacag taagggtcta 5640

gtatgtccaa atcttaataa taaagaagaa aagtaaaggc ttcaagctag caatgtattc 5700

gaattacagt tttcagattg tggctccagg ccttgtgttt ctcatttaag tagcaccttt 5760

taataaaaac cgtttctttg tgtaggcaaa agcacaagtg tttcaaatgt aaatagcagg 5820

aaaaaaaaag agtttacaga gatagcattg ctgcacagaa taattgctac tgagtatttc 5880

ttatagaatt tgtggaactg aaagatgagg tttattctgt caagttcaag ttcattctgt 5940

tcaacactgt tttcttattg tttgtgtata gcaaccgggt attattgttt tatcatttgt 6000

aaaattgtaa aataaattaa tccctttttt tcactgaaaa aaaaaaaaaa a 6051

<210> 159
<211> 2627
<212> DNA
<213> Homo sapiens

<400> 159
gtacgccccc tccccccgtc cctatcggca gaaccggagg ccaaccttcg cgatcccttg 60

ctgcgggccc ggagatcaaa cgtggcccgc ccccggcagg gcacagcgcg ctgggcaacc 120

gcgatccggc gccggactgg aggggtcgat gcgcggcgcg ctggggcgca caggggacgg 180

agcccgggtc ttgctcccca ttggccgcca cctcggcggg aggggccgcc tccggctcgt 240

ggcacctggc gtctcccctc cgcggggaaa tcggcggcta cccggcaccg gcgcctggag 300

gtaaaggtac ccgggggtgg ggaaggcccc tggccgcggc agagctccct gccaccggcc 360

ctgtcctgct tgccgcccca gcgcctgcga cgcttggatg cccccagctc cggccgcgcc 420

aaccagaaac tttgcccctc gaagtccccc gcctgcctga ccgcaccgtg aggcgccaag 480

tcggccgcgc gcccccgggg gccccggaac cccgcgagtc attgcctggc cgccccgtac 540

cccgcgcccg cggcgcccgg tagccaggat gcgctacgcg gacccctcgg ccaaccggga 600

tttgttgggg agccgaactt tgctcttcat cttcatctgc gcctttgcct tggtgacctt 660

gctgcaacag atcctgtatg gcaggaacta cattaagagg tactttgaat tttatgaggg 720

gccttttgaa tataactcca caagatgcct ggagctgagg cacgaaatat tggaagtgaa 780

ggtgctgtcc atggtgaagc agtcagagct gttcgacagg tggaagagcc tccagatgtg 840

caaatgggcg atgaacatct ctgaggccaa ccagttcaag tctactctgt ccaggtgctg 900

caacgcccct gcctttctct tcaccaccca gaagaacact cccctgggga caaagctcaa 960

gtatgaggtg gacaccagtg gcatctacca catcaaccag gagatcttcc gcatgtttcc 1020

caaggacatg ccctactacc ggtcccagtt taagaagtgt gctgtagtgg gcaacggagg 1080

catcttgaag aacagccgct gcgggaggga gatcaacagc gccgacttcg tcttccggtg 1140

caacctgccc cccatctcag agaagtacac catggatgtg ggggtgaaga cggatgtggt 1200

cactgtgaac cccagcatca tcacagagag gttccacaag ctggagaagt ggcggcggcc 1260

gttctatcgc gtgctgcagg tgtacgagaa cgcgtcggtg ctgctgcctg ccttctacaa 1320

cacgcgcaac accgacgtgt ccatccgcgt caagtacgtg ctggacgact tcgaatcgcc 1380

gcaagctgtc tactacttcc atccgcagta cctggtcaac gtgtcgcgct actggctcag 1440

cctgggggtg cgcgccaagc gcatcagcac cggcctcatt ctggtcactg cggcgctgga 1500
gctctgtgag gaggtgcacc tctttggctt ctgggccttc cccatgaacc cctcgggcct 1560
ctacatcact caccactact atgacaacgt caagccgcgt cccggcttcc acgccatgcc 1620
ctctgagatc ttcaacttcc tgcacttgca cagccgaggc atcctccgcg tgcacacggg 1680
cacctgcagc tgctgctgat ggctgccagc caggctgccc ggcaagcggc gccccctctc 1740
ctgctgtccc tcctggtgga actgggagcc ccgaaccggg cagcgtgggg tcctggcgtt 1800
caggctgtct ctccctccat tgttcagctc tgtacttgga tcctggggta gggaggcagg 1860
gttaggaacc ggggcagagt cagctctgtg ctgagcctcc tggcctggcc cccaccggtg 1920
catccgccca gtgcgtccac ctgccctggc tagcatgctg ctgggccaca ccccaagatc 1980
aggggccctg gggacggcaa gtgaataaag cacatttcca cccaattttg tcatccgaga 2040
gagagcacaa actgcaggcc cttgttgcag ctgaaggaag accctacaga gaatggaatt 2100
gagagtggag acaggacact tcacaggaca cttgagcaca gtcaagattt tattcacact 2160
tttggttcct gtgttttata tcgaagactt agctatgaat tgctatctaa atctcagagc 2220
ttagaagcca acccagtgac tagaccttcc agtgaagaaa gtgatctcaa gaggtccagg 2280
gacttaacag ccaagccaca ccacccgaca ggttcttctg tgacaccgag agatctaacc 2340
caaggcctgg gttatgtctt agtcgagaca tcatcatctg agaagcacca catcctttca 2400
gacacatcct cggacacaca tgcggtcttg gtgcccccag gttcaatcct gcgttagctc 2460
ttttgatgag aaggaaataa accaaaccag ccacttgcac tatggcttcc aagccaatgt 2520
tattgactca ataagtgctt agcaattggc ttcctctttt gtcttcctat tttcagctcc 2580
atttaaacca ggagttattt ataaagacct cttcctctca tgtaaaa 2627

<210> 160
<211> 1398
<212> DNA
<213> Homo sapiens

<400> 160
   atgcggccgg ggggcgcact gctcgccctg ctcgccagcc tgctgctgct gctgctgctg 60
   cgcctgctct ggtgcccggc agacgcgccc ggccgcgcca ggattctggt ggaggaaagc 120
   agggaggcca cccacggcac ccccgcagcg ctgaggacgc tccggagccc ggcgaccgcg 180
   gtaccgcgcg ccactaacag cacatatctg aatgagaagt cgctccaact gacggagaaa 240
   tgcaaaaatc tgcaatatgg cattgagtct ttctctaaca aaacgaaagg gtattcagag 300
   aacgactacc ttcagattat cacagatata cagagttgtc catggaaacg gcaagcagaa 360
   gaatatgcaa attttagagc caaacttgct tcctgctgtg atgctgttca aaactttgtt 420
   gtttctcaga ataacactcc agttgggact aatatgagtt acgaggtgga aagcaaaaaa 480
   gaaatcccaa ttaagaagaa catttttcat atgtttccag tgtcccagcc ttttgtggac 540
   tacccttata atcagtgtgc agtggtcgga aatgggggaa ttctgaataa gtctctctgt 600
   ggaactgaaa tagataaatc cgacttcgtt tttaggtgta acctaccccc aaccacagga 660
   gatgttagta aagatgttgg cagtaaaaca aatcttgtga ctataaatcc aagcatcata 720
   actctgaaat atgggaactt aaaggaaaaa aaagccctat ttctggagga cattgcaacc 780
   tatggagatg catttttcct tctgccagca ttttccttca gggccaacac gggtacctct 840
   ttcaaagtat actacacgct cgaagagtct aaagcaagac aaaaggttct atttttccat 900
   cccaagtacc tgaaagatct ggcccttttc tggagaacta aaggtgtgac tgcataccgc 960
   ttgtccaccg gcttgatgat cacaagtgtt gcagtggaac tgtgtaaaaa tgtgaagctg 1020
   tatggattct ggcccttctc taaaactgta gaagacatac ctgtcagcca tcactattat 1080
   gacaacaagc tacctaaaca tggtttccat cagatgccca aagaatacag ccagatcctc 1140
   caacttcaca tgaaaggaat cctcaaactg caatttagca aatgtgaagt cgcctaaaca 1200
   aagtatctta aaatgggaat aattttaata taatgcagta ggtgattaac aatgtctcca 1260
   aacaccaaag gaggtggcta aagagtattt tgagatgagc cccaaaattt ggtttgacca 1320
   aagcttcccc actcattttg caatgatggc aagtcattca atccttctca tcttcatttt 1380
   ttctccttat aacatgga 1398

<210> 161
<211> 2323
<212> DNA
<213> Homo sapiens

<400> 161
ctgtgctccc ggcgctgacg tgtctgggcg gtcggcttcc actccttcag gcgtcggcag 60
ccactagtcg tggcgagagg ggcggggtgg ccggggctgg cgctccactt ggcccccgct 120
cccggcccgc cccgccgccg cggccccccg gatgagggta tatattcgga gcgagcgcgg 180
gacgccgatg agtggccgcg cggaaggagc tggagacggt cgtagctgcg gtcgcgccga 240
gaaaggttta caggtacata cattacaccc ctatttctac aaagcttggc tattagagca 300
ttatgaacat taatgacctc aaactcacgt tgtccaaagc tgggcaagag cacctactac 360
gtttctggaa tgagcttgaa gaagcccaac aggtagaact ttatgcagag ctccaggcca 420
tgaactttga ggagctgaac ttctttttcc aaaaggccat tgaaggtttt aaccagtctt 480
cttaccaaaa gaatgtggat gcacgaatgg aacctgtgcc tcgagaggta ttaggcagtg 540
ctacaaggga tcaagatcag ctccaggcct gggaaagtga aggacttttc cagatttctc 600
agaataaagt agcagttctt cttctagctg gtgggcaggg gacaagactc ggcgttgcat 660
atcctaaggg gatgtatgat gttggtttgc catcccgtaa gacacttttt cagattcaag 720
cagagcgtat cctgaagcta cagcaggttg ctgaaaaata ttatggcaac aaatgcatta 780
ttccatggta tataatgacc agtggcagaa caatggaatc tacaaaggag ttcttcacca 840
agcacaagta ctttggttta aaaaaagaga atgtaatctt ttttcagcaa ggaatgctcc 900
ccgccatgag ttttgatggg aaaattattt tggaagagaa gaacaaagtt tctatggctc 960
cagatgggaa tggtggtctt tatcgggcac ttgcagccca gaatattgtg gaggatatgg 1020

agcaaagagg catttggagc attcatgtct attgtgttga caacatatta gtaaaagtgg 1080

cagacccacg gttcattgga ttttgcattc agaaaggagc agactgtgga gcaaaggtgg 1140

tagagaaaac gaaccctaca gaaccagttg gagtggtttg ccgagtggat ggagtttacc 1200

aggtggtaga atatagtgag atttccctgg caacagctca aaaacgaagc tcagacggac 1260

gactgctgtt caatgcgggg aacattgcca accatttctt cactgtacca tttctgagag 1320

atgttgtcaa tgtttatgaa cctcagttgc agcaccatgt ggctcaaaag aagattcctt 1380

atgtggatac ccaaggacag ttaattaagc cagacaaacc caatggaata aagatggaaa 1440

aatttgtctt tgacatcttc cagtttgcaa agaagtttgt ggtatatgaa gtattgcgag 1500

aagatgagtt ttccccacta aagaatgctg atagtcagaa tgggaaagac aaccctacta 1560

ctgcaaggca tgctttgatg tcccttcatc attgctgggt cctcaatgca gggggccatt 1620

tcatagatga aaatggctct cgccttccag caattccccg cttgaaggat gccaatgatg 1680

taccaatcca atgtgaaatc tctcctctta tctcctatgc tggagaagga ttagaaagtt 1740

atgtggcaga taaagaattc catgcacctc taatcatcga tgagaatgga gttcatgagc 1800

tggtgaaaaa tggtatttga accagatacc aagttttgtt tgccacgata ggaatagctt 1860

ttatttttga tagaccaact gtgaacctac aagacgtctt ggacaactga agtttaaata 1920

tccacagggt tttattttgc ttgttgaact cttagagcta ttgcaaactt cccaagatcc 1980

agatgactga atttcagata gcatttttat gattcccaac tcattgaagg tcttatttat 2040

ataatttttt ccaagccaag gagaccattg gccatccagg aaatttcgta cagctgaaat 2100

ataggcagga tgttcaacat cagtttactt gcagctggaa gcatttgttt ttgaagttgt 2160

acatagtaat aatatgtcat tgtacatgtt gaaaggtttc tatggtacta aaagtttgtt 2220

ttattttatc aaacattaag cttttttaag aaaataattg ggcagtgaaa taaatgtatc 2280

ttcttgtctc tggaaaaaaa aaaaaaaaaa aaaaaaaaaa aaa 2323

<210> 162
<211> 3682
<212> DNA
<213> Homo sapiens

<400> 162
gcgagcgcag cggagcctgg agagaaggcg ctgggctgcg agggcgcgag ggcgcgaggg 60

cagggggcaa ccggaccccg cccgcaccca tggcgcccgt cgccgtctgg gccgcgctgg 120

ccgtcggact ggagctctgg gctgcggcgc acgccttgcc cgcccaggtg gcatttacac 180

cctacgcccc ggagcccggg agcacatgcc ggctcagaga atactatgac cagacagctc 240

agatgtgctg cagcaaatgc tcgccgggcc aacatgcaaa agtcttctgt accaagacct 300

cggacaccgt gtgtgactcc tgtgaggaca gcacatacac ccagctctgg aactgggttc 360

ccgagtgctt gagctgtggc tcccgctgta gctctgacca ggtggaaact caagcctgca 420

ctcgggaaca gaaccgcatc tgcacctgca ggcccggctg gtactgcgcg ctgagcaagc 480

aggaggggtg ccggctgtgc gcgccgctgc gcaagtgccg cccgggcttc ggcgtggcca 540
gaccaggaac tgaaacatca gacgtggtgt gcaagccctg tgccccgggg acgttctcca 600
acacgacttc atccacggat atttgcaggc cccaccagat ctgtaacgtg gtggccatcc 660
ctgggaatgc aagcatggat gcagtctgca cgtccacgtc ccccacccgg agtatggccc 720
caggggcagt acacttaccc cagccagtgt ccacacgatc ccaacacacg cagccaactc 780
cagaacccag cactgctcca agcacctcct tcctgctccc aatgggcccc agccccccag 840
ctgaagggag cactggcgac ttcgctcttc cagttggact gattgtgggt gtgacagcct 900
tgggtctact aataatagga gtggtgaact gtgtcatcat gacccaggtg aaaaagaagc 960
ccttgtgcct gcagagagaa gccaaggtgc ctcacttgcc tgccgataag gcccggggta 1020
cacagggccc cgagcagcag cacctgctga tcacagcgcc gagctccagc agcagctccc 1080
tggagagctc ggccagtgcg ttggacagaa gggcgcccac tcggaaccag ccacaggcac 1140
caggcgtgga ggccagtggg gccggggagg cccgggccag caccgggagc tcagattctt 1200
cccctggtgg ccatgggacc caggtcaatg tcacctgcat cgtgaacgtc tgtagcagct 1260
ctgaccacag ctcacagtgc tcctcccaag ccagctccac aatgggagac acagattcca 1320
gcccctcgga gtccccgaag gacgagcagg tccccttctc caaggaggaa tgtgcctttc 1380
ggtcacagct ggagacgcca gagaccctgc tggggagcac cgaagagaag cccctgcccc 1440
ttggagtgcc tgatgctggg atgaagccca gttaaccagg ccggtgtggg ctgtgtcgta 1500
gccaaggtgg gctgagccct ggcaggatga ccctgcgaag gggccctggt ccttccaggc 1560
ccccaccact aggactctga ggctctttct gggccaagtt cctctagtgc cctccacagc 1620
cgcagcctcc ctctgacctg caggccaaga gcagaggcag cgagttgtgg aaagcctctg 1680
ctgccatggc gtgtccctct cggaaggctg gctgggcatg gacgttcggg gcatgctggg 1740
gcaagtccct gactctctgt gacctgcccc gcccagctgc acctgccagc ctggcttctg 1800
gagcccttgg gttttttgtt tgtttgtttg tttgtttgtt tgtttctccc cctgggctct 1860
gccccagctc tggcttccag aaaaccccag catccttttc tgcagagggg ctttctggag 1920
aggagggatg ctgcctgagt cacccatgaa gacaggacag tgcttcagcc tgaggctgag 1980
actgcgggat ggtcctgggg ctctgtgcag ggaggaggtg gcagccctgt agggaacggg 2040
gtccttcaag ttagctcagg aggcttggaa agcatcacct caggccaggt gcagtggctc 2100
acgcctatga tcccagcact ttgggaggct gaggcgggtg gatcacctga ggttaggagt 2160
tcgagaccag cctggccaac atggtaaaac cccatctcta ctaaaaatac agaaattagc 2220
cgggcgtggt ggcgggcacc tatagtccca gctactcaga agcctgaggc tgggaaatcg 2280
tttgaacccg ggaagcggag gttgcaggga gccgagatca cgccactgca ctccagcctg 2340
ggcgacagag cgagagtctg tctcaaaaga aaaaaaaaag caccgcctcc aaatgccaac 2400
ttgtcctttt gtaccatggt gtgaaagtca gatgcccaga gggcccaggc aggccaccat 2460
attcagtgct gtggcctggg caagataacg cacttctaac tagaaatctg ccaatttttt 2520

aaaaaagtaa gtaccactca ggccaacaag ccaacgacaa agccaaactc tgccagccac 2580

atccaacccc ccacctgcca tttgcaccct ccgccttcac tccggtgtgc ctgcagcccc 2640

gcgcctcctt ccttgctgtc ctaggccaca ccatctcctt tcagggaatt tcaggaacta 2700

gagatgactg agtcctcgta gccatctctc tactcctacc tcagcctaga ccctcctcct 2760

cccccagagg ggtgggttcc tcttccccac tccccacctt caattcctgg gccccaaacg 2820

ggctgccctg ccactttggt acatggccag tgtgatccca agtgccagtc ttgtgtctgc 2880

gtctgtgttg cgtgtcgtgg gtgtgtgtag ccaaggtcgg taagttgaat ggcctgcctt 2940

gaagccactg aagctgggat tcctccccat tagagtcagc cttccccctc ccagggccag 3000

ggccctgcag aggggaaacc agtgtagcct tgcccggatt ctgggaggaa gcaggttgag 3060

gggctcctgg aaaggctcag tctcaggagc atggggataa aggagaaggc atgaaattgt 3120

ctagcagagc aggggcaggg tgataaattg ttgataaatt ccactggact tgagcttggc 3180

agctgaacta ttggagggtg ggagagccca gccattacca tggagacaag aagggttttc 3240

caccctggaa tcaagatgtc agactggctg gctgcagtga cgtgcacctg tactcaggag 3300

gctgagggga ggatcactgg agcccaggag tttgaggctg cagcgagcta tgatcgcgcc 3360

actacactcc agcctgagca acagagtgag accctgtctc ttaaagaaaa aaaaagtcag 3420

actgctggga ctggccaggt ttctgcccac attggaccca catgaggaca tgatggagcg 3480

cacctgcccc ctggtggaca gtcctgggag aacctcaggc ttccttggca tcacagggca 3540

gagccgggaa gcgatgaatt tggagactct gtggggcctt ggttcccttg tgtgtgtgtg 3600

ttgatcccaa gacaatgaaa gtttgcactg tatgctggac ggcattcctg cttatcaata 3660

aacctgtttg ttttaaaaaa aa 3682

<210> 163
<211> 1528
<212> DNA
<213> Homo sapiens

<400> 163
cggcgagcga gcaccttcga cgcggtccgg ggaccccctc gtcgctgtcc tcccgacgcg 60

gacccgcgtg ccccaggcct cgcgctgccc ggccggctcc tcgtgtccca ctcccggcgc 120

acgccctccc gcgagtcccg ggcccctccc gcgcccctct tctcggcgcg cgcgcagcat 180

ggcgcccccg caggtcctcg cgttcgggct tctgcttgcc gcggcgacgg cgacttttgc 240

cgcagctcag gaagaatgtg tctgtgaaaa ctacaagctg gccgtaaact gctttgtgaa 300

taataatcgt caatgccagt gtacttcagt tggtgcacaa aatactgtca tttgctcaaa 360

gctggctgcc aaatgtttgg tgatgaaggc agaaatgaat ggctcaaaac ttgggagaag 420

agcaaaacct gaaggggccc tccagaacaa tgatgggctt tatgatcctg actgcgatga 480

gagcgggctc tttaaggcca agcagtgcaa cggcacctcc acgtgctggt gtgtgaacac 540

tgctggggtc agaagaacag acaaggacac tgaaataacc tgctctgagc gagtgagaac 600

ctactggatc atcattgaac taaaacacaa agcaagagaa aaaccttatg atagtaaaag 660

tttgcggact gcacttcaga aggagatcac aacgcgttat caactggatc caaaatttat 720
cacgagtatt ttgtatgaga ataatgttat cactattgat ctggttcaaa attcttctca 780
aaaaactcag aatgatgtgg acatagctga tgtggcttat tattttgaaa aagatgttaa 840
aggtgaatcc ttgtttcatt ctaagaaaat ggacctgaca gtaaatgggg aacaactgga 900
tctggatcct ggtcaaactt taatttatta tgttgatgaa aaagcacctg aattctcaat 960
gcagggtcta aaagctggtg ttattgctgt tattgtggtt gtggtgatag cagttgttgc 1020
tggaattgtt gtgctggtta tttccagaaa gaagagaatg gcaaagtatg agaaggctga 1080
gataaaggag atgggtgaga tgcataggga actcaatgca taactatata atttgaagat 1140
tatagaagaa gggaaatagc aaatggacac aaattacaaa tgtgtgtgcg tgggacgaag 1200
acatctttga aggtcatgag tttgttagtt taacatcata tatttgtaat agtgaaacct 1260
gtactcaaaa tataagcagc ttgaaactgg ctttaccaat cttgaaattt gaccacaagt 1320
gtcttatata tgcagatcta atgtaaaatc cagaacttgg actccatcgt taaaattatt 1380
tatgtgtaac attcaaatgt gtgcattaaa tatgcttcca cagtaaaatc tgaaaaactg 1440
atttgtgatt gaaagctgcc tttctattta cttgagtctt gtacatacat acttttttat 1500
gagctatgaa ataaaacatt ttaaactg 1528

<210> 164
<211> 2166
<212> DNA
<213> Homo sapiens

<400> 164
agtacagctc cggccgccgc gccgcctggc tttcgtattc gttgttctcg gcgggctgtg 60
gggcctccgc gccgcggccg ttagtcatgt cggattctgg aagttacggt cagtctgggg 120
gtgagcagca aagttattct acctatggaa atccaggcag ccaaggctat ggacaagcat 180
cacaaagcta ttctggctat gggcaaacga ctgattcctc ttatggacag aactacagcg 240
gttactccag ttatggacaa agttattcac agtcctatgg tggttatgag aatcaaaagc 300
agagctcata tagccagcaa ccatataata accagggaca gcagcaaaac atggaatcat 360
caggaagcca aggtggaaga gcaccttcct atgaccagcc agactatggt caacaagatt 420
catatgacca gcagtcaggc tatgatcaac atcaaggctc atatgatgag cagtcaaatt 480
atgatcagca gcatgattcc tatagtcaaa accagcagtc ctatcattca caaagggaaa 540
actacagcca ccacacacaa gatgaccgtc gtgatgtgag taggtatgga gaagataata 600
gaggatatgg cgggtcacag ggaggaggta gagggcgtgg gggatatgac aaggatggaa 660
gaggtcctat gacaggatca agtggtggtg accgcggtgg cttcaaaaat tttggtggtc 720
acagggatta tggacccaga acagatgctg attcagaatc tgataattca gataacaaca 780
caatctttgt gcaaggactt ggggagggtg tgtctacaga tcaagttggg gagttcttta 840
aacaaatagg aattatcaag acaaataaga agaccggaaa accaatgata aatctttata 900

cagacaagga cacaggaaag ccaaaggggg aggcaacagt gtcatttgat gaccctcctt 960

cagctaaggc agccattgac tggtttgatg gaaaagaatt ccatggcaac atcattaaag 1020

tgtcctttgc cactagaaga cctgaattca tgagaggagg tggaagtgga ggtgggcggc 1080

gaggccgtgg aggatataga ggtcgtggag gctttcaagg gagaggtgga gaccccaaaa 1140

gtggggattg ggtttgccct aatccgtcat gcggaaatat gaactttgct cgaaggaatt 1200

cctgcaatca gtgcaatgag cctagaccag aggactctcg tccctcagga ggagatttcc 1260

gggggagagg ctacggtgga gagaggggct acagaggtcg tgggggcaga ggtggagacc 1320

gaggcggcta tggtggagac agaagtgggg gtggctatgg tggagacaga agcagcggtg 1380

gtggctacag cggagataga agtgggggcg gctatggtgg agacagaagt gggggtggct 1440

atggtgggga cagaggcggc ggctatggtg gggacagagg aggcggctat ggaggagacc 1500

gaggaggtgg ctatggagga gatcgaggtg gctatggagg agaccgaggt ggaggctatg 1560

gtggagaccg aggaggctat ggaggagatc gaggaggtta cggaggagat cgaggaggtt 1620

atggaggaga tcgaggaggc tatggaggag acagaagccg ggggggctat ggaggagacc 1680

gtggtggtgg cagtggctac ggtggagacc gaagtggagg ctatggagga gacaggagtg 1740

gtggcggcta tggaggagac cgaggtgggg gctacggagg agaccgaggt ggctatggag 1800

gcaaaatggg aggaagaaac gactacagaa atgatcagcg caaccgacca tactgatgac 1860

tgttttgaat gttcctttgt ctctgacatg atccatagtg aaattgccag agttttgcct 1920

gctgctttcc tcgtggcctc ttcttgggta gtgaaattaa gtgacatttg gatttttatt 1980

tgggtgggag ggctgggaca gtttttcttc tagaaatgtc tgttgagatt tcccccttta 2040

gtttccaacc ttctccccaa cccttggagc taaatgcgtt gtaaaatatt gccaaaatga 2100

aaagtgtttt gtaatactgc aataaaggct gcttgttttt gtggaaaaaa aaaaaaaaaa 2160

aaaaaa 2166

<210> 165
<211> 3466
<212> DNA
<213> Homo sapiens

<400> 165
agtgacgcaa ggcctactgt cggctgggag gggaggtgta gccggtcttt gggggtaggc 60

ggtagtggcg gaagaggttc ggcggctgat ggcggatcag gatcggaagc ctgcgtaact 120

ttctcccttg atccgggagt ctttccactg gattcacaat gacatccttt caagaagtcc 180

cattgcagac ttccaacttt gcccatgtca tctttcaaaa tgtggccaag agttaccttc 240

ctaatgcaca cctggaatgt cattacacct taactccata tattcatcca catccaaaag 300

attgggttgg tatattcaag gttggatgga gtactgctcg tgattattac acgtttttat 360

ggtcccctat gcctgaacat tatgtggaag gatcaacagt caattgtgta ctagcattcc 420

aaggatatta ccttccaaat gatgatggag aattttatca gttctgttac gttacccata 480

agggtgaaat tcgtggagca agtacacctt tccagtttcg agcttcttct ccagttgaag 540

agctgcttac tatggaagat gaaggaaatt ctgacatgtt agtggtgacc acaaaagcag 600
gccttcttga gttgaaaatt gagaaaacca tgaaagaaaa agaagaactg ttaaagttaa 660
ttgccgttct ggaaaaagaa acagcacaac ttcgagaaca agttgggaga atggaaagag 720
aacttaacca tgagaaagaa agatgtgacc aactgcaagc agaacaaaag ggtcttactg 780
aagtaacaca aagcttaaaa atggaaaatg aagagtttaa gaagaggttc agtgatgcta 840
catccaaagc ccatcagctt gaggaagata ttgtgtcagt aacacataaa gcaattgaaa 900
aagaaaccga attagacagt ttaaaggaca aactcaagaa ggcacaacat gaaagagaac 960
aacttgaatg tcagttgaag acagagaagg atgaaaagga actttataag gtacatttga 1020
agaatacaga aatagaaaat accaagctta tgtcagaggt ccagacttta aaaaatttag 1080
atgggaacaa agaaagcgtg attactcatt tcaaagaaga gattggcagg ctgcagttat 1140
gtttggctga aaaggaaaat ctgcaaagaa ctttcctgct tacaacctca agtaaagaag 1200
atacttgttt tttaaaggag caacttcgta aagcagagga acaggttcag gcaactcggc 1260
aagaagttgt ctttctggct aaagaactca gtgatgctgt caacgtacga gacagaacga 1320
tggcagacct gcatactgca cgcttggaaa acgagaaagt gaaaaagcag ttagctgatg 1380
cagtggcaga acttaaacta aatgctatga aaaaagatca ggacaagact gatacactgg 1440
aacacgaact aagaagagaa gttgaagatc tgaaactccg tcttcagatg gctgcagacc 1500
attataaaga aaaatttaag gaatgccaaa ggctccaaaa acaaataaac aaactttcag 1560
atcaatcagc taataataat aatgtcttca caaagaaaac ggggaatcag cagaaagtga 1620
atgatgcttc agtaaacaca gacccagcca cttctgcctc tactgtagat gtaaagccat 1680
caccttctgc agcagaggca gattttgaca tagtaacaaa ggggcaagtc tgtgaaatga 1740
ccaaagaaat tgctgacaaa acagaaaagt ataataaatg taaacaactc ttgcaggatg 1800
agaaagcaaa atgcaataaa tatgctgatg aacttgcaaa aatggagctg aaatggaaag 1860
aacaagtgaa aattgctgaa aatgtaaaac ttgaactagc tgaagtacag gacaattata 1920
aagaacttaa aaggagtcta gaaaatccag cagaaaggaa aatggaaggt cagaattccc 1980
agagtcctca atgtttcaaa acatgctcag agcaaaatgg ttatgttctc acattgtcaa 2040
atgcacaacc agttctgcaa tatggtaatc cttatgcatc tcaggaaaca agagatggag 2100
cagatggtgc tttttaccca gatgaaatac aaaggccacc tgtcagagtc ccctcttggg 2160
gactggaaga caatgttgtc tgcagccagc ctgctcgaaa ctttagtcgg cctgatggct 2220
tagaggactc tgaggatagc aaagaagatg agaatgtgcc tactgctcct gatcctccaa 2280
gtcaacattt acgtgggcat gggacaggct tttgctttga ttccagcttt gatgttcaca 2340
agaagtgtcc cctctgtgag ttaatgtttc ctcctaacta tgatcagagc aaatttgaag 2400
aacatgttga aagtcactgg aaggtgtgcc cgatgtgcag cgagcagttc cctcctgact 2460
atgaccagca ggtgtttgaa aggcatgtgc agacccattt tgatcagaat gttctaaatt 2520
ttgactagtt actttttatt atgagttaat atagtttagc agtaaaaaaa aaaaaaaaaa 2580

ccacacctaa aatagaccac tgaggagacc atagagcgga tgctttcatg caccctttac 2640
tgcactttct gaccaggagc tactttgagt ttggtgttac taggatcagg gtcagtcttt 2700
ggcttatcaa taaattttaa tctctgttaa tcttacctgc tttaaaaaaa agttcttgtg 2760
tgttcgtatc tttatttatt ccctagtttg cagaactgtc tgaataaagg atacaaggat 2820
tatttcaatg ttactgcact gaaaaacgtg tatgtattag tgtgctagat tatttagcag 2880
aatattcaca agtttctgtt gaccttgttg attgagcatg actactaaat attatgtaat 2940
aaaaagcatt tgtcataaca gtcttatgaa gtagttcttc gaatatagaa agttctataa 3000
tttagcccat gaaatgatag gtttttaatt ttcagaaatg gagctgcatg tagaatgaga 3060
tcacatgctt ttatatgtga aatattggtt ttagcaatta acagaaggca tactttgcta 3120
attttatggc aaaattttag aataacctga atgattattt ttaaactatc ttgaagttgt 3180
atgtatatat ctaatgggaa aatggaacaa gagatgtcag tataattgtt ttcctattaa 3240
atgatctcat aacaggggtg tttgtggcat aaaaatcata gaatatatat cagtatcttt 3300
tttacatttt cctgatgaga atataaattt gaaatattct cagtaagtga ttaaaagtac 3360
aatacataaa cccaaatcaa agtaatgtgt ttcttcagtt atgtctgtgc tgtatagaac 3420
tgtcactaac attaaagaaa gaggacacat ttttcaagat ctgtaa 3466

<210> 166
<211> 8382
<212> DNA
<213> Homo sapiens

<400> 166
cgcagcggcg gcggcagcgg cggcggcggc tacagcagct ccgccggcag ccgcgggagc 60
acggcgacgc cagcggcggg aagggaaaag gccgaggcat cagcgtgtga agaccgcaaa 120
gacgatcccg agtacagttg tgaacagcat tgctgctagg ctcctcctgc agatcatctg 180
aaatgaacct ctcttattga tttttattgg cctagagcca ggagtactgc attcagttga 240
ctttcagggt aaaaagaaaa cagtcctggt tgttgtcatc ataaacatat ggaccagtgt 300
gatggtgaaa tgagatgagg ctccgcaatg gaactgtagc cactgcttta gcatttatca 360
cttccttcct tactttgtct tggtatacta catggcaaaa tgggaaagaa aaactgattg 420
cttatcaacg agaattcctt gctttgaaag aacgtcttcg aatagctgaa cacagaatct 480
cacagcgctc ttctgaatta aatacgattg tgcaacagtt caagcgtgta ggagcagaaa 540
caaatggaag taaggatgcg ttgaataagt tttcagataa taccctaaag ctgttaaagg 600
agttaacaag caaaaaatct cttcaagtgc caagtattta ttatcatttg cctcatttat 660
tgaaaaatga aggaagtctt caacctgctg tacagattgg caacggaaga acaggagttt 720
caatagtcat gggcattccc acagtgaaga gagaagttaa atcttacctc atagaaactc 780
ttcattccct tattgataac ctgtatcctg aagagaagtt ggactgtgtt atagtagtct 840
tcataggaga gacagatatt gattatgtac atggtgttgt agccaacctg gagaaagaat 900

tttctaaaga aatcagttct ggcttggtgg aagtcatatc accccctgaa agctattatc 960
ctgacttgac aaacctaaag gagacatttg gagactccaa agaaagagta agatggagaa 1020
caaagcaaaa cctagattac tgttttctaa tgatgtatgc tcaagaaaag ggcatatatt 1080
acattcagct tgaagatgat attattgtca aacaaaatta ttttaatacc ataaaaaatt 1140
ttgcacttca actttcttct gaggaatgga tgattctaga gttttcccag ctgggcttca 1200
ttggtaaaat gtttcaagcg ccggatctta ctctgattgt agaattcata ttcatgtttt 1260
acaaggagaa acccattgat tggctcctgg accatattct ctgggtgaaa gtctgcaacc 1320
ctgaaaaaga tgcaaaacat tgtgatagac agaaagcaaa tctgcgaatt cgcttcagac 1380
cttccctttt ccaacatgtt ggtctgcact catcactatc aggaaaaatc caaaaactca 1440
cggataaaga ttatatgaaa ccattacttc ttaaaatcca tgtaaaccca cctgcggagg 1500
tatctacttc cttgaaggtc taccaagggc atacgctgga gaaaacttac atgggagagg 1560
atttcttctg ggctatcaca ccgatagctg gagactacat cttgtttaaa tttgataaac 1620
cagtcaatgt agaaagttat ttgttccata gcggcaacca agaacatcct ggagatattc 1680
tgctaaacac aactgtggaa gttttgcctt ttaagagtga aggtttggaa ataagcaaag 1740
aaaccaaaga caaacgatta gaagatggct atttcagaat aggaaaattt gagaatggtg 1800
ttgcagaagg aatggtggat ccaagtctca atcccatttc agcctttcga ctttcagtta 1860
ttcagaattc tgctgtttgg gccattctta atgagattca tattaaaaaa gccaccaact 1920
gatcatctga gaaaccaaca cattttttcc tgtgaatttg ttaattaaag atagttaagc 1980
atgtatcttt tttttatttc tacttgaaca ctacctcttg tgaagtctac tgtagataag 2040
acgattgtca tttccacttg gaaagtgaat ctcccataat aattgtattt gtttgaaact 2100
aagctgtcct cagattttaa cttgactcaa acatttttca attatgacag cctgttaata 2160
tgacttgtac tattttggta ttatactaat acataagagt tgtacatatt gttacattct 2220
ttaaatttga gaaaaactaa tgttacatac attttatgaa gggggtactt ttgaggttca 2280
cttattttac tattatagac cctcttttat agattatcag ggattatata tataaatata 2340
taaatataca taaaaatgtt atggaattaa tttattagaa acacttaaga agtacatatt 2400
tttgtgcagt agatttttga atcgatactt tttttgaaaa ccagtttcct tgctttttta 2460
agttctttct atatttttct ttggaaatgc aaacattaca aaccaatgcc atttttcaaa 2520
tgcactgcca tttaagattg attatagatg gatttcttaa ttgaagtact tttataatca 2580
cagtgactga acaaaatatt ttcaaagaca tttgtcattc cttaaagcca agattttaaa 2640
gactaatgtc cttcctgagg gttactttac tatactgtgt atggtgtata gccacagaaa 2700
gtcagtctga taaattttca atgtgtaagt gtgatgcatt caacccagat tattgtagac 2760
ttaacttatt atatttcctc tgatatgtaa aatgacaaac tcttctttcc tttttaatct 2820
ttgtctttaa tccatgaatt aggtgcatta tctgctgttg attcttttat atctgcactc 2880
attttatgac gtcagacaaa atgttttcta ggtgttgcta ttgtatgagt cataattggt 2940

ataattttaa agaaaattgt aatattgttt tttggtgaaa tatattatta gtagtatatt 3000
ttccaaaatg attttattgt agtatgaact gataagtgag tcccgtgtcc aagagactgt 3060
atttattact cttaaacacg ctattccaaa tgatattact actataaaca aagaatatta 3120
ccggtctcag caatcagtga tctttataaa agtactttta aaaaatgatg aattatcacc 3180
aggcgctgtg gctcacgcct gtaatcccag cactttggga ggccgaggcg ggcggatcac 3240
gaggtcagga gatcaagacc atcctggcta acacggtgaa accctgtctc tactaaaaat 3300
acaaaaaatt agccaggcgt ggtggcaggt gcctgtaatc ccagctactt gggaggctga 3360
ggcaggagaa tggcgtgaac ccaggaggca gagctggcag tgagctgaga tcgcaccact 3420
gcactccagc ctgggcgaca cagctagact ccgtctcaaa aaaaaaaaaa gatgaagtat 3480
caacatcagt tcccagccag tctgcttgga cacactgggg tgccacaagt gggttgcagg 3540
ttggttgcag acgggcagag atggattctg ctgagctttc agagtgacca ctggctgggg 3600
cctagcgctg gggcaaggtt ccagctggct gcctctagcc tggaggagct tcatctagtt 3660
acccaatgtg ctgaaccaac agcatgaaat agcatcattt cctgtgctgt gttacaaagt 3720
gcaaacgttt ggaaacactg atacagtaca attatttcca tcactccatg ttgttataat 3780
ttttagttac ttttcttgat tgaaaagtca atcacattgc caaaaatgat ttttatgtca 3840
gttaaaggcc agttgaaatt tttttattag gatgttttaa atttactttt cactttcttg 3900
aatttttttc ttctcaaatt tccttggccc agtgcccatg agcaaatttc attcaaaaac 3960
aggaataacc ttttgtcata aaattttggc accagcttgg tatactaaat tactgcaaag 4020
ataactgagt accagaagta aaaatctctg caggtgggct taggagtact cttgggtccc 4080
tggttcctca gttgccaact ctggtttcat gaagaaagac catgctctga aaatctaggg 4140
aggagaaagt tattgcagca cctggtgtga tttgttacaa cttctttaaa gacagcctgc 4200
ctggctagct ttttcctgtt ttcatgcagt ttgccctccc agcattggtc cacctgttgt 4260
tcccaagaaa ctcattcttg ttcccaagac tccttttagc caaatctttc ctatcattcc 4320
aggacttttt tgtgcttctc tccccatgaa tctacccgtg cctagggtgt gtgccaggca 4380
cgtggcaggt ggccagtcag catctggatg tgagaccaat tattccagcc tgccctcctc 4440
tttctctttt tatcattttg tattattttt gagacagggt ctcgctttgt tgcccaggct 4500
ggagtgcagt ggtgctctca tagctcactg cagcctcgat ctctggcctc aagcaatcct 4560
cctgcctcag cctcccaaag tgctgggatt ataggtgtgt gccaccatgt ccagccccct 4620
ttctctctct taactccctc tccctgctgt cgttgccata ctcttgcata cacaatcggt 4680
tgtctagttt ttgattgttt gtgtgtatgg tgtgtgctgt ctctctgacc agatttcagg 4740
ttcctgaggc gagcctgcag ctcatactgc tcatctgtcc tctcctatgg tgggtgctca 4800
gggcctctca ctgttagtta ctccctcctt tctgcccagt tctgcactca actagtagaa 4860
gcagccatcc tttccccaag caggaaattg tagtggtcgc ccttaagagc agtgtgaggg 4920
cagaagatta agggagggga agagtccctg gaactggaag aaggtaaata ctttgccttg 4980

agagggcgcc gaatcatttt accaaaatag taaatggaaa aagtgtcaaa ggttgggact 5040
agttttaaaa acacatatag ccagtagaca catgggggct gtttaaataa aaacattttt 5100
ttacccggtt ctgccatcac acacactagt gacattccaa gtactcagtg gccatgtgga 5160
gctagtggcc accacacttg acaccacgga tgcagaactt ttccatcact gcagaaagct 5220
ctgttgaaca gtactgtgtc cagagtgcta attatgaaca gcttagaaac cacaattaat 5280
acattctcat gaggttaaaa gcagacatgg gaatagaaca taatgcaaat gatgatgaat 5340
attttgggtg agaagagaaa gctgaggacc catttctagg attctaagat gtaagatttc 5400
ttaagttctt tatcttagtc tcatgcattc tccacatcac gcgctgtacc atactgtgta 5460
gtcagaacag acagtgtgat tgaaaagctt tggaaaaagt taacacaaag gattatttag 5520
cacataggct gtagatacgt atgtgtgtat ttgttcaaca attggagatg gttgaatacc 5580
cttgaacaaa gtgtgtatct tctcaaatca gtggttgcac tagtcaataa ttagaaggtg 5640
ttgttatttt taaaactata agcaaaatta tgaaggcctt taaaaaatct atcataataa 5700
tgaaaaagag gttgtctccc aacagtgctg tccctcaaag aaaagactgg ttatgtggaa 5760
acagcacgtt tggagagatt attctagtga ataacagtgt atatttgtgg taggcaaatt 5820
tctaagaatg acccctactg acacccccac caatgacctt gtataatatc ctcccacttt 5880
gagcatgggt ggaacctgtt tgatccatga ttatatcatg ttatgtagca aaaggaagac 5940
tgtctggggg tggctaatca aatcttgtga ctcctttaaa agcagagagt tttctcgggc 6000
tggtggcaga agtcaagaga caaagcagaa ggacctcagg caccattgct gttttaaggt 6060
agagggggct gcatgagaag gaatgcacat ggctttgagg agctgagaga ggccttggct 6120
aacagcaaag gagctgggaa ctgcagtcct acagccacgg gaactgaatt cagcccataa 6180
gctcattcat aggcttggaa aggcattcat ctccagtctc cagataagac ccagcctggc 6240
caacactttg aatccagcct tgtgagaccc taagccgaga actcacttaa gcctgcttgg 6300
acttctgacc tacagaacta tgagataata aatcagtggt gttttaagct gctaaatttg 6360
tggcaatttg ttatgtagga ataggaaatg aatacagtat tttaaaagag gtaaagagga 6420
aatcttcata cttgattccc tgtagtatgc tggatatttc ttggtaaatg catgaataaa 6480
gtctttagta gcaagtcatg tacctggacc ttgttgggga tgttctcgcc agagatctca 6540
attagtcttc attggacgtt tgttggcaaa gttgcagcat cttactgagt agcgtttggt 6600
aaaggaactg ctagaatgag actataggaa taggactgaa acagtgtgta agggatcttt 6660
tttatttcta gaagaggtgt tactttatac tgatttagac tgctctgagc ctgtcagaag 6720
taaattcaag aatgattaac agtggtattt tagatacatt aagagcaaaa ataacctttg 6780
ccttgtagag atcagataat ctgtgtatcg agggatttaa ttttttttaa ccaaaactta 6840
gtttctcttt ttatcatcta gcacagatca aatcctgtat gggccagcca aatgtttgta 6900
gtttgagaat gttttgcaaa tacctctctg cttccaagcc attaatagct aatgaagttt 6960
tattttggtt tgtccattaa tttttctgct tatttttact gtattgccat gtccttgata 7020

cgagacaagt agaactaaaa gagagaaaat gagaggttaa gactgtcaga agatgagaaa 7080
tgtgagtcat aaccatacct tagtgtttta agggaacttt atccagtcca atccctttat 7140
agaaaaggaa cttttccaag ttatgttgca gtatttatag tgtgtaaaat tttgtttgac 7200
tttggtagtt gtgcatttta aattatgaat cttatatgct acgataacta gtatgcttac 7260
tttctttaat gcatgtgatc atacaatgct aaaaaggtat tttttaaaga cccaaccaaa 7320
ctgaggattt tgttatacat atctgtatca acacctatat ataaactctt cttcttacct 7380
gaaacactcc ttcacccggt ttgggcacta actagatcat tttcaaagga acaaacagga 7440
aggagcaagc tggcactcca gggtgcatct gagctgagaa tttagaagac ctcattcaag 7500
aaagtaaact gagcattgat gggaaagcac tttgcgcgaa gtaacattcc cactgttaat 7560
gttgcagtca aaattgtgga aaaaaaaaag tggtgctaaa attgttctga aatgaagatt 7620
gtttgttgtg gggaatattt taatactctt ggactttagg gaggctattt tatgtgtcac 7680
ttgctcctag ttgtttcatt gtattagtaa ttagcaaact gtcctggaac caagggtttg 7740
gtacttttgc ctgattagca ttccattatt tatttgaaat gctcagtgtt tgctcaaatt 7800
tggtgaaaat atcttttgaa gacatattct ctgagcctta aaaatggctg ctatcgacag 7860
tactgtaaga tgcactgcca gtagatttga tacttttcat ttcagtgtag gcagtaatgt 7920
tttctaagaa agatttagaa atcagcaaag tgtaacaatt gtgtttcatt tatggaatgg 7980
aaaaactgaa gtgtcccatt cagaacactt tggaaaggtt gtctgcatta ggggatgttt 8040
ctggaagcat cccttagaga agctgcctga ggaatctcct tgccaggttg taccagattt 8100
ttttttttct taaatgtatt gtatatattt tccttaaaat gtcttcattg gctgaatgat 8160
tcctatccgt agtaatattt taggtacttg ttggattttt aaaggaagat tatttatttt 8220
cagatttgct gctataattg aaaacctagt aactggtcct gttgattgtg ccttcatcac 8280
tgtttctctg tgccacgacc gttcatgtgt atttgaaata aagaagttta aaaagccatg 8340
ttgaattcaa gattccttct aataaaaata ggtgaaactt aa 8382

<210> 167
<211> 5445
<212> DNA
<213> Homo sapiens

<400> 167
ggccgaggag ccgtcgccgc catttcaaga ccgtactagg tagatggtca attagagttc 60
ccagggtttg aagcctgtaa ctgctgccgc cgctcaagcc ctccagagca ttgctacggc 120
tgctgccctt gtactactac ctccaaatac gttcttgctg gtagtggcgg cagcaggacc 180
aattacctct tttttgctct ccctcgagaa gctccagatg gcgtcttccg tgggcaacgt 240
ggccgacagc acagggttag ctgagttggc acatcgagaa tatcaggcag gagattttga 300
ggcagctgag agacactgca tgcagctctg gagacaagag ccagacaata ctggtgtgct 360
tttattactt tcatctatac acttccagtg tcgaaggctg gacagatctg ctcactttag 420
cactctggca attaaacaga acccccttct ggcagaagct tattcgaatt tggggaatgt 480

gtacaaggaa agagggcagt tgcaggaggc aattgagcat tatcgacatg cattgcgtct 540
caaacctgat ttcatcgatg gttatattaa cctggcagcc gccttggtag cagcgggtga 600
catggaaggg gcagtacaag cttacgtctc tgctcttcag tacaatcctg atttgtactg 660
tgttcgcagt gacctgggga acctgctcaa agccctgggt cgcttggaag aagccaaggc 720
atgttatttg aaagcaattg agacgcaacc gaactttgca gtagcttgga gtaatcttgg 780
ctgtgttttc aatgcacaag gggaaatttg gcttgcaatt catcactttg aaaaggctgt 840
cacccttgac ccaaactttc tggatgctta tatcaattta ggaaatgtct tgaaagaggc 900
acgcattttt gacagagctg tggcagctta tcttcgtgcc ctaagtttga gtccaaatca 960
cgcagtggtg cacggcaacc tggcttgtgt atactatgag caaggcctga tagatctggc 1020
aatagacacc tacaggcggg ctatcgaact acaaccacat ttccctgatg cttactgcaa 1080
cctagccaat gctctcaaag agaagggcag tgttgctgaa gcagaagatt gttataatac 1140
agctctccgt ctgtgtccca cccatgcaga ctctctgaat aacctagcca atatcaaacg 1200
agaacaggga aacattgaag aggcagttcg cttgtatcgt aaagcattag aagtcttccc 1260
agagtttgct gctgcccatt caaatttagc aagtgtactg cagcagcagg gaaaactgca 1320
ggaagctctg atgcattata aggaggctat tcgaatcagt cctacctttg ctgatgccta 1380
ctctaatatg ggaaacactc taaaggagat gcaggatgtt cagggagcct tgcagtgtta 1440
tacgcgtgcc atccaaatta atcctgcatt tgcagatgca catagcaatc tggcttccat 1500
tcataaggat tcagggaata ttccagaagc catagcttct taccgcacgg ctctgaaact 1560
taagcctgat tttcctgatg cttattgtaa cttggctcat tgcctgcaga ttgtctgtga 1620
ttggacagac tatgatgagc gaatgaagaa gttggtcagt attgtggctg accagttaga 1680
gaagaatagg ttgccttctg tgcatcctca tcatagtatg ctatatcctc tttctcatgg 1740
cttcaggaag gctattgctg agaggcacgg caacctgtgc ttagataaga ttaatgttct 1800
tcataaacca ccatatgaac atccaaaaga cttgaagctc agtgatggtc ggctgcgtgt 1860
aggatatgtg agttccgact ttgggaatca tcctacttct caccttatgc agtctattcc 1920
aggcatgcac aatcctgata aatttgaggt gttctgttat gccctgagcc cagacgatgg 1980
cacaaacttc cgagtgaagg tgatggcaga agccaatcat ttcattgatc tttctcagat 2040
tccatgcaat ggaaaagcag ctgatcgcat ccatcaggat ggaattcata tccttgtaaa 2100
tatgaatggc tatactaagg gcgctcgaaa tgagcttttt gctctcaggc cagctcctat 2160
tcaggcaatg tggctgggat accctgggac gagtggtgcg cttttcatgg attatattat 2220
cactgatcag gaaacttcgc cagctgaagt tgctgagcag tattccgaga aattggctta 2280
tatgccccac acttttttta ttggtgatca tgctaatatg ttccctcacc tgaagaaaaa 2340
agcagtcatc gattttaagt ccaatgggca catttatgac aatcggatag ttctgaatgg 2400
catcgacctc aaagcatttc ttgatagtct accagatgtg aaaattgtca agatgaagtg 2460
tcctgatgga ggagacaatg cagatagcag taacacagct cttaatatgc ctgttattcc 2520

tatgaatact attgcagaag cagttattga aatgattaac cgaggacaga ttcaaataac 2580
aattaatgga ttcagtatta gcaatggact ggcaactact cagatcaaca ataaggctgc 2640
aactggagag gaggttcccc gtaccattat tgtaaccacc cgttctcagt acgggttacc 2700
agaagatgcc atcgtatact gtaactttaa tcagttgtat aaaattgacc cttctacttt 2760
gcagatgtgg gcaaacattc tgaagcgtgt tcccaatagt gtactctggc tgttgcgttt 2820
tccagcagta ggagaaccta atattcaaca gtatgcacaa aacatgggcc tgccccagaa 2880
ccgtatcatt ttttcacctg ttgctcctaa agaggaacac gtcaggagag gccagctggc 2940
tgatgtctgc ttggacactc cactctgtaa tgggcacacc acagggatgg atgtcctctg 3000
ggcagggacc cccatggtga ctatgccagg agagactctt gcttctcgag ttgcagcatc 3060
ccagctcact tgcttaggtt gtcttgagct tattgctaaa aacagacaag aatatgaaga 3120
catagctgtg aagctgggaa ctgatctaga atacctgaag aaagttcgtg gcaaagtctg 3180
gaagcaaaga atatctagcc ctctgttcaa caccaaacaa tacacaatgg aactagagcg 3240
gctctatcta cagatgtggg agcattatgc agctggcaac aaacctgacc acatgattaa 3300
gcctgttgaa gtcactgagt cagcataaat aaagactgca caggagaatt acccctatac 3360
ctgagcctca accttctggg ggaaagggaa ctagataaca tacttcttac ttgtctgtac 3420
agtaccttgt tgcagatggg tgatatataa tggtaataga atagcacagc cagacttgct 3480
tcctgcatgg tagggagaga cacaaaagat gggaaactgc ttttccacaa ggaatctccg 3540
tagaattttg cggcgaccag atggtgcata ggtctggaag gtctgatctc ccttggtctt 3600
ccatgggatg gttagtgtgg aggggagata tagattgtcc ggccgctttg tgattccatg 3660
gattgattca gtcttctgga tttttttttc tttatatttt gggtactgga gcttttaaaa 3720
atgtttggtt tcaggtattt ttattcatgt gaagtgtata tgattctctt gagataaggt 3780
tttaagctaa aatgttactc cctgttttag tttctgaact ctgacagatt gacagggact 3840
ttgctggtgt agtcttttta taggttttat aaaccacttg agcctatatc agtcgtttta 3900
gtgtctgacc taatatttgg agctatcagt gctttgttga tttagatgat gactcaagat 3960
tttttctggt ccatttccca tttccttttc ttccctgacc cccataccct cacccttaaa 4020
attctcctgt aactcaacta acaaaatcaa gcctgattca aaacatccta gggtgtttta 4080
aacacaccat ctggtgccaa atgaagattt ttaggagtga ttactaatta tcaagggcac 4140
agttgtggta ctgtcattga taataatata gttttttttt ttttcctaat tttgacctgt 4200
ttcaccagtg ttttaccctt gactgcccct tctatgctgc ttccaaaagt gatagtgtgt 4260
gtaagatttt taccttcctt tctaaagttt tttttttttt tttttaagtg agtcctgttc 4320
ttcctatttc tttcagcaga aatgaaatcc caggtaagta taagtattca agtatttgat 4380
cagtaagtca cagttatctc cagtgcatta aataaccttc atcaagaaat aggttatagg 4440
taaaatctct gaaggatcat ctatgtattc aagtaattat tttttagata ataactgtct 4500
tctggacttg gtcttgaagt ctgtacagat tcagcctcag tagtagcgaa ctgcactgct 4560

gtttggtttg gagtacaaat tagacttata gtcctcctgg aacttgagtt attaaaatca 4620

taggaataaa attatgggat ctcaacaaag ggtcgagggt ttgaggctta aacaagccaa 4680

catatgaata tatgttttgt ctcgctatac tgcacttacg ctatccagtt gcaggtaatt 4740

ttttgtctgc tagtagtgtt ctagattatg tctttccaaa gcgctgaggc tgtgcaccta 4800

ttctgtagtt gcagctgatg cctgaatgta tcctagctga caaattattg attaataaga 4860

acttgaattt ctggaagatt cttactgtta accaaatttt gagcaaggag tctcaaaggt 4920

aattctgaac cagaattaca tgttaatgaa cagtgtacct tttaacagtg taaatcacgg 4980

aatatccgtg aagggatttc ttaatttatt ttttaccggt tgattgaaat atcagttaaa 5040

ggttgccagc atggttgcag ataaactgat gtttgaaatt cgctgaaata cttaatgtgg 5100

aataggataa tatacttcca atgccctcaa ggctgtgacc ttacagccat tttacatagc 5160

acatcattcc tcctataggg atgaactttt tcctggcacg aaaagtagcc gctctggttg 5220

aagctttgct tattgtaaca ggcttttatt tccaggtaat atgtcttgga agacttaatt 5280

ctgattagag atatagatat tactggaaac taattgtttt ttttctattg tactctgctt 5340

tatcaaagaa gtaaaacatt taaatcgtac tacagaaatt aagatgttgt cttgcgatcc 5400

ttaataaatg aatgatttcc cttttaaaaa aaaaaaaaaa aaaaa 5445

<210> 168
<211> 1990
<212> DNA
<213> Homo sapiens

<400> 168
ggagtgccca ggatggagtg cagtggctat tcacaggttt aatcatagct cactaccgtc 60

ttgaatccct gggctcaagt gatcctcctg cctcagcccc tgagtagctg gtactaaagt 120

taccttccac gctgtatgtc cgtgagaaat acaatgaaga cggtctgaat aatgaactgg 180

taatcttcga taatatgaaa cgaatttaag agaacatatc aaagagaagg ctctcaaaat 240

aggctgctgc aaaagctggt atattgtccg gttgctctct cagaatctcg cgtgtcagcc 300

cttcaagaag attcccaaat ccttgtggaa ttcggtagtg ggtgttggag aatggaatcg 360

acatcttctt ggtattggtt ttcgctttgc ttcatacatc aataatgata tggtgctgca 420

gaaggagcct gctggggcag tgatatgggg cttcggtaca cctggagcca cagtgaccgt 480

gaccctgcgc caaggtcagg aaaccatcat gaagaaagtg accagtgtga aagctcactc 540

tgatacgtgg atggtggtac tggatcctat gaagcctgga ggacctttcg aagtgatggc 600

acaacagact ttggagaaaa taaacttcac cctgagagtt catgacgtcc tgtttggaga 660

tgtctggctc tgtagtgggc agagtaacat gcagatgact gtgttacaga tatttaatgc 720

tacaagggag ttgtctaaca ctgcggcata tcagtctgtc cgcatcctct ctgtctctcc 780

cattcaagca gagcaggagc tggaggacct tgttgcggtt gacttgcagt ggtctaagcc 840

cacctcagaa aacttaggcc atggatattt caagtacatg tcagcagtgt gctggctctt 900

tggacgtcac ctttatgaca ctctgcagta tcccatcggg ctgatcgcct ccagctgggg 960
cgggacaccc attgaagcct ggtcatctgg acggtcactg aaagcctgtg gggtccctaa 1020
acaagggtcc attccatacg attctgtaac tggtcccagt aagcactctg ttctctggaa 1080
tgccatgatc catccactgt gcaatatgac tctgaaaggg gtagtatggt accaggggga 1140
gtccaatata aattataaca cggatctgta caattgcaca ttccctgcac tcatcgaaga 1200
ctggcgtgaa accttccacc gtggttccca ggggcagacg gagcgtttct tcccatttgg 1260
acttgtccag ttatcttcag atttgtctaa gaagagctca gacgatggat ttccccagat 1320
ccgttggcat caaacagcag acttcggcta tgtccccaac ccaaagatgc ccaatacttt 1380
catggctgta gctatggatc tctgtgatag agactcgcct tttggcagca tccaccctcg 1440
agataaacag actgtggctt atcggctgca tttgggggcc cgtgctctgg cttatggtga 1500
gaagaatttg acctttgaag gaccactgcc tgagaagata gaactcttgg ctcacaaggg 1560
gctgctcaat ctcacatatt accagcaaat ccaggtgcag aaaaaggaca acaagatatt 1620
tgagatctcc tgttgcagtg accatcgatg caagtggctt ccagcttcta tgaacaccgt 1680
ctccacccag tccctgaccc tggcgatcga ttcttgtcat ggcactgtgg ttgctctccg 1740
ctatgcttgg accacgtggc cttgtgaata taagcagtgt cccctatacc accccagtag 1800
tgccctgcca gcccctccct tcattgcttt cattacagac cagggtcctg gacatcagag 1860
caatgttgct aaatgactgt ttcagtatga tcagaactta gatataagga tgggtccttc 1920
agattttagc atttaggagt ttcaataata accattgctt ttaaaggaaa gtaaaaaaaa 1980
aaaaaaaaaa 1990

<210> 169
<211> 2926
<212> DNA
<213> Homo sapiens

<400> 169
   gcgaaaacaa acagctgggg ctgcgagcgc ccccgccccg gccccgagag cacgccggcc 60
   cagtccccca cctggggcgc ccgtctgccc accatgagga agatccgcgc caatgccatc 120
   gccatcctga ccgtagcctg gatcctgggc actttctact acttatggca ggacaaccga 180
   gcccacgcag catcctccgg cggccggggc gcgcagaggg caggcaggag gtcggagcag 240
   ctccgcgagg accgcaccat cccgctcatt gtgacaggaa ctccctcgaa aggctttgat 300
   gagaaggcct acctgtcggc caagcagctg aaggctggag aggaccccta cagacagcac 360
   gccttcaacc agctggagag tgacaagctg agcccagacc ggcccatccg ggacacccgc 420
   cattacagct gcccatctgt gtcctactcc tcggacctgc cagccaccag cgtcatcatc 480
   accttccaca atgaggcccg ttccaccctg ctgcgcacag tgaagagtgt cctgaaccga 540
   actcctgcca acttgatcca ggagatcatt ttagtggatg acttcagctc agatccggaa 600
   gactgtctac tcctgaccag gatccccaag gtcaagtgcc tgcgcaatga tcggcgggaa 660
   gggctgatcc ggtcccgagt gcgtggggcg gacatggctg cagctaccgt tctcaccttt 720
   ctggatagcc actgcgaagt gaacaccgag tggctgccgc ccatgctgca gcgggtgaag 780
   gaggaccaca cccgcgtggt gagtcccatc attgatgtca tcagtctgga taattttgcc 840
   taccttgcag catctgctga ccttcgtgga gggttcgact ggagcctgca tttcaagtgg 900
   gagcagatcc ctcttgagca gaagatgacc cggacagacc ccaccaggcc cataaggacg 960
   cctgtcatag ctggaggaat cttcgtgatc gacaagtcct ggtttaacca cttgggaaag 1020
   tatgatgccc agatggacat ctggggggga gagaattttg agctctcctt cagggtgtgg 1080
   atgtgtggtg gcagtctgga gatcgtcccc tgcagccggg tgggccatgt cttcaggaaa 1140
   cggcacccct acaacttccc tgagggtaat gccctcacct acatcaggaa tactaagcgc 1200
   actgcagaag tgtggatgga tgaatacaag caatactact atgaggcccg gccctcggcc 1260
   atcgggaagg ccttcggcag tgtggctacg cggatagagc agaggaagaa gatgaactgc 1320
   aagtccttcc gctggtacct ggagaacgtc tacccagagc tcacggtccc cgtgaaggaa 1380
   gcactccccg gcatcattaa gcagggggtg aactgcttag aatctcaggg ccagaacaca 1440
   gctggtgact tcctgcttgg aatggggatc tgcagagggt ctgccaagaa cccgcagccc 1500
   gcccaggcat ggctgttcag tgaccacctc atccagcagc aggggaagtg cctggctgcc 1560
   acctccacct taatgtcctc ccctggatcc ccagtcatac tgcagatgtg caaccctaga 1620
   gaaggcaagc agaaatggag gagaaaagga tctttcatcc agcattcagt cagtggcctc 1680
   tgcctggaga caaagcctgc ccagctggtg accagcaagt gtcaggctga cgcccaggcc 1740
   cagcagtggc agctgttgcc acacacatga cggagggatt ttcttgaagg gtcagctatt 1800
   gcactgtgct gggaggctgg ctgtggctcc tgtttgtgaa gagaactccc agttcctttt 1860
   cacagccgct gagaacacat ccacacatct accccacggc cttgtcttgg aactgctcct 1920
   gctatcccca caccctgctt cctcacccca ctggctcttg ggcaaatgac tgttggtacc 1980
   aggaccctgg gggtctcccc tacaaagcag accagcctgg ggcagatata cctaccacag 2040
   gagccccctg tctttcatag gccaaggatc cacataccca tagagttcat ggattctaga 2100
   gggtctagga atctcttgaa attgtcccta aaatactgca tgtgtgtgca tacatgcatt 2160
   ttcctgggga aaaagtccat ggcttctaaa agaggtccct gatccccaaa gggcctgaac 2220
   ctctgctctg gattgaagcc actgtctgcc caagctgcca ccccctaatc ttcctccctg 2280
   gcgtgctcaa actgccatcg cctgctccct ccacacggcc cttggggtca gcagccaagt 2340
   gtctgtggtc cgcagcacct gctctggagg gctctccagc agtttcgcct cctgactctc 2400
   accagcgtcc tctcggcagc actcccgtgc ccaagtgcac ccctctggac ttgccagcgc 2460
   aggccccttc gctcccaggg ccatgctttg cctgtcctct gtcgtgatgt ttcttccgca 2520
   gccaggtgca gcctcagatc ccctgttcat ctgggaagcc tcctgccaca gcttaggaca 2580
   gaactgggcc cagggcaaag gcctctcccc agaggatgga ctagaaggcc tgggccacac 2640
   tcgggcatgg acctttgggg ctggggagcc ggggctgcgc ctgttgaatg taagaggact 2700
   gctgaccaga gggccttcaa gagggtctct ctgtcctttg ctgtggtcag atcaggctct 2760
   gcacttatca gccggtcctt tgtggcaacg cagccctgtt ctgtttttgc ttttcctctt 2820
   cttgaccaaa gcatgtgcca ctagctgtcc ttgaggacct cgtctttatg aaacacacac 2880
   ctggaataaa accacttctt acatgtcaaa aaaaaaaaaa aaaaaa 2926

<210> 170
<211> 6736
<212> DNA
<213> Homo sapiens

<400> 170
atgggcttct tgcccaagct tctcctcctg gcctcattct tcccagcagg ccaggcctca 60

tggggcgtct ccagtcccca ggacgtgcag ggtgtgaagg ggtcttgcct gcttatcccc 120

tgcatcttca gcttccctgc cgacgtggag gtgcccgacg gcatcacggc catctggtac 180

tacgactact cgggccagcg gcaggtggtg agccactcgg cggaccccaa gctggtggag 240

gcccgcttcc gcggccgcac cgagttcatg gggaaccccg agcacagggt gtgcaacctg 300

ctgctgaagg acctgcagcc cgaggactct ggttcctaca acttccgctt cgagatcagt 360

gaggtcaacc gctggtcaga tgtgaaaggc accttggtca cagtaacaga ggagcccagg 420

gtgcccacca ttgcctcccc ggtggagctt ctcgagggca cagaggtgga cttcaactgc 480

tccactccct acgtatgcct gcaggagcag gtcagactgc agtggcaagg ccaggaccct 540

gctcgctctg tcaccttcaa cagccagaag tttgagccca ccggcgtcgg ccacctggag 600

accctccaca tggccatgtc ctggcaggac cacggccgga tcctgcgctg ccagctctcc 660

gtggccaatc acagggctca gagcgagatt cacctccaag tgaagtatgc ccccaagggt 720

gtgaagatcc tcctcagccc ctcggggagg aacatccttc caggtgagct ggtcacactc 780

acctgccagg tgaacagcag ctaccctgca gtcagttcca ttaagtggct caaggatggg 840

gtacgcctcc aaaccaagac tggtgtgctg cacctgcccc aggcagcctg gagcgatgct 900

ggcgtctaca cctgccaagc tgagaacggc gtgggctctt tggtctcacc ccccatcagc 960

ctccacatct tcatggctga ggtccaggtg agcccagcag gtcccatcct ggagaaccag 1020

acagtgacac tagtctgcaa cacacccaat gaggcaccca gtgatctccg ctacagctgg 1080

tacaagaacc atgtcctgct ggaggatgcc cactcccata ccctccggct gcacttggcc 1140

actagggctg atactggctt ctacttctgt gaggtgcaga acgtccatgg cagcgagcgc 1200

tcgggccctg tcagcgtggt agtcaaccac ccgcctctca ctccagtcct gacagccttc 1260

ctggagaccc aggcgggact tgtgggcatc cttcactgct ctgtggtcag tgagcccctg 1320

gccacactgg tgctgtcaca tgggggtcat atcctggcct ccacctccgg ggacagtgat 1380

cacagcccac gcttcagtgg tacctctggt cccaactccc tgcgcctgga gatccgagac 1440

ctggaggaaa ctgacagtgg ggagtacaag tgctcagcca ccaactccct tggaaatgca 1500

acctccaccc tggacttcca tgccaatgcc gcccgtctcc tcatcagccc ggcagccgag 1560

gtggtggaag gacaggcagt gacactgagc tgcagaagcg gcctaagccc cacacctgat 1620

gcccgcttct cctggtacct gaatggagcc ctgcttcacg agggtcccgg cagcagcctc 1680
ctgctccccg cggcctccag cactgacgcc ggctcatacc actgccgggc ccgggacggc 1740
cacagtgcca gtggcccctc ttcgccagct gttctcactg tgctctaccc ccctcgacaa 1800
ccaacattca ccaccaggct ggaccttgat gccgctgggg ccggggctgg acggcgaggc 1860
ctccttttgt gccgtgtgga cagcgacccc cccgccaggc tgcagctgct ccacaaggac 1920
cgtgttgtgg ccacttccct gccatcaggg ggtggctgca gcacctgtgg gggctgttcc 1980
ccacgcatga aggtcaccaa agcccccaac ttgctgcgtg tggagattca caaccctttg 2040
ctggaagagg agggcttgta cctctgtgag gccagcaatg ccctgggcaa cgcctccacc 2100
tcagccacct tcaatggcca ggccactgtc ctggccattg caccatcaca cacacttcag 2160
gagggcacag aagccaactt gacttgcaac gtgagccggg aagctgctgg cagccctgct 2220
aacttctcct ggttccgaaa tggggtgctg tgggcccagg gtcccctgga gaccgtgaca 2280
ctgctgcccg tggccagaac tgatgctgcc ctttacgcct gccgcatcct gactgaggct 2340
ggtgcccagc tctccactcc cgtgctcctg agtgtactct atcccccgga ccgtccaaag 2400
ctgtcagccc tcctagacat gggccagggc cacatggctc tgttcatctg cactgtggac 2460
agccgccccc tggccttgct ggccttgttc catggggagc acctcctggc caccagcctg 2520
ggtccccagg tcccatccca tggtcggttc caggctaaag ctgaggccaa ctccctgaag 2580
ttagaggtcc gagaactggg ccttggggac tctggcagct accgctgtga ggccacaaat 2640
gttcttggat catccaacac ctcactcttc ttccaggtcc gaggagcctg ggtccaggtg 2700
tcaccatcac ctgagctcca agagggccag gctgtggtcc tgagctgcca ggtacacaca 2760
ggagtcccag aggggacctc atatcgttgg tatcgggatg gccagcccct ccaggagtcg 2820
acctcggcca cgctccgctt tgcagccata actttgacac aagctggggc ctatcattgc 2880
caagcccagg ccccaggctc agccaccacg agcctagctg cacccatcag cctccacgtg 2940
tcctatgccc cacgccacgt cacactcact accctgatgg acacaggccc tggacgactg 3000
ggcctcctcc tgtgccgtgt ggacagtgac cctccggccc agctgcggct gctccacggg 3060
gatcgccttg tggcctccac cctacaaggt gtggggggac ccgaaggcag ctctcccagg 3120
ctgcatgtgg ctgtggcccc caacacactg cgtctggaga tccacggggc tatgctggag 3180
gatgagggtg tctatatctg tgaggcctcc aacaccctgg gccaggcctc ggcctcagct 3240
gacttcgacg ctcaagctgt gaatgtgcag gtgtggcccg gggctaccgt gcgggagggg 3300
cagctggtga acctgacctg ccttgtgtgg accactcacc cggcccagct cacctacaca 3360
tggtaccagg atgggcagca gcgcctggat gcccactcca tccccctgcc caacgtcaca 3420
gtcagggatg ccacctccta ccgctgcggt gtgggccccc ctggtcgggc accccgcctc 3480
tccagaccta tcaccttgga cgtcctctac gcgccccgca acctgcgcct gacctacctc 3540
ctggagagcc atggcgggca gctggccctg gtactgtgca ctgtggacag ccgcccgccc 3600
gcccagctgg ccctcagcca cgccggtcgc ctcttggcct cctcgacagc agcctctgtc 3660

cccaacaccc tgcgcctgga gctgcgaggg ccacagccca gggatgaggg tttctacagc 3720
tgctctgccc gcagccctct gggccaggcc aacacgtccc tggagctgcg gctggagggt 3780
gtgcgggtga tcctggctcc ggaggctgcc gtgcctgaag gtgcccccat cacagtgacc 3840
tgtgcggacc ctgctgccca cgcacccaca ctctatactt ggtaccacaa cggtcgttgg 3900
ctgcaggagg gtccagctgc ctcactctca ttcctggtgg ccacgcgggc tcatgcaggc 3960
gcctactctt gccaggccca ggatgcccag ggcacccgca gctcccgtcc tgctgccctg 4020
caagtcctct atgcccctca ggacgctgtc ctgtcctcct tccgggactc cagggccaga 4080
tccatggctg tgatacagtg cactgtggac agtgagccac ctgctgagct ggccctatct 4140
catgatggca aggtgctggc cacgagcagc ggggtccaca gcttggcatc agggacaggc 4200
catgtccagg tggcccgaaa cgccctacgg ctgcaggtgc aagatgtgcc tgcaggtgat 4260
gacacctatg tttgcacagc ccaaaacttg ctgggctcaa tcagcaccat cgggcggttg 4320
caggtagaag gtgcacgcgt ggtggcagag cctggcctgg acgtgcctga gggcgctgcc 4380
ctgaacctca gctgccgcct cctgggtggc cctgggcctg tgggcaactc cacctttgca 4440
tggttctgga atgaccggcg gctgcacgcg gagcctgtgc ccactctcgc cttcacccac 4500
gtggctcgtg ctcaagctgg gatgtaccac tgcctggctg agctccccac tggggctgct 4560
gcctctgctc cagtcatgct ccgtgtgctc taccctccca agacgcccac catgatggtc 4620
ttcgtggagc ctgagggtgg cctccggggc atcctggatt gccgagtgga cagcgagccg 4680
ctcgccagcc tgactctcca ccttggcagt cgactggtgg cctccagtca gccccagggt 4740
gctcctgcag agccacacat ccatgtcctg gcttccccca atgccctgag ggtggacatc 4800
gaggcgctga ggcccagcga ccaaggggaa tacatctgtt ctgcctcaaa tgtcctgggc 4860
tctgcctcta cctccaccta ctttggggtc agagccctgc accgcctgca tcagttccag 4920
cagctgctct gggtcctggg actgctggtg ggcctcctgc tcctgctgtt gggcctgggg 4980
gcctgctaca cctggagaag gaggcgtgtt tgtaagcaga gcatgggcga gaattcggtg 5040
gagatggctt ttcagaaaga gaccacgcag ctcattgatc ctgatgcagc cacatgtgag 5100
acctcaacct gtgccccacc cctgggctga ccagtggtgt tgcctgccct ccggaggaga 5160
aagtggccag aatctgtgat gactccagcc tatgaatgtg aatgaggcag tgttgagtcc 5220
tgcccgcctc tacgaaaaca gctctgtgac atctgacttt ttatgacctg gccccaagcc 5280
tcttgccccc ccaaaaatgg gtggtgagag gtctgcccag gagggtgttg accctggagg 5340
acactgaaga gcactgagct gatctcgctc tctcttctct ggatctcctc ccttctctcc 5400
atttctccct caaaggaagc cctgcccttt cacatccttc tcctcgaaag tcaccctgga 5460
ctttggttgg attgcagcat cctgcatcct cagaggctca ccaaggcatt ctgtattcaa 5520
cagagtatca gtcagcctgc tctaacaaga gaccaaatac agtgacttca acatgataga 5580
attttatttt tctctcccac gctagtctgg ctgttacgat ggtttatgat gttggggctc 5640
aggatccttc tatcttcctt ttctctatcc ctaaaatgat gcctttgatt gtgaggctca 5700

ccatggcccc gctttgtcca catgccctcc agccagaaga aggaagagtg gaggtagaag 5760
cacacccatg cccatggtgg acgcaactca gaagctgcac aggacttttc cactcacttc 5820
ccattggctg gagtattgtc acatggctac tgcaagctac aagggagact gggaaatgta 5880
gtttttattt tgagtccaga ggacatttgg aattggactt ccaaaggact cccaactgtg 5940
agctcatccc tgagactttt gacattgttg ggaatgccac cagcaggcca tgttttgtct 6000
cagtgcccat ctactgaggg ccagggtgtg cccctggcca ttctggttgt gggcttcctg 6060
gaagaggtga tcactctcac actaagactg aggaaataaa aaaggtttgg tgttttccta 6120
gggagagagc atgccaggca gtggagttgc ctaagcagac atccttgtgc cagatttggc 6180
ccctgaaaga agagatgccc tcattcccac caccaccccc cctaccccca gggactgggt 6240
actaccttac tggcccttac aagagtggag ggcagacaca gatgttgtca gcatccttat 6300
tcctgctcca gatgcatctc tgttcatgac tgtgtgagct cctgtccttt tcctggagac 6360
cctgtgtcgg gctgttaaag agaatgagtt accaagaagg aatgacgtgc ccctgcgaat 6420
cagggaccaa caggagagag ctcttgagtg ggctagtgac tccccctgca gcctggtgga 6480
gatggtgtga ggagcgaaga gccctctgct ctaggatttg ggttgaaaaa cagagagaga 6540
agtggggagt tgccacagga gctaacacgc tgggaggcag ttgggggcgg gtgaactttg 6600
tgtagccgag gccgcaccct ccctcattcc aggctcattc attttcatgc tccattgcca 6660
gactcttgct gggagcccgt ccagaatgtc ctcccaataa aactccatcc tatgacgcaa 6720
aaaaaaaaaa aaaaaa 6736

<210> 171
<211> 2327
<212> DNA
<213> Homo sapiens

<400> 171
   agcccagcga ggcctgtgct gcgaggggct gtgctgtgcc aggggaggcc ccgcacccgg 60
   gaagcccaga ggagagtgtg cactcacgga gccctcctgg cagggccgcg tctctggagc 120
   agcagacccg tgtccctctg ggaggtcaac tgcggcctca cagctgggcc tgtcccagca 180
   cagcccttgt tggagggcgc acccccgtgt cctcagcacc ccagtgttga gtgcattgac 240
   ctgcagaggg gagcctgtgc tggtcccggg cgtctgtgtg gctcctgggt gcccatctgc 300
   acccctggcc ctgctctggg cttcagtgga gccgtgccac cgtgggagtg tccagggtcc 360
   tgacgtagcc ctgagacgtg gccagctccc tggcctgctg cccgcacagt gggcccttgt 420
   ctctgctctg ggtcaggcga ggcagccacc catgatgagc tctgcagcct tcccaaggtg 480
   gctgagcatg ggggtccctc gtaccccttc acggacagtg ctcttcgagc gggagaggac 540
   gggcctgacc taccgcgtgc cctcgctgct ccccgtgccc cccgggccca ccctgctggc 600
   ctttgtggag cagcggctca gccctgacga ctcccacgcc caccgcctgg tgctgaggag 660
   gggcacgctg gccgggggct ccgtgcggtg gggtgccctg cacgtgctgg ggacagcagc 720
   cctggcggag caccggtcca tgaacccctg ccctgtgcac gatgctggca cgggcaccgt 780
   cttcctcttc ttcatcgcgg tgctgggcca cacgcctgag gccgtgcaga tcgccacggg 840
   aaggaacgcc gcgcgcctct gctgtgtggc cagccgtgac gccggcctct cgtggggcag 900
   cgcccgggac ctcaccgagg aggccatcgg tggtgccgtg caggactggg ccacattcgc 960
   tgtgggtccc ggccacggcg tgcagctgcc ctcaggccgc ctgctggtac ccgcctacac 1020
   ctaccgcgtg gaccgccgag agtgttttgg caagatctgc cggaccagcc ctcactcctt 1080
   cgccttctac agcgatgacc acggccgcac ctggcgctgt ggaggcctcg tgcccaacct 1140
   gcgctcaggc gagtgccagc tggcggcggt ggacggtggg caggccggca gcttcctcta 1200
   ctgcaatgcc cggagcccac tgggcagccg tgtgcaggcg ctcagcactg acgagggcac 1260
   ctccttcctg cccgcagagc gcgtggcttc cctgcccgag actgcctggg gctgccaggg 1320
   cagcatcgtg ggcttcccag cccccgcccc caacaggcca cgggatgaca gttggtcagt 1380
   gggccccggg agtcccctcc agcctccact cctcggtcct ggagtccacg aacccccaga 1440
   ggaggctgct gtagaccccc gtggaggcca ggtgcctggt gggcccttca gccgtctgca 1500
   gcctcggggg gatggcccca ggcagcctgg ccccaggcct ggggtcagtg gggatgtggg 1560
   gtcctggacc ctggcactcc ccatgccctt tgctgccccg ccccagagcc ccacgtggct 1620
   gctgtactcc cacccagtgg ggcgcagggc tcggctacac atgggtatcc gcctgagcca 1680
   gtccccgctg gacccgcgca gctggacaga gccctgggtg atctacgagg gccccagcgg 1740
   ctactccgac ctggcgtcca tcgggccggc tcctgagggg ggcctggttt ttgcctgcct 1800
   gtacgagagc ggggccagga cctcctatga tgagatttcc ttttgtacat tctccctgcg 1860
   tgaggtcctg gagaacgtgc ccgccagccc caagccgccc aaccttgggg acaagcctcg 1920
   ggggtgctgc tggccctcct gacaggcctt ctggccgtgc ccatgcccct tgggtgcctg 1980
   gggcagaggg gtggaatacg ttggggtgcc ccacgatagc tgtggggggg gctcttagtg 2040
   caagatcctg tggattagaa acaagttgct cctcagagct ctcaagcagg gactgctctt 2100
   taggaagggg agcagcggct gggagtgagc agggcagggt gggggcaggg tgggggcacg 2160
   aagtgggccc tgggtgaccc ccacagctcc cttccgaggc tgcagggcca ggcgcgggac 2220
   cgcaggtagc ccagggtgtt gtgggtggca gcacttgttt actggctgct ttctggctcg 2280
   aaataaagga atcgtgcttg tgaaaaaaaa aaaaaaaaaa aaaaaaa 2327

<210> 172
<211> 641
<212> DNA
<213> Homo sapiens

<400> 172
atttaaattc tgcagctcag agattcacac agaagtctgg acacaattca gaagagccac 60
ccagaaggag acaacaatgt ccctgctacc cgtgccatac acagaggctg cctctttgtc 120
tactggttct actgtgacaa tcaaagggcg accacttgcc tgtttcttga atgaaccata 180
tctgcaggtg gatttccaca ctgagatgaa ggaggaatca gacattgtct tccatttcca 240

agtgtgcttt ggtcgtcgtg tggtcatgaa cagccgtgag tatggggcct ggaagcagca 300

ggtggaatcc aagaatatgc cctttcagga tggccaagaa tttgaactga gcatctcagt 360

gctgccagat aagtaccagg taatggtcaa tggccaatcc tcttacacct ttgaccatag 420

aatcaagcct gaggctgtga agatggtgca agtgtggaga gatatctccc tgaccaaatt 480

taatgtcagc tatttaaaga gataaccaga cttcatgttg ccaaggaatc cctgtctcta 540

cgtgaacttg ggattccaaa gccagctaac agcatgatct tttctcactt caatccttac 600

tcctgctcat taaaacttaa tcaaacttca aaaaaaaaaa a 641

<210> 173
<211> 1696
<212> DNA
<213> Homo sapiens

<400> 173
actgtgtggc cccgcaaccc agcacaacca ggtatctgct tggaacccag ccaccataaa 60

gcctgctagc taaaaaaaat tttacatctc tcagttcatt cggcacagac ccctgcctca 120

ttcagctgtg actctgcttg gaaaattcat cagttacaaa gcagccaatg caattatctc 180

aagggaaatt gaaaaatgga cctttgaaaa tgctagattt acaatgagaa atgccataat 240

tcaaggttta ttctatgggt ccttgacatt tgggatctgg acagctctgt tattcatata 300

tttgcaccat aatcatgtga gcagctggca gaagaaaagc caggagcctc tgtcagcttg 360

gtcccctgga aaaaaagtgc atcagcaaat tatctatggc tcagagcaaa taccaaaacc 420

tcatgtaata gtcaaaagga ctgatgaaga taaagcaaag tctatgttag gtacagattt 480

taaccataca aacccagaac ttcataaaga acttttaaaa tatggattta atgtgattat 540

cagtagaagc ttgggcatcg aaagagaagt gccagatacc aggagtaaaa tgcgtcttca 600

aaaacattac ccagcccgcc tcccgactgc cagcattgtc atttgcttct ataatgaaga 660

atgtaatgcc ttgtttcaga ccatgtccag tgtcacgaac ctcacgccac actattttct 720

tgaagaaatt attttggtag atgacatgag caaagttgat gatttgaaag aaaaactaga 780

ctatcacctg gaaacttttc ggggaaaggt taaaataata agaaacaaaa agagagaggg 840

gctgattcga gcaaggctga ttggagcttc tcatgcttca ggggatgttc tggtgttcct 900

ggacagccac tgtgaggtga acagagtatg gctggagccc ctgctgcatg ccattgccaa 960

ggaccccaaa atggtggtgt gccccctgat agatgtcatt gatgatagaa ctctggagta 1020

taagccctct cctcttgtaa ggggaacttt tgattggaac ctacaattta aatgggataa 1080

tgttttctct tatgagatgg atggaccaga aggatctact aaaccaatcc ggtcacctgc 1140

aatgtctgga ggaatttttg ctatacgtcg gcattatttt aatgaaattg gacagtatga 1200

caaggatatg gatttttggg gaagagaaaa tttggaactt tcactaagga tctggatgtg 1260

tggaggccaa ctctttataa tcccctgctc tcgagtagga catatcagta agaaacaaac 1320

tggaaaacct tctacaatca tcagtgctat gacacataac tacctaagac tggtgcacgt 1380

ttggctggat gaatataagg agcagttttt tcttcgaaag cctggtctga aatatgtcac 1440

ctacggaaat attcgcgagc gtgttgagtt aaggaaacga ctgggttgca agtcatttca 1500
gtggtatttg gataatgtct tcccagagtt ggaggcatct gtgaacagcc tgtgaaagga 1560
aaacaaatca ctttcattaa taaagggtta aaagtctcct agtcattcaa catagtgtca 1620
caagagtgta agtttggaac atcgtggaat tacgtgaaat gcaattaaaa aaatatgacc 1680
aaaaaaaaaa aaaaaa 1696

<210> 174
<211> 3298
<212> DNA
<213> Homo sapiens

<400> 174
atggcttcac tgagaagagt caaagtgctg ttggtgttga acttgatcgc ggtagccggc 60
ttcgtgctct tcctggccaa gtgccggccc atcgcggtgc gcagcggaga cgccttccac 120
gagatccggc cgcgcgccga ggtggccaac ctcagcgcgc acagcgccag ccccatccag 180
gatgcggtcc tgaagcgcct gtcgctgctg gaggacatcg tgtaccggca gctgaatggc 240
ttatccaaat cccttgggct cattgaaggt tatggtgggc ggggtaaagg gggccttccg 300
gctactcttt ccccggctga agaagaaaag gctaagggac cccatgagaa gtatggctac 360
aattcatacc tcagtgaaaa aatttcactg gaccgttcca ttccggatta tcgtcccacc 420
aagtgtaagg agctcaagta ctccaaggac ctgccccaga tatccatcat attcatcttc 480
gtgaacgagg ccctgtcggt gatcctgcgg tccgtgcaca gtgccgtcaa tcacacgccc 540
acacacctgc tgaaggaaat cattctggtg gatgacaaca gcgacgaaga ggagctgaag 600
gtccccctag aggagtatgt ccacaaacgc taccccgggc tggtgaaggt ggtaagaaat 660
cagaagaggg aaggcctgat ccgcgctcgc attgagggct ggaaggtggc taccgggcag 720
gtcactggct tctttgatgc ccacgtggaa ttcaccgctg gctgggctga gccggttcta 780
tcccgcatcc aggaaaaccg gaagcgtgtg atcctcccct ccattgacaa catcaaacag 840
gacaactttg aggtgcagcg gtacgagaac tcggcccacg ggtacagctg ggagctgtgg 900
tgcatgtaca tcagcccccc aaaagactgg tgggacgccg gagacccttc tctccccatc 960
aggaccccag ccatgatagg ctgctcgttc gtggtcaaca ggaagttctt cggtgaaatt 1020
ggtcttctgg atcctggcat ggatgtatac ggaggagaaa atattgaact gggaatcaag 1080
gtatggctct gtgggggcag catggaggtc cttccttgct cacgggtggc ccacattgag 1140
cggaagaaga agccatataa tagcaacatt ggcttctaca ccaagaggaa tgctcttcgc 1200
gttgctgagg tctggatgga cgattacaag tctcatgtgt acatagcgtg gaacctgccg 1260
ctggagaatc cgggaattga catcggtgat gtctccgaaa gaagagcatt aaggaaaagt 1320
ttaaagtgta agaatttcca gtggtacctg gaccatgttt acccagaaat gagaagatac 1380
aataataccg ttgcttacgg ggagcttcgc aacaacaagg caaaagacgt ctgcttggac 1440
caggggccgc tggagaacca cacagcaata ttgtatccgt gccatggctg gggaccacag 1500

cttgcccgct acaccaagga aggcttcctg cacttgggtg ccctggggac caccacactc 1560
ctccctgaca cccgctgcct ggtggacaac tccaagagtc ggctgcccca gctcctggac 1620
tgcgacaagg tcaagagcag cctgtacaag cgctggaact tcatccagaa tggagccatc 1680
atgaacaagg gcacgggacg ctgcctggag gtggagaacc ggggcctggc tggcatcgac 1740
ctcatcctcc gcagctgcac aggtcagagg tggaccatta agaactccat caagtagagg 1800
gagggagctg gggcactgga gcctggcccc caggacatgg ctgctccccc caacatctgg 1860
accagctgcc ctggcggaga gacagcaagg ggccggcagg tgctcgatgg gccccccagg 1920
gcttctccag ggcagcacag ggaccccgga tgaagactct gtcccccctc aggcattcag 1980
ctgcccacaa gtttcctgca ccctggaaaa gccccccacc cttcctctgg gaaactgaca 2040
gctgtcttcc acagcctctg atgtggacct ggtactgagg agcaagactg tccagttctc 2100
ctccacatct cccatcccag aatcaggatc tgggactggc agggtcccct cctgtgtctc 2160
atctcttgca gcagcagctg ctgaactcca gccatcaaca cggtgggagg cagcgggggc 2220
ttcagccatg tcctagctcc ccgccctaaa aggaggcagt gaggaccagg cactatttcc 2280
tccgaggtta cttctaccca gatgacacct gcctgttcac gccccaaggc agctactgcc 2340
cctaaccctt cccaccaggg tagctttggg cactgcagct ctggactttt ctggcccctc 2400
ctgagatgac ctgatggagc tgatgctttc tctcctaatc cctgggcact aggctcttat 2460
cagtgtgctt gggccagctc tcctgcctgt gtctagagga agccagagac agaaataggc 2520
taagcctgca gtaggatctc agccacaagg gccccgcagg atggagctgg gtcaaggacc 2580
agggagccct gactcccaga ggctgccacc ggggagaagc agcggtcctc catccagaac 2640
ctaagggctg aagcaaaggc tgccaggacc cttgaagatg cttttggctc acctcatttc 2700
accccacgct ctgctggctg gcagaggaga aggcagtcgt ttcctctctg aagagtattt 2760
ttttcgattg ccctctggtt agggtgcaca tataaatcag agttaatata tgaacgcgtg 2820
tgcatgcaca agtgtgtgtg tgcctgcgtg ctgtgcgtgg cagggtgtgt gtgtgtgtgt 2880
ctggctgtgc gttccggagt gtgtgacgat gctgacctag ctgtgtggcc ttgggcttgc 2940
tgcttcatta ctcacctgga tggggacgag ggatgagaag ggtgtgggtt tggccccatg 3000
tcactggccg gaaggatgtg tctcagccct gccctgtggg gtgcccccga tgggaggctg 3060
tcccatctcc cagtccccat ctctttttcc ccacactgtc cctggccaag ccctgcccag 3120
agctgaaccc tgtagctgcc cccttgccct gtgtgggatt cgcagtgtct catttggtga 3180
cgtcttactg gtgatcatct cctcacccca tctcccacct tgtggaataa atacatgtta 3240
gcacttccca aaaaaaaaaa aaaaaaaaaa aaaaaaaaga aaaaaaaaaa aaaaaaa 3298

<210> 175
<211> 3131
<212> DNA
<213> Homo sapiens

<400> 175
agtgtttatc agaacttagc cagggccagc caagcaggca cagatgctct gctatgaaat 60

gccacgcagg cagagactga caagcggtag gaactgagct ttccccttgg actgctgctt 120
cctgctgtgt tcaggggagg gggtcacttt ctggcaactc tgctgctgct gctgctgctg 180
ctgctacttc agcttcctct ccactcaagg taagcaggct aagggagggc aggctgctag 240
ggaaagcttt gtaccatgaa caggatccga aagtttttcc gaggaagtgg gcgagtcttg 300
gcatttatct ttgtagcttc tgtcatctgg ctcctctttg acatggcagc tctccgcctc 360
tcattcagtg agatcaacac tcgggtcatc aaggaagaca ttgtgaggag ggagcggata 420
ggattcagag ttcagccaga ccaaggaaaa attttttaca gcagcataaa agagatgaaa 480
cctcccctaa ggggacatgg gaaaggggca tggggcaaag agaatgttag aaaaactgag 540
gagagtgtgc tcaaggttga ggtggacttg gaccaaaccc agagggaaag aaaaatgcag 600
aatgccctgg gaaggggcaa ggttgtgccg ttgtggcatc ctgcacatct gcagaccctc 660
cctgtgactc ctaacaagca gaagacagac gggagaggca ccaaacctga agcctcctct 720
caccagggga caccaaagca aacgacagct cagggggctc caaagacctc attcatagca 780
gcaaaaggaa ctcaggtagt caaaatatca gtacacatgg gacgtgtcag tttaaaacag 840
gagccccgga agagtcatag tcccagcagt gacacatcaa aactagcagc tgaaagggac 900
ttgaatgtga ccatcagtct tagtactgat agaccaaagc agcgatcaca ggcagtagca 960
aacgagaggg cacaccctgc cagcacagca gtgccgaagt ctggggaagc catggcctta 1020
aacaaaacta agactcagag caaagaagtc aatgcaaata aacacaaagc caatacgagt 1080
cttccttttc ctaagttcac tgtcaattca aatcgcttaa ggaagcaatc tattaatgag 1140
acacctttgg gaagtttgtc aaaggatgat ggagctagag gggctcatgg gaagaaactc 1200
aatttctctg aaagccatct tgtgattata accaaagagg aagagcaaaa ggcagacccc 1260
aaagaggtct ctaattctaa aaccaaaaca atatttccta aagtattggg taaaagccaa 1320
agtaaacaca tttccaggaa tagaagtgag atgtcttcct cttcacttgc tccacataga 1380
gtgccactgt cccaaactaa ccatgcttta actggagggc tagagccagc aaaaatcaac 1440
ataactgcca aagccccctc tacagaatac aaccagagtc atataaaagc ccttttacct 1500
gaagacagtg gaacgcacca ggtgttaaga attgatgtga cactttctcc aagggacccc 1560
aaagctccag ggcagtttgg gcgtcctgta gttgtccccc atggaaagga gaaggaggca 1620
gaaagaagat ggaaagaagg aaacttcaat gtctacctta gcgatttgat cccagtggat 1680
agagccattg aagacaccag acctgctgga tgtgcagagc agctagttca caataacctc 1740
ccaaccacca gtgtcatcat gtgctttgtg gatgaagtgt ggtccactct cctgagatct 1800
gttcacagtg tcatcaatcg ctctcctcca cacctcatca aggagattct gctggtagat 1860
gacttcagca ccaaagacta tctaaaagat aatttggata aatacatgtc ccagtttcca 1920
aaagttcgga ttcttcgcct caaagagaga catggcttaa taagggccag gctggcagga 1980
gcacagaatg caacaggtga tgtgttgaca tttttagatt ctcatgtgga atgtaacgtt 2040
ggttggttgg aacctcttct ggaaagagtt tatttaagta gaaagaaagt ggcctgtcca 2100

gtaatcgaag tcatcaatga taaggatatg agttacatga cagtggataa ctttcaaaga 2160

ggcatctttg tgtggcccat gaactttggt tggagaacaa ttcctccaga tgtcattgca 2220

aaaaacagaa ttaaagaaac tgatacaata aggtgccctg tcatggctgg tggattgttt 2280

tctattgaca aaagttactt ttttgaactt ggaacatacg accctggcct tgatgtttgg 2340

ggtggggaaa atatggagct ctcattcaag gtgtggatgt gtggtggtga aattgagatc 2400

attccctgct cccgagtggg ccatatattc agaaatgaca atccatattc cttccccaaa 2460

gaccggatga agacagtgga gcggaacttg gtgcgggttg ccgaggtctg gctggatgag 2520

tataaggagc tgttctatgg ccacggagac cacctcatcg accaagggct agatgttggc 2580

aacctcaccc agcaaaggga gctgcgaaag aaactgaagt gcaaaagttt caaatggtac 2640

ttggagaatg tctttcctga cttaagggct cccattgtga gagctagtgg tgtgcttatt 2700

aatgtggctt tgggtaaatg catttccatt gaaaacacta cagtcattct ggaagactgc 2760

gatgggagca aagagcttca acaatttaat tacacctggt taagacttat taaatgtgga 2820

gaatggtgta tagcccccat ccctgataaa ggagccgtaa ggctgcaccc ttgtgataac 2880

agaaacaaag ggctaaaatg gctgcataaa tcaacatcag tctttcatcc agaactggtg 2940

aatcacattg tttttgaaaa caatcagcaa ttattatgct tggaaggaaa tttttctcaa 3000

aagatcctga aagtagctgc ctgtgaccca gtgaagccat atcaaaagtg gaaatttgaa 3060

aaatattatg aagcctgaag tgtaactgat gtttttatat agtaaaccca ttaaatactg 3120

tgaaaataac a 3131

<210> 176
<211> 5249
<212> DNA
<213> Homo sapiens

<400> 176
cttccctccc cgactgtgcg ccgcggctgg ctcgggttcc cgggccgaca tgggcgccgc 60

cgcgtgggca cggccgctga gcgtgtcttt cctgctgctg cttctgccgc tcccggggat 120

gcctgcgggc tcctgggacc cggccggtta cctgctctac tgcccctgca tggggcgctt 180

tgggaaccag gccgatcact tcttgggctc tctggcattt gcaaagctgc taaaccgtac 240

cttggctgtc cctccttgga ttgagtacca gcatcacaag cctcctttca ccaacctcca 300

tgtgtcctac cagaagtact tcaagctgga gcccctccag gcttaccatc gggtcatcag 360

cttggaggat ttcatggaga agctggcacc cacccactgg ccccctgaga agcgggtggc 420

atactgcttt gaggtggcag cccagcgaag cccagataag aagacgtgcc ccatgaagga 480

aggaaacccc tttggcccat tctgggatca gtttcatgtg agtttcaaca agtcggagct 540

ttttacaggc atttccttca gtgcttccta cagagaacaa tggagccaga gattttctcc 600

aaaggaacat ccggtgcttg ccctgccagg agccccagcc cagttccccg tcctagagga 660

acacaggcca ctacagaagt acatggtatg gtcagacgaa atggtgaaga cgggagaggc 720

ccagattcat gcccaccttg tccggcccta tgtgggcatt catctgcgca ttggctctga 780
ctggaagaac gcctgtgcca tgctgaagga cgggactgca ggctcgcact tcatggcctc 840
tccgcagtgt gtgggctaca gccgcagcac agcggccccc ctcacgatga ctatgtgcct 900
gcctgacctg aaggagatcc agagggctgt gaagctctgg gtgaggtcgc tggatgccca 960
gtcggtctac gttgctactg attccgagag ttatgtgcct gagctccaac agctcttcaa 1020
agggaaggtg aaggtggtga gcctgaagcc tgaggtggcc caggtcgacc tgtacatcct 1080
cggccaagcc gaccacttta ttggcaactg tgtctcctcc ttcactgcct ttgtgaagcg 1140
ggagcgggac ctccagggga ggccgtcttc tttcttcggc atggacaggc cccctaagct 1200
gcgggacgag ttctgattct ggccggagca ccagaccctc tgatcctgga gggaccagag 1260
tctgagctgg tccttccagc caggcctggc agccagaggt gctccgggat tgcaaactcc 1320
tcttctcacc tgccaaagat ggagaagagt gccagggacc cctcaaggag ggagacgctc 1380
catatcccag ggcataggac ttgcaggttc ctaggagcag gagcatctcc catcgcacgt 1440
gctttctgct cttctgggaa tttctcacac tggcaaagca gtccagcctc cgtcttctgg 1500
tccactctgc tctgagcagc ctgggatgct gaactcttca gagagatttt tttatagaga 1560
gatttctata attttgatac aaggtcatga ctatcctaga actctctgtg gtttttgaaa 1620
atcattgaat tctattaatg taggtaccta aagtgacctt aactgaatgt ggatgaggct 1680
ggggctggtg tgggtctttt ggctgctttt caaggtgtcc cccaatgtgg ccctcaagag 1740
ccatccccac tgcctggcca gagccattgt tgtcccctac ttcctaggcc atttctgggg 1800
cttgggggat gaatgctgtc ctgtgctgta aacactatgc aaatggaagt tatcggttgt 1860
ggtgctgtgc agcgctctgt gggcgactaa gtgccactca cgcagcatgt tcctggcaag 1920
gagcacatac catcaagcca cactatcatg gtattgttct cacagtcttt tggtggttga 1980
tggccactgc aaacctggca ccatcagatc tcttctgatc tcttgcccca gtggggcctg 2040
gttggtagaa tgttggcatt cggttgatat ccaaagcctg ttctcccagc cgtcctcctg 2100
cagctggagc cttcaggccg tattctcacg agggaacgtt tgccaaggct ctgacctcac 2160
agaagatgcc cagggcccag aagccatcag aattatcagt ggagaagcac cttttgactc 2220
ttcccttcca atgtaatctc tgccaacacc atgaggctta aggtgctcta agtcatgagt 2280
gttttggtct caaatgctgc agttttaata atctgtgact cctgagagcc catggttttt 2340
tgaccttgtg gttctaaaat tccttgtctg acccctgtag atcttttcct tgccatgtca 2400
cctcccttgg cctttgatcc tggaaaggtg gcagagcctc cactgagcca ggcccagagc 2460
tccttgcagt gccttcttcc ttgtttacct gtgggaggaa acactttttt tgtcaggggc 2520
agcctggttc agagctcaga ggtcacactg tatcaaagat ctcaaacagc aaagtcagca 2580
tttgctgtat agagctgcca cccaactcta agcaggagaa actgtacaga aagggctttg 2640
ctatttttcc cttttgggaa aacaatgaag tgttttaagt cctgggtgga ctgagagatg 2700
gtttgcctgt ccagacttgc tctcaagcct catccagaga aggagctgca gatgagggag 2760

cccgtacact ccctgccacc actaggttgt aagcctgtag ctggctggct gatttcattt 2820
tggaattcat ttgccatcca cagccttaca ctaggcacac actttagagt ctggggctcc 2880
agtggggccc gcctaatttt ttttcccccc aagacagggc cttgctctgt ctcccaggct 2940
ggagtgcagt ggcatgatca tggcttactg cagccttgat ctcccaggct caagcgatcc 3000
ttctgcctca gcctctctgg tagctgagac tgcatgccca gctccaaatc accttgattc 3060
atatcagcag taataatcac ttgtgttctg aaagaaaggg caccagaagt tctagcaaaa 3120
ttcagttgtg ttctgtgagc tagcactttt tcctctgacc caattttctt acctataaaa 3180
tggtgataaa aaccgacagg ttgttcaaag gcccagatca gctaaagcat gtatataaga 3240
gcacgttgta aacttgaaag agacaaaggc acaaatgtgg ctgttgatta atttgactgc 3300
ttctcgttgc tcgtcacctc catgccaggc actgtgcttg ctaattgctt tatgggggca 3360
ttctcttatt tattccccag ccctgggaaa taggagctgt cattatcctt ctctttctgc 3420
acaaggaaaa attaatgccc tgagaattgt cataattttc ccaaggctgc ccagctggtg 3480
gtgttaagcc agaatttgac ctcccagagc cagtttccat tagctgccat gctctgctgc 3540
ctctaattca cagaatgcac tttctaccct gtgtgccatg gagacctcct atggaaaaat 3600
gatcagccac cttaccttct actgggtacc tgctgtgagt ctgcctatgc cagaaggatt 3660
aaggagggga ggttacccaa gaaacaaagc ctacatgccg cttacagccc ccgttggatg 3720
gttgctcagt acaacagtct tgcattcagc aggtgtttgt tcatcaccta ctatgtgtca 3780
ggctctatgc taggtactgg ggatacagga gagaatcaag cgtaaagtct ttgttctcaa 3840
ggaatttgca ttctagaaag tagaagatgt aataaatgta ctgtgggaca tgttaataag 3900
tgctataaag aaatataaag ggtttgggag caaaaagagg gagtggatct attttagatg 3960
agcccaggta agacctctct gaagagctgt catgaaggag ggagggagca cattcctggc 4020
agagaaaaca gcacgtgcaa aggccccgag actggagtgt gttcctgaag agcagccagg 4080
aggccagcat ggctggagag gcaggcatag gcagggaacc gagcagcagg tcagagcagg 4140
cgagctgaca ttctgcagcc tggacggcca tggcaggaag cttttagttg gagagataca 4200
ggaagcctcc tagggttctg agcagaagag gggcatgagc tgattcacat tctgaaggac 4260
ctctctagct ggccagtgct gaggaggttg gagagagaaa gggtgaaagc agagagacca 4320
gtgcagggct gttaacaggg ttgcaggcga gagactgggg tgctgggctc ccctagacta 4380
ggactccagt gccctcctct cccaagagac aaaggccatt gcattgaagg aggtgggaaa 4440
tgattagatt ctgaacatat gtaattattt ttcagtcttt ttcaaagata caaatattta 4500
catagtttta atcatgtaat atatacaatt taatgtccta gtgttttact taatagtgta 4560
tcatgttttc cctgttggta tgtagcctgg ataaatgctc ttaattataa aaaattctgt 4620
cgaggagtgt tccatagttt attgttttcc tattatgaga atttaggcca agtgtggtgg 4680
ctcatgcctg taatcccagc actttgcgag gccgaggtgg gcagatcact tgaggtgagg 4740
agttcaagac cagcctggcc aacatggtga attatctcta ctaaaaatac aaaaaaataa 4800

taataatagc caggcgtggt ggcacatgcc tgtattccca gctgcttggg aggctgaggc 4860

aggagaatgg cttgaacctg ggaggtggag gttgcagtga gccgagatgg tgccactgca 4920

ttccagcctg ggcaacagag cgagactcca tctcaaaaaa aaggagactt catgtgcccc 4980

caatttttca ctattgttat ttgaaaaaat atttttattt gtaagagttt ttctttattt 5040

aaaatgttca ttaataaagt tgttggacgg gaagcaaaaa aaaaaagttg tttaagataa 5100

attcccagaa gtgaatttgt tagatcaaac acttaaaact ttttgttatg gaagaattca 5160

aatataaata aaaaattgtg agtaataaaa tgaactcaca gtttcaacaa tgacccacaa 5220

aaaaaaaaaa aaaaaaaaaa aaaaaaaaa 5249

<210> 177
<211> 4843
<212> DNA
<213> Homo sapiens

<400> 177
cggaagtgcg tggccgcccg gggccatggc gacactcagc ttcgtcttcc tgctgctggg 60

ggcagtgtcc tggcctccgg cttctgcctc cggccaggag ttctggcccg gacaatcggc 120

ggccgatatt ctgtcggggg cggcttcccg cagacggtat cttctgtatg acgtcaaccc 180

cccggaaggc ttcaacctgc gcagggatgt ctatatccga atcgcctctc tcctgaagac 240

tctgctgaag acggaggagt gggtgcttgt cctgcctcca tggggccgcc tctatcactg 300

gcagagtcct gacatccacc aggtccggat tccctggtct gagttttttg atcttccaag 360

tctcaataaa aacatccccg tcatcgagta tgagcagttc atcgcagaat ctggtgggcc 420

ctttattgac caggtttacg tcctgcaaag ttacgcagag gggtggaaag aagggacctg 480

ggaagagaag gtggacgagc ggccgtgtat tgatcagctc ctgtactccc aggacaagca 540

cgagtactac agaggatggt tttggggtta tgaggagacc aggggtctaa acgtctcctg 600

tctgtccgtc cagggctcag cctccatcgt ggcgcccctg ctgctgagaa acacatcagc 660

ccggtccgtg atgttagaca gagccgagaa cctacttcac gaccactatg gagggaaaga 720

atactgggat acccgtcgca gcatggtgtt tgccaggcac ctgcgggagg tgggagacga 780

gttcaggagc agacatctca actccacgga cgacgcagac aggatcccct tccaggagga 840

ctggatgaag atgaaggtca agctgggctc cgcgctaggg ggcccctacc tgggagtcca 900

cctgagaaga aaagatttca tctggggtca cagacaggat gtacccagtc tggaaggggc 960

cgtgaggaag atccgcagcc tcatgaagac ccaccggctg gacaaggtgt ttgtggccac 1020

agatgccgtc agaaaggaat atgaagagct aaaaaagctg ttacccgaga tggtgaggtt 1080

tgaacccacg tgggaggagc tggagctcta caaggacgga ggcgttgcga ttattgacca 1140

gtggatctgc gcacacgcca ggtgcctgcc cacgtcactg tcggccgaga gcgggtcggg 1200

tggctttcaa aggttcttct gtcccaagta ctcggtgtca gagcagatgg tcgcctgtgt 1260

tcacagtggt catttccata ctgtttgcct cctcgtctga gtctcctgta gcatctggtt 1320

cagtgtttcc ctgggctgaa gttaattgtt catcttgccc ctttagttct catgcacaga 1380

attcctccat agcaggctgt tggcatagct ggcctcgtct cagaacctct tcttgtgtcg 1440
cattttccca tcattcccgg tttctgcccc tgtctgcccc ctgccctgag agttgcccgt 1500
gccctggact tgggcatgtc cttgttgctg tgttgttgag catccgtgag cgtccccgag 1560
gccgggagcg tgggccctcg tgtgatcatt ctcgtggggc tgccatgagc gtccccaagg 1620
ctgggagcat gggccctcgt gtgatcgttc ttgtggggct gccgtgagcg tccccgaggc 1680
cgggagcgtg ggccctcgtg tgatcattct cgtggggctg ccgtgagcgt cccagaggcc 1740
gggagcgtgg gccctgcgtg cagtcattcc tctggggttg ctgtgggagg tacgcctggg 1800
cctctgttcc tccaaagacc tgcctgccca tctgcatagg agatgaaggc tggggttagg 1860
gtgaaacggt ttgagttaaa tggaaaatga aagtagaggg aatgatcttc cccgtggtta 1920
gcactgtgca cacgcgtgcg tctctgtggg ttagtctgtc tctctcctgc ccgaggaatg 1980
ctgagcgccc tgagccggtg cctcttcaca catctgctat ttcctgtggt gttctgggca 2040
tggtgtgtaa gacccacaga ggctccgggt gatgctgtct gctgggtgtg ggtccctttc 2100
cctgttaagc agacaggatg cagcgctgac ttcttaggtc agggcggagg tgtgcaggag 2160
cccagttacg aactcacccc tgcttctcag gtgtggcctt gggattttga ctgcgacctt 2220
ggcggtgctg tctccgcagc ccaggaagcc tgctgtgggg aggcttcgca ctgagctctc 2280
agcctcctgc cctcagctgc gcgaagcgct cggcccagct cactgaagct gccctgcctc 2340
cgggccggcg cggcctgctc tggcaggccc ctgtgtgtgg ggtggtgagg gtctccccac 2400
cagtgctgca ccccgcagca gcatacaggc ctgtgtggcc tgctggccct gtggctctgt 2460
gtacagcgct gtgcatgtta catttgctct ggaaacatct ctggggtttg cttgttcacg 2520
aagttcatga agtgccgctg gagagccaga gaccagctgc gcaggagccg gaggaacggg 2580
caggccgctg acctgaggtc tggagaaacc cctggagaag ggtgtcccca ccagcccata 2640
cagcgtgtgt gtggaggggg ccttgacctc cgtgatgtct actgtgcctc aggataagga 2700
cccgccatgc cctggctaga cagtgtgtgg ttagtaggaa tctctcattg ttcaccatgt 2760
gacccccagg aggtattcga cctgcactgg cgtgcctggc ctgggatttg gtgacggaga 2820
ggagggctcc cagggggaca tgcggtggga caggagcgtg gcggctgctg actgtggggt 2880
gtggatgggg ctgcagcacc aggcagagcc cctcagggcg ccgatgtcga gggcacctga 2940
gcgaggggtg ccagcaaggg ggcggccggc tggtgggtgc tcgggggacg gccgtgttgg 3000
ttccatgtaa ctgtggaact catttcactc agctgctcct ctcagttctc cctgactctg 3060
gaacctctgt gacgccagtt ctccctgact ctggaacctc tgtgatcctt tgcaggtttt 3120
ttattggcac ctcagtctca acattttctt ttcggattca tgaggaaaga gaaatcctgg 3180
ggttggaccc caagacgacg tacaacaggt tctgcggaga ccaagagaag gcgtgtgagc 3240
aacccaccca ctggaagatc acctactgag gaggatcctc cagggccgct ccccggaccc 3300
gacaggcgcg ggtggatgca ggttctgtcg ccgtggagtc accgtctact gccagccggg 3360
agctgggcgg acaggaccgt ccctcgcagg gtcccaggcc cagaagaggc cccacgcctc 3420

tagagctggg ctccgtcctc ggcgttgcca gccgccatgg ctgatgaaga ggctccgctg 3480
ctctcggggg tggcggttgt tttcaggcag cgtctgtgaa cccacagctc ggttgccagc 3540
agtgcccgcg tggtgaccca gaagcaggag tgtttgtcag gctcccgctc tggcctttcc 3600
agccaccttt catgtcttca tattttaagt gcattgagga tagatgcagg cgggtgagct 3660
gccctccgtc aggtggaccc gggctgacat ttccctggga gctggtgcaa ggagaagcgt 3720
cattttaaat gtctgcagag cgaccagggg cctcatgaat ctctccgttg ccctccgcgc 3780
agcaggaggc tgcctgtgtg tttcctcctg ggatgcgtgc aaggcagacc tggtgctgca 3840
aaggaaaggg cctgaggcct cagggagccc cgtggaggga tgacagttca ggccctactg 3900
ctggcacgtc agagcactgg gaagtttttc agtgacgtct ctggggcact cagtggattg 3960
tctgtaggaa acttgcagct ctgctcctca caccaggccc ggctggccac ccaccctcgc 4020
ccccactggc cacccctccc tcgccccgac tgccccgccc caccctcacc ccgactgccc 4080
cgccctcgcc cggctggccg tccctgccct cgccccggct ggcaggtgca catggggcct 4140
ccaggtctgc cattcgctat tgagaactag aaatgaggaa ggacagttac gctaactcca 4200
aaaggctgtc taggatgagc tgctttatca gggagctcct tgtacccatt ttacagaaat 4260
catttttagg tctttgtgcc accaccacga ggggcatctg caaagagggc aacgctagac 4320
acagaatccg tggaaggtgc agcagtgcct caggggtcct cagggtcagg gagcccccct 4380
caccctcttg gcccgttacc ctttgtgact ttccaccatg gtgtcgtgtg accctcagtc 4440
aggttggtgg gggctgagtc ctcactgagc agccactttc cacatctgct agaggaacag 4500
tgacatggac acctgtgaca gagagaggac agttaggagg gacagacagc tcttcctttc 4560
ggagcctggc tagtctagga catcaccttg ctgtgtcttc tcaagctttt aaaattgacc 4620
ctgaacgtgt gacagggtcc tatggtgtta ctcaaagctg tgcagggtaa atgatgacat 4680
atttattctt tttccatttg ttctagaaac agtgcctttt tcatcagttg cattttccag 4740
gctgagagct gtataaaaca ttttggactg tgaccatgta ccttcctttt taagaaaaat 4800
aaactgcttt atggaagttg gtaaaaaaaa aaaaaaaaaa aaa 4843

<210> 178
<211> 2962
<212> DNA
<213> Homo sapiens

<400> 178
ggggcggagc gcagccaggg ctgcgcggcg cgccccggct cccgcccgct cccagccggg 60
ccccccagcg gtcggcggga cggctcccgg ctgcagtctg cccgcccgcc ccgcgcgggg 120
gccgagtcgc gaagcgcgcc tgcgacccgg cgtccgggcg cgctggagag gacgcgagga 180
gccatgaggc gccagcctgc gaaggtggcg gcgctgctgc tcgggctgct cttggagtgc 240
acagaagcca aaaagcattg ctggtatttc gaaggactct atccaaccta ttatatatgc 300
cgctcctacg aggactgctg tggctccagg tgctgtgtgc gggccctctc catacagagg 360

ctgtggtact tctggttcct tctgatgatg ggcgtgcttt tctgctgcgg agccggcttc 420
ttcatccgga ggcgcatgta ccccccgccg ctgatcgagg agccagcctt caatgtgtcc 480
tacaccaggc agcccccaaa tcccggccca ggagcccagc agccggggcc gccctattac 540
accgacccag gaggaccggg gatgaaccct gtcgggaatt ccatggcaat ggctttccag 600
gtcccaccca actcacccca ggggagtgtg gcctgcccgc cccctccagc ctactgcaac 660
acgcctccgc ccccgtacga acaggtagtg aaggccaagt agtggggtgc ccacgtgcaa 720
gaggagagac aggagagggc ctttccctgg cctttctgtc ttcgttgatg ttcacttcca 780
ggaacggtct cgtgggctgc taagggcagt tcctctgata tcctcacagc aagcacagct 840
ctctttcagg ctttccatgg agtacaatat atgaactcac actttgtctc ctctgttgct 900
tctgtttctg acgcagtctg tgctctcaca tggtagtgtg gtgacagtcc ccgagggctg 960
acgtccttac ggtggcgtga ccagatctac aggagagaga ctgagaggaa gaaggcagtg 1020
ctggaggtgc aggtggcatg tagaggggcc aggccgagca tcccaggcaa gcatccttct 1080
gcccgggtat taataggaag ccccatgccg ggcggctcag ccgatgaagc agcagccgac 1140
tgagctgagc ccagcaggtc atctgctcca gcctgtcctc tcgtcagcct tcctcttcca 1200
gaagctgttg gagagacatt caggagagag caagcccctt gtcatgtttc tgtctctgtt 1260
catatcctaa agatagactt ctcctgcacc gccagggaag ggtagcacgt gcagctctca 1320
ccgcaggatg gggcctagaa tcaggcttgc cttggaggcc tgacagtgat ctgacatcca 1380
ctaagcaaat ttatttaaat tcatgggaaa tcacttcctg ccccaaactg agacattgca 1440
ttttgtgagc tcttggtctg atttggagaa aggactgtta cccatttttt tggtgtgttt 1500
atggaagtgc atgtagagcg tcctgccctt tgaaatcaga ctgggtgtgt gtcttccctg 1560
gacatcactg cctctccagg gcattctcag gcccgggggt ctccttccct caggcagctc 1620
cagtggtggg ttctgaaggg tgctttcaaa acggggcaca tctggctggg aagtcacatg 1680
gactcttcca gggagagaga ccagctgagg cgtctctctc tgaggttgtg ttgggtctaa 1740
gcgggtgtgt gctgggctcc aaggaggagg agcttgctgg gaaaagacag gagaagtact 1800
gactcaactg cactgaccat gttgtcataa ttagaataaa gaagaagtgg tcggaaatgc 1860
acattcctgg ataggaatca cagctcaccc caggatctca caggtagtct cctgagtagt 1920
tgacggctag cggggagcta gttccgccgc atagttatag tgttgatgtg tgaacgctga 1980
cctgtcctgt gtgctaagag ctatgcagct tagctgaggc gcctagatta ctagatgtgc 2040
tgtatcacgg ggaatgaggt gggggtgctt attttttaat gaactaatca gagcctcttg 2100
agaaattgtt actcattgaa ctggagcatc aagacatctc atggaagtgg atacggagtg 2160
atttggtgtc catgcttttc actctgagga catttaatcg gagaacctcc tggggaattt 2220
tgtgggagac acttgggaac aaaacagaca ccctgggaat gcagttgcaa gcacagatgc 2280
tgccaccagt gtctctgacc accctggtgt gactgctgac tgccagcgtg gtacctccca 2340
tgctgcaggc ctccatctaa atgagacaac aaagcacaat gttcactgtt tacaaccaag 2400

acaactgcgt gggtccaaac actcctcttc ctccaggtca tttgttttgc atttttaatg 2460

tctttatttt ttgtaatgaa aaagcacact aagctgcccc tggaatcggg tgcagctgaa 2520

taggcaccca aaagtccgtg actaaatttc gtttgtcttt ttgatagcaa attatgttaa 2580

gagacagtga tggctagggc tcaacaattt tgtattccca tgtttgtgtg agacagagtt 2640

tgttttccct tgaacttggt tagaattgtg ctactgtgaa cgctgatcct gcatatggaa 2700

gtcccacttc ggtgacattt cctggccatt cttgtttcca ttgtgtggat ggtgggttgt 2760

gcccacttcc tggagtgaga cagctcctgg tgtgtagaat tcccggagcg tccgtggttc 2820

agagtaaact tgaagcagat ctgtgcatgc ttttcctctg caacaattgg ctcgtttctc 2880

ttttttgttc tcttttgata ggatcctgtt tcctatgtgt gcaaaataaa aataaatttg 2940

ggcaaaaaaa aaaaaaaaaa aa 2962

<210> 179
<211> 1793
<212> DNA
<213> Homo sapiens

<400> 179
   cgcgtccgcc ccgcgagcac agagcctcgc ctttgccgat ccgccgcccg tccacacccg 60
   ccgccagctc accatggatg atgatatcgc cgcgctcgtc gtcgacaacg gctccggcat 120
   gtgcaaggcc ggcttcgcgg gcgacgatgc cccccgggcc gtcttcccct ccatcgtggg 180
   gcgccccagg caccagggcg tgatggtggg catgggtcag aaggattcct atgtgggcga 240
   cgaggcccag agcaagagag gcatcctcac cctgaagtac cccatcgagc acggcatcgt 300
   caccaactgg gacgacatgg agaaaatctg gcaccacacc ttctacaatg agctgcgtgt 360
   ggctcccgag gagcaccccg tgctgctgac cgaggccccc ctgaacccca aggccaaccg 420
   cgagaagatg acccagatca tgtttgagac cttcaacacc ccagccatgt acgttgctat 480
   ccaggctgtg ctatccctgt acgcctctgg ccgtaccact ggcatcgtga tggactccgg 540
   tgacggggtc acccacactg tgcccatcta cgaggggtat gccctccccc atgccatcct 600
   gcgtctggac ctggctggcc gggacctgac tgactacctc atgaagatcc tcaccgagcg 660
   cggctacagc ttcaccacca cggccgagcg ggaaatcgtg cgtgacatta aggagaagct 720
   gtgctacgtc gccctggact tcgagcaaga gatggccacg gctgcttcca gctcctccct 780
   ggagaagagc tacgagctgc ctgacggcca ggtcatcacc attggcaatg agcggttccg 840
   ctgccctgag gcactcttcc agccttcctt cctgggcatg gagtcctgtg gcatccacga 900
   aactaccttc aactccatca tgaagtgtga cgtggacatc cgcaaagacc tgtacgccaa 960
   cacagtgctg tctggcggca ccaccatgta ccctggcatt gccgacagga tgcagaagga 1020
   gatcactgcc ctggcaccca gcacaatgaa gatcaagatc attgctcctc ctgagcgcaa 1080
   gtactccgtg tggatcggcg gctccatcct ggcctcgctg tccaccttcc agcagatgtg 1140
   gatcagcaag caggagtatg acgagtccgg cccctccatc gtccaccgca aatgcttcta 1200
   ggcggactat gacttagttg cgttacaccc tttcttgaca aaacctaact tgcgcagaaa 1260
   acaagatgag attggcatgg ctttatttgt tttttttgtt ttgttttggt tttttttttt 1320
   tttttggctt gactcaggat ttaaaaactg gaacggtgaa ggtgacagca gtcggttgga 1380
   gcgagcatcc cccaaagttc acaatgtggc cgaggacttt gattgcacat tgttgttttt 1440
   ttaatagtca ttccaaatat gagatgcatt gttacaggaa gtcccttgcc atcctaaaag 1500
   ccaccccact tctctctaag gagaatggcc cagtcctctc ccaagtccac acaggggagg 1560
   tgatagcatt gctttcgtgt aaattatgta atgcaaaatt tttttaatct tcgccttaat 1620
   acttttttat tttgttttat tttgaatgat gagccttcgt gccccccctt cccccttttt 1680
   gtcccccaac ttgagatgta tgaaggcttt tggtctccct gggagtgggt ggaggcagcc 1740
   agggcttacc tgtacactga cttgagacca gttgaataaa agtgcacacc tta 1793

<210> 180
<211> 2762
<212> DNA
<213> Homo sapiens

<400> 180
gggcccagat agaacaagaa ggcagaagaa atgtgaattc ctctttctct gctggagctg 60
ggatattctt cttctcctgc ccttggacat cagagctgca ggctctctgg cctttggacc 120
cgaggattta taccaagcag gtttctgggt tctcaggcct ttggccttgg actgatagtt 180
acaccattgg catatctggt tctgaggctc ttggtcttgg actgagccac actcctggca 240
tcccagcgtc tccagcttgc atggcctgtc acgtgattcc tgtcagaatc accatttttg 300
gtaaacaaac caagcccaga acctgataat tatggagcat tctacactga cagttctttg 360
agacaaattt cctcttggca tttacactgt ggctttagct ttcaaaccag aggttcctct 420
tacccagcaa aaaatggctt tcccgaagat gagattgatg tatatttgcc ttctggttct 480
gggggctctt tgtttgtatt ttagcatgta cagtctaaat cctttcaaag aacagtcctt 540
tgtttacaag aaagacggga acttccttaa gctcccagat acagactgca ggcagacacc 600
tcccttcctc gtcctgctgg tgacctcatc ccacaaacag ttggctgagc gcatggccat 660
ccggcagacg tgggggaaag agaggatggt gaagggaaag cagctgaaga cattcttcct 720
cctggggacc accagcagtg cagcggaaac gaaagaggtg gaccaggaga gccagcgaca 780
cggggacatt atccagaagg atttcctaga cgtctattac aatctgaccc tgaagaccat 840
gatgggcata gaatgggtcc atcgcttttg tcctcaggcg gcgtttgtga tgaaaacaga 900
ctcagacatg ttcatcaatg ttgactatct gactgaactg cttctgaaga aaaacagaac 960
aaccaggttt ttcactggct tcttgaaact caatgagttt cccatcaggc agccattcag 1020
caagtggttt gtcagtaaat ctgaatatcc gtgggacagg tacccaccat tctgctccgg 1080
caccggctac gtgttttctg gcgacgtggc gagtcaggtg tacaatgtct ccaagagcgt 1140
cccatacatt aaactggaag acgtgtttgt ggggctctgc ctcgaaaggc tgaacatcag 1200
attggaggag ctccactccc agccgacctt ttttccaggg ggcttacgct tctccgtatg 1260

cctcttcagg aggatcgtgg cctgccactt catcaagcct cggactctct tggactactg 1320

gcaggctcta gagaattccc ggggggaaga ttgtccgcct gtctgagggg agcccagagg 1380

cacatccgga caagtttcag ataacccgtg gggatagttt ttgctagatt ttggaagagg 1440

gggcgggaca gaggatgctg ttcttcagtg ctgaaatcca cgccagaatg tcggtgttca 1500

tgaagtcact gattagttcc cacttggtgc cccaggcaat aataggcccg tctcttgggc 1560

acgcacactc ttcatactaa gtgtttgaca tacacctgga tttttgcatt tcaggggtca 1620

gtatcctatg acatgatggg tgttaccatc ctaattttac aggcaaggac acagcagctg 1680

cgagaggtac agaaacttgt cccaaggctc acagccagta ggcataggag cgggaatgaa 1740

aatcgagcac tgtcagaatc tggtgggcag cccctgactt gaaccactcc cacgtgctgc 1800

ctcccttagg aggggacact gatgatgagg tctcggagcc ggcatccttc catccctgtc 1860

gagtcccctc cacctcagct cccagtcctt gtgctttttg gagctaagcc tgggatgacc 1920

aaattcaccc cagctccttc attcacaggg ctggatgtag ctgggattga gtccatgtta 1980

tcggctcggt actcaacaca acccaagttt catccgagga aatgtccccg cagtggatgc 2040

agctcacatg ctgaggaaca cccagctctg gacagagttc ttataaatgt ataaattagg 2100

ctcagaaacc actgcattct gacctgctgt acagactgcc cacactgctg acctgcctag 2160

cgagcaggac atcccttctg agccatctgc tgctctctca tttcatcacc ccaactgtcc 2220

cttgtttttg atcaatgggg accagccact gccccaggag cactttaggg ctctcagttc 2280

aaactgaagg acagttgaac tcagatgggg ttcatgtggg attctgggag ctttctggga 2340

attcagttgg agtcaagtca ggatgctctc aaggacccct cgggctcaga gccctaaagt 2400

gggccctggt gaagcagggt ggtcctgcgt ccacttccca agcctgagcc aagctcatct 2460

tcattgaatg tctcatttgg ccgaggaaca actgaacttt gtggtttgct gtttagcctt 2520

cagtttgctc cgctgcctcc tacccagagg tttgtgcgag cctgtgttgc agggttgtat 2580

aaaaccaagg tacttcgtta gttttgccca ttcagccatg gtcacgtgac atgcaaagta 2640

atcttgctcc taattataga aatgattttt cttttaattt tttactttac cagactttac 2700

tttgtactca gagaagaggc ctcacatggc tgtgtcacat ataaatgttg gactaaactc 2760

tt 2762

<210> 181
<211> 3523
<212> DNA
<213> Homo sapiens

<400> 181
atggctgtgc gctctcgccg cccgtggatg agcgtggcat tagggctggt gctgggcttc 60

accgccgcgt cctggctcat cgcccccagg gtggcggagc tgagcgagag gaagagacgt 120

ggctccagcc tctgctccta ctacggtcgc tctgctgctg gcccccgcgc cggcgctcag 180

cagccgctcc cccagcccca gtcccgacca cggcaggagc agtcgccgcc ccccgcgcgc 240

caggatctcc aggggccacc gctgcccgag gcagcacccg ggatcaccag ttttcgaagc 300

agcccctggc agcagccacc tccgctgcag cagcggcggc gaggacgcga gcctgagggc 360
gcgacggggc ttcccggtgc tccagcggcc gagggggagc ccgaggagga ggacgggggc 420
gcggctgggc agcggagaga cggccggccg gggagtagcc acaacggcag cggggacggg 480
ggcgctgccg ccccgagcgc ccgaccccgg gacttcctgt acgtgggggt gatgaccgcg 540
cagaagtacc tgggcagccg cgcgctggcc gcgcagcgga cctgggcgcg tttcatcccg 600
ggccgcgtgg agttcttttc cagccagcag ccccccaacg ccggccagcc cccgccaccc 660
ctgcctgtca tcgcgctacc gggtgtggac gactcctatc ctccccagaa aaagtccttc 720
atgatgatca agtacatgca cgaccactac ctggacaagt atgagtggtt catgcgcgcc 780
gacgacgatg tctacatcaa aggtgataaa ttagaagagt ttcttagatc gctaaacagc 840
agtaagcctc tctacctggg acagactggc ctggggaata ttgaagagct tggaaagctg 900
ggactggagc ctggggaaaa cttctgtatg ggaggacctg gcatgatctt tagccgagaa 960
gttctcagga ggatggtgcc acatattggt gaatgcctta gagaaatgta cacgactcat 1020
gaggatgtgg aagtaggaag atgcgttcgc cgttttggtg ggactcagtg tgtctggtct 1080
tacgagatgc aacaactgtt ccatgaaaat tatgaacaca atcggaaggg ttacatccaa 1140
gaccttcaca atagcaaaat ccatgcagcc ataacacttc atcccaacaa aaggcctgca 1200
taccaataca ggctgcataa ttacatgctc agccgcaaaa tttctgaact tcgctaccgc 1260
accatccagc tccacaggga aagtgccctg atgagcaagc tcagtaacac agaagtgagc 1320
aaagaggacc agcagctggg agtgatacct tctttcaacc acttccagcc tcgggagaga 1380
aatgaagtga tagaatggga gttcctgaca gggaagcttc tatactcagc agctgagaac 1440
cagccccctc gacagagcct cagtagcatt ttaagaacag cactggatga taccgtccta 1500
caggtgatgg agatgatcaa tgagaatgcc aagagcagag gacggctcat tgacttcaag 1560
gaaattcagt atggctaccg cagagttaac cccatgcacg gggtggagta cattttggat 1620
ttactccttt tatacaaaag acacaaggga aggaaactga ctgtgccagt gagacgtcat 1680
gcctatcttc agcagttgtt cagcaagcct ttcttcagag agaccgaaga gctagatgtc 1740
aacagtcttg tggagagtat taacagtgaa actcagtcat tctcctttat atctaattct 1800
ttaaagatat tatcttcttt tcaaggtgcc aaagaaatgg gagggcacaa tgaaaagaaa 1860
gtacacattc tcgttcctct catcggaagg tatgacattt tcttgagatt catggagaac 1920
tttgaaaaca tgtgtcttat cccaaagcag aatgtaaagt tggtcattat ccttttcagt 1980
agggattctg gccaagactc cagcaagcat attgagctga taaaagggta ccagaacaag 2040
taccccaaag cagaaatgac cctgatccca atgaagggag agttttccag aggtcttggt 2100
cttgaaatgg cttctgccca gtttgacaat gacactttgc tgctattttg tgatgttgac 2160
ttgatcttca gagaagattt tctccaacga tgtagagaca atacaattca gggacaacag 2220
gtgtactatc ccatcatctt tagccagtat gacccaaagg taacaaacgg gggaaatcct 2280
cccactgatg attacttcat attctcaaaa aagactggat tttggagaga ctatggatat 2340

ggcatcacct gtatttacaa aagtgatctt ctaggtgcag gtggatttga tacctcaata 2400

caaggctggg gactagaaga tgtagatctc tacaataaag tcattctatc tggcttaagg 2460

ccattcagaa gccaagaagt aggagtggtg catattttcc atccagttca ttgtgatcct 2520

aacttggacc ctaagcagta taagatgtgc ttaggatcca aggcaagtac tttcgcctca 2580

accatgcaac tggctgaact ctggcttgaa aaacatttag gtgtcaggta caatcgaact 2640

ctctcctgac agtccaggca acacattttg ccttttttaa ggggagttta cctcattgtt 2700

ggttgttgtt atttttattg tattattgtt attttattat tattattgtt ataattttat 2760

tttgttgtcc tggtcttaaa ctactcttgg ttgtcttcct aagggtgttt gttgacctca 2820

agcaagaaga gtctgcagtt cactgatgtt tcagatttct actgaagtca atatgttatt 2880

acttttatat aactttattt gagattgagt taaatcatag caatcaaatg acttttgaaa 2940

cgcattcatc acaagagaca gcttagaaga atgttctctt ggggcttaaa gatgcaatat 3000

cgacttttat ttggtttctc aaattcaatg tgatacaaat atttactggt gaaaggtacc 3060

acaaaagtgc tttatgcttc ctatggggaa gggactctgt aacataaact tgagttttgt 3120

aatttataca aggacttaaa tatataaaca ataattttct tcatctttag aatttttaaa 3180

gtaaatgaat acctatgatt gtatgtttat tttttaaaaa aagttttaaa aattgaggag 3240

ttttgttcca caagcaaccg gtactggcta ccctggtctt atgacaatta tgagctcctc 3300

acaactgtct gctataaatg cgaatgaact ttattttctc aaggaaatat gatttaatta 3360

aatattcatg tacattttag aagctttatg aaacaatgtc cttcatttgc tggcaagaag 3420

ataaaatatg acagaacctg tttatttaaa ataaaacaca ggtatagcag tattcttttt 3480

caaaaacact gaaaaaaaaa aaaaaaaaaa aaaaaaaaaa aaa 3523

<210> 182
<211> 2098
<212> DNA
<213> Homo sapiens

<400> 182
cgcggcgcgg catactcagc ggcagagacg gaagaacagc gctcccgagg ccgcgggagc 60

ctgcagagag gacagccggc ctgcgccggg acatgcggcc ccaggagctc cccaggctcg 120

cgttcccgtt gctgctgttg ctgttgctgc tgctgccgcc gccgccgtgc cctgcccaca 180

gcgccacgcg cttcgacccc acctgggagt ccctggacgc ccgccagctg cccgcgtggt 240

ttgaccaggc caagttcggc atcttcatcc actggggagt gttttccgtg cccagcttcg 300

gtagcgagtg gttctggtgg tattggcaaa aggaaaagat accgaagtat gtggaattta 360

tgaaagataa ttaccctcct agtttcaaat atgaagattt tggaccacta tttacagcaa 420

aattttttaa tgccaaccag tgggcagata tttttcaggc ctctggtgcc aaatacattg 480

tcttaacttc caaacatcat gaaggcttta ccttgtgggg gtcagaatat tcgtggaact 540

ggaatgccat agatgagggg cccaagaggg acattgtcaa ggaacttgag gtagccatta 600

ggaacagaac tgacctgcgt tttggactgt actattccct ttttgaatgg tttcatccgc 660

tcttccttga ggatgaatcc agttcattcc ataagcggca atttccagtt tctaagacat 720

tgccagagct ctatgagtta gtgaacaact atcagcctga ggttctgtgg tcggatggtg 780

acggaggagc accggatcaa tactggaaca gcacaggctt cttggcctgg ttatataatg 840

aaagcccagt tcggggcaca gtagtcacca atgatcgttg gggagctggt agcatctgta 900

agcatggtgg cttctatacc tgcagtgatc gttataaccc aggacatctt ttgccacata 960

aatgggaaaa ctgcatgaca atagacaaac tgtcctgggg ctataggagg gaagctggaa 1020

tctctgacta tcttacaatt gaagaattgg tgaagcaact tgtagagaca gtttcatgtg 1080

gaggaaatct tttgatgaat attgggccca cactagatgg caccatttct gtagtttttg 1140

aggagcgact gaggcaaatg gggtcctggc taaaagtcaa tggagaagct atttatgaaa 1200

cccatacctg gcgatcccag aatgacactg tcaccccaga tgtgtggtac acatccaagc 1260

ctaaagaaaa attagtctat gccatttttc ttaaatggcc cacatcagga cagctgttcc 1320

ttggccatcc caaagctatt ctgggggcaa cagaggtgaa actactgggc catggacagc 1380

cacttaactg gatttctttg gagcaaaatg gcattatggt agaactgcca cagctaacca 1440

ttcatcagat gccgtgtaaa tggggctggg ctctagccct gactaatgtg atctaaagtg 1500

cagcagagtg gctgatgctg caagttatgt ctaaggctag gaactatcag gtgtctataa 1560

ttgtagcaca tggagaaagc aaatgtaaaa ctggataaga aaattatttt ggcagttcag 1620

ccctttccct ttttcccact aaattttttc ttaaattacc catgtaacca ttttaactct 1680

ccagtgcact ttgccattaa agtctcttca cattgatttg tttccatgtg tgactcagag 1740

gtgagaattt tttcacatta tagtagcaag gaattggtgg tattatggac cgaactgaaa 1800

attttatgtt gaagccatat cccccatgat tatatagtta tgcatcactt aatatgggga 1860

tattttctgg gaaatgcatt gctagtcaat ttttttttgt gccaacatca tagagtgtat 1920

ttacaaaatc ctagatggca tagcctacta cacacctaat gtgtatggta tagactgttg 1980

ctcctaggct acagacatat acagcatgtt actgaatact gtaggcaata gtaacagtgg 2040

tatttgtata tcgaaacata tggaaacata gagaaggtac agtaaaaata ctgtaaaa 2098

<210> 183
<211> 3577
<212> DNA
<213> Homo sapiens

<400> 183
gaaaggaaac cggcactgcc tgccggcttt acatctgttg atctgacctg actggaagcg 60

tccaaagagg gacggctgtc agccctgctt gactgagaac ccaccagctc atcccagaca 120

cctcatagca acctatttat acaaaggggg aaagaaacac ctgagcagaa tggaatcatt 180

atttttttcc caaggagaaa accggggtaa agggagggaa gcaattcaat ttgaagtccc 240

tgtgaatggg ctttcagaag gcaattaaag aaatccactc agagaggact tggggtgaaa 300

cttgggtcct gtggttttct gattgtaagt ggaagcaggt cttgcacacg ctgttggcaa 360

atgtcaggac caggttaagt gactggcaga aaaacttcca ggtggaacaa gcaacccagg 420
ttctgctgca agcttgaagg agcctggagc gggagaaagc taacttgaac atgacctgtt 480
gcatttggca agttctagca acatgctcct aaggaagcga tacaggcaca gaccatgcag 540
actccagttc ctcctgctgc tcctgatgct gggatgcgtc ctgatgatgg tggcgatgtt 600
gcaccctccc caccacaccc tgcaccagac tgacacagcc caagccagca agcacagccc 660
tgaagccagg taccgcctgg actttgggga atcccaggat tgggtactgg aagctgagga 720
tgagggtgaa gagtacagcc ctctggaggg cctgccaccc tttatctcac tgcgggagga 780
tcagctgctg gtggccgtgg ccttacccca ggccagaagg aaccagagcc agggcaggag 840
aggtgggagc taccgcctca tcaagcagcc aaggaggcag gataaggaag ccccaaagag 900
ggactggggg gctgatgagg acggggaggt gtctgaagaa gaggagttga ccccgttcag 960
cctggaccca cgtggcctcc aggaggcact cagtgcccgc atccccctcc agagggctct 1020
gcccgaggtg cggcacccac tgtgtctgca gcagcaccct caggacagcc tgcccacagc 1080
cagcgtcatc ctctgtttcc atgatgaggc ctggtccact ctcctgcgga ctgtacacag 1140
catcctcgac acagtgccca gggccttcct gaaggagatc atcctcgtgg acgacctcag 1200
ccagcaagga caactcaagt ctgctctcag cgaatatgtg gccaggctgg agggggtgaa 1260
gttactcagg agcaacaaga ggctgggtgc catcagggcc cggatgctgg gggccaccag 1320
agccaccggg gatgtgctcg tcttcatgga tgcccactgc gagtgccacc caggctggct 1380
ggagcccctc ctcagcagaa tagctggtga caggagccga gtggtatctc cggtgataga 1440
tgtgattgac tggaagactt tccagtatta cccctcaaag gacctgcagc gtggggtgtt 1500
ggactggaag ctggatttcc actgggaacc tttgccagag catgtgagga aggccctcca 1560
gtcccccata agccccatca ggagccctgt ggtgcccgga gaggtggtgg ccatggacag 1620
acattacttc caaaacactg gagcgtatga ctctcttatg tcgctgcgag gtggtgaaaa 1680
cctcgaactg tctttcaagg cctggctctg tggtggctct gttgaaatcc ttccctgctc 1740
tcgggtagga cacatctacc aaaatcagga ttcccattcc cccctcgacc aggaggccac 1800
cctgaggaac agggttcgca ttgctgagac ctggctgggg tcattcaaag aaaccttcta 1860
caagcatagc ccagaggcct tctccttgag caaggctgag aagccagact gcatggaacg 1920
cttgcagctg caaaggagac tgggttgtcg gacattccac tggtttctgg ctaatgtcta 1980
ccctgagctg tacccatctg aacccaggcc cagtttctct ggaaagctcc acaacactgg 2040
acttgggctc tgtgcagact gccaggcaga aggggacatc ctgggctgtc ccatggtgtt 2100
ggctccttgc agtgacagcc ggcagcaaca gtacctgcag cacaccagca ggaaggagat 2160
tcactttggc agcccacagc acctgtgctt tgctgtcagg caggagcagg tgattcttca 2220
gaactgcacg gaggaaggcc tggccatcca ccagcagcac tgggacttcc aggagaatgg 2280
gatgattgtc cacattcttt ctgggaaatg catggaagct gtggtgcaag aaaacaataa 2340
agatttgtac ctgcgtccgt gtgatggaaa agcccgccag cagtggcgtt ttgaccagat 2400

caatgctgtg gatgaacgat gaatgtcaat gtcagaagga aaagagaatt ttggccatca 2460
aaatccagct ccaagtgaac ttaaagagct tatatatttc atgaagctga tccttttgtg 2520
tgtgtgctcc tggtgttagg agagaaaaaa agctctatga aagaatatag gaagtttctc 2580
cttttcacac cttatttcat tgactgctgg ctgctttaaa aaaaaaaaaa aggatccatt 2640
gtaccgttgt cttcatcact gggaaatgat tattacatag tacagaagat tctttgtttt 2700
tctccactga gcacttaaca attgctttct ctctggcctg gacattctct ggcagcacct 2760
ccaggataca taaattcaat ggatcaattt atttgtcttc aaatggcctt aacttggatt 2820
gtctgtttgg ccaaccatga aaattaaaga gtgaagcaga tgtaatggcc tgacattcca 2880
aaaactctga attgggttta ttagcacaaa tgttgtgttc atttgttgag ccatatctca 2940
gaagaaggaa agggagctac agaaaggagg tttaggattg cagagaagat gcaagagcac 3000
tttggcccaa ttctccagct caacccagca gctgaaaagc ttcaagagat ctaggaaaag 3060
acattttcat gttaatgaga atttccacca ttgtagagaa tttccttcct actgagaatc 3120
tacctctatt ccccctgccc tagctcttct ctaacttggt taaccataac cataaccaga 3180
ttcccttgca atcgatttct ctttagtcgt tggtgttaga agtaccagca caatttgagc 3240
attcccatta acaaaggtgt tcacagttga gaaactctcc tgccgggcgc ggtggctcat 3300
gcctgtaatt ccagcacttt gggaggcaga gttgggagga tcacctgatg tcaggggttt 3360
gagaccagcc tggtcaacat tgcaaaacct tgtctctact aaaaatacaa aaattagctg 3420
ggcatggtgg cgcatacctg taatcccagc tacttgggag gctgaggcaa gagaatcgct 3480
tgaacccagg aggcagaggt tgcagtgagc tgagatcatg ccattgcact ccaacctggg 3540
tgacagagtg agactccatc tcaaaaaaaa aaaaaaa 3577

<210> 184
<211> 2762
<212> DNA
<213> Homo sapiens

<400> 184
ccgccccgcc ccgccttgcc ccaacccacg atggtctggg agctgcgccc agggcttggc 60
gctggcggcc ccgcaacagc accgagcgtt tcggtcggcg ggcggcggta gcgccccctc 120
tcagagcccc gctcactccc acctcggctc gctccgagtc ggcctgtctg tcgggcccgc 180
cctccccgct cactccctcc gccctcgtgc tcctcccggg gtgcttggca cagcctcgga 240
ttcctccctc tcgctgctcg agtcagtttc cctatcggcg gcagcgggca aggcggcggc 300
ggcggcggcg gcagccgcgg tggcggcgtg gggaacatct cggcagccac cgcgcttctc 360
ccgctggagc gggcgtccag cttggctgcc ctcggtcctt ccctgccacg tttcgggtcg 420
ccctgcaccc cccacccagg ctcgcttctc ttcgaagcgg gaagggcgcc ttgcaggatc 480
ctgccgcccc tccaaccgga tcctgggtct agagctcccc agagcgaggc gctcgccagg 540
actcctgccc cgccaaccct gaccgccggg gggtgccccc gggacgtagc gccgcggaga 600

ggaagcggca aaggggacca tgcggcgcct gactcgtcgg ctggttctgc cagtcttcgg 660
ggtgctctgg atcacggtgc tgctgttctt ctgggtaacc aagaggaagt tggaggtgcc 720
gacgggacct gaagtgcaga cccctaagcc ttcggacgct gactgggacg acctgtggga 780
ccagtttgat gagcggcggt atctgaatgc caaaaagtgg cgcgttggtg acgaccccta 840
taagctgtat gctttcaacc agcgggagag tgagcggatc tccagcaatc gggccatccc 900
ggacactcgc catctgagat gcacactgct ggtgtattgc acggaccttc cacccactag 960
catcatcatc accttccaca acgaggcccg ctccacgctg ctcaggacca tccgcagtgt 1020
attaaaccgc acccctacgc atctgatccg ggaaatcata ttagtggatg acttcagcaa 1080
tgaccctgat gactgtaaac agctcatcaa gttgcccaag gtgaaatgct tgcgcaataa 1140
tgaacggcaa ggtctggtcc ggtcccggat tcggggcgct gacatcgccc agggcaccac 1200
tctgactttc ctcgacagcc actgtgaggt gaacagggac tggctccagc ctctgttgca 1260
cagggtcaaa gaggactaca cgcgggtggt gtgccctgtg atcgatatca ttaacctgga 1320
caccttcacc tacatcgagt ctgcctcgga gctcagaggg gggtttgact ggagcctcca 1380
cttccagtgg gagcagctct ccccagagca gaaggctcgg cgcctggacc ccacggagcc 1440
catcaggact cctatcatag ctggagggct cttcgtgatc gacaaagctt ggtttgatta 1500
cctggggaaa tatgatatgg acatggacat ctggggtggg gagaactttg aaatctcctt 1560
ccgagtgtgg atgtgcgggg gcagcctaga gatcgtcccc tgcagccgag tggggcacgt 1620
cttccggaag aagcacccct acgttttccc tgatggaaat gccaacacgt atataaagaa 1680
caccaagcgg acagctgaag tgtggatgga tgaatacaag caatactatt acgctgcccg 1740
gccattcgcc ctggagaggc ccttcgggaa tgttgagagc agattggacc tgaggaagaa 1800
tctgcgctgc cagagcttca agtggtacct ggagaatatc taccctgaac tcagcatccc 1860
caaggagtcc tccatccaga agggcaatat ccgacagaga cagaagtgcc tggaatctca 1920
aaggcagaac aaccaagaaa ccccaaacct aaagttgagc ccctgtgcca aggtcaaagg 1980
cgaagatgca aagtcccagg tatgggcctt cacatacacc cagcagatcc tccaggagga 2040
gctgtgcctg tcagtcatca ccttgttccc tggcgcccca gtggttcttg tcctttgcaa 2100
gaatggagat gaccgacagc aatggaccaa aactggttcc cacatcgagc acatagcatc 2160
ccacctctgc ctcgatacag atatgttcgg tgatggcacc gagaacggca aggaaatcgt 2220
cgtcaaccca tgtgagtcct cactcatgag ccagcactgg gacatggtga gctcttgagg 2280
acccctgcca gaagcagcaa gggccatggg gtggtgcttc cctggaccag aacagactgg 2340
aaactgggca gcaagcagcc tgcaaccacc tcagacatcc tggactggga ggtggaggca 2400
gagcccccca ggacaggagc aactgtctca gggaggacag aggaaaacat cacaagccaa 2460
tggggctcaa agacaaatcc cacatgttct caaggccgtt aagttccagt cctggccagt 2520
cattccctga ttggtatctg gagacagaaa cctaatggga agtgtttatt gttccttttc 2580
ctacaaagga agcagtctct ggaggccaga aagaaaagcc ttctttttca ctaggccagg 2640

actacattga gagatgaaga atggaggttg tttccaaaag aaataaagag aaacttagaa 2700

gttgtctctg gaaaaaaaaa aaaaaaaaaa aaaaaaaaaa aaaaaaaaaa aaaaaaaaaa 2760

aa 2762

<210> 185
<211> 1310
<212> DNA
<213> Homo sapiens

<400> 185
aaattgagcc cgcagcctcc cgcttcgctc tctgctcctc ctgttcgaca gtcagccgca 60

tcttcttttg cgtcgccagc cgagccacat cgctcagaca ccatggggaa ggtgaaggtc 120

ggagtcaacg gatttggtcg tattgggcgc ctggtcacca gggctgcttt taactctggt 180

aaagtggata ttgttgccat caatgacccc ttcattgacc tcaactacat ggtttacatg 240

ttccaatatg attccaccca tggcaaattc catggcaccg tcaaggctga gaacgggaag 300

cttgtcatca atggaaatcc catcaccatc ttccaggagc gagatccctc caaaatcaag 360

tggggcgatg ctggcgctga gtacgtcgtg gagtccactg gcgtcttcac caccatggag 420

aaggctgggg ctcatttgca ggggggagcc aaaagggtca tcatctctgc cccctctgct 480

gatgccccca tgttcgtcat gggtgtgaac catgagaagt atgacaacag cctcaagatc 540

atcagcaatg cctcctgcac caccaactgc ttagcacccc tggccaaggt catccatgac 600

aactttggta tcgtggaagg actcatgacc acagtccatg ccatcactgc cacccagaag 660

actgtggatg gcccctccgg gaaactgtgg cgtgatggcc gcggggctct ccagaacatc 720

atccctgcct ctactggcgc tgccaaggct gtgggcaagg tcatccctga gctgaacggg 780

aagctcactg gcatggcctt ccgtgtcccc actgccaacg tgtcagtggt ggacctgacc 840

tgccgtctag aaaaacctgc caaatatgat gacatcaaga aggtggtgaa gcaggcgtcg 900

gagggccccc tcaagggcat cctgggctac actgagcacc aggtggtctc ctctgacttc 960

aacagcgaca cccactcctc cacctttgac gctggggctg gcattgccct caacgaccac 1020

tttgtcaagc tcatttcctg gtatgacaac gaatttggct acagcaacag ggtggtggac 1080

ctcatggccc acatggcctc caaggagtaa gacccctgga ccaccagccc cagcaagagc 1140

acaagaggaa gagagagacc ctcactgctg gggagtccct gccacactca gtcccccacc 1200

acactgaatc tcccctcctc acagttgcca tgtagacccc ttgaagaggg gaggggccta 1260

gggagccgca ccttgtcatg taccatcaat aaagtaccct gtgctcaacc 1310

<210> 186
<211> 2484
<212> DNA
<213> Homo sapiens

<400> 186
catcgaacaa acaactgaga tcctgttgtg cctatcacct gttgaagttg ccagtcttaa 60

ggaaggaatc aatttctttc gcaataagag cactggcaaa gactacgtct tgtacaagaa 120

taagagccga ctgagggcat gcaagaatat gtgcaagcac caaggaggcc tgttcataaa 180
agatatcgag gatttagccg gaagttgttg aaatggatga aaacaacgga cttttgcttt 240
tagaactgaa tcctcctaac ccttgggact tacagcccag atctcctgaa gagttggctt 300
ttggagaagt acagataaca tatctcactc atgcctgcat ggacctcaag ttaggagaca 360
agagaatggt gtttgaccct tggttaatcg gtcctgcttt tgcccgtgga tggtggttgc 420
tccatgagcc tccatctgat tggctggaga ggctgtgcca ggcagacctc atttacatca 480
gtcatctgca ctcagaccac ctgagttacc ccacactgaa aaagcttgct gggagaagac 540
cagatattcc catttatgtt ggaaacacag aaaggcctgt attttggaat ctgaatcaga 600
gcggtgtcca gttgactaat atcaatgtcg tgccatttgg aatatggcag caggtggaca 660
aaaatcttcg attcatgatc ttgatggacg gtgttcatcc tgagatggac acttgcatta 720
ttgtggagta caaaggtcat aaaatactca atatagtaga ctgcaccaga cccaatgggg 780
gaaggctgcc tatgaaggtt gctctaatga tgagtgattt tgctggagga gcatcaggct 840
ttccaatgac tttcagtggt ggaaaattta cggaggaatg gaaagcccaa ttcattaaaa 900
cagaaaggaa gaagctcctg aactacaagg ctcggctggt gaagaacctg caaccccgaa 960
tttattgtcc ctttgctggg tattttgtgg aatctcaccc atcagacaag tacatcaagg 1020
aaacaaacac caaaaatgac ccaaatgaac tcaacaatct tatcaagaaa aactctgatg 1080
tgataacatg gacccctcga ccgggagcca cccttgatct gggaagaatg ctgaaggatc 1140
gaacagacag caagggcatc atagagcctc cagaggggac aaaaatttac aaggattcct 1200
gggattttga accttatttg gaaatcttga atgctgctct aggagatgaa atatttcttc 1260
actcatcctg gataaaagaa tacttcactt gggctggatt taaagattac aaccttgtgg 1320
tcaggatgat tgagacagat gaggacttca atccttttcc tggaggatat gactatttgg 1380
ttgacttttt agatttatcc ttcccaaaag aaagaccaca acgagaacat ccctatgagg 1440
aaatccatag ccgggtggat gtcatcagac acgtggtgaa gaatggtcta ctctgggatg 1500
agttgtatat aggattccaa acacggctcc agcgggatcc tgacatatac catcacctgt 1560
tttggaatca ttttcaaata aaactccccc tcacaccacc caactggaag tcattcctga 1620
tgtgctgtga gcagaatggg cctgtgattt tgcaagaatg caaaaccaca tgaaaatttc 1680
aagaattcac tgatctgatg caaaataaaa atttatcatt acatcttgaa cccaggaagc 1740
ttacagcaaa gagactatgc tttatgacgt cagcaataga taattccacg ttgcctttgt 1800
gatttgtata tatagcttac atttgtggat cactacatag ccagattcaa aaatatttta 1860
cttgttccat ccacagttct ctacagaaag aaccaatgaa cccaatagga acaaattctc 1920
tgtggaaaac aaagcatagc tgtagtagat acgaatccaa tcacagagga aacaggaaga 1980
gaaaaacatc caagactaca gtgaaaactg gaaatggtct gttttcgtga tattcgtatg 2040
attaagatgc aaattttttc ttaggaaaat gtgattgtta actagcattc tgttttacat 2100
gttgacattt ctaacacaca caccactgat ttgaacttca aaatttattt tctgattata 2160

tatgctaggt ctgattctga agatacaaga attcaatggt ggaatttgtc tcctgaaatt 2220

tctagattca taattagaag tgtttattac caacttctca cttagattaa catcagtgat 2280

gtttgattat tctggttaac cgctcacatg cataacaata atgctagaaa ttcaggaatt 2340

ataattttgt gatttatact gtgtggtaaa ttattcagtt ctcactgcaa ataaatataa 2400

tgtatatcaa tgtaatgtag gtcagaatgt ctatggtgag caaataaaag attattttaa 2460

aatatgaaaa aaaaaaaaaa aaaa 2484

<210> 187
<211> 2836
<212> DNA
<213> Homo sapiens

<400> 187
ccagctcctc tgctaccatg aatagggccc ctctgaagcg gtccaggatc ctgcacatgg 60

cgctgaccgg ggcctcagac ccctctgcag aggcagaggc caacggggag aagccctttc 120

tgctgcgggc attgcagatc gcgctggtgg tctccctcta ctcggtcacc tccatctcca 180

tggtgttcct taataagtac ctgctggaca gcccctccct gcggctggac acccccatct 240

tcgtcacctt ctaccagtgc ctggtgacca cgctgctgtg caaaggcctc agcgctctgg 300

ccgcctgctg ccctggtgcc gtggacttcc ccagcttgcg cctggacctc agggtggccc 360

gcagcgtcct gcccctgtcg gtggtcttca tcggcatgat caccttcaat aacctctgcc 420

tcaagtacgt cggtgtggcc ttctacaatg tgggccgctc actcaccacc gtcttcaacg 480

tgctgctctc ctacctgctg ctcaagcaga ccacctcctt ctatgccctg ctcacctgcg 540

gtatcatcat cgggggcttc tggcttggtg tggaccagga gggggcagaa ggcaccctgt 600

cgtggctggg caccgtcttc ggcgtgctgg ctagcctctg tgtctcgctc aacgccatct 660

acaccacgaa ggtgctcccg gcggtggacg gcagcatctg gcgcctgact ttctacaaca 720

acgtcaacgc ctgcatcctc ttcctgcccc tgctcctgct gctcggggag cttcaggccc 780

tgcgtgactt tgcccagctg ggcagtgccc acttctgggg gatgatgacg ctgggcggcc 840

tgtttggctt tgccatcggc tacgtgacag gactgcagat caagttcacc agtccgctga 900

cccacaatgt gtcgggcacg gccaaggcct gtgcccagac agtgctggcc gtgctctact 960

acgaggagac caagagcttc ctctggtgga cgagcaacat gatggtgctg ggcggctcct 1020

ccgcctacac ctgggtcagg ggctgggaga tgaagaagac tccggaggag cccagcccca 1080

aagacagcga gaagagcgcc atgggggtgt gagcaccaca ggcaccctgg atggcccggc 1140

cccggggccc gtacacaggc ggggccagca cagtagtgaa ggcggtctcc tggaccccag 1200

aagcgtgctg tggtgtggac tgggtgctac ttatagaccc aatcagaata cggtggttga 1260

gaaggaacca gtgtttacaa gtaatatcag aaagttgaag gaaccagtgt ttacaagtaa 1320

taccagaaag ttgccaaacc cttctctatc ctctcgtatt tctgagtttt tgtccttccc 1380

gagggagcac cctagtgaga gttgaacccc ttccttctgc ctccagggcc tgtctgcctc 1440

cacatcactc tgaggacagg gacaggcaac aaccttgaag ggacagcaat ggcaaagcca 1500

caaaggcttc actgtactca ggggagatgg ccctaccaca gccacctgga gagggttggg 1560
aagccttccg tctgcggtgg gcaggcagcc tcaccctggg cccacagacc ggccttcctt 1620
tggaggaaag tgtggcctgg actgagggag gaaatgagcg agttccctct gacaccagca 1680
gatccccagg ggctgctggg cagtggcctg ggaatggggt ggattgtgag aaagtgctca 1740
ccatctatac accctgtatg tccagctttt gaacacaagg gaaccatgct tctcttagag 1800
gttaagcagg gtcattaaca tcctccccca gtccctaaca tcacattgtc ctgcgtggct 1860
cctctggccc tgagtggcac ctgtccctct ggtctcccag cacctggccc aggtaacagc 1920
cttctgaaag cagagccaag gagctgcttc tctcttctcc cagttctacc tccccagaag 1980
ccttcctccc caggtggggc tgatggagca agggtccaga ctaggagcct tccaccccag 2040
ctgtgtctgg cgcccctaga tctctgcaag ggaggtgtta cagctggttc tgagccgctt 2100
gccttgtgat ggtaagacac caacctttac attcttccct gaggttgtgg ctgacagagc 2160
ctgcttggcc ccactgttag tccagcgagc tcctatatca aaatgccgta ggccgggtgg 2220
cttacaaaca acagaaacgt attgctcaca gtcctggggg gctgggacgc ccaagatcaa 2280
gaggccagca gattcggact ccgctgaggg ctgtttcccg atccatagat ggtgccttct 2340
cgctgtatcc tcaatggtag aagcacaaac aagcaagctc cttcctgcct cttttataag 2400
gactccaacc ctgttcatga gggttccgcc cccatgaccc aatcagctcc aaaggcccca 2460
cctcctaata ctgtcacctt gggggtgaga attccaatgt gaatttgcag ggggagtggg 2520
ggacacacac aaatttcggg gccataccac ccttcaccac accctcctgc gctcagggtg 2580
gcttgcagtc cctggccctt ctggtgggca tttggtatgt cctttctctt ggggtgattt 2640
ctgatgtttt tactctatat agtgaaaagc tagggagagc gggtcttctc ccccctccct 2700
ctccagtccc ctcacaatcc cagatgggtt ctaatgcagc tgctggggcc tgatgccctg 2760
agttgtttgt gattcaataa agaatccata agaaaaaaaa aaaaaaaaaa aaaaaaaaaa 2820
aaaaaaaaaa aaaaaa 2836

<210> 188
<211> 3319
<212> DNA
<213> Homo sapiens

<400> 188
gcttccggcc gcggccctgg cgcgtggtct gcgcgcgccc ggccgtgcgt gcggatgcgg 60
ggaggctgcg tgtgtgcgca gggagagaac gccggccacc ttcccgcttc cgagctgggt 120
gcgcgccgag cacaggagat tgcctgcgtt taggaggtgg ctgcgttgtg ggaaaagcta 180
tcaaggaaga aattgccaaa ccatgtcttt ttttctgttt tcagagtagt tcacaacaga 240
tctgagtgtt ttaattaagc atggaataca gaaaacaaca aaaaacttaa gctttaattt 300
catctggaat tccacagttt tcttagctcc ctggacccgg ttgacctgtt ggctcttccc 360
gctggctgct ctatcacgtg gtgctctccg actactcacc ccgagtgtaa agaaccttcg 420

gctcgcgtgc ttctgagctg ctgtggatgg cctcggctct ctggactgtc cttccgagta 480
ggatgtcact gagatccctc aaatggagcc tcctgctgct gtcactcctg agtttctttg 540
tgatgtggta cctcagcctt ccccactaca atgtgataga acgcgtgaac tggatgtact 600
tctatgagta tgagccgatt tacagacaag actttcactt cacacttcga gagcattcaa 660
actgctctca tcaaaatcca tttctggtca ttctggtgac ctcccaccct tcagatgtga 720
aagccaggca ggccattaga gttacttggg gtgaaaaaaa gtcttggtgg ggatatgagg 780
ttcttacatt tttcttatta ggccaagagg ctgaaaagga agacaaaatg ttggcattgt 840
ccttagagga tgaacacctt ctttatggtg acataatccg acaagatttt ttagacacat 900
ataataacct gaccttgaaa accattatgg cattcaggtg ggtaactgag ttttgcccca 960
atgccaagta cgtaatgaag acagacactg atgttttcat caatactggc aatttagtga 1020
agtatctttt aaacctaaac cactcagaga agtttttcac aggttatcct ctaattgata 1080
attattccta tagaggattt taccaaaaaa cccatatttc ttaccaggag tatcctttca 1140
aggtgttccc tccatactgc agtgggttgg gttatataat gtccagagat ttggtgccaa 1200
ggatctatga aatgatgggt cacgtaaaac ccatcaagtt tgaagatgtt tatgtcggga 1260
tctgtttgaa tttattaaaa gtgaacattc atattccaga agacacaaat cttttctttc 1320
tatatagaat ccatttggat gtctgtcaac tgagacgtgt gattgcagcc catggctttt 1380
cttccaagga gatcatcact ttttggcagg tcatgctaag gaacaccaca tgccattatt 1440
aacttcacat tctacaaaaa gcctagaagg acaggatact ttgtggaaag tgttaaataa 1500
agtaggtact gtggaaaatt catggggagg tcagtgtgct ggcttacact gaactgaaac 1560
tcatgaaaaa cccagactgg agactggagg gttacacttg tgatttatta gtcaggccct 1620
tcaaagatga tatgtggagg aattaaatat aaaggaattg gaggtttttg ctaaagaaat 1680
taataggacc aaacaatttg gacatgtcat tctgtagact agaatttctt aaaagggtgt 1740
tactgagtta taagctcact aggctgtaaa aacaaaacaa tgtagagttt tatttattga 1800
acaatgtagt cacttgaagg ttttgtgtat atcttatgtg gattaccaat ttaaaaatat 1860
atgtagttct gtgtcaaaaa acttcttcac tgaagttata ctgaacaaaa ttttacctgt 1920
ttttggtcat ttataaagta cttcaagatg ttgcagtatt tcacagttat tattatttaa 1980
aattacttca actttgtgtt tttaaatgtt ttgacgattt caatacaaga taaaaaggat 2040
agtgaatcat tctttacatg caaacatttt ccagttactt aactgatcag tttattattg 2100
atacatcact ccattaatgt aaagtcatag gtcattattg catatcagta atctcttgga 2160
ctttgttaaa tattttactg tggtaatata gagaagaatt aaagcaagaa aatctgaagt 2220
attgtcttgt ttttaaaaaa tacagttcct agtgttttta gaagtcactt aatttgtctc 2280
atttttccac ctggaaatta ggaataatgt agaatgcaag gcagtaattt ccttttggaa 2340
aggactctga aggcagaaaa gaagggagag aacctcatgg gcagaatatt ataaaaagag 2400
tgtcatattc cagcatttga attggaaaga gaagagtgaa gatccaagtt gcattattaa 2460

tctgccctgt gttttttcct tttaacaatc agtttgagct gctgctgtta tgagtttctc 2520

atcaagatga aagccctaat atgtaaagtc aaatccgatt taaattttgt gcttttatag 2580

aaagaaattt cttcatagac gtggtgatat atcatttgtt ggacctgcta atagtaggtc 2640

aaaggggagc actccttgcc ccctgttcct gggtttatgc agttttcttt ttagagttta 2700

tatagggcaa gtggttcttt ttctctgaat tacaggatgg aaaaaggtca tatcctttgt 2760

caggaaatat aaacttgaaa gtatgtagtc agctcttgta atactcatat ttatgattgt 2820

cctatatgaa aaacaacttc agttaaaact ataatgtgtg attctgtata acaaggtgat 2880

gtctgtttcc cagggctcag acctaatcca gttataataa aatcaattaa atgaaatatt 2940

ctatagaatc gatctatgcc cttgttaatc tccatccata taggagtcac gttctttaag 3000

acagatggtg gtagttattt ttgtggcatg gttagatttg actggttttg cagaagatta 3060

cagttatgta ctgcataatg acatatacaa tagtggtccc ataaaattat aatggagcag 3120

aaaatctatt gcctcatgat gttttagccg ttttaatgtc atagcctagt gcattactca 3180

cgtgtctgtg gagatgctgg tgtaaacaaa cctactacac tgccagttgt ataaaagtac 3240

agcacattca gttatgtaca gtatgtaata ctgataatga caataaatga caccggtttg 3300

tgtatttact ttttataaa 3319

<210> 189
<211> 4620
<212> DNA
<213> Homo sapiens

<400> 189
ccggaggatg tgcagctgcg gcggcggcgc cggctacgaa gaggacgggg acaggcgccg 60

tgcgaaccga gcccagccag ccggaggacg cgggcagggc gggacgggag cccggactcg 120

tctgccgccg ccgtcgtcgc cgtcgtgccg gccccgcgtc cccgcgcgcg agcgggagga 180

gccgccgcca cctcgcgccc gagccgccgc tagcgcgcgc cgggcatggt cccctcttaa 240

aggcgcaggc cgcggcggcg ggggcgggcg tgcggaacaa agcgccggcg cggggcctgc 300

gggcggctcg ggggccgcga tgggcgcggc gggcccgcgg cggcggcggc gctgcccggg 360

ccgggcctcg cggcgctagg gcgggctggc ctccgcgggc gggggcagcg ggctgagggc 420

gcgcggagcc tgcggcggcg gcggcggcgg cggcggcggc ccggcgggcg gagcggcgcg 480

ggcatggccg cgcgcggccg gcgcgcctgg ctcagcgtgc tgctcgggct cgtcctgggc 540

ttcgtgctgg cctcgcggct cgtcctgccc cgggcttccg agctgaagcg agcgggccca 600

cggcgccgcg ccagccccga gggctgccgg tccgggcagg cggcggcttc ccaggccggc 660

ggggcgcgcg gcgatgcgcg cggggcgcag ctctggccgc ccggctcgga cccagatggc 720

ggcccgcgcg acaggaactt tctcttcgtg ggagtcatga ccgcccagaa atacctgcag 780

actcgggccg tggccgccta cagaacatgg tccaagacaa ttcctgggaa agttcagttc 840

ttctcaagtg agggttctga cacatctgta ccaattccag tagtgccact acggggtgtg 900

gacgactcct acccgcccca gaagaagtcc ttcatgatgc tcaagtacat gcacgaccac 960

tacttggaca agtatgaatg gtttatgaga gcagatgatg acgtgtacat caaaggagac 1020
cgtctggaga acttcctgag gagtttgaac agcagcgagc ccctctttct tgggcagaca 1080
ggcctgggca ccacggaaga aatgggaaaa ctggccctgg agcctggtga gaacttctgc 1140
atgggggggc ctggcgtgat catgagccgg gaggtgcttc ggagaatggt gccgcacatt 1200
ggcaagtgtc tccgggagat gtacaccacc catgaggacg tggaggtggg aaggtgtgtc 1260
cggaggtttg caggggtgca gtgtgtctgg tcttatgaga tgcagcagct tttttatgag 1320
aattacgagc agaacaaaaa ggggtacatt agagatctcc ataacagtaa aattcaccaa 1380
gctatcacat tacaccccaa caaaaaccca ccctaccagt acaggctcca cagctacatg 1440
ctgagccgca agatatccga gctccgccat cgcacaatac agctgcaccg cgaaattgtc 1500
ctgatgagca aatacagcaa cacagaaatt cataaagagg acctccagct gggaatccct 1560
ccctccttca tgaggtttca gccccgccag cgagaggaga ttctggaatg ggagtttctg 1620
actggaaaat acttgtattc ggcagttgac ggccagcccc ctcgaagagg aatggactcc 1680
gcccagaggg aagccttgga cgacattgtc atgcaggtca tggagatgat caatgccaac 1740
gccaagacca gagggcgcat cattgacttc aaagagatcc agtacggcta ccgccgggtg 1800
aaccccatgt atggggctga gtacatcctg gacctgctgc ttctgtacaa aaagcacaaa 1860
gggaagaaaa tgacggtccc tgtgaggagg cacgcgtatt tacagcagac tttcagcaaa 1920
atccagtttg tggagcatga ggagctggat gcacaagagt tggccaagag aatcaatcag 1980
gaatctggat ccttgtcctt tctctcaaac tccctgaaga agctcgtccc ctttcagctc 2040
cctgggtcga agagtgagca caaagaaccc aaagataaaa agataaacat actgattcct 2100
ttgtctgggc gtttcgacat gtttgtgaga tttatgggaa actttgagaa gacgtgtctt 2160
atccccaatc agaacgtcaa gctcgtggtt ctgcttttca attctgactc caaccctgac 2220
aaggccaaac aagttgaact gatgagagat taccgcatta agtaccctaa agccgacatg 2280
cagattttgc ctgtgtctgg agagttttca agagccctgg ccctggaagt aggatcctcc 2340
cagtttaaca atgaatcttt gctcttcttc tgcgacgtcg acctcgtgtt tactacagaa 2400
ttccttcagc gatgtcgagc aaatacagtt ctgggccaac aaatatattt tccaatcatc 2460
ttcagccagt atgacccaaa gattgtttat agtgggaaag ttcccagtga caaccatttt 2520
gcctttactc agaaaactgg cttctggaga aactatgggt ttggcatcac gtgtatttat 2580
aagggagatc ttgtccgagt gggtggcttt gatgtttcca tccaaggctg ggggctggag 2640
gatgtggacc ttttcaacaa ggttgtccag gcaggtttga agacgtttag gagccaggaa 2700
gtaggagtag tccacgtcca ccatcctgtc ttttgtgatc ccaatcttga ccccaaacag 2760
tacaaaatgt gcttggggtc caaagcatcg acctatgggt ccacacagca gctggctgag 2820
atgtggctgg aaaaaaatga tccaagttac agtaaaagca gcaataataa tggctcagtg 2880
aggacagcct aatgtccagc tttgctggaa aagacgtttt taattatcta atttattttt 2940
caaaaatttt ttgtatgatc agtttttgaa gtccgtatac aaggatatat tttacaagtg 3000

gttttcttac ataggactcc tttaagattg agctttctga acaagaaggt gatcagtgtt 3060
tgcctttgaa cacatcttct tgctgaacat tatgtagcag acctgcttaa ctttgacttg 3120
aaatgtacct gatgaacaaa acttttttaa aaaaatgttt tcttttgaga ccctttgctc 3180
cagtcctatg gcagaaaacg tgaacattcc tgcaaagtat tattgtaaca aaacactgta 3240
actctggtaa atgttctgtt gtgattgtta acattccaca gattctacct tttgtgtttt 3300
gttttttttt tttacaattg ttttaaagcc atttcatgtt ccagttgtaa gataaggaaa 3360
tgtgataata gctgtttcat cattgtcttc aggagagctt tccagagttg atcatttccc 3420
ctcatggtac tctgctcagc atggccacgt aggttttttg tttgttttgt tttgttcttt 3480
ttttgagacg gagtctcact ctgttaccca ggctggaatg cagtggcgca atcttggctc 3540
actttaacct ccacttccct ggttcaagca attcccctgc ctttgcctcc cgagtagctg 3600
ggattacagg cacacaccac cacgcccagc tagttttttt gtatttttag tagagacggg 3660
gtttcaccat gcaagcccag ctggccacgt aggttttaaa gcaaggggcg tgaagaaggc 3720
acagtgaggt atgtggctgt tctcgtggta gttcattcgg cctaaataga cctggcatta 3780
aatttcaaga aggatttggc attttctctt cttgaccctt ctctttaaag ggtaaaatat 3840
taatgtttag aatgacaaag atgaattatt acaataaatc tgatgtacac agactgaaac 3900
acacacacat acaccctaat caaaacgttg gggaaaaatg tatttggttt tgttcctttc 3960
atcctgtctg tgttatgtgg gtggagatgg ttttcattct ttcattactg ttttgtttta 4020
tcctttgtat ctgaaatacc tttaatttat ttaatatctg ttgttcagag ctctgccatt 4080
tcttgagtac ctgttagtta gtattattta tgtgtatcgg gagtgtgttt agtctgtttt 4140
atttgcagta aaccgatctc caaagatttc cttttggaaa cgctttttcc cctccttaat 4200
ttttatattc cttactgttt tactaaatat taagtgttct ttgacaattt tggtgctcat 4260
gtgttttggg gacaaaagtg aaatgaatct gtcattatac cagaaagtta aattctcaga 4320
tcaaatgtgc cttaataaat ttgttttcat ttagatttca aacagtgata gacttgccat 4380
tttaatacac gtcattggag ggctgcgtat ttgtaaatag cctgatgctc atttggaaaa 4440
ataaaccagt gaacaatatt tttctattgt acttttcgaa ccattttgtc tcattattcc 4500
tgttttagct gaagaattgt attacatttg gagagtaaaa aacttaaaca cgaaaaaaaa 4560
aaaaaaaaaa aaaaaaaaaa aaaaaaaaaa aaaaaaaaaa aaaaaaaaaa aaaaaaaaaa 4620

<210> 190
<211> 2546
<212> DNA
<213> Homo sapiens

<400> 190
   gacggcaaat ggcggacttc gacacctacg acgatcgggc ctacagcagc ttcggcggcg 60
   gcagagggtc ccgcggcagt gctggtggcc atggttcccg tagccagaag gagttgccca 120
   cagagccccc ctacacagca tacgtaggaa atctaccttt caatacggtt cagggcgaca 180
   tagatgctat ctttaaggat ctcagcataa ggagtgtacg gctagtcaga gacaaagaca 240
   cagataaatt taaaggattc tgctatgtag aattcgatga agtggattcc cttaaggaag 300
   ccttgacata cgatggtgca ctgttgggcg atcggtcact tcgtgtggac attgcagaag 360
   gcagaaaaca agataaaggt ggctttggat tcagaaaagg tggaccagat gacagaggaa 420
   tgggtagctc tcgagaatct agaggtggat gggattcccg ggatgacttc aattctggct 480
   tcagggatga cttcttaggg ggcaggggag gtagtcgccc aggcgaccgg cgaacaggcc 540
   cccccatggg cagccgcttc agagatggcc ctcccctccg tggatccaac atggatttca 600
   gagaacccac agaagaggaa agagcacaga gaccacgact ccagcttaaa cctcgaacag 660
   tcgcgacgcc cctcaatcaa gtagccaatc ccaactctgc tatcttcggg ggtgccaggc 720
   ctagagagga agtcgttcaa aaggagcaag aatgagcctg cggttgggag ggaatggggc 780
   gtggggggtt agagcaggac cacagcctgg tgagtccccg ggcagccgtc ctgcagccgc 840
   cactcctgcg cctgccattg gcctcctcac agcggaaaca cagcttgtga gtgcatgtca 900
   gctgttaaca agtggttttt agtacattct gggctttgct gtatctatct agtgcctgtt 960
   tgtgcgtttt tttctttctt ccgctgcttc cccattttcc ttctgtcctt tttctcctgc 1020
   tccttgtttt cccagcagca catggggttc ctcggaggag cagaggtggc cgccgtgggg 1080
   gggcgtttgg gctgcggtgc tgcgtcattt ttcctttgct ttctctttac tttagacact 1140
   ggcccaactc caggcgtttc ctttcattcc ctcagtgctt ctcttctgac ctgcatgttg 1200
   agttctgtat tgctggggct tccaacaaaa accagagtca ctgacagagg gaacagcaga 1260
   gaccttgttg gtattcagct gtgatggata tagagaatca gaggcacctt gttttcacaa 1320
   ctaggataaa aatatctgca gggtcctttc cattcctatt tagagggagt cctggctcca 1380
   tgaccccctc ccgagtggac tgtccaagca gataggctca cacgagaaac agtgaggctg 1440
   aaaggggggg ctatggaaga gcggtaggga gtccacggag aagatgcagt gaatgcttgc 1500
   atgcattcac acgtgtgtgt gtcccagcta gttcactcct ttcgccgtgc gtggtggagg 1560
   ctggcctctc tggctgggtg cagtgaatgg ccagcgggtt tcttttctgc tgggccaagg 1620
   cgctttgggg gtggaggggg tggtgctggt gctgcactgg gctgactgcg gcgctgacgc 1680
   agcgtttccc cccatccctg ttgcctgtgt gttgtgtgga tctgttccta gtataggcaa 1740
   cataatgaga tactgtgctt cccacctccc cttcagttca gagccaaaat gggtctagaa 1800
   tctggcactt tactcatttc ctttgataaa ttgtactatg cagagctgtc aggaaccttc 1860
   agatagcagt agaggactgc agctgtctag gtctgcggcc acatcttggg gacacactgg 1920
   actgttccca tgtgcagggt tcagcagtta tgtgggagtg ctaggggtta ggcttttgag 1980
   cttgaacgcc tgcgtgtgaa cagatgaaaa atccttcagt acccaagtcc cagtctgtcc 2040
   tatggggagc agtttggggg cggccggcag caggagcctg ggaaagaggc cctcgccagg 2100
   tgatggcagg gccagggtgg cctggggcac ccagcggaat gtgcttagta tttggtcacc 2160
   agccgtcatc ctgggctttt cctactgtgt cttgttacaa ggcctcagca atccacagaa 2220
   ctctctctcc ttccttccac ctgtcagctt ctctgcttct gagataagaa ccatttgtgt 2280
   aacaccaaca cttaacttca gaaagacatg cattatgtgg tgtaatcaaa cccgatgctt 2340
   tcagatgacc tacttacatc ttcaatgtgg ataagataaa gaacaaaaca catgcatcta 2400
   aactgctggg caatccagtt gacttttaaa tgtaagaatg gaattccaaa cacttaacac 2460
   attcagctat atgacagaaa gtaaatctat ggatatggta ttttgtgaat gatcttttaa 2520
   ataaaagaaa accttacgta atattt 2546

<210> 191
<211> 2956
<212> DNA
<213> Homo sapiens

<400> 191
cgaggcgcgg ctccggggat tcggctcggg ccgctggctc tgctctgcgg ggagggagcg 60

ggcccgcccg cggggcccga gccctccgga tccgccccct ccccggtccc gccccctcgg 120

agactcctct ggctgctctg ggggttcgcc ggggccgggg acccgcggtc cgggcgccat 180

gcgggcatcg ctgctgctgt cggtgctgcg gcccgcaggg cccgtggccg tgggcatctc 240

cctgggcttc accctgagcc tgctcagcgt cacctgggtg gaggagccgt gcggcccagg 300

cccgccccaa cctggagact ctgagctgcc gccgcgcggc aacaccaacg cggcgcgccg 360

gcccaactcg gtgcagcccg gagcggagcg cgagaagccc ggggccggcg aaggcgccgg 420

ggagaattgg gagccgcgcg tcttgcccta ccaccctgca cagcccggcc aggccgccaa 480

aaaggccgtc aggacccgct acatcagcac ggagctgggc atcaggcaga ggctgctggt 540

ggcggtgctg acctctcaga ccacgctgcc cacgctgggc gtggccgtga accgcacgct 600

ggggcaccgg ctggagcgtg tggtgttcct gacgggcgca cggggccgcc gggccccacc 660

tggcatggca gtggtgacgc tgggcgagga gcgacccatt ggacacctgc acctggcgct 720

gcgccacctg ctggagcagc acggcgacga ctttgactgg ttcttcctgg tgcctgacac 780

cacctacacc gaggcgcacg gcctggcacg cctaactggc cacctcagtc tggcctccgc 840

cgcccacctg tacctgggcc ggccccagga cttcatcggc ggagagccca cccccggccg 900

ctactgccac ggaggctttg gggtgctgct gtcgcgcatg ctgctgcaac aactgcgccc 960

ccacctggaa ggctgccgca acgacatcgt cagtgcgcgc cctgacgagt ggctgggtcg 1020

ctgcattctc gatgccaccg gggtgggctg cactggtgac cacgaggggg tgcactatag 1080

ccatctggag ctgagccctg gggagccagt gcaggagggg gaccctcatt tccgaagtgc 1140

cctgacagcc caccctgtgc gtgaccctgt gcacatgtac cagctgcaca aagctttcgc 1200

ccgagctgaa ctggaacgca cgtaccagga gatccaggag ttacagtggg agatccagaa 1260

taccagccat ctggccgttg atggggaccg ggcagctgct tggcccgtgg gtattccagc 1320

accatcccgc ccggcctccc gctttgaggt gctgcgctgg gactacttca cggagcagca 1380

cgctttctcc tgcgccgatg gctcaccccg ctgcccactg cgtggggctg accgggctga 1440

tgtggccgat gttctgggga cagctctaga ggagctgaac cgccgctacc acccggcctt 1500

gcggctccag aagcagcagc tggtgaatgg ctaccgacgc tttgatccgg cccggggtat 1560
ggaatacacg ctggacttgc agctggaggc actgaccccc cagggaggcc gccggcccct 1620
cactcgccga gtgcagctgc tccggccgct gagccgcgtg gagatcttgc ctgtgcccta 1680
tgtcactgag gcctcacgtc tcactgtgct gctgcctcta gctgcggctg agcgtgacct 1740
ggcccctggc ttcttggagg cctttgccac tgcagcactg gagcctggtg atgctgcggc 1800
agccctgacc ctgctgctac tgtatgagcc gcgccaggcc cagcgcgtgg cccatgcaga 1860
tgtcttcgca cctgtcaagg cccacgtggc agagctggag cggcgtttcc ccggtgcccg 1920
ggtgccatgg ctcagtgtgc agacagccgc accctcacca ctgcgcctca tggatctact 1980
ctccaagaag cacccgctgg acacactgtt cctgctggcc gggccagaca cggtgctcac 2040
gcctgacttc ctgaaccgct gccgcatgca tgccatctcc ggctggcagg ccttctttcc 2100
catgcatttc caagccttcc acccagctgt ggccccacca caagggcctg ggcccccaga 2160
gctgggccgt gacactggcc gctttgatcg ccaggcagcc agcgaggcct gcttctacaa 2220
ctccgactac gtggcagccc gtgggcgcct ggcggcagcc tcagaacaag aagaggagct 2280
gctggagagc ctggatgtgt acgagctgtt cctccacttc tccagtctgc atgtgctgcg 2340
ggcggtggag ccggcgctgc tgcagcgcta ccgggcccag acgtgcagcg cgaggctcag 2400
tgaggacctg taccaccgct gcctccagag cgtgcttgag ggcctcggct cccgaaccca 2460
gctggccatg ctactctttg aacaggagca gggcaacagc acctgacccc accctgtccc 2520
cgtgggccgt ggcatggcca caccccaccc cacttctccc ccaaaaccag agccacctgc 2580
cagcctcgct gggcagggct ggccgtagcc agaccccaag ctggcccact ggtcccctct 2640
ctggctctgt gggtccctgg gctctggaca agcactgggg gacgtgcccc cagagccacc 2700
cacttctcat cccaaaccca gtttccctgc cccctgacgc tgctgattcg ggctgtggcc 2760
tccacgtatt tatgcagtac agtctgcctg acgccagccc tgcctctggg ccctgggggc 2820
tgggctgtag aagagttgtt ggggaaggag ggagctgagg agggggcatc tcccaacttc 2880
tcccttttgg accctgccga agctccctgc ctttaataaa ctggccaagt gtgaaaaaaa 2940
aaaaaaaaaa aaaaaa 2956

<210> 192
<211> 3937
<212> DNA
<213> Homo sapiens

<400> 192
gcggcgccct cgtccgcttc caagcccagc acagacttcg gtttctccgc ctccatcccc 60
tttacgagaa gaggagatca aaaaatattg ccctttaggt gctcgtgatg gtcctgtggg 120
ttcgttcagt tgccagaaaa gaatggtaac atgaacagcg gagcgtgaga gcgtcaccgg 180
gggaagggac gagattgatg ggcagagcgc tcacttctca gaggatcgca ttccgaggac 240
cgcgcgagga agggacgtcg cggcgccacc acttcctggc tggactccgc tgacatctgt 300

agcctccttt ccaccaggtt cccggggctc ccagccgctg cctgcagagg agcgtgtaga 360
cagcgcgcga gggtccggca cttcgtggac ctccatccga accggagttc tgcccggatc 420
ccgagtccct ggatcccggt ggagcccgcc ggcaagctgg ctccggaggg agttgaactt 480
gagttcagat ggccaactcc ctctgaatcc tgcagattgg tgctgagcac gcaacaaaag 540
tttgtagctt ctcagtttct ggtgcttcgc aggggagagg aaaggaattt gacattaaac 600
acaagaagca gtcagggagc catctccttt aacttttctt ctctgttacc atttgcaatg 660
aagaggaaac agaagagatt tctgcagatg actttgttgt tcacggtggc tttaattttc 720
ctgcctaacg ttggtctctg gtctctgtac aaggataagc acctggtgaa gtcagcggag 780
cccggggagc agcagacatt tccactgggc ctgggagatg ggcaattcta ttcatggaca 840
gatggtttga gaagaaagga ctggcatgac tatgaaagca ttcagaaaga ggctatgcgc 900
tcaaggaaag gtgaacatgg gaaaccttac ccccttactg aagaggacca tgatgactca 960
gcttacaggg aaaatggttt taatattttc gtcagcaaca atattgctct agagaggtct 1020
ctgccagata ttcgtcatgc taactgtaag cataagatgt atctggaaag gctgccaaac 1080
accagcatca ttatcccatt tcataatgaa ggttggactt cactcctgcg gaccatacac 1140
agtataatta accgaacccc agggagtctg atagcagaaa tcattctagt agatgacttc 1200
agtgagagag aacacctgaa ggataaattg gaagaataca tggcccgatt ttccaaagtg 1260
aggattgttc gcaccaagaa aagagaagga ctcatccgga cccgtctcct gggggcatct 1320
atggccagag gagaagtcct gacattcctg gactcccact gcgaggtcaa tgtgaactgg 1380
ctgcccccac tcctcaacca aattccacta aaccacaaaa ccatcgtgtg tcccatgatc 1440
gatgtcattg accacaatca cttcgggtat gaggcacaag ctggggatgc catgcgagga 1500
gccttcgact gggaaatgta ctacaaaaga atccccatcc ctccagagct ccagagggca 1560
gatcccagcg acccttttga gtctcctgtt atggctggag gtctctttgc tgtggatcgg 1620
aaatggtttt gggaattggg tggctatgat ccaggtttag aaatctgggg aggagaacag 1680
tatgaaatct cttttaaggt ttggatgtgt ggaggagaaa tgtttgatgt tccatgttct 1740
agagttggtc atatctacag gaagtacgtt ccatacaaag ttccatctgg gacaagcctg 1800
gcaagaaacc tgaagcgggt agctgagacc tggatggatg aatttgccga gtacatttac 1860
cagcggcggc cggagtacag gcatctctcc acgggggaca tctctgccca gaaggagctg 1920
cgcaagcagc tcaagtgcaa ggacttcaaa tggttcatgg ctgctgtggc ctgggacgtg 1980
cctaaatact accctccagt ggagcccccg cctgctgcct ggggggagat ccgaaatgtg 2040
gcagcaaatc tctgtgtgga cagcaagcat ggagccaccg gaacagagct gaggctggac 2100
atctgtgtca aggatggttc tgaaagaaca tggtctcatg aacagctttt tacctttgga 2160
tggagagaag atattcgacc cggtgagcca ctgcataccc ggaaattctg ctttgatgcg 2220
atctcacaca acagccccgt tacactctat gactgtcatg gcatgaaggg gaaccagctc 2280
tggggatacc ggaaggacag aacattattc catcctgtga gcaacagctg catggattgc 2340

aaccacgcag agaagaagat tttcatggcc agatgtgacc ctctctctga gactcagcag 2400

tggatttttg aacacattaa tatgactgtt ttagaaaaat ttaaccacca tgccaactcc 2460

tagaaagaag aaaggaagaa agagtgatta cctacaggtt ataaattaaa ttttagccag 2520

catctgggtc gaaggagtca ggaataacat ttcctcgatc caggaaggct ggtttaaaaa 2580

tttgcaaatg ccatgtcaaa gtaggttgtt ttgaagaact tcctgcatct gaagaacttg 2640

gctgagaatc tcaccagctg cttctgagaa ctcgagacta gcagttccct tgctggccaa 2700

gggcaaagat aatttaggga cttttcaaaa caactgctga gctaataaat cctagcattt 2760

ctcaggtcaa atcctgcagt agtgagtagc tatgaatgga tcctattaac tgggtgggag 2820

gggggtgaaa atggagtgca tgactgctct gacaacctga ggatatcaca ggtttagaac 2880

tagccaagct taaggggtga gctgtactct ttggtgccat tctctgacta agaatgggtt 2940

cagcaggttt aacccttaaa agtgctgcag atgatttcag agtgctcaca ctattctgtt 3000

tttttatttt ttatttttaa atgagggtgc aatatctaat gtaagactta aattacataa 3060

tgaaatggac atccagtgtt cccattcttt cgtttatatt tgccattttt ttttcagagc 3120

aaagtttctt ttttatgaaa gttcatgctc catgaatcca ctgaatttct aattcagtgc 3180

ccttctatga atccagttaa aaacaaaagc aaaagcaaaa atctatgcta tcaagttact 3240

ccaaagaaag atttcacaga agcagcaaaa ccatataaga tttaggagag ggacttccct 3300

gggaaatcta tttttacttt tctattattt ttaaaatctt aaaagtctga tatttgtcca 3360

gtcataattt ttattaagca aggaaatgga gtgagtttga tgtaatttgt ttaggagatt 3420

cttaaaccca aataaatcta cactccaagt gatatgtgaa aatggcttag ttcacttcaa 3480

ttctgtttag tcagtggtgg gagcttagaa gtggttttat ttgttcgttt gtttgggttt 3540

tgcttttgtt ttttattttt ttcttgtttt ttttttgtag tgttgaatta atgatgttct 3600

tttatttgga agctcataaa aggaattacc attaataaag tgtttctgac accattttct 3660

gttaatggga caatatttcg gggatgaaga aaggaatgct ccgaaaacta aatggaacac 3720

ttaatcctta aagcaatttt ttactgttca ataatatcac attgggggaa gaaaacacag 3780

gtatcaaggt ggttcttggg aaaaaagaat catgtttatt atatatctct caagtttttt 3840

tggtataaaa atgttttaac tttgctttct ttttggtttt tgtttgaagt tggttttcct 3900

gttaataaaa atttatataa caaaaaaaaa aaaaaaa 3937

<210> 193
<211> 2786
<212> DNA
<213> Homo sapiens

<400> 193
atggctgtgc gctctcgccg cccgtggatg agcgtggcat tagggctggt gctgggcttc 60

accgccgcgt cctggctcat cgcccccagg gtggcggagc tgagcgagag gaagagacgt 120

ggctccagcc tctgctccta ctacggtcgc tctgctgctg gcccccgcgc cggcgctcag 180

cagccgctcc cccagcccca gtcccgacca cggcaggagc agtcgccgcc ccccgcgcgc 240

caggatctcc aggggccacc gctgcccgag gcagcacccg ggatcaccag ttttcgaagc 300
agcccctggc agcagccacc tccgctgcag cagcggcggc gaggacgcga gcctgagggc 360
gcgacggggc ttcccggtgc tccagcggcc gagggggagc ccgaggagga ggacgggggc 420
gcggctgggc agcggagaga cggccggccg gggagtagcc acaacggcag cggggacggg 480
ggcgctgccg ccccgagcgc ccgaccccgg gacttcctgt acgtgggggt gatgaccgcg 540
cagaagtacc tgggcagccg cgcgctggcc gcgcagcgga cctgggcgcg tttcatcccg 600
ggccgcgtgg agttcttttc cagccagcag ccccccaacg ccggccagcc cccgccaccc 660
ctgcctgtca tcgcgctacc gggtgtggac gactcctatc ctccccagaa aaagtccttc 720
atgatgatca agtacatgca cgaccactac ctggacaagt atgagtggtt catgcgcgcc 780
gacgacgatg tctacatcaa aggtgacaaa ttagaagagt ttcttagatc gctaaacagc 840
agtaagcctc tctacctggg acagactggc ctggggaata ttgaagagct tggaaagctg 900
ggactggagc ctggggaaaa cttctgtatg ggaggacctg gcatgatctt tagccgagaa 960
gttctcagga ggatggtgcc acatattggt gaatgcctta gagaaatgca cacgactcat 1020
gaggatgtgg aagtaggaag atgcgttcgc cgctttggta gggctcagtg tgtgtggtct 1080
tttcagatgc aacaactgtt ccatgaaaat tatgaacaca atcggaaggg ttacatccaa 1140
gaccttcaca atagcaaaat ccatgcagcc ataacacttc atcccaacaa aaggcctgca 1200
taccaataca ggctgcataa ttacatgctc agccgcaaaa tttctgaact tcgctaccgc 1260
accatccagc tccacaggga aagtgccctg atgagcaagc tcagtaacac agaagtgagc 1320
aaagaggacc agcagctggg agtgatacct tctttcaacc acttccagcc tcgggagaga 1380
aatgaagtga tagaatggga gttcctgaca gggaagcttc tatactcagc agctgagaac 1440
cagccccctc gacagagcct cagtagcatt ttaagaacag cactggatga taccgtccta 1500
caggtgatgg agatgatcaa tgagaatgcc aagagcagag gacggctcat tgacttcaag 1560
gaaattcagt atggctaccg cagagttaac cccatgcacg gggtggagta cattttggat 1620
ttactccttt tatacaaaag acacaaggga aggaaactga ctgtgccagt gagacgtcat 1680
gcctatcttc agcagttgtt cagcaagcct ttcttcagag agaccgaaga gctagatgtc 1740
aacagtcttg tggagagtat taacagtgaa actcagtcat tctcctttat atctaattct 1800
ttaaagatat tatcttcttt tcaaggtgcc aaagaaatgg gagggcacaa tgaaaagaaa 1860
gtacacattc tcgttcctct catcggaagg tatgacattt tcttgagatt catggagaac 1920
tttgaaaaca tgtgtcttat cccaaagcag aatgtaaagt tggtcattat ccttttcagt 1980
agggattctg gccaagactc cagcaagcat attgagctga taaaagggta ccagaacaag 2040
taccccaaag cagaaatgac cctgatccca atgaagggag agttttccag aggtcttggt 2100
cttgaaatgg cttctgccca gtttgacaat gacactttgc tgctattttg tgatgttgac 2160
ttgatcttca gagaagattt tctccaacga tgtagagaca atacaattca gggacaacag 2220
gtgtactatc ccatcatctt tagccagtat gacccaaagg taacaaacgg gggaaatcct 2280

cccactgatg attacttcat attctcaaaa aagactggat tttggagaga ctatggatat 2340

ggcatcacct gtatttacaa aagtgatctt ctaggtgcag gtggatttga tacctcaata 2400

caaggctggg gactagaaga tgtagatctc tacaataaag tcattctatc tggcttaagg 2460

ccattcagaa gccaagaagt aggagtggtg catattttcc atccagttca ttgtgatcct 2520

aacttggacc ctaagcagta taagatgtgc ttaggatcca aggcaagtac tttcgcctca 2580

accatgcaac tggctgaact ctggcttgaa aaacatttag gtgtcaggta caatcgaact 2640

ctctcctgac agtccaggca acacattttg ccttttttaa ggggagttta cctcattgtt 2700

ggttgttgtt atttttattg tattattgtt attttattat tattattgtt ataattttat 2760

tttgttgtcc tggtcttaaa ctactc 2786

<210> 194
<211> 2170
<212> DNA
<213> Homo sapiens

<400> 194
ggctcgagag ttatacgtga acggagacct gaggctgcag cagaagcagc cgctgagctg 60

tggtggccta gatgcccgat acaacatatc cgtgatcaac gggaccagcc cctttgccta 120

tgactacgac ctcacccata ttgttgctgc ctaccaggag aggaacgaaa gctcccagtg 180

aggaactggt cttctggaga ctctgtgtgg catagagtga ttcaaccacc ttaagaagac 240

ctctggcttt cctggaacac agatgtcgag acatctccca tggatttgtg atcagcgttg 300

cagctctccc agcagccctg gacggtggcc cccagccgcc cgcatgtggc tgccacggtt 360

ctccagcaag acagtgacag tgctcctcct ggcacagacc acctgcctcc tgctcttcat 420

catctcccgg ccagggccct catccccagc cggcggcgag gatcgtgtgc acgtgctggt 480

gctgtcctcg tggcgctcgg gctcatcctt cttgggccag ctcttcagcc agcaccccga 540

cgtcttctac ctgatggagc ccgcgtggca tgtgtggacc accctgtcgc agggcagcgc 600

ggcaacgctg cacatggccg tgcgcgacct gatgcgctct atctttttgt gcgacatgga 660

cgtgtttgat gcctacatgc cacagagccg aaacctgtcc gcctttttca actgggcaac 720

gagccgcgcg ctgtgctcgc cgcccgcctg cagcgccttt ccccgaggca ccatcagcaa 780

gcaggacgta tgcaagacac tgtgcacgcg gcagccattc agcctggccc gggaggcctg 840

ccgctcctac agccacgtgg tgctcaagga ggtgcgcttc ttcaacctgc aggtgctcta 900

cccgctgctc agcgaccccg cgctcaacct gcgcatcgtg cacctggtgc gcgacccgcg 960

ggccgtgctg cgctcccggg aggcggcggg cccgatactg gcacgcgaca acggcatcgt 1020

gctgggcacc aacggcaagt gggtggaggc cgaccctcac ctgcgcctga ttcgcgaggt 1080

gtgccgcagc cacgtgcgca tcgccgaggc cgccacactc aagccgccac ccttcctgcg 1140

cggccgctac cgcctggtgc gcttcgagga cctggcgcgg gagccgctgg cagagatccg 1200

cgcactctac gccttcaccg gcctgaccct cacgccacag ctcgaggcct ggatccacaa 1260

catcacccac gggtcgggga tcggcaagcc aatcgaggcc ttccatactt cgtctaggaa 1320
tgcgcgcaac gtctcccagg cctggcgcca cgcgttgccc ttcactaaga tcctgcgcgt 1380
gcaggaggtg tgcgccggcg cgctgcagct gctgggctac cggcctgtgt actctgcgga 1440
ccagcagcgt gacctcaccc tggatctggt gctgccacga ggcccagacc acttcagctg 1500
ggcatcgcct gactgagaac tctgggcctt agagcaagcc ccgaactgtg gtcgccaggc 1560
ccaggaggcg actgcatggt ggagagggag ctggggcgca tggggaagca ggtccctact 1620
atcaaccggg agtttggggt cctcccctga agtaggcaag gactgcacgt ttctttctct 1680
cctgattctc ggttttcctt tgagtcttct ggagctgcct tctcatcagg tgcactcttc 1740
atggaaagca actcttgccc ctacctcttc tgggcgcagg gagtaagtta ctgctaaatt 1800
aaattaaatg tgtgccaggc cgggtgcggt ggctcatgcc tgtaatccca gcattttgag 1860
aggctgaggc gggtggatca cctgaggtca ggattcaaaa ccagcctggc caacatagtg 1920
aaaccccctc tctactaaaa atgcaaaaat tagtccggcg tggtggcaca ctcctgtaat 1980
cccagctact taggaggctg aggtgggaga atcacttgga ctccaaaggt ggaggttgca 2040
gtaagctgaa atcatgccac tgcaccctag cttgggtggc aaagcaaaac tctatcaaaa 2100
aaataattaa taaatttgtt caaaagtcct gccgaaaaaa aaaaaaaaaa aaaaaaaaaa 2160
aaaaaaaaaa 2170

<210> 195
<211> 1206
<212> DNA
<213> Homo sapiens

<400> 195
atgggctctt ggaagcactg tctttttagc gcgtctctta tctctgccct gatttttgta 60
tttgtttaca atactgagtt atgggagaat aaacgttttc tgagggcagc tctgtccaat 120
gcttcactgt tagcagaagc ctgtcatcag atttttgagg ggaaagtttt ttacccaaca 180
gaaaatgcat tgaaaactac ccttgatgaa gctacctgct atgagtacat ggttcgaagc 240
cactatgtaa cagaaacact ctctgaagaa gaggctgggt tccctttagc ttacacagtg 300
accatccaca aagacttcgg cacttttgag aggctcttca gggcgattta tatgccccaa 360
aatgtctact gtgtgcacct ggatcagaag gcgacggatg cctttaaagg tgcagtgaaa 420
cagttactca gctgcttccc aaatgctttt ctggcttcca agaaggagtc ggttgtctat 480
ggggggatct ccaggctcca ggctgacctg aactgcctgg aagaccttgt ggcctctgaa 540
gttccctgga agtatgtcat caacacctgc gggcaagact ttcccctgaa aaccaacagg 600
gaaatagttc agtatctgaa gggatttaaa gggaaaaata tcacccccgg agtgctgcct 660
cctgaccacg ctgttggacg gactaaatac gtccaccaag aactgttaaa ccacaaaaat 720
tcctacgtga ttaaaacaac aaaattaaaa actcctcctc ctcatgacat ggtgatttac 780
tttggcacgg cctacgtggc tctcacaagg gactttgcta acttcgtcct ccaagaccag 840
ctcgcacttg acttactctc ctggtccaag gacacctaca gccccgacga acatttctgg 900

gtgacactca acaggattcc cggtgttcct ggctctatgc caaatgcatc ctggactgga 960

aacctcagag ctataaagtg gagtgacatg gaagacagac acggaggctg ccacggccac 1020

tatgtacatg gtatttgtat ctatggaaac ggagacttaa agtggctggt taattcacca 1080

agcctgtttg ctaacaagtt tgagcttaat acctaccccc ttactgtgga atgcctagaa 1140

ctgaggcatc gcgaaagaac cctcaatcag agtgaaactg cgatacaacc cagctggtat 1200

ttttga 1206

<210> 196
<211> 5674
<212> DNA
<213> Homo sapiens

<400> 196
gggagaagtg accaggcggc ggcggctctg ctgaggtgac aacgtgctag cagccctcgc 60

tcgctctggg cgcctcctcg gcctcagcgt ccgctctggc cgcgctcgag gagcccttca 120

gcccgccgct gcgctatgaa ggcccctctc tggggctggc cgaggctgga gccggctccc 180

tctgctcgcg ggcaagtgtg aagagagagg cgcgggcggg agccggggct gcgcgcggcg 240

ctcgcgggcc ggcgcgggtt ccaggtgagc gcggactcgg cgagccccgc actcggcgcg 300

gccggccggc gcctgctggg cttgatctga ggctgaatcc cgtgtgtgga ccgccgttcc 360

ctcttcgcgg gatcgttggc caggatagca gatgcatttt tgtagaagca ttttgaggat 420

gaatgtttgc aaatcatttt gaaattgcca agtgccacat acatggaatg tatcctgctt 480

tcatcttgga gttcacctgt gtggcttgga tttatcacag tagcatttgt cttcaatctg 540

tgtgttaact agaaatcaag gaaagacatg aggagatttg tctactgcaa ggtggttcta 600

gccacttcgc tgatgtgggt tcttgttgat gtcttcttac tgctgtactt cagtgaatgt 660

aacaaatgtg atgacaagaa ggagagatct ctgctgcctg cattgagggc tgttatttca 720

agaaaccaag aagggccagg agaaatggga aaagctgtgt tgattcctaa agatgaccag 780

gagaaaatga aagagctgtt taaaatcaat cagtttaacc ttatggccag tgatttgatt 840

gcccttaata gaagtctgcc agatgtaaga ttagaaggat gtaagacaaa agtctaccct 900

gatgaacttc caaacacaag tgtagtcatt gtgtttcata atgaagcttg gagcactctc 960

cttagaactg tttacagtgt gataaatcgt tccccacact atctactctc agaggtcatc 1020

ttggtagatg atgccagtga aagagatttt ctcaagttga cattagagaa ttacgtgaaa 1080

aatttagaag tgccagtaaa aattattagg atggaagaac gctctgggtt aatacgtgcc 1140

cgtcttcgag gagcagctgc ttcaaaaggg caggtcataa cttttcttga tgcacactgt 1200

gaatgcacgt taggatggct ggagcctttg ctggcaagaa taaaggaaga caggaaaacg 1260

gttgtctgcc ctatcattga tgtgattagt gatgatactt ttgaatatat ggctgggtca 1320

gacatgactt atgggggttt taactggaaa ctgaatttcc gctggtatcc tgttccccaa 1380

agagaaatgg acaggaggaa aggagacaga acattacctg tcaggacccc tactatggct 1440

ggtggcctat tttctattga cagaaactac tttgaagaga taggaactta cgatgcagga 1500
atggatatct ggggtggaga gaatcttgaa atgtctttta ggatttggca atgtggaggc 1560
tccttggaga ttgttacttg ctcccatgtt ggtcatgttt ttcggaaggc aactccatac 1620
acttttcctg gtggcactgg tcatgtcatc aacaagaaca acaggagact ggcagaagtt 1680
tggatggatg aatttaaaga tttcttctac atcatatccc caggtgttgt caaagtggat 1740
tatggagatg tgtcagtcag aaaaacacta agagaaaatc tgaagtgtaa gcccttttct 1800
tggtacctag aaaacatcta tccggactcc cagatcccaa gacgttatta ctcacttggt 1860
gagataagaa atgttgaaac caatcagtgt ttagacaaca tgggccgcaa ggaaaatgaa 1920
aaagtgggta tattcaactg tcatggtatg ggaggaaatc aggtattttc ttacactgct 1980
gacaaagaaa tccgaaccga tgacttgtgc ttggatgttt ctagactcaa tggacctgta 2040
atcatgttaa aatgccacca tatgagagga aatcagttat gggaatatga tgctgagaga 2100
ctcacgttgc gacatgttaa cagtaaccaa tgtctcgatg aaccttctga agaagacaaa 2160
atggtgccta caatgcagga ctgtagtgga agcagatccc aacagtggct gctaaggaac 2220
atgaccttgg gcacatgaag atcatgtcct ccaagccatg aaagtgtcta cgcttttgtt 2280
tttccattat ttcaattggg ggaaaatatt aactttgctg aattgaaagt tttaaaaatc 2340
cttttagtat tctaaaacac aattgtttct aattcgtttc tagaaatgtt tgcttatttc 2400
cctactaaaa tttgtatctg atcaaagcac ataagaatat aaataatagc aaactactat 2460
taaacaacag aacaacttgt aaaacaaatt gtgtttgctt taagaaaaat ctttattgca 2520
ctcatgtcat agggttaatt ggaggttatt ttatttttgg ttgtcatggt gattgaaaga 2580
gataatgtaa atgccttata aaatcttcat tatgaaatat tatcagttgc tttataaact 2640
cactcttttt atggatcctt catggaaaca tgtttgattt ctgtgtctac aacagggcca 2700
catattaaat tacttctgaa tggtgaattc atcttacaaa atgttccaag ttttggacaa 2760
ggaaaaacat tacattggat attgaattca tggagccttt acagaagcat cacatttaaa 2820
ccaatgtgaa ttcaaaaaag agaaggaaac tttttaaatt caatttagag tacataaaaa 2880
aagaaatccg cgaagcattg aggggaacaa gatgagtcta atccgcagca cttcgatcac 2940
tgtgagagaa ctcaaagtgg gttgcaatca ttcctaacac aggctgaaac taaacaatct 3000
tgctcctaga gtttatgttg gatactcaat tcttaaccaa aatcttggtc tccacaaact 3060
ctaccatccc ttttctcttc actctataga cagtggcatg ccattgatgc tgtacagaat 3120
tgcaggtgaa agggagaatt ttagacttaa tttttaactc tattgcactt aaagatttta 3180
gttaggttac cactgtcatt ttcattttct atgttaaaga atacctttca gtgctgtgct 3240
ccagtatcta aaaattttat caccagggga ataaactcaa tacacattca ttaaactttt 3300
gttgtaatta aactgctata tattgtttgc catatattat ggcccaagga atatagttat 3360
aatcaggcta cattcacatt ttcttttctc atcttaaaac cctttatgtt caaaatacat 3420
tagcagaggc catgaaagtg aaaaaaaatc agatttttgc tagtaagttt ttatatttga 3480

catcatttta aatgctcaaa gagttgccct atatatgcat gttatccatt ataaatgcac 3540
tcagcagcta agagaaattt gagaggaaaa gtggaagaag tgttttctgg agcagaagat 3600
tccacttctt tggtcctcat ctttacttaa aaagctctta tagaaaggat taatcatttt 3660
gactgcatac aaactatgtc tctgacatgc acatacactg ctttataatg aaaatgagga 3720
cactttctga tggtcagtaa aatcttaaat gatttttgcc actggtattc cttcctcttt 3780
tgacttttta ttggtacaac tgtagggaag aatctacaac ctggagcatt tcaggtttgc 3840
tctttaaata tagacacccc tcaccatgag ctctctgtga atccgtgaat ttcctaaaat 3900
tgtaagcaaa atgttgaata tcagggcatt tttttttctg aggtgggagt atgtcactca 3960
cagcaaactt ggaaagaagg ctatgactcc caagaggcag tgaaccattg ccttaaatta 4020
atagcagcct ctcaagtcca ttgaggcctc atgtaaaatt cccatttttc atctttacac 4080
tccttttctc ccctcatttc ctcttctcct tactaaggta aaaagaccct gcacagcatt 4140
attacttaag ttgaaaaagc ttcaactctc aaggactcat gtataccata tgttccactt 4200
attgctttgt tagtcaaaat gcatgttatg tagaaaatgg tcaatggcaa atttttataa 4260
atacagctac cccatcaaag ctgaacttga aacgttttag cagaatttta aatatgtgaa 4320
agttcattgt ggtcatggtg agaaaactaa aactacatga gtttcatgtt agaaaagaat 4380
ttttaaacaa tcttcagatg gcttgaacac gtgccattat agtatctaac aaaaagtggc 4440
ataaaatatt acaaaacatc caacactaga aagcaatgac taacatttaa tttgtttaca 4500
aaaatgttta ttgtcttcag tacaaaaagt taaaaccatg attggattca taatgcactt 4560
gtcttatccc ttatttatgg agctagactg acatatttca aaccaatctc ttctgtggtc 4620
ttctccatct cagttaatgc aacttcaacc ttccagatgc tcaggtcaaa aactgaaagt 4680
tgcttttgac tcctctattt ctgtttcaac tctcgtctaa tccaacagca aatccccttg 4740
atgctacctt tgaaatataa ctagaaaaga atgacttgcc accatatcca ctaggtgtca 4800
ctacctctaa cctttttaat cgcaatatgc ttattattta gttccctaat ttcccagtcc 4860
ttgtcttgaa caatcttttc tcagtacaac agccatagtg attctgttaa ccatgaattt 4920
gattgtgtca cttccctgct cacaatcttc caatgacttc tccctccggt ggccttcaag 4980
gtcctttatg agtgttaccc ctctacacgt ccacaccact caccacacat acatacacat 5040
gcacacacac acacatgcaa ttctaccttt ttctaacacc atctcttatc tctcattatt 5100
ctgcctctta cttaccctat tatggctccc aagaaagttc ctgctttagt gttttgttcc 5160
ctctgcccca gagagctccc cttagtgatc tcagttcaaa tattatctta tcagtaagac 5220
aaacccttct cccatctgcc ttccctatcc cctgtacctg acaatctatg agtttgcttc 5280
tttatttctg tctatacttc cccctcccca gccccaaaca tgcccatcac atgccctaga 5340
aggtaagtcc catgaaggca ggggctttgt cagttttgtt catttggtat ttctggcata 5400
tatattctct aaatatttct tgaattaatg aactgaaaaa tgtgtgttaa agttgctaag 5460
tgtaactgta tcatactttt tttgtatttt aaattttaaa ataaaggcta atatatttag 5520

acaggatttc caaaaacttt gtcttcaaat tttaattttc ttcctaatat tcttcacatt 5580

aaactatgga ctctaatttt ctggataaaa tatcctgcct tatggaaatg aaatatgaaa 5640

ttttattaaa atgctgctat attaaaaaaa aaaa 5674

<210> 197
<211> 1312
<212> DNA
<213> Homo sapiens

<400> 197
ctagaattca gcggccgctg aattctagaa cccaggtgca actgacatgg gtgaacccca 60

gggatccatg cggattctag tgacaggggg ctctgggctg gtaggcaaag ccatccagaa 120

ggtggtagca gatggagctg gacttcctgg agaggactgg gtgtttgtct cctctaaaga 180

cgccgatctc acggatacag cacagacccg cgccctgttt gagaaggtcc aacccacaca 240

cgtcatccat cttgctgcaa tggtgggggg cctgttccgg aatatcaaat acaatttgga 300

cttctggagg aaaaacgtgc acatgaacga caacgtcctg cactcggcct ttgaggtggg 360

ggcccgcaag gtggtgtcct gcctgtccac ctgtatcttc cctgacaaga cgacctaccc 420

gatagatgag accatgatcc acaatgggcc tccccacaac agcaattttg ggtactcgta 480

tgccaagagg atgatcgacg tgcagaacag ggcctacttc cagcagtacg gctgcacctt 540

caccgctgtc atccccacca acgttttcgg gccccacgac aacttcaaca tcgaggatgg 600

ccacgtgctg cctggcctca tccacaaggt gcacctggcc aagagcagcg gctcggccct 660

gacggtgtgg ggtacaggga atccgcggag gcagttcata tactcgctgg acctggccca 720

gctctttatc tgggtcctgc gggagtacaa tgaagtggag cccatcatcc tctccgtggg 780

cgaggaagat gaggtctcca tcaaggaggc agccgaggcg gtggtggagg ccatggactt 840

ccatggggaa gtcacctttg atacaaccaa gtcggatggg cagtttaaga agacagccag 900

taacagcaag ctgaggacct acctgcccga cttccggttc acacccttca agcaggcggt 960

gaaggagacc tgtgcttggt tcactgacaa ctacgagcag gcccggaagt gaagctggaa 1020

gacaggatca ggtgccagcg gaccatcggc tggcagagcc cagcggccac cacccgtcaa 1080

ccctgccagg agctgagggc accacccagc aacctgggcc tgcattccat ccgctctgca 1140

gccccaagca tctttccagt ggggccccca ttcacgttgg tcctcaggga aaccagggtc 1200

cggggcaggc ccggcgcttt gctccccaca ccagccccct gcgcgtgtcc actctgatcc 1260

tgcatcccac tccctgggag ccaataaagt gcattttcac agaaaaaaaa aa 1312

<210> 198
<211> 2567
<212> DNA
<213> Homo sapiens

<400> 198
ctccggcgca gtgttgggac tgtctgggta tcggaaagca agcctacgtt gctcactatt 60

acgtataatc cttttctttt caagatgcct gaggaagtgc accatggaga ggaggaggtg 120

gagacttttg cctttcaggc agaaattgcc caactcatgt ccctcatcat caataccttc 180
tattccaaca aggagatttt ccttcgggag ttgatctcta atgcttctga tgccttggac 240
aagattcgct atgagagcct gacagaccct tcgaagttgg acagtggtaa agagctgaaa 300
attgacatca tccccaaccc tcaggaacgt accctgactt tggtagacac aggcattggc 360
atgaccaaag ctgatctcat aaataatttg ggaaccattg ccaagtctgg tactaaagca 420
ttcatggagg ctcttcaggc tggtgcagac atctccatga ttgggcagtt tggtgttggc 480
ttttattctg cctacttggt ggcagagaaa gtggttgtga tcacaaagca caacgatgat 540
gaacagtatg cttgggagtc ttctgctgga ggttccttca ctgtgcgtgc tgaccatggt 600
gagcccattg gcaggggtac caaagtgatc ctccatctta aagaagatca gacagagtac 660
ctagaagaga ggcgggtcaa agaagtagtg aagaagcatt ctcagttcat aggctatccc 720
atcacccttt atttggagaa ggaacgagag aaggaaatta gtgatgatga ggcagaggaa 780
gagaaaggtg agaaagaaga ggaagataaa gatgatgaag aaaaacccaa gatcgaagat 840
gtgggttcag atgaggagga tgacagcggt aaggataaga agaagaaaac taagaagatc 900
aaagagaaat acattgatca ggaagaacta aacaagacca agcctatttg gaccagaaac 960
cctgatgaca tcacccaaga ggagtatgga gaattctaca agagcctcac taatgactgg 1020
gaagaccact tggcagtcaa gcacttttct gtagaaggtc agttggaatt cagggcattg 1080
ctatttattc ctcgtcgggc tccctttgac ctttttgaga acaagaagaa aaagaacaac 1140
atcaaactct atgtccgccg tgtgttcatc atggacagct gtgatgagtt gataccagag 1200
tatctcaatt ttatccgtgg tgtggttgac tctgaggatc tgcccctgaa catctcccga 1260
gaaatgctcc agcagagcaa aatcttgaaa gtcattcgca aaaacattgt taagaagtgc 1320
cttgagctct tctctgagct ggcagaagac aaggagaatt acaagaaatt ctatgaggca 1380
ttctctaaaa atctcaagct tggaatccac gaagactcca ctaaccgccg ccgcctgtct 1440
gagctgctgc gctatcatac ctcccagtct ggagatgaga tgacatctct gtcagagtat 1500
gtttctcgca tgaaggagac acagaagtcc atctattaca tcactggtga gagcaaagag 1560
caggtggcca actcagcttt tgtggagcga gtgcggaaac ggggcttcga ggtggtatat 1620
atgaccgagc ccattgacga gtactgtgtg cagcagctca aggaatttga tgggaagagc 1680
ctggtctcag ttaccaagga gggtctggag ctgcctgagg atgaggagga gaagaagaag 1740
atggaagaga gcaaggcaaa gtttgagaac ctctgcaagc tcatgaaaga aatcttagat 1800
aagaaggttg agaaggtgac aatctccaat agacttgtgt cttcaccttg ctgcattgtg 1860
accagcacct acggctggac agccaatatg gagcggatca tgaaagccca ggcacttcgg 1920
gacaactcca ccatgggcta tatgatggcc aaaaagcacc tggagatcaa ccctgaccac 1980
cccattgtgg agacgctgcg gcagaaggct gaggccgaca agaatgataa ggcagttaag 2040
gacctggtgg tgctgctgtt tgaaaccgcc ctgctatctt ctggcttttc ccttgaggat 2100
ccccagaccc actccaaccg catctatcgc atgatcaagc taggtctagg tattgatgaa 2160

gatgaagtgg cagcagagga acccaatgct gcagttcctg atgagatccc ccctctcgag 2220

ggcgatgagg atgcgtctcg catggaagaa gtcgattagg ttaggagttc atagttggaa 2280

aacttgtgcc cttgtatagt gtccccatgg gctcccactg cagcctcgag tgcccctgtc 2340

ccacctggct ccccctgctg gtgtctagtg tttttttccc tctcctgtcc ttgtgttgaa 2400

ggcagtaaac taagggtgtc aagccccatt ccctctctac tcttgacagc aggattggat 2460

gttgtgtatt gtggtttatt ttattttctt cattttgttc tgaaattaaa gtatgcaaaa 2520

taaagaatat gccgttttaa aaaaaaaaaa aaaaaaaaaa aaaaaaa 2567

<210> 199
<211> 3745
<212> DNA
<213> Homo sapiens

<400> 199
   cggcggcggg cccaaggcgt gaggcgccgc ccgggtgtcc ccgcggcgca ggaggcggtg 60
   gagcgcagag cgggcgagcg cgaaaaatca ctaccaatat aatggatttt atatatcaga 120
   ttgctttatt ctggatatca tggtaacaat acagaaagta tacataattt cccatttctg 180
   caagtagtca tgactgctga agaaagaaaa acttaaagct acggcagaat tattttatgg 240
   aaattctgat tttgttttta atttttgata actttttact aaaggtatga acacacaaag 300
   agcttatttt gttaggcaaa tacacattaa taagaatgcc tagaagagga ctgattcttc 360
   acacccggac ccactggttg ctgttgggcc ttgctttgct ctgcagtttg gtattattta 420
   tgtacctcct ggaatgtgcc ccccagactg atggaaatgc atctcttcct ggtgttgttg 480
   gggaaaatta tggtaaagag tattatcaag ccctcctaca ggaacaagaa gaacattatc 540
   agaccagggc aaccagtctg aaacgccaaa ttgcccaact aaaacaagaa ttacaagaaa 600
   tgagtgagaa gatgcggtca ctgcaagaaa gaaggaatgt aggggctaat ggcataggct 660
   atcagagcaa caaagagcaa gcacctagtg atcttttaga gtttcttcat tcccaaattg 720
   acaaagctga agttagcata ggggccaaac tacccagtga gtatggggtc attccctttg 780
   aaagttttac cttaatgaaa gtatttcaat tggaaatggg tctcactcgc catcctgaag 840
   aaaagccagt tagaaaagac aaacgagatg aattggtgga agttattgaa gcgggcttgg 900
   aggtcattaa taatcctgat gaagatgatg aacaagaaga tgaggagggt ccccttggag 960
   agaaactgat atttaatgaa aatgacttcg tagaaggtta ttatcgcact gagagagata 1020
   agggcacaca gtatgaactc ttttttaaga aagcagacct tacggaatat agacatgtga 1080
   ccctcttccg cccttttgga cctctcatga aagtgaagag tgagatgatt gacatcacta 1140
   gatcaattat taatatcatt gtgccacttg ctgaaagaac tgaagcattt gtacaattta 1200
   tgcagaactt cagggatgtt tgtattcatc aagacaagaa gattcatctc acagtggtgt 1260
   attttggtaa agaaggactg tctaaagtca agtctatcct agaatctgtc accagtgagt 1320
   ctaattttca caattacacc ttggtctcat tgaatgaaga atttaatcgt ggacgaggac 1380
   taaatgtggg tgcccgagct tgggacaagg gagaggtctt gatgtttttc tgtgatgttg 1440
   atatctattt ctcagccgaa ttccttaaca gctgccggtt aaatgctgag ccaggtaaga 1500
   aggtgtttta ccctgtggtg ttcagtcttt acaatcctgc cattgtttat gccaaccagg 1560
   aagtgccacc acctgtggag cagcagctgg ttcacaaaaa ggattctggc ttttggcgag 1620
   attttggctt tggaatgact tgtcagtatc gttcagattt cctgaccatt ggtggatttg 1680
   acatggaagt gaaaggttgg ggtggagaag atgttcatct ttatcgaaaa tacttacatg 1740
   gtgacctcat tgtgattcgg actccggttc ctggtctttt ccacctctgg catgaaaagc 1800
   gctgtgctga tgagctgacc cccgagcagt accgcatgtg catccagtct aaagccatga 1860
   atgaggcctc tcactcccac ctgggaatgc tggtcttcag ggaggaaata gagacgcatc 1920
   ttcataaaca ggcatacagg acaaacagtg aagctgttgg ttgaaatcat aattaatgcg 1980
   ttactgtatg aaccacaaaa cagcactatt tatttagcct tacttctact tccagatgca 2040
   gtgcctcttt tggagaagac atgtttattt ttcatgttct ttctgacatt actttagcaa 2100
   ttcaacttga tgtgagaaga aaaaacaaat gtttcaacac aaaatctctg ttttgtgaga 2160
   atactgcact atggaataat tgacaaattg aaatctcata tttgtcccaa aagttgtttt 2220
   gagttagttc tacctggtgc ccatgttctg attgtgtgtg ggattgcatg gtgtcctgat 2280
   tgcatctagg tggagcggat ggaatgtgct gggccactgt tgggtggaga gcagcacatt 2340
   cttacagagg agatggagcg ttatgagcat agtatgtgga taggtatctt cacctgcccg 2400
   cccctgagtc agcctccttg acttgatagc ttgaagaatc cttttccact gaaatagagg 2460
   ataattaatt gacacatctg aaatccccaa tcaatcaatc aagagaaagg tagaactaaa 2520
   aactccttaa cttactgttg cttacacccc tgaaagtctg tttttaagca aatgggtaat 2580
   agtagaaaat aggttagaat ctatggcttg attaaaaata tgttattaca ttatcatgtt 2640
   caggattagg attagtagtc agttgctgta aactattttg aacaaacaga aaagaacacg 2700
   gaaacatttt taacagagca tttaattatg ttggaataca ggatcctagc tctgtctggg 2760
   aacattagtt tatgtgagcc agctctatca gggtcttccc atggtggttc agaatagatg 2820
   agcatagcat ggttttgttt gtttttgctt tcaattttct aatttggcat ggatccatat 2880
   gtatttacta tcctttttct aatatattaa tatatgctac atttgtattt gcattactat 2940
   aatactttga gttgaaaaag agtttcattg tggagagaaa aagcaaatgg tatgccacaa 3000
   gatcactctg atttgagaaa agggaggagg ggaagatagt ctgaatggaa atctgaaata 3060
   cggaatgttt tagagaaata tgtcacttgc atatagaatg ttttaattga ggtataaatt 3120
   aatgagacaa agtgaaaaag aaattatatt cagataggac tgcactacat tatttgtcac 3180
   acatggatct gttaccatca ggtcaattcc tagtatgcat aaatttttta acccttttaa 3240
   aagagaccta tgttgaaaac ccctgaaaat tcactgaaga aaaatcatta ctctttttct 3300
   cagtaaatca tatcatctga aatattacaa atttcaaatt tctaggtgct atattaattc 3360
   aatattacaa taactcttac ctaattattc ttacaagttt taagttgtgg tagtttagtg 3420
   atttttttaa aagatgtgtg aaatgttctc tgcaaaataa ttcaggccac tgtctccttt 3480
   tatatattat tataattatt tattatgaag accagtgaat tacgatattt aaagtgagag 3540
   aacttaatta tttgcaaagg taagttacag cttgtttttt gagagaatca aatgagttta 3600
   cttttgttcc tgttgttttt aactagcttt aagtttaaag atggaagcta agcaatggaa 3660
   atgctatacg tttttgacat ttattaaatg gtaccaataa agtattttat taccaaaagt 3720
   taaaaaaaaa aaaaaaaaaa aaaaa 3745

<210> 200
<211> 1380
<212> DNA
<213> Homo sapiens

<400> 200
gtctctgccc atccgcgcac ccgggcttcg gctggagagg gccagctcgc ttcaggaggc 60

cgaaccccgt tcccaccaac cctctcagct cagacgcggg gtgctgagtc acgggggggg 120

ggtggttctg tggatagttg gaatgcatac acagaggaaa gggggatgcg gcaccagcag 180

acagagagac aagaccccag ccagcccctg tccaggcagc atggcacata ccgccagatc 240

ttccatccag agcagctcat cacaggcaag gaagatgctg ccaataacta tgcctggggc 300

cactacacca ttgggaagga gttcatcgac ctgctactgg accggattcg gaagctggct 360

gaccagtgca caggacttca gggcttcctg gtgttccaca gccttggtcg gggcactggc 420

tctgacgtca cctcattcct gatggagtgg ctttctgtta actatggcaa gaaatccaag 480

ctgggattct ccatctaccc agccccccag gtgtctacag ccatggtcca gccctacaac 540

tctatcctga ccacccacac caccctggag cactcagact gtgccttcat ggtggacaac 600

aaagcaatct atgacatctg ccaccgcaac ctagacattg agcgcccaac ctacaccaac 660

ctcaatcgcc tcattagcca aattgtctcc tccatcacag cttctctgcg ctttgacggg 720

gccctcaatg tggacctgac agagttccag accaacctgg tgtcctacct cacatccact 780

tccccctggc cacctatgca ccagtcatct ctgcagaaaa agtataccac gagcagctgt 840

cggtggcaga gattaccaat gcctgctttg agcctgccaa ccagatggtg aagtgtgatc 900

cccggcacgg caagtacatg gcctgctgcc tgctatacca tggagatgtg gtgcccaagg 960

atgtcaacgc tgccattgct gccatcaaga ccaagtgcag cattcagttt gtggactggt 1020

gccccacagg ctttaaggtt gatatcaatc accagcctcc cactgtggtg cctgggagtg 1080

acctggtaaa gtgcaacgtg ccatgtgcat gctgagcaac atgacagcca tcactatggc 1140

ctgggcccgc ctggaccaca agtttgacct gatgtatgcc aagagggcgt ttgggcactg 1200

atatgtgggt gagggcatgg aggagggtga gttctccaag gcccatgagg atatgactgc 1260

cctggagaag gattacaagg aggtgggcat ggatagtgtg gagtgtgggg aagaaaagat 1320

aggggggatg aatactaggg gaatactgtg tgtctgtcct acataaagtg ctgtggcctt 1380

<210> 201
<211> 1664
<212> DNA

<213>  Homo sapiens

<400>  201
aattcataaa caagagctcc agttccctgg aacctgagtc gggagacctg cagctcactt      60

tctccctgtt tgctggccag cagggtttgt ggccgcctct gctgtgacgc cacagcctgg     120

tgaggagggg gctgcagttc ctcaggaaac tgccagtgga caaagtcata aacaagagtt     180

ccagttccct ggaacctgag tcgggaggcc tgcaactcac tttctccctg tttgctggcc     240

cagacaggcc tgtgctagag ggctggatag tgtggtagtg tggtggatag agtgctgcct     300

ctatccacaa cgcagccata tgctgactga agataacttt gtgtctggaa gagccctctg     360

atttcttaca ctaatggcct tacacgacat ggaggatttt acatttgatg gaacaaagcg     420

cttaagtgtc aactacgtga agggaattct tcaaccgaca gacacctgtg acatctggga     480

taagatctgg aacttccaag ccaagcctga tgacctgctt atttctacct atcctaaagc     540

aggaacaaca tggactcagg agatagtgga attaatacaa aatgaaggtg atgtggagaa     600

aagtaaacgg gcaccgactc atcaacgatt tcctttcctc gaaatgaaaa tcccatcctt     660

aggatctggt ttggaacaag ctcatgcaat gccctcacca cggatcctga aaacacatct     720

tccctttcac ttgctgccac catccttgct agagaaaaac tgtaagataa tctatgtagc     780

aagaaatccc aaggacaaca tggtgtccta ttaccatttc caaagaatga ataaagctct     840

tcctgctcca ggaacatggg aagagtattt tgagactttt ctggctggga aagtgtgctg     900

gggctcctgg catgaacatg tgaaaggatg gtgggaagcc aaagacaaac accgtattct     960

ctatctcttc tatgaggaca tgaagaagaa cccaaagcat gaaattcaga agctggcaga    1020

atttattggg aagaaattag atgacaaagt tctagataaa attgtccatt acacttcgtt    1080

tgatgtcatg aaacagaatc caatggcaaa ctattcatcg attcctgctg aaatcatgga    1140

ccactccatt tctccattca tgagaaaagg ggcagtggga gactggaaga aacacttcac    1200

cgtggctcag aatgagagat ttgatgaaga ttacaagaag aaaatgactg ataccagact    1260

aactttccac ttccagttct agtaaggaag aaaaactgaa aatgttttag tttattaccc    1320

agtatatttg ggtaataatg aaagtttaat tctcataaca aatgatatca gattccagtt    1380

atcagaatag tttactgtgt ttgctcttat tcactctact aaaaaattat tttaaaaggc    1440

tggaggcaag gtggtgacag gcagcagggt ggcgacatgg agagagggaa gctcaataaa    1500

tcactaggta caatcctggt atcattgcca attatcatca tatttacatt ttctacaatc    1560

atgtgcttat taacatattg gaaatgcttc agtcctcaca tgataataca ctaacatttt    1620

cagattaagt tttggttcaa gttaattttt tttttttttt tttt                     1664

<210>  202
<211>  5748
<212>  DNA
<213>  Homo sapiens

<400>  202
   gagaagaaag ccagtgcgtc tctgggcgca ggggccagtg gggctcggag gcacaggcac      60
   cccgcgacac tccaggttcc ccgacccacg tccctggcag ccccgattat ttacagcctc 120
   agcagagcac ggggcggggg cagaggggcc cgcccgggag ggctgctact tcttaaaacc 180
   tctgcgggct gcttagtcac agcccccctt gcttgggtgt gtccttcgct cgctccctcc 240
   ctccgtctta ggtcactgtt ttcaacctcg aataaaaact gcagccaact tccgaggcag 300
   cctcattgcc cagcggaccc cagcctctgc caggttcggt ccgccatcct cgtcccgtcc 360
   tccgccggcc cctgccccgc gcccagggat cctccagctc ctttcgcccg cgccctccgt 420
   tcgctccgga caccatggac aagttttggt ggcacgcagc ctggggactc tgcctcgtgc 480
   cgctgagcct ggcgcagatc gatttgaata taacctgccg ctttgcaggt gtattccacg 540
   tggagaaaaa tggtcgctac agcatctctc ggacggaggc cgctgacctc tgcaaggctt 600
   tcaatagcac cttgcccaca atggcccaga tggagaaagc tctgagcatc ggatttgaga 660
   cctgcaggta tgggttcata gaagggcacg tggtgattcc ccggatccac cccaactcca 720
   tctgtgcagc aaacaacaca ggggtgtaca tcctcacatc caacacctcc cagtatgaca 780
   catattgctt caatgcttca gctccacctg aagaagattg tacatcagtc acagacctgc 840
   ccaatgcctt tgatggacca attaccataa ctattgttaa ccgtgatggc acccgctatg 900
   tccagaaagg agaatacaga acgaatcctg aagacatcta ccccagcaac cctactgatg 960
   atgacgtgag cagcggctcc tccagtgaaa ggagcagcac ttcaggaggt tacatctttt 1020
   acaccttttc tactgtacac cccatcccag acgaagacag tccctggatc accgacagca 1080
   cagacagaat ccctgctacc actttgatga gcactagtgc tacagcaact gagacagcaa 1140
   ccaagaggca agaaacctgg gattggtttt catggttgtt tctaccatca gagtcaaaga 1200
   atcatcttca cacaacaaca caaatggctg gtacgtcttc aaataccatc tcagcaggct 1260
   gggagccaaa tgaagaaaat gaagatgaaa gagacagaca cctcagtttt tctggatcag 1320
   gcattgatga tgatgaagat tttatctcca gcaccatttc aaccacacca cgggcttttg 1380
   accacacaaa acagaaccag gactggaccc agtggaaccc aagccattca aatccggaag 1440
   tgctacttca gacaaccaca aggatgactg atgtagacag aaatggcacc actgcttatg 1500
   aaggaaactg gaacccagaa gcacaccctc ccctcattca ccatgagcat catgaggaag 1560
   aagagacccc acattctaca agcacaatcc aggcaactcc tagtagtaca acggaagaaa 1620
   cagctaccca gaaggaacag tggtttggca acagatggca tgagggatat cgccaaacac 1680
   ccaaagaaga ctcccattcg acaacaggga cagctgcagc ctcagctcat accagccatc 1740
   caatgcaagg aaggacaaca ccaagcccag aggacagttc ctggactgat ttcttcaacc 1800
   caatctcaca ccccatggga cgaggtcatc aagcaggaag aaggatggat atggactcca 1860
   gtcatagtat aacgcttcag cctactgcaa atccaaacac aggtttggtg gaagatttgg 1920
   acaggacagg acctctttca atgacaacgc agcagagtaa ttctcagagc ttctctacat 1980
   cacatgaagg cttggaagaa gataaagacc atccaacaac ttctactctg acatcaagca 2040
   ataggaatga tgtcacaggt ggaagaagag acccaaatca ttctgaaggc tcaactactt 2100
   tactggaagg ttatacctct cattacccac acacgaagga aagcaggacc ttcatcccag 2160
   tgacctcagc taagactggg tcctttggag ttactgcagt tactgttgga gattccaact 2220
   ctaatgtcaa tcgttcctta tcaggagacc aagacacatt ccaccccagt ggggggtccc 2280
   ataccactca tggatctgaa tcagatggac actcacatgg gagtcaagaa ggtggagcaa 2340
   acacaacctc tggtcctata aggacacccc aaattccaga atggctgatc atcttggcat 2400
   ccctcttggc cttggctttg attcttgcag tttgcattgc agtcaacagt cgaagaaggt 2460
   gtgggcagaa gaaaaagcta gtgatcaaca gtggcaatgg agctgtggag gacagaaagc 2520
   caagtggact caacggagag gccagcaagt ctcaggaaat ggtgcatttg gtgaacaagg 2580
   agtcgtcaga aactccagac cagtttatga cagctgatga gacaaggaac ctgcagaatg 2640
   tggacatgaa gattggggtg taacacctac accattatct tggaaagaaa caaccgttgg 2700
   aaacataacc attacaggga gctgggacac ttaacagatg caatgtgcta ctgattgttt 2760
   cattgcgaat cttttttagc ataaaatttt ctactctttt tgttttttgt gttttgttct 2820
   ttaaagtcag gtccaatttg taaaaacagc attgctttct gaaattaggg cccaattaat 2880
   aatcagcaag aatttgatcg ttccagttcc cacttggagg cctttcatcc ctcgggtgtg 2940
   ctatggatgg cttctaacaa aaactacaca tatgtattcc tgatcgccaa cctttccccc 3000
   accagctaag gacatttccc agggttaata gggcctggtc cctgggagga aatttgaatg 3060
   ggtccatttt gcccttccat agcctaatcc ctgggcattg ctttccactg aggttggggg 3120
   ttggggtgta ctagttacac atcttcaaca gaccccctct agaaattttt cagatgcttc 3180
   tgggagacac ccaaagggtg aagctattta tctgtagtaa actatttatc tgtgtttttg 3240
   aaatattaaa ccctggatca gtcctttgat cagtataatt ttttaaagtt actttgtcag 3300
   aggcacaaaa gggtttaaac tgattcataa taaatatctg tacttcttcg atcttcacct 3360
   tttgtgctgt gattcttcag tttctaaacc agcactgtct gggtccctac aatgtatcag 3420
   gaagagctga gaatggtaag gagactcttc taagtcttca tctcagagac cctgagttcc 3480
   cactcagacc cactcagcca aatctcatgg aagaccaagg agggcagcac tgtttttgtt 3540
   ttttgttttt tgtttttttt ttttgacact gtccaaaggt tttccatcct gtcctggaat 3600
   cagagttgga agctgaggag cttcagcctc ttttatggtt taatggccac ctgttctctc 3660
   ctgtgaaagg ctttgcaaag tcacattaag tttgcatgac ctgttatccc tggggcccta 3720
   tttcatagag gctggcccta ttagtgattt ccaaaaacaa tatggaagtg ccttttgatg 3780
   tcttacaata agagaagaag ccaatggaaa tgaaagagat tggcaaaggg gaaggatgat 3840
   gccatgtaga tcctgtttga catttttatg gctgtatttg taaacttaaa cacaccagtg 3900
   tctgttcttg atgcagttgc tatttaggat gagttaagtg cctggggagt ccctcaaaag 3960
   gttaaaggga ttcccatcat tggaatctta tcaccagata ggcaagttta tgaccaaaca 4020
   agagagtact ggctttatcc tctaacctca tattttctcc cacttggcaa gtcctttgtg 4080
   gcatttattc atcagtcagg gtgtccgatt ggtcctagaa cttccaaagg ctgcttgtca 4140
   tagaagccat tgcatctata aagcaacggc tcctgttaaa tggtatctcc tttctgaggc 4200
   tcctactaaa agtcatttgt tacctaaact tatgtgctta acaggcaatg cttctcagac 4260
   cacaaagcag aaagaagaag aaaagctcct gactaaatca gggctgggct tagacagagt 4320
   tgatctgtag aatatcttta aaggagagat gtcaactttc tgcactattc ccagcctctg 4380
   ctcctccctg tctaccctct cccctccctc tctccctcca cttcacccca caatcttgaa 4440
   aaacttcctt tctcttctgt gaacatcatt ggccagatcc attttcagtg gtctggattt 4500
   ctttttattt tcttttcaac ttgaaagaaa ctggacatta ggccactatg tgttgttact 4560
   gccactagtg ttcaagtgcc tcttgttttc ccagagattt cctgggtctg ccagaggccc 4620
   agacaggctc actcaagctc tttaactgaa aagcaacaag ccactccagg acaaggttca 4680
   aaatggttac aacagcctct acctgtcgcc ccagggagaa aggggtagtg atacaagtct 4740
   catagccaga gatggttttc cactccttct agatattccc aaaaagaggc tgagacagga 4800
   ggttattttc aattttattt tggaattaaa tacttttttc cctttattac tgttgtagtc 4860
   cctcacttgg atatacctct gttttcacga tagaaataag ggaggtctag agcttctatt 4920
   ccttggccat tgtcaacgga gagctggcca agtcttcaca aacccttgca acattgcctg 4980
   aagtttatgg aataagatgt attctcactc ccttgatctc aagggcgtaa ctctggaagc 5040
   acagcttgac tacacgtcat ttttaccaat gattttcagg tgacctgggc taagtcattt 5100
   aaactgggtc tttataaaag taaaaggcca acatttaatt attttgcaaa gcaacctaag 5160
   agctaaagat gtaatttttc ttgcaattgt aaatcttttg tgtctcctga agacttccct 5220
   taaaattagc tctgagtgaa aaatcaaaag agacaaaaga catcttcgaa tccatatttc 5280
   aagcctggta gaattggctt ttctagcaga acctttccaa aagttttata ttgagattca 5340
   taacaacacc aagaattgat tttgtagcca acattcattc aatactgtta tatcagagga 5400
   gtaggagaga ggaaacattt gacttatctg gaaaagcaaa atgtacttaa gaataagaat 5460
   aacatggtcc attcaccttt atgttataga tatgtctttg tgtaaatcat ttgttttgag 5520
   ttttcaaaga atagcccatt gttcattctt gtgctgtaca atgaccactg ttattgttac 5580
   tttgactttt cagagcacac ccttcctctg gtttttgtat atttattgat ggatcaataa 5640
   taatgaggaa agcatgatat gtatattgct gagttgaaag cacttattgg aaaatattaa 5700
   aaggctaaca ttaaaagact aaaggaaaca gaaaaaaaaa aaaaaaaa 5748

<210> 203
<211> 2799
<212> DNA
<213> Homo sapiens

<400> 203
agcttcgagg ccagtgggag gagggagggg ccaggcagct gagggccagg aaagatgtga 60
aaaactctag ctggtgaccg agaggaggag tagagtgtgc ccttagttca tatgaactag 120
agggagttgg tatttgcaca gcagtcaggg tcacatgagt gatcatggta cagtgagaag 180
ttctccctcc cagggccagg tcacagggtt tgtttctgtt caatccggat tcttccagta 240

aaagcttcaa cttcccacac tgaagctgag agcctcccaa agtgctggct acctgctgag 300
cgcccccgta actctgacac agtagtaatt tgagcctctg caattgccgt ctgcttcctg 360
tgaaagtcct ttccgtgccc actgaccctt gagtgggcct ttgagctgct gactttcagc 420
tggaacttga agggacccca accctgagac actatggccc tgacctcaga cctggggaaa 480
cagataaaac tgaaagaggt ggaggggacc ctcctgcagc ctgcaactgt ggacaactgg 540
agccagatcc agagcttcga ggccaaacca gatgatctcc tcatctgcac ctaccctaaa 600
gcagggacaa cgtggattca ggaaattgtg gatatgattg aacagaatgg ggacgtggag 660
aagtgccagc gagccatcat ccaacaccgc catcctttca ttgagtgggc tcggccaccc 720
caaccttctg gtgtggaaaa agccaaagca atgccctctc cacggatact aaagactcac 780
ctttccactc agctgctgcc accgtctttc tgggaaaaca actgcaagtt cctttatgta 840
gctcgaaatg ccaaagactg tatggtttcc tactaccatt tccaaaggat gaaccacatg 900
cttcctgacc ctggtacctg ggaagagtat tttgaaacct tcatcaatgg aaaagtggtt 960
tggggttcct ggtttgacca cgtgaaagga tggtgggaga tgaaagacag acaccagatt 1020
ctcttcctct tctatgagga cataaagagg gacccaaagc atgaaattcg gaaggtgatg 1080
cagttcatgg gaaagaaggt ggatgaaaca gtgctagata aaattgtcca ggagacgtca 1140
tttgagaaaa tgaaagaaaa tcccatgaca aatcgttcta cagtttccaa atctatcttg 1200
gaccagtcaa tttcctcctt catgagaaaa ggaactgtgg gggattggaa aaaccacttc 1260
actgttgccc agaatgagag gtttgatgaa atctatagaa gaaagatgga aggaacctcc 1320
ataaacttct gcatggaact ctgagcaaga tgtaaataaa attaaaaggt ggatggcaag 1380
agtgcaaata ctatcttcaa tccttcagtc ccagccagaa gaatctctga aagcatattg 1440
tgaatgtata caatgtagta caaacaatct ctgtgatgat taacagtatg tcaccacttc 1500
attttttaaa aaggatcacg tctaatgccc attttcccaa ctattctttc caaagtaaga 1560
tataaggtag cttaataaac taagtaaaac gtatgacttg agtacaaaag gattgtttta 1620
atccccatta ttctggaaag tgcatcctag tctcccagtc tataacatca taataccttg 1680
agtataagtc caaatattag gttatatcta tattaaaaac aaaatttctg tcatctgtcc 1740
tggccattca ggcaactcca gcctgggctc aatcctggag ttctgtctgg tcactatcag 1800
aaggaacact ttgagggaaa ccctggtgca gccagccctg aggaaacatg gcctgagtgc 1860
cctcactggt gggtgggaat aaaatggaag tgcacagagg agatgtcaga agaccaaaac 1920
ttggtgaata gtcccagtgc taggtcatat aggaaacaga aagcatgaca gtggcctttt 1980
gggaacccaa gttacgtcct ggtgaaagca gaaaggagga gacaggatgg ctgtcaacaa 2040
cgtcagcatg ggcatggctc ccaggcatgg aggtaattgg gtcttggctt caatgccacc 2100
cttttgggaa gccctaaacc aagtgaggtg ttcttttccc ttgcttccca tatttgtccc 2160
agttgcagcc ctgttgttat gtatttactc ttcaatttct tatttatacc tctcactcaa 2220
ttgttaattc tttgggttca gtctcatgcc atatttactc ctagtaccta atgcagtgcc 2280

tggcacacag caagtgccct tgagaacttg tagagggagt aaaaaagtgc atggataaat 2340

gactgcagca aatcacaaat tttgaacttt gacccttttc ttttagtccc aaatagtggc 2400

attccatagc tgagggtagt aggagcagtt caccctggga gcagacaata agaggaagaa 2460

tgaagccaac taaaattctg aatgcttttt gttggatttc gtagggtatt ttttttaaaa 2520

tagggtcttg ctatgttgcc caggctggtc ttaactgact gctttttatt attaccatgc 2580

actggcaatt ccaaacaatg tcagtgttaa aatgcttctc cctgaaaaag agaaaaaaaa 2640

aaggaaaaaa agaaaagaaa agtgaaaaga aaaactctta ttggcctaag ttctaaataa 2700

tagctaggtt accactgagt tttaactata tgtatatgag cttcaaataa gcaccttttt 2760

attatgtaaa taaataaaag ttatctgtat ccccaaaaa 2799

<210> 204
<211> 1504
<212> DNA
<213> Homo sapiens

<400> 204
ggttgaggtc aagtagtagc gttgggctgc ggcagcggag gagctcaaca tgcgtgagtg 60

tatctctatc cacgtggggc aggcaggagt ccagatcggc aatgcctgct gggaactgta 120

ctgcctggaa catggaattc agcccgatgg tcagatgcca agtgataaaa ccattggtgg 180

tggggacgac tccttcaaca cgttcttcag tgagactgga gctggcaagc acgtgcccag 240

agcagtgttt gtggacctgg agcccactgt ggtcgatgaa gtgcgcacag gaacctatag 300

gcagctcttc cacccagagc agctgatcac cgggaaggaa gatgcggcca ataattacgc 360

cagaggccat tacaccatcg gcaaggagat cgtcgacctg gtcctggacc ggatccgcaa 420

actggcggat ctgtgcacgg gactgcaggg cttcctcatc ttccacagtt ttgggggtgg 480

cactggctct gggttcgcat ctctgctcat ggagcggctc tcagtggatt acggcaagaa 540

gtccaagcta gaatttgcca tttacccagc cccccaggtc tccacggccg tggtggagcc 600

ctacaactcc atcctgacca cccacacgac cctggaacat tctgactgtg ccttcatggt 660

cgacaatgaa gccatctatg acatatgtcg gcgcaacctg gacatcgagc gtcccacgta 720

caccaacctc aatcgcctga ttgggcagat cgtgtcctcc atcacggcct ccctgcgatt 780

tgacggggcc ctgaatgtgg acttgacgga attccagacc aacctagtgc cgtacccccg 840

catccacttc cccctggcca cctacgcccc ggtcatctca gccgagaagg cctaccacga 900

gcagctgtcc gtggctgaga tcaccaatgc ctgcttcgag ccagccaatc agatggtcaa 960

gtgtgaccct cgccacggca agtacatggc ctgctgcatg ttgtacaggg gggatgtggt 1020

cccgaaagat gtcaacgcgg ccatcgccac catcaagacc aagcgcacca tccagtttgt 1080

agattggtgc ccaactggat ttaaggtggg cattaactac cagcccccca cggtggtccc 1140

tgggggagac ctggccaagg tgcagcgggc tgtgtgcatg ctgagcaaca ccacggccat 1200

cgcggaggcc tgggctcgcc tggaccataa gttcgatctc atgtatgcca agcgggcctt 1260

tgtgcactgg tacgtgggag aaggcatgga ggagggggag ttctctgagg cccgcgagga 1320

cctggcagct ctggagaagg attatgaaga ggtgggcgtg gattccgtgg aagccgaggc 1380

tgaagaaggt gaagaatact gaggggaggg tgtggtgggt tctccactcc actgccaccc 1440

ccagcgtggc tgctttcaag ttctttgcaa ttaaaggttc tgtataaaaa aaaaaaaaaa 1500

aaaa 1504

<210> 205
<211> 3426
<212> DNA
<213> Homo sapiens

<400> 205
   gagcaatgat gtagccacct cctaaccttc ccttcttgaa cccccaggtc ccctcttgct 60
   gttggctgca catcaggaag gctgtgatgg gaatgaaggt gaaaacttgg agatttcact 120
   tcagtcattg cttctgcctg caagatcatc ctttaaaagt agagaagctg ctctgtgtgg 180
   tggttaactc caagaggcag aactcgttct agaaggaaat ggatgcaagc agctccgggg 240
   gccccaaacg catgcttcct gtgatctagc ccagggaagc ccttccgtgg gggccccggc 300
   tttgagggat gccaccggtt ctggacgcat ggctgattct gaatgatgat ggttcgccgg 360
   gggctgcttg cgtggatttc ccgggtggtg gttttgctgg tgctcctctg ctgtgctatc 420
   tctgtcctgt acatgttggc ctgcacccca aaaggtgacg aggagcagct ggcactgccc 480
   agggccaaca gccccacggg gaaggagggg taccaggccg tccttcagga gtgggaggag 540
   cagcaccgca actacgtgag cagcctgaag cggcagatcg cacagctcaa ggaggagctg 600
   caggagagga gtgagcagct caggaatggg cagtaccaag ccagcgatgc tgctggcctg 660
   ggtctggaca ggagcccccc agagaaaacc caggccgacc tcctggcctt cctgcactcg 720
   caggtggaca aggcagaggt gaatgctggc gtcaagctgg ccacagagta tgcagcagtg 780
   cctttcgata gctttactct acagaaggtg taccagctgg agactggcct tacccgccac 840
   cccgaggaga agcctgtgag gaaggacaag cgggatgagt tggtggaagc cattgaatca 900
   gccttggaga ccctgaacaa tcctgcagag aacagcccca atcaccgtcc ttacacggcc 960
   tctgatttca tagaagggat ctaccgaaca gaaagggaca aagggacatt gtatgagctc 1020
   accttcaaag gggaccacaa acatgaattc aaacggctca tcttatttcg accattcggc 1080
   cccatcatga aagtgaaaaa tgaaaagctc aacatggcca acacgcttat caatgttatc 1140
   gtgcctctag caaaaagggt ggacaagttc cggcagttca tgcagaattt cagggagatg 1200
   tgcattgagc aggatgggag agtccatctc actgttgttt actttgggaa agaagaaata 1260
   aatgaagtca aaggaatact tgaaaacact tccaaagctg ccaacttcag gaactttacc 1320
   ttcatccagc tgaatggaga attttctcgg ggaaagggac ttgatgttgg agcccgcttc 1380
   tggaagggaa gcaacgtcct tctctttttc tgtgatgtgg acatctactt cacatctgaa 1440
   ttcctcaata cgtgtaggct gaatacacag ccagggaaga aggtatttta tccagttctt 1500
   ttcagtcagt acaatcctgg cataatatac ggccaccatg atgcagtccc tcccttggaa 1560
   cagcagctgg tcataaagaa ggaaactgga ttttggagag actttggatt tgggatgacg 1620
   tgtcagtatc ggtcagactt catcaatata ggtgggtttg atctggacat caaaggctgg 1680
   ggcggagagg atgtgcacct ttatcgcaag tatctccaca gcaacctcat agtggtacgg 1740
   acgcctgtgc gaggactctt ccacctctgg catgagaagc gctgcatgga cgagctgacc 1800
   cccgagcagt acaagatgtg catgcagtcc aaggccatga acgaggcatc ccacggccag 1860
   ctgggcatgc tggtgttcag gcacgagata gaggctcacc ttcgcaaaca gaaacagaag 1920
   acaagtagca aaaaaacatg aactcccaga gaaggattgt gggagacact ttttctttcc 1980
   ttttgcaatt actgaaagtg gctgcaacag agaaaagact tccataaagg acgacaaaag 2040
   aattggactg atgggtcaga gatgagaaag cctccgattt ctctctgttg ggctttttac 2100
   aacagaaatc aaaatctccg ctttgcctgc aaaagtaacc cagttgcacc ctgtgaagtg 2160
   tctgacaaag gcagaatgct tgtgagatta taagcctaat ggtgtggagg ttttgatggt 2220
   gtttacaata cactgagacc tgttgttttg tgtgctcatt gaaatattca tgatttaaga 2280
   gcagttttgt aaaaaattca ttagcatgaa aggcaagcat atttctcctc atatgaatga 2340
   gcctatcagc agggctctag tttctaggaa tgctaaaata tcagaaggca ggagaggaga 2400
   taggcttatt atgatactag tgagtacatt aagtaaaata aaatggacca gaaaagaaaa 2460
   gaaaccataa atatcgtgtc atattttccc caagattaac caaaaataat ctgcttatct 2520
   ttttggttgt ccttttaact gtctccgttt ttttctttta tttaaaaatg cacttttttt 2580
   cccttgtgag ttatagtctg cttatttaat taccactttg caagccttac aagagagcac 2640
   aagttggcct acatttttat attttttaag aagatacttt gagatgcatt atgagaactt 2700
   tcagttcaaa gcatcaaatt gatgccatat ccaaggacat gccaaatgct gattctgtca 2760
   ggcactgaat gtcaggcatt gagacatagg gaaggaatgg tttgtactaa tacagacgta 2820
   cagatacttt ctctgaagag tattttcgaa gaggagcaac tgaacactgg aggaaaagaa 2880
   aatgacactt tctgctttac agaaaaggaa actcattcag actggtgata tcgtgatgta 2940
   cctaaaagtc agaaaccaca ttttctcctc agaagtaggg accgctttct tacctgttta 3000
   aataaaccaa agtataccgt gtgaaccaaa caatctcttt tcaaaacagg gtgctcctcc 3060
   tggcttctgg cttccataag aagaaatgga gaaaaatata tatatatata tatatattgt 3120
   gaaagatcaa tccatctgcc agaatctagt gggatggaag tttttgctac atgttatcca 3180
   ccccaggcca ggtggaagta actgaattat tttttaaatt aagcagttct actcgatcac 3240
   caagatgctt ctgaaaattg cattttatta ccatttcaaa ctatttttta aaaataaata 3300
   cagttaacat agagtggttt cttcattcat gtgaaaatta ttagccagca ccagatgcat 3360
   gagctaatta tctctttgag tccttgcttc tgtttgctca cagtaagctc attgtttaaa 3420
   agcttc 3426

<210> 206
<211> 519

<212>  DNA
<213>  Homo sapiens

<400>  206
agcatagttt catttcctgc tcttgaatat ctggttgaac tacttaagct taatttgtta      60

aactccggta agtacctagc ccacatgatt tgactcagag attctctttt gtccacagac     120

agtcatctca ggagcagaaa gaaaagagct cccaaatgct atatctattc aggggctctc     180

aagaacaatg gaatatcatc ctgatttaga aaatttggat gaagatggat atactcaatt     240

acacttcgac tctcaaagca ataccaggat agctgttgtt tcagagaaag gatcgtgtgc     300

tgcatctcct ccttggcgcc tcattgctgt aattttggga atcctatgct tggtaatact     360

ggtgatagct gtggtcctgg gtaccatggc tggtttcaaa gctgtggaat tcaaaggata     420

aattaatgaa gaaaacaagc ggagctgaag aagaaagtac aatatggtgc tgtcttccta     480

atgaaataaa ttcactaaat ggacattaaa aaaaaaaaa                            519


<210>  207
<211>  55
<212>  DNA
<213>  artificial sequence

<220>
<223>  preferred oligonucleotide for gene A4GALT

<400>  207
cgcccgcatt ataaacacag gagaataatc aatagaataa aagtgaccga ctgtc           55


<210>  208
<211>  63
<212>  DNA
<213>  artificial sequence

<220>
<223>  preferred oligonucleotide for gene A4GNT

<400>  208
atcgcaagca ctgtcccagg acttacaggg acctgattaa aggcccagag gggtcagtga      60

ctg                                                                    63


<210>  209
<211>  50
<212>  DNA
<213>  artificial sequence

<220>
<223>  preferred oligonucleotide for gene ACT

<400>  209
aatatgagat gcattgttac aggaagtccc ttgccatcct aaaagccacc                 50


<210>  210
<211>  50
<212>  DNA
<213>  artificial sequence

<220>
<223>  preferred oligonucleotide for gene AFG3L2

<400>  210
cagaccattt gcggaaaaat ctacctatga agaatttgtg gaaggcactg                50

<210>  211
<211>  50
<212>  DNA
<213>  artificial sequence

<220>
<223>  preferred oligonucleotide for gene ANXA4

<400>  211
ctatttgcac tgtgcctttc aactccagaa acattctaag atgtacttgg                50

<210>  212
<211>  50
<212>  DNA
<213>  artificial sequence

<220>
<223>  preferred oligonucleotide for gene ARAF

<400>  212
atacttctaa attttgggag ctcctccatc tccaatggct gggatttgtg                50

<210>  213
<211>  50
<212>  DNA
<213>  artificial sequence

<220>
<223>  preferred oligonucleotide for gene B2M

<400>  213
gaaccatgtg actttgtcac agcccaagat agttaagtgg gatcgagaca                50

<210>  214
<211>  52
<212>  DNA
<213>  artificial sequence

<220>
<223>  preferred oligonucleotide for gene B3GALNT1

<400>  214
aacaaggtga tgtctgtttc ccagggctca gacctaatcc agttataata aa             52

<210>  215
<211>  52
<212>  DNA
<213>  artificial sequence

<220>
<223>  preferred oligonucleotide for gene B3GALNT2

<400>  215
atccttgtcg atgtcaagca agataacagg gacttgaatt agcagagtct aa             52

<210>  216
<211>  51
<212>  DNA

<213>  artificial sequence

<220>
<223>  preferred oligonucleotide for gene B3GALT1

<400>  216
aagtgacttc caaacaactc ttaggattga cgtaccgtgc atctgagata a          51


<210>  217
<211>  53
<212>  DNA
<213>  artificial sequence

<220>
<223>  preferred oligonucleotide for gene B3GALT2

<400>  217
atacgggaag tttatttcct gacagcaggt gtacacatgc caacacttaa tca        53


<210>  218
<211>  51
<212>  DNA
<213>  artificial sequence

<220>
<223>  preferred oligonucleotide for gene B3GALT4

<400>  218
catcgggaag agaaaaagaa aaaaatgctg cagttgttct ctcaagctag g          51


<210>  219
<211>  55
<212>  DNA
<213>  artificial sequence

<220>
<223>  preferred oligonucleotide for gene B3GALT5

<400>  219
ccaaggtact tcgttagttt tgcccattca gccatggtca cgtgacatgc aaagt      55


<210>  220
<211>  51
<212>  DNA
<213>  artificial sequence

<220>
<223>  preferred oligonucleotide for gene B3GALT6

<400>  220
cccccaacag gaagagtcta aaaatccagt ttgcaaccac ttctgaccta c          51


<210>  221
<211>  52
<212>  DNA
<213>  artificial sequence

<220>
<223>  preferred oligonucleotide for gene B3GNT1

<400>  221
ttgccgctaa caaccatatc gtgttagtga attttcaata tggaccagga ag         52

<210> 222
<211> 52
<212> DNA
<213> artificial sequence

<220>
<223> preferred oligonucleotide for gene B3GNT2

<400> 222
ttgccgctaa caaccatatc gtgttagtga attttcaata tggaccagga ag 52


<210> 223
<211> 51
<212> DNA
<213> artificial sequence

<220>
<223> preferred oligonucleotide for gene B3GNT3

<400> 223
gtttgagtca aagtcttact tccctgctct cacctactca cagacgggat g 51


<210> 224
<211> 53
<212> DNA
<213> artificial sequence

<220>
<223> preferred oligonucleotide for gene B3GNT4

<400> 224
ctacagaaaa tgccaacttg gttttttaac tcctctcacc ctgttagctc tga 53


<210> 225
<211> 50
<212> DNA
<213> artificial sequence

<220>
<223> preferred oligonucleotide for gene B3GNT5

<400> 225
aaaacataag tgtctctggc catcaaagtg atcttgttta cagcagtgct 50


<210> 226
<211> 58
<212> DNA
<213> artificial sequence

<220>
<223> preferred oligonucleotide for gene B3GNT6

<400> 226
aggtagaaat cttcaccgtg caaagtggaa accagaggcc cggtcagaca gtgactaa 58


<210> 227
<211> 58
<212> DNA
<213> artificial sequence

<220>
<223> preferred oligonucleotide for gene B3GNT7

<400>  227
tcgtgggcga tgtcctgcag cacgctcggc ccattcgcag gaaagacaac aaatacta        58


<210>  228
<211>  58
<212>  DNA
<213>  artificial sequence

<220>
<223>  preferred oligonucleotide for gene B3GNT8

<400>  228
gagtccttct tcgaaggtgg ctacccagcc tatgcaagcg ggggtggcta cgtcattg        58


<210>  229
<211>  50
<212>  DNA
<213>  artificial sequence

<220>
<223>  preferred oligonucleotide for gene B3GNTL1

<400>  229
gaacaagatc aggaaaggct tctattgcca cgaggactct caggaaagac                50


<210>  230
<211>  51
<212>  DNA
<213>  artificial sequence

<220>
<223>  preferred oligonucleotide for gene B4GALNT1

<400>  230
ggagacatgt caggaataga ttaggcaccc ctcttcctta atgaaatgtg g              51


<210>  231
<211>  51
<212>  DNA
<213>  artificial sequence

<220>
<223>  preferred oligonucleotide for gene B4GALNT2

<400>  231
taaagacaac cacgtggagt attacactat gccctttggg aagggttggt t              51


<210>  232
<211>  70
<212>  DNA
<213>  artificial sequence

<220>
<223>  preferred oligonucleotide for gene B4GALTN3

<400>  232
gttccaggtc accggtgacc cacacttcaa catcgtcatc actgactata gcagtgagga     60

catggatgtt                                                            70


<210>  233

<211>  53
<212>  DNA
<213>  artificial sequence

<220>
<223>  preferred oligonucleotide for gene B4GALNT4

<400>  233
tccgactgcg gaatttctat caccactacc actccaagag gggcatgtgg agc          53


<210>  234
<211>  51
<212>  DNA
<213>  artificial sequence

<220>
<223>  preferred oligonucleotide for gene B4GALT1

<400>  234
agtatgtggt tcattcttag aaccaaggga aatactgctc cccccatttg c          51


<210>  235
<211>  50
<212>  DNA
<213>  artificial sequence

<220>
<223>  preferred oligonucleotide for gene B4GALT2

<400>  235
gtacattgca cagaaacttg tgtgggtgct ttagtaaaaa acgtgaatgg          50


<210>  236
<211>  51
<212>  DNA
<213>  artificial sequence

<220>
<223>  preferred oligonucleotide for gene B4GALT3

<400>  236
ctgtctacct taatcatgaa accgaattca tggggttgta ttctccccac c          51


<210>  237
<211>  51
<212>  DNA
<213>  artificial sequence

<220>
<223>  preferred oligonucleotide for gene B4GALT4

<400>  237
aggtatgcct tccagtgatg cccaccagag aatacattct ctattagttt t          51


<210>  238
<211>  52
<212>  DNA
<213>  artificial sequence

<220>
<223>  preferred oligonucleotide for gene B4GALT5

<400>  238

tgaccttgtg catatattgg tagctgaaaa tcttcaaggc tactgatggg tg 52

<210> 239
<211> 50
<212> DNA
<213> artificial sequence

<220>
<223> preferred oligonucleotide for gene B4GALT6

<400> 239
taatatgttt ctgtgagagt gacagagctg tgcccgttta tcttcattgt 50

<210> 240
<211> 51
<212> DNA
<213> artificial sequence

<220>
<223> preferred oligonucleotide for gene B4GALT7

<400> 240
tgggcctagt gttgccagat cttctgattt ttcgaaagaa actagaatgc t 51

<210> 241
<211> 51
<212> DNA
<213> artificial sequence

<220>
<223> preferred oligonucleotide for gene C1GALT1

<400> 241
gtatttcttc cagaagaagc taaaataaga ctggcactta ccctgaagtg c 51

<210> 242
<211> 55
<212> DNA
<213> artificial sequence

<220>
<223> preferred oligonucleotide for gene C1GALT1C1

<400> 242
ctgtccccaa gccgttctag acgcgggaaa aatgctttct gaaagcagct ccttt 55

<210> 243
<211> 55
<212> DNA
<213> artificial sequence

<220>
<223> preferred oligonucleotide for gene CD44

<400> 243
tggcaagtcc tttgtggcat ttattcatca gtcagggtgt ccgattggtc ctaga 55

<210> 244
<211> 60
<212> DNA
<213> artificial sequence

<220>
<223>  preferred oligonucleotide for gene CD69

<400>  244
tgtgcaatat gtgatgtggc aaatctctat taggaaatat tctgtaatct tcagacctag      60


<210>  245
<211>  50
<212>  DNA
<213>  artificial sequence

<220>
<223>  preferred oligonucleotide for gene CDC2L1

<400>  245
aacgcttctt cggtgaagtt cttttgtact tccaaatgtc gcagtctgat                 50


<210>  246
<211>  51
<212>  DNA
<213>  artificial sequence

<220>
<223>  preferred oligonucleotide for gene CDC2L2

<400>  246
gtaccgcgcc ccagagctgc tgcttggtgc caaggaatac tccacggccg t               51


<210>  247
<211>  51
<212>  DNA
<213>  artificial sequence

<220>
<223>  preferred oligonucleotide for gene CDH17

<400>  247
ttggtctccc aatacaggca tgaaccactg cacccaccta cttagatatt t               51


<210>  248
<211>  70
<212>  DNA
<213>  artificial sequence

<220>
<223>  preferred oligonucleotide for gene CHPF

<400>  248
tctcccaact tctccctttt ggaccctgcc gaagctccct gcctttaata aactggccaa      60

gtgtgaaaaa                                                             70


<210>  249
<211>  52
<212>  DNA
<213>  artificial sequence

<220>
<223>  preferred oligonucleotide for gene CHST4

<400>  249
gaggcctatt aagcacgaca cagtatcagt ggaattgatc cataaacctc cc              52

<210>  250
<211>  54
<212>  DNA
<213>  artificial sequence

<220>
<223>  preferred oligonucleotide for gene CHST5

<400>  250
acctcttctg ggcgcaggga gtaagttact gctaaattaa attaaatgtg tgcc          54


<210>  251
<211>  51
<212>  DNA
<213>  artificial sequence

<220>
<223>  preferred oligonucleotide for gene CHST6

<400>  251
actatgggat ggtaaatatc cgagaggaag ctgatgcaac aggatttgtc t             51


<210>  252
<211>  50
<212>  DNA
<213>  artificial sequence

<220>
<223>  preferred oligonucleotide for gene CHST8

<400>  252
ttacctgatg ttcaactatt ccaagccctt tgcagatctg tactgagggg               50


<210>  253
<211>  55
<212>  DNA
<213>  artificial sequence

<220>
<223>  preferred oligonucleotide for gene CHST9

<400>  253
ggcacaaagt tataggaaaa tcacctacag gagatgtaaa caacttgagt tgctc         55


<210>  254
<211>  67
<212>  DNA
<213>  artificial sequence

<220>
<223>  preferred oligonucleotide for gene CHSY1

<400>  254
ttgttttcat ttagatttca aacagtgata gacttgccat tttaatacac gtcattggag    60

ggctgcg                                                              67


<210>  255
<211>  70
<212>  DNA
<213>  artificial sequence

<220>
<223>  preferred oligonucleotide for gene CHSY-2/CSS1

<400>  255
aaaggtacca caaaagtgct ttatgcttcc tatggggaag ggactctgta acataaactt      60

gagttttgta                                                              70


<210>  256
<211>  70
<212>  DNA
<213>  artificial sequence

<220>
<223>  preferred oligonucleotide for gene CLEC2B

<400>  256
tttacccacc actgtcacac accaatcaga acttgccagc tcctcaaaac tttactaggg      60

ccaataaact                                                              70


<210>  257
<211>  70
<212>  DNA
<213>  artificial sequence

<220>
<223>  preferred oligonucleotide for gene CLEC7A

<400>  257
tgggtaccat ggctggtttc aaagctgtgg aattcaaagg ataaattaat gaagaaaaca      60

agcggagctg                                                              70


<210>  258
<211>  70
<212>  DNA
<213>  artificial sequence

<220>
<223>  preferred oligonucleotide for gene CMAH

<400>  258
tggaaaacaa agcatagctg tagtagatac gaatccaatc acagaggaaa caggaagaga      60

aaaacatcca                                                              70


<210>  259
<211>  50
<212>  DNA
<213>  artificial sequence

<220>
<223>  preferred oligonucleotide for gene CMAS

<400>  259
ctcagaaagg gcctgttcaa agcagacgct gtcttcttta aaactggatt                  50


<210>  260
<211>  70
<212>  DNA
<213>  artificial sequence

<220>
<223>  preferred oligonucleotide for gene CS-GALNACT1

<400>  260
catgtgaaaa ttattagcca gcaccagatg catgagctaa ttatctcttt gagtccttgc      60

ttctgtttgc                                                              70


<210>  261
<211>  70
<212>  DNA
<213>  artificial sequence

<220>
<223>  preferred oligonucleotide for gene CS-GALNACT2

<400>  261
cactctgatt tgagaaaagg gaggagggga agatagtctg aatggaaatc tgaaatacgg      60

aatgttttag                                                              70


<210>  262
<211>  55
<212>  DNA
<213>  artificial sequence

<220>
<223>  preferred oligonucleotide for gene CSS2

<400>  262
gcgttcgccg ctttggtagg gctcagtgtg tgtggtcttt tcagatgcaa caact           55


<210>  263
<211>  50
<212>  DNA
<213>  artificial sequence

<220>
<223>  preferred oligonucleotide for gene CXADR

<400>  263
gtgtcctcca tcaattctgt attccagact tgggaggatg tacagttgct                 50


<210>  264
<211>  50
<212>  DNA
<213>  artificial sequence

<220>
<223>  preferred oligonucleotide for gene DPAGT1

<400>  264
gtgttctgat tagtaagaac taagcagggg gccacatgct ctcaatggag                 50


<210>  265
<211>  50
<212>  DNA
<213>  artificial sequence

<220>
<223>  preferred oligonucleotide for gene DSP

<400>  265

gcgtctgaag tgctaatcag ttgtaacaat agcacaaatc gaacttagga 50

<210> 266
<211> 51
<212> DNA
<213> artificial sequence

<220>
<223> preferred oligonucleotide for gene ECOP

<400> 266
gttagaattg tgctactgtg aacgctgatc ctgcatatgg aagtcccact t 51

<210> 267
<211> 52
<212> DNA
<213> artificial sequence

<220>
<223> preferred oligonucleotide for gene ETFB

<400> 267
gggcggattt gagcccctcc cagagatggc aataaaactg actctcaaca tc 52

<210> 268
<211> 70
<212> DNA
<213> artificial sequence

<220>
<223> preferred oligonucleotide for gene FPGT

<400> 268
tctacagctt cacattcaaa ctcttcagca ctttcagagc tattgatatg catatgtcca 60

cctagtacca 70

<210> 269
<211> 52
<212> DNA
<213> artificial sequence

<220>
<223> preferred oligonucleotide for gene FUCA1

<400> 269
gtccccgcag agtttgcttg gactataaag ctgacaggag tgaagtaatc at 52

<210> 270
<211> 65
<212> DNA
<213> artificial sequence

<220>
<223> preferred oligonucleotide for gene FUCA2

<400> 270
ttacaaaatc ctagatggca tagcctacta cacacctaat gtgtatggta tagactgttg 60

ctcct 65

<210> 271

<211>  70
<212>  DNA
<213>  artificial sequence

<220>
<223>  preferred oligonucleotide for gene FucT1/SLC35C1

<400>  271
atgtcctttc tcttggggtg atttctgatg tttttactct atatagtgaa aagctaggga      60

gagcgggtct                                                              70


<210>  272
<211>  55
<212>  DNA
<213>  artificial sequence

<220>
<223>  preferred oligonucleotide for gene FUK

<400>  272
tgccatccca ggccttaact agcaaaacta cggagcgtgc caagtgacct ggtgc           55


<210>  273
<211>  51
<212>  DNA
<213>  artificial sequence

<220>
<223>  preferred oligonucleotide for gene FUT1

<400>  273
tttggagggt gaaagaagaa accttaaaca tagtcagctc tgatcacatc c               51


<210>  274
<211>  60
<212>  DNA
<213>  artificial sequence

<220>
<223>  preferred oligonucleotide for gene FUT10

<400>  274
cagccgagca tgaacatgaa atacattaga acttggcaat gagagatccc atctgttggt      60


<210>  275
<211>  50
<212>  DNA
<213>  artificial sequence

<220>
<223>  preferred oligonucleotide for gene FUT11

<400>  275
attctaccat gggacataag gagcatccac tgcacaaacc cttaatgaac                 50


<210>  276
<211>  50
<212>  DNA
<213>  artificial sequence

<220>
<223>  preferred oligonucleotide for gene FUT2

<400>  276
ttttaatcac cagtgtgcat acagtcatgg agctgggtat tcccagctac                50


<210>  277
<211>  50
<212>  DNA
<213>  artificial sequence

<220>
<223>  preferred oligonucleotide for gene FUT3

<400>  277
cacggtttcc agatgtaata ctgcaaggaa aaatgatgac gtgtctcctc                50


<210>  278
<211>  53
<212>  DNA
<213>  artificial sequence

<220>
<223>  preferred oligonucleotide for gene FUT4

<400>  278
cattctacta actggcaccc taaggttgaa agtgcttatc tgctttccaa tgt            53


<210>  279
<211>  50
<212>  DNA
<213>  artificial sequence

<220>
<223>  preferred oligonucleotide for gene FUT5

<400>  279
gtggttttat tgctgttgtt aaccacccat gaggggtgca aacagataat                50


<210>  280
<211>  52
<212>  DNA
<213>  artificial sequence

<220>
<223>  preferred oligonucleotide for gene FUT6

<400>  280
tctctattaa aaaatacaaa aattagtggg gcgtggtctc aggctcctgc ag             52


<210>  281
<211>  58
<212>  DNA
<213>  artificial sequence

<220>
<223>  preferred oligonucleotide for gene FUT7

<400>  281
agcctgaagt ttgtgggggc caaacctggg accccgagct tcctcggtag cagaggcc       58


<210>  282
<211>  60
<212>  DNA

<213>  artificial sequence

<220>
<223>  preferred oligonucleotide for gene FUT8

<400>  282
cggaacagct ccttactctg aggaagttga ttcttatttg atggtggtat tgtgaccact     60


<210>  283
<211>  50
<212>  DNA
<213>  artificial sequence

<220>
<223>  preferred oligonucleotide for gene FUT9

<400>  283
gcacatatga ccatgatgtt cagggcttta tagaaccaaa taaacctacc               50


<210>  284
<211>  56
<212>  DNA
<213>  artificial sequence

<220>
<223>  preferred oligonucleotide for gene FX/TSTA3

<400>  284
gaaggagacc tgtgcttggt tcactgacaa ctacgagcag gcccggaagt gaagct         56


<210>  285
<211>  50
<212>  DNA
<213>  artificial sequence

<220>
<223>  preferred oligonucleotide for gene GAK

<400>  285
aggcacatga agagaaagca ttccaaagcc tctgattgtt gtttcctttt               50


<210>  286
<211>  50
<212>  DNA
<213>  artificial sequence

<220>
<223>  preferred oligonucleotide for gene GAL3ST2

<400>  286
ggactggcgc ctgtacgagc atttcaaccg caccctctgg gcgcagctgc               50


<210>  287
<211>  50
<212>  DNA
<213>  artificial sequence

<220>
<223>  preferred oligonucleotide for gene GALE

<400>  287
tcctcccagg cactcattta tattgctctg aaagagcttt ccaaagtatt               50

<210> 288
<211> 50
<212> DNA
<213> artificial sequence

<220>
<223> preferred oligonucleotide for gene GALNT1

<400> 288
gaggaatctc attaagacat ttttcctgat atgtagagca gtctgttggc 50


<210> 289
<211> 53
<212> DNA
<213> artificial sequence

<220>
<223> preferred oligonucleotide for gene GALNT10

<400> 289
ggaccaactg atcaattgcc agtactaagc cactcattgt tatggcttcc ttt 53


<210> 290
<211> 50
<212> DNA
<213> artificial sequence

<220>
<223> preferred oligonucleotide for gene GALNT11

<400> 290
gagctgctgt taaggaattt cttgcttata gaggcaaacc acagtatcat 50


<210> 291
<211> 50
<212> DNA
<213> artificial sequence

<220>
<223> preferred oligonucleotide for gene GALNT12

<400> 291
agtatggatg gatgaattta aagagctcta ctaccatcgc aacccccgtg 50


<210> 292
<211> 55
<212> DNA
<213> artificial sequence

<220>
<223> preferred oligonucleotide for gene GALNT13

<400> 292
gccccaaaca tgcccatcac atgccctaga aggtaagtcc catgaaggca ggggc 55


<210> 293
<211> 50
<212> DNA
<213> artificial sequence

<220>
<223> preferred oligonucleotide for gene GALNT14

<400>  293
agtatggatg gatgaattta aagagctcta ctaccatcgc aacccccgtg                50

<210>  294
<211>  70
<212>  DNA
<213>  artificial sequence

<220>
<223>  preferred oligonucleotide for gene GALNT15/GALNTL2

<400>  294
aagtaccagc acaatttgag cattcccatt aacaaaggtg ttcacagttg agaaactctc      60

ctgccgggcg                                                            70

<210>  295
<211>  70
<212>  DNA
<213>  artificial sequence

<220>
<223>  preferred oligonucleotide for gene GALNT17

<400>  295
aataaagtgt ttctgacacc attttctgtt aatgggacaa tatttcgggg atgaagaaag      60

gaatgctccg                                                            70

<210>  296
<211>  50
<212>  DNA
<213>  artificial sequence

<220>
<223>  preferred oligonucleotide for gene GALNT2

<400>  296
gtgacttact gcggttgcgt tagtttctgt catcaggaca ctatctcacc                50

<210>  297
<211>  50
<212>  DNA
<213>  artificial sequence

<220>
<223>  preferred oligonucleotide for gene GALNT3

<400>  297
ctcagctgta cgaaaaagag catatactgg ttattgaccc tatcttctca                50

<210>  298
<211>  52
<212>  DNA
<213>  artificial sequence

<220>
<223>  preferred oligonucleotide for gene GALNT4

<400>  298
ccgattccct aattcgaatt tattggtacc aaagctctct ttggctatag ac             52

<210> 299
<211> 70
<212> DNA
<213> artificial sequence

<220>
<223> preferred oligonucleotide for gene GALNT5

<400> 299
gcttggaagg aaatttttct caaaagatcc tgaaagtagc tgcctgtgac ccagtgaagc 60

catatcaaaa 70


<210> 300
<211> 51
<212> DNA
<213> artificial sequence

<220>
<223> preferred oligonucleotide for gene GALNT6

<400> 300
cttgatcatc caaagcacct gcagagtcca gtgaaatgat atattctggc t 51


<210> 301
<211> 60
<212> DNA
<213> artificial sequence

<220>
<223> preferred oligonucleotide for gene GALNT7

<400> 301
tactgtaggc tgctttggaa ataattccca tatccttgct ttgtaagttg gtaatatcac 60


<210> 302
<211> 50
<212> DNA
<213> artificial sequence

<220>
<223> preferred oligonucleotide for gene GALNT8

<400> 302
tactatcacc taactgggga gctctatgtg ggacaactga ttgcagaggc 50


<210> 303
<211> 50
<212> DNA
<213> artificial sequence

<220>
<223> preferred oligonucleotide for gene GALNT9

<400> 303
agacctaagt cacacatcat aatgcagctc cttgtggtct ggtaagaggg 50


<210> 304
<211> 70
<212> DNA
<213> artificial sequence

<220>
<223>  preferred oligonucleotide for gene GALNTL1/GALNT16

<400>  304
cttgaccaaa gcatgtgcca ctagctgtcc ttgaggacct cgtctttatg aaacacacac      60

ctggaataaa                                                              70


<210>  305
<211>  51
<212>  DNA
<213>  artificial sequence

<220>
<223>  preferred oligonucleotide for gene GALNTL4

<400>  305
ggaaccaggt tagtgggccc ccaagaagag ctttttattt cctattcaat t               51


<210>  306
<211>  70
<212>  DNA
<213>  artificial sequence

<220>
<223>  preferred oligonucleotide for gene GALNTL5

<400>  306
gggttaaaag tctcctagtc attcaacata gtgtcacaag agtgtaagtt tggaacatcg      60

tggaattacg                                                              70


<210>  307
<211>  50
<212>  DNA
<213>  artificial sequence

<220>
<223>  preferred oligonucleotide for gene GAPDH

<400>  307
gaccactttg tcaagctcat ttcctggtat gacaacgaat ttggctacag                 50


<210>  308
<211>  55
<212>  DNA
<213>  artificial sequence

<220>
<223>  preferred oligonucleotide for gene GCNT1

<400>  308
tttctcatat taaatacaga cgctcctcaa cttatgatgt gggtaggtcc cagta           55


<210>  309
<211>  56
<212>  DNA
<213>  artificial sequence

<220>
<223>  preferred oligonucleotide for gene GCNT2

<400>  309

tactttaatg ttggaagagc atagtgtttg ggctagtggg tttctgacag cccatg 56

<210> 310
<211> 50
<212> DNA
<213> artificial sequence

<220>
<223> preferred oligonucleotide for gene GCNT3

<400> 310
gtgaaggagg gatatgtggt agagcacttg atttcagttg aatgcctgct 50

<210> 311
<211> 57
<212> DNA
<213> artificial sequence

<220>
<223> preferred oligonucleotide for gene GCNT4

<400> 311
cttctctggc aactgtccaa ctggaatcca gatttaaggt gataaaagct ctaagtt 57

<210> 312
<211> 52
<212> DNA
<213> artificial sequence

<220>
<223> preferred oligonucleotide for gene GLB1

<400> 312
atgaaagcct gtgtctttga gggattctac cctgaacata cctcacagat cc 52

<210> 313
<211> 70
<212> DNA
<213> artificial sequence

<220>
<223> preferred oligonucleotide for gene GNE

<400> 313
tctgttcttt ggtgtaatct ctcatggtga aatctgctct gtacatcaga caattgcatt 60

gctaccaagt 70

<210> 314
<211> 54
<212> DNA
<213> artificial sequence

<220>
<223> preferred oligonucleotide for gene GNPTG

<400> 314
ctgaccaggc ttgtgctcag agaagcagac aaaacaaaga ttcaaggttt taat 54

<210> 315
<211> 58
<212> DNA

<213>  artificial sequence

<220>
<223>  preferred oligonucleotide for gene GPAA1

<400>  315
tctggaagtg agatctgcct gtccgggctg ggacagagac tccccaagga ccccattc      58


<210>  316
<211>  51
<212>  DNA
<213>  artificial sequence

<220>
<223>  preferred oligonucleotide for gene HDAC1

<400>  316
gccaggtctg ggtgaaaggg atacttttat gcaaccataa gacaaactcc t             51


<210>  317
<211>  51
<212>  DNA
<213>  artificial sequence

<220>
<223>  preferred oligonucleotide for gene HSP90AB1(HSPCB)

<400>  317
gaaggcagta aactaagggt gtcaagcccc attccctctc tactcttgac a             51


<210>  318
<211>  55
<212>  DNA
<213>  artificial sequence

<220>
<223>  preferred oligonucleotide for gene  IGnT2

<400>  318
ctgcctggaa gaccttgtgg cctctgaagt tccctggaag tatgtcatca acacc         55


<210>  319
<211>  60
<212>  DNA
<213>  artificial sequence

<220>
<223>  preferred oligonucleotide for gene ITLN1

<400>  319
tcctcccaac catgagatcc caaggatgga gaacaactta cccagtagct agaatgttaa    60


<210>  320
<211>  52
<212>  DNA
<213>  artificial sequence

<220>
<223>  preferred oligonucleotide for gene ITLN2

<400>  320
tttggtgatg ctaagaagac tgcatcttat tactcaccgt atggtcaacg gg            52

<210>  321
<211>  51
<212>  DNA
<213>  artificial sequence

<220>
<223>  preferred oligonucleotide for gene KDELR1

<400>  321
atcccatggc tctcacacca tgtttttgta cagaactgat ggttgaatct t          51


<210>  322
<211>  50
<212>  DNA
<213>  artificial sequence

<220>
<223>  preferred oligonucleotide for gene KDELR2

<400>  322
ggtgctcgga cagaattctt accacagcaa aggcataaga tgcttgatac          50


<210>  323
<211>  51
<212>  DNA
<213>  artificial sequence

<220>
<223>  preferred oligonucleotide for gene KIAA0174

<400>  323
tgcaaagttt gtcttgacag ttgaaggcct cgcttagttg tactggattc t          51


<210>  324
<211>  58
<212>  DNA
<213>  artificial sequence

<220>
<223>  preferred oligonucleotide for gene LGALS1

<400>  324
tgtgcatcac cttcgaccag gccaacctga ccgtcaagct gccagatgga tacgaatt          58


<210>  325
<211>  70
<212>  DNA
<213>  artificial sequence

<220>
<223>  preferred oligonucleotide for gene LGALS10 / CLC

<400>  325
ccctgtctct acgtgaactt gggattccaa agccagctaa cagcatgatc ttttctcact          60

tcaatcctta          70


<210>  326
<211>  51
<212>  DNA
<213>  artificial sequence

<220>
<223>  preferred oligonucleotide for gene LGALS12

<400>  326
gggcctggac tgcactcaca gaggcaagtg ttgtagacta acaaagatac t          51


<210>  327
<211>  52
<212>  DNA
<213>  artificial sequence

<220>
<223>  preferred oligonucleotide for gene LGALS13

<400>  327
agtatgagat aaaggtcaat ggcatacgca tttacggctt tgtccatcga at          52


<210>  328
<211>  51
<212>  DNA
<213>  artificial sequence

<220>
<223>  preferred oligonucleotide for gene LGALS14

<400>  328
caaaccattt gagctgtgca tctatgtgcg tcacaaggaa tacaaggtaa t          51


<210>  329
<211>  50
<212>  DNA
<213>  artificial sequence

<220>
<223>  preferred oligonucleotide for gene LGALS2

<400>  329
attctaggat ataagcttga ccacgagttg agaccctgag cacaggcctc          50


<210>  330
<211>  51
<212>  DNA
<213>  artificial sequence

<220>
<223>  preferred oligonucleotide for gene LGALS3

<400>  330
cagcaaactg ggaatttctg gtgacataga cctcaccagt gcttcatata c          51


<210>  331
<211>  58
<212>  DNA
<213>  artificial sequence

<220>
<223>  preferred oligonucleotide for gene LGALS3BP

<400>  331
caccattgcc tacgaaaaca aagccctgat gctctgcgaa gggctcttcg tggcagac          58


<210>  332

<211>  52
<212>  DNA
<213>  artificial sequence

<220>
<223>  preferred oligonucleotide for gene LGALS4

<400>  332
gggccataac tcatgggaaa acagaattat cccctaggac tcctttctaa gc          52


<210>  333
<211>  58
<212>  DNA
<213>  artificial sequence

<220>
<223>  preferred oligonucleotide for gene LGALS7

<400>  333
cacggtgctg agaattcgcg gcttggttcc tcccaatgcc agcaggttcc atgtaaac    58


<210>  334
<211>  51
<212>  DNA
<213>  artificial sequence

<220>
<223>  preferred oligonucleotide for gene LGALS8

<400>  334
tttcatatta atgtatgcag agtctcacca agctcaaaga cactggttgg g           51


<210>  335
<211>  50
<212>  DNA
<213>  artificial sequence

<220>
<223>  preferred oligonucleotide for gene LGALS9

<400>  335
tcttcagagg actggctcct ttcccagtgt ccttaaaata aagaaatgaa             50


<210>  336
<211>  60
<212>  DNA
<213>  artificial sequence

<220>
<223>  preferred oligonucleotide for gene LLGL2

<400>  336
agcaccctcg agaccccaac cagatcctga tcggctacag ccgaggcctc gttgtcatct  60


<210>  337
<211>  56
<212>  DNA
<213>  artificial sequence

<220>
<223>  preferred oligonucleotide for gene LYPLA2

<400>  337

cccagataat cgtgtctcct gcctccactc agctgcttct cagtcatgaa tgtggc          56

<210>  338
<211>  50
<212>  DNA
<213>  artificial sequence

<220>
<223>  preferred oligonucleotide for gene MAPKAPK2

<400>  338
ccacttagtg ctggtcctag gtcctctgag gcactcatct actgaatgac                50

<210>  339
<211>  50
<212>  DNA
<213>  artificial sequence

<220>
<223>  preferred oligonucleotide for gene MGAT1

<400>  339
ggacatattt gtggccaaaa tgatactaac caaaggggct tccttgtcag                50

<210>  340
<211>  60
<212>  DNA
<213>  artificial sequence

<220>
<223>  preferred oligonucleotide for gene MGAT3

<400>  340
cccagaatgg cgacttccca cgctggggtg actacgagga caagcgggac ctgaactaca     60

<210>  341
<211>  50
<212>  DNA
<213>  artificial sequence

<220>
<223>  preferred oligonucleotide for gene MGAT4A

<400>  341
cactgtttct ctgtgccacg accgttcatg tgtatttgaa ataaagaagt                50

<210>  342
<211>  51
<212>  DNA
<213>  artificial sequence

<220>
<223>  preferred oligonucleotide for gene MGAT4B

<400>  342
tctctggcgc gaacactgga atgcatatac tactttatgt gctgtgtttt t              51

<210>  343
<211>  50
<212>  DNA
<213>  artificial sequence

<220>
<223>  preferred oligonucleotide for gene MGAT5

<400>  343
atatgaattt acgtgcgagg ggatgctaca gagaatcaat gctttcattg                50


<210>  344
<211>  50
<212>  DNA
<213>  artificial sequence

<220>
<223>  preferred oligonucleotide for gene MGRN1

<400>  344
tctgatgtgc tcagatgggc tcatcgttgg ttcgttttta ctgtatattt                50


<210>  345
<211>  53
<212>  DNA
<213>  artificial sequence

<220>
<223>  preferred oligonucleotide for gene NAGK

<400>  345
cacatcgtag cagtgttgcc cgagattgac ccggtcttgt tccagggcaa gat            53


<210>  346
<211>  52
<212>  DNA
<213>  artificial sequence

<220>
<223>  preferred oligonucleotide for gene NANS

<400>  346
gacatcttta atctagtggg caagaaggtc ctggtcactg ttgaagagga tg             52


<210>  347
<211>  60
<212>  DNA
<213>  artificial sequence

<220>
<223>  preferred oligonucleotide for gene NDST1

<400>  347
ctgcttcgca cagaacctgc caaagtgatg gacatggtgc agaagttcct tggggtgacc     60


<210>  348
<211>  60
<212>  DNA
<213>  artificial sequence

<220>
<223>  preferred oligonucleotide for gene NEU1

<400>  348
ttgggcaggt ctagtgagct gtagaattga atcaatgtga actcagggaa ctggggaagg     60


<210>  349

<211>  50
<212>  DNA
<213>  artificial sequence

<220>
<223>  preferred oligonucleotide for gene NEU2

<400>  349
agccaatgat tacgaggaga ttgtctttct catgttcacc ctgaagcaag                50


<210>  350
<211>  51
<212>  DNA
<213>  artificial sequence

<220>
<223>  preferred oligonucleotide for gene NEU3

<400>  350
caggatattg tcctggccca gctactcctt tacctgtgag aagatcttca c              51


<210>  351
<211>  70
<212>  DNA
<213>  artificial sequence

<220>
<223>  preferred oligonucleotide for gene NEU4

<400>  351
gggtgttgtg ggtggcagca cttgtttact ggctgctttc tggctcgaaa taaaggaatc     60

gtgcttgtga                                                            70


<210>  352
<211>  50
<212>  DNA
<213>  artificial sequence

<220>
<223>  preferred oligonucleotide for gene NPL

<400>  352
tcctagcaaa tgaaatctca caataagcat tgaggtacct tttgtgagcc                50


<210>  353
<211>  62
<212>  DNA
<213>  artificial sequence

<220>
<223>  preferred oligonucleotide for gene O-acetylesterase

<400>  353
cctcagcccc tgagtagctg gtactaaagt taccttccac gctgtatgtc cgtgagaaat     60

ac                                                                    62


<210>  354
<211>  65
<212>  DNA
<213>  artificial sequence

<220>
<223>  preferred oligonucleotide for gene OGT

<400>  354
ttcctggcac gaaaagtagc cgctctggtt gaagctttgc ttattgtaac aggcttttat      60

ttcca                                                                  65


<210>  355
<211>  53
<212>  DNA
<213>  artificial sequence

<220>
<223>  preferred oligonucleotide for gene PGM3

<400>  355
ggccccaacc aggtttctga agataatttt catattcctg agaaactgga ctt             53


<210>  356
<211>  58
<212>  DNA
<213>  artificial sequence

<220>
<223>  preferred oligonucleotide for gene POFUT1

<400>  356
gactaggact ccagtgccct cctctcccaa gagacaaagg ccattgcatt gaaggagg        58


<210>  357
<211>  62
<212>  DNA
<213>  artificial sequence

<220>
<223>  preferred oligonucleotide for gene POFUT2

<400>  357
agttgcattt tccaggctga gagctgtata aaacattttg gactgtgacc atgtaccttc      60

ct                                                                     62


<210>  358
<211>  51
<212>  DNA
<213>  artificial sequence

<220>
<223>  preferred oligonucleotide for gene POMGNT1 (FLJ20277)

<400>  358
tggctcagaa tctaacctat ttattgactg tcctgagggc cttgaaaaca g               51


<210>  359
<211>  51
<212>  DNA
<213>  artificial sequence

<220>
<223>  preferred oligonucleotide for gene PPIA

<400>  359

ttacagatgt gagccaccgc accctacctc attttctgta acaaagctaa g 51

<210> 360
<211> 60
<212> DNA
<213> artificial sequence

<220>
<223> preferred oligonucleotide for gene RABGAP1L

<400> 360
ttactgcttt gtgagggttt gaacataatc tttcatgtag ctttggctct cctaaagacc 60

<210> 361
<211> 51
<212> DNA
<213> artificial sequence

<220>
<223> preferred oligonucleotide for gene RENBP

<400> 361
ccagtttcgc gatcccgagt acggggaatg gtttggctac ctgagccgag a 51

<210> 362
<211> 53
<212> DNA
<213> artificial sequence

<220>
<223> preferred oligonucleotide for gene RERE

<400> 362
cctcaccaga taaactacag tttgtttagg aggccctgac cttcatggtg tct 53

<210> 363
<211> 51
<212> DNA
<213> artificial sequence

<220>
<223> preferred oligonucleotide for gene RNASE4

<400> 363
taatggaaga attgagacaa aaatccagtg tagctggcca cttatccagg g 51

<210> 364
<211> 50
<212> DNA
<213> artificial sequence

<220>
<223> preferred oligonucleotide for gene RPL13A

<400> 364
ttggactttc cacctggtca tatactctgc agctgttaga atgtgcaagc 50

<210> 365
<211> 50
<212> DNA
<213> artificial sequence

<220>
<223>  preferred oligonucleotide for gene SELE

<400>  365
aggagtgtga gaatcaaaac tctcctacac ttccattaac ttagcatgtg          50


<210>  366
<211>  60
<212>  DNA
<213>  artificial sequence

<220>
<223>  preferred oligonucleotide for gene SELL

<400>  366
ccaatatgtc aaaaattgga caaaagtttc tcaatgatta aggagggtga ttataacccc    60


<210>  367
<211>  50
<212>  DNA
<213>  artificial sequence

<220>
<223>  preferred oligonucleotide for gene SELP

<400>  367
tatttgcact gtagcctcgc cgtctgtgaa ttggaccatc ctatttaact          50


<210>  368
<211>  52
<212>  DNA
<213>  artificial sequence

<220>
<223>  preferred oligonucleotide for gene SEPT9

<400>  368
gtaaccagac tttgaaaaat tgttcgtttc atcaggctct gttcctcaat gg        52


<210>  369
<211>  52
<212>  DNA
<213>  artificial sequence

<220>
<223>  preferred oligonucleotide for gene SIAE / OAE

<400>  369
tgactgccct aaggaatgaa aagcaagggc caggtgggag tagcccagcg aa        52


<210>  370
<211>  55
<212>  DNA
<213>  artificial sequence

<220>
<223>  preferred oligonucleotide for gene SIGLEC1 / CD169

<400>  370
gactcttgct gggagcccgt ccagaatgtc ctcccaataa aactccatcc tatga     55


<210>  371

<211>  52
<212>  DNA
<213>  artificial sequence

<220>
<223>  preferred oligonucleotide for gene SIGLEC10

<400>  371
gtagtcccag ctgtcagaca tttaaaccag agcaactcca tctggaatag ga          52


<210>  372
<211>  51
<212>  DNA
<213>  artificial sequence

<220>
<223>  preferred oligonucleotide for gene SIGLEC11

<400>  372
aattcagagc gagtttgaag atgacacttt gaggcatcgt gtctatggtt c          51


<210>  373
<211>  51
<212>  DNA
<213>  artificial sequence

<220>
<223>  preferred oligonucleotide for gene SIGLEC12

<400>  373
gctatgagta ctccgagatc aacatcccca agtgagaaac tgcagagact c          51


<210>  374
<211>  50
<212>  DNA
<213>  artificial sequence

<220>
<223>  preferred oligonucleotide for gene SIGLEC2 / CD22

<400>  374
ttccactctc cttgctaccc agaaatcatc taaatacctg ccctgacatg          50


<210>  375
<211>  51
<212>  DNA
<213>  artificial sequence

<220>
<223>  preferred oligonucleotide for gene SIGLEC3 / CD33

<400>  375
taacacccca caggcaatgg gtttatagac attatgtgag tttcctgcta t          51


<210>  376
<211>  55
<212>  DNA
<213>  artificial sequence

<220>
<223>  preferred oligonucleotide for gene SIGLEC5

<400>  376

tgtttaagtg ctccaagcag agcagaaaga aaacagatga tggaattaga gaggt          55

<210>  377
<211>  50
<212>  DNA
<213>  artificial sequence

<220>
<223>  preferred oligonucleotide for gene SIGLEC6

<400>  377
agaccacagg acttctgtcc aagagaactg agttcaactc catctatgcc               50

<210>  378
<211>  50
<212>  DNA
<213>  artificial sequence

<220>
<223>  preferred oligonucleotide for gene SIGLEC7

<400>  378
caccattctc ctctgtactt ctctaaggat gactacttta gattccgaat               50

<210>  379
<211>  51
<212>  DNA
<213>  artificial sequence

<220>
<223>  preferred oligonucleotide for gene SIGLEC8

<400>  379
cattgacttt acaaaagagg gagcagcaat ggcctagagt ctcaggaaca a             51

<210>  380
<211>  50
<212>  DNA
<213>  artificial sequence

<220>
<223>  preferred oligonucleotide for gene SIGLEC9

<400>  380
caggctttag agtcaaagta tctcaaacct gaatccacac tgtgccctcc               50

<210>  381
<211>  50
<212>  DNA
<213>  artificial sequence

<220>
<223>  preferred oligonucleotide for gene SLC2A1

<400>  381
ggttctagtc tcctttgcac tgagggccac actattacca tgagaagagg               50

<210>  382
<211>  50
<212>  DNA
<213>  artificial sequence

<220>
<223>  preferred oligonucleotide for gene SLC35A1

<400>  382
atctgagtga actgctaata cagaaactta atgtagacct gtttggggtc                50


<210>  383
<211>  52
<212>  DNA
<213>  artificial sequence

<220>
<223>  preferred oligonucleotide for gene SLC35A2

<400>  383
cccctgccag ctccacacta tctcttagct gagtttttgc aaataaaatg tg             52


<210>  384
<211>  55
<212>  DNA
<213>  artificial sequence

<220>
<223>  preferred oligonucleotide for gene SLC35A3

<400>  384
tgaaagcaat ccagttgctc ctgtgctaga tggtagccag agaattttat agtaa          55


<210>  385
<211>  53
<212>  DNA
<213>  artificial sequence

<220>
<223>  preferred oligonucleotide for gene SLC35D1

<400>  385
tggcagttcc tacagagaca ctgaggtgtc ttgattatgg aggaaataac cat            53


<210>  386
<211>  50
<212>  DNA
<213>  artificial sequence

<220>
<223>  preferred oligonucleotide for gene ST3GAL1

<400>  386
actgatttta atcttcgaat catgacactg agtgcagagg aggtggcatt                50


<210>  387
<211>  51
<212>  DNA
<213>  artificial sequence

<220>
<223>  preferred oligonucleotide for gene ST3GAL2

<400>  387
atctacaacc cagccttctt caagtatatc cacgacaggt ggacagagca t              51


<210>  388

<211>  50
<212>  DNA
<213>  artificial sequence

<220>
<223>  preferred oligonucleotide for gene ST3GAL3

<400>  388
gctgccggaa tcacttctcc aatcagtgtt tggtgtatta tcattttgtg          50


<210>  389
<211>  51
<212>  DNA
<213>  artificial sequence

<220>
<223>  preferred oligonucleotide for gene ST3GAL4

<400>  389
aggccagtag agaattctgc ccacttttta taaaaactta cagcgatggc c          51


<210>  390
<211>  52
<212>  DNA
<213>  artificial sequence

<220>
<223>  preferred oligonucleotide for gene ST3GAL5

<400>  390
acaaaacttc tattatccag agtcatggga gagtacaccc tttccaggaa ta          52


<210>  391
<211>  50
<212>  DNA
<213>  artificial sequence

<220>
<223>  preferred oligonucleotide for gene ST3GAL6

<400>  391
atctaagttc atactggttt aattaagctc tctcctgaca acccccagaa          50


<210>  392
<211>  50
<212>  DNA
<213>  artificial sequence

<220>
<223>  preferred oligonucleotide for gene ST6GAL1

<400>  392
actgatttta atcttcgaat catgacactg agtgcagagg aggtggcatt          50


<210>  393
<211>  50
<212>  DNA
<213>  artificial sequence

<220>
<223>  preferred oligonucleotide for gene ST6GAL2

<400>  393

ccggattaca acctgttcac tccatatatt cagcatcgtc agagaaaccc 50

<210> 394
<211> 52
<212> DNA
<213> artificial sequence

<220>
<223> preferred oligonucleotide for gene ST6GALNAC1

<400> 394
tcttcagtac attgctgtag gtcctgaggc cagggatttt taattaaatg gg 52

<210> 395
<211> 50
<212> DNA
<213> artificial sequence

<220>
<223> preferred oligonucleotide for gene ST6GALNAC2

<400> 395
aagattttca tcgaggtctg ttcaatatag gacaccccag cttgtccttg 50

<210> 396
<211> 52
<212> DNA
<213> artificial sequence

<220>
<223> preferred oligonucleotide for gene ST6GALNAC3

<400> 396
acaaaggatg gtcttggcca tatgctaggg cccctgtgaa aataagtagt tt 52

<210> 397
<211> 2052
<212> DNA
<213> Homo sapiens

<400> 397
tgtgtgtgtg ctcacaccca cacgtgtgac tttgcacgca tttgtctttg agagagagat 60

aatagttttt gtttttcttt cagtagccaa agtttaaacc attgcttttt ctttgattct 120

atcgctttta aggtgatctt tcttaacaca ggataagaaa gcagtaatgt cctaaagaat 180

gctgttgaat taattagtat cagcctctcc tacagttctg gaaggatacg tggttaagta 240

ccactcctgg atgaatcaga aaaacagcca cttgagcaat ttcgtgttct gtggttcaga 300

tgagaatccc agttgagaaa gtaagaaaat atgtgtttat tcttcagctt taaattgtta 360

tatcacaaac ttccttcctc cttggcaagc tttctttttt gacgagaaga aaattgtaat 420

aaacaacagt atgactaata gatagtcaca tggttacttc cagtttgcaa aatgctattt 480

atactttgag ttactcttca aaagtggtat acctctagtt tggagctgtg ctgtaaaaac 540

aagagtaaca tttttatatt aaagttaaat aaagttacaa ctttgaagag agtttctgca 600

agacatgaca caaagctgct agcagaaaat caaaacgctg attaaaagaa gcacggtatg 660

atgaccaaac ataaaaagtg ttttataatt gttggtgttt taataacaac taatattatt 720

actctgatag ttaaactaac tcgagattct cagagtttat gcccctatga ttggattggt 780
ttccaaaaca aatgctatta tttctctaaa gaagaaggag attggaattc aagtaaatac 840
aactgttcca ctcaacatgc cgacctaact ataattgaca acatagaaga aatgaatttt 900
cttaggcggt ataaatgcag ttctgatcac tggattggac tgaagatggc aaaaaatcga 960
acaggacaat gggtagatgg agctacattt accaaatcgt ttggcatgag agggagtgaa 1020
ggatgtgcct acctcagcga tgatggtgca gcaacagcta gatgttacac cgaaagaaaa 1080
tggatttgca ggaaaagaat acactaagtt aatgtctaag ataatgggga aaatagaaaa 1140
taacattatt aagtgtaaaa ccagcaaagt acttttttaa ttaaacaaag ttcgagtttt 1200
gtacctgtct ggttaattct gcttacgtgt caggctacac ataaaagcca cttcaaagat 1260
tggcaaaaaa tggttacagg tattctgctg agtttttttc tttctttctt tctctctctc 1320
tctttctttc tttctttctt tctttctttt tttaactgta aaccagaaaa caattgtggg 1380
gatcaaggac agacatagca cttagacagg actttcatga cccaatatgg aacaatttga 1440
gtaccagaat aggagattat ggcctattga acaaaatgtg aaactgggag tccacaaaga 1500
cataaatgga aagatgaagt acctcctgac agagcaagaa tggcactgaa caatgttata 1560
tggacaatgg ctattttatt caagactatt acaataggga aaagagacta ggactcagtc 1620
tgaactgaaa tctgtcaaaa caaagggggg tggggcttta agagtgaagg tgaggaggag 1680
atcacacacc gtatgtgttt gctaactggc tttacccaaa agaaaattaa actttctttg 1740
atctgtacaa gttgatcaaa caaatggggt cattcttgtc atatgcaact aaaacagggt 1800
agacaggcca ggggaaaaag gcactcaggg cacacagcat tgcttcaaaa tataattctc 1860
tacaaaccta gttgctaaaa ctacctgttg taacctaaaa ccagttttat ctaacagcta 1920
ttaaaacaac ctactgtgac tgcaaaactg gttttaccca ccactgtcac acaccaatca 1980
gaacttgcca gctcctcaaa actttactag ggccaataaa ctttctttca aaacaataaa 2040
aaaaaaaaaa aa 2052

<210> 398
<211> 50
<212> DNA
<213> artificial sequence

<220>
<223> preferred oligonucleotide for gene ST6GALNAC5

<400> 398
ccctaggctt caggaaaact gctcttttgt aaaaagaata gcgatgacat 50

<210> 399
<211> 54
<212> DNA
<213> artificial sequence

<220>
<223> preferred oligonucleotide for gene ST6GALNAC6

<400>  399
cccagacttg gttttgtaat gatttgtaca ggaataaaca cacctacgct ccga          54


<210>  400
<211>  51
<212>  DNA
<213>  artificial sequence

<220>
<223>  preferred oligonucleotide for gene ST8SIA1

<400>  400
atttctccaa ctctggtatc ttcataaaat cggtgcactg agaatgcagc t             51


<210>  401
<211>  50
<212>  DNA
<213>  artificial sequence

<220>
<223>  preferred oligonucleotide for gene ST8SIA2

<400>  401
cctttccgct ggatcagaac cagaacccag tcaagtacca ctattatgac               50


<210>  402
<211>  52
<212>  DNA
<213>  artificial sequence

<220>
<223>  preferred oligonucleotide for gene ST8SIA3

<400>  402
ctaaacggct gagcacaggt attcttatgt acacccttgc atcagcaata tg            52


<210>  403
<211>  53
<212>  DNA
<213>  artificial sequence

<220>
<223>  preferred oligonucleotide for gene ST8SIA4

<400>  403
gttatgctaa catcttcagg gtctgcactg tgaactcccc tggagataag taa           53


<210>  404
<211>  51
<212>  DNA
<213>  artificial sequence

<220>
<223>  preferred oligonucleotide for gene ST8SIA5

<400>  404
cccaggttca atcctgcgtt agctcttttg atgagaagga aataaaccaa a             51


<210>  405
<211>  70
<212>  DNA
<213>  artificial sequence

<220>
<223>  preferred oligonucleotide for gene ST8SIA6

<400>  405
aggaggtggc taaagagtat tttgagatga gccccaaaat ttggtttgac caaagcttcc      60

ccactcattt                                                              70


<210>  406
<211>  51
<212>  DNA
<213>  artificial sequence

<220>
<223>  preferred oligonucleotide for gene SULT1C2

<400>  406
ttgggttcag tctcatgcca tatttactcc tagtacctaa tgcagtgcct g               51


<210>  407
<211>  50
<212>  DNA
<213>  artificial sequence

<220>
<223>  preferred oligonucleotide for gene SULT1C4

<400>  407
agagggaagc tcaataaatc actaggtaca atcctggtat cattgccaat                 50


<210>  408
<211>  50
<212>  DNA
<213>  artificial sequence

<220>
<223>  preferred oligonucleotide for gene TACSTD1

<400>  408
aggtgaatcc ttgtttcatt ctaagaaaat ggacctgaca gtaaatgggg                 50


<210>  409
<211>  50
<212>  DNA
<213>  artificial sequence

<220>
<223>  preferred oligonucleotide for gene TAF15

<400>  409
ctgctgcttt cctcgtggcc tcttcttggg tagtgaaatt aagtgacatt                 50


<210>  410
<211>  52
<212>  DNA
<213>  artificial sequence

<220>
<223>  preferred oligonucleotide for gene TAX1BP1

<400>  410
tgcactttct gaccaggagc tactttgagt ttggtgttac taggatcagg gt              52

<210> 411
<211> 53
<212> DNA
<213> artificial sequence

<220>
<223> preferred oligonucleotide for gene TNFRSF1B

<400> 411
gcaacagagt gagaccctgt ctcttaaaga aaaaaaaagt cagactgctg gga 53

<210> 412
<211> 51
<212> DNA
<213> artificial sequence

<220>
<223> preferred oligonucleotide for gene TUBA3C

<400> 412
ccccagcgtg gctgctttca agttctttgc aattaaaggt tctgtataaa a 51

<210> 413
<211> 53
<212> DNA
<213> artificial sequence

<220>
<223> preferred oligonucleotide for gene TUBA4

<400> 413
aaagataggg gggatgaata ctaggggaat actgtgtgtc tgtcctacat aaa 53

<210> 414
<211> 50
<212> DNA
<213> artificial sequence

<220>
<223> preferred oligonucleotide for gene UAP1

<400> 414
tgaaatatag gcaggatgtt caacatcagt ttacttgcag ctggaagcat 50

<210> 415
<211> 52
<212> DNA
<213> artificial sequence

<220>
<223> preferred oligonucleotide for gene WBSCR1/EIF4H

<400> 415
gcattatgtg gtgtaatcaa acccgatgct ttcagatgac ctacttacat ct 52

<210> 416
<211> 69
<212> DNA
<213> artificial sequence

<220>

<223>  preferred oligonucleotide for gene WBSCR17 /GALNTL3

<400>  416
ctcatttggt gacgtcttac tggtgatcat ctcctcaccc catctcccac cttgtggaat      60

aaatacatg                                                              69


<210>  417
<211>  54
<212>  DNA
<213>  artificial sequence

<220>
<223>  preferred oligonucleotide for gene ACT

<400>  417
ggcggactat gacttagttg cgttacaccc tttcttgaca aaacctaact tgcg            54


<210>  418
<211>  57
<212>  DNA
<213>  artificial sequence

<220>
<223>  preferred oligonucleotide for gene ANXA4

<400>  418
ggctagtagt ttcttcattt gaaattgtaa gcatccggtc agtaagaatg cccatcc         57


<210>  419
<211>  50
<212>  DNA
<213>  artificial sequence

<220>
<223>  preferred oligonucleotide for gene ARAF

<400>  419
ccccgccatt caaggactcc tctctttctt caccaagaag cacagaattc                 50


<210>  420
<211>  60
<212>  DNA
<213>  artificial sequence

<220>
<223>  preferred oligonucleotide for gene B3GNT2

<400>  420
tcctggtggt atcatagtgt aattttagta tttgaaaatc agtgtgattc cttaatggcc      60


<210>  421
<211>  70
<212>  DNA
<213>  artificial sequence

<220>
<223>  preferred oligonucleotide for gene B3GNT5

<400>  421
aaatgtcaac aaagggaaaa taaactatca gcttggatgg tcacttgaat agaagatggt      60

tatacacagt                                                             70

<210>  422
<211>  53
<212>  DNA
<213>  artificial sequence

<220>
<223>  preferred oligonucleotide for gene B4GALNT1

<400>  422
tccccaccct tttcttcttg atcttcctca gaccctttcc caaacccctc tcc          53


<210>  423
<211>  70
<212>  DNA
<213>  artificial sequence

<220>
<223>  preferred oligonucleotide for gene B4GALNT3

<400>  423
gcctactacc aagaccggtt cagctttcag gagtacatca agattgacca gcctgagaag     60

caggggctgg                                                            70


<210>  424
<211>  58
<212>  DNA
<213>  artificial sequence

<220>
<223>  preferred oligonucleotide for gene B4GALT1

<400>  424
aaagatcaga gaggcagagg tggcacagga cagtaaagga gatgctgtgc tgcctaca       58


<210>  425
<211>  50
<212>  DNA
<213>  artificial sequence

<220>
<223>  preferred oligonucleotide for gene C1GALT1C1

<400>  425
tccccaagtt ccaatggatt tgccttctca aaatgtacaa ctaagcaact                50


<210>  426
<211>  56
<212>  DNA
<213>  artificial sequence

<220>
<223>  preferred oligonucleotide for gene CD69

<400>  426
gaccaataac catgcccaag aatgagaaga atgactatgc aacctttgga tgcact         56


<210>  427
<211>  60
<212>  DNA
<213>  artificial sequence

<220>
<223>  preferred oligonucleotide for gene CHST6

<400>  427
ggccagctga gcccagcact gcggccaagt cgaagcttta ggagcaataa aagtgcttat      60


<210>  428
<211>  52
<212>  DNA
<213>  artificial sequence

<220>
<223>  preferred oligonucleotide for gene CXADR

<400>  428
gccagccaga tcaaggatgt agtatctcat agttcccagg tgatattttt ct              52


<210>  429
<211>  58
<212>  DNA
<213>  artificial sequence

<220>
<223>  preferred oligonucleotide for gene ETFB

<400>  429
ctacgccacg ctgcccaaca tcatgaaagc caagaagaag aagatcgagg tgatcaag        58


<210>  430
<211>  54
<212>  DNA
<213>  artificial sequence

<220>
<223>  preferred oligonucleotide for gene FUCA1

<400>  430
atccagataa aggtctcttc atctctctac cccagttgcc accctctgct gtcc            54


<210>  431
<211>  58
<212>  DNA
<213>  artificial sequence

<220>
<223>  preferred oligonucleotide for gene FUT1

<400>  431
ccttaaacat agtcagctct gatcacatcc cctgtctact catccagacc ccatgcct        58


<210>  432
<211>  60
<212>  DNA
<213>  artificial sequence

<220>
<223>  preferred oligonucleotide for gene FUT10

<400>  432
cagccgagca tgaacatgaa atacattaga acttggcaat gagagatccc atctgttggt      60

<210>  433
<211>  57
<212>  DNA
<213>  artificial sequence

<220>
<223>  preferred oligonucleotide for gene FUT2

<400>  433
cccggcccta gagctcactg ttttctagtt agtccatctg gaagtggagc ctttttc          57


<210>  434
<211>  50
<212>  DNA
<213>  artificial sequence

<220>
<223>  preferred oligonucleotide for gene FUT3

<400>  434
cagagttcag acaggtccaa gttcaagccc aggaccacca cttatagggt                  50


<210>  435
<211>  50
<212>  DNA
<213>  artificial sequence

<220>
<223>  preferred oligonucleotide for gene FUT4

<400>  435
gaaccagcca tgcttctgca aatcacaacc tctttgagcc tctgtcacct                  50


<210>  436
<211>  50
<212>  DNA
<213>  artificial sequence

<220>
<223>  preferred oligonucleotide for gene FUT5

<400>  436
actatgggtg tgacctcggc gtgactctga tagtgcctgt ggatgtgttg                  50


<210>  437
<211>  53
<212>  DNA
<213>  artificial sequence

<220>
<223>  preferred oligonucleotide for gene FUT6

<400>  437
caccgtttcc agatgtaata ctgcaaagaa aaccgatgat gaggccgggt gcg              53


<210>  438
<211>  50
<212>  DNA
<213>  artificial sequence

<220>
<223>  preferred oligonucleotide for gene FUT8

<400>  438
actgacatag gcttcaattg gtggaattcc tctttaacaa gggctgcaat                50

<210>  439
<211>  53
<212>  DNA
<213>  artificial sequence

<220>
<223>  preferred oligonucleotide for gene GALNT7

<400>  439
aacatcagaa gctgctgcca taatgactat tttctactgt aggctgcttt gga            53

<210>  440
<211>  51
<212>  DNA
<213>  artificial sequence

<220>
<223>  preferred oligonucleotide for gene GAPDH

<400>  440
caccacactg aatctcccct cctcacagtt gccatgtaga ccccttgaag a              51

<210>  441
<211>  50
<212>  DNA
<213>  artificial sequence

<220>
<223>  preferred oligonucleotide for gene GCNT1

<400>  441
tgggtaggaa gaggccatgt ttcagttcgt tttctctgta gggtcgattg                50

<210>  442
<211>  70
<212>  DNA
<213>  artificial sequence

<220>
<223>  preferred oligonucleotide for gene GCNT2

<400>  442
tgttaaccgt ttcaggacca cgtttatagc ttcaggacct ggctacgtaa ttatacttaa      60

aatatccact                                                            70

<210>  443
<211>  60
<212>  DNA
<213>  artificial sequence

<220>
<223>  preferred oligonucleotide for gene GCNT3

<400>  443
tttgaagaac cctaaatccc aacaactgat tgaatgggta aaagacactt atagcccaga      60

<210>  444
<211>  50

<212>  DNA
<213>  artificial sequence

<220>
<223>  preferred oligonucleotide for gene GLB1

<400>  444
atcccagact tgccccagga cacctttatc cagtttcctg gatggaccaa          50


<210>  445
<211>  50
<212>  DNA
<213>  artificial sequence

<220>
<223>  preferred oligonucleotide for gene GNE

<400>  445
gtgggttagt gtgaaaaaga gtttggctga ttcctaaaac tctgccagcc          50


<210>  446
<211>  60
<212>  DNA
<213>  artificial sequence

<220>
<223>  preferred oligonucleotide for gene HDAC1

<400>  446
aacatcagcc atttttagat tggttctgtt ttcgtacctt cccactggcc tcaagtgagc    60


<210>  447
<211>  54
<212>  DNA
<213>  artificial sequence

<220>
<223>  preferred oligonucleotide for gene LGALS1

<400>  447
acggtgactt caagatcaaa tgtgtggcct ttgactgaaa tcagccagcc catg        54


<210>  448
<211>  57
<212>  DNA
<213>  artificial sequence

<220>
<223>  preferred oligonucleotide for gene LGALS3

<400>  448
acctgaccac ttcaaggttg cagtgaatga tgctcacttg ttgcagtaca atcatcg     57


<210>  449
<211>  50
<212>  DNA
<213>  artificial sequence

<220>
<223>  preferred oligonucleotide for gene LGALS7

<400>  449
gagaattcgc ggcttggttc ctcccaatgc cagcaggttc catgtaaacc          50

<210> 450
<211> 52
<212> DNA
<213> artificial sequence

<220>
<223> preferred oligonucleotide for gene LGALS9

<400> 450
atctgggctt taatgcagag gccatgtcct tgtctggtcc tgcttctggc ta 52


<210> 451
<211> 50
<212> DNA
<213> artificial sequence

<220>
<223> preferred oligonucleotide for gene LLGL2

<400> 451
ttgtagtggg ctgggtttta agttataaat gttaactgcc tctgggtgaa 50


<210> 452
<211> 60
<212> DNA
<213> artificial sequence

<220>
<223> preferred oligonucleotide for gene MGAT5

<400> 452
tttaaatcag aagattgagc catacatgcc atatgaattt acgtgcgagg ggatgctaca 60


<210> 453
<211> 53
<212> DNA
<213> artificial sequence

<220>
<223> preferred oligonucleotide for gene NDST1

<400> 453
tgcatttagt agctgcctga ccatcttcct cccaggccct cagtttcttc atc 53


<210> 454
<211> 54
<212> DNA
<213> artificial sequence

<220>
<223> preferred oligonucleotide for gene NEU1

<400> 454
agagttccga gtgaacctga ccctgcgatg gagcttcagc aatggtacct catg 54


<210> 455
<211> 60
<212> DNA
<213> artificial sequence

<220>

<223>  preferred oligonucleotide for gene PPIA

<400>  455
gtggacaact cgaataagtt tgacttgtgt tttatcttaa ccaccagatc attccttctg     60


<210>  456
<211>  57
<212>  DNA
<213>  artificial sequence

<220>
<223>  preferred oligonucleotide for gene SELE

<400>  456
agagaagttc tggctgaaca ctggcaacga caaagccaac agtcaaaaca gagatgt        57


<210>  457
<211>  54
<212>  DNA
<213>  artificial sequence

<220>
<223>  preferred oligonucleotide for gene SELL

<400>  457
gcctccctca accccaccac ttcttttata actagtcctt tactaatcca accc           54


<210>  458
<211>  51
<212>  DNA
<213>  artificial sequence

<220>
<223>  preferred oligonucleotide for gene SIGLEC2 / CD22

<400>  458
atctgctcgt cattttcctt cccttctcca tctctctggc cctctacccc t              51


<210>  459
<211>  51
<212>  DNA
<213>  artificial sequence

<220>
<223>  preferred oligonucleotide for gene SIGLEC3 / CD33

<400>  459
agagaaccat ccagctcaac gtcacctatg ttccacagaa cccaacaact g              51


<210>  460
<211>  60
<212>  DNA
<213>  artificial sequence

<220>
<223>  preferred oligonucleotide for gene ST3GAL4

<400>  460
cccagacgcc tacaacaaga agcagaccat tcactactat gagcagatca cgctcaagtc     60


<210>  461
<211>  60

<212>  DNA
<213>  artificial sequence

<220>
<223>  preferred oligonucleotide for gene ST6GALNAC2

<400>  461
taccccagat gctaaagtga ttcaaggact ccaggacacc tcttaagagc ctttctaaga      60


<210>  462
<211>  50
<212>  DNA
<213>  artificial sequence

<220>
<223>  preferred oligonucleotide for gene SULT1C2

<400>  462
tgccaccctt ttgggaagcc ctaaaccaag tgaggtgttc ttttcccttg                 50


<210>  463
<211>  54
<212>  DNA
<213>  artificial sequence

<220>
<223>  preferred oligonucleotide for gene TNFRSF1B

<400>  463
gagagcccag ccattaccat ggagacaaga agggttttcc accctggaat caag            54


<210>  464
<211>  60
<212>  DNA
<213>  artificial sequence

<220>
<223>  preferred oligonucleotide for gene TUBA3C

<400>  464
acggcaagta catggcctgc tgcatgttgt acagggggga tgtggtcccg aaagatgtca      60


<210>  465
<211>  2868
<212>  DNA
<213>  Homo sapiens

<400>  465
cgggaggctg cgaggactgc aaaagggtgg agtcggcctc gcccccgccc aggccccgcc      60

cctgccggga acccactttc ccagtcctag gcggcggtca gatccttgca agcatggtcg     120

cgccggggct tgtactcggg ctggtgctgc cattaatcct gtgggccgac agaagtgcag     180

gtattggttt tcgctttgct tcatacatca ataatgatat ggtgctgcag aaggagcctg     240

ctggggcagt gatatggggc ttcggtacac ctggagccac agtgaccgtg accctgcgcc     300

aaggtcagga aaccatcatg aagaaagtga ccagtgtgaa agctcactct gatacgtgga     360

tggtggtact ggatcctatg aagcctggag gacctttcga agtgatggca caacagactt     420

tggagaaaat aaacttcacc ctgagagttc atgacgtcct gtttggagat gtctggctct     480

gtagtgggca gagtaacatg cagatgactg tgttacagat atttaatgct acaagggagt 540
tgtctaacac tgcggcatat cagtctgtcc gcatcctctc tgtctctccc attcaagcag 600
agcaggagct ggaggacctt gttgcggttg acttgcagtg gtctaagccc acctcagaaa 660
acttaggcca tggatatttc aagtacatgt cagcagtgtg ctggctcttt ggacgtcacc 720
tttatgacac tctgcagtat cccatcgggc tgatcgcctc cagctggggc gggacaccca 780
ttgaagcctg gtcatctgga cggtcactga aagcctgtgg ggtccctaaa caagggtcca 840
ttccatacga ttctgtaact ggtcccagta agcactctgt tctctggaat gccatgatcc 900
atccactgtg caatatgact ctgaaagggg tagtatggta ccagggggag tccaatataa 960
attataacac ggatctgtac aattgcacat tccctgcact catcgaagac tggcgtgaaa 1020
ccttccaccg tggttcccag gggcagacgg agcgtttctt cccatttgga cttgtccagt 1080
tatcttcaga tttgtctaag aagagctcag acgatggatt tccccagatc cgttggcatc 1140
aaacagcaga cttcggctat gtccccaacc caaagatgcc caatactttc atggctgtag 1200
ctatggatct ctgtgataga gactcgcctt ttggcagcat ccaccctcga gataaacaga 1260
ctgtggctta tcggctgcat ttgggggccc gtgctctggc ttatggtgag aagaatttga 1320
cctttgaagg accactgcct gagaagatag aactcttggc tcacaagggg ctgctcaatc 1380
tcacatatta ccagcaaatc caggtgcaga aaaaggacaa caagatattt gagatctcct 1440
gttgcagtga ccatcgatgc aagtggcttc cagcttctat gaacaccgtc tccacccagt 1500
ccctgaccct ggcgatcgat tcttgtcatg gcactgtggt tgctctccgc tatgcttgga 1560
ccacgtggcc ttgtgaatat aagcagtgtc ccctatacca ccccagtagt gccctgccag 1620
cccctccctt cattgctttc attacagacc agggtcctgg acatcagagc aatgttgcta 1680
aatgactgtt tcagtatgat cagaacttag atataaggat gggtccttca gattttagca 1740
tttaggagtt tcaataataa ccattgcttt taaaggaaat taatagaaag cctcattgaa 1800
tggctttcag ctagcacatg gctgtttcta tattctgatg agcccaggct tataggtaac 1860
ttgaaatgct tgctttttgt tccctagttg gtctaagggt ctgtattgga ctaattctga 1920
actacagaca aattggacct caatgtcatt tacttccctc atattaatgg gagtgaaatg 1980
tctaatactt ttgccccttt ttatccagag ttgtgggaat ctcaggattg gaagagattt 2040
taaaggccac ataggccagc tagtgttcat gtgttcttta taaaatttct cccatccaag 2100
tactaaccag gcccgaccct gcttagcttc cgagatcaga tgagatcagg cgcgttcagg 2160
gtgatatggc cgtagacgtc tttacaaaat tcctgacagg tggttactga atctctctat 2220
gaactttcca ttcaaaactt tccaagtttt tccttatgtg gaaccgaaat ctttctttct 2280
cccgtgaaac tttactacta tcagataatt gaagacagat ctctttgtat tctcttcaag 2340
cccaaaccaa ttctgttcct tcaatctaaa tagtggtaat atgaatgttt aagaaatgaa 2400
ataagaaaca tgtgcaggca ctttggaagg tgctaagtga ctgccctaag gaatgaaaag 2460
caagggccag gtgggagtag cccagcgaag gcacttgggc tgccaggaac aggaggcgtg 2520

ggaaactctg gcttaggaaa acatgaacac aggggcaaca gaggcaaact gttgttcgag 2580

ttaaatataa atctcaggct ctttaaaggt aaaaggttta aggataatcc atttggaaga 2640

agaaaagagt gaggctgaaa gtaaagccac atgacaagca tataaaaaaa aatgcagatg 2700

atacaaatat gaaagaggcc ttcagtgttt gtttattaag aatcttaatg cagtttactg 2760

atggattaaa aacagctaac attgtctgaa aattatgtta cctataagaa gttggaaata 2820

aataaaagca taatcactaa aaaaaaaaaa aaaaaaaaaa aaaaaaaa 2868

<210> 466
<211> 3409
<212> DNA
<213> Homo sapiens

<400> 466
gggcccagat agaacaagaa ggcagaagaa atgtgaattc ctctttctct gctggagctg 60

ggatattctt cttctcctgc ccttggacat cagagctgca ggctctctgg cctttggacc 120

cgaggattta taccaagcag gtttctgggt tctcaggcct ttggccttgg actgatagtt 180

acaccattgg catatctggt tctgaggctc ttggtcttgg actgagccac actcctggca 240

tcccagcgtc tccagcttgc atggcctgtc acgtgattcc tgtcagaatc accatttttg 300

gtaaacaaac caagcccaga acctgataat tatggagcat tctacactga cagttctttg 360

agacaaattt cctcttggca tttacactgt ggctttagct ttcaaaccag aggttcctct 420

tacccagcaa aaagtgagtt atacgctttc ttaatgttat aacgttacca tggatgatcc 480

tgaacttgcc gaggatagca gagacgggtg ggcagaacag gaaagaatca gatcagagac 540

tgtaaaaagt aacttaaaaa aaaataattc tggcagagac agaatttgaa ggtacttgtg 600

cacatcagaa cactggactt gcttttttct gggagcagga atgctgctta attagatcag 660

agaagaatgc aagtggtcca tacatttaga tctacaatgc gtggtttcca gacctgcagc 720

ttgttttgct gcgcttcatc atggagtcat agaagggcag agctggagga ccgagtgagg 780

gacctggtgc catatcccta cagacaggca attggagact cccgtaggtt aagggctgca 840

gagcctggac caatgcccag aatctctgag ctttttatct tacaccatga agtgacagat 900

gctggcagat gttagacctt tgtgcttaac tgtttaacca cacagcaccc gacttctgta 960

tgcagcgagg ttctagagtt tccaaaacac gggtctcctc tcccacctca gcctcctagc 1020

ataaaactag acacatcctc atgcttttga ggtctaatca ttggattttg ttcctttcag 1080

atggctttcc cgaagatgag attgatgtat atttgccttc tggttctggg ggctctttgt 1140

ttgtatttta gcatgtacag tctaaatcct ttcaaagaac agtcctttgt ttacaagaaa 1200

gacgggaact tccttaagct cccagataca gactgcaggc agacacctcc cttcctcgtc 1260

ctgctggtga cctcatccca caaacagttg gctgagcgca tggccatccg gcagacgtgg 1320

gggaaagaga ggatggtgaa gggaaagcag ctgaagacat tcttcctcct ggggaccacc 1380

agcagtgcag cggaaacgaa agaggtggac caggagagcc agcgacacgg ggacattatc 1440

cagaaggatt tcctagacgt ctattacaat ctgaccctga agaccatgat gggcatagaa 1500

tgggtccatc gcttttgtcc tcaggcggcg tttgtgatga aaacagactc agacatgttc 1560
atcaatgttg actatctgac tgaactgctt ctgaagaaaa acagaacaac caggtttttc 1620
actggcttct tgaaactcaa tgagtttccc atcaggcagc cattcagcaa gtggtttgtc 1680
agtaaatctg aatatccgtg ggacaggtac ccaccattct gctccggcac cggctacgtg 1740
ttttctggcg acgtggcgag tcaggtgtac aatgtctcca agagcgtccc atacattaaa 1800
ctggaagacg tgtttgtggg gctctgcctc gaaaggctga acatcagatt ggaggagctc 1860
cactcccagc cgaccttttt tccagggggc ttacgcttct ccgtatgcct cttcaggagg 1920
atcgtggcct gccacttcat caagcctcgg actctcttgg actactggca ggctctagag 1980
aattcccggg gggaagattg tccgcctgtc tgaggggagc ccagaggcac atccggacaa 2040
gtttcagata acccgtgggg atagtttttg ctagattttg gaagaggggg cgggacagag 2100
gatgctgttc ttcagtgctg aaatccacgc cagaatgtcg gtgttcatga agtcactgat 2160
tagttcccac ttggtgcccc aggcaataat aggcccgtct cttgggcacg cacactcttc 2220
atactaagtg tttgacatac acctggattt ttgcatttca ggggtcagta tcctatgaca 2280
tgatgggtgt taccatccta attttacagg caaggacaca gcagctgcga gaggtacaga 2340
aacttgtccc aaggctcaca gccagtaggc ataggagcgg gaatgaaaat cgagcactgt 2400
cagaatctgg tgggcagccc ctgacttgaa ccactcccac gtgctgcctc ccttaggagg 2460
ggacactgat gatgaggtct cggagccggc atccttccat ccctgtcgag tcccctccac 2520
ctcagctccc agtccttgtg ctttttggag ctaagcctgg gatgaccaaa ttcaccccag 2580
ctccttcatt cacagggctg gatgtagctg ggattgagtc catgttatcg gctcggtact 2640
caacacaacc caagtttcat ccgaggaaat gtccccgcag tggatgcagc tcacatgctg 2700
aggaacaccc agctctggac agagttctta taaatgtata aattaggctc agaaaccact 2760
gcattctgac ctgctgtaca gactgcccac actgctgacc tgcctagcga gcaggacatc 2820
ccttctgagc catctgctgc tctctcattt catcacccca actgtccctt gtttttgatc 2880
aatggggacc agccactgcc ccaggagcac tttagggctc tcagttcaaa ctgaaggaca 2940
gttgaactca gatggggttc atgtgggatt ctgggagctt tctgggaatt cagttggagt 3000
caagtcagga tgctctcaag gacccctcgg gctcagagcc ctaaagtggg ccctggtgaa 3060
gcagggtggt cctgcgtcca cttcccaagc ctgagccaag ctcatcttca ttgaatgtct 3120
catttggccg aggaacaact gaactttgtg gtttgctgtt tagccttcag tttgctccgc 3180
tgcctcctac ccagaggttt gtgcgagcct gtgttgcagg gttgtataaa accaaggtac 3240
ttcgttagtt ttgcccattc agccatggtc acgtgacatg caaagtaatc ttgctcctaa 3300
ttatagaaat gatttttctt ttaatttttt actttaccag actttacttt gtactcagag 3360
aagaggcctc acatggctgt gtcacatata aatgttggac taaactctt 3409

<210> 467
<211> 4219

<212>  DNA
<213>  Homo sapiens

<400>  467
gcagaccaaa gtgagagagg gacgcaccgc atctccaggc acatccaaaa aggatggacg      60
agacaccgaa gcagaggata caggaggatt aaaggattca ggaaagcaag cagccctccg     120
gagaagctgt cgaaattcaa gactggcaag agaagcaaat tcaacctctc acaccgatca     180
tttctcattc cctgaaaaga agagattgtt tccccaggga agtgaaaata atgaactttt     240
ggaggtactg cttttttgct ttcactctgc tcagcgtggt catttttgtg agattttaca     300
gtagccaatt gagcccgcca aaaagttatg agaagctgaa cagttccagt gaaaggtatt     360
ttaggaaaac tgcctgtaat cacgccttag agaaaatgcc agtctttttg tgggaaaata     420
tattaccatc acctttgcga agtgtccctt gcaaggatta cctgacccag aatcactaca     480
tcacaagtcc cctgtcggaa gaagaggctg cattcccttt ggcctatgtc atggtcatcc     540
ataaggactt tgacaccttt gaaaggctct ttagggctat ctatatgccc caaaatgtct     600
actgtgttca cgtggatgag aaagccccag ctgagtataa ggaatctgtg aggcagttac     660
tgagttgctt ccaaaatgct ttcattgctt caaagacaga gtctgtggtt tatgcaggca     720
tttccagact ccaggctgac ctgaactgtc tgaaagacct tgtcgcctct gaggttccct     780
ggaagtacgt catcaacacc tgtggacaag acttccccct gaaaaccaac cgggagatag     840
ttcagcatct gaaaggattt aaagggaaaa atatcacccc aggggtgctg cctcctgacc     900
atgcaattaa gcgaactaaa tatgtccacc aagagcatac agataaaggt ggcttttttg     960
tgaaaaatac taatattttg aaaacttcac ctccacatca gctgaccatc tactttggca    1020
ctgcctatgt ggcgcttacc agagactttg tcgactttgt tctacgtgac caaagggcca    1080
ttgatctact acaatggtca aaagatacct atagtcctga tgagcatttc tgggtgacac    1140
ttaatagggt ttcaggtgtt cctggctcta tgccaaatgc atcctggact ggaaacctca    1200
gagctataaa gtggagtgac atggaagaca gacacggagg ctgccacggc cactatgtac    1260
atggtatttg tatctatgga aacggagact taaagtggct ggttaattca ccaagcctgt    1320
ttgctaacaa gtttgagctt aatacctacc cccttactgt ggaatgccta gaactgaggc    1380
atcgcgaaag aaccctcaat cagagtgaaa ctgcgataca acccagctgg tatttttgag    1440
ctattcatga gctactcatg actgaaggga aactgcagct gggaagagga gcctgttttt    1500
gtgagagact tttgccttcg taatgttaac cgtttcagga ccacgtttat agcttcagga    1560
cctggctacg taattatact taaaatatcc actggacact gtgaaataca ctaacaggat    1620
ggctgggtag agcaatctgg gcactttggc caattttagt cttgctgttt cttgatgctc    1680
acctctatat tagtttattg ttaggatcaa tgataaattt aaatgacctc agatctttgc    1740
accagatact catcatatac aaatgtttta gtaaaaaaga gaattgtaga taatactgtc    1800
taggaaaata agaattaggt ttctttgaag aaggaatctt ttataacacc ttaacagtca    1860
ccactgtgct caaccagaca gatagtgaaa cagctttctg ggtaattcac caatttcctt    1920

taaaacataa gctacctgaa tggagaatac atcttgtttc tgagtttcaa cactagcatt 1980
tttggcttac tcatggacaa agttctgtat atagtataaa gtcattaaca agaaacagga 2040
tatgctttaa gacagaattc actgtctgtt gcttcagtaa aaggacctcg gggaataaaa 2100
catttctctc ttatatgcca gaatgtaggc tggtccctat gtcatgtctt ccattaagaa 2160
cactaaaaag tccttgcaag aatggagata tgcattcaag agaggtgcta tcacatagat 2220
ctagtctgaa gtctggaaca ctttcctctt ctatgacccc tctctcccca gtattatctt 2280
acttgcaaaa tggagaccaa attctatcct gtgaggcttt taattgcacc atagtatgct 2340
ctgagtagct ttacactgcc tggtactgat agtagtggct cgatttttaa gagccttcaa 2400
ttgtagatga acatctctgt tatttatccc tcattcatcc atccgttcat tcattcagcc 2460
ttcaatcaac atctcttgag tgtctattat gtacaggaca tgtactgaga caaaaaggaa 2520
acataagagc tttttcactc taaaaatctt ggcaataatg tcaacaccag aaagcctcct 2580
ctggagaatc ttacagagtg attgtagttt aatacaggaa cacacagggc tgtgtagcat 2640
gataccaggc ccaggagatc agtaattaca aattaagggt taaatcagag attattcaac 2700
agagagggag aaaggaggag acagagggag gacctgttgt gttccagcca ttctggtatt 2760
cctttatgta tctaatttca ttcaaacctc acaacagtct tgtgaggccc ttatataatt 2820
actcccattt tgcagatgaa gtaactgagg cttagaaagg ttaatagcac cggggaacaa 2880
tttctctggg tgagaattgg gactctgttg ctggtcttct cagttcattt cctgaggtgg 2940
atttactgag agaaggtgaa ataaagccat atttagtata ccagagaagg tagattttaa 3000
gaatggtctc agtgttaata ctgagaaaaa gtcctgtcag ttcagaaaaa atgtgaagtc 3060
tactttagta ttcctgtaat actaaaccgt tgagtttcta aatatttatt tattctaaca 3120
aaaagcaatt actacaaatg gatgacacat ttaatgaaca caattttatt ttttttctgt 3180
aactgtgctt gttgaatgtc aatcatattt aaagggaatg actttgaagt aaaacctttt 3240
ttcttgctac tgaaaaaaat ggagttgttt tgggtggtaa agtgttaagg aatagggaca 3300
gctggtcaca caaggaactc ttgaaggcca catgtgaaaa cctgtcactt gcacagaggc 3360
cagtcccact aaggtgacca gagtgggctc caagcacaaa ctgccattgg ctatagatgg 3420
gactgtgtcc ccccaaaatt catgtgttgg agccttaacc ctcaatgtga tggtatttga 3480
gatggggcct ttggtaaggg aagtttagat gaggtcacga gggtaggacc ctcatgatgg 3540
gatgagtccc cttacaagac ctctggcttg ggccgggcgt ggtggctcac acctgtaatc 3600
ccaacacttt gggaggccaa ggcaggtaga tcacttgatg ccaggagttc cagaccaggc 3660
tggccgacat ggtgaaaccc catctctact aaaaaatata aaaattagcc gggctttgtg 3720
gcatgtgcct gtaatcccag ctatttggca ggctgaggca tgagaatcgc ttgaacccag 3780
gaggtggagg ttacagtgag ctgagagtgc cccactgcac tccagcctgg gtgacagagc 3840
gagactttgt cccaaaacaa aataggtgag gggatagcga atgcactcag ggtcagcagt 3900
ggagtttaaa aattgtctct tttcaactta tttaaatgac agcacctgag aagaggaacc 3960

gttttacact ggatgtttct catgtagaac aagaaatctt tctggaattg atgtttacat 4020
gtctgttgtt ggtcatctct cctgtgtctt aaatacttta atgttggaag agcatagtgt 4080
ttgggctagt gggtttctga cagcccatgg gaatgccctg aaactactgt atctgatgtt 4140
tgttttcgat gaggttccat gttttgtttt cttgggaata aattaatata ttgttttcca 4200
aaaaaaaaaa aaaaaaaaa 4219

<210> 468
<211> 4423
<212> DNA
<213> Homo sapiens

<400> 468
   gttactagac tctggccggg acaagaggct gaggttaagg tcttagagcc gatgacttgc 60
   agaatggtca cctcacccac ttgtaatttc atggtgggaa caggtggacc ccctggaatc 120
   agaacctctc tgaggacatc tgtttttgtg tagacacagg ttgcaggtta gcaggagaac 180
   aggcaagcca aatgcaaagg agccacttca gaaatgtgtc acagaaaagt gaaaatgcaa 240
   cctagtggta agtgaagagg ggaagaagaa agaaaaagga ccagaaccgt gaactgaagg 300
   gacagggaac agccagacga gagcttcagc catcacgagg atgatttcgg aacctggaga 360
   aaatgtaagt taaatatatc tacactctga tcctatctca agagagagat attttactca 420
   tttcctggtt gtgaatgatg ggctcttgga agcactgtct ttttagcgcg tctcttatct 480
   ctgccctgat ttttgtattt gtttacaata ctgagttatg ggagaataaa cgttttctga 540
   gggcagctct gtccaatgct tcactgttag cagaagcctg tcatcagatt tttgagggga 600
   aagtttttta cccaacagaa aatgcattga aaactaccct tgatgaagct acctgctatg 660
   agtacatggt tcgaagccac tatgtaacag aaacactctc tgaagaagag gctgggttcc 720
   ctttagctta cacagtgacc atccacaaag acttcggcac ttttgagagg ctcttcaggg 780
   cgatttatat gccccaaaat gtctactgtg tgcacctgga tcagaaggcg acggatgcct 840
   ttaaaggtgc agtgaaacag ttactcagct gcttcccaaa tgcttttctg gcttccaaga 900
   aggagtcggt tgtctatggg gggatctcca ggctccaggc tgacctgaac tgcctggaag 960
   accttgtggc ctctgaagtt ccctggaagt atgtcatcaa cacctgcggg caagactttc 1020
   ccctgaaaac caacagggaa atagttcagt atctgaaggg atttaaaggg aaaaatatca 1080
   cccccggagt gctgcctcct gaccacgctg ttggacggac taaatacgtc caccaagaac 1140
   tgttaaacca caaaaattcc tacgtgatta aaacaacaaa attaaaaact cctcctcctc 1200
   atgacatggt gatttacttt ggcacggcct acgtggctct cacaagggac tttgctaact 1260
   tcgtcctcca agaccagctc gcacttgact tactctcctg gtccaaggac acctacagcc 1320
   ccgacgaaca tttctgggtg acactcaaca ggattcccgg tgttcctggc tctatgccaa 1380
   atgcatcctg gactggaaac ctcagagcta taaagtggag tgacatggaa gacagacacg 1440
   gaggctgcca cggccactat gtacatggta tttgtatcta tggaaacgga gacttaaagt 1500
   ggctggttaa ttcaccaagc ctgtttgcta acaagtttga gcttaatacc taccccctta 1560
   ctgtggaatg cctagaactg aggcatcgcg aaagaaccct caatcagagt gaaactgcga 1620
   tacaacccag ctggtatttt tgagctattc atgagctact catgactgaa gggaaactgc 1680
   agctgggaag aggagcctgt ttttgtgaga gacttttgcc ttcgtaatgt taaccgtttc 1740
   aggaccacgt ttatagcttc aggacctggc tacgtaatta tacttaaaat atccactgga 1800
   cactgtgaaa tacactaaca ggatggctgg gtagagcaat ctgggcactt tggccaattt 1860
   tagtcttgct gtttcttgat gctcacctct atattagttt attgttagga tcaatgataa 1920
   atttaaatga cctcagatct ttgcaccaga tactcatcat atacaaatgt tttagtaaaa 1980
   aagagaattg tagataatac tgtctaggaa aataagaatt aggtttcttt gaagaaggaa 2040
   tcttttataa caccttaaca gtcaccactg tgctcaacca gacagatagt gaaacagctt 2100
   tctgggtaat tcaccaattt cctttaaaac ataagctacc tgaatggaga atacatcttg 2160
   tttctgagtt tcaacactag catttttggc ttactcatgg acaaagttct gtatatagta 2220
   taaagtcatt aacaagaaac aggatatgct ttaagacaga attcactgtc tgttgcttca 2280
   gtaaaaggac ctcggggaat aaaacatttc tctcttatat gccagaatgt aggctggtcc 2340
   ctatgtcatg tcttccatta agaacactaa aaagtccttg caagaatgga gatatgcatt 2400
   caagagaggt gctatcacat agatctagtc tgaagtctgg aacactttcc tcttctatga 2460
   cccctctctc cccagtatta tcttacttgc aaaatggaga ccaaattcta tcctgtgagg 2520
   cttttaattg caccatagta tgctctgagt agctttacac tgcctggtac tgatagtagt 2580
   ggctcgattt ttaagagcct tcaattgtag atgaacatct ctgttattta tccctcattc 2640
   atccatccgt tcattcattc agccttcaat caacatctct tgagtgtcta ttatgtacag 2700
   gacatgtact gagacaaaaa ggaaacataa gagctttttc actctaaaaa tcttggcaat 2760
   aatgtcaaca ccagaaagcc tcctctggag aatcttacag agtgattgta gtttaataca 2820
   ggaacacaca gggctgtgta gcatgatacc aggcccagga gatcagtaat tacaaattaa 2880
   gggttaaatc agagattatt caacagagag ggagaaagga ggagacagag ggaggacctg 2940
   ttgtgttcca gccattctgg tattccttta tgtatctaat ttcattcaaa cctcacaaca 3000
   gtcttgtgag gcccttatat aattactccc attttgcaga tgaagtaact gaggcttaga 3060
   aaggttaata gcaccgggga acaatttctc tgggtgagaa ttgggactct gttgctggtc 3120
   ttctcagttc atttcctgag gtggatttac tgagagaagg tgaaataaag ccatatttag 3180
   tataccagag aaggtagatt ttaagaatgg tctcagtgtt aatactgaga aaaagtcctg 3240
   tcagttcaga aaaaatgtga agtctacttt agtattcctg taatactaaa ccgttgagtt 3300
   tctaaatatt tatttattct aacaaaaagc aattactaca aatggatgac acatttaatg 3360
   aacacaattt tatttttttt ctgtaactgt gcttgttgaa tgtcaatcat atttaaaggg 3420
   aatgactttg aagtaaaacc ttttttcttg ctactgaaaa aaatggagtt gttttgggtg 3480
   gtaaagtgtt aaggaatagg gacagctggt cacacaagga actcttgaag gccacatgtg 3540
   aaaacctgtc acttgcacag aggccagtcc cactaaggtg accagagtgg gctccaagca 3600
   caaactgcca ttggctatag atgggactgt gtccccccaa aattcatgtg ttggagcctt 3660
   aaccctcaat gtgatggtat ttgagatggg gcctttggta agggaagttt agatgaggtc 3720
   acgagggtag gaccctcatg atgggatgag tccccttaca agacctctgg cttgggccgg 3780
   gcgtggtggc tcacacctgt aatcccaaca ctttgggagg ccaaggcagg tagatcactt 3840
   gatgccagga gttccagacc aggctggccg acatggtgaa accccatctc tactaaaaaa 3900
   tataaaaatt agccgggctt tgtggcatgt gcctgtaatc ccagctattt ggcaggctga 3960
   ggcatgagaa tcgcttgaac ccaggaggtg gaggttacag tgagctgaga gtgccccact 4020
   gcactccagc ctgggtgaca gagcgagact ttgtcccaaa acaaaatagg tgaggggata 4080
   gcgaatgcac tcagggtcag cagtggagtt taaaaattgt ctcttttcaa cttatttaaa 4140
   tgacagcacc tgagaagagg aaccgtttta cactggatgt ttctcatgta gaacaagaaa 4200
   tctttctgga attgatgttt acatgtctgt tgttggtcat ctctcctgtg tcttaaatac 4260
   tttaatgttg gaagagcata gtgtttgggc tagtgggttt ctgacagccc atgggaatgc 4320
   cctgaaacta ctgtatctga tgtttgtttt cgatgaggtt ccatgttttg ttttcttggg 4380
   aataaattaa tatattgttt tccaaaaaaa aaaaaaaaaa aaa 4423